# EUROPEAN PATENT SPECIFICATION

(11) **EP 3 307 870 B1**
(45) Date of publication and mention of the grant of the patent: **02.08.2023**
(21) Application number: 16733783.1
(22) Date of filing: 10.06.2016
(51) Int. Cl.: C12N 1/00, A61K 35/74

(54) **BACTERIA ENGINEERED TO TREAT DISORDERS INVOLVING THE CATABOLISM OF A BRANCHED CHAIN AMINO ACID**
MANIPULIERTE BAKTERIEN ZUR BEHANDLUNG EINER KRANKHEIT ODER EINER STÖRUNG
BACTÉRIES MANIPULÉES POUR LE TRAITEMENT D'UNE MALADIE OU D'UN TROUBLE

(30) Priority: 10.06.2015 US 201562173761 P; 13.05.2016 US 201662336338 P; 13.05.2016 WO PCT/US2016/032565
(43) Date of publication of application: 18.04.2018
(73) Proprietor: Synlogic Operating Company, Inc., County of New Castle, DE 19808 (US)
(72) Inventor: FALB, Dean, Sherborn, MA 01770 (US); MILLER, Paul, Salem, CT 06420 (US); MILLET, Yves, Newton, MA 02460 (US); ISABELLA, Vincent, Cambridge, MA 02139 (US); KOTULA, Jonathan, Berkeley, CA 94710 (US); TUCKER, Alex, Somerville, MA 02143 (US)
(74) Representative: Ford, Hazel
(86) International application number: PCT/US2016/037098
(87) International publication number: WO 2016/201380

(56) References cited:
- KARSTEN LANG ET AL: "Metabolic engineering of Pseudomonas sp. strain VLB120 as platform biocatalyst for the production of isobutyric acid and other secondary metabolites", MICROBIAL CELL FACTORIES, BIOMED CENTRAL, GB, vol. 13, no. 1, 7 January 2014 (2014-01-07), page 2, XP021172488, ISSN: 1475-2859, DOI: 10.1186/1475-2859-13-2
- AKITA HIRONAGA ET AL: "Bacterial production of isobutanol without expensive reagents", APPLIED MICROBIOLOGY AND BIOTECHNOLOGY, SPRINGER, DE, vol. 99, no. 2, 31 October 2014 (2014-10-31), pages 991-999, XP035435528, ISSN: 0175-7598, DOI: 10.1007/S00253-014-6173-X [retrieved on 2014-10-31]
- DAVOODI J ET AL: "Overexpression and characterization of the human mitochondrial and cytosolic branched-chain aminotransferases", JOURNAL OF BIOLOGICAL CHEMISTRY, AMERICAN SOCIETY FOR BIOCHEMISTRY AND MOLECULAR BIOLOGY, US, vol. 273, no. 9, 27 February 1998 (1998-02-27), pages 4982-4989, XP002266434, ISSN: 0021-9258, DOI: 10.1074/JBC.273.9.4982
- HENGST DEN C D ET AL: "IDENTIFICATION AND FUNCTIONAL CHARACTERIZATION OF THE LACTOCOCCUS LACTIS CODY-REGULATED BRANCHED-CHAIN AMINO ACID PERMEASE BCAP (CTRA)", JOURNAL OF BACTERIOLOGY, AMERICAN SOCIETY FOR MICROBIOLOGY, US, vol. 188, no. 9, 1 May 2006 (2006-05-01), pages 3280-3289, XP008065531, ISSN: 0021-9193, DOI: 10.1128/JB.188.9.3280-3289.2006
- J. M. BACHER ET AL: "Genetic Code Ambiguity Confers a Selective Advantage on Acinetobacter baylyi", JOURNAL OF BACTERIOLOGY, vol. 189, no. 17, 1 September 2007 (2007-09-01), pages 6494-6496, XP055304178, US ISSN: 0021-9193, DOI: 10.1128/JB.00622-07
- BABA TOMOYA ET AL: "Construction of Escherichia coli K-12 in-frame, single-gene knockout mutants: the Keio collection", MOLECULAR SYSTEMS BIOLOGY, THE MACMILLAN BUILDING, LONDON, GB, vol. 2, 1 February 2006 (2006-02-01), XP002519600, ISSN: 1744-4292, DOI: 10.1038/MSB4100050 [retrieved on 2006-02-21]
- L. C. BURRAGE ET AL: "Branched-chain amino acid metabolism: from rare Mendelian diseases to more common disorders", HUMAN MOLECULAR GENETICS, vol. 23, no. R1, 20 March 2014 (2014-03-20), pages R1-R8, XP055304583, gb ISSN: 0964-6906, DOI: 10.1093/hmg/ddu123
- INA KNERR ET AL: "Advances and challenges in the treatment of branched-chain amino/keto acid metabolic defects", JOURNAL OF INHERITED METABOLIC DISEASE, KLUWER ACADEMIC PUBLISHERS, DO, vol. 35, no. 1, 3 February 2011 (2011-02-03), pages 29-40, XP019994128, ISSN: 1573-2665, DOI: 10.1007/S10545-010-9269-1

## Description

### Field of the Invention

The invention relates to compositions for reducing one or more branched chain amino acids.

### Background

The three branched chain amino acids (BCAAs), leucine, isoleucine, and valine, play an important role in the metabolism of living organisms. Transamination of branched chain amino acids gives rise to their corresponding branched chain α-keto acids (BCKAs) (α-keto-β-methylvalerate, α-ketoisocaproate, and α-ketoisovalerate), which undergo further oxidative decarboxylation to produce acyl-CoA derivatives that enter the TCA cycle. Branched chain amino acids provide a nonspecific carbon source of oxidation for production of energy and also act as a precursor for muscle protein synthesis (Monirujjaman and Ferdouse, Advances in Molec. Biol., 2014, Article ID 36976, 6 pages, 2014).

Enzyme deficiencies or mutations which lead to the toxic accumulation of branched chain amino acids and their corresponding alpha-keto acids in the blood, cerebrospinal fluid, and tissues result in the development of metabolic disorders associated with the abnormal catabolism of branched chain amino acids in subjects, such as maple syrup urine disease (MSUD), isovaleric acidemia, propionic acidemia, methylmalonic acidemia, and diabetes ketoacidosis. Clinical manifestations of the disease vary depending on the degree of enzyme deficiency and include neurological dysfunction, seizures and death (Homanics *et al.* 2009).

Branched chain amino acids, such as leucine, or their corresponding alpha-keto acids, have also been linked to mTor activation (see, for example, Harlan et al., Cell Metabolism, 17:599-606, 2013) which is, in turn, associated with diseases such as cancer, obesity, type 2 diabetes, neurodegeneration, autism, Alzheimer's disease, Lymphangioleiomyomatosis (LAM), transplant rejection, glycogen storage disease, obesity, tuberous sclerosis, hypertension, cardiovascular disease, hypothalamic activation, musculoskeletal disease, Parkinson's disease, Huntington's disease, psoriasis, rheumatoid arthritis, lupus, multiple sclerosis, Leigh's syndrome, and Friedrich's ataxia (see Laplante and Sabatini, Cell, 149(2):74-293, 2012).

Currently available treatments for disorders involving the catabolism of branched chain amino acids are inadequate for the long term management of the disorders and have severe limitations (Svkvorak, J. Inherit. Metab. Dis., 32(2):229-246, 2009). A low protein/BCAA-restricted diet, with micronutrient and vitamin supplementation, as necessary, is the widely accepted long-term disease management strategy for many such disorders (Homanics et al., BMC Med. Genet., 7:33, 2006). However, BCAA-intake restrictions can be particularly problematic since branched chain amino acids can only be acquired through diet and are necessary for metabolic activities including protein synthesis and branched-chain fatty acid synthesis (Skvorak, 2009). Thus, even with proper monitoring and patient compliance, branched chain amino acid dietary restrictions result in a high incidence of mental retardation and mortality (Skvorak, 2009; Homanics *et al.,* 2009). A few cases of MSUD have been treated by liver transplantation (Popescu and Dima, Liver Transpl., 1:22-28, 2012). However, the limited availability of donor organs, the costs associated with the transplantation itself, and the undesirable effects associated with continued immunosuppressant therapy limit the practicality of liver transplantation for treatment of disorders involving the catabolism of a branched chain amino acid (Homanics et al., 2012; Popescu and Dima, 2012). Therefore, there is significant unmet need for effective, reliable, and/or long-term treatment for disorders involving the catabolism of branched chain amino acids.
Lang et al (Microbial Cell Factories 2014, 13:2) relates to the engineering of *Pseudomonas* sp. Strain VLB120 as platform biocatalyst for the production of isobutyric acid and other secondary metabolites. Akita et al (Appl Microbiol Biotechnol (2015) 99:991-999) discusses the bacterial production of isobutanol. Davoodi et al (J Biol Chem (1998) 273:4982-4989) relates to overexpression systems for human branched-chain aminotransferase isoenzymes. Den Hengst et al (J Bacteriology (2006) 188:3280-3289) relates to the *Lactococcus lactis* CodY-Regulated Branched-Chain Amino Acid Permease BcaP (CtrA). Bacher et al (J Bacteriology (2007) 189:6494-6496) relates to strains of *Acinetobacter baylyi* that may have a growth rate advantage. Baba et al (Molecular Systems Biology (2006) 2:2006.0008) relates to single gene deletions of non-essential genes in *Escherichia coli* K-12.

### Summary of the Invention

The present invention provides a pharmaceutical composition comprising a bacterium comprising gene sequence(s) encoding one or more branched chain amino acid catabolism enzyme(s) operably linked to a promoter that is not associated with the branched chain amino acid catabolism enzyme gene in nature, and gene sequence(s) encoding one or more transporter(s) of a branched chain amino acid that is operably linked to a promoter that is not associated with the transporter gene in nature, wherein the transporter is capable of importing the branched chain amino acid or metabolite thereof.

### Detailed Description

The technical information set out below may in some respects go beyond the scope of the invention, which is defined by the appended claims. The additional technical information is provided to place the actual invention in a broader technical context and to illustrate possible related technical developments.
Any incidental references to methods for treatment of the human or animal body by surgery or therapy and diagnostic methods practised on the human or animal body are not to be construed as claiming protection for such methods as such, but are instead to be construed as referring to products, in particular substances or compositions, for use in any of these methods.

The present disclosure relates to compositions and therapies for reducing one or more excess branched chain amino acids, and/or an accumulated metabolite(s) thereof, for example, by converting the one or more excess branched chain amino acid(s) or accumulated melabolite(s) into alternate by product(s). In certain aspects, the disclosure relates to genetically engineered microorganisms, e.g., bacteria or viruses, that are capable of reducing one or more excess branched chain amino acids, and/or an accumulated metabolite(s) thereof, particularly in low-oxygen conditions. such as in the mammalian gut. In certain aspects, the compositions and methods disclosed herein may be used for modulating excess branched chain amino acids and/or an accumulated metabolite(s) thereof. In certain aspects, the compositions disclosed herein may be used to treat disorders associated with excess branched chain amino acids and/or an accumulated metabolite(s) thereof, e.g., MMSUD, isovaleric acidemia (IVA), propionic acidemia, methylmalonic acidemia, and diabetes, ketoacidosis, as well as other diseases, for example, 3-MCC Deficiency, 3-Methylglutaconyl-CoA hydratase Deficiency, HMG-CoA Lyase Deficiency, Acetyl-CoA Carboxylase Deficiency, Malonyl-CoA Decarboxylase Deficiency, short-branched chain acylCoA dehydrogenase deficiency, 2-methyl-3-hydroxybutyric acidemia, beta-ketothiolase deficiency, isobutyryl-CoA dehydrogenase deficiency, HIBCH deficiency), and 3-Hydroxyisobutyric aciduria.

In certain aspects, the invention provides genetically engineered bacteria that are capable of reducing one or more branched chain amino acids (BCAA) or metabolite(s) thereof. In some aspects, the engineered bacteria can convert the BCAA, or metabolite thereof, into one or more alternate byproduct(s). In some aspects, the branched chain amino acid(s) or metabolite(s) thereof are present in excess amount(s) compared with a normal or reference range amount. For example, in certain aspects, the invention provides genetically engineered bacteria that are capable of reducing one or more leucine, isoleucine, and/or valine or a metabolite(s) thereof, including, for example, α-ketoisocaproate, α-keto-β-methylvalerate, and/or α-ketovalerate.In certain embodiments, the genetically engineered bacteria reduce excess BCAA and convert BCAA, or one or more metabolites thereof, into alternate byproducts selectively in low-oxygen environments, e.g., in the gut. The genetically engineered bacteria are non-pathogenic and may be introduced into the gut in order to reduce excess levels of BCAA. Another aspect of the disclosure provides methods for selecting or targeting genetically engineered bacteria based on increased levels of BCAA or metabolite consumption, and/or increase of uptake of branched chain amino acid into the bacterial cell. The disclosure also provides pharmaceutical compositions comprising the genetically engineered bacteria, methods for modulating the levels of BCAA(s), e.g, reducing excess levels of BCAA(s), and compositions for use in treating diseases or disorders associated with one or more excess BCAA(s), e.g., MSUD, isovaleric acidemia (IVA), propionic acidemia, methylmalonic acidemia, diabetes ketoacidosis, 3-MCC Deficiency, 3-Methylglutaconyl-CoA hydratase Deficiency, HMG-CoA Lyase Deficiency, Acetyl-CoA Carboxylase Deficiency, Malonyl-CoA Decarboxylase Deficiency, short-branched chain acylCoA dehydrogenase deficiency, 2-methyl-3-hydroxybutyric acidemia, beta-ketothiolase deficiency, isobutyryl-CoA dehydrogenase deficiency, HIBCH deficiency), and 3-Hydroxyisobutyric aciduria.

The present disclosure provides recombinant microorganisms that have been engineered with genetic circuitry which allow the recombinant microroganism to import and/or metabolize one or more branched chain amino acid and/or one or more metabolite(s) thereof. The invention relates to a pharmaceutical composition comprising a bacterium comprising gene sequence(s) encoding one or more branched chain amino acid catabolism enzyme(s) operably linked to a promoter that is not associated with the branched chain amino acid catabolism enzyme gene in nature, and gene sequence(s) encoding one or more transporter(s) of a branched chain amino acid that is operably linked to a promoter that is not associated with the transporter gene in nature, wherein the transporter is capable of importing the branched chain amino acid or metabolite thereof.

In some embodiments, the engineered microorganism is capable of sensing a patient's internal environment, e.g., the gut, and responding by turning an engineered metabolic pathway on or off. When turned on, the engineered microorganism, e.g., bacterial or virus cell, expresses one or more enzymes in a metabolic pathway to achieve a therapeutic effect in a host subject,

In certain aspects, the present disclosure provides engineered bacterial cells, pharmaceutical compositions thereof, and methods of modulating BCAA(s) and/or metabolite(s) thereof and treating diseases associated with the catabolism of branched chain amino acids. Specifically, the engineered bacteria disclosed herein have been modified to comprise gene sequence(s) encoding one or more enzymes involved in branched chain amino acid catabolism, as well as other circuitry, e.g., to regulate gene expression, including, for example, sequences for one or more inducible promoter(s), sequences for importing one or more BCAA(s) and/or metabolite(s) thereof into the bacterial cell (e.g., transporter sequence(s)), sequences for the secretion or non-sceretion of BCAA(s), metabolites or byproducts (e.g., exporter(s) or exporter knockouts), and circuitry to guarantee the safety and non-colonization of the subject that is administered the recombinant bacteria, such as auxotrophies, kill switches, etc. These engineered bacteria are safe and well tolerated and augment the innate activities of the subject's microbiome to achieve a therapeutic effect.

In some embodiments, the present disclosure provides a bacterium comprising gene sequence(s) encoding one of more branched chain amino acid catabolism enzyme(s). In the invention as claimed, the bacterium comprises gene sequence(s) encoding one or more branched chain amino acid catabolism enzytne(s)operably linked to a directly or indirectly inducible promoter that is not associated with the branched chain amino acid catabolism enzyme gene in nature. In some embodiments, the present disclosure provides a bacterium comprising gene sequence(s) encoding one or more branched chain amino acid catabolism enzyme(s) that are capable of convening a branched chain amino acid α-ketoacid, e.g., a-ketoisocaproate, α-keto-β-methylvalerate, and/or α-ketoisovalerate to its corresponding branched chain amino acid aldehyde, e.g., isovaleraldehyde, 2-methylbulyraldehyde, and/or isobutyraldehyde, respectively. In some embodiments, the bacterium comprises gene sequence(s) encoding one or more α-ketoocid decarboxylase(s). In some embodiments, the α-ketoacid decarboxylase is KivD, e.g., the bacterium comprises gene sequence(s) encoding one or more *kivD* genes. In some embodiments, the *kivD* gene is derived from a *Lactococcus lactis*, e.g., lactococcus lactis IFPL730.

In some embodiments, the bacterium comprises gene sequence(s) encoding one or more branched chain amino acid catabolism enzyme(s) that are capable of converting leucine, isoleucine and/or valine to α-ketoisocaproate, α-keto-β-methylvalerate, and/or α-ketoisovalerate, respectively. In some embodiments, the bacterium comprises gene sequence(s) encoding one or more branched chain amino acid deamination enzymes. In some embodiments, the branched chain amino acid deamination enzyme is selected from a branched chain amino acid dehydrogenase, branched chain amino acid aminotransferase, and amino acid oxidase. Thus, in some embodiments, the bacterium comprises gene sequence(s) encoding a gene selected from a branched chain amino acid dehydrogenase, branched chain amino acid aminotransferase, and amino acid oxidase. In some embodiments, the branched chain amino acid dehydrogenase is leucine dehydrogenase. In some embodiments, the leucine dehydrogenase is a *bacillus cereus* leucine dehydrogenase. In some embodiments, the branched chain amino acid aminotransferase is *ilvE.* In some embodiments, the amino acid oxidase is *L-AAD.* In some embodiments, the *L-AAD* gene is derived *from proteus vulgaris* or *proteus mirabilis.*

In some embodiments, the present disclosure provides a bacterium comprising gene sequence(s) encoding one or more branched chain amino acid catabolism enzyme(s) that are capable of converting a branched chain amino acid α-ketoacid, e.g., α-ketoisocaproate, α-keto-β-methylvalerate, and/or α-ketoisovalerate to its corresponding branched chain amino acid aldehyde, e.g., isovaleraldehyde, 2-methylbutyraldehyde, and/or isobutyraldehyde, respectively and gene sequence(s) encoding one or more branched chain amino acid catabolism enzyme(s) that are capable of converting leucine, isoleucine and/or valine to α-ketoisocaproate, α-keto-β-methylvalerate, and/or α-ketoisovalerate, respectively. In some embodiments, the branched chain amino acid catabolism enzyme(s) capable of converting a branched chain amino acid α-ketoacid, to its corresponding branched chain amino acid aldehyde is a α-ketoacid decarboxylase. In some embodiments, the α-ketoacid decarboxylase is KivD, e.g., the bacterium comprises gene sequence(s) encoding one or more *kivD* genes. In some embodiments, the *kivD* gene is derived from a *Lactococcus lactis,* e.g., lactococcus lactis IFPL730. In some embodiments, the branched chain amino acid catabolism enzyme(s) capable of converting leucine, isoleucine and/or valine to their corresponding branched chain α-ketoacids is a branched chain amino acid deamination enzyme. In some embodiments, the branched chain amino acid deamination enzyme is a branched chain amino acid dehydrogenase, branched chain amino acid aminotransferase, and /or amino acid oxidase. Thus, in some embodiments, the bacterium comprises gene sequence(s) encoding a gene selected from a branched chain amino acid dehydrogenase, branched chain amino acid aminotransferase, and amino acid oxidase. In some embodiments, the branched chain amino acid dehydrogenase is leucine dehydrogenase. In some embodiments, the leucine dehydrogenase is a *bacillus cereus* leucine dehydrogenase. In some embodiments, the branched chain amino acid aminotransferase is *ilvE.* In some embodiments, the amino acid oxidase is *L-AAD.* In some embodiments, the *L-AAD* gene is derived from *proteus vulgaris* or *proteus mirabilis.*

In some embodiments, the bacterium comprises gene sequence(s) encoding one or more branched chain amino acid catabolism enzyme(s) that are capable of converting a branched chain amino acid aldehyde, e.g., isovaleraldehyde, isobutyraldehyde and/or 2-methylbutyraldehyde to its corresponding alcohol, e.g., isopentanol, isobutanol, and/or 2-methybutanol, respectively. In some embodiments, the bacterium comprises gene sequence(s) encoding one or more branched chain amino acid alcohol dehydrogenases. In some embodiments, the branched chain amino acid alcohol dehydrogenase gene is selected from *adh1, adh2, adh3, adh4, adh5, adh6, adh7, sfa1, and yqhD.* In some embodiments, the branched chain amino acid alcohol dehydrogenase gene is *adh2.* In some embodiments, the *adh2* is derived from S. *cerevisiae adh2.* In some embodiments, the branched chain amino acid alcohol dehydrogenase gene is *yqhD.* In some embodiments, the *yqhD* gene is derived from *E. Coli.* In any of these embodiments wherein the bacteria comprises gene sequence(s) encoding a branched chain amino acid alcohol dehydrogenase, the bacterium may further comprise gene sequence(s) encoding one or more branched chain amino acid catabolism enzyme(s) that are capable of converting a branched chain amino acid α-ketoacid to its corresponding branched chain amino acid aldehyde and/or gene sequence(s) encoding one or more branched chain amino acid catabolism enzyme(s) that are capable of converting leucine, isoleucine and/or valine to α-ketoisocaproate, α-keto-β-methylvalerate, and/or α-ketoisovalerate, respectively. In some embodiments, the α-ketoacid decarboxylase is KivD, e.g., the bacterium comprises gene sequence(s) encoding one or more *kivD* genes. In some embodiments, the branched chain amino acid deamination enzyme is a branched chain amino acid dehydrogenase, branched chain amino acid aminotransferase, and /or amino acid oxidase. Thus, in some embodiments, the bacterium comprises gene sequence(s) encoding a gene selected from a branched chain amino acid dehydrogenase, branched chain amino acid aminotransferase, and amino acid oxidase. In some embodiments, the branched chain amino acid dehydrogenase is leucine dehydrogenase, e.g., derived from *bacillus cereus.* In some embodiments, the branched chain amino acid aminotransferase is *ilvE.* In some embodiments, the amino acid oxidase is *L-AAD,* e.g., derived from *proteus vulgaris* or *proteus mirabilis.*

In some embodiments, the bacterium comprises gene sequence(s) encoding one or more branched chain amino acid catabolism enzyme(s) that are capable of converting isovaleraldehyde, isobutyraldehyde and/or 2-methylbutyraldehyde to isovalerate, isobutyrate, and/or 2-methybutyrate, respectively. In some embodiments, the branched chain amino acid catabolism enzyme that is capable of converting isovaleraldehyde, isobutyraldehyde and/or 2-methylbutyraldehyde to it corresponding branche chain amino acid carboxylaic acid ia an aldehyde dehydrogenase. Thus, in some embodiments, the bacterium comprises gene sequence(s) encoding one or more branched chain amino acid aldehyde dehydrogenases. In some embodiments, the branched chain amino acid aldehyde dehydrogenase gene is *padA.* In some embodiments, the *padA* is an *E. Coli padA.* In any of these embodiments wherein the bacteria comprises gene sequence(s) encoding a branched chain amino acid aldehyde dehydrogenase, the bacterium may further comprise gene sequence(s) encoding one or more branched chain amino acid catabolism enzyme(s) that are capable of converting a branched chain amino acid α-ketoacid to its corresponding branched chain amino acid aldehyde and/or gene sequence(s) encoding one or more branched chain amino acid catabolism enzyme(s) that are capable of converting leucine, isoleucine and/or valine to α-ketoisocaproate, α-keto-β-methylvalerate, and/or α-ketoisovalerate, respectively, and/or gene sequence(s) encoding one or more branched chain amino acid catabolism enzyme(s) that are capable of converting a branched chain amino acid aldehyde to its corresponding alcohol. In some embodiments, the α-ketoacid decarboxylase is KivD, e.g., the bacterium comprises gene sequence(s) encoding one or more *kivD* genes. In some embodiments, the branched chain amino acid deamination enzyme is a branched chain amino acid dehydrogenase, branched chain amino acid aminotransferase, and /or amino acid oxidase. Thus, in some embodiments, the bacterium comprises gene sequence(s) encoding a gene selected from a branched chain amino acid dehydrogenase, branched chain amino acid aminotransferase, and amino acid oxidase. In some embodiments, the branched chain amino acid dehydrogenase is leucine dehydrogenase, e.g., derived from *bacillus cereus.* In some embodiments, the branched chain amino acid aminotransferase is *ilvE.* In some embodiments, the amino acid oxidase is *L-AAD,* e.g., derived from *proteus vulgaris* or *proteus mirabilis.* In some embodiments, the bacterium comprises gene sequence(s) encoding one or more branched chain amino acid alcohol dehydrogenases, e.g., selected from *adh1, adh2, adh3, adh4, adh5, adh6, adh7, sfa1, and yqhD.* In some embodiments, the branched chain amino acid alcohol dehydrogenase gene is *adh2.* In some embodiments, the *adh2* is derived from *S*. *cerevisiae adh2.* In some embodiments, the branched chain amino acid alcohol dehydrogenase gene is *yqhD.* In some embodiments, the *yqhD* gene is derived from *E*. *Coli.*

In the invention , the bacterium comprises gene sequence(s) encoding one or more transporter(s) of a branched chain amino acid. In the invention , the bacterium comprises gene sequence(s) encoding one or more transporter(s) of a branched chain amino acid operably linked to a promoter that is not associated with the transporter gene in nature. In some embodiments, the promoter is a directly or indirectly inducible promoter. In some embodiments, the transporter of branched chain amino acid is selected from *livKHMGF* and *brnQ.* In some embodiments, the *livKHMGF* is an *E*. *Coli livKHMGF* gene. In some embodiments, the gene sequence encoding one or more transporters is present in a chromosome in the bacteria. In some embodiments, the gene sequence encoding one or more transporters is present in one or more plasmids.

In the invention , the bacterium comprises gene sequence(s) encoding one or more transporter(s) of a branched chain amino acid and gene sequence encoding one or more branched chain amino acid catabolism enzymes. In some embodiments, in which the bacterium comprises gene sequence(s) encoding one or more transporter(s) of a branched chain amino acid and gene sequence encoding one or more branched chain amino acid catabolism enzymes, the branched chain amino acid catabolism enzyme(s) is capable of converting α-ketoisocaproate, α-keto-β-methylvalerate, and/or α-ketoisovalerate to isovaleraldehyde, 2-methylbutyraldehyde, and/or isobutyraldehyde, respectively, e.g., the bacterium comprises gene sequence(s) encoding one or more α-ketoacid decarboxylase(s). In some embodiments, the α-ketoacid decarboxylase is *kivD.* In some embodiments, in which the bacterium comprises gene sequence(s) encoding one or more transporter(s) of a branched chain amino acid and gene sequence encoding one or more branched chain amino acid catabolism enzymes, the branched chain amino acid catabolism enzyme(s) is capable of converting leucine, isoleucine and/or valine to α-ketoisocaproate, α-keto-β-methylvalerate, and/or α-ketoisovalerate, respectively, e.g., the bacterium comprises gene sequence(s) encoding one or more branched chain amino acid deamination enzymes, for example, selected from a branched chain amino acid dehydrogenase, branched chain amino acid aminotransferase, and amino acid oxidase. In some embodiments, the branched chain amino acid dehydrogenase is leucine dehydrogenase. In some embodiments, the branched chain amino acid aminotransferase is *ilvE.* In some embodiments, the amino acid oxidase is *L-AAD.* In some embodiments, in which the bacterium comprises gene sequence(s) encoding one or more transporter(s) of a branched chain amino acid and gene sequence encoding one or more branched chain amino acid catabolism enzymes, the branched chain amino acid catabolism enzyme(s) is capable of converting isovaleraldehyde, isobutyraldehyde and/or 2-methylbutyraldehyde to isopentanol, isobutanol, and/or 2-methybutanol, respectively, e.g., is a branched chain amino acid alcohol dehydrogenases, for example, selected from *adh1, adh2, adh3, adh4, adh5, adh6, adh7, sfal,* and *yqhD* and/or is capable of converting isovaleraldehyde, isobutyraldehyde and/or 2-methylbutyraldehyde to isovalerate, isobutyrate, and/or 2-methybutyrate, respectively, e.g., is a branched chain amino acid aldehyde dehydrogenase, for example, *padA.*

Thus, in some embodiments, the bacterium comprising gene sequence(s) encoding one or more transporters of branched chain amino acids, e.g., *livKHMGF* and/or *brnQ* and gene sequence(s) encoding one or more α-ketoacid decarboxylase(s), e.g., *kivD.* In some embodiments, the bacterium comprising gene sequence(s) encoding one or more transporters of branched chain amino acids, e.g., *livKHMGF* and/or *brnQ,* and gene sequence(s) encoding *kivD.* In some embodiments, the bacterium comprising gene sequence(s) encoding one or more transporters of branched chain amino acids, e.g., *livKHMGF* and/or *brnQ,* and gene sequence(s) encoding one or more branched chain amino acid deamination enzymes, for example, selected from a branched chain amino acid dehydrogenase, e.g., LeuDH, branched chain amino acid aminotransferase, e.g., ilvE, and amino acid oxidase, e.g., *L-AAD.* In some embodiments, the bacterium comprising gene sequence(s) encoding one or more transporters of branched chain amino acids, e.g., *livKHMGF* and/or *brnQ,* and gene sequence(s) encoding one or more α-ketoacid decarboxylase(s), e.g., *kivD* and gene sequence(s) encoding a branched chain amino acid dehydrogenase, e.g., LeuDH, a branched chain amino acid aminotransferase, e.g., ilvE, and/or an amino acid oxidase, e.g., *L-AAD.* Thus, in some embodiments, the bacterium comprise gene sequence(s) encoding one or more transporters of branched chain amino acids, e.g., *livKHMGF* and/or *brnQ,* gene sequence(s) encoding *kivD,* and gene sequence(s) encoding LeuDH. In some embodiments, the bacterium comprise gene sequence(s) encoding one or more transporters of branched chain amino acids, e.g., *livKHMGF* and/or *brnQ,* gene sequence(s) encoding *kivD,* and gene sequence(s) encoding LeuDH and/or ilvE, and/or *L-AAD.* In some embodiments, the bacterium comprising gene sequence(s) encoding one or more transporters of branched chain amino acids, e.g., *livKHMGF* and/or *brnQ,* and gene sequence(s) encoding one or more branched chain amino acid alcohol dehydrogenases, for example, selected from *adh1, adh2, adh3, adh4, adh5, adh6, adh7, sfa1,* and *yqhD.* In some embodiments, the bacterium comprising gene sequence(s) encoding one or more transporters of branched chain amino acids, e.g., *livKHMGF* and/or *brnQ,* gene sequence(s) encoding one or more branched chain amino acid deamination enzymes, for example, selected from a branched chain amino acid dehydrogenase, e.g., LeuDH, branched chain amino acid aminotransferase, e.g., ilvE, and amino acid oxidase, e.g., *L-AAD,* and gene sequence(s) encoding one or more branched chain amino acid aldehyde dehydrogenase, for example, *padA* and/or gene sequence(s) encoding one or more branched chain amino acid alcohol dehydrogenases, for example, selected from *adh1, adh2, adh3, adh4, adh5, adh6, adh7, sfal,* and *yqhD.* In some embodiments, the bacterium comprising gene sequence(s) encoding one or more transporters of branched chain amino acids, e.g., *livKHMGF* and/or *brnQ,* gene sequence(s) encoding one or more α-ketoacid decarboxylase(s), e.g., *kivD,* and gene sequence(s) encoding one or more branched chain amino acid aldehyde dehydrogenase, for example, *padA* and/or gene sequence(s) encoding one or more branched chain amino acid alcohol dehydrogenases, for example, selected from *adh1, adh2, adh3, adh4, adh5, adh6, adh7, sfa1,* and *yqhD.* In some embodiments, the bacterium comprising gene sequence(s) encoding one or more transporters of branched chain amino acids, e.g., *livKHMGF* and/or *brnQ,* gene sequence(s) encoding one or more α-ketoacid decarboxylase(s), e.g., *kivD,* gene sequence(s) encoding one or more branched chain amino acid deamination enzymes, for example, selected from a branched chain amino acid dehydrogenase, e.g., LeuDH, branched chain amino acid aminotransferase, e.g., ilvE, and amino acid oxidase, e.g., *L-AAD,* and gene sequence(s) encoding one or more branched chain amino acid aldehyde dehydrogenase, for example, *padA* and/or gene sequence(s) encoding one or more branched chain amino acid alcohol dehydrogenases, for example, selected from *adh1, adh2, adh3, adh4, adh5, adh6, adh7, sfa1,* and *yqhD.* Thus, in some embodiments the bacterium comprises gene sequence(s) encoding one or more transporters of branched chain amino acids, e.g., *livKHMGF* and/or *brnQ,* gene sequence(s) encoding *kivD,* and gene sequence(s) encoding LeuDH and/or ilvE, and/or L-AAD. In some embodiments the bacterium comprises gene sequence(s) encoding one or more transporters of branched chain amino acids, e.g., *livKHMGF* and/or *brnQ,* gene sequence(s) encoding *kivD,* and gene sequence(s) encoding LeuDH. In some embodiments the bacterium comprises gene sequence(s) encoding one or more transporters of branched chain amino acids, e.g., *livKHMGF* and/or *brnQ,* gene sequence(s) encoding *kivD,* gene sequence(s) encoding LeuDH and/or ilvE, and/or L-AAD, and gene sequence encoding *padA.* In some embodiments the bacterium comprises gene sequence(s) encoding one or more transporters of branched chain amino acids, e.g., *livKHMGF* and/or *brnQ,* gene sequence(s) encoding *kivD,* gene sequence(s) encoding LeuDH, and gene sequence encoding *padA.* In some embodiments the bacterium comprises gene sequence(s) encoding one or more transporters of branched chain amino acids, e.g., *livKHMGF* and/or *brnQ,* gene sequence(s) encoding *kivD,* gene sequence(s) encoding LeuDH and/or ilvE, and/or L-AAD, and gene sequence encoding *adh2* or *yqhD.* In some embodiments the bacterium comprises gene sequence(s) encoding one or more transporters of branched chain amino acids, e.g., *livKHMGF* and/or *brnQ,* gene sequence(s) encoding *kivD,* gene sequence(s) encoding LeuDH, and gene sequence encoding *adh2* or *yqhD.* In some embodiments, the bacterium comprising gene sequence(s) encoding one or more transporters of branched chain amino acids, e.g., *livKHMGF* and/or *brnQ,* gene sequence(s) encoding *kivD,* gene sequence(s) encoding LeuDH and/or ilvE, and/or L-AAD, gene sequence encoding *adh2* or *yqhD,* and gene sequence encoding *padA.* In some embodiments, the bacterium comprising gene sequence(s) encoding one or more transporters of branched chain amino acids, e.g., *livKHMGF* and/or *brnQ,* gene sequence(s) encoding *kivD,* gene sequence(s) encoding LeuDH, gene sequence encoding *adh2* or *yqhD,* and gene sequence encoding *padA.*
In any of these embodiments, the bacterium further comprises a genetic modification that reduces export of a branched chain amino acid from the bacterium. In some embodiments, the genetic modification that reduces export of a branched chain amino acid from the bacterium is gene modification in the *leuE* gene, for example, the *leuE* gene is deleted from the bacterium. In any of these embodiments, the bacterium further comprises a genetic modification that reduces endogenous biosynthesis of a branched chain amino acid in the bacterium. In some embodiments, the genetic modification that reduces endogenous biosynthesis of a branched chain amino acid in the bacterium is a gene modification in the *ilvC* gene, e.g., the *ilvC* gene is deleted from the bacterium. In any of these embodiments, the bacterium further comprises gene sequence(s) encoding one or more branched chain amino acid binding protein(s), e.g., further comprises gene sequence(s) encoding *ilvJ.*
In any of these embodiments, wherein the promoter operably linked to the gene sequence(s) encoding a branched chain amino acid catabolism enzyme and the promoter operably linked to the gene sequence(s) encoding a transporter of a branched chain amino acid are separate copies of the same promoter. In some embodiments, the promoter operably linked to the gene sequence(s) encoding a branched chain amino acid catabolism enzyme and the promoter operably linked to the gene sequence(s) encoding a transporter of a branched chain amino acid are the same copy of the same promoter. Ins some embodiments, the promoter operably linked to the gene sequence(s) encoding a branched chain amino acid catabolism enzyme and the promoter operably linked to the gene sequence(s) encoding a transporter of a branched chain amino acid are different promoters. In some embodiment, the promoter operably linked to the gene sequence(s) encoding a branched chain amino acid catabolism enzyme is directly or indirectly induced by exogenous environmental conditions found in the mammalian gut. In some embodiments, the promoter operably linked to the gene sequence(s) encoding a branched chain amino acid catabolism enzyme is directly or indirectly induced under low-oxygen or anaerobic conditions. In some embodiments, the promoter operably linked to the gene sequence(s) encoding a branched chain amino acid catabolism enzyme is selected from the group consisting of an FNR-responsive promoter, an ANR-responsive promoter, and a DNR-responsive promoter. In some embodiments, the promoter operably linked to the gene sequence(s) encoding a branched chain amino acid catabolism enzyme is an FNRS promoter. In some embodiments, he promoter operably linked to the gene sequence(s) encoding a transporter of a branched chain amino acid is directly or indirectly induced by exogenous environmental conditions found in the mammalian gut. In some embodiments, the promoter operably linked to the gene sequence(s) encoding a transporter of a branched chain amino acid is directly or indirectly induced under low-oxygen or anaerobic conditions. In some embodiments, the promoter operably linked to the gene sequence(s) encoding a transporter of a branched chain amino acid is selected from the group consisting of an FNR-responsive promoter, an ANR-responsive promoter, and a DNR-responsive promoter. In some mebodiments, the promoter operably linked to the gene sequence(s) encoding a transporter of a branched chain amino acid catabolism enzyme is an FNRS promoter.

In some embodiments, the gene sequence(s) encoding a branched chain amino acid catabolism enzyme is located on a chromosome in the bacterium. In some embodiments, the gene sequence(s) encoding a branched chain amino acid catabolism enzyme is located on a plasmid in the bacterium. In some embodiments, at least one gene sequence(s) encoding a branched chain amino acid catabolism enzyme is located on a plasmid in the bacterium and at least one gene sequence(s) encoding a branched chain amino acid catabolism enzyme is located on a chromosome in the bacterium. In some embodiments, the gene sequence(s) encoding a transporter of a branched chain amino acid is located on a chromosome in the bacterium. In some embodiments, the gene sequence(s) encoding a transporter of a branched chain amino acid is located on a plasmid in the bacterium.
In some embodiments, the gene sequence(s) encoding a transporter of a branched chain amino acid is located on a plasmid in the bacterium and at least one gene sequence(s) encoding a transporter of a branched chain amino acid is located on a chromosome in the bacterium.
In any of these embodiments, the bacterium is an auxotroph in diaminopimelic acid or an enzyme in the thymidine biosynthetic pathway. In any of these embodiments, the bacterium is further engineered to harbor a gene encoding a substance toxic to the bacterium, wherein the gene is under the control of a promoter that is directly or indirectly induced by an environmental factor not naturally present in a mammalian gut.
The present disclosure provides a composition for use in a method of reducing the level of a branched amino acid or treating a disease associated with excess branched chain amino acid comprising the step of administering to a subject in need thereof, a composition comprising any of the bacterium described herein. In some embodiments, the disclosure provides a composition for use in reducing the level of a branched amino acid metabolite or treating a disease associated with excess branched chain amino acid metabolite comprising the step of administering to a subject in need thereof, a composition comprising any of the bacterium described herein. In some embodiments, the branch chain amino acid metabolite is selected from α-ketoisocaproate, α-keto-β-methylyalerate, and α-ketoisovalerate. In some embodiments, the disease is selected from the group consisting of: MSUD, isovaleric acidemia (IVA), propionic acidemia, methylmalonic acidemia, and diabetes ketoacidosis, as well as other diseases, for example, 3-MCC Deficiency, 3-Methylglutaconyl-CoA hydratase Deficiency, HMG-CoA Lyase Deficiency, Acetyl-CoA Carboxylase Deficiency, Malonyl-CoA Decarboxylase Deficiency, short-branched chain acylCoA dehydrogenase deficiency, 2-methyl-3-hydroxybutyric acidemia, beta-ketothiolase deficiency, isobutyryl-CoA dehydrogenase deficiency, HIBCH deficiency), and 3-Hydroxyisobutyric aciduria. In some embodiments, the disclosure provides for treating a metabolic disorder involving the abnormal catabolism of a branched amino acid in a subject, comprising administering a composition comprising any of the bacterium described herein and thereby treating the metabolic disorder involving the abnormal catabolism of a branched chain amino acid in the subject.

### Brief Description of the Drawings

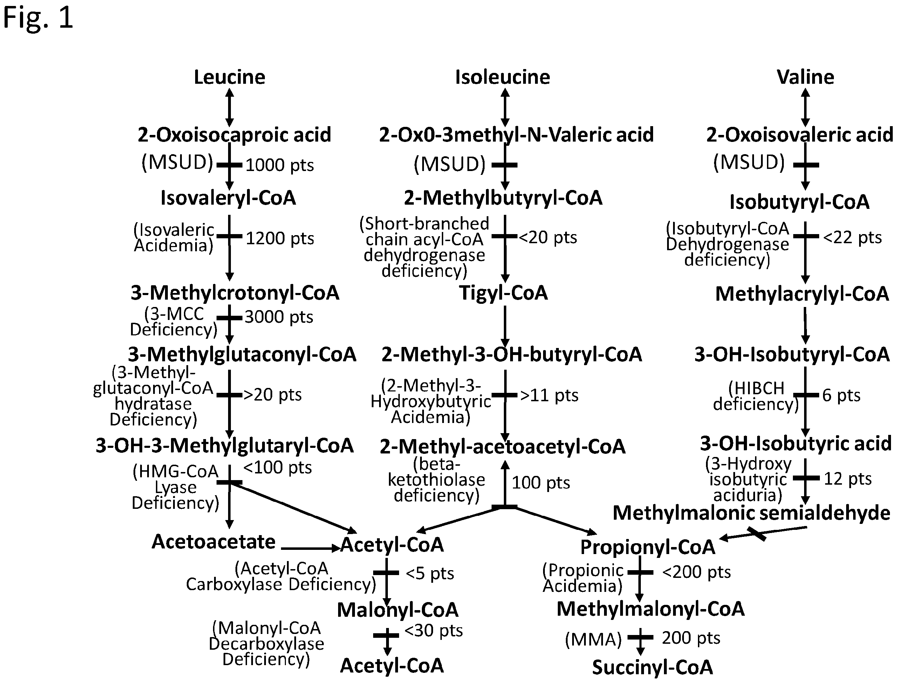

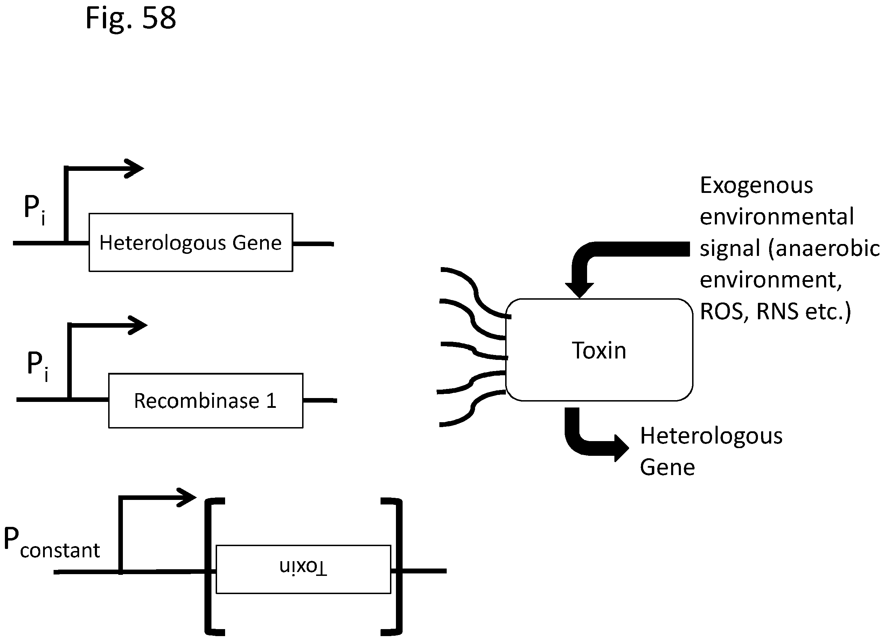

**Fig. 1** depicts various branched chain amino acid degradative pathways and the metabolites and associated diseases relating to BCAA metabolism.

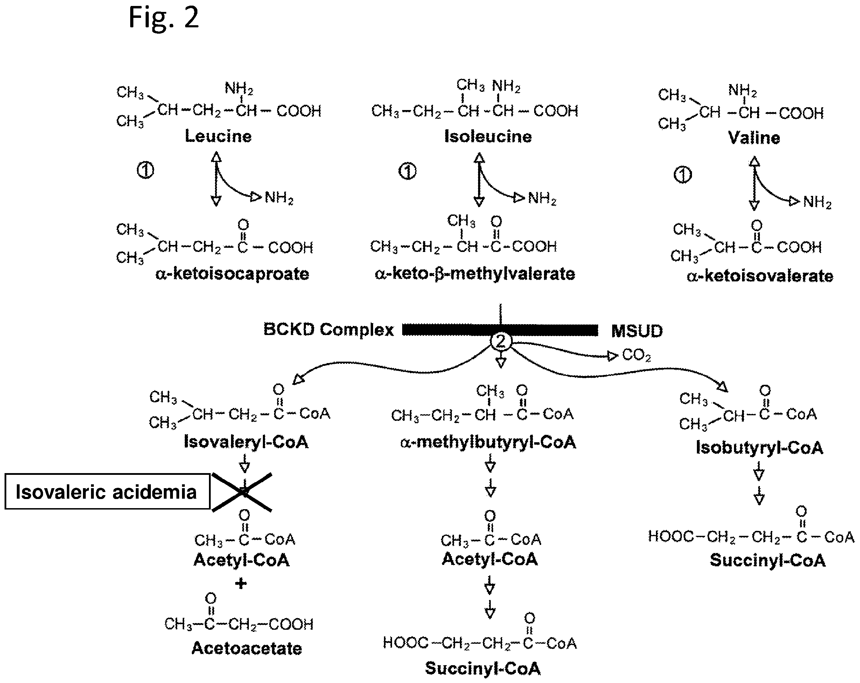

**Fig. 2** depicts aspects of the branched chain amino acid degradative pathways for leucine, isoleucine, and valine.

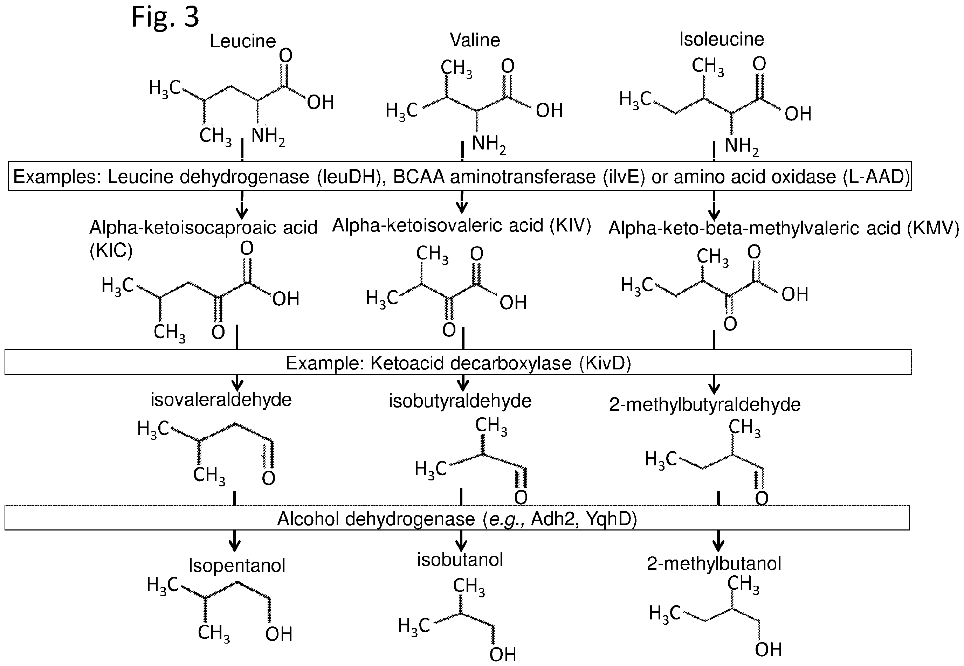

**Fig. 3** depicts aspects of alternate branched chain amino acid degradative pathways for leucine, isoleucine, and valine involving a ketoacid decarboxylase and an alcohol dehydrogenase, resulting in isopentanol, isobutanol, and 2-methylbutanol, respectively.

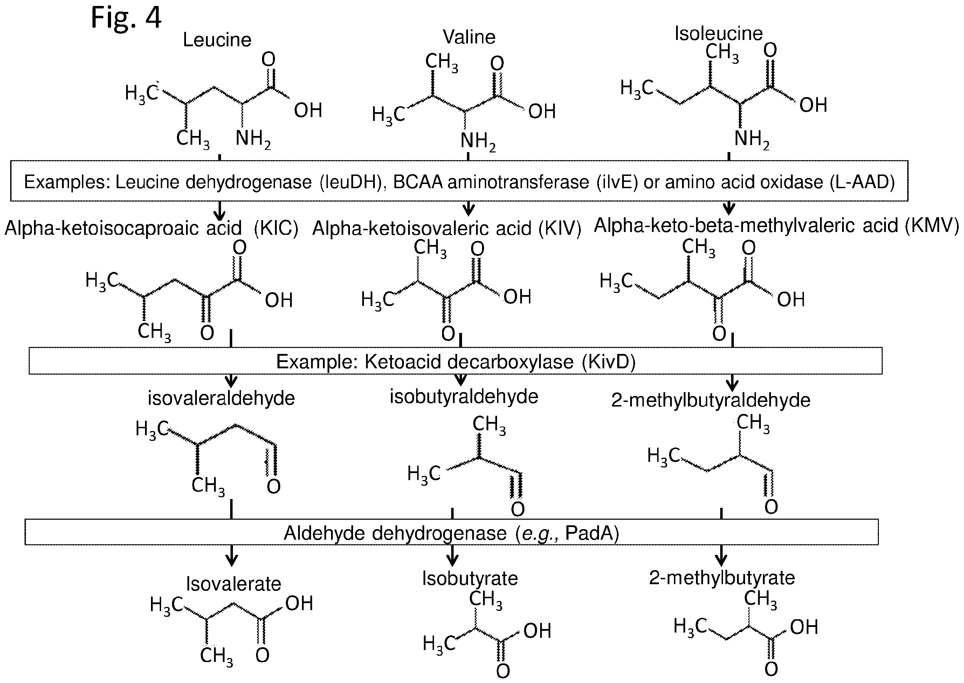

**Fig. 4** depicts aspects of alternate branched chain amino acid degradative pathways for leucine, isoleucine, and valine involving a ketoacid decarboxylase and an aldehyde dehydrogenase, resulting in isovalerate, isobutyrate, and 2-methylbutyrate, respectively.

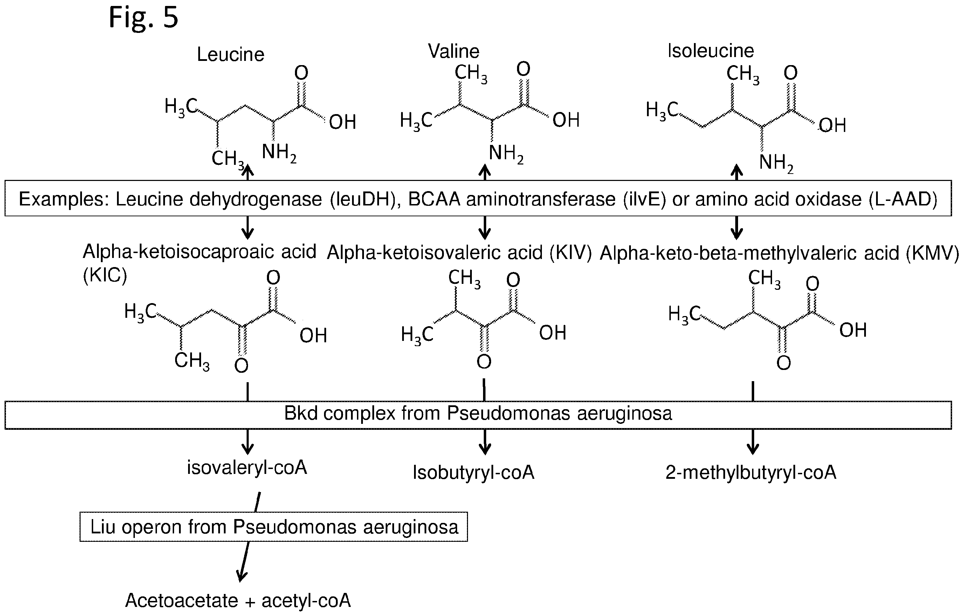

**Fig. 5****.** depicts aspects of alternate branched chain amino acid degradative pathways for leucine, isoleucine, and valine involving a branched chain keto acid dehydrogenase complex (bkd), and the Liu operon from Pseudomonas aeruginosa, resulting in the acylCoA derivative of BCAA. In the case of leucine, the Liu operon coverts isovalerylCoA into acetoacetate and acetyl CoA.

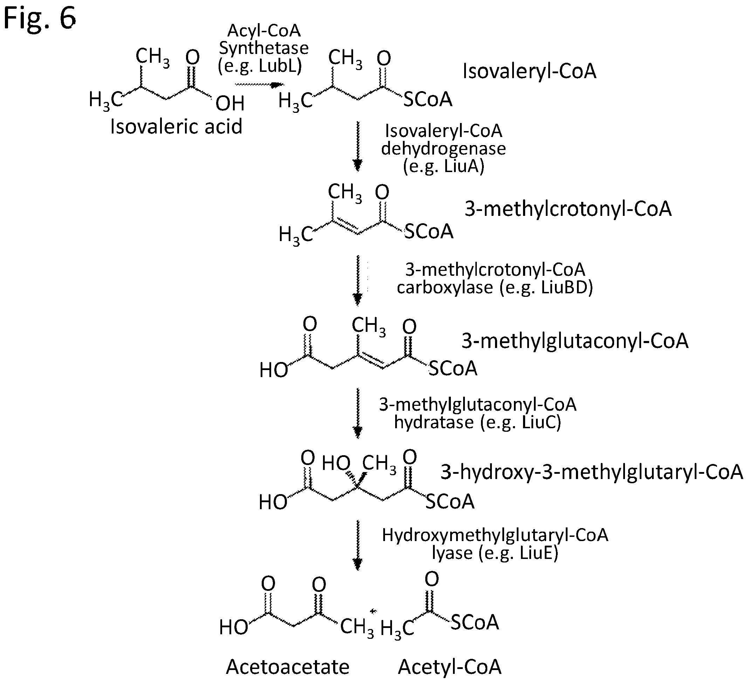

Fig. 6 depicts the the conversion of isovaleryCoA to acetoacetate and acetylCoA by the Liu operon enzymes. In the case of isovaleric acidemia, accumulating isovaleric acid can be activated into isoveralyCoA by an acylCoA synthetase, such as LbuL from Steptomyces lividans.

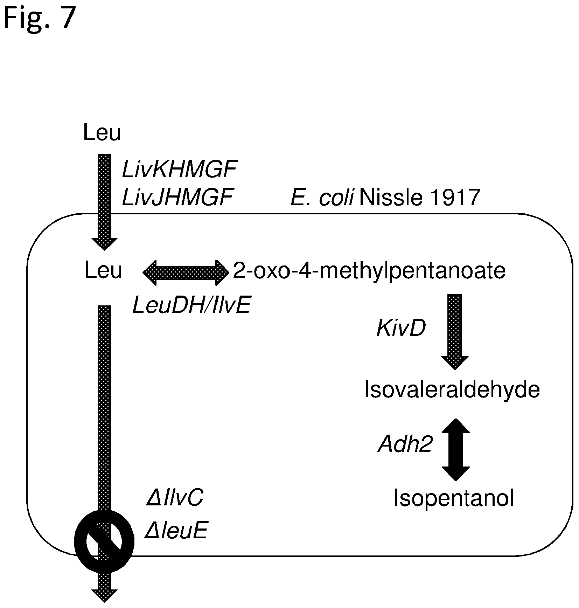

**Fig. 7** depicts possible components of a branched chain amino acid synthetic biotic disclosed herein. An exemplary modified bacterium (*E*. *Coli Nissle* 1917) for metabolizing leucine to isopentanol may comprise gene sequence(s) for encoding one or more of the following: (1) livKHMGF (a high affinity leucine transporter that can transport leucine into the bacterial cell); (2) LivJHMGF (a high affinity BCAA transporter that can transport leucine, isoleucine, and valine into the bacterial cell); (3) leuDH (leucine dehydrogenase, e.g., derived from *P*. *aeruginosa* PA01 or *Bacillus cereus* which converts the BCAA into its corresponding α-ketoacid); (4) IlvE (branched chain amino acid aminotransferase, which also converts BCAA into its corresponding α-ketoacid); (5) KivD (branched chain α-ketoacid decarboxylase, e.g., derived from *Lactococcus lactis* IFPL730, which converts the α-ketoacid to its corresponding aldehyde); and (6) Adh2 (an alcohol dehydrogenase, e.g., derived from *S. cerevisiae;* which converts the aldehyde to its corresponding alcohol). The bacterium may further be a gene knockout for the gene encoding LeuE (leucine exporter; knocking out this gene keeps intracellular leucine concentration high) and/or the gene encoding IlvC (keto acid reductoisomerase, which is required for BCAA synthesis; knocking out this gene creates an auxotroph and requires the bacterial cell to import isoleucine and valine to survive).

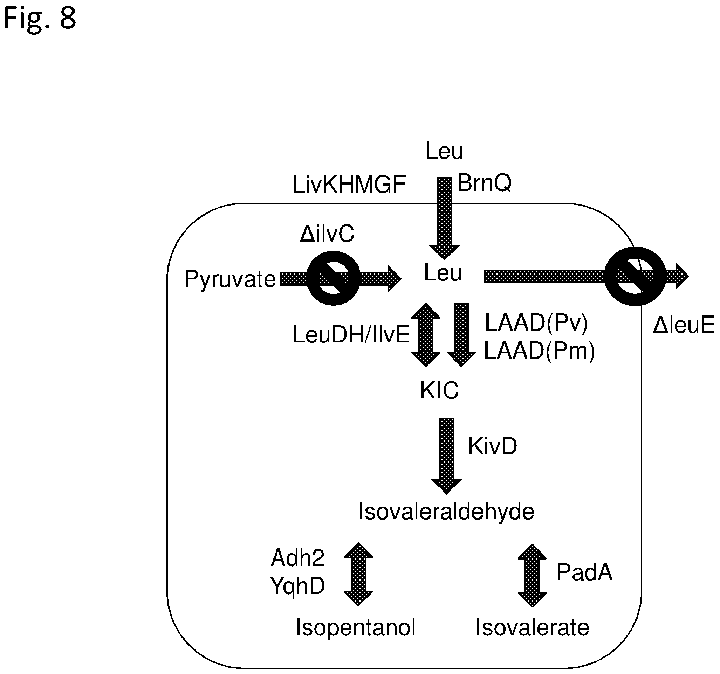

**Fig. 8** depicts possible components of a branched chain amino acid synthetic biotic disclosed herein. An exemplary modified bacterium for metabolizing leucine to isopentanol may comprise gene sequence(s) for encoding one or more of the following: (1) livKHMGF (a high affinity leucine transporter that can transport leucine into the bacterial cell); (2) BrnQ (a low affinity BCAA transporter that can transport branched chain amino acids into the bacterial cell); (3) leuDH (leucine dehydrogenase, e.g., derived from *P*. *aeruginosa* PA01 or *Bacillus cereus,* which converts the BCAA into its corresponding α-ketoacid); (4) IlvE (branched chain amino acid aminotransferase, which also converts BCAA into its corresponding α-ketoacid); (5) L-AAD (amino acid oxidase, which also converts BCAA into its corresponding α-ketoacid; LAAD(Pv)/LAAD(Pm) are from Proteus vulgaris and Proteus mirabilis, respectively); (6) KivD (branched chain α-ketoacid decarboxylase, e.g., derived from *Lactococcus lactis* IFPL730, which converts the α-ketoacid to its corresponding aldehyde); and (7) an alcohol dehydrogenase (e.g., Adh2, e.g., derived from S. *cerevisiae;* YghD, e.g., derived from *E*. *coli,* which converts the aldehyde to its corresponding alcohol). The bacterium may further be a gene knockout for the gene encoding LeuE (leucine exporter; knocking out this gene keeps intracellular leucine concentration high) and/or the gene encoding IlvC (keto acid reductoisomerase, which is required for BCAA synthesis; knocking out this gene creates an auxotroph and requires the bacterial cell to import isoleucin and valine to survive). An exemplary modified bacterium for metabolizing leucine to isovalerate may comprise gene sequence(s) for encoding one or more of the following: (1) livKHMGF (a high affinity leucine transporter that can transport leucine into the bacterial cell); (2) BrnQ (a low affinity BCAA transporter that can transport branched chain amino acids into the bacterial cell); (3) leudh (leucine dehydrogenase, e.g., derived from *P. aeruginosa* PA01 or *Bacillus cereus,* which converts the BCAA into its corresponding α-ketoacid); (4) IlvE (branched chain amino acid aminotransferase, which also converts BCAA into its corresponding α-ketoacid); (5) L-AAD (amino acid oxidase, which also converts BCAA into its corresponding α-ketoacid); (6) KivD (branched chain α-ketoacid decarboxylase, e.g., derived from *Lactococcus lactis* IFPL730, which converts the α-ketoacid to its corresponding aldehyde); and (7) an aldehyde dehydrogenase (e.g., PadA, e.g., derived from *E. coli* K12, which converts the aldehyde to its corresponding carboxylic acid). The bacterium may further be a gene knockout for the gene encoding LeuE (leucine exporter; knocking out this gene keeps intracellular leucine concentration high) and/or the gene encoding IlvC (keto acid reductoisomerase, which is required for BCAA synthesis; knocking out this gene creates an auxotroph and requires the bacterial cell to import isoleucine and valine to survive).

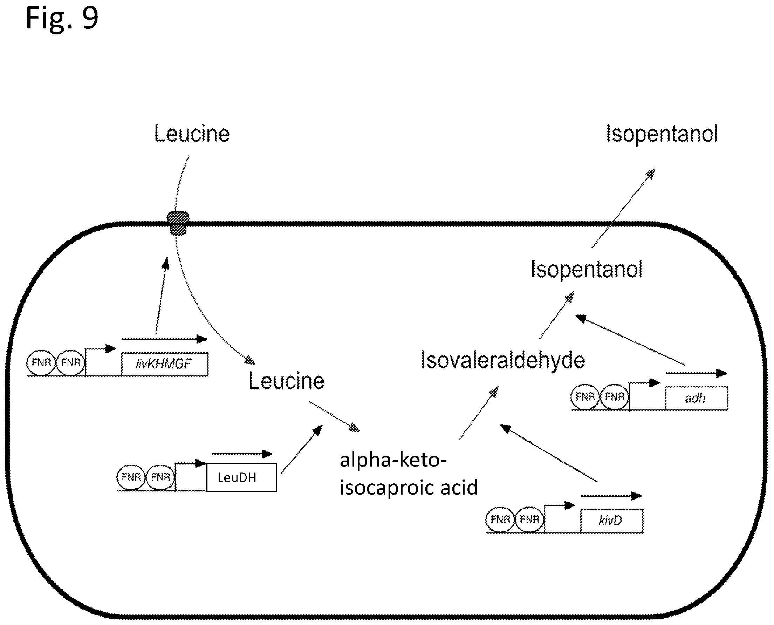

**Fig. 9** depicts possible components of a branched chain amino acid synthetic biotic disclosed herein. An exemplary modified bacterium for metabolizing leucine to isopentanol is shown. Leucine is transported into the bacterium via the high affinity leucine transporter, LivKHMGF, where it is converted to alpha-ketoisocaproic acid using leuDH (Leucine dehydrogenase). The alpha-ketoisocaproic acid is converted to isovalderaldehyde using KivD (BCAA α-ketoacid decarboxylase) and further converted to isopentanol using Adh (alcohol dehydrogenase 2). One or more of the catabolic enzymes, transporters, or other genes may be under the control of an inducible promoter that is induced under exogenous environmental conditions, such as any of the inducible promoters provided herein, e.g., a promoter induced under low oxygen or anaerobic conditions.

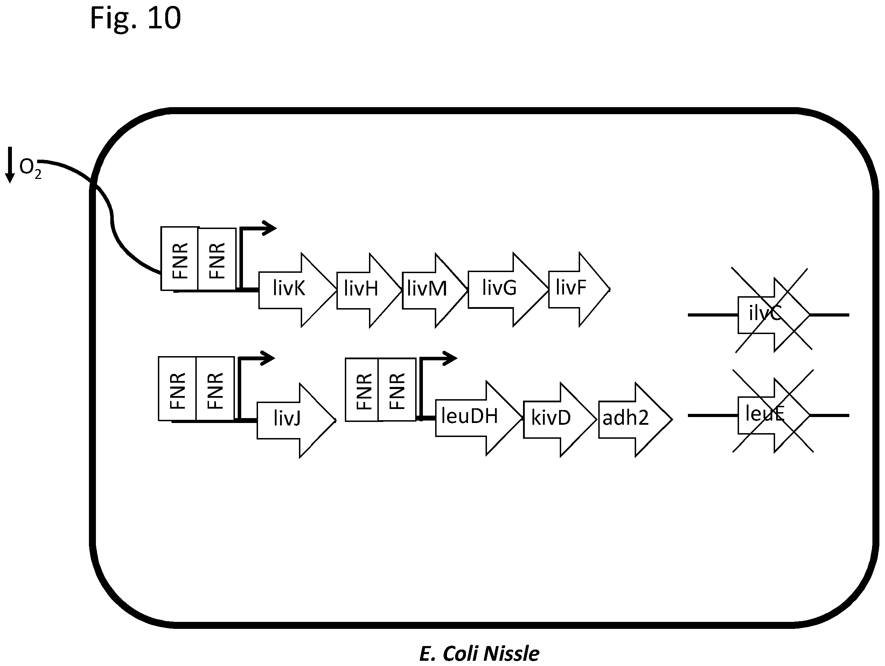

**Fig. 10** depicts one exemplary branched chain amino acid circuit. Genes shown are high affinity leucine transporter complex (LivKHMGF), leucine dehydrogenase (leuDH), e.g., from *Pseudomonas aeruginosa,* the branched chain α-ketoacid decarboxylase (KivD), e.g., from *Lactococcus lactis,* and alcohol dehydrogenase 2 (Adh2), e.g., from *Saccharomyces cerevisiae,.* The genes for the leucine exporter (LeuE) and IlvC (keto acid reductoisomerase, required for BCAA synthesis) have been deleted. The gene for LivJ (a BCAA binding protein that can transport branched chain amino acids into the bacterial cell) is added which can be under the control of the native promoter or the constitutive promoter Ptac. One or more of the genes encoding a catabolic enzyme, transporter, and/or other genes (e.g., livJ) may be under the control of an inducible promoter that is induced under exogenous environmental conditions, such as any of the inducible promoters provided herein, e.g., a promoter induced under low oxygen or anaerobic conditions.

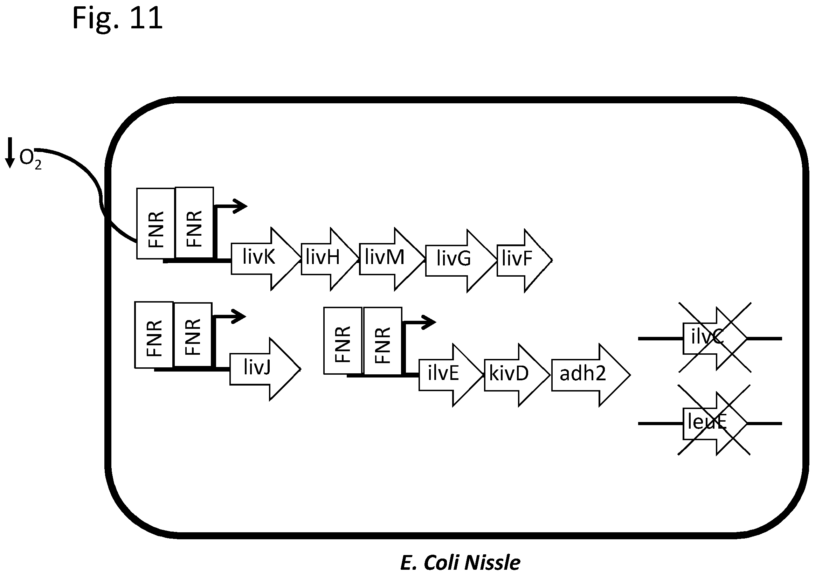

**Fig. 11** depicts one exemplary branched chain amino acid circuit. Genes shown are high affinity leucine transporter complex (LivKHMGF), BCAA amino transferase (ilvE), the branched chain α-ketoacid decarboxylase (KivD), e.g., from *Lactococcus lactis,* and alcohol dehydrogenase 2 (Adh2), e.g., from *Saccharomyces cerevisiae.* The genes for the leucine exporter (LeuE) and keto acid reductoisomerase (IlvC) have been deleted. The gene for LivJ is added which can be under the control of the native promoter or the constitutive promoter Ptac. One or more of the genes encoding a catabolic enzyme, transporter, and/or other genes (e.g., livJ) may be under the control of an inducible promoter that is induced under exogenous environmental conditions, such as any of the inducible promoters provided herein, e.g., a promoter induced under low oxygen or anaerobic conditions.

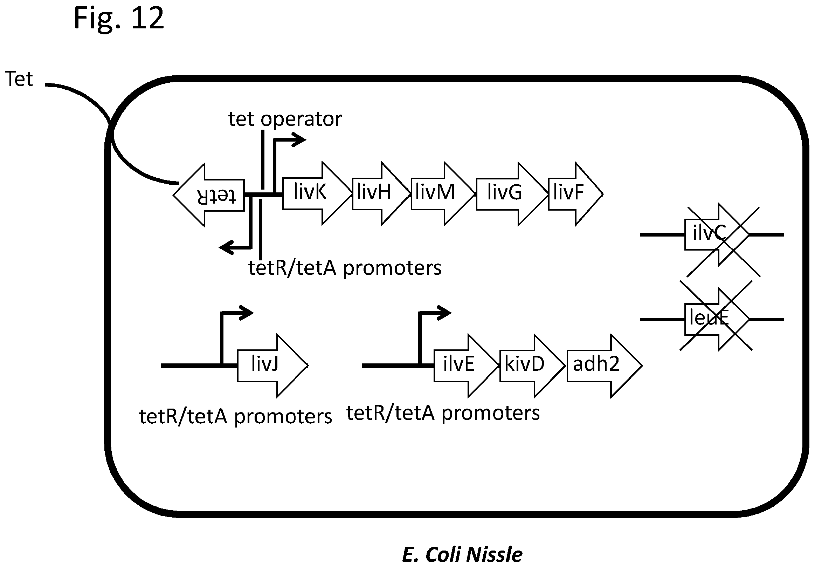

**Fig. 12** depicts one exemplary branched chain amino acid circuit. Genes shown are high affinity leucine transporter complex (LivKHMGF), BCAA amino transferase (ilvE), the branched chain α-ketoacid decarboxylase (KivD), e.g., from *Lactococcus lactis,* and alcohol dehydrogenase 2 (Adh2), e.g., from *Saccharomyces cerevisiae.* The genes for the leucine exporter (LeuE) and keto acid reductoisomerase (ilvC) have been deleted. The gene for LivJ is added which can be under the control of the native promoter or the constitutive promoter Ptac. One or more of the genes encoding a catabolic enzyme, transporter, and/or other genes (e.g., livJ) may be under the control of an inducible promoter that is induced under exogenous environmental conditions, such as any of the inducible promoters provided herein, e.g., a promoter induced under low oxygen or anaerobic conditions. In certain embodiments, any of the genes may be under the control of a tetR/tetA promoter. For example, the construct may comprise a (constitutive or inducible) promoter driving expression of the Tet repressor (TetR) from the tetR gene, which is linked to a second promoter comprising a TetR binding site that drives expression of any of the leucine import cassette(s) described above. TetR is (either constitutively or inducibly) expressed and inhibits the expression of the leucine import cassette(s). Upon addition of anhydrotetracylcine (ATC), TetR binds to ATC removing the inhibition by TetR allowing expression of the the leucine import cassette(s).

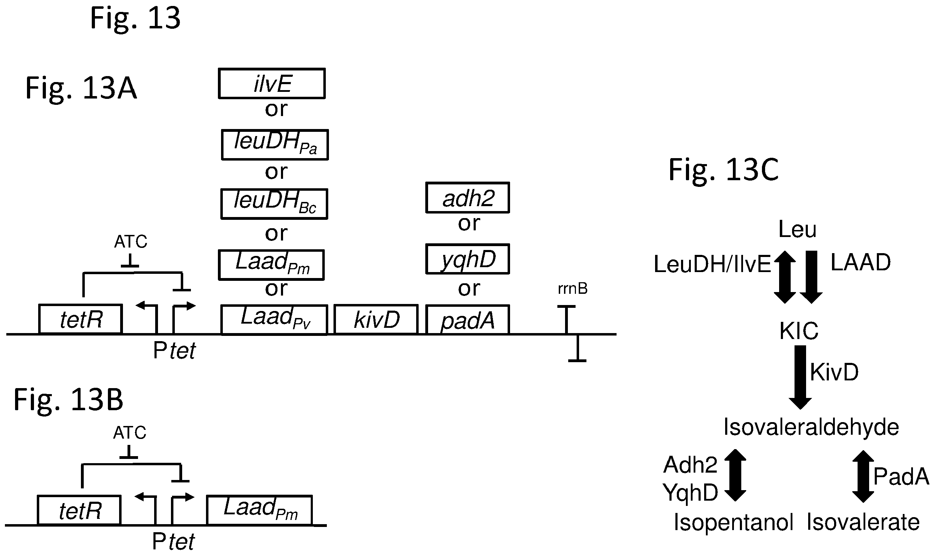

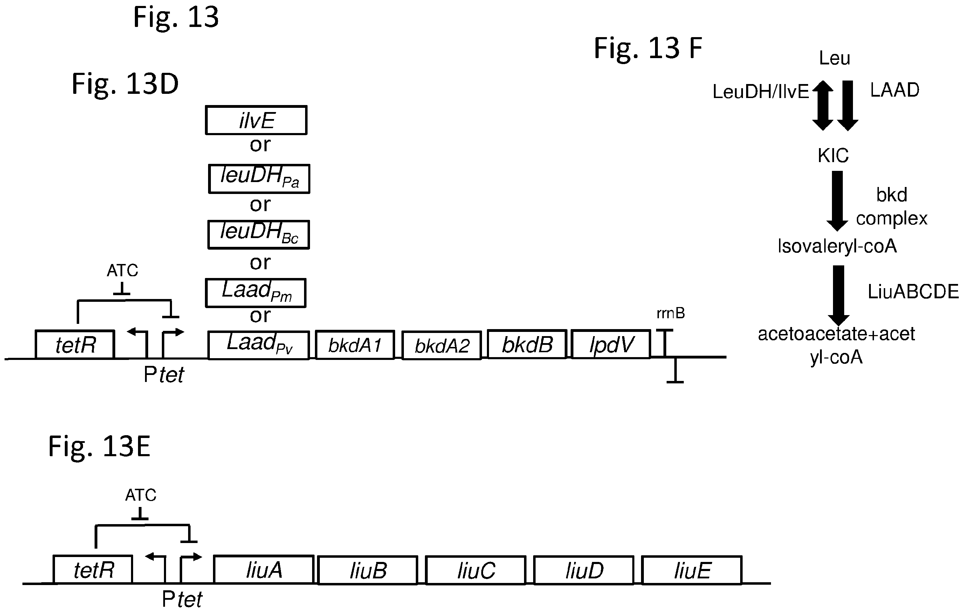

**Fig. 13** depicts exemplary components of branched chain amino acid synthetic biotics. **Fig. 13A and Fig. 13A** depicts 2 exemplary components of a branched chain amino acid synthetic biotic disclosed herein for leucine catabolism to isopentanol or isovalerate (**Fig. 13A**) or alpha-ketoisocaproic acid (**Fig.13B**), wherein the second step is catalyzed by Ketoacid decarboxylase (KivD). **Fig. 13C** depicts a schematic of the corresponding metabolic pathway for Fig. A and Fig. B. In some embodiments, both circuits can be expressed in the same strain. Alternatively, the circuits can each be expressed individually. Genes shown in Fig13A and B are amino transferase (ilvE), leuDH (derived from *P*. *aeruginosa* PA01 or *Bacillus cereus*) and/or LAAD (derived from *Proteus mirabilis* or *Proteus vulgaris*) for conversion of BCAA to the α-keto acid; the branched chain α-ketoacid decarboxylase (KivD) for conversion from the α-keto acid to the corresponding aldehyde; and alcohol dehydrogenase 2 (Adh2; yqhD) for conversion to the corresponding alcohol or aldehyde dehydrogenase (padA) for conversion to the corresponding carboxylic acid. The genes for the leucine exporter (LeuE) and keto acid reductoisomerase (ilvC) can be deleted. **Fig. 13D** and **Fig. 13E** depict 2 exemplary components of a branched chain amino acid synthetic biotic disclosed herein for leucine catabolism to isovalerylCoA (**Fig. 13E**) or alpha-ketoisocaproic acid (**Fig.13E** and **Fig.13F**), wherein the second step is catalyzed by Bkd complex from Pseudomonas aeruginosa. **Fig. 13F** depicts a schematic of the corresponding metabolic pathway for **Fig. D** and **Fig. E.** In some embodiments, both circuits can be expressed in the same strain. Alternatively, the circuit shown in **Fig. 13D** can each be expressed individually, without the circuit of **Fig. 13E****.** The circuit in **Fig. 13E** (the Liu operon) requires the circuit of **Fig. 13D** to to generate its substrate, isovalerylCoA, and therefore is used together with the the cicuit of **Fig. 13E****.** Genes shown in Fig13D and Fig13E are amino transferase (ilvE), leuDH (derived from *P*. *aeruginosa* PA01 or *Bacillus cereus*) and/or LAAD (derived from *Proteus mirabilis* or *Proteus vulgaris*) for conversion of BCAA to the α-keto acid; the Bkd complex (comprising bkdA1, bkdA2, bkdB, and lpdV) for conversion from the α-keto acid to the corresponding CoA thioester, and the Liu operon (comprising liuA, liuB, liuC, liuD, and liuE) for conversion of isovaleryl-CoA to acetoacetate and acetylCoA.
   One or more of the genes encoding a catabolic enzyme, transporter, and/or other genes may be under the control of an inducible promoter that is induced under exogenous environmental conditions, such as any of the inducible promoters provided herein, e.g., a promoter induced under low oxygen or anaerobic conditions. In certain embodiments, the constructs are expressed on a high copy plasmid. In certain embodiments, any of the genes may be under the control of a tetR promoter. For example, the construct may comprise a (constitutive or inducible) promoter driving expression of the Tet repressor (TetR) from the tetR gene, which is linked to a second promoter comprising a TetR binding site that drives expression of any of the BCAA catabolic cassettes described above. TetR is (either constitutively or inducibly) expressed and inhibits the expression of the BCAA catabolic cassette(s). Upon addition of anhydrotetracylcine (ATC), TetR binds to ATC and binds removing the inhibition by TetR allowing expression of the BCAA catabolic cassettes.

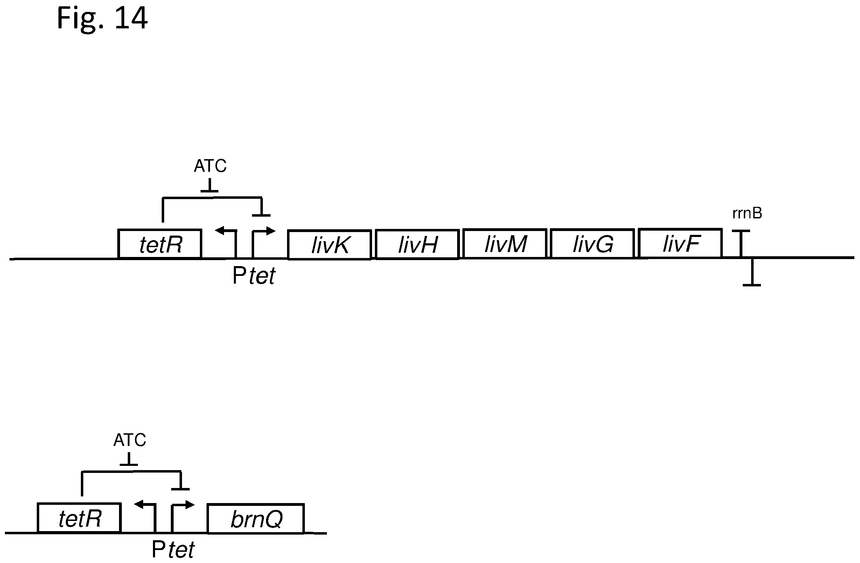

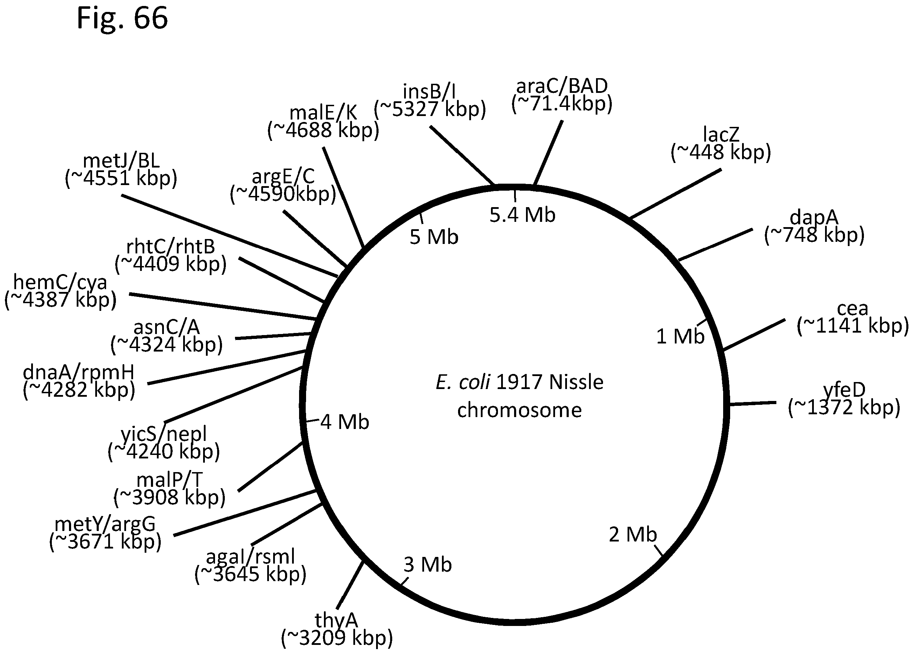

**Fig. 14** depicts exemplary components of a branched chain amino acid synthetic biotic disclosed herein for leucine import. Genes shown are high affinity leucine transporter complex (LivKHMGF) and low affinity BCAA transporter (brnQ). One or more of the genes encoding a transporter may be under the control of an inducible promoter that is induced under exogenous environmental conditions, such as any of the inducible promoters provided herein, e.g., a promoter induced under low oxygen or anaerobic conditions. In certain embodiments, any of the genes may be under the control of a tetR promoter. In some embodiments, both circuits can be expressed in the same strain. Alternatively the circuits can each be expressed individually. In some embodiments, the high affinity leucine transporter complex (LivKHMGF) and/or the low affinity BCAA transporter (brnQ) is integrated into the chromosome. Exemplary chromosomal insertion sites are shown in **Fig. 66****,** e.g., lacZ. In some embodiments, the high affinity leucine transporter complex (LivKHMGF) and/or the low affinity BCAA transporter (brnQ) is located on a plasmid, e.g., a low copy plasmid.

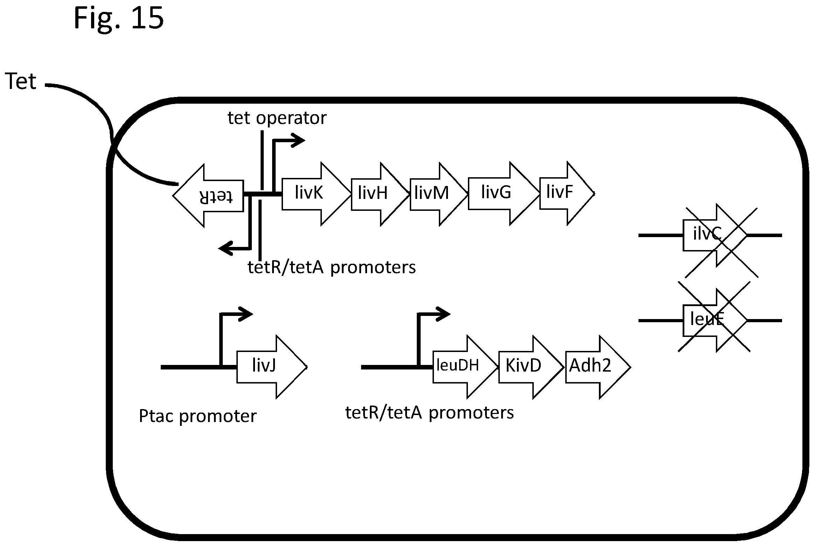

**Fig. 15** depicts one exemplary branched chain amino acid circuit.Genes shown are high affinity leucine transporter complex (LivKHMGF), leuDH (e.g., derived from P. *aeruginosa* PA01 or *Bacillus cereus*), the branched chain α-ketoacid decarboxylase (KivD), e.g., from *Lactococcus lactis,* and alcohol dehydrogenase 2 (Adh2), e.g., from *Saccharomyces cerevisiae.* The genes for the leucine exporter (LeuE) and keto acid reductoisomerase (IlvC) have been deleted. The gene for LivJ is added which can be under the control of the native promoter or the constitutive promoter Ptac (a hybrid synthetic promoter derived from trp and lac). In certain embodiments, any of the genes may be under the control of a tetR/tetA promoter. For example, the construct may comprise a (constitutive or inducible) promoter driving expression of the Tet repressor (TetR) from the tetR gene, which is linked to a second promoter comprising a TetR binding site that drives expression of any of the leucine catabolic cassettes described above. TetR is (either constitutively or inducibly) expressed and inhibits the expression of the leucine catabolic cassette(s). Upon addition of anhydrotetracylcine (ATC), TetR binds to ATC and binds removing the inhibition by TetR allowing expression of the leucine catabolic cassettes.

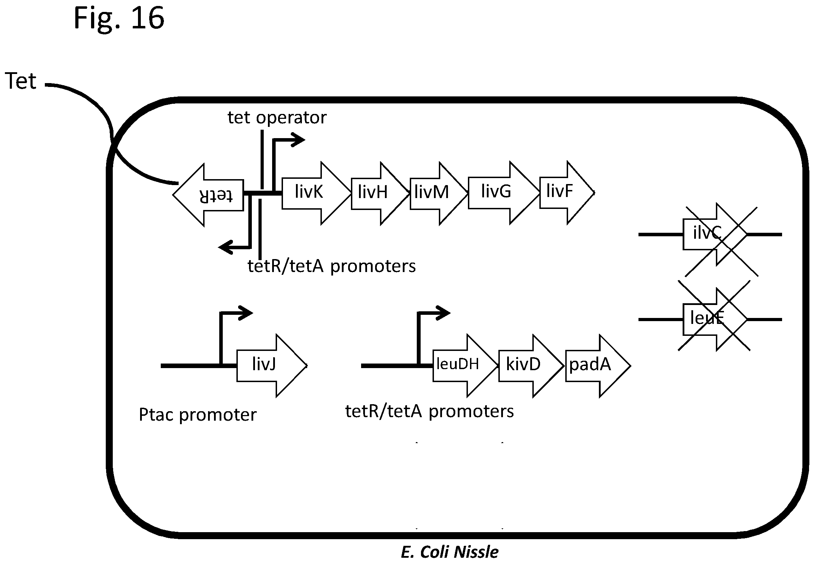

**Fig. 16** depicts one exemplary branched chain amino acid circuit. Genes shown are high affinity leucine transporter complex (LivKHMGF), leuDH (e.g., derived from Pseudomonas aeruginosa PA01 or Bacillus cereus), the branched chain α-ketoacid decarboxylase (KivD) e.g., from *Lactococcus lactis,* aldehyde dehydrogenase (PadA) e.g., from *E. Coli* K12. The genes for the leucine exporter (LeuE) and IlvC have been deleted. In certain embodiments, any of the genes may be under the control of a tetR/tetA promoter. For example, the construct may comprise a (constitutive or inducible) promoter driving expression of the Tet repressor (TetR) from the tetR gene, which is linked to a second promoter comprising a TetR binding site that drives expression of any of the leucine catabolic cassettes described above. TetR is (either constitutively or inducibly) expressed and inhibits the expression of the leucine catabolic cassette(s). Upon addition of anhydrotetracylcine (ATC), TetR binds to ATC and binds removing the inhibition by TetR allowing expression of the leucine catabolic cassettes.

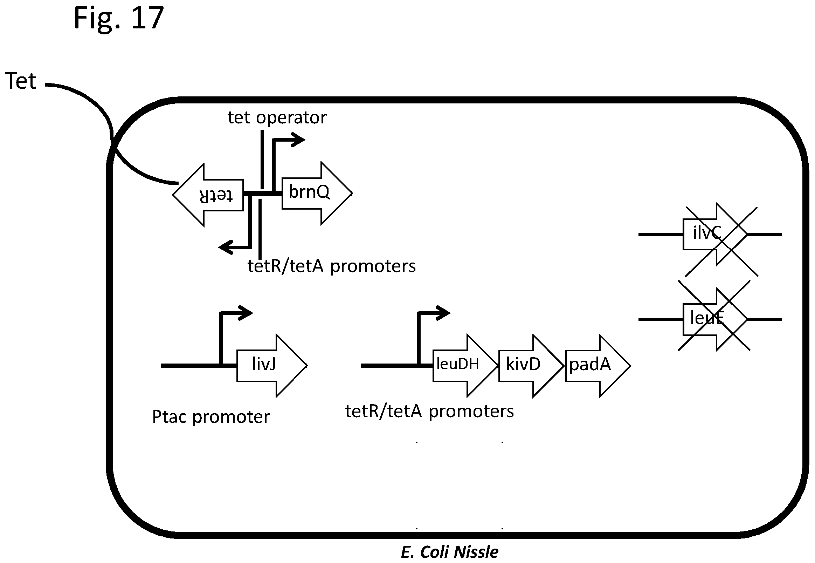

**Fig. 17** depicts one exemplary branched chain amino acid circuit. Genes shown are low affinity BCAA transporter (BrnQ), leuDH (e.g., derived from Pseudomonas aeruginosa PA01 or Bacillus cereus), the branched chain α-ketoacid decarboxylase (KivD), e.g., from *Lactococcus lactis,* aldehyde dehydrogenase (PadA), e.g., from *E. Coli* K-12. The genes for the leucine exporter (LeuE) and IlvC have been deleted. The gene for ilvE is added. In certain embodiments, any of the genes may be under the control of a tetR/tetA promoter. For example, the construct may comprise a (constitutive or inducible) promoter driving expression of the Tet repressor (TetR) from the tetR gene, which is linked to a second promoter comprising a TetR binding site that drives expression of any of the leucine catabolic cassettes described above. TetR is (either constitutively or inducibly) expressed and inhibits the expression of the leucine catabolic cassette(s). Upon addition of anhydrotetracylcine (ATC), TetR binds to ATC and binds removing the inhibition by TetR allowing expression of the leucine catabolic cassettes.

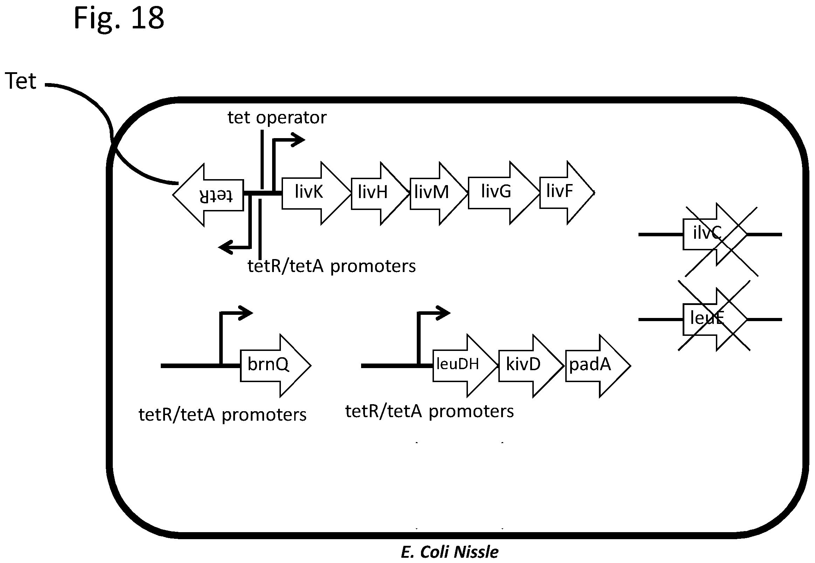

**Fig. 18** depicts one exemplary branched chain amino acid circuit. Genes shown are high affinity leucine transporter complex (LivKHMGF), leuDH, e.g., derived from Pseudomonas aeruginosa PA01 or Bacillus cereus, the branched chain α-ketoacid decarboxylase (KivD), e.g., from *Lactococcus lactis,* and aldehyde dehydrogenase (PadA), e.g., from *E. Coli* K-12. The genes for the leucine exporter (LeuE) and IlvC have been deleted. The gene for BrnQ is added. In certain embodiments, any of the genes may be under the control of a tetR/tetA promoter.. In some embodiments, the high affinity leucine transporter complex (LivKHMGF) and/or the low affinity BCAA transporter (brnQ) is integrated into the chromosome. Exemplary chromosomal insertion sites are shown in **Fig. 66****,** e.g., lacZ. In some embodiments, the transporters are expressed from a plasmid.

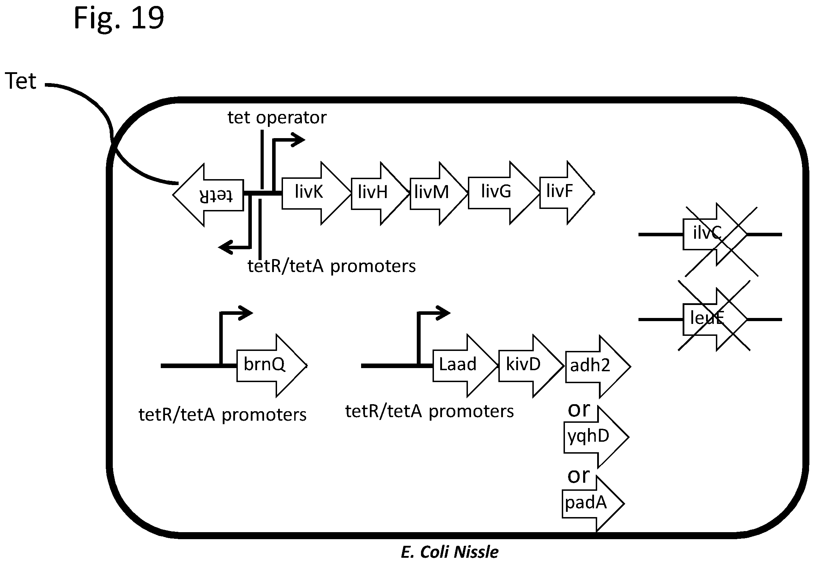

**Fig. 19** depicts one exemplary branched chain amino acid circuit. Genes shown are high affinity leucine transporter complex (LivKHMGF), L-AAD, e.g., derived from Proteus vulgaris or Proteus mirabilis, the branched chain α-ketoacid decarboxylase (KivD), e.g., from *Lactococcus lactis,* and either aldehyde dehydrogenase (PadA), e.g., from *E. Coli* K-12, alcohol dehydrogenase YqhD, e.g., from *E. Coli,* or alcohol dehydrogenase Adh2, e.g., from S. *cerevisiae.* The genes for the leucine exporter (LeuE) and IlvC have been deleted. The gene for BrnQ is added. In certain embodiments, any of the genes may be under the control of a tetR/tetA promoter.

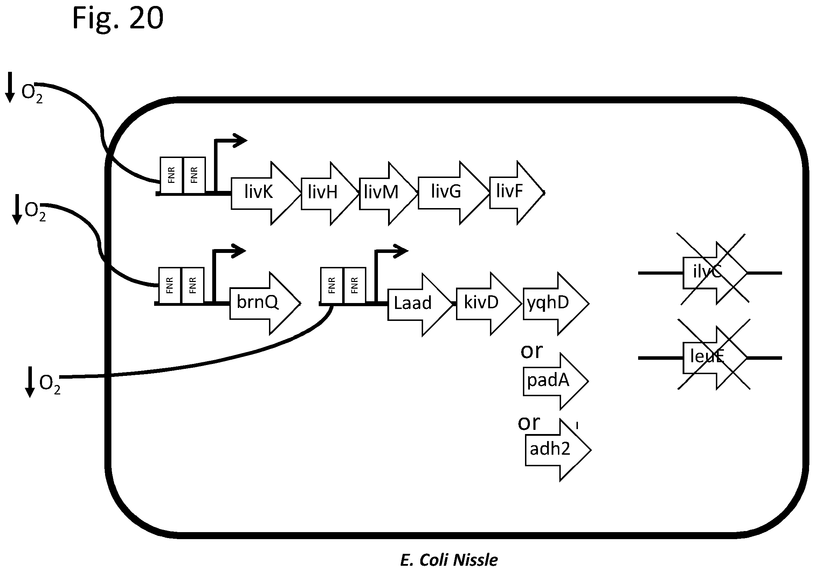

**Fig. 20** depicts one exemplary branched chain amino acid circuit. Genes shown are high affinity leucine transporter complex (LivKHMGF), L-AAD, e.g., derived from Proteus vulgaris or Proteus mirabilis, the branched chain α-ketoacid decarboxylase (KivD), e.g., from *Lactococcus lactis,* and either aldehyde dehydrogenase (PadA), e.g., from *E. Coli* K-12, alcohol dehydrogenase YqhD, e.g., from *E.Coli,* or alcohol dehydrogenase Adh2 from S. *cerevisiae.* The genes for the leucine exporter (LeuE) and IlvC have been deleted. The gene for BrnQ is added. In some embodiments, any of the genes may be under the control of a promoter inducible under low oxygen or anaerobic conditions, e.g., an FNR promoter.

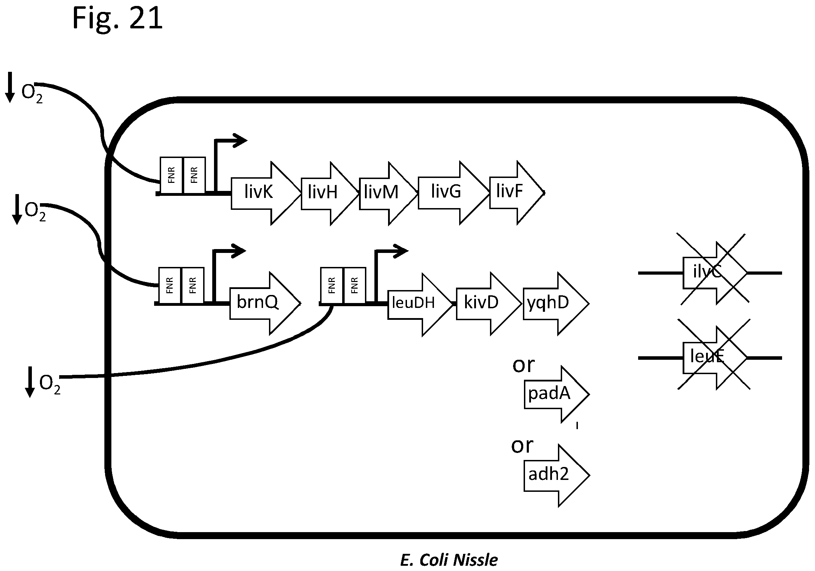

**Fig. 21** depicts one exemplary branched chain amino acid circuit. Genes shown are high affinity leucine transporter complex (LivKHMGF), LeuDh, e.g., derived from Pseudomonas aeruginosa PA01 or Bacillus cereus, the branched chain α-ketoacid decarboxylase (KivD), e.g., from *Lactococcus lactis,* and either aldehyde dehydrogenase (PadA), e.g., from *E. Coli* K-12, alcohol dehydrogenase YqhD from *E.Coli,* or alcohol dehydrogenase Adh2, e.g., from S. *cerevisiae.* The genes for the leucine exporter (LeuE) and IlvC have been deleted. The gene for BrnQ is added. In some embodiments, any of the genes may be under the control of a promoter inducible under low oxygen or anaerobic conditions, e.g., an FNR promoter.

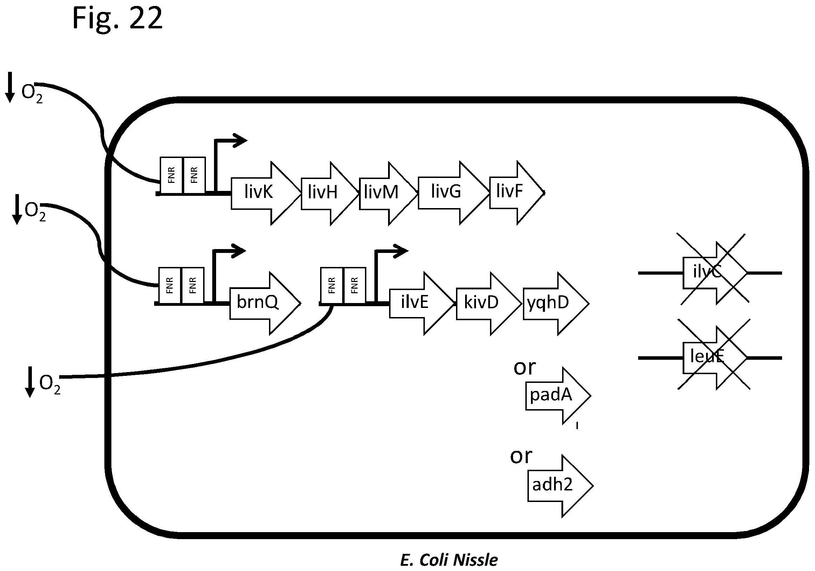

**Fig. 22** depicts one exemplary branched chain amino acid circuit. Genes shown are high affinity leucine transporter complex (LivKHMGF), low affinity BCAA transporter (brnQ), a leucine dehydrogenase leuDH (from Pseudomonas aeruginosa or Bacillus cereus), Bkd complex (comprising bkdA1, bkdA2, bkdB, and lpdV) for conversion from the α-keto acid to the corresponding CoA thioester. The genes for the leucine exporter (LeuE) and IlvC have been deleted. The gene for BrnQ is added. In some embodiments, any of the genes may be under the control of a promoter inducible under low oxygen or anaerobic conditions, e.g., an FNR promoter.

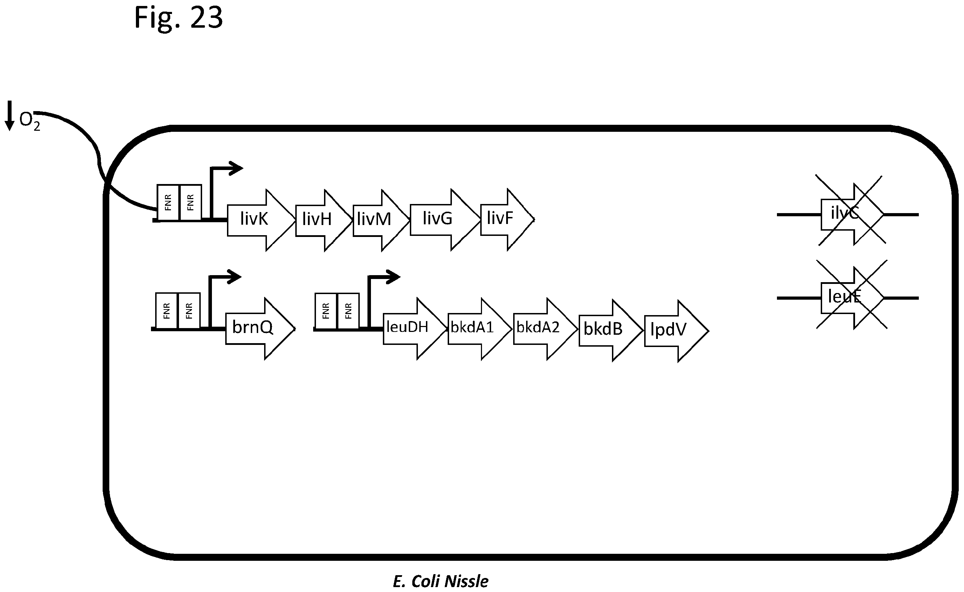

**Fig. 23** depicts one exemplary branched chain amino acid circuit. Genes shown are high affinity leucine transporter complex (LivKHMGF), low affinity BCAA transporter (brnQ), a leucine dehydrogenase leuDH (from Pseudomonas aeruginosa or Bacillus cereus), the Bkd complex (comprising bkdA1, bkdA2, bkdB, and lpdV) for conversion from the α-keto acid to the corresponding CoA thioester, and Liu operon (comprising liuA, liuB, liuC, liuD, and liuE) for conversion of isovaleryl-CoA to acetoacetate and acetylCoA. The genes for the leucine exporter (LeuE) and IlvC have been deleted. The gene for BrnQ is added. In some embodiments, any of the genes may be under the control of a promoter inducible under low oxygen or anaerobic conditions, e.g., an FNR promoter.
**Fig. 23** depicts one exemplary branched chain amino acid circuit.

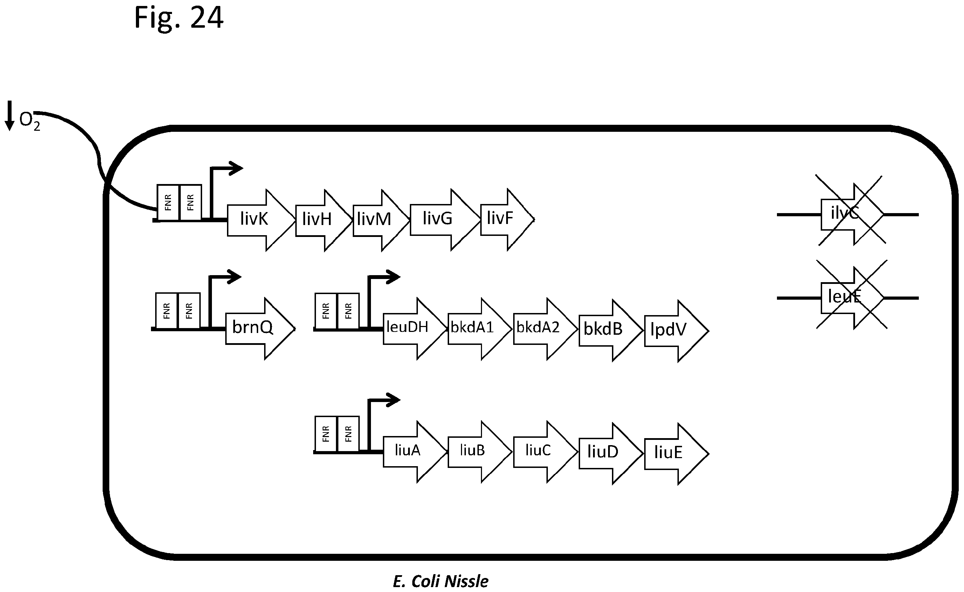

**Fig. 24** depicts one exemplary branched chain amino acid circuit.

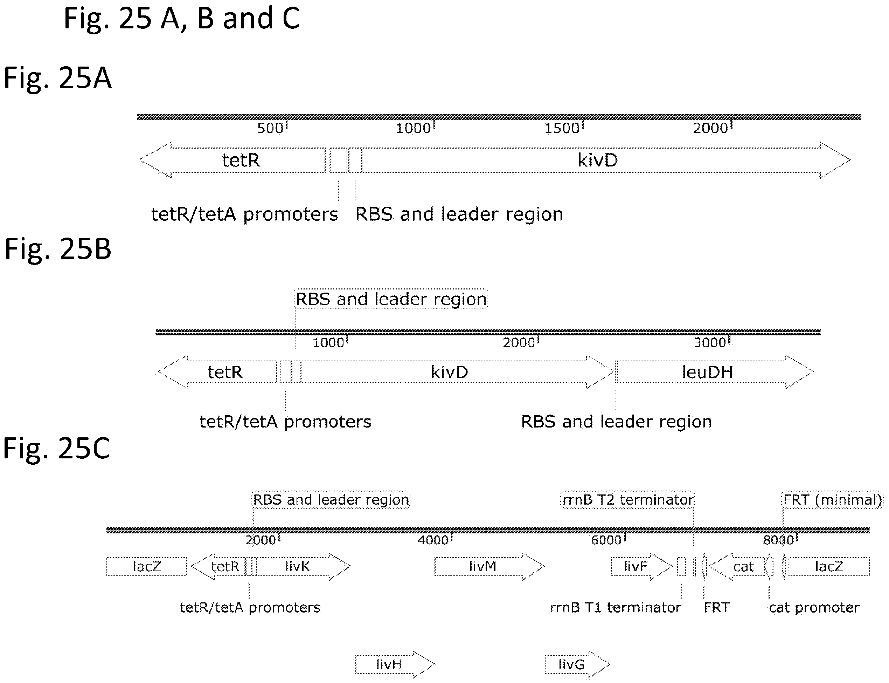

**Fig. 25** depicts exemplary constructs of circuit components for *LeuDH, kivD* and *livKHMGF* inducible expression in *E. coli.* **Fig. 25A** depicts *kivD* under the control of the Tet promoter, e.g., cloned in a high-copy plasmid. **Fig. 25B** depicts *kivD* and *LeuDH* under the control of the Tet promoter, e.g., cloned into a high-copy plasmid. **Fig 25C** depicts *livKHMGF* operon under the control of the Tet promoter, flanked by the *lacZ* homologous region for chromosomal integration by lamba-red recombination.

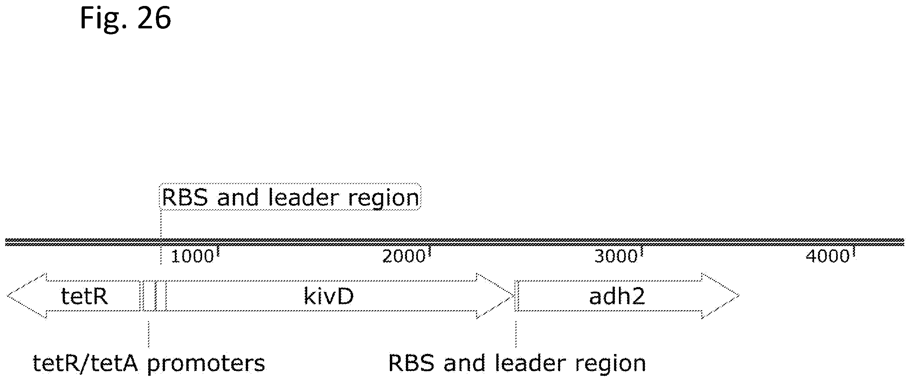

**Fig. 26** depicts the gene organization of a Tet-kivD-adh2 construct.

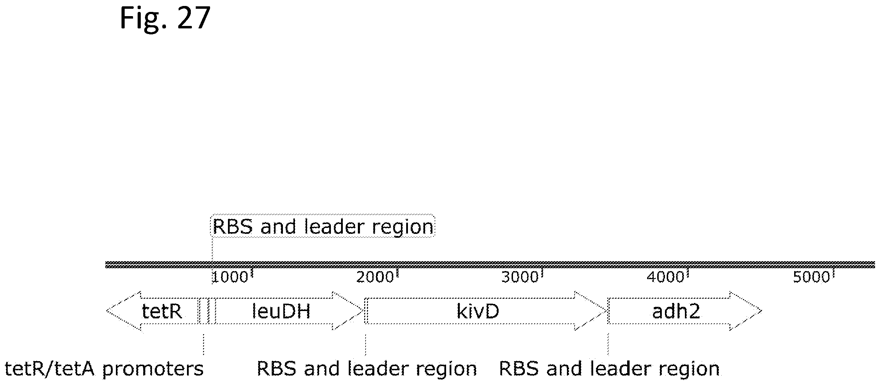

**Fig. 27** depicts the gene organization of a Tet-LeuDH-kivD-adh2 construct.

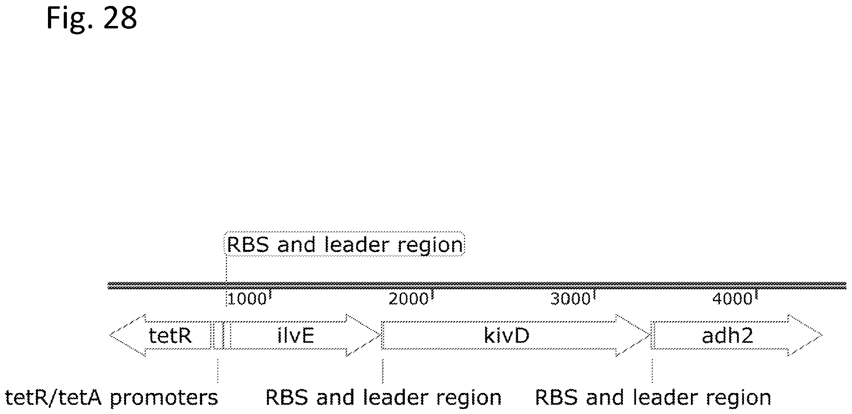

**Fig. 28** depicts the gene organization of aTet-ilvE-kivD-adh2 construct.

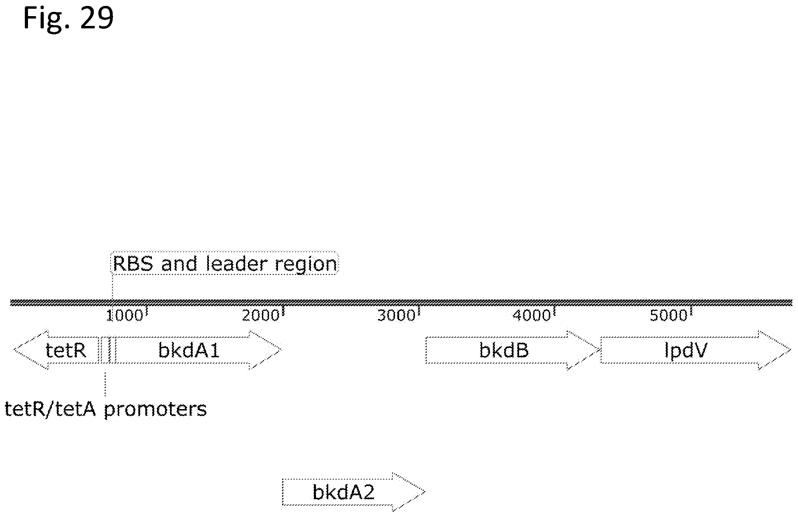

**Fig. 29** depicts the gene organization of the Tet-bkd operon construct.

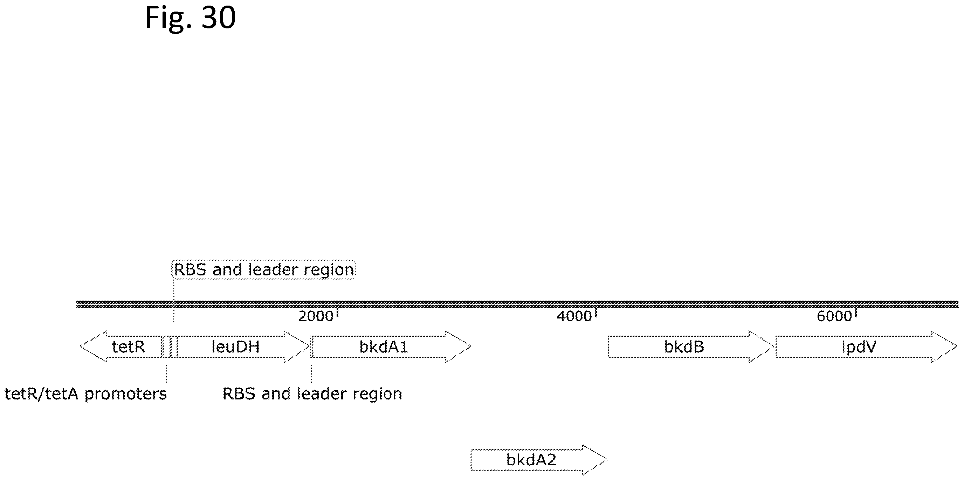

**Fig. 30** depicts the gene organization of the Tet-Leudh-bkd operon construct.

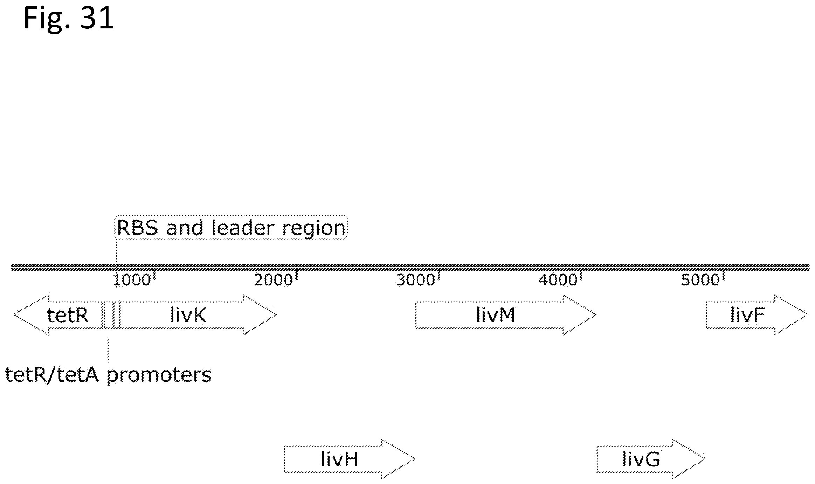

**Fig. 31** depicts the gene organization of the Tet-livKHMGF construct.

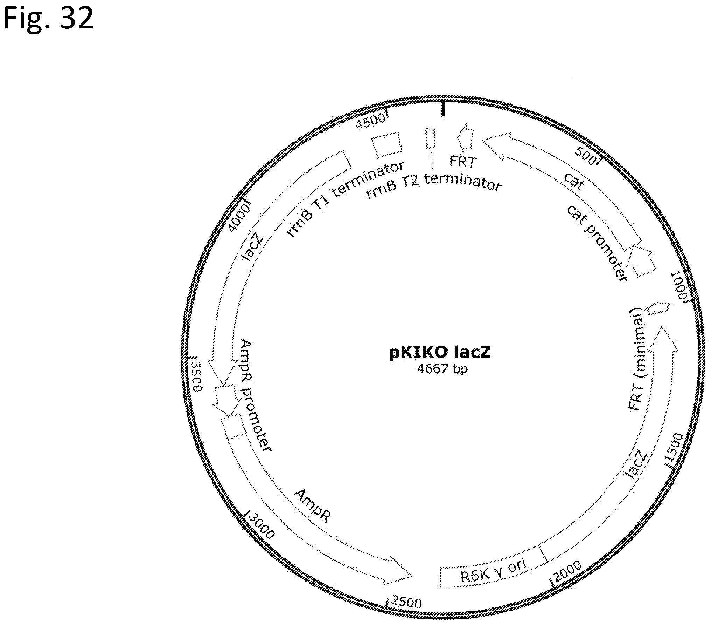

**Fig. 32** depicts the gene organization of the pKIKO-lacZ plasmid used to clone the Tet-livKHMGF construct.

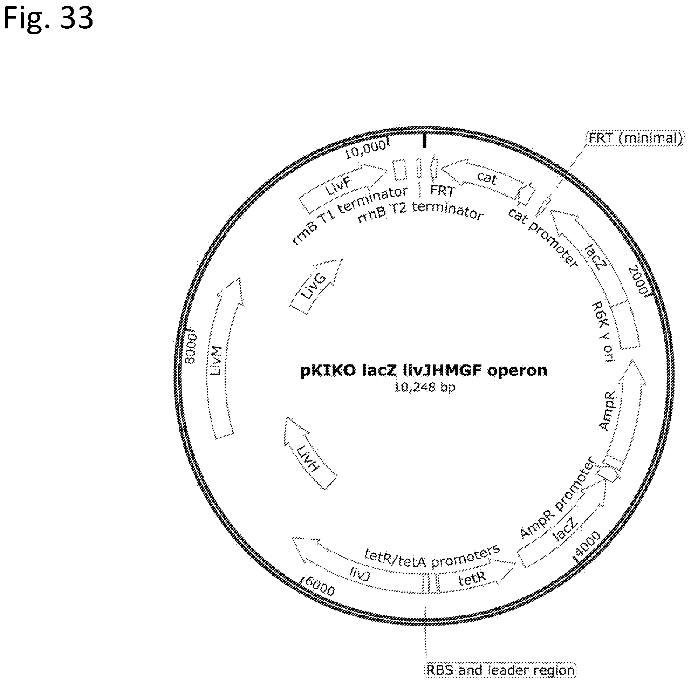

**Fig. 33** depicts the gene organization of the pTet-livKHMGF plasmid used to generate the PCR fragment used to integrate the Tet-livKHMGF into *E*. *coli* Nissle lacZ locus.

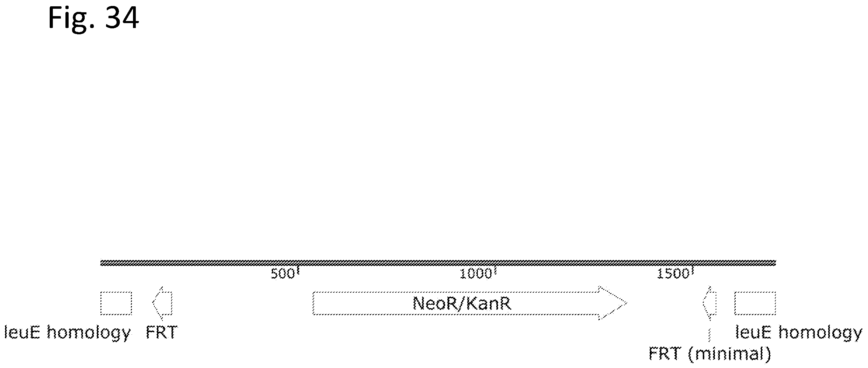

**Fig. 34** depicts the gene organization of the DNA fragment used to generate the *E*. *coli* Nissle ΔleuE deletion strain.

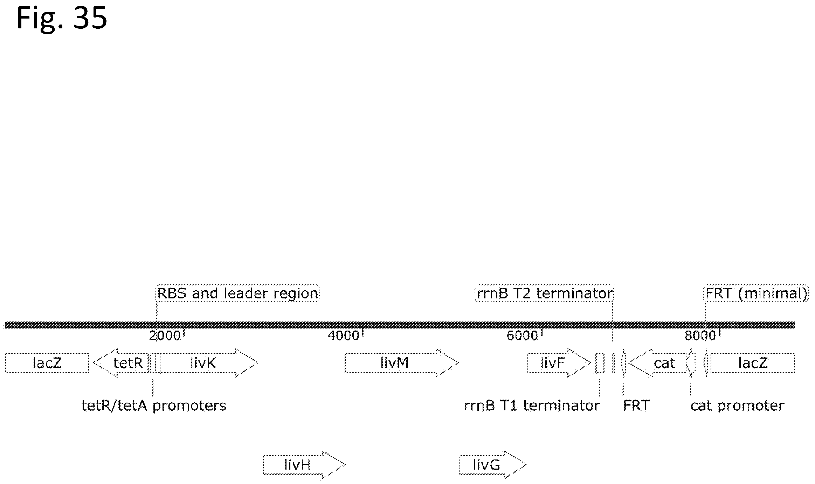

**Fig. 35** depicts the gene organization of the DNA fragment used to integrate the Tet-livKHMGF into the *E. coli* Nissle lacZ locus.

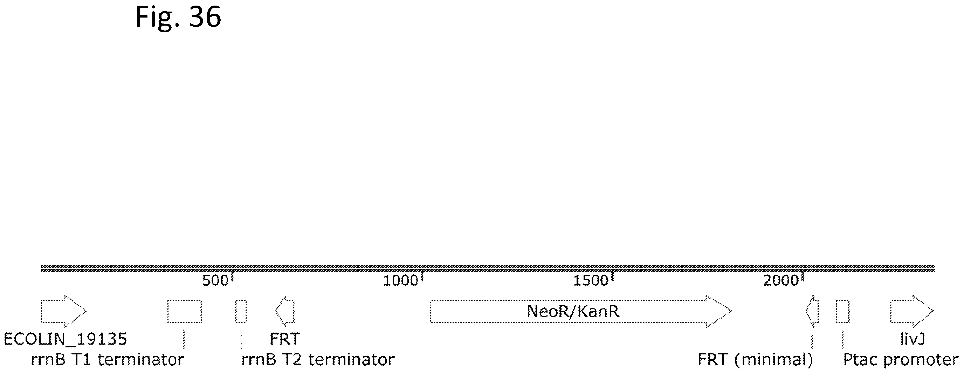

**Fig. 36** depicts the organization of the DNA fragment used to exchange the endogenous livJ promoter with the constitutive promoter Ptac.

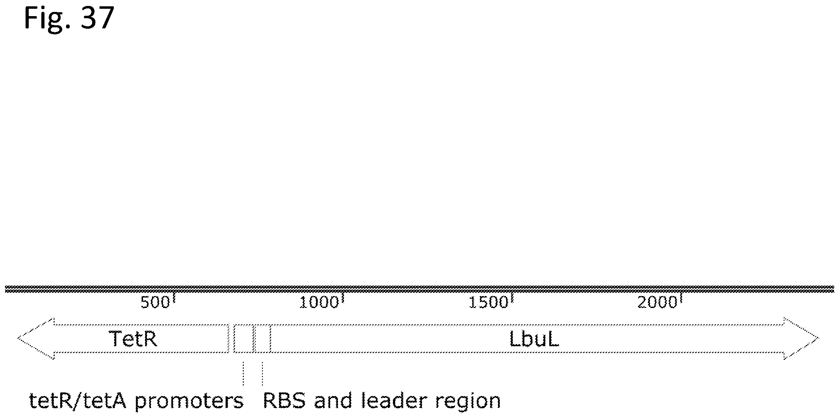

**Fig. 37** depicts the gene organization of a LBUL construct.

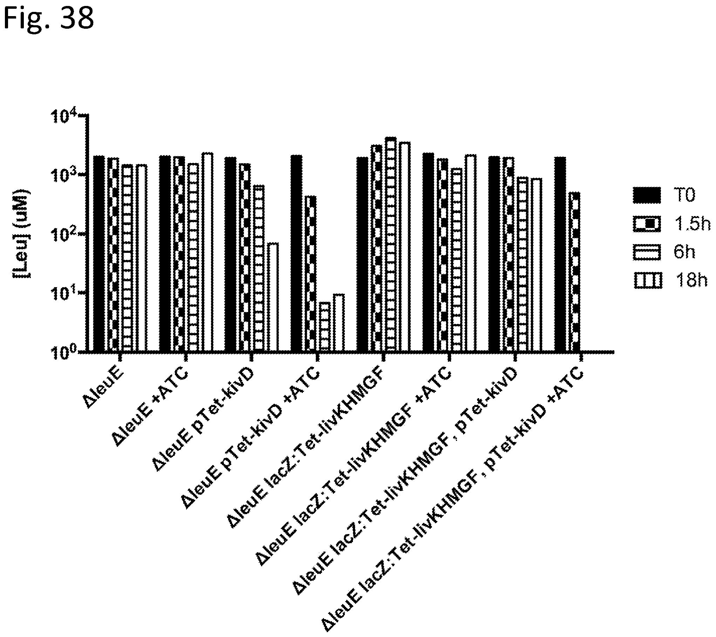

**Fig. 38** depicts leucine levels in the Nissle ΔleuE deletion strain harboring a high-copy plasmid expressing *kivD* from the Tet promoter or further with a copy of the *livKHMGF* operon driven by the Tet promoter integrated into the chromosome at the *lacZ* locus, which were induced with ATC and incubated in culture medium supplemented with 2 mM leucine. Samples were removed at 0, 1.5, 6 and 18 h, and leucine concentration was determined by liquid chromatography tandem mass spectrometry.

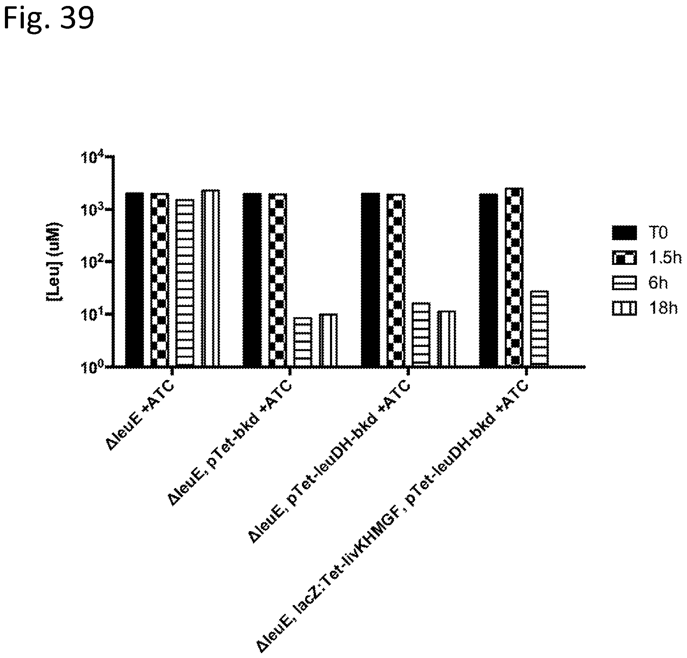

**Fig. 39** depicts leucine degradation in the Nissle ΔleuE deletion strain harboring a high-copy plasmid expressing the branch-chain keto-acid dehydrogenase (bkd) complex (comprising bkdA1, bkdA2, bkdB, and lpdV) with or without expression of a leucine dehydrogenase (LeuDH) from the Tet promoter or further with a copy of the leucine importer livKHMGF driven by the Tet promoter integrated into the chromosome at the lacZ locus, which were induced with ATC and incubated in culture medium supplemented with 2mM leucine. Samples were removed at 0, 1.5, 6 and 18h, and leucine concentration was determined by liquid chromatography tandem mass spectrometry.

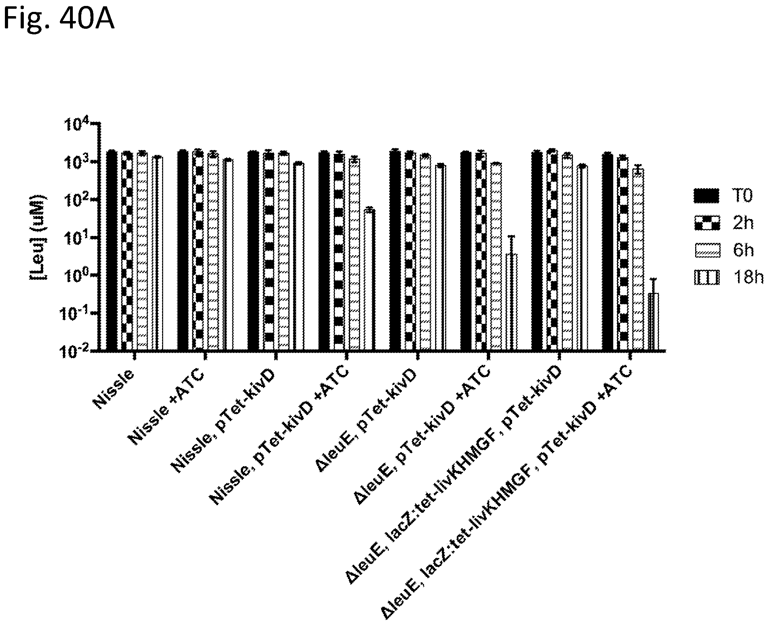

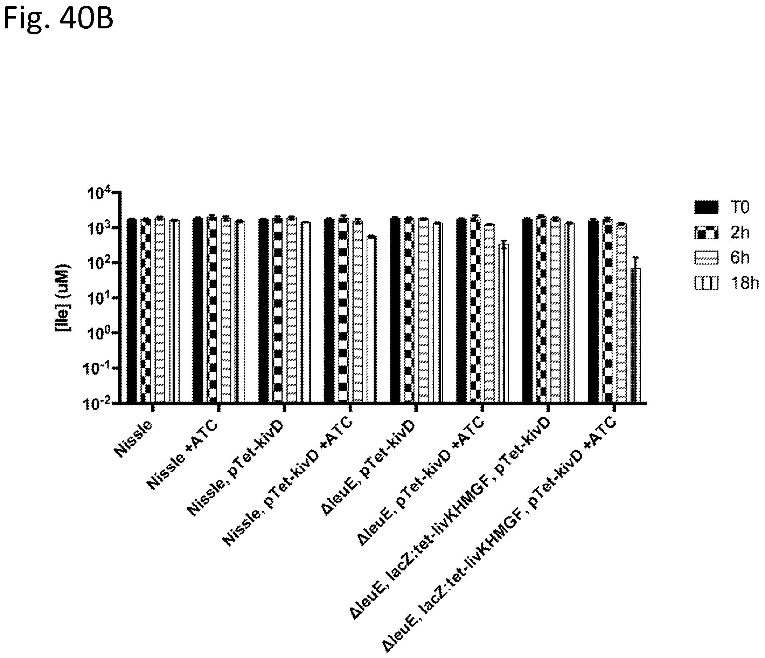

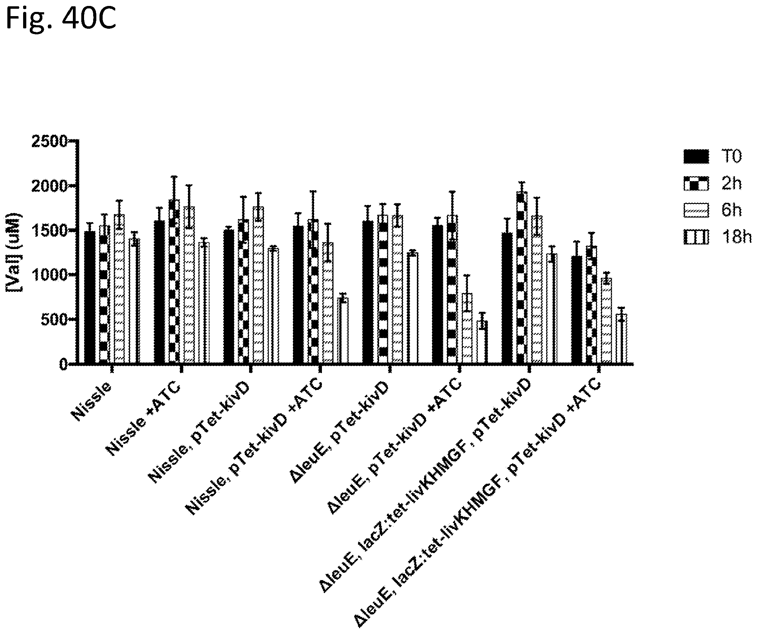

**Figs. 40A****,** **40B****, and** **40C** depict the simultaneous degradation of leucine (**Fig. 40A**), isoleucine **(****Fig. 40B****),** and valine **(****Fig. 40C****)** by *E. coli* Nissle and its ΔleuE deletion strain harboring a high-copy plasmid expressing the keto-acid decarboxylase kivD from the Tet promoter or further with a copy of the livKHMGF operon driven by the Tet promoter integrated into the chromosome at the lacZ locus, which were induced with ATC and incubated in culture medium supplemented with 2mM leucine, 2mM isoleucine and 2mM valine. Samples were removed at 0, 1.5, 6 and 18h, and BCAA concentration was determined by liquid chromatography tandem mass spectrometry. The strains were grown overnight at 37°C in LB media, and the overnight culture was used to inoculate a new batch at a 1/100 dilution in LB, which was grown for three hours at 37°C. Induction was for two hours with 100 ng/mL ATC. The cells were then collected by centrifugation and resuspended in M9 + 0.5% glucose and 2mM each of leucine, isoleucine, and valine. Samples were removed at 0, 1.5, 6 and 18h, and BCAA concentration was determined by liquid chromatography tandem mass spectrometry. The results demonstrate that isoleucine and valine were also consumed by leucine-degrading strains. Moreover, deletion of *leuE* and expression of *livKHMGF* improved the rate of BCAA degradation.

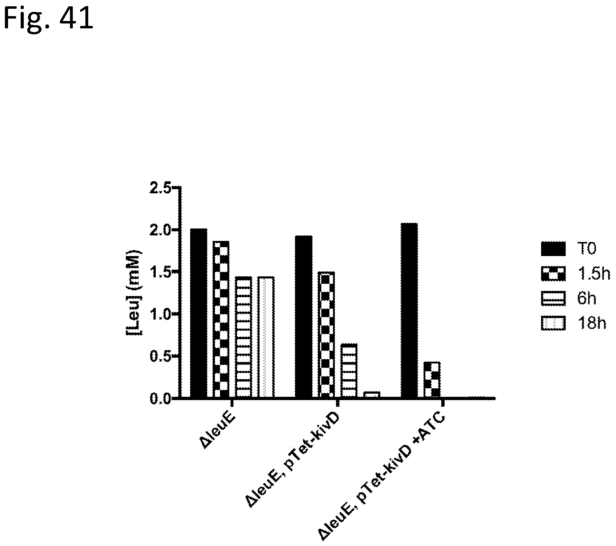

**Fig. 41** depicts a bar graph showing that the expression of *kivD* in *E. coli* Nissle leads to leucine degradation *in vitro.* The strains were grown overnight at 37°C in LB media, and the overnight culture was used to inoculate a new batch at a 1/100 dilution in LB. Induction was for two hours with 100 ng/mL ATC. The cells were then collected by centrifugation and resuspended in M9 + 0.5% glucose and 2mM leucine. Aliquots were removed at the indicated times for leucine determination by mass spectrometry. Inclusion of *kivD* resulted in increased bacterial cell consumption of leucine.

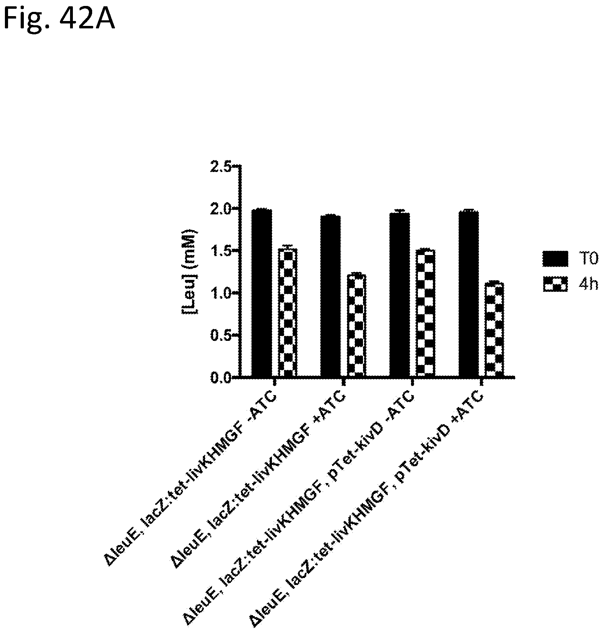

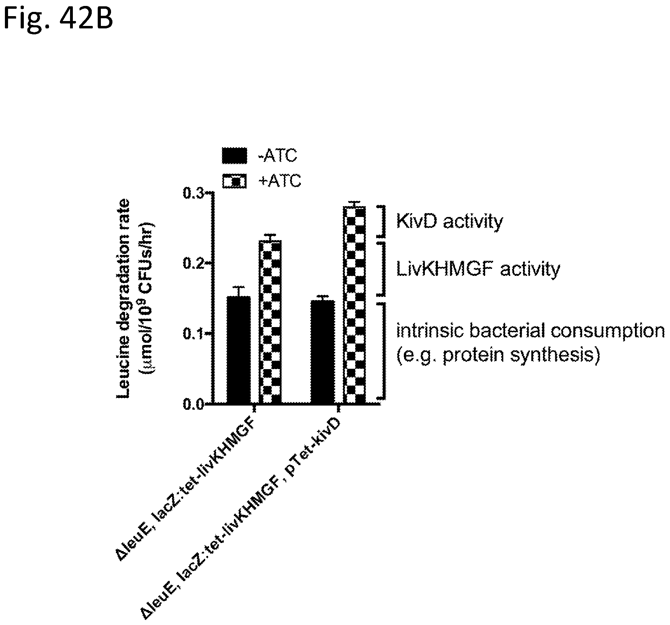

**Figs. 42A** **and** **42B** depict the determination of the leucine degradation rate, as mediated by KivD. The strains were grown overnight at 37°C in LB media, and the overnight culture was used to inoculate a new batch at a 1/100 dilution in LB, which was grown for two hours at 37°C. Induction was for one hour with 100 ng/mL ATC. The cells were then collected by centrifugation and resuspended in M9 + 0.5% glucose and 2mM leucine at OD₆₀₀ = 1. Samples were collected at 3 hours. The total degradation rate was about 250 nmol/10⁹ CFU/hour. The degradation rate attributable to KivD was about 50 nmol/10⁹ CFU/hour.

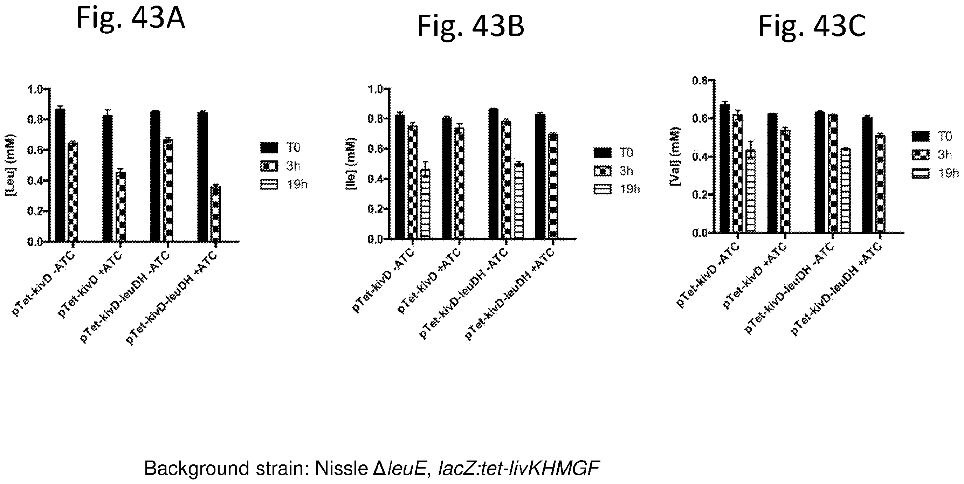

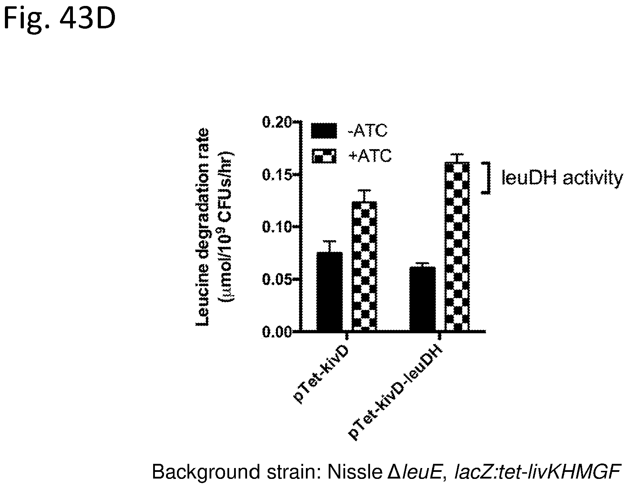

**Figs. 43A,43B, and 43C** depicts bargraphs which shows the efficient degradation of leucine (**Fig. 43A**), isoleucine (**Fig 43B**), and valine (**Fig. 43C**) by the engineered strains. **Fig. 43D** depicts a bargraph showing that expression of leucine dehydrogenase (LeuDH from *Pseudomonas aeruginosa*) improves the rate of leucine degradation to about 160 nmol/10⁹ CFU/hour. The background strain is Nissle Δ*leuE, lacZ:tet-livKHMGF.*

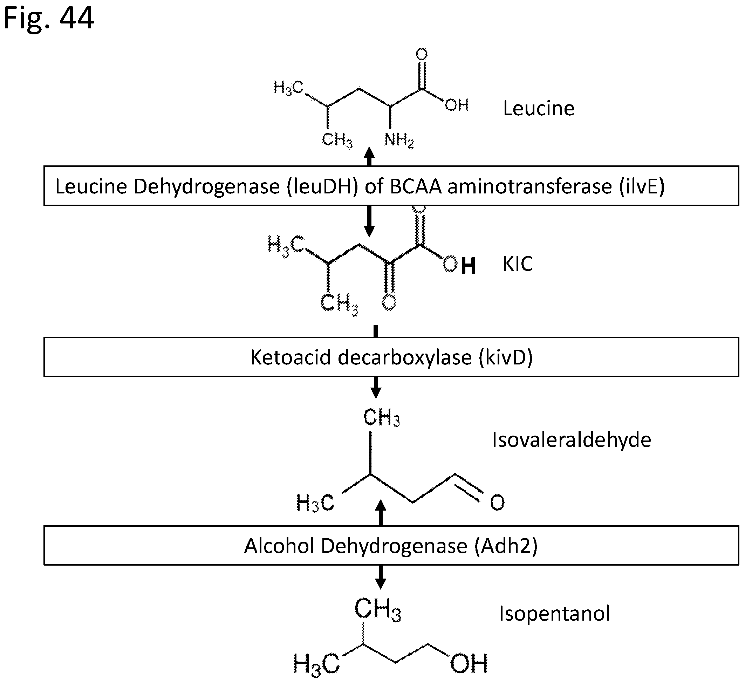

**Fig. 44** depicts the pathway of leucine degradation and KIC degradation engineered into the SYN469 strain.

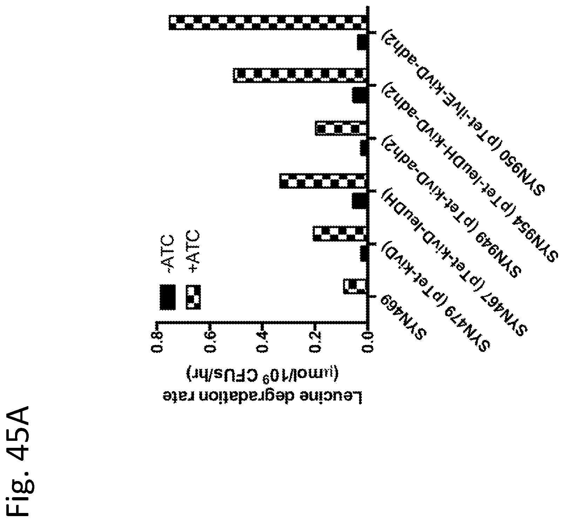

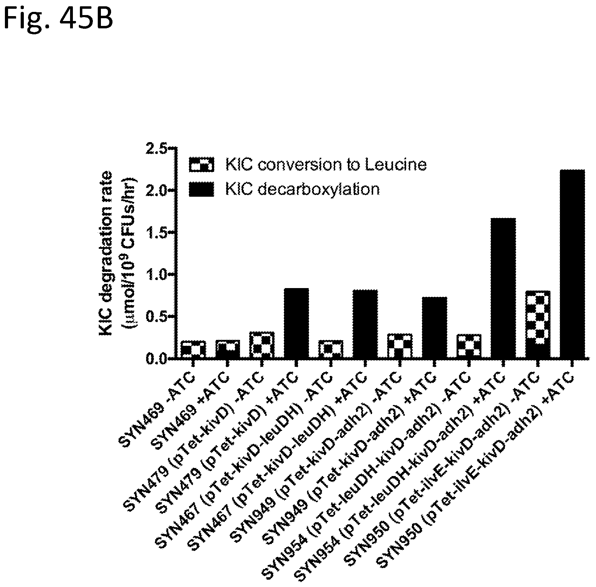

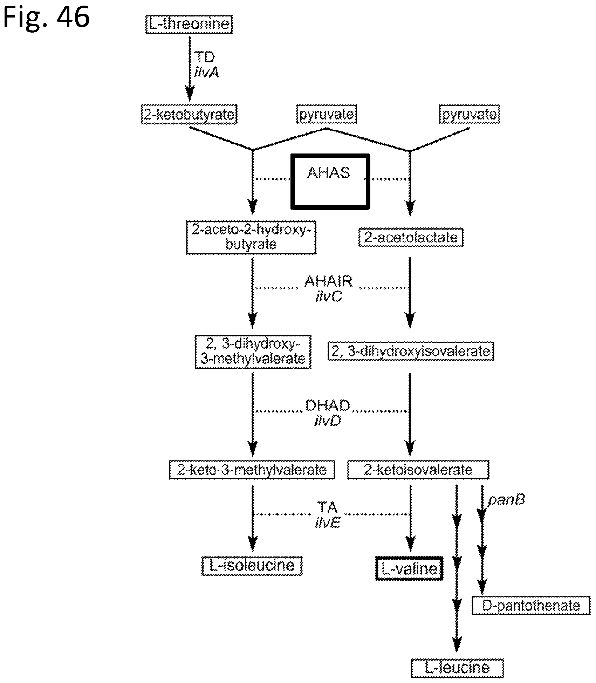

**Fig. 45A** **and** **45B** depicts the rate of leucine degradation or KIC degradation in several different engineered bacteria. The background strain used was SYN469 (Δ*leuE*Δ*ilvC, lacZ::tet-livKHMGF*), and the circuit was under the control of the Tet promoter on a high-copy plasmid. SYN479, SYN467, SYN949, SYN954, and SYN950 strains were fed leucine (**Fig. 45A**) or ketoisocaproate (KIC, also known as 4-methyl-2-oxopentanoate) (**Fig. 45B**), and products were monitored. A higher conversion of KIC than leucine to end-products demonstrates that leucine uptake and/or conversion to KIC is rate-limiting. **Fig. 46** depicts the use of valine sensitivity in *E*. *coli* as a genetic screening tool. There are three AHAS (acetohydroxybutanoate synthase) isozymes in *E*. *coli* (AHAS I: ilvBN, AHAS II: ilvGM, and AHAS III: ilvIH). Valine and leucine exert feedback inhibition on AHAS I and AHAS III; AHAS II is resistant to Val and Leu inhibition. *E. coli* K12 has a frameshift mutation in *ilvG* (AHAS II) and is unable to produce isoleucine and leucine in the presence of valine. Nissle has a functional *ilvG* and is insensitive to valine and leucine. A genetically engineered strain derived from E. coli K12, which more efficiently degrades leucine, has a greater reduction in sensitivity to leucine (through relieving the feedback inhibition on AHAS I and III). As a result, this pathway can be used as a tool to select snf identify a strain with improved resistance to leucine.

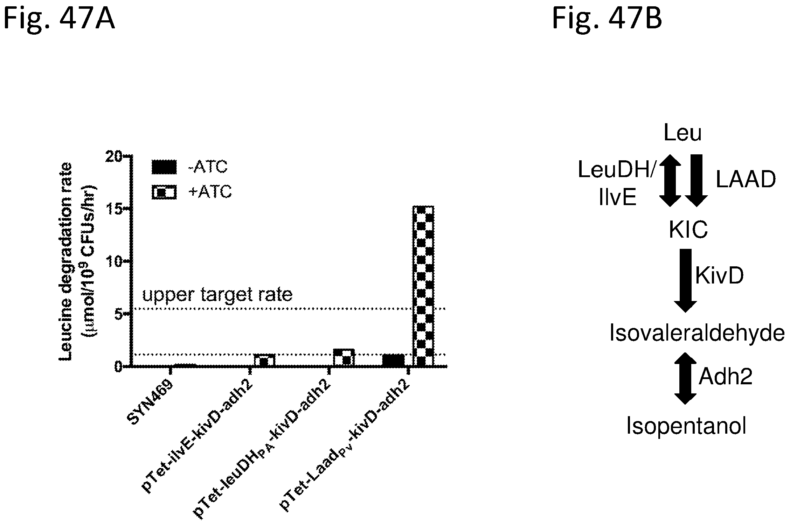

**Fig. 47A** depicts a bar graph showing the the leucine degration rates for various engineered bacterial strains. Bacterial strain Syn 469 is a leuE and ilvC knockout and comprises the leucine transporter under the control of tet promoter. Other tested engineered bacterial strains include: (1) strain having ilvE, kivD, and adh2; (2) strain having leuDh, kivD, and adh2; and (3) strain having L-AAD, kivD, and adh2. The strains are tet-inducible constructs on a high copy plasmid. The results show that L-amino acid deaminase (L-AAD) provides the best leucine degradation rate. **Fig.47B** depicts a schematic of the corresponding pathways.

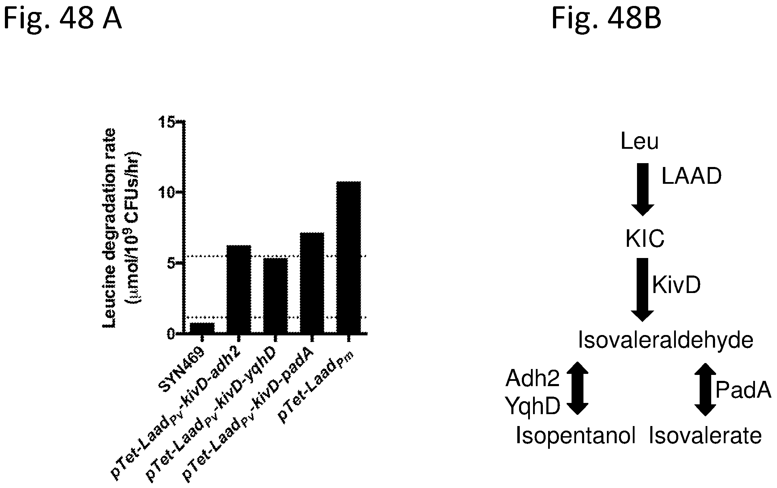

**Fig. 48A** shows the leucine degration rates for various engineered bacterial strains. Bacterial strain Syn 469 is a leuE and ilvC knockout and comprises the leucine transporter under the control of tet promoter. Other tested engineered bacterial strains include: (1) strain having L-AAD derived from P. vulgaris, kivD, and adh2; (2) strain having L-AAD derived from *P. vulgaris* (LAAD_{Pv}), kivD, and yqhD; (3) strain having L-AAD derived from *P. vulgaris,* kivD, and padA and (4) strain having L-AAD derived from P. *mirabilis* (LAAD_{Pm}). The results show that yqhD, adh2, and padAhave similar activities and that LAAD_{Pm} is a good alternative to LAAD_{Pv.} **Fig.48B** depicts a schematic of the corresponding pathways.

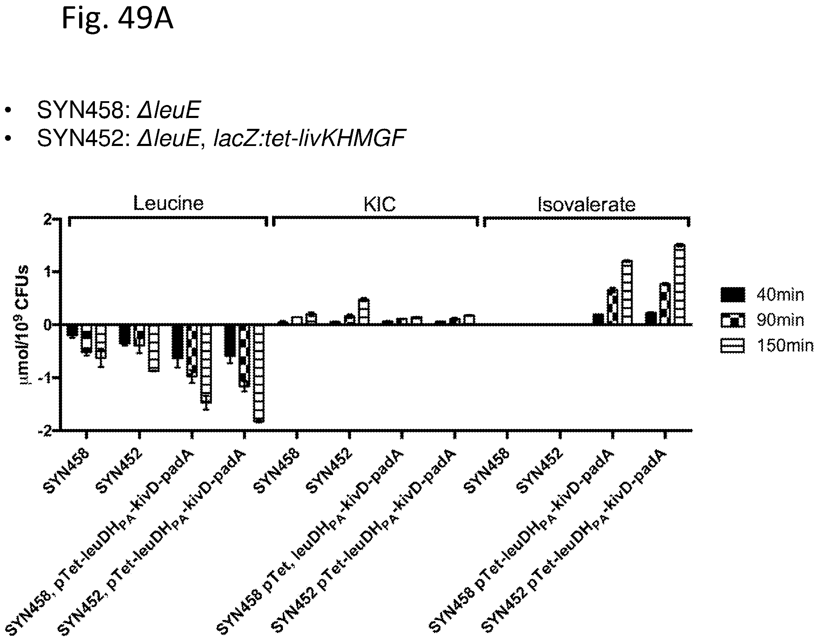

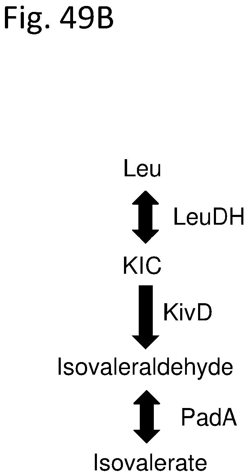

**Fig. 49A** shows the leucine degration rates for various engineered bacterial strains. Bacterial strain Syn 458 is a leuE knockout. Syn 452 is a leuE knockout and comprises the leucine transporter under the control of tet promoter. These background strains were tested with bacterial strains additionally having leuDH derived from *P. aeruginosa,* kivD, and padA. The results show that overexpression of the high affinity leucine transporter livKHMGF does not dramatically improved the rate of leucine degradation in a LeuE knockout strain having LeuDH, kivD, and padA with and without the leucine transporter livKHMGF under the control of tet promoter as measured by leucine degradation, KIC production, and isovalerate production. **Fig.49B** depicts a schematic of the corresponding pathways.

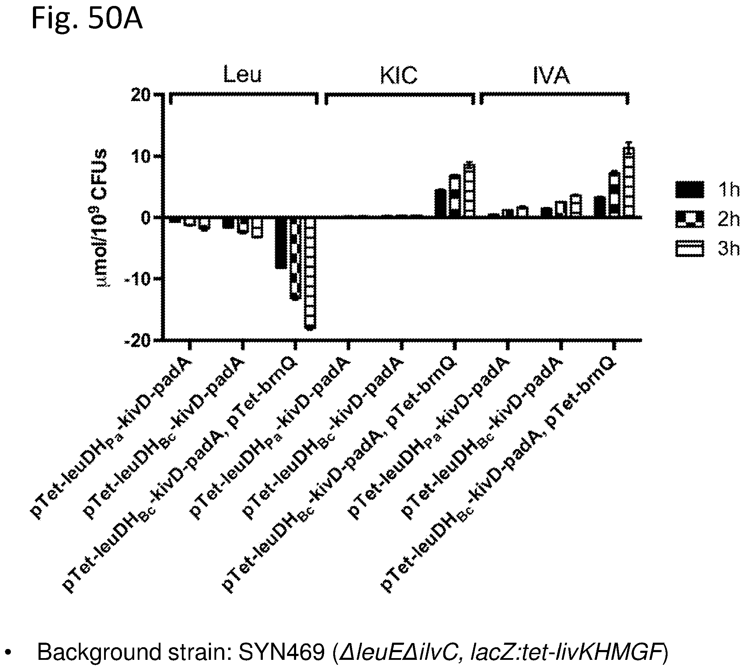

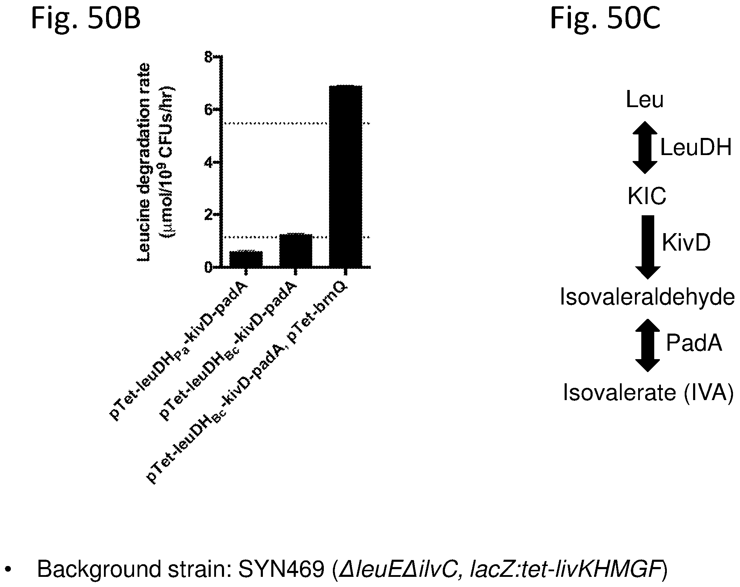

**Figs. 50A** **and** **50B** depict a bar graph which shows the leucine degration rates for various engineered bacterial strains. SYN469 is a LeuE and ilvC knockout bacterial strain and comprises the leucine transporter under the control of a tet promoter. The tet inducible leuDH-kivD-padA construct was expressed on a high copy plasmid. Two different leucine dehydrogenases were used in the tested constructs: leuDH_{PA} derived from *P. aeruginosa* and leuDH_{BC} derived from *Bacillus cereus.* The tet inducible brnQ construct was expressed on a low copy plasmid. **Fig. 50A** depicts a bargraph which shows that overexpression of the low-affinity BCAA transporter BrnQ greatly improves the rate of leucine degradation in a LeuE and ilvC knockout bacterial strain having either LeuDH derived from *P. aeruginosa* or LeuDH derived from *Bacillus cereus,* kivD, and padA with and without the BCAA transporter brnQ under the control of tet promoter as measured by leucine degradation, KIC production, and isovalerate production. **Fig. 50B** depicts a bar graph wihc shows the overexpression of the low-affinity BCAA transporter BrnQ greatly improves the rate of leucine degradation in leuDH-kivD-padA constructs. Fig. 50C depicts a schematic of the corresponding pathways.

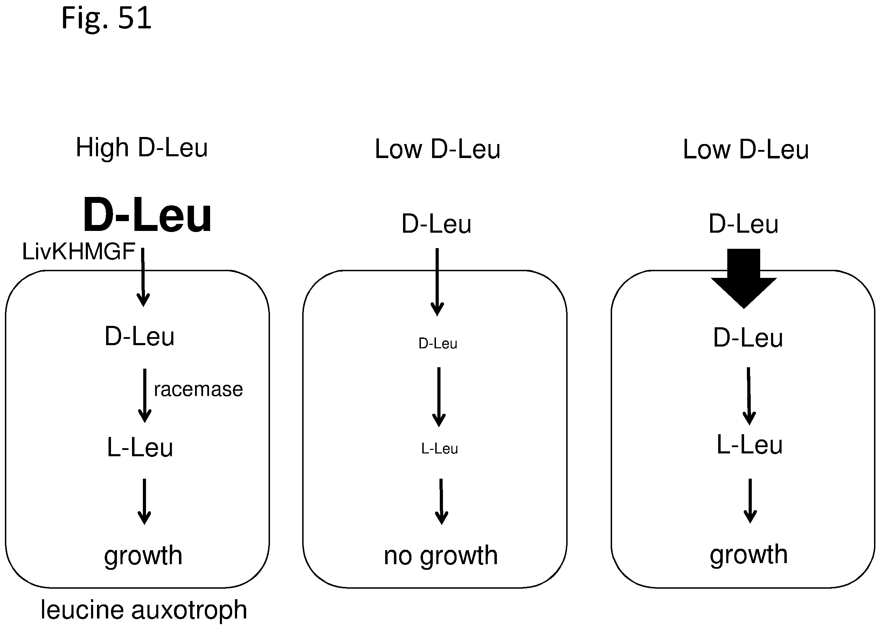

**Fig. 51** depicts a screening strategy used to identify bacterial mutants with increased Leucine transport into the bacterial cell using a leucine auxotroph. L-leucine is replaced with D-Leucine in the media. The bacteria can grow in the presence of D-leucine, because the bacterial stain has a racemase, which can convert D-leucine to L-leucine. However, the uptake of D-leucine through LivKHMGF is less efficient than the uptake of L-leucine. The leucine auxotroph can still grow if high concentrations of D-Leucine are provided, even though the D-leucine uptake is less efficient than L-leucine uptake. When concentrations of D-leucine in the media are lowered, the cells can no longer grow, unless transport efficiency is increased, ergo mutants with increased D-leucine uptake can be selected.

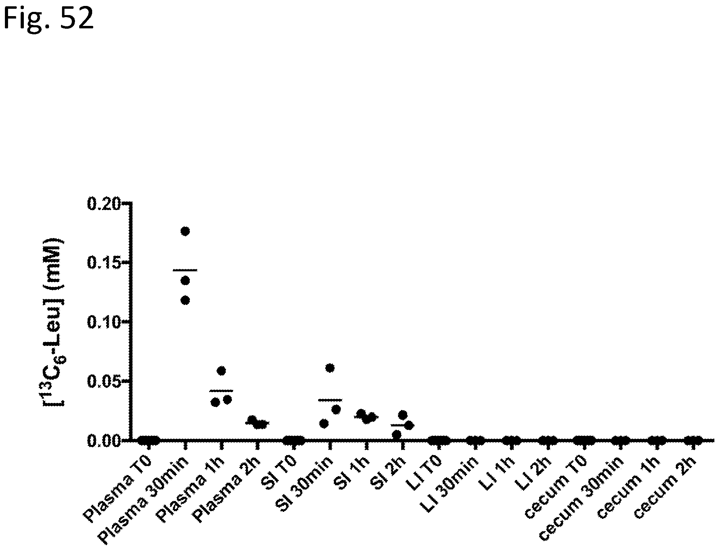

**Fig. 52** depicts a graph which shows that leucine is able to recirculate from the the periphery into the small intestine. BL6 animals were subjected to subcutaneous injection of isotopic leucine (¹³C₆) (0.1mg/g). Plasma, small intestine (SI), large intestine (LI) and cecum effluent was tested for the presence of ¹³C₆-Leucine. Ths experiment can be repeated in iMSUD animals (-/- or -/+).

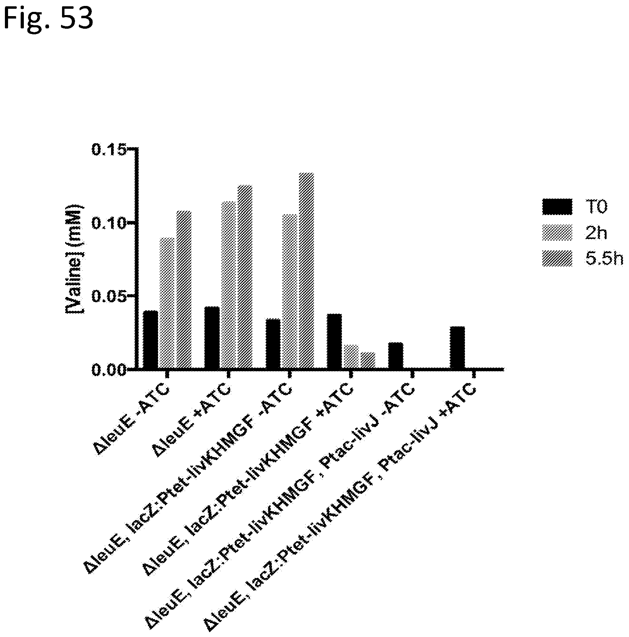

**Fig. 53** depicts a bar graph showing the efficient import of valine by the expression of an inducible leucine high affinity transporter, livKHMGF, and the constitutive expression of livJ encoding for the BCAA binding protein of the BCAA high affinity tranporter livJHMHGF. The natural secretion of valine by E. coli Nissle is observed for the ΔleuE strain. The secretion of valine is strongly reduced for ΔleuE, lacZ:Ptet-livKHMGF in the presence of ATC. This strongly suggests that the secreted valine is efficiently imported back into the cell by livKHMGF. The secretion of valine is abolished in the ΔleuE, lacZ:Ptet-livKHMGF, Ptac-livJ strain, with or without ATC. This strongly suggests that the constitutive expression of livJ is sufficient to import back the entire amount of valine secreted by the cell via the livJHMGF transporter.

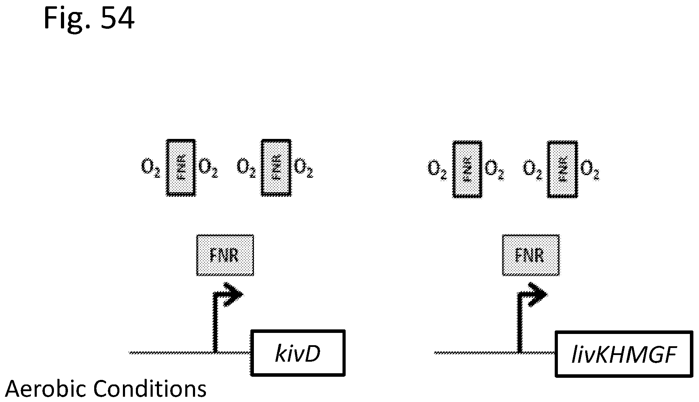

**Fig. 54** depicts a schematic of the state of non-limiting embodiments of the disclosure. **Fig. 54** depicts a schematic of the state of one non-limiting embodiment of the *kivD* or *livKHMGF* construct under non-inducing conditions, and relatively low KivD and LivKHMGF production under aerobic conditions due to oxygen (O₂) preventing FNR from dimerizing and activating the FNR responsive promoter and the *kivD* or *livKHMGF* genes under its control.

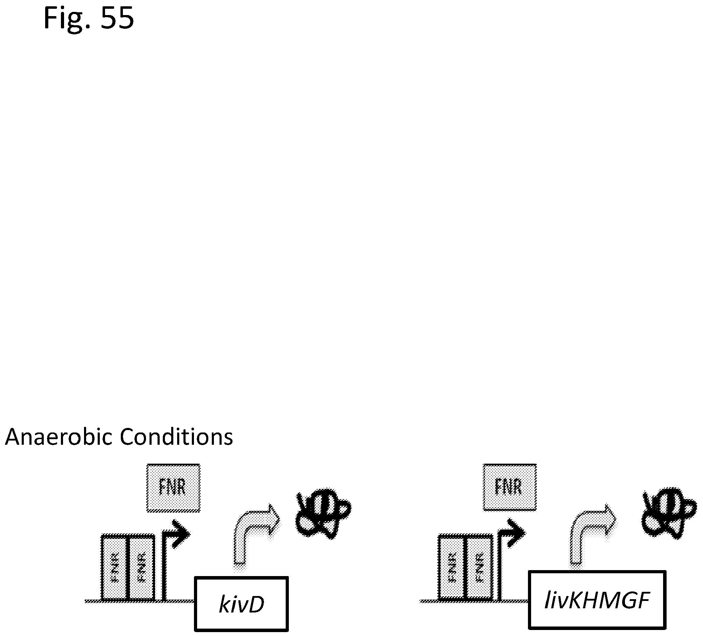

**Fig. 55** depicts a schematic of the state of non-limiting embodiments of the disclosure. **Fig. 55** depicts a schematic of the state of one non-limiting embodiment of the *kivD* or *livKHMGF* construct under inducing (low oxygen or anaerobic) conditions. **Fig. 55** depicts up-regulated KivD and LivHKMGF production under anaerobic conditions due to FNR dimerizing and inducing FNR responsive promoter-mediated expression of *kivD* and *livKHMGF* (squiggle above kivD and livKHMGF). Each arrow adjacent to one or a cluster of rectangles depicts the promoter responsible for driving transcription, in the direction of the arrow, of such gene(s). Arrows above each rectangle depict the expression product of each gene.

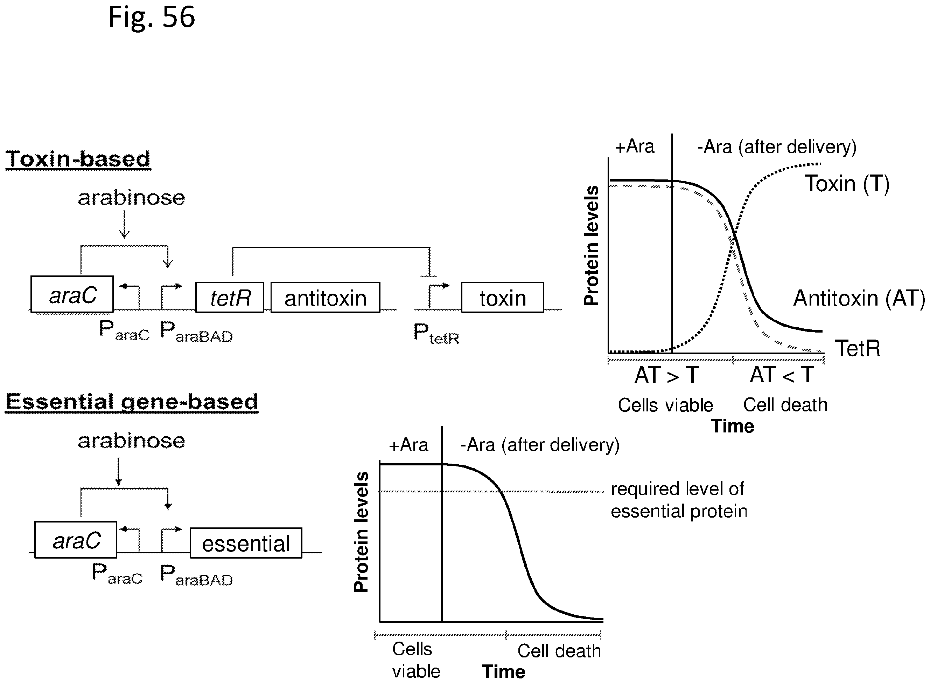

**Fig. 56** depicts a schematic of another non-limiting embodiment of the disclosure, wherein the expression of a heterologous gene is activated by an exogenous environmental signal, e.g., low-oxygen conditions. In the absence of arabinose, the AraC transcription factor adopts a conformation that represses transcription. In the presence of arabinose, the AraC transcription factor undergoes a conformational change that allows it to bind to and activate the araBAD promoter, which induces expression of TetR (tet repressor) and an anti-toxin. The anti-toxin builds up in the recombinant bacterial cell, while TetR prevents expression of a toxin (which is under the control of a promoter having a TetR binding site). However, when arabinose is not present, both the anti-toxin and TetR are not expressed. Since TetR is not present to repress expression of the toxin, the toxin is expressed and kills the cell. **Fig. 56** also depicts another non-limiting embodiment of the disclosure, wherein the expression of an essential gene not found in the recombinant bacteria is activated by an exogenous environmental signal. In the absence of arabinose, the AraC transcription factor adopts a conformation that represses transcription of the essential gene under the control of the araBAD promoter and the bacterial cell cannot survive. In the presence of arabinose, the AraC transcription factor undergoes a conformational change that allows it to bind to and activate the araBAD promoter, which induces expression of the essential gene and maintains viability of the bacterial cell.

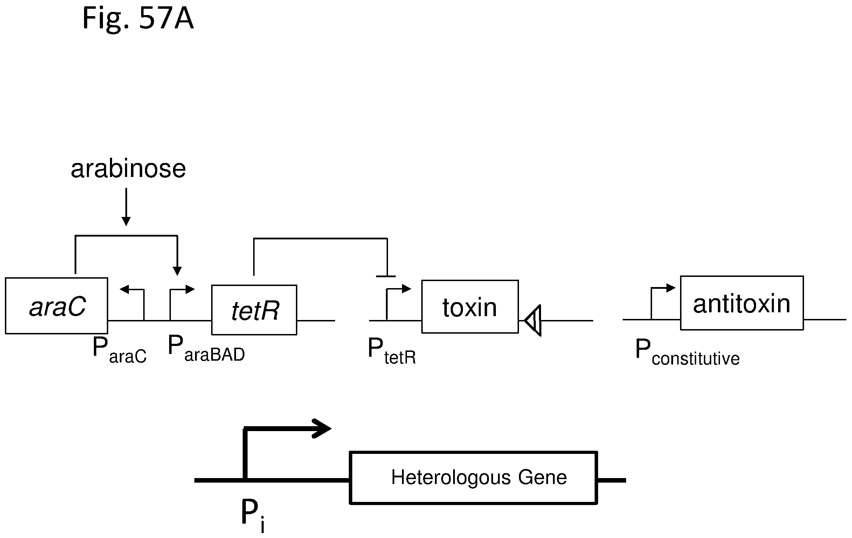

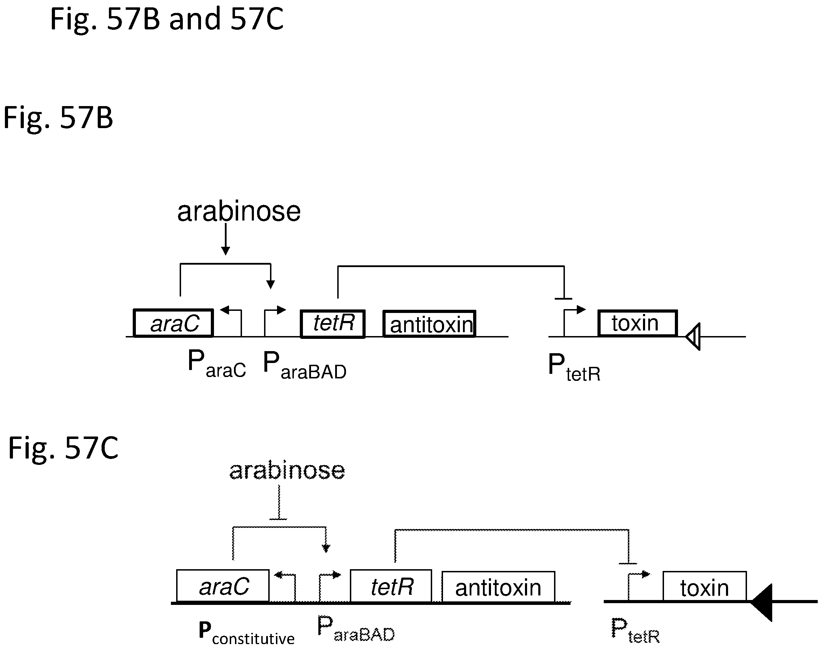

**Fig. 57** depicts schematics of non-limiting examples of the disclosure. **Fig. 57A** depicts a schematic of a non-limiting embodiment of the disclosure, where an anti-toxin is expressed from a constitutive promoter, and expression of a heterologous gene is activated by an exogenous environmental signal. In the absence of arabinose, the AraC transcription factor adopts a conformation that represses transcription. In the presence of arabinose, the AraC transcription factor undergoes a conformational change that allows it to bind to and activate the araBAD promoter, which induces expression of TetR, thus preventing expression of a toxin. However, when arabinose is not present, TetR is not expressed, and the toxin is expressed, eventually overcoming the anti-toxin and killing the cell. The constitutive promoter regulating expression of the anti-toxin should be a weaker promoter than the promoter driving expression of the toxin. The *araC* gene is under the control of a constitutive promoter in this circuit. **Fig. 57B** depicts a schematic of a repression-based kill switch in which the AraC transcription factor is activated in the presence of arabinose and induces expression of TetR and an anti-toxin. TetR prevents the expression of the toxin. When arabinose is removed, TetR and the anti-toxin do not get made and the toxin is produced which kills the cell. **Fig. 57C** depicts another non-limiting embodiment of the disclosure, wherein the expression of a heterologous gene is activated by an exogenous environmental signal. In the absence of arabinose, the AraC transcription factor adopts a conformation that represses transcription. In the presence of arabinose, the AraC transcription factor undergoes a conformational change that allows it to bind to and activate the araBAD promoter, which induces expression of TetR (tet repressor) and an anti-toxin. The anti-toxin builds up in the recombinant bacterial cell, while TetR prevents expression of a toxin (which is under the control of a promoter having a TetR binding site). However, when arabinose is not present, both the anti-toxin and TetR are not expressed. Since TetR is not present to repress expression of the toxin, the toxin is expressed and kills the cell. The *araC* gene is under the control of a constitutive promoter in this circuit.
[02] **Fig. 58** depicts a schematic of one non-limiting embodiment of the disclosure, where an exogenous environmental condition, e.g., low-oxygen conditions, or one or more environmental signals activates expression of a heterologous gene and at least one recombinase from an inducible promoter or inducible promoters. The recombinase then flips a toxin gene into an activated conformation, and the natural kinetics of the recombinase create a time delay in expression of the toxin, allowing the heterologous gene to be fully expressed. Once the toxin is expressed, it kills the cell.

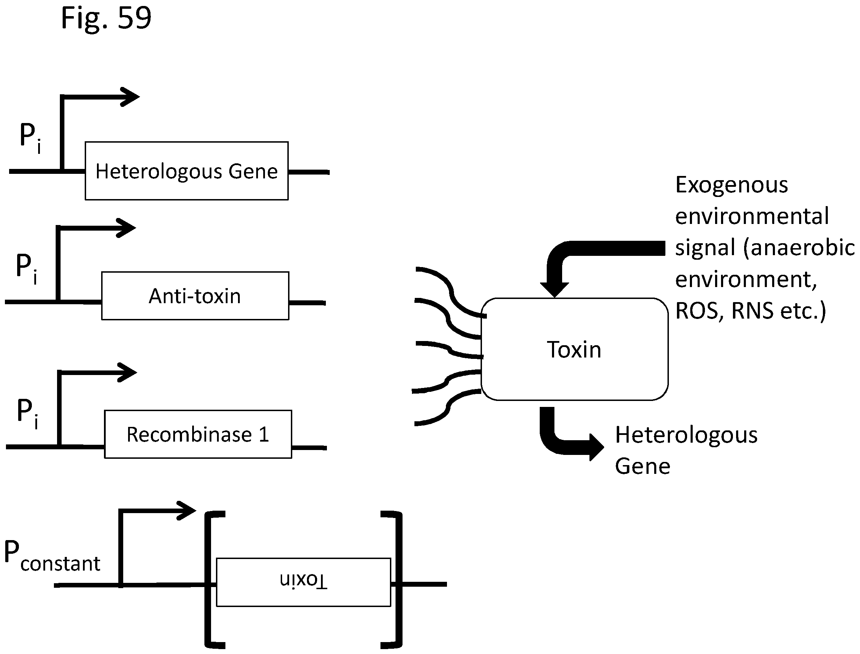

**Fig. 59** depicts a schematic of another non-limiting embodiment of the disclosure, where an exogenous environmental condition, e.g., low-oxygen conditions, or one or more environmental signals activates expression of a heterologous gene, an anti-toxin, and at least one recombinase from an inducible promoter or inducible promoters. The recombinase then flips a toxin gene into an activated conformation, but the presence of the accumulated anti-toxin suppresses the activity of the toxin. Once the exogenous environmental condition or cue(s) is no longer present, expression of the anti-toxin is turned off. The toxin is constitutively expressed, continues to accumulate, and kills the bacterial cell.

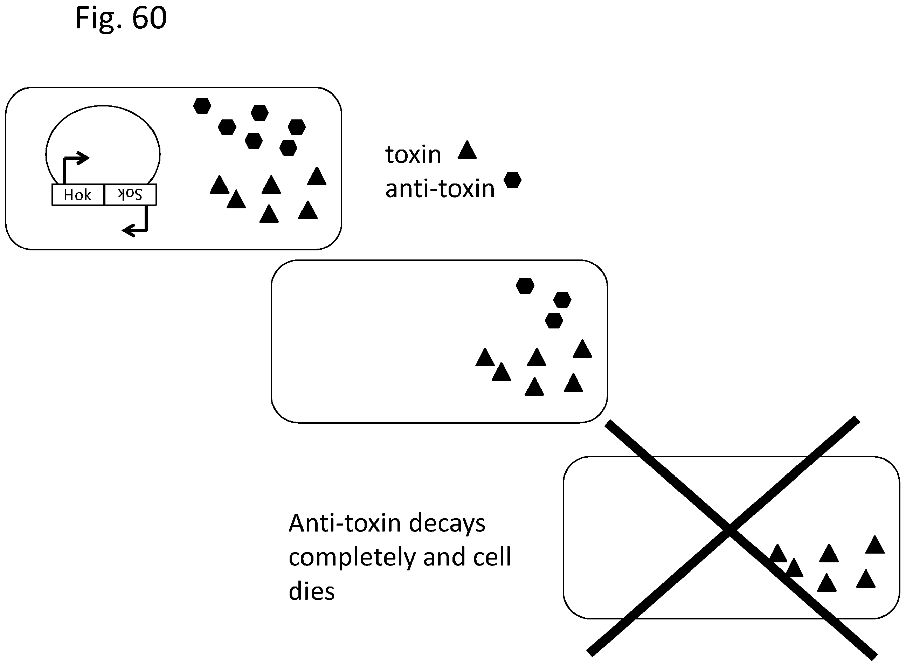

**Fig. 60** depicts aschematic of one non-limiting embodiment of the disclosure, in which the genetically engineered bacteria produces equal amount of a Hok toxin and a short-lived Sok anti-toxin. When the cell loses the plasmid, the anti-toxin decays, and the cell dies. In the upper panel, the cell produces equal amounts of toxin and anti-toxin and is stable. In the center panel, the cell loses the plasmid and anti-toxin begins to decay. In the lower panel, the anti-toxin decays completely, and the cell dies.

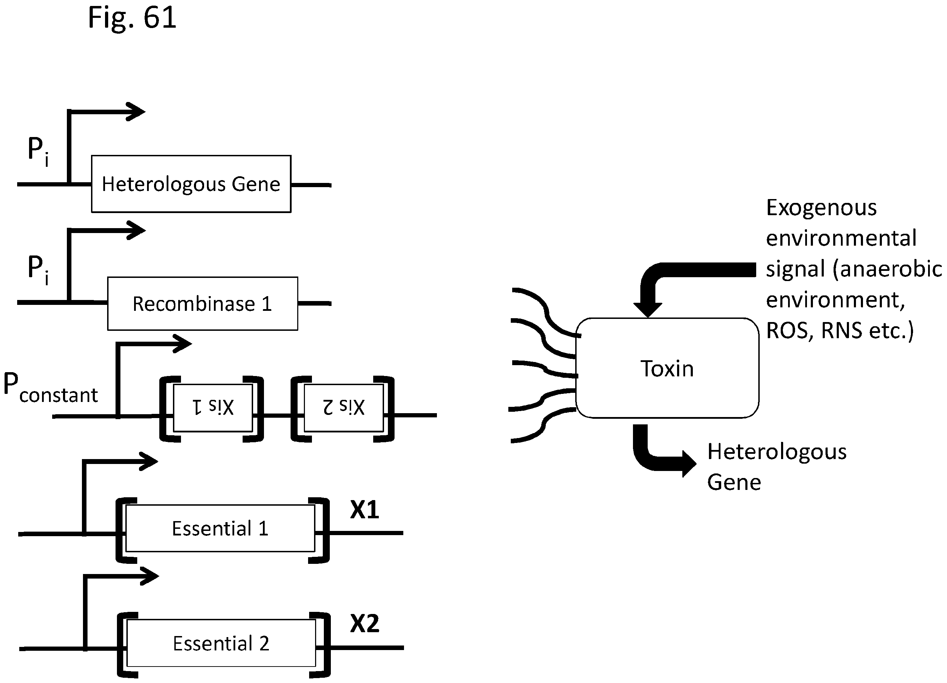

**Fig. 61** depicts a schematic of another non-limiting embodiment of the disclosure, where an exogenous environmental condition, e.g., low-oxygen conditions, or one or more environmental signals activates expression of a heterologous gene and at least one recombinase from an inducible promoter or inducible promoters. The recombinase then flips at least one excision enzyme into an activated conformation. The at least one excision enzyme then excises one or more essential genes, leading to senescence, and eventual cell death. The natural kinetics of the recombinase and excision genes cause a time delay, the kinetics of which can be altered and optimized depending on the number and choice of essential genes to be excised, allowing cell death to occur within a matter of hours or days. The presence of multiple nested recombinases can be used to further control the timing of cell death.

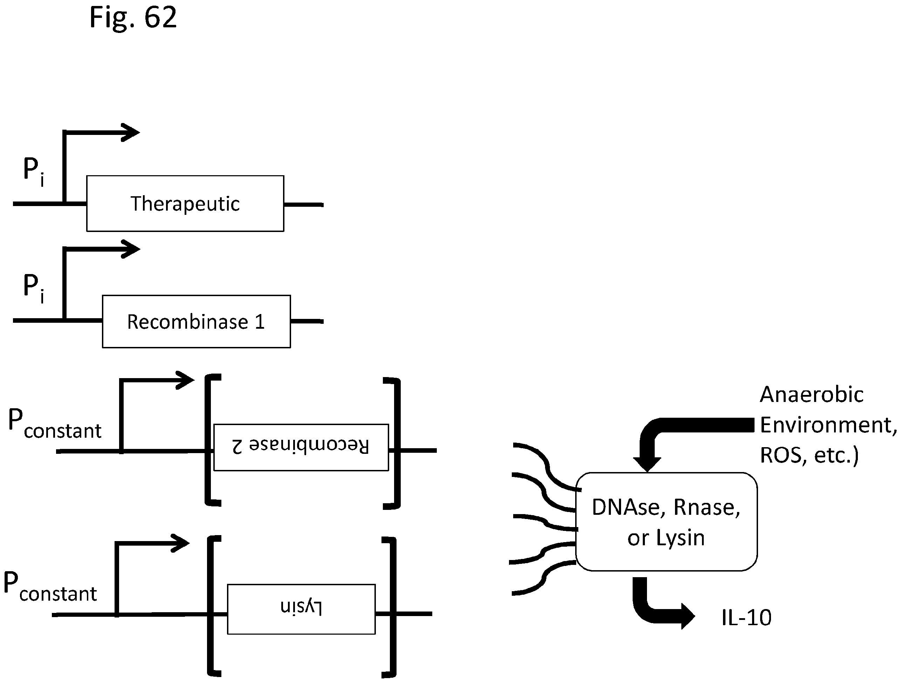

**Fig. 62** depicts a schematic of another non-limiting embodiment of the disclosure, where an exogenous environmental condition, e.g., low-oxygen conditions, or one or more environmental signals activates expression of a heterologous gene, an anti-toxin, and at least one recombinase from an inducible promoter or inducible promoters. The recombinase then flips a toxin gene into an activated conformation, but the presence of the accumulated anti-toxin suppresses the activity of the toxin. Once the exogenous environmental condition or cue(s) is no longer present, expression of the anti-toxin is turned off. The toxin is constitutively expressed, continues to accumulate, and kills the bacterial cell.

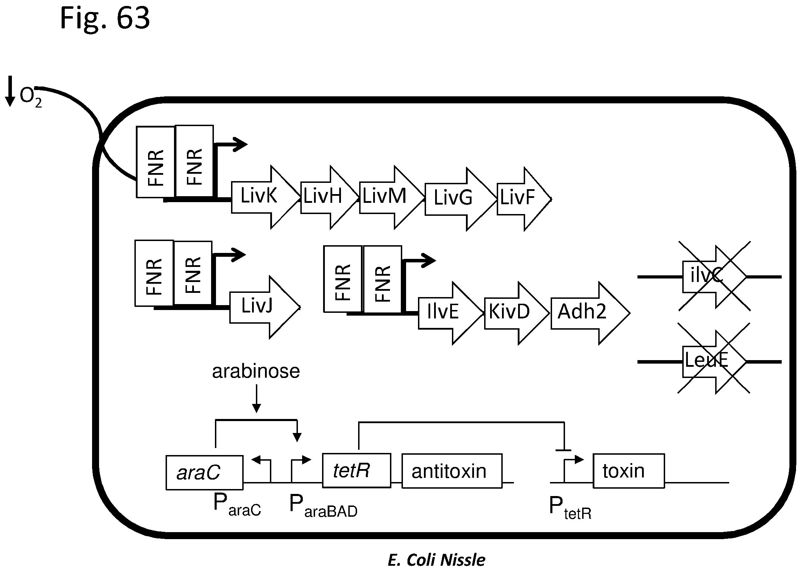

**Fig. 63** depicts a schematic of one exemplary branched chain amino acid circuit and an exemplary of kill switch design combined in one strain. Genes shown are high affinity leucine transporter complex (LivKHMGF), BCAA amino transferase (ilvE), the branched chain α-ketoacid decarboxylase (KivD), e.g., from *Lactococcus lactis,* and alcohol dehydrogenase 2 (Adh2), e.g., from *Saccharomyces cerevisiae.* The genes for the leucine exporter (LeuE) and keto acid reductoisomerase (IlvC) have been deleted. The gene for LivJ is added which can be under the control of the native promoter or the constitutive promoter Ptac. One or more of the genes encoding a catabolic enzyme, transporter, and/or other genes (e.g., livJ) may be under the control of an inducible promoter that is induced under exogenous environmental conditions, such as any of the inducible promoters provided herein, e.g., a promoter induced under low oxygen or anaerobic conditions. The strain also comprises a repression-based kill switch in which the AraC transcription factor is activated in the presence of arabinose and induces expression of TetR and an anti-toxin. TetR prevents the expression of the toxin. When arabinose is removed, TetR and the anti-toxin do not get made and the toxin is produced which kills the cell.

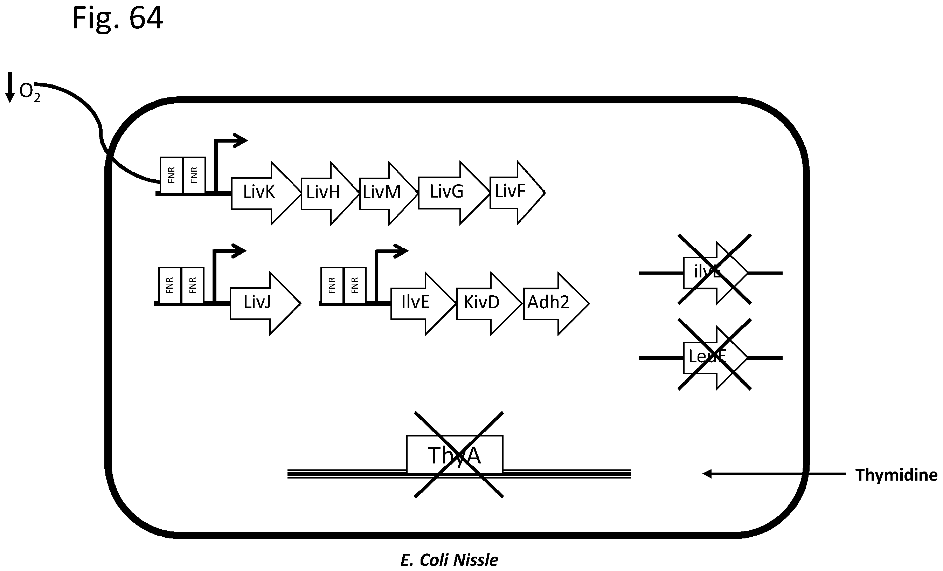

**Fig. 64** depicts a schematic of one exemplary branched chain amino acid circuit and a ThyA auxotrophy. Genes shown are high affinity leucine transporter complex (LivKHMGF), BCAA amino transferase (ilvE), the branched chain α-ketoacid decarboxylase (KivD), e.g., from *Lactococcus lactis,* and alcohol dehydrogenase 2 (Adh2), e.g., from *Saccharomyces cerevisiae.* The genes for the leucine exporter (LeuE) and keto acid reductoisomerase (IlvC) have been deleted. The gene for LivJ is added which can be under the control of the native promoter or the constitutive promoter Ptac. One or more of the genes encoding a catabolic enzyme, transporter, and/or other genes (e.g., livJ) may be under the control of an inducible promoter that is induced under exogenous environmental conditions, such as any of the inducible promoters provided herein, e.g., a promoter induced under low oxygen or anaerobic conditions.

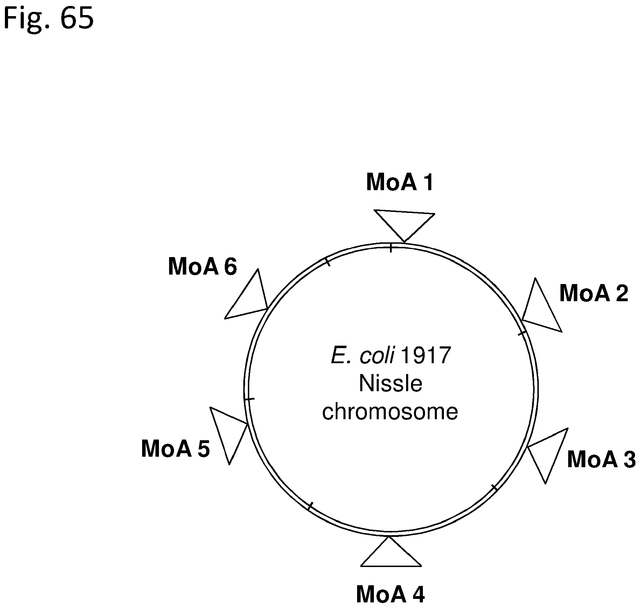

**Fig. 65** depicts an exemplary schematic of the E. coli 1917 Nissle chromosome comprising multiple mechanisms of action (MoAs).
**Fig. 66** depicts a map of exemplary integration sites within the *E. coli* 1917 Nissle chromosome. These sites indicate regions where circuit components may be inserted into the chromosome without interfering with essential gene expression. Backslashes (/) are used to show that the insertion will occur between divergently or convergently expressed genes. Insertions within biosynthetic genes, such as *thyA,* can be useful for creating nutrient auxotrophies. In some embodiments, an individual circuit component is inserted into more than one of the indicated sites.

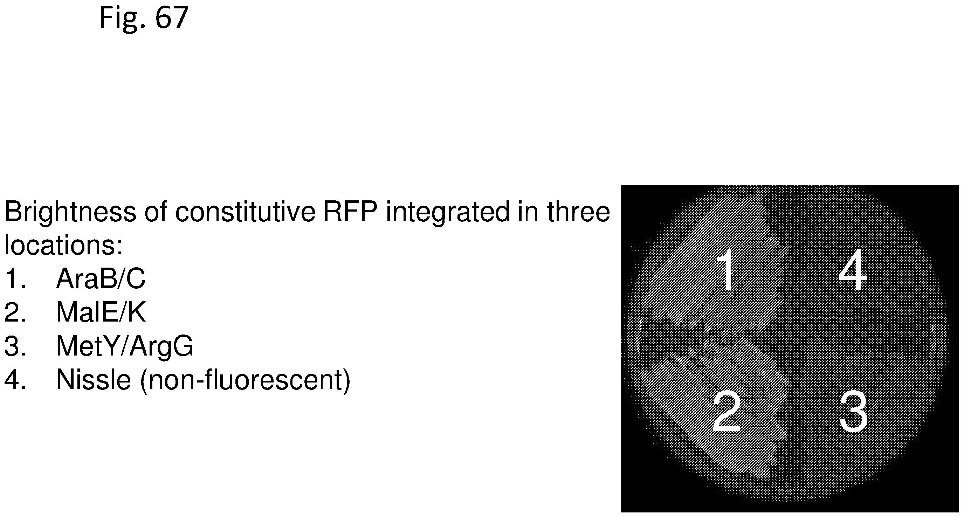

**Fig. 67** depicts three bacterial strains which constitutively express red fluorescent protein (RFP). In strains 1-3, the *rfp* gene has been inserted into different sites within the bacterial chromosome, and results in varying degrees of brightness under fluorescent light. Unmodified *E. coli* Nissle (strain 4) is non-fluorescent.

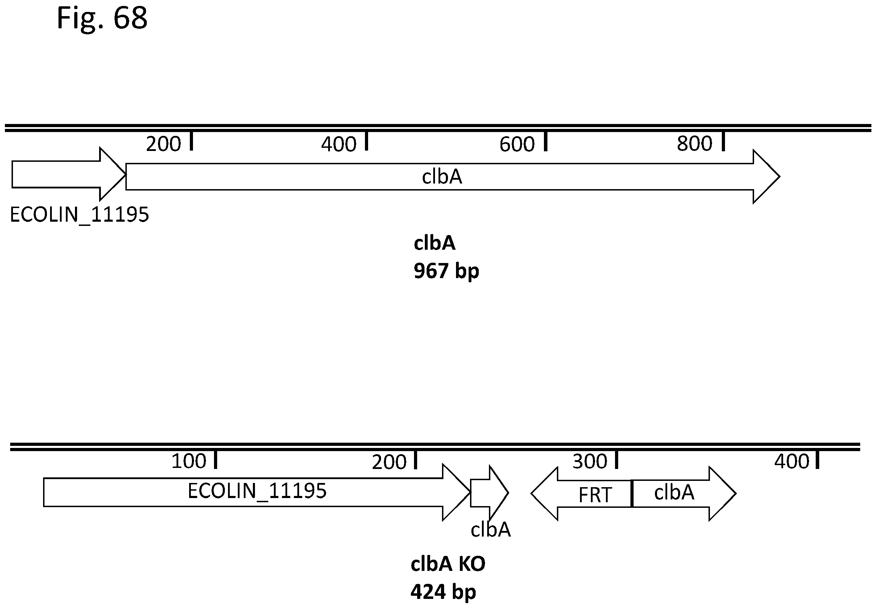

Fig. 68A and 68B depicts a schematic diagram of a wild-type clbA construct (upper panel) and a schematic diagram of a clbA knockout construct (lower panel).

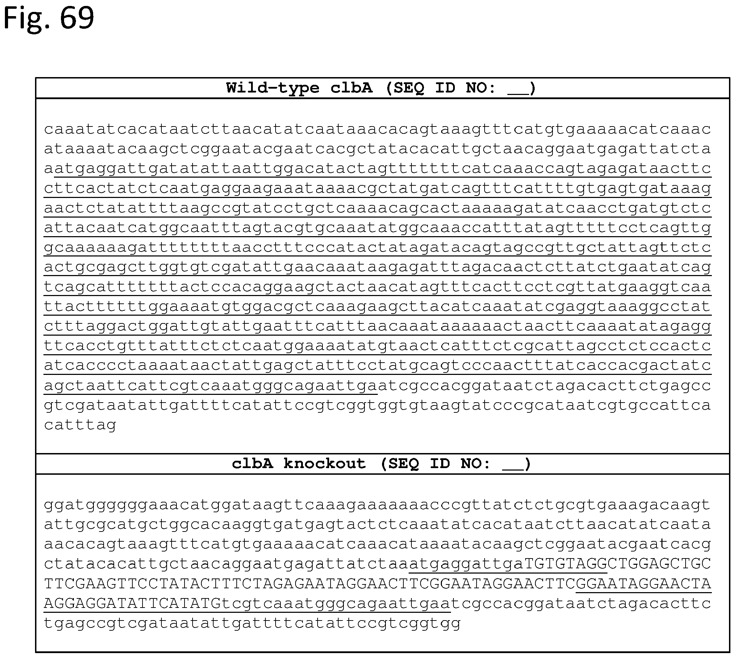

**Fig. 69** depicts exemplary sequences of a wild-type *clbA* construct and a *clbA* knock-out construct.

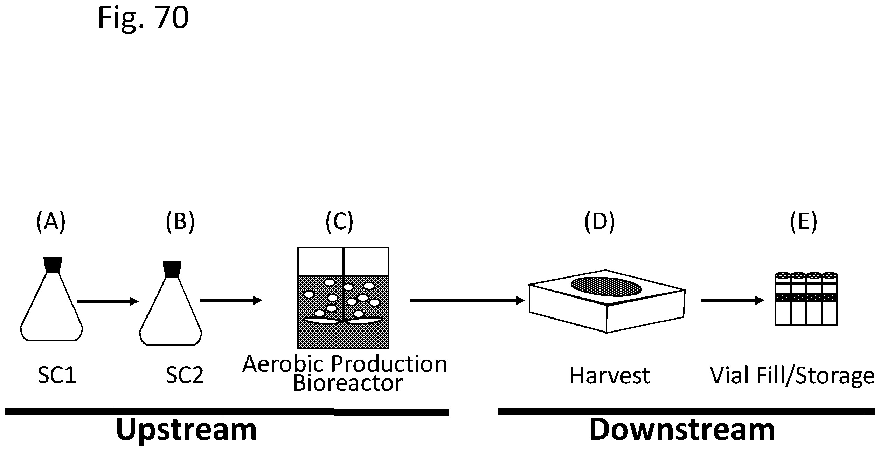

**Figs. 70A****, B, C**, **D,** and **E** depict a schematic of non-limiting manufacturing processes for upstream and downstream production of the genetically engineered bacteria of the present disclosure. **Fig. 70A** depicts the parameters for starter culture 1 (SC1): loop full - glycerol stock, duration overnight, temperature 37° C, shaking at 250 rpm. **Fig. 70B** depicts the parameters for starter culture 2 (SC2): 1/100 dilution from SC1, duration 1.5 hours, temperature 37° C, shaking at 250 rpm. **Fig. 70C** depicts the parameters for the production bioreactor: inoculum - SC2, temperature 37° C, pH set point 7.00, pH dead band 0.05, dissolved oxygen set point 50%, dissolved oxygen cascade agitation/gas FLO, agitation limits 300-1200 rpm, gas FLO limits 0.5-20 standard liters per minute, duration 24 hours. **Fig. 70D** depicts the parameters for harvest: centrifugation at speed 4000 rpm and duration 30 minutes, wash 1X 10% glycerol/PBS, centrifugation, re-suspension 10% glycerol/PBS. Fig. 70F depicts the parameters for vial fill/storage: 1-2 mL aliquots, -80° C.

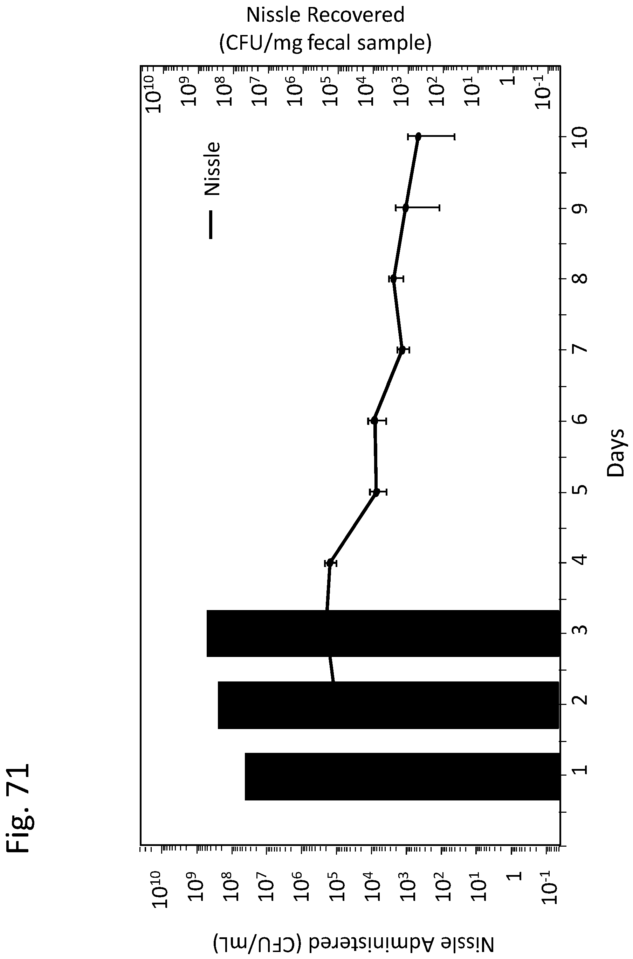

**Fig. 71** depicts a graph of Nissle residence *in vivo.* Streptomycin-resistant Nissle was administered to mice via oral gavage without antibiotic pre-treatment. Fecal pellets from six total mice were monitored post-administration to determine the amount of administered Nissle still residing within the mouse gastrointestinal tract. The bars represent the number of bacteria administered to the mice. The line represents the number of Nissle recovered from the fecal samples each day for 10 consecutive days.

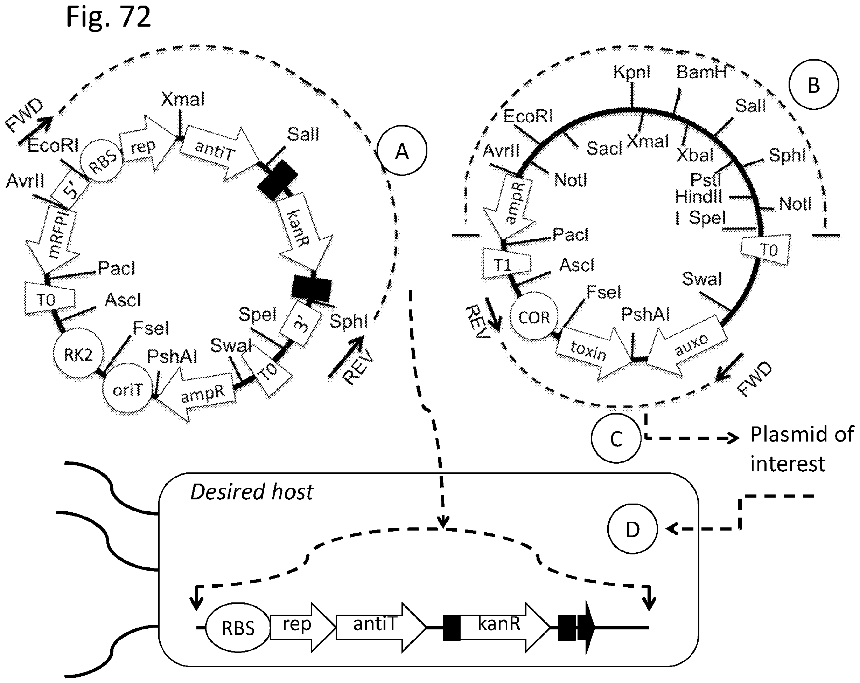

**Fig. 72** depicts an example of a genetically engineered bacteria that comprises a plasmid that has been modified to create a host-plasmid mutual dependency, such as the GeneGuard system described in more detail herein.

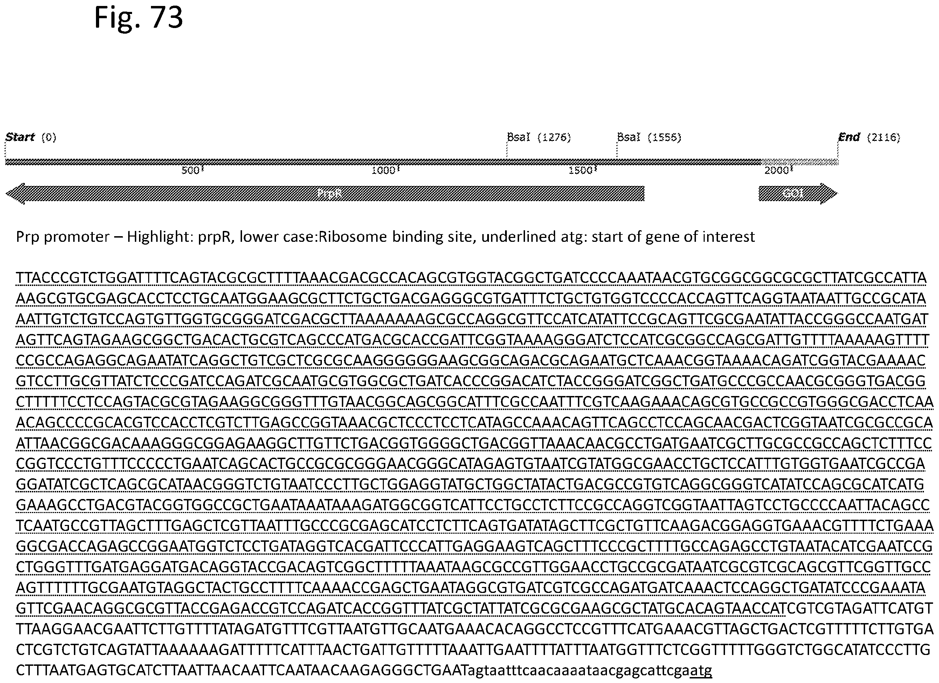

**Fig. 73** depicts the *prpR* propionate-responsive inducible promoter. The sequence for one propionate-responsive promoter is also disclosed herein as SEQ ID NO: 13.

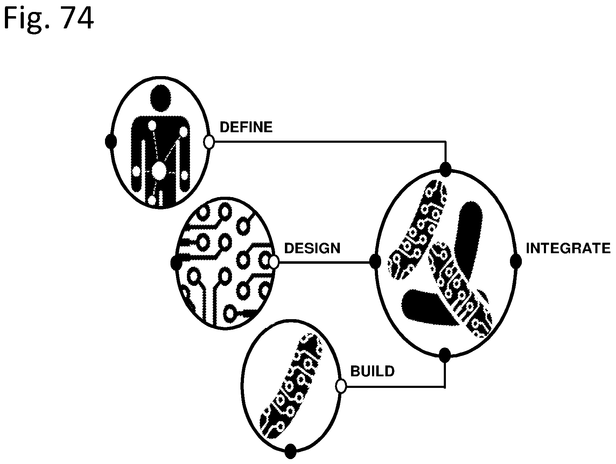

**Fig. 74** depicts a schematic of non-limiting processes for designing and producing the genetically engineered bacteria of the present disclosure. The step of "defining" comprises 1. Identification of diverse candidate approaches based on microbial physiology and disease biology; 2. Use of bioinformatics to determine candidate metabolic pathways; the use of prospective tools to determine performance targets required of optimized engineered synthetic biotics. The step of "designing" comprises the use of 1. Cutting-edge DNA assembly to enable combinatorial testing of pathway organization; 2. Mathematical models to predict pathway efficiency; 3. Internal stable of proprietary switches and parts to permit control and tuning of engineered circuits. The step of "Building" comprises 1. Building core structures "chassies" 2. Stably integrating engineered circuits into optimal chromosomal locations for efficient expression; 3. Employing unique functional assays to assess genetic circuit fidelity and activity. The step of "integrating" comprises 1. Use of chromosomal markers, which enable monitoring of synthetic biotic localization and transit times in animal models; 2. Leveraging expert microbiome network and bioinformatics support to expand understanding of how specific disease states affect GI microbial flora and the behaviors of synthetic biotics in that environment; 3. Activating process development research and optimization in-house during the discovery phase, enabling rapid and seamless transition of development candidates to pre-clinical progression; Drawing upon extensive experience in specialized disease animal model refinement, which supports prudent, high quality testing of candidate synthetic biotics.

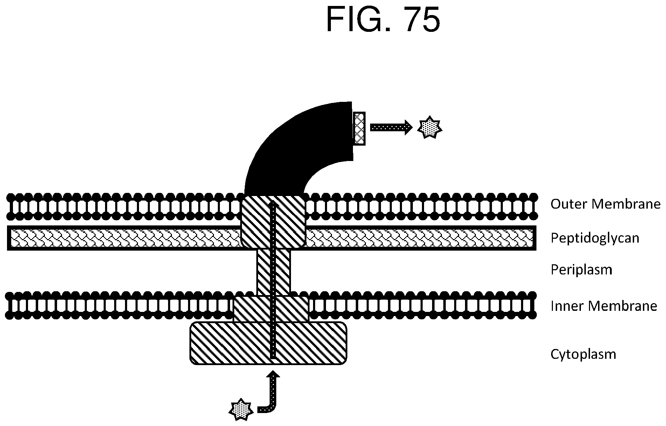

**Fig. 75** depicts a schematic of a secretion system based on the flagellar type III secretion in which an incomplete flagellum is used to secrete a therapeutic peptide of interest (star) by recombinantly fusing the peptide to an N-terminal flagellar secretion signal of a native flagellar component so that the intracellularly expressed chimeric peptide can be mobilized across the inner and outer membranes into the surrounding host environment.

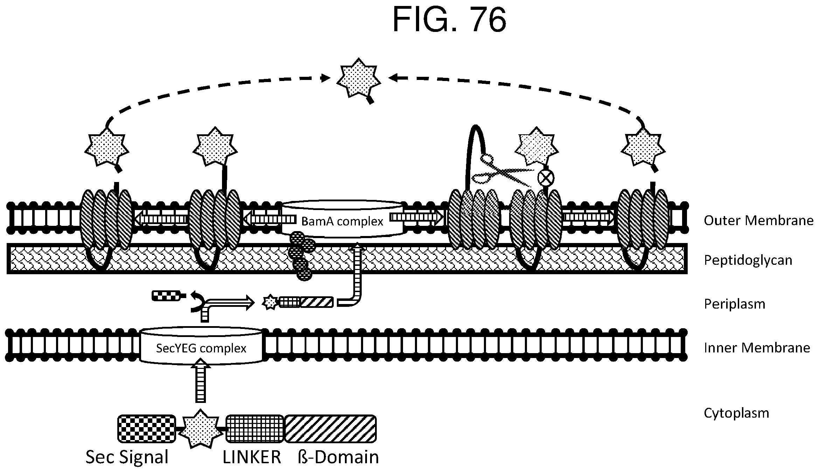

**Fig. 76** depicts a schematic of a type V secretion system for the extracellular production of recombinant proteins in which a therapeutic peptide (star) can be fused to an N-terminal secretion signal, a linker and the beta-domain of an autotransporter. In this system, the N-terminal signal sequence directs the protein to the SecA-YEG machinery which moves the protein across the inner membrane into the periplasm, followed by subsequent cleavage of the signal sequence. The beta-domain is recruited to the Bam complex where the beta-domain is folded and inserted into the outer membrane as a beta-barrel structure. The therapeutic peptide is then thread through the hollow pore of the beta-barrel structure ahead of the linker sequence. The therapeutic peptide is freed from the linker system by an autocatalytic cleavage or by targeting of a membrane-associated peptidase (scissors) to a complementary protease cut site in the linker.

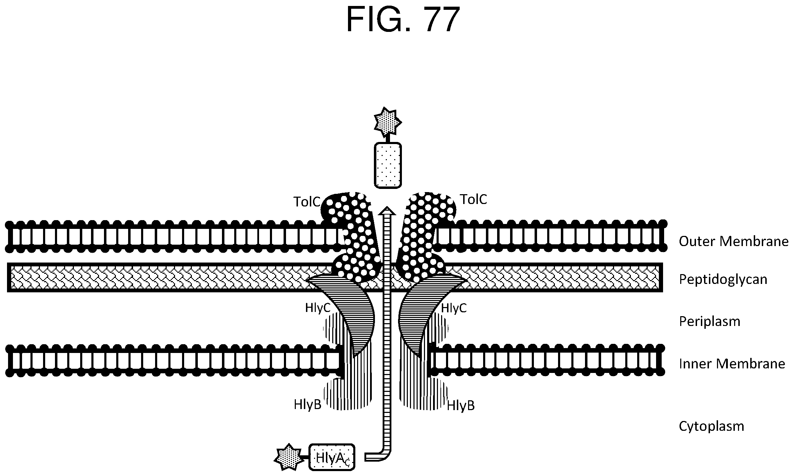

**Fig. 77** depicts a schematic of a type I secretion system, which translocates a passenger peptide directly from the cytoplasm to the extracellular space using HlyB (an ATP-binding cassette transporter); HlyD (a membrane fusion protein); and TolC (an outer membrane protein) which form a channel through both the inner and outer membranes. The secretion signal-containing C-terminal portion of HlyA is fused to the C-terminal portion of a therapeutic peptide (star) to mediate secretion of this peptide.

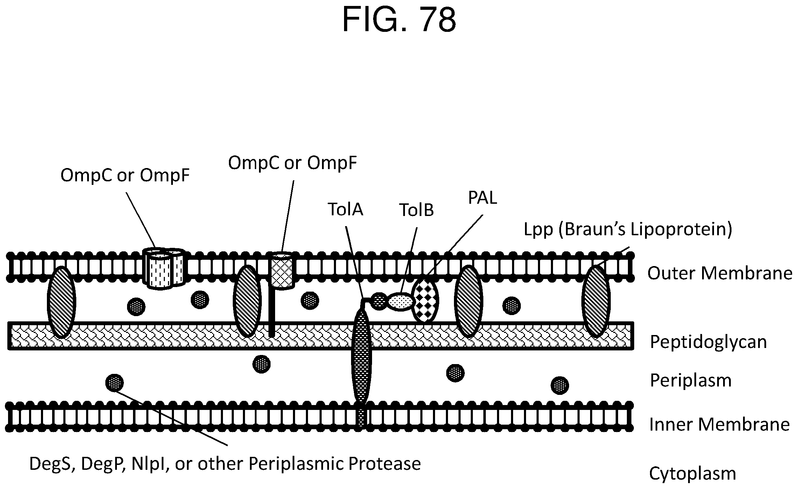

**Fig. 78** depicts a schematic of the outer and inner membranes of a gram-negative bacterium, and several deletion targets for generating a leaky or destabilized outer membrane, thereby facilitating the translocation of a therapeutic polypeptides to the extracellular space, e.g., therapeutic polypeptides of eukaryotic origin containing disulphide bonds. Deactivating mutations of one or more genes encoding a protein that tethers the outer membrane to the peptidoglycan skeleton, e.g., lpp, ompC, ompA, ompF, tolA, tolB, pal, and/or one or more genes encoding a periplasmic protease, e.g., degS, degP, nlpl, generates a leaky phenotype. Combinations of mutations may synergistically enhance the leaky phenotype.

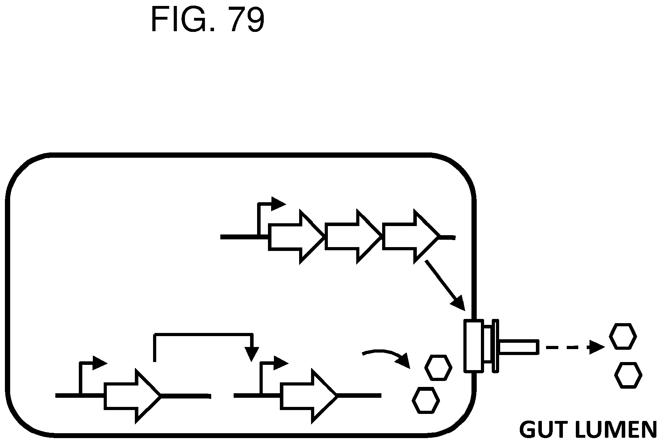

**Fig. 79** depicts a modified type 3 secretion system (T3SS) to allow the bacteria to inject secreted therapeutic proteins into the gut lumen. An inducible promoter (small arrow, top), e.g. a FNR-inducible promoter, drives expression of the T3 secretion system gene cassette (3 large arrows, top) that produces the apparatus that secretes tagged peptides out of the cell. An inducible promoter (small arrow, bottom), e.g. a FNR-inducible promoter, drives expression of a regulatory factor, e.g. T7 polymerase, that then activates the expression of the tagged therapeutic peptide (hexagons).

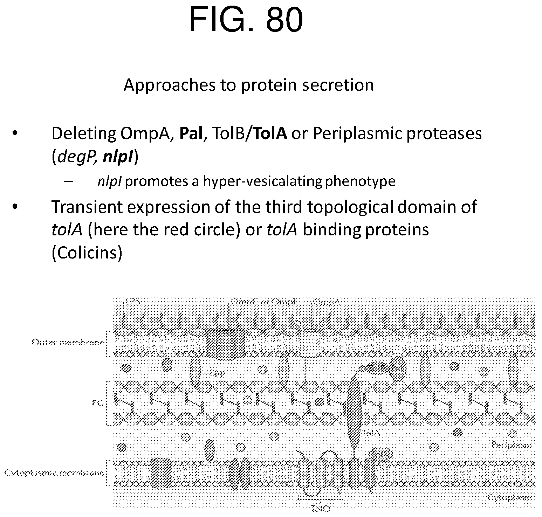

**Fig. 80** depicts approaches to protein secretion, including deletion of OmpA, Pal, TolB/TolA, or periplasmic proteases such as DegP or nlpl; or by traisient expression of the third topological domain of tolA (circle) or tolA binding proteins (colicins).

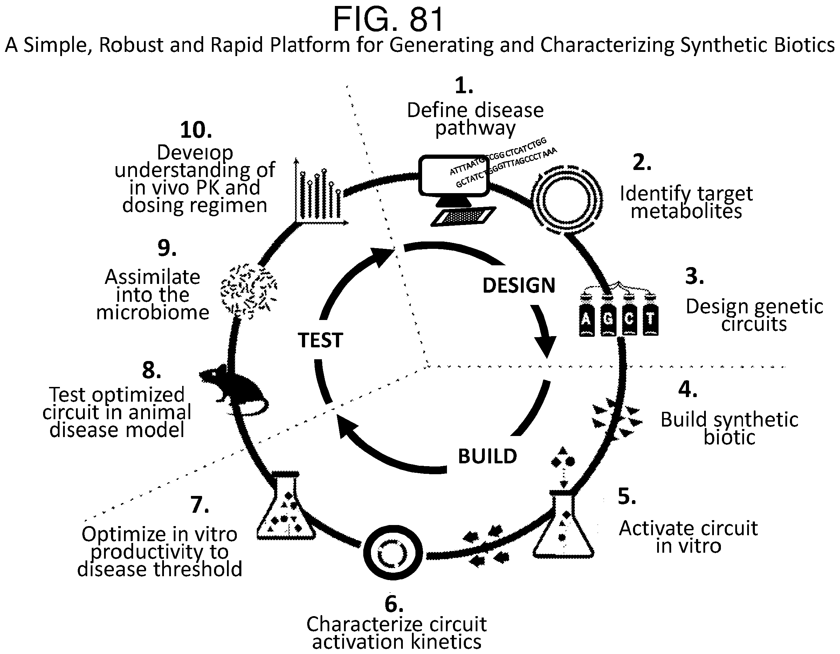

**Fig. 81** depicts a simple, robust, and rapid platform for generating and characterizing synthetic biotics, including testing, design, and building steps.

### Further Detailed Description

The disclosure includes engineered and programmed microorganisms, e.g., bacteria and viruses, pharmaceutical compositions thereof, and compositions for use in modulating and treating disorders involving the catabolism of a branched chain amino acid, such as leucine, valine, and isoleucine. In some embodiments, the microorganism, e.g., bacterium or virus, has been engineered to comprise heterologous gene sequence(s) encoding one or more branched chain amino acid catabolism enzyme(s). In some embodiments, the engineered microorganism, e.g., engineered bacterium, comprises heterologous gene sequence(s) encoding one or more branched chain amino acid catabolism enzyme(s) and is capable of reducing the level of one or more branched chain amino acidsand/or corresponding alpha-keto acid(s)and/or other corresponding metabolite(s). For example, the engineered bacterium, may comprise a BCAA transporter, such as livKHMGF and/or BrnQ. In some embodiments, the engineered bacterium comprises heterologous gene sequence(s) encoding one or more branched chain amino acid catabolism enzyme(s) and is capable of metabolizing one or more branched chain amino acidsand/or corresponding alpha-keto acid(s)and/or other corresponding metabolite(s). In some embodiments, the engineered bacterium comprises heterologous gene sequence(s) encoding one or more branched chain amino acid catabolism enzyme(s) and is capable of transporting one or more branched chain amino acidsand/or corresponding alpha-keto acid(s)and/or other corresponding metabolite(s) into the bacterium. In some embodiments, the engineered bacterium comprises heterologous gene sequence(s) encoding one or more branched chain amino acid catabolism enzyme(s) and is capable of reducing the level of and/or metabolizing one or more branched chain amino acidsand/or corresponding alpha-keto acid(s)and/or other corresponding metabolite(s) in low-oxygen environments, e.g., the gut. In some embodiments, the engineered bacteria convert the branched chain amino acid(s) and/or corresponding alpha-keto acid(s) and/or other corresponding metabolite(s) to a non-toxic or low toxicity metabolite, e.g., isovaleraldehyde, isobutyraldehyde, 2-methylbutyraldehyde, isovaleric acid, isobutyric acid, 2-methylbutyric acid, isopentanol, isobutanol, and 2-methylbutanol. In some embodiments, the engineered bacterium comprises a genetic modification that reduces export of a branched chain amino acid from the bacterial cell, for example, the bacterial cell may comprise a knockout or knock-down of a gene that encodes a BCAA exporter, such as leuE (which encodes a leucine exporter). In some embodiments, the engineered bacterium comprises gene sequence(s) or gene cassette(s) encoding one or more transporters of a branched chain amino acid, which transporters function to import one or more BCAA(s) into the bacterial cell. In some embodiments, the bacterium has been engineered to comprise a genetic modification that reduces or inhibits endogenous production of one or more branched chain amino acids and/or one or more corresponding alpha-keto acids or other metabolite(s), for example, the bacterium may comprise a knockout or knock-down of a gene that encodes a molecule required for BCAA synthesis, such as IlvC (which encodes keto acid reductoisomerase). In some embodiments, the bacterium has been engineered to comprise an auxotroph, including, for example, a BCAA auxotrophy, such as IlvC (which is required for BCAA synthesis and requires the cell to import isoleucine and valine to survive ) or other auxotrophy, as provided herein and known in the art, e.g., thyA auxotrophy. In some embodiments, the bacterium has been engineered to comprise a kill-switch, such as any of the kill-switches provided herein and known in the art. In some embodiments, the bacterium has been engineered to comprise antibiotic resistance, such as any of the antibiotic resistance provided herein and known in the art. In any of these embodiments, the gene sequence(s) encoding one or more branched chain amino acid catabolism enzyme(s), transporter(s), and other molecules can be integrated into the bacterial chromosome and/or can be present on a plasmid(s) (low copy and/or high copy). In any of these embodiments, the gene sequence(s) encoding one or more branched chain amino acid catabolism enzyme(s), transporter(s), and other molecules can be under the control of an inducible or constitutive promoter. Exemplary inducible promoters described herein include oxygen level-dependent promoters (e.g., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline.

Thus, the recombinant bacterial cells and pharmaceutical compositions comprising the bacterial cells disclosed herein may be used to catabolize branched chain amino acids, *e.g.,* leucine, isoleucine, valine, and/or their corresponding alpha-keto acid counterparts, to modify, ameliorate, treat and/or prevent conditions associated with disorders involving the catabolism of a branched chain amino acid. In one embodiment, the disorder involving the catabolism of a branched chain amino acid is a metabolic disorder involving the abnormal catabolism of a branched chain amino acid, including but not limited to maple syrup urine disease (MSUD), isovaleric acidemia, propionic acidemia, methylmalonic acidemia, or diabetes ketoacidosis, 3-MCC Deficiency, 3-Methylglutaconyl-CoA hydratase Deficiency, HMG-CoA Lyase Deficiency, Acetyl-CoA Carboxylase Deficiency, Malonyl-CoA Decarboxylase Deficiency, short-branched chain acylCoA dehydrogenase deficiency, 2-methyl-3-hydroxybutyric acidemia, beta-ketothiolase deficiency, isobutyryl-CoA dehydrogenase deficiency, HIBCH deficiency), and 3-Hydroxyisobutyric aciduria. In another embodiment, the disorder involving the catabolism of a branched chain amino acid is a disorder caused by the activation of mTor, for example, cancer, obesity, type 2 diabetes, neurodegeneration, autism, Alzheimer's disease, Lymphangioleiomyomatosis (LAM), transplant rejection, glycogen storage disease, obesity, tuberous sclerosis, hypertension, cardiovascular disease, hypothalamic activation, musculoskeletal disease, Parkinson's disease, Huntington's disease, psoriasis, rheumatoid arthritis, lupus, multiple sclerosis, Leigh's syndrome, and Friedrich's ataxia.

In order that the disclosure may be more readily understood, certain terms are first defined. These definitions should be read in light of the remainder of the disclosure and as understood by a person of ordinary skill in the art. Unless defined otherwise, all technical and scientific terms used herein have the same meaning as commonly understood by a person of ordinary skill in the art. Additional definitions are set forth throughout the detailed description.

As used herein, the term "recombinant microorganism" refers to a microorganism, e.g., bacterial or viral cell, or bacteria or virus, that has been genetically modified from its native state. Thus, a "recombinant bacterial cell" or "recombinant bacteria" refers to a bacterial cell or bacteria that have been genetically modified from their native state. For instance, a recombinant bacterial cell may have nucleotide insertions, nucleotide deletions, nucleotide rearrangements, and nucleotide modifications introduced into their DNA. These genetic modifications may be present in the chromosome of the bacteria or bacterial cell, or on a plasmid in the bacteria or bacterial cell. Recombinant bacterial cells disclosed herein may comprise exogenous nucleotide sequences on plasmids. Alternatively, recombinant bacterial cells may comprise exogenous nucleotide sequences stably incorporated into their chromosome.

A "programmed or engineered microorganism" refers to a microorganism, e.g., bacterial or viral cell, or bacteria or virus, that has been genetically modified from its native state to perform a specific function. Thus, a "programmed or engineered bacterial cell" or "programmed or engineered bacteria" refers to a bacterial cell or bacteria that has been genetically modified from its native state to perform a specific function, e.g., to metabolize a branched chain amino acid. In certain embodiments, the programmed or engineered bacterial cell has been modified to express one or more proteins, for example, one or more proteins that have a therapeutic activity or serve a therapeutic purpose. The programmed or engineered bacterial cell may additionally have the ability to stop growing or to destroy itself once the protein(s) of interest have been expressed.

As used herein, the term "gene" refers to a nucleic acid fragment that encodes a protein or fragment thereof, optionally including regulatory sequences preceding (5' non-coding sequences) and following (3' non-coding sequences) the coding sequence. In one embodiment, a "gene" does not include regulatory sequences preceding and following the coding sequence. A "native gene" refers to a gene as found in nature, optionally with its own regulatory sequences preceding and following the coding sequence. A "chimeric gene" refers to any gene that is not a native gene, optionally comprising regulatory sequences preceding and following the coding sequence, wherein the coding sequences and/or the regulatory sequences, in whole or in part, are not found together in nature. Thus, a chimeric gene may comprise regulatory sequences and coding sequences that are derived from different sources, or regulatory and coding sequences that are derived from the same source, but arranged differently than is found in nature.

As used herein, the term "gene sequence" is meant to refer to a genetic sequence, e.g., a nucleic acid sequence. The gene sequence or genetic sequence is meant to include a complete gene sequence or a partial gene sequence. The gene sequence or genetic sequence is meant to include sequence that encodes a protein or polypeptide and is also menat to include genetic sequence that does not encode a protein or polypeptide, e.g., a regulatory sequence, leader sequence, signal sequence, or other non-protein coding sequence.

As used herein, a "heterologous" gene or "heterologous sequence" refers to a nucleotide sequence that is not normally found in a given cell in nature. As used herein, a heterologous sequence encompasses a nucleic acid sequence that is exogenously introduced into a given cell and can be a native sequence (naturally found or expressed in the cell) or non-native sequence (not naturally found or expressed in the cell) and can be a natural or wild-type sequence or a variant, non-natural, or synthetic sequence. "Heterologous gene" includes a native gene, or fragment thereof, that has been introduced into the host cell in a form that is different from the corresponding native gene. For example, a heterologous gene may include a native coding sequence that is a portion of a chimeric gene to include non-native regulatory regions that is reintroduced into the host cell. A heterologous gene may also include a native gene, or fragment thereof, introduced into a non-native host cell. Thus, a heterologous gene may be foreign or native to the recipient cell; a nucleic acid sequence that is naturally found in a given cell but expresses an unnatural amount of the nucleic acid and/or the polypeptide which it encodes; and/or two or more nucleic acid sequences that are not found in the same relationship to each other in nature. As used herein, the term "endogenous gene" refers to a native gene in its natural location in the genome of an organism. As used herein, the term "transgene" refers to a gene that has been introduced into the host organism, e.g., host bacterial cell, genome.

As used herein, a "non-native" nucleic acid sequence refers to a nucleic acid sequence not normally present in a microorganism, e.g., an extra copy of an endogenous sequence, or a heterologous sequence such as a sequence from a different species, strain, or substrain of bacteria or virus, or a sequence that is modified and/or mutated as compared to the unmodified sequence from bacteria or virus of the same subtype. In some embodiments, the non-native nucleic acid sequence is a synthetic, non-naturally occurring sequence (see, e.g., Purcell et al., 2013). The non-native nucleic acid sequence may be a regulatory region, a promoter, a gene, and/or one or more genes in gene cassette. In some embodiments, "non-native" refers to two or more nucleic acid sequences that are not found in the same relationship to each other in nature. The non-native nucleic acid sequence may be present on a plasmid or chromosome. In some embodiments, the genetically engineered microorganism of the disclosure comprises a gene that is operably linked to a promoter that is not associated with said gene in nature. For example, in some embodiments, the genetically engineered bacteria disclosed herein comprise a gene that is operably linked to a directly or indirectly inducible promoter that is not associated with said gene in nature, *e.g.,* an FNR responsive promoter (or other promoter disclosed herein) operably linked to a gene encoding a branched chain amino acid catabolism enzyme. In some embodiments, the genetically engineered virus of the disclosure comprises a gene that is operably linked to a directly or indirectly inducible promoter that is not associated with said gene in nature, e.g., a promoter operably linked to a gene encoding a a branched chain amino acid catabolism enzyme.

As used herein, the term "coding region" refers to a nucleotide sequence that codes for a specific amino acid sequence. The term "regulatory sequence" refers to a nucleotide sequence located upstream (5' non-coding sequences), within, or downstream (3' non-coding sequences) of a coding sequence, and which influences the transcription, RNA processing, RNA stability, or translation of the associated coding sequence. Examples of regulatory sequences include, but are not limited to, promoters, translation leader sequences, effector binding sites, signal sequences, and stem-loop structures. In one embodiment, the regulatory sequence comprises a promoter, e.g., an FNR responsive promoter or other promoter disclosed herein.

**[1]** "Operably linked" refers to the association of nucleic acid sequences on a single nucleic acid fragment so that the function of one is affected by the other. A regulatory element is operably linked with a coding sequence when it is capable of affecting the expression of the gene coding sequence, regardless of the distance between the regulatory element and the coding sequence. More specifically, operably linked refers to a nucleic acid sequence, *e.g.,* a gene encoding a branched chain amino acid catabolism enzyme, that is joined to a regulatory sequence in a manner which allows expression of the nucleic acid sequence, *e.g.,* the gene encoding the branched chain amino acid catabolism enzyme. In other words, the regulatory sequence acts in *cis.* In one embodiment, a gene may be "directly linked" to a regulatory sequence in a manner which allows expression of the gene. In another embodiment, a gene may be "indirectly linked" to a regulatory sequence in a manner which allows expression of the gene. In one embodiment, two or more genes may be directly or indirectly linked to a regulatory sequence in a manner which allows expression of the two or more genes. A regulatory region or sequence is a nucleic acid that can direct transcription of a gene of interest and may comprise promoter sequences, enhancer sequences, response elements, protein recognition sites, inducible elements, promoter control elements, protein binding sequences, 5' and 3' untranslated regions, transcriptional start sites, termination sequences, polyadenylation sequences, and introns.

A "promoter" as used herein, refers to a nucleotide sequence that is capable of controlling the expression of a coding sequence or gene. Promoters are generally located 5' of the sequence that they regulate. Promoters may be derived in their entirety from a native gene, or be composed of different elements derived from promoters found in nature, and/or comprise synthetic nucleotide segments. Those skilled in the art will readily ascertain that different promoters may regulate expression of a coding sequence or gene in response to a particular stimulus, e.g., in a cell- or tissue-specific manner, in response to different environmental or physiological conditions, or in response to specific compounds. Prokaryotic promoters are typically classified into two classes: inducible and constitutive. A "constitutive promoter" refers to a promoter that allows for continual transcription of the coding sequence or gene under its control.

"Constitutive promoter" refers to a promoter that is capable of facilitating continuous transcription of a coding sequence or gene under its control and/or to which it is operably linked. Constitutive promoters and variants are well known in the art and include, but are not limited to, Ptac promoter, BBa_J23100, a constitutive *Escherichia coli* σ^{S} promoter (e.g., an osmY promoter (International Genetically Engineered Machine (iGEM) Registry of Standard Biological Parts Name BBa_J45992; BBa_J45993)), a constitutive *Escherichia coli* σ³² promoter (*e.g.,* htpG heat shock promoter (BBa_J45504)), a constitutive *Escherichia coli* σ⁷⁰ promoter (*e.g.,* lacq promoter (BBa_J54200; BBa_J56015), *E*. *coli* CreABCD phosphate sensing operon promoter (BBa_J64951), GlnRS promoter (BBa_K088007), lacZ promoter (BBa_K119000; BBa_K119001); M13K07 gene I promoter (BBa_M13101); M13K07 gene II promoter (BBa_M13102), M13K07 gene III promoter (BBa_M13103), M13K07 gene IV promoter (BBa_M13104), M13K07 gene V promoter (BBa_M13105), M13K07 gene VI promoter (BBa_M13106), M13K07 gene VIII promoter (BBa_M13108), M13110 (BBa_M13110)), a constitutive *Bacillus subtilis* σ^{A} promoter (*e.g.,* promoter veg (BBa_K143013), promoter 43 (BBa_K143013), P_{liaG} (BBa_K823000), P_{lepA} (BBa_K823002), P_{veg} (BBa_K823003)), a constitutive *Bacillus subtilis* σ^{B} promoter (*e.g.,* promoter etc (BBa_K143010), promoter gsiB (BBa_K143011)), a *Salmonella* promoter (*e.g.,* Pspv2 from *Salmonella* (BBa_K112706), Pspv from *Salmonella* (BBa_K112707)), a bacteriophage T7 promoter (*e.g*., T7 promoter (BBa_I712074; BBa_I719005; BBa_J34814; BBa_J64997; BBa_K113010; BBa_K113011; BBa_K113012; BBa_R0085; BBa_R0180; BBa_R0181; BBa_R0182; BBa_R0183; BBa_Z0251; BBa_Z0252; BBa_Z0253)), and a bacteriophage SP6 promoter (*e.g*., SP6 promoter (BBa_J64998)).

An "inducible promoter" refers to a regulatory region that is operably linked to one or more genes, wherein expression of the gene(s) is increased in the presence of an inducer of said regulatory region. An "inducible promoter" refers to a promoter that initiates increased levels of transcription of the coding sequence or gene under its control in response to a stimulus or an exogenous environmental condition. A "directly inducible promoter" refers to a regulatory region, wherein the regulatory region is operably linked to a gene encoding a protein or polypeptide, where, in the presence of an inducer of said regulatory region, the protein or polypeptide is expressed. An "indirectly inducible promoter" refers to a regulatory system comprising two or more regulatory regions, for example, a first regulatory region that is operably linked to a first gene encoding a first protein, polypeptide, or factor, *e.g.,* a transcriptional regulator, which is capable of regulating a second regulatory region that is operably linked to a second gene, the second regulatory region may be activated or repressed, thereby activating or repressing expression of the second gene. Both a directly inducible promoter and an indirectly inducible promoter are encompassed by "inducible promoter." Exemplary inducible promoters described herein include oxygen level-dependent promoters (e.g., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein. Examples of other inducible promoters are provided herein below.

As used herein, "stably maintained" or "stable" bacterium is used to refer to a bacterial host cell carrying non-native genetic material, *e.g.,* a gene encoding a branched chain amino acid catabolism enzyme, which is incorporated into the host genome or propagated on a self-replicating extra-chromosomal plasmid, such that the non-native genetic material is retained, expressed, and propagated. The stable bacterium is capable of survival and/or growth *in vitro, e.g.,* in medium, and/or *in vivo, e.g.,* in the gut. For example, the stable bacterium may be a genetically engineered bacterium comprising a gene encoding a branched chain amino acid catabolism enzyme, in which the plasmid or chromosome carrying the gene is stably maintained in the bacterium, such that branched chain amino acid catabolism enzyme can be expressed in the bacterium, and the bacterium is capable of survival and/or growth *in vitro* and/or *in vivo.* In some embodiments, copy number affects the stability of expression of the non-native genetic material. In some embodiments, copy number affects the level of expression of the non-native genetic material.

As used herein, the term "expression" refers to the transcription and stable accumulation of sense (mRNA) or anti-sense RNA derived from a nucleic acid, and/or to translation of an mRNA into a polypeptide.

As used herein, the term "plasmid" or "vector" refers to an extrachromosomal nucleic acid, *e.g.,* DNA, construct that is not integrated into a bacterial cell's genome. Plasmids are usually circular and capable of autonomous replication. Plasmids may be low-copy, medium-copy, or high-copy, as is well known in the art. Plasmids may optionally comprise a selectable marker, such as an antibiotic resistance gene, which helps select for bacterial cells containing the plasmid and which ensures that the plasmid is retained in the bacterial cell. A plasmid disclosed herein may comprise a nucleic acid sequence encoding a heterologous gene, *e.g.,* a gene encoding a branched chain amino acid catabolism enzyme.

As used herein, the term "transform" or "transformation" refers to the transfer of a nucleic acid fragment into a host bacterial cell, resulting in genetically-stable inheritance. Host bacterial cells comprising the transformed nucleic acid fragment are referred to as "recombinant" or "transgenic" or "transformed" organisms.

The term "genetic modification," as used herein, refers to any genetic change. Exemplary genetic modifications include those that increase, decrease, or abolish the expression of a gene, including, for example, modifications of native chromosomal or extrachromosomal genetic material. Exemplary genetic modifications also include the introduction of at least one plasmid, modification, mutation, base deletion, base addition, base substitution, and/or codon modification of chromosomal or extrachromosomal genetic sequence(s), gene over-expression, gene amplification, gene suppression, promoter modification or substitution, gene addition (either single or multi-copy), antisense expression or suppression, or any other change to the genetic elements of a host cell, whether the change produces a change in phenotype or not. Genetic modification can include the introduction of a plasmid, *e.g.,* a plasmid comprising a branched chain amino acid catabolism enzyme operably linked to a promoter, into a bacterial cell. Genetic modification can also involve a targeted replacement in the chromosome, *e.g.,* to replace a native gene promoter with an inducible promoter, regulated promoter, strong promoter, or constitutive promoter. Genetic modification can also involve gene amplification, *e.g.,* introduction of at least one additional copy of a native gene into the chromosome of the cell. Alternatively, chromosomal genetic modification can involve a genetic mutation.

As used herein, the term "genetic mutation" refers to a change or changes in a nucleotide sequence of a gene or related regulatory region that alters the nucleotide sequence as compared to its native or wild-type sequence. Mutations include, for example, substitutions, additions, and deletions, in whole or in part, within the wild-type sequence. Such substitutions, additions, or deletions can be single nucleotide changes (*e.g.,* one or more point mutations), or can be two or more nucleotide changes, which may result in substantial changes to the sequence. Mutations can occur within the coding region of the gene as well as within the non-coding and regulatory sequence of the gene. The term "genetic mutation" is intended to include silent and conservative mutations within a coding region as well as changes which alter the amino acid sequence of the polypeptide encoded by the gene. A genetic mutation in a gene coding sequence may, for example, increase, decrease, or otherwise alter the activity (*e.g.,* enzymatic activity) of the gene's polypeptide product. A genetic mutation in a regulatory sequence may increase, decrease, or otherwise alter the expression of sequences operably linked to the altered regulatory sequence.

Specifically, the term "genetic modification that reduces export of a branched chain amino acid from the bacterial cell" refers to a genetic modification that reduces the rate of export or quantity of export of a branched chain amino acid from the bacterial cell, as compared to the rate of export or quantity of export of the branched chain amino acid from a bacterial cell not having said modification, *e.g.,* a wild-type bacterial cell. In one embodiment, a recombinant bacterial cell having a genetic modification that reduces export of a branched chain amino acid from the bacterial cell comprises a genetic mutation in a native gene, *e.g.,* a *leuE* gene. In another embodiment, a recombinant bacterial cell having a genetic modification that reduces export of a branched chain amino acid from the bacterial cell comprises a genetic mutation in a native promoter, *e.g.,* a *leuE* promoter, which reduces or inhibits transcription of the *leuE* gene. In another embodiment, a recombinant bacterial cell having a genetic modification that reduces export of a branched chain amino acid from the bacterial cell comprises a genetic mutation leading to overexpression of a repressor of an exporter of a branched chain amino acid. In another embodiment, a recombinant bacterial cell having a genetic modification that reduces export of a branched chain amino acid from the bacterial cell comprises a genetic mutation which reduces or inhibits translation of the gene encoding the exporter, *e.g.,* the *leuE* gene.

**[2]** Moreover, the term "genetic modification that increases import of a branched chain amino acid into the bacterial cell" refers to a genetic modification that increases the uptake rate or increases the uptake quantity of a branched chain amino acid into the cytosol of the bacterial cell, as compared to the uptake rate or uptake quantity of the branched chain amino acid into the cytosol of a bacterial cell not having said modification, *e.g.,* a wild-type bacterial cell. In some embodiments, an engineered bacterial cell having a genetic modification that increases import of a branched chain amino acid into the bacterial cell refers to a bacterial cell comprising heterologous gene sequence (native or non-native) encoding one or more importer(s) (transporter(s)) of a branched chain amino acid. In some embodiments, the genetically engineered bacteria comprising genetic modification that increases import of a branched chain amino acid into the bacterial cell comprise gene sequence(s) encoding a BCAA transporter or other amino acid transporter that transports one or more BCAA(s) into the bacterial cell, for example a transporter that is capable of transporting leucine, valine, and/or isoleucine into a bacterial cell. The transporter can be any transporter that assists or allows import of a BCAA into the cell. In certain embodiments, the BCAA transporter is a leucine transporter, e.g., a high-affinity leucine transporter, e.g., LivKHMGF. In certain embodiments, the engineered bacterial cell contains gene sequence encoding one or more livK, livH, livM, livG, and livF genes. In certain embodiments, the BCAA transporter is a BCAA transporter, e.g., a low affinity BCAA transporter, e.g., BrnQ. In certain embodiments, the engineered bacterial cell contains gene sequence encoding brnQ gene. In some embodiments, the engineered bacteria comprise more than one copy of gene sequence encoding a BCAA transporter. In some embodiments, the engineered bacteria comprise gene sequence(s) encoding more than one BCAA transporter, e.g., two or more different BCAA transporters.

[3] As used herein, the term "transporter" is meant to refer to a mechanism, e.g., protein, proteins, or protein complex, for importing a molecule, e.g., amino acid, peptide (dipeptide, tri-peptide, polypeptide, etc), toxin, metabolite, substrate, as well as other biomolecules into the microorganism from the extracellular milieu.

The term "branched chain amino acid" or "BCAA," as used herein, refers to an amino acid which comprises a branched side chain. Leucine, isoleucine, and valine are naturally occurring amino acids comprising a branched side chain. However, non-naturally occurring, usual, and/or modified amino acids comprising a branched side chain are also encompassed by the term branched chain amino acid.

Conversion of a branched chain amino acid to its corresponding alpha-keto acid is the first step in branched chain amino acid catabolism and is reversible when catalyzed by a leucine dehydrogenase (leuDH) or a branched chain amino acid aminotransferase (ilvE), or irreversible when catalyzed by an amino acid oxidase (also referred as amino acid deaminase, e.g., L-AAD). As used herein, the terms "alpha-keto acid" or "α-keto acid" refers to the molecules which are produced after deamination of a branched chain amino acid, and include the naturally occurring alpha-keto acids: α-ketoisocaproic acid (KIC) (also known as 4-methyl-2-oxopentanoate), α-ketoisovaleric acid (KIV) (also known as 2-oxoisopentanoate), and α-keto-beta-methylvaleric acid (KMV) (also known as 3-methyl-2-oxopentanoate). However, non-naturally occurring, unusual, or modified alpha-keto acids are also encompassed by the term "alpha-keto acid." See Figs. 2-4.
Conversion of a branched chain alpha-keto acid to its corresponding branched acyl-CoA derivative is the second step in branched chain amino acid catabolism and is irreversible. As used herein, the term "acyl-CoA derivative" refers to the molecules which are produced after dehydrogenation of a branched chain alpha-keto acid, and include the naturally occurring acyl-CoA derivatives, propionyl-CoA and acetyl-CoA (See Fig. 2) However, non-naturally occurring, unusual, or modified acyl-CoA derivatives are also encompassed by the term "acyl-CoA derivative."
In an alternative BCAA catabolism pathway, known as the "Ehrlich pathway", a branched chain alpha-keto acid is irreversibly decarboxylated to its corresponding branched chain amino acid-derived aldehyde. This irreversible catabolic conversion of a branched chain alpha-keto acid, e.g., alpha-keto acids α-ketoisocaproic acid (KIC), α-ketoisovaleric acid (KIV), or α-keto-beta-methylvaleric acid (KMV) to its corresponding branched chain amino acid-derived aldehyde, e.g., isovaleraldehyde, isobutyraldehyde, or 2-methylbutyraldehyde, is catalyzed by ketoacid decarboxylase (KivD). As used herein, the term "branched chain amino acid-derived aldehyde" refers to the molecules which are produced after decarboxylation of a branched chain alpha-keto acid, and include the naturally occurring aldehydes isovaleraldehyde, 2-methyl butyraldehyde, and isobutyraldehyde. However, non-naturally occurring, unusual, or modified aldehydes are also encompassed by the term "aldehyde." BCAA-derived aldehydes can then be converted to alcohols (e.g., isopentanol, isobutanol, 2-methylbutanol) by an alcohol dehydrogenase, e.g., Adh2 or Ygh.D. Alternatively, BCAA-derived aldehydes can be converted to their respective carboxylic acids (e.g., isovalerate, isobutyrate, and 2-methylbutyrate) by an aldehyde dehydrogenase, e.g., PadA.

[4] As used herein, the phrase "exogenous environmental condition" or "exogenous environment signal" refers to settings, circumstances, stimuli, or biological molecules under which a promoter described herein is directly or indirectly induced. The phrase "exogenous environmental conditions" is meant to refer to the environmental conditions external to the engineered micororganism, but endogenous or native to the host subject environment. Thus, "exogenous" and "endogenous" may be used interchangeably to refer to environmental conditions in which the environmental conditions are endogenous to a mammalian body, but external or exogenous to an intact microorganism cell. In some embodiments, the exogenous environmental conditions are specific to the gut of a mammal. In some embodiments, the exogenous environmental conditions are specific to the upper gastrointestinal tract of a mammal. In some embodiments, the exogenous environmental conditions are specific to the lower gastrointestinal tract of a mammal. In some embodiments, the exogenous environmental conditions are specific to the small intestine of a mammal. In some embodiments, the exogenous environmental conditions are low-oxygen, microaerobic, or anaerobic conditions, such as the environment of the mammalian gut. In some embodiments, exogenous environmental conditions are molecules or metabolites that are specific to the mammalian gut, e.g., propionate. In some embodiments, the exogenous environmental condition is a tissue-specific or disease-specific metabolite or molecule(s). In some embodiments, the exogenous environmental condition is specific to a branched chain amino acid catabolic enzyme disease, e.g., MSUD. In some embodiments, the exogenous environmental condition is a low-pH environment. In some embodiments, the genetically engineered microorganism of the disclosure comprises a pH-dependent promoter. In some embodiments, the genetically engineered microorganism of the diclosure comprise an oxygen level-dependent promoter. In some aspects, bacteria have evolved transcription factors that are capable of sensing oxygen levels. Different signaling pathways may be triggered by different oxygen levels and occur with different kinetics. An "oxygen level-dependent promoter" or "oxygen level-dependent regulatory region" refers to a nucleic acid sequence to which one or more oxygen level-sensing transcription factors is capable of binding, wherein the binding and/or activation of the corresponding transcription factor activates downstream gene expression.

[5] Examples of oxygen level-dependent transcription factors include, but are not limited to, FNR (fumarate and nitrate reductase), ANR, and DNR. Corresponding FNR-responsive promoters, ANR (anaerobic nitrate respiration)-responsive promoters, and DNR (dissimilatory nitrate respiration regulator)-responsive promoters are known in the art (see, e.g., Castiglione et al., 2009; Eiglmeier et al., 1989; Galimand et al., 1991; Hasegawa et al., 1998; Hoeren et al., 1993; Salmon et al., 2003), and non-limiting examples are shown in Table 1.

[6] In a non-limiting example, a promoter (PfnrS) was derived from the E. coli Nissle fumarate and nitrate reductase gene S (fnrS) that is known to be highly expressed under conditions of low or no environmental oxygen (Durand and Storz, 2010; Boysen et al, 2010). The PfnrS promoter is activated under anaerobic conditions by the global transcriptional regulator FNR that is naturally found in Nissle. Under anaerobic conditions, FNR forms a dimer and binds to specific sequences in the promoters of specific genes under its control, thereby activating their expression. However, under aerobic conditions, oxygen reacts with iron-sulfur clusters in FNR dimers and converts them to an inactive form. In this way, the PfnrS inducible promoter is adopted to modulate the expression of proteins or RNA. PfnrS is used interchangeably in this application as FNRS, fnrs, FNR, P-FNRS promoter and other such related designations to indicate the promoter PfnrS.

**Table 1. Examples of transcription factors and responsive genes and regulatory regions**

| **Transcription Factor** | **Examples of responsive genes, promoters, and/or regulatory regions:** |
|---|---|
| FNR | *nirB, ydfZ, pdhR, focA, ndH, hlyE, narK, narX, narG, yfiD, tdcD* |
| ANR | *arcDABC* |
| DNR | *norb, norC* |

In some embodiments, the exogenous environmental conditions are the presence or absence of reactive oxygen species (ROS). In other embodiments, the exogenous environmental conditions are the presence or absence of reactive nitrogen species (RNS). In some embodiments, exogenous environmental conditions are biological molecules that are involved in the inflammatory response, for example, molecules present in an inflammatory disorder of the gut. In some embodiments, the exogenous environmental conditions or signals exist naturally or are naturally absent in the environment in which the recombinant bacterial cell resides. In some embodiments, the exogenous environmental conditions or signals are artificially created, for example, by the creation or removal of biological conditions and/or the administration or removal of biological molecules.

**[7]** In some embodiments, the exogenous environmental condition(s) and/or signal(s) stimulates the activity of an inducible promoter. In some embodiments, the exogenous environmental condition(s) and/or signal(s) that serves to activate the inducible promoter is not naturally present within the gut of a mammal. In some embodiments, the inducible promoter is stimulated by a molecule or metabolite that is administered in combination with the pharmaceutical composition of the disclosure, for example, tetracycline, arabinose, or any biological molecule that serves to activate an inducible promoter. In some embodiments, the exogenous environmental condition(s) and/or signal(s) is added to culture media comprising a recombinant bacterial cell of the disclosure. In some embodiments, the exogenous environmental condition that serves to activate the inducible promoter is naturally present within the gut of a mammal (for example, low oxygen or anaerobic conditions, or biological molecules involved in an inflammatory response). In some embodiments, the loss of exposure to an exogenous environmental condition (for example, *in vivo*) inhibits the activity of an inducible promoter, as the exogenous environmental condition is not present to induce the promoter (for example, an aerobic environment outside the gut). "Gut" refers to the organs, glands, tracts, and systems that are responsible for the transfer and digestion of food, absorption of nutrients, and excretion of waste. In humans, the gut comprises the gastrointestinal (GI) tract, which starts at the mouth and ends at the anus, and additionally comprises the esophagus, stomach, small intestine, and large intestine. The gut also comprises accessory organs and glands, such as the spleen, liver, gallbladder, and pancreas. The upper gastrointestinal tract comprises the esophagus, stomach, and duodenum of the small intestine. The lower gastrointestinal tract comprises the remainder of the small intestine, *i.e.,* the jejunum and ileum, and all of the large intestine, *i.e.,* the cecum, colon, rectum, and anal canal. Bacteria can be found throughout the gut, *e.g.,* in the gastrointestinal tract, and particularly in the intestines.

"Microorganism" refers to an organism or microbe of microscopic, submicroscopic, or ultramicroscopic size that typically consists of a single cell. Examples of microrganisms include bacteria, viruses, parasites, fungi, certain algae, and protozoa. In some aspects, the microorganism is engineered ("engineered microorganism") to produce one or more therpauetic molecules, e.g., lysosomal enzyme(s). In certain embodiments, the engineered microorganism is an engineered bacterium. In certain embodiments, the engineered microorganism is an engineered virus.

"Non-pathogenic bacteria" refer to bacteria that are not capable of causing disease or harmful responses in a host. In some embodiments, non-pathogenic bacteria are Gram-negative bacteria. In some embodiments, non-pathogenic bacteria are Gram-positive bacteria. In some embodiments, non-pathogenic bacteria do not contain lipopolysaccharides (LPS). In some embodiments, non-pathogenic bacteria are commensal bacteria. Examples of non-pathogenic bacteria include, but are not limited to certain strains belonging to the genus *Bacillus, Bacteroides, Bifidobacterium, Brevibacteria, Clostridium, Enterococcus, Escherichia coli, Lactobacillus, Lactococcus,* Saccharomyces, and *Staphylococcus, e.g., Bacillus coagulans, Bacillus subtilis, Bacteroides fragilis, Bacteroides subtilis, Bacteroides thetaiotaomicron, Bifidobacterium bifidum, Bifidobacterium infantis, Bifidobacterium lactis, Bifidobacterium longum, Clostridium butyricum, Enterococcus faecium, Escherichia coli, Escherichia coli Nissle, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus,Lactococcus lactis* and *Saccharomyces boulardii* (Sonnenborn et al., 2009; Dinleyici et al., 2014; U.S. Patent No. 6,835,376; U.S. Patent No. 6,203,797; U.S. Patent No. 5,589,168; U.S. Patent No. 7,731,976). Non-pathogenic bacteria also include commensal bacteria, which are present in the indigenous microbiota of the gut. In one embodiment, the disclosure further includes non-pathogenic *Saccharomyces,* such as *Saccharomyces boulardii.* Naturally pathogenic bacteria may be genetically engineered to reduce or eliminate pathogenicity.

"Probiotic" is used to refer to live, non-pathogenic microorganisms, e.g., bacteria, which can confer health benefits to a host organism that contains an appropriate amount of the microorganism. In some embodiments, the host organism is a mammal. In some embodiments, the host organism is a human. In some embodiments, the probiotic bacteria are Gram-negative bacteria. In some embodiments, the probiotic bacteria are Gram-positive bacteria. Some species, strains, and/or subtypes of non-pathogenic bacteria are currently recognized as probiotic bacteria. Examples of probiotic bacteria include, but are not limited to, certain strains belonging to the genus *Bifidobacteria, Escherichia Coli, Lactobacillus,* and *Saccharomyces e.g., Bifidobacterium bifidum, Enterococcus faecium, Escherichia coli* strain Nissle, *Lactobacillus acidophilus, Lactobacillus bulgaricus, Lactobacillus paracasei,* and *Lactobacillus plantarum,* and *Saccharomyces boulardii* (Dinleyici et al., 2014; U.S. Patent No. 5,589,168; U.S. Patent No. 6,203,797; U.S. Patent 6,835,376). The probiotic may be a variant or a mutant strain of bacterium (Arthur *et al.,* 2012; Cuevas-Ramos *et al.,* 2010; Olier *et al.,* 2012; Nougayrede *et al.,* 2006). Non-pathogenic bacteria may be genetically engineered to enhance or improve desired biological properties, e.g., survivability. Non-pathogenic bacteria may be genetically engineered to provide probiotic properties. Probiotic bacteria may be genetically engineered to enhance or improve probiotic properties.

As used herein, the term "auxotroph" or "auxotrophic" refers to an organism that requires a specific factor, *e.g.,* an amino acid, a sugar, or other nutrient) to support its growth. An "auxotrophic modification" is a genetic modification that causes the organism to die in the absence of an exogenously added nutrient essential for survival or growth because it is unable to produce said nutrient. As used herein, the term "essential gene" refers to a gene which is necessary to for cell growth and/or survival. Essential genes are described in more detail *infra* and include, but are not limited to, DNA synthesis genes (such as *thyA*), cell wall synthesis genes (such as *dapA*), and amino acid genes (such as *serA* and *metA*).

**[8]** As used herein, the terms "modulate" and "treat" and their cognates refer to an amelioration of a disease, disorder, and/or condition, or at least one discernible symptom thereof. In another embodiment, "modulate" and "treat" refer to an amelioration of at least one measurable physical parameter, not necessarily discernible by the patient. In another embodiment, "modulate" and "treat" refer to inhibiting the progression of a disease, disorder, and/or condition, either physically (e.g., stabilization of a discernible symptom), physiologically (e.g., stabilization of a physical parameter), or both. In another embodiment, "modulate" and "treat" refer to slowing the progression or reversing the progression of a disease, disorder, and/or condition. As used herein, "prevent" and its cognates refer to delaying the onset or reducing the risk of acquiring a given disease, disorder and/or condition or a symptom associated with such disease, disorder, and/or condition.

Those in need of treatment may include individuals already having a particular medical disease, as well as those at risk of having, or who may ultimately acquire the disease. The need for treatment is assessed, for example, by the presence of one or more risk factors associated with the development of a disease, the presence or progression of a disease, or likely receptiveness to treatment of a subject having the disease. Diseases associated with the catabolism of a branched chain amino acid, *e.g.,* MSUD, may be caused by inborn genetic mutations for which there are no known cures. Diseases can also be secondary to other conditions, e.g., liver diseases. Treating diseases involving the catabolism of a branched chain amino acid, such as MSUD, may encompass reducing normal levels of branched chain amino acids, reducing excess levels of branched chain amino acids, or eliminating branched chain amino acids, *e.g.,* leucine, and does not necessarily encompass the elimination of the underlying disease.

As used herein, the term "catabolism" refers to the breakdown of a molecule into a smaller unit. As used herein, the term "branched chain amino acid catabolism" refers to the conversion of a branched chain amino acid, such as leucine, isoleucine, or valine, into a corresponding metabolite, for example a corresponding alpha keto acid, acyl-CoA derivative, aldehyde, alcohol, or other metabolite, such as any of the BCAA metabolites disclosed herein; or the conversion of a branched chain alpha keto acid into its corresponding acyl-CoA derivative, aldehydes, alcohols or other metabolites, such as any of the BCAA metabolites disclosed herein. The "branched chain amino acid catabolism" refers to both native and non-native conversion of a branched chain amino acid, such as leucine, isoleucine, or valine, into a corresponding metabolite. Thus, the term additionally covers catabolism of BCAA that may not occur in nature and is artificially induced as a result of genetic engineering. In one embodiment, "abnormal catabolism" refers to a decrease in the rate or the level of conversion of a branched chain amino acid or its corresponding alpha-keto acid to a corresponding metabolite, leading to the accumulation of the branched chain amino acid, accumulation of the branched chain alpha-keto acid, and/or accumulation of a BCAA metabolite that is toxic or that accumulates to a toxic level in a subject (e.g., see Figure 1). In one embodiment, the branched chain amino acid,branched chain alpha-keto acid, or other metabolite thereof, accumulates to a toxic level in the blood or the brain of a subject, leading to the development of a disease or disorder associated with the abnormal catabolism of the branched chain amino acid in the subject. In one embodiment, "abnormal leucine catabolism" refers to a level of greater than 4 mg/dL of leucine in the plasma of a subject. In another embodiment, "normal leucine catabolism" refers to a level of less than 4 mg/dL of leucine in the plasma of a subject.

As used herein, the term "disorder involving the abnormal catabolism of a branched amino acid" or "disease involving the abnormal catabolism of a branched amino acid" or "branched chain amino acid disease" or "disease associated with excess branched chain amino acid" refers to a disease or disorder wherein the catabolism of a branched chain amino acid or a branched chain alpha-keto acid is abnormal. Such diseases are genetic disorders that result from deficiency in at least one of the enzymes required to catabolize a branched chain amino acid, e.g., lecine, isoleucine, or valine. As a result, individuals suffering from branched chain amino acid disease have accumulated branched chain amino acids in their cells and tissues. Examples of branched chain amino acid diseases include, but are not limited to, MSUD, isovaleric acidemia, 3-MCC deficiency, 3-methylglutaconyl-CoA hydralase deficiency, HMG-CoA lysate deficiency, Acetyl CoA carboxylase deficiency, malonylCoA decarboxylase deficiency, short branched chain acyl-CoA dehydrogenase, 2-methyl-3-hydroxybutyric acidemia, beta-ketothiolase deficiency, isobutyl-coA dehydrogenase deficiency, HIBCH deficiency, 3-hydroxyisobutyric aciduria, proprionic acidemis, methylmalonic acidemia, as well as those diseases resulting from mTor activation, including but not limited to cancer, obesity, type 2 diabetes, neurodegeneration, autism, Alzheimer's disease, Lymphangioleiomyomatosis (LAM), transplant rejection, glycogen storage disease, obesity, tuberous sclerosis, hypertension, cardiovascular disease, hypothalamic activation, musculoskeletal disease, Parkinson's disease, Huntington's disease, psoriasis, rheumatoid arthritis, lupus, multiple sclerosis, Leigh's syndrome, and Friedrich's ataxia. In one embodiment, a "disease or disorder involving the catabolism of a branched chain amino acid" is a metabolic disease or disorder involving the abnormal catabolism of a branched chain amino acid. In another embodiment, a "disease or disorder involving the catabolism of a branched chain amino acid" is a disease or disorder caused by the activation of mTor. In one embodiment, the activation of mTor is abnormal.

In one embodiment, "abnormal catabolism" refers to a decrease in the rate or the level of conversion of the branched chain amino acid or its alpha-keto acid counterpart, leading to the accumulation of the branched chain amino acid or alpha-keto acid in a subject. In one embodiment, accumulation of the branched chain amino acid in the blood or the brain of a subject becomes toxic and leads to the development of a disease or disorder associated with the abnormal catabolism of the branched chain amino acid in the subject.

As used herein, the term "disease caused by the activation of mTor" or "disorder caused by the activation of mTor" refers to a disease or a disorder wherein the levels of branched chain amino acid may be normal, and wherein the branched chain amino acid causes the activation of mTor at a level higher than the normal level of mTor activity. In another embodiment, the subject having a disorder caused by the activation of mTor may have higher levels of a branched chain amino acid than normal. Diseases caused by the activation of mTor are known in the art. See, for example, Laplante and Sabatini, Cell, 149(2):74-293, 2012. As used herein, the term "disease caused by the activation of mTor" includes cancer, obesity, type 2 diabetes, neurodegeneration, autism, Alzheimer's disease, Lymphangioleiomyomatosis (LAM), transplant rejection, glycogen storage disease, obesity, tuberous sclerosis, hypertension, cardiovascular disease, hypothalamic activation, musculoskeletal disease, Parkinson's disease, Huntington's disease, psoriasis, rheumatoid arthritis, lupus, multiple sclerosis, Leigh's syndrome, and Friedrich's ataxia. In one embodiment, the subject has normal levels of a branched chain amino acid, such as leucine, before administration of the engineered bacteria of the present disclosure. In another embodiment, the subject has decreased levels of the branched chain amino acid after the administration of the engineered bacteria of the present disclosure, thereby decreasing the levels of mTor or the activity of mTor, thereby treating the disorder in the subject. In one embodiment, the pharmaceutical composition disclosed herein decreases the activity of mTor by at least about 2-fold, 3-fold, 4-fold, or 5-fold in the subject.

As used herein, the term "anabolism" refers the conversion of a branched chain alpha-keto acid or an acyl-CoA derivative or other metabolite into its corresponding branched chain amino acid, such as leucine, isoleucine, or valine or alpha-keto acid, respectively.

As used herein a "pharmaceutical composition" refers to a preparation of genetically engineered microorganism of the disclosure, e.g., genetically engineered bacteria or virus, with other components such as a physiologically suitable carrier and/or excipient.

The phrases "physiologically acceptable carrier" and "pharmaceutically acceptable carrier" which may be used interchangeably refer to a carrier or a diluent that does not cause significant irritation to an organism and does not abrogate the biological activity and properties of the administered bacterial or viral compound. An adjuvant is included under these phrases.

The term "excipient" refers to an inert substance added to a pharmaceutical composition to further facilitate administration of an active ingredient. Examples include, but are not limited to, calcium bicarbonate, sodium bicarbonate calcium phosphate, various sugars and types of starch, cellulose derivatives, gelatin, vegetable oils, polyethylene glycols, and surfactants, including, for example, polysorbate 20.

The terms "therapeutically effective dose" and "therapeutically effective amount" are used to refer to an amount of a compound that results in prevention, delay of onset of symptoms, or amelioration of symptoms of a condition, e.g., lysosomal storage disease (LSD). A therapeutically effective amount may, for example, be sufficient to treat, prevent, reduce the severity, delay the onset, and/or reduce the risk of occurrence of one or more symptoms of a disorder associated with lysosomal storage disease. A therapeutically effective amount, as well as a therapeutically effective frequency of administration, can be determined by methods known in the art and discussed below.

As used herein, the term "bacteriostatic" or "cytostatic" refers to a molecule or protein which is capable of arresting, retarding, or inhibiting the growth, division, multiplication or replication of recombinant bacterial cell of the disclosure.

As used herein, the term "bactericidal" refers to a molecule or protein which is capable of killing the recombinant bacterial cell of the disclosure.

As used herein, the term "toxin" refers to a protein, enzyme, or polypeptide fragment thereof, or other molecule which is capable of arresting, retarding, or inhibiting the growth, division, multiplication or replication of the recombinant bacterial cell of the disclosure, or which is capable of killing the recombinant bacterial cell of the disclosure. The term "toxin" is intended to include bacteriostatic proteins and bactericidal proteins. The term "toxin" is intended to include, but not limited to, lytic proteins, bacteriocins (e.g., microcins and colicins), gyrase inhibitors, polymerase inhibitors, transcription inhibitors, translation inhibitors, DNases, and RNases. The term "anti-toxin" or "antitoxin," as used herein, refers to a protein or enzyme which is capable of inhibiting the activity of a toxin. The term anti-toxin is intended to include, but not limited to, immunity modulators, and inhibitors of toxin expression. Examples of toxins and antitoxins are known in the art and described in more detail *infra.*

**[9]** As used herein, the term "branched chain amino acid catabolic or catabolism enzyme" or "BCAA catabolic or catabolism enzyme" or "branched chain or BCAA amino acid metabolic enzyme" refers to any enzyme that is capable of metabolizing a branched chain amino acid or capable of reducing accumulated branched chain amino acid or that can lessen, ameliorate, or prevent one or more branched chain amino acid diseases or disease symptoms. Examples of branched chain amino acid metabolic enzymes include, but are not limited to, leucine dehydrogenase (e.g., LeuDH), branched chain amino acid aminotransferase (e.g., IlvE), branched chain α-ketoacid dehydrogenase (e.g., KivD), L-Amino acid deaminase (e.g., L-AAD), alcohol dehydrogenase (e.g., Adh2, YqhD)), and aldehyde dehydrogenase (e.g., PadA), and any other enzymes that catabolizes BCAA. Functional deficiencies in these proteins result in the accumulation of BCAA or its corresponding α-keto acid in cells and tissues. BCAA metabolic enzymes of the present disclosure include both wild-type or modified BCAA metabolic enzymes and can be produced using recombinant and synthetic methods or purified from nature sources. BCAA metabolic enzymes include full-length polypeptides and functional fragments thereof, as well as homologs and variants thereof. BCAA metabolic enzymes include polypeptides that have been modified from the wild-type sequence, including, for example, polypeptides having one or more amino acid deletions, insertions, and/or substitutions and may include, for example, fusion polypeptides and polypeptides having additional sequence, e.g., regulatory peptide sequence, linker peptide sequence, and other peptide sequence.

**[10]** As used herein, "payload" refers to one or more molecules of interest to be produced by a genetically engineered microorganism, such as a bacteria or a virus. In some embodiments, the payload is a therapeutic payload, e.g., a branched chain amino acid catabolic enzyme or a BCAA transporter polypeptide. In some embodiments, the payload is a regulatory molecule, e.g., a transcriptional regulator such as FNR. In some embodiments, the payload comprises a regulatory element, such as a promoter or a repressor. In some embodiments, the payload comprises an inducible promoter, such as from FNRS. In some embodiments the payload comprises a repressor element, such as a kill switch. In some embodiments the payload comprises a an antibiotic resistance gene or genes. In some embodiments, the payload is encoded by a gene, multiple genes, gene cassette, or an operon. In alternate embodiments, the payload is produced by a biosynthetic or biochemical pathway, wherein the biosynthetic or biochemical pathway may optionally be endogenous to the microorganism. In alternate embodiments, the payload is produced by a biosynthetic or biochemical pathway, wherein the biosynthetic or biochemical pathway is not endogenous to the microorganism. In some embodiments, the genetically engineered microorganism comprises two or more payloads.

**[11]** As used herein, the term "conventional branched chain amino acid or BCAA disease treatment" or "conventional branched chain amino acid or BCAA disease therapy" refers to treatment or therapy that is currently accepted, considered current standard of care, and/or used by most healthcare professionals for treating a disease or disorder associated with BCAA. It is different from alternative or complementary therapies, which are not as widely used.

**[12]** As used herein, the term "polypeptide" includes "polypeptide" as well as "polypeptides," and refers to a molecule composed of amino acid monomers linearly linked by amide bonds (i.e., peptide bonds). The term "polypeptide" refers to any chain or chains of two or more amino acids, and does not refer to a specific length of the product. Thus, "peptides," "dipeptides," "tripeptides, "oligopeptides," "protein," "amino acid chain," or any other term used to refer to a chain or chains of two or more amino acids, are included within the definition of "polypeptide," and the term "polypeptide" may be used instead of, or interchangeably with any of these terms. The term "polypeptide" is also intended to refer to the products of post-expression modifications of the polypeptide, including but not limited to glycosylation, acetylation, phosphorylation, amidation, derivatization, proteolytic cleavage, or modification by non-naturally occurring amino acids. A polypeptide may be derived from a natural biological source or produced by recombinant technology. In other embodiments, the polypeptide is produced by the genetically engineered bacteria or virus of the current invention. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides, which do not possess a defined three-dimensional structure, but rather can adopt a large number of different conformations, are referred to as unfolded. The term "peptide" or "polypeptide" may refer to an amino acid sequence that corresponds to a protein or a portion of a protein or may refer to an amino acid sequence that corresponds with non-protein sequence, e.g., a sequence selected from a regulatory peptide sequence, leader peptide sequence, signal peptide sequence, linker peptide sequence, and other peptide sequence.

**[13]** An "isolated" polypeptide or a fragment, variant, or derivative thereof refers to a polypeptide that is not in its natural milieu. No particular level of purification is required. Recombinantly produced polypeptides and proteins expressed in host cells, including but not limited to bacterial or mammalian cells, are considered isolated for purposed of the invention, as are native or recombinant polypeptides which have been separated, fractionated, or partially or substantially purified by any suitable technique. Recombinant peptides, polypeptides or proteins refer to peptides, polypeptides or proteins produced by recombinant DNA techniques, i.e. produced from cells, microbial or mammalian, transformed by an exogenous recombinant DNA expression construct encoding the polypeptide. Proteins or peptides expressed in most bacterial cultures will typically be free of glycan. Fragments, derivatives, analogs or variants of the foregoing polypeptides, and any combination thereof are also included as polypeptides. The terms "fragment," "variant," "derivative" and "analog" include polypeptides having an amino acid sequence sufficiently similar to the amino acid sequence of the original peptide and include any polypeptides, which retain at least one or more properties of the corresponding original polypeptide. Fragments of polypeptides of the present invention include proteolytic fragments, as well as deletion fragments. Fragments also include specific antibody or bioactive fragments or immunologically active fragments derived from any polypeptides described herein. Variants may occur naturally or be non-naturally occurring. Non-naturally occurring variants may be produced using mutagenesis methods known in the art. Variant polypeptides may comprise conservative or non-conservative amino acid substitutions, deletions or additions.

**[14]** Polypeptides also include fusion proteins. As used herein, the term "variant" includes a fusion protein, which comprises a sequence of the original peptide or sufficiently similar to the original peptide. As used herein, the term "fusion protein" refers to a chimeric protein comprising amino acid sequences of two or more different proteins. Typically, fusion proteins result from well known in vitro recombination techniques. Fusion proteins may have a similar structural function (but not necessarily to the same extent), and/or similar regulatory function (but not necessarily to the same extent), and/or similar biochemical function (but not necessarily to the same extent) and/or immunological activity (but not necessarily to the same extent) as the individual original proteins which are the components of the fusion proteins. "Derivatives" include but are not limited to peptides, which contain one or more naturally occurring amino acid derivatives of the twenty standard amino acids. "Similarity" between two peptides is determined by comparing the amino acid sequence of one peptide to the sequence of a second peptide. An amino acid of one peptide is similar to the corresponding amino acid of a second peptide if it is identical or a conservative amino acid substitution. Conservative substitutions include those described in Dayhoff, M. O., ed., The Atlas of Protein Sequence and Structure 5, National Biomedical Research Foundation, Washington, D.C. (1978), and in Argos, EMBO J. 8 (1989), 779-785. For example, amino acids belonging to one of the following groups represent conservative changes or substitutions: -Ala, Pro, Gly, Gln, Asn, Ser, Thr; -Cys, Ser, Tyr, Thr; -Val, Ile, Leu, Met, Ala, Phe; -Lys, Arg, His; -Phe, Tyr, Trp, His; and -Asp, Glu.

[15] As used herein, the term "sufficiently similar" means a first amino acid sequence that contains a sufficient or minimum number of identical or equivalent amino acid residues relative to a second amino acid sequence such that the first and second amino acid sequences have a common structural domain and/or common functional activity. For example, amino acid sequences that comprise a common structural domain that is at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100%, identical are defined herein as sufficiently similar. Preferably, variants will be sufficiently similar to the amino acid sequence of the peptides of the invention. Such variants generally retain the functional activity of the peptides of the present invention. Variants include peptides that differ in amino acid sequence from the native and wt peptide, respectively, by way of one or more amino acid deletion(s), addition(s), and/or substitution(s). These may be naturally occurring variants as well as artificially designed ones.

[16] As used herein the term "linker", "linker peptide" or "peptide linkers" or "linker" refers to synthetic or non-native or non-naturally-occurring amino acid sequences that connect or link two polypeptide sequences, e.g., that link two polypeptide domains. As used herein the term "synthetic" refers to amino acid sequences that are not naturally occurring. Exemplary linkers are described herein. Additional exemplary linkers are provided in US 20140079701.

[17] As used herein the term "codon-optimized" refers to the modification of codons in the gene or coding regions of a nucleic acid molecule to reflect the typical codon usage of the host organism without altering the polypeptide encoded by the nucleic acid molecule. Such optimization includes replacing at least one, or more than one, or a significant number, of codons with one or more codons that are more frequently used in the genes of the host organism. A "codon-optimized sequence" refers to a sequence, which was modified from an existing coding sequence, or designed, for example, to improve translation in an expression host cell or organism of a transcript RNA molecule transcribed from the coding sequence, or to improve transcription of a coding sequence. Codon optimization includes, but is not limited to, processes including selecting codons for the coding sequence to suit the codon preference of the expression host organism. Many organisms display a bias or preference for use of particular codons to code for insertion of a particular amino acid in a growing polypeptide chain. Codon preference or codon bias, differences in codon usage between organisms, is allowed by the degeneracy of the genetic code, and is well documented among many organisms. Codon bias often correlates with the efficiency of translation of messenger RNA (mRNA), which is in turn believed to be dependent on, inter alia, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization.

[18] As used herein, the terms "secretion system" or "secretion protein" refers to a native or non-native secretion mechanism capable of secreting or exporting a biomolecule, e.g., polypeptide from the microbial, e.g., bacterial cytoplasm. The secretion system may comprise a single protein or may comprise two or more proteins assembled in a complex e.g.,HlyBD. Non-limiting examples of secretion systems for gram negative bacteria include the modified type III flagellar, type I (e.g., hemolysin secretion system), type II, type IV, type V, type VI, and type VII secretion systems, resistance-nodulation-division (RND) multi-drug efflux pumps, various single membrane secretion systems. Non-liming examples of secretion systems for gram positive bacteria include Sec and TAT secretion systems. In some embodiments, the polypeptide to be secreted include a "secretion tag" of either RNA or peptide origin to direct the polypeptide to specific secretion systems. In some embodiments, the secretion system is able to remove this tag before secreting the polyppetide from the engineered bacteria. For example, in Type V auto-secretion-mediated secretion the N-terminal peptide secretion tag is removed upon translocation of the "passenger" peptide from the cytoplasm into the periplasmic compartment by the native Sec system. Further, once the auto-secretor is translocated across the outer membrane the C-terminal secretion tag can be removed by either an autocatalytic or protease-catalyzed e.g., OmpT cleavage thereby releasing the lysosomal enzyme(s) into the extracellular milieu. In some embodiments, the secretion system involves the generation of a "leaky" or de-stabilized outer membrane, which may be accomplished by deleting or mutagenizing genes responsible for tethering the outer membrane to the rigid peptidoglycan skeleton, including for example, lpp, ompC, ompA, ompF, tolA, tolB, pal, degS, degP, and nlpl. Lpp functions as the primary 'staple' of the bacterial cell wall to the peptidoglycan. TolA-PAL and OmpA complexes function similarly to Lpp and are other deletion targets to generate a leaky phenotype. Additionally, leaky phenotypes have been observed when periplasmic proteases, such as *degS, degP* or *nlpI*, are deactivated. Thus, in some embodiments, the engineered bacteria have one or more deleted or mutated membrane genes, e.g., selected from lpp, ompA, ompA, ompF, tolA, tolB, and pal genes. In some embodiments, the engineered bacteria have one or more deleted or mutated periplasmic protease genes, e.g., selected from degS, degP, and nlpl. In some embodiments, the engineered bacteria have one or more deleted or mutated gene(s), selected from lpp, ompA, ompA, ompF, tolA, tolB, pal, degS, degP, and nlpl genes.

[19] The articles "a" and "an," as used herein, should be understood to mean "at least one," unless clearly indicated to the contrary.

[20] The phrase "and/or," when used between elements in a list, is intended to mean either (1) that only a single listed element is present, or (2) that more than one element of the list is present. For example, "A, B, and/or C" indicates that the selection may be A alone; B alone; C alone; A and B; A and C; B and C; or A, B, and C. The phrase "and/or" may be used interchangeably with "at least one of' or "one or more of' the elements in a list.

[21] Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

### Recombinant Bacteria

**[22]** The genetically engineered microorganisms, or programmed microorganisms, such as genetically engineered bacteria of the disclosure are capable of producing one or more enzymes for metabolizing a branched amino acid and/or a metabolite thereof. In some aspects, the disclosure provides a bacterial cell that comprises one or more heterologous gene sequence(s) encoding a branched chain amino acid catabolic enzyme or other protein that results in a decrease in BCAA levels.

[23] In certain embodiments, the genetically engineered bacteria are obligate anaerobic bacteria. In certain embodiments, the genetically engineered bacteria are facultative anaerobic bacteria. In certain embodiments, the genetically engineered bacteria are aerobic bacteria. In some embodiments, the genetically engineered bacteria are Gram-positive bacteria. In some embodiments, the genetically engineered bacteria are Gram-positive bacteria and lack LPS. In some embodiments, the genetically engineered bacteria are Gram-negative bacteria. In some embodiments, the genetically engineered bacteria are Gram-positive and obligate anaerobic bacteria. In some embodiments, the genetically engineered bacteria are Gram-positive and facultative anaerobic bacteria. In some embodiments, the genetically engineered bacteria are non-pathogenic bacteria. In some embodiments, the genetically engineered bacteria are commensal bacteria. In some embodiments, the genetically engineered bacteria are probiotic bacteria. In some embodiments, the genetically engineered bacteria are naturally pathogenic bacteria that are modified or mutated to reduce or eliminate pathogenicity. Exemplary bacteria include, but are not limited to, Bacillus, Bacteroides, Bifidobacterium, Brevibacteria, Caulobacter, Clostridium, Enterococcus, Escherichia coli, Lactobacillus, Lactococcus, Listeria, Mycobacterium, Saccharomyces, Salmonella, Staphylococcus, Streptococcus, Vibrio, Bacillus coagulans, Bacillus subtilis, Bacteroides fragilis, Bacteroides subtilis, Bacteroides thetaiotaomicron, Bifidobacterium adolescentis, Bifidobacterium bifidum, Bifidobacterium breve UCC2003, Bifidobacterium infantis, Bifidobacterium lactis, Bifidobacterium longum, Clostridium acetobutylicum, Clostridium butyricum, Clostridium butyricum M-55, Clostridium cochlearum, Clostridium felsineum, Clostridium histolyticum, Clostridium multifermentans, Clostridium novyi-NT, Clostridium paraputrificum, Clostridium pasteureanum, Clostridium pectinovorum, Clostridium perfringens, Clostridium roseum, Clostridium sporogenes, Clostridium tertium, Clostridium tetani, Clostridium tyrobutyricum, Corynebacterium parvum, Escherichia coli MG1655, Escherichia coli Nissle 1917, Listeria monocytogenes, Mycobacterium bovis, Salmonella choleraesuis, Salmonella typhimurium, and Vibrio cholera. In certain embodiments, the genetically engineered bacteria are selected from the group consisting of Enterococcus faecium, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, and Saccharomyces boulardii. In certain embodiments, the the genetically engineered bacteria are selected from *Bacteroides fragilis, Bacteroides thetaiotaomicron*, *Bacteroides subtilis, Bifidobacterium bifidum, Bifidobacterium infantis, Bifidobacterium lactis, Clostridium butyricum*, *Escherichia coli, Escherichia coli Nissle, Lactobacillus acidophilus, Lactobacillus plantarum, Lactobacillus reuteri,* and *Lactococcus lactis* bacterial cell. In one embodiment, the bacterial cell is a *Bacteroides fragilis* bacterial cell. In one embodiment, the bacterial cell is a *Bacteroides thetaiotaomicron* bacterial cell. In one embodiment, the bacterial cell is a *Bacteroides subtilis* bacterial cell. In one embodiment, the bacterial cell is a *Bifidobacterium bifidum* bacterial cell. In one embodiment, the bacterial cell is a *Bifidobacterium infantis* bacterial cell. In one embodiment, the bacterial cell is a *Bifidobacterium lactis* bacterial cell. In one embodiment, the bacterial cell is a *Clostridium butyricum* bacterial cell. In one embodiment, the bacterial cell is an *Escherichia coli* bacterial cell. In one embodiment, the bacterial cell is a *Lactobacillus acidophilus* bacterial cell. In one embodiment, the bacterial cell is a *Lactobacillus plantarum* bacterial cell. In one embodiment, the bacterial cell is a *Lactobacillus reuteri* bacterial cell. In one embodiment, the bacterial cell is a *Lactococcus lactis* bacterial cell.

**[24]** In some embodiments, the genetically engineered bacteria are Escherichia coli strain Nissle 1917 (E. coli Nissle), a Gram-negative bacterium of the Enterobacteriaceae family that has evolved into one of the best characterized probiotics (Ukena et al., 2007). The strain is characterized by its complete harmlessness (Schultz, 2008), and has GRAS (generally recognized as safe) status (Reister et al., 2014, emphasis added). Genomic sequencing confirmed that E. coli Nissle lacks prominent virulence factors (e.g., E. coli α-hemolysin, P-fimbrial adhesins) (Schultz, 2008). In addition, it has been shown that E. coli Nissle does not carry pathogenic adhesion factors, does not produce any enterotoxins or cytotoxins, is not invasive, and not uropathogenic (Sonnenborn et al., 2009). As early as in 1917, E. coli Nissle was packaged into medicinal capsules, called Mutaflor, for therapeutic use. E. coli Nissle has since been used to treat ulcerative colitis in humans in vivo (Rembacken et al., 1999), to treat inflammatory bowel disease, Crohn's disease, and pouchitis in humans in vivo (Schultz, 2008), and to inhibit enteroinvasive Salmonella, Legionella, Yersinia, and Shigella in vitro (Altenhoefer et al., 2004). It is commonly accepted that E. coli Nissle's therapeutic efficacy and safety have convincingly been proven (Ukena et al., 2007).

One of ordinary skill in the art would appreciate that the genetic modifications disclosed herein may be adapted for other species, strains, and subtypes of bacteria. Furthermore, genes from one or more different species can be introduced into one another, *e.g.,* the *kivD* gene from *Lactococcus lactis* (SEQ ID NO: 1)can be expressed in *Escherichia coli.* In one embodiment, the recombinant bacterial cell does not colonize the subject having the disorder. Unmodified E. coli Nissle and the genetically engineered bacteria of the invention may be destroyed, e.g., by defense factors in the gut or blood serum (Sonnenborn et al., 2009). In some embodiments, the residence time is calculated for a human subject. In some embodiments, residence time in vivo is calculated for the genetically engineered bacteria of the invention.

In some embodiments, the bacterial cell is a genetically engineered bacterial cell. In another embodiment, the bacterial cell is a recombinant bacterial cell. In some embodiments, the disclosure comprises a colony of bacterial cells disclosed herein.

In another aspect, the disclosure provides a recombinant bacterial culture which comprises bacterial cells disclosed herein. In one aspect, the disclosure provides a recombinant bacterial culture which reduces levels of a branched chain amino acid, *e.g.,* leucine, in the media of the culture. In one embodiment, the levels of the branched chain amino acid, *e.g.,* leucine, are reduced by about 50%, about 75%, or about 100% in the media of the cell culture. In another embodiment, the levels of the branched chain amino acid, *e.g.,* leucine, are reduced by about two-fold, three-fold, four-fold, five-fold, six-fold, seven-fold, eight-fold, nine-fold, or ten-fold in the media of the cell culture. In one embodiment, the levels of the branched chain amino acid, *e.g.,* leucine, are reduced below the limit of detection in the media of the cell culture.

In some embodiments of the above described genetically engineered bacteria, the gene encoding a branched chain amino acid catabolism enzyme is present on a plasmid in the bacterium and operatively linked on the plasmid to a promoter that is induced under low-oxygen or anaerobic conditions, such as any of the promoters disclosed herein. In other embodiments, the gene encoding a branched chain amino acid catabolism enzyme is present in the bacterial chromosome and is operatively linked in the chromosome to the promoter that is induced under low-oxygen or anaerobic conditions, such as any of the promoters disclosed herein. In some embodiments of the above described genetically engineered bacteria, the gene encoding a branched chain amino acid catabolic enzyme is present on a plasmid in the bacterium and operatively linked on the plasmid to the promoter that is induced under inflammatory conditions, such as any of the promoters disclosed herein. In other embodiments, the gene encoding a branched chain amino acid catabolic enzyme is present in the bacterial chromosome and is operatively linked in the chromosome to the promoter that is induced under inflammatory conditions, such as any of the promoters disclosed herein.

In some embodiments, the genetically engineered bacteria comprising gene sequence encoding a branched chain amino acid catabolic enzyme is an auxotroph. In one embodiment, the genetically engineered bacteria is an auxotroph selected from a *cysE, glnA, ilvD, leuB, lysA, serA, metA, glyA, hisB, ilvA, pheA, proA, thrC, trpC, tyrA, thyA, uraA, dapA, dapB, dapD, dapE, dapF, flhD, metB, metC, proAB, and thi1* auxotroph. In some embodiments, the engineered bacteria have more than one auxotrophy, for example, they may be a *ΔthyA* and Δ*dapA auxotroph.* ,In some embodiments, the genetically engineered bacteria comprising gene sequence encoding a branched chain amino acid catabolic enzyme lacks functional *ilvC* gene sequence, e.g., is a *ilvC* auxotroph. IlvC encodes keto acid reductoisomerase, which enzyme is required for BCAA synthesis. Knock out of *ilvC* creates an auxotroph and requires the bacterial cell to import isoleucin and valine to survive.

In some embodiments, the genetically engineered bacteria comprising gene sequence encoding a branched chain amino acid catabolism enzyme further comprise gene sequence(s) encoding a BCAA transporter or other amino acid transporter that transports one or more BCAA(s) into the bacterial cell, for example a transporter that is capable of transporting leucine, valine, and/or isoleucine into a bacterial cell. In certain embodiments, the BCAA transporter is a leucine transporter, e.g., a high-affinity leucine transporter. In certain embodiments, the bacterial cell contains gene sequence encoding livK, livH, livM, livG, and livF genes. In certain embodiments, the BCAA transporter is a BCAA transporter, e.g., a low affinity BCAA transporter. In certain embodiments, the the bacterial cell contains gene sequence encoding brnQ gene.

In some embodiments, the genetically engineered bacteria comprising gene sequence encoding a branched chain amino acid catabolism enzyme further comprise gene sequence(s) encoding a secretion protein or protein complex for secreting a biomolecule, such as any of the secretion systems disclosed herein.

In some embodiments, the genetically engineered bacteria comprising gene sequence encoding a branched chain amino acid catabolism enzyme further comprise gene sequence(s) encoding one or more antibiotic gene(s), such as any of the antibiotic genes disclosed herein.

In some embodiments, the genetically engineered bacteria comprising a branched chain amino acid catabolism enzyme further comprise a kill-switch circuit, such as any of the kill-switch circuits provided herein. For example, in some embodiments, the genetically engineered bacteria further comprise one or more genes encoding one or more recombinase(s) under the control of an inducible promoter, and an inverted toxin sequence. In some embodiments, the genetically engineered bacteria further comprise one or more genes encoding an antitoxin. In some embodiments, the engineered bacteria further comprise one or more genes encoding one or more recombinase(s) under the control of an inducible promoter and one or more inverted excision genes, wherein the excision gene(s) encode an enzyme that deletes an essential gene. In some embodiments, the genetically engineered bacteria further comprise one or more genes encoding an antitoxin. In some embodiments, the engineered bacteria further comprise one or more genes encoding a toxin under the control of a promoter having a TetR repressor binding site and a gene encoding the TetR under the control of an inducible promoter that is induced by arabinose, such as P_{araBAD}. In some embodiments, the genetically engineered bacteria further comprise one or more genes encoding an antitoxin.

In some embodiments, the genetically engineered bacteria is an auxotroph comprising gene sequence encoding a branched chain amino acid catabolism enzyme and further comprises a kill-switch circuit, such as any of the kill-switch circuits described herein. In some embodiments of the above described genetically engineered bacteria, the gene encoding a branched chain amino acid catabolism enzyme is present on a plasmid in the bacterium. In some embodiments, the gene encoding a branched chain amino acid catabolism enzyme is present in the bacterial chromosome. In some embodiments, the gene sequence(s) encoding a BCAA transporter or other amino acid transporter that transports one or more BCAA(s) into the bacterial cell, for example a transporter that is capable of transporting leucine, valine, and/or isoleucine into a bacterial cell, is present on a plasmid in the bacterium. In some embodiments, the gene sequence(s) encoding a BCAA transporter or other amino acid transporter that transports one or more BCAA(s) into the bacterial cell, for example a transporter that is capable of transporting leucine, valine, and/or isoleucine into a bacterial cell, is present in the bacterial chromosome. In some embodiments, the gene sequence encoding a secretion protein or protein complex for secreting a biomolecule, such as any of the secretion systems disclosed herein, is present on a plasmid in the bacterium. In some embodiments, the gene sequence encoding a secretion protein or protein complex for secreting a biomolecule, such as any of the secretion systems disclosed herein, is present in the bacterial chromosome. In some embodiments, the gene sequence(s) encoding an antibiotic resistance gene is present on a plasmid in the bacterium. In some embodiments, the gene sequence(s) encoding an antibiotic resistance gene is present in the bacterial chromosome.

### Branched Chain Amino Acid Catabolism Enzymes

As used herein, the term "branched chain amino acid catabolic or catabolism enzyme" refers to an enzyme involved in the catabolism of a branched chain amino acid to its corresponding α-keto acid counterpart; or the catabolism of an alpha-keto acid to its corresponding aldehyde, acyl-CoA, alcohol, carboxylic acid, or other metabolite counterpart. In some embodiments, the present disclosure provides an engineered bacteria comprising gene sequence(s) encoding one or more branched chain amino acid catabolism enzyme(s). In some embodiments, the branched chain amino acid catabolism enzyme is used to convert a branched chain amino acid, e.g., leucine, valine, isoleucine, to its corresponding α-keto-acid, e.g., α-ketoisocaproate, α-keto-β-methylvalerate, and α-ketoisovalerate. In some embodiments, wherein a branched chain amino acid catabolism enzyme is used to convert a branched chain amino acid, e.g., leucine, valine, isoleucine, to its corresponding α-keto-acid, the engineered bacteria further comprise one or more branched chain amino acid catabolism enzyme(s) to convert an α-keto-acid to its corresponding acetyl CoA, e.g., isovaleryl-CoA, α-methylbutyryl-CoA, and isobutyryl-CoA. In some embodiments, wherein a branched chain amino acid catabolism enzyme is used to convert a branched chain amino acid, e.g., leucine, valine, isoleucine, to its corresponding α-keto-acid, the engineered bacteria further comprise one or more branched chain amino acid catabolism enzyme(s) to convert an α-keto-acid to its corresponding aldehyde, e.g., isovaeraldehyde, isobutyraldehyde, and 2-methylbutyraldehyde. In some embodiments, the engineered bacteria may further comprise an alcohol dehydrogenase enzyme in order to convert the branched chain amino acid-derived aldehyde (e.g., isovaleraldehyde, isobutyraldehyde, 2-methylbutyraldehyde) to its respective alcohol. In some embodiments, the engineered bacteria may further comprise an aldehyde dehydrogenase enzyme in order to convert the branched chain amino acid-derived aldehyde (e.g., isovaleraldehyde, isobutyraldehyde, 2-methylbutyraldehyde) to its respective carboxylic acid.

Enzymes involved in the catabolism of branched chain amino acids are well known to those of skill in the art. For example, in bacteria, leucine dehydrogenase (LeuDH), branched achain amino acid transferase (IlvE), amino acid oxidase (also known as amino acid deaminase) (L-AAD), as well as other known enzymes, can be used to convert a BCAA to its corresponding α-keto acid, e.g., ketoisocaproate (KIC), ketoisovalerate (KIV), and ketomethylvalerate (KMV). Leucine dehydrogenases, branched chain amino acid transamination enzymes (EC 2.6.1.42), and L-amino acid deaminases (L-AAD), which oxidatively deaminate branched chain amino acids into their respective alpha-keto acid, are known (Baker et al., Structure, 3(7):693-705, 1995; Peng et al., J. Bact., 139(2):339-45, 1979; and Kline et al., J. Bact., 130(2):951-3, 1977). In bacteria, branched chain keto acid dehydrogenases ("BCKDs") are enzyme complexes that oxidatively decarboxylate all three branched chain keto acids into their respective acyl-CoA derivatives. Thus, in one embodiment, the branched chain amino acid catabolism enzyme is a branched chain keto acid dehydrogenase (BCKD). Moreover, in mammals, dehydrogenases specific for 2-ketoisovalerate (EC 1.2.4.4) and 2-keto-3-methylvalerate and 2-keto-isocaproate (EC 1.2.4.3) have been identified (see, for example, Massey et al., Bacteriol Rev., 40(1):42-54, 1976). In bacteria, branched chain keto acid dehydrogenases ("BCKDs") are enzyme complexes that oxidatively decarboxylate all three branched chain keto acids into their respective acyl-CoA derivatives. Also, for example, in bacteria, α-ketoisovalerate decarboxylase (KivD) enzymes are capable of converting α-keto acids into aldehydes (e.g., isovaleraldehyde, isobutyraldehyde, 2-methylbutyraldehyde). Specifically, the α-ketoisovalerate decarboxylase enzyme KivD is capable of metabolizing valine by converting α-ketoisovalerate to isobutyraldehyde (see, for example, de la Plaza et al., FEMS Microbiol. Lett. 2004, 238(2):367-374). KivD is capable of metabolizing leucine by converting α-ketoisocaproate (KIC) to isovaleraldehyde. KivD is also capable of metabolizing isoleucine by converting α-ketomethylvalerate (KMV) to 2-methylbutyraldehyde. In addition, enzymes for converting isovaleraldehyde, isobutyraldehyde, and 2-methylbutyraldehyde to their respective alcohols or carboxylic acids are known and available. For example, alcohol dehydrogenases (e.g., Adh2, YqhD) can convert isovaleraldehyde, isobutyraldehyde, 2-methylbutyraldehyde to isopentanol, isobutanol, and 2-methylbutanol, respectively. Aldehyde dehydrogenases (e.g., PadA) can convert isovaleraldehyde, isobutyraldehyde, 2-methylbutyraldehyde to isovalerate, isobutyrate, and 2-methylbutyrate, respectively.

In some embodiments, the branched chain amino acid catabolism enzyme increases the rate of branched chain amino acid catabolism. In some embodiments, the branched chain amino acid catabolism enzyme decreases the level of one or more branched chain amino acids, e.g., leucine, isoleucine, and/or valine, in a cell, tissue, or organism. In some embodiments, the branched chain amino acid catabolism enzyme decreases the level of alpha-keto acid derived from BCAA in a cell, tissue, or organism. In some embodiments, the branched chain amino acid catabolism enzyme decreases the level of branched chain amino acid as compared to the level of its corresponding alpha-keto acid in a cell, tissue, or organism. In other embodiments, the branched chain amino acid catabolism enzyme increases the level of alpha-keto acid as compared to the level of its corresponding branched chain amino acid in a cell, tissue, or organism. In some embodiments, the branched chain amino acid catabolism enzyme decreases the level of the branched chain amino acid as compared to the level of its corresponding Acyl-CoA derivative in a cell, tissue, or organism. In some embodiments, the branched chain amino acid catabolism enzyme increases the level of the Acyl-CoA derivative as compared to the level of the branched chain amino acid in a cell, tissue, or organism. In some embodiments, the branched chain amino acid catabolism enzyme decreases the level of alpha-keto aldehyde derived from BCAA, e.g., isovaleraldehyde, isobutyraldehyde, and 2-methylbutyraldehyde, in a cell, tissue, or organism. In some embodiments, the branched chain amino acid catabolism enzyme decreases the level of branched chain amino acid as compared to the level of its corresponding alpha-keto aldehyde, e.g., isovaleraldehyde, isobutyraldehyde, and 2-methylbutyraldehyde, in a cell, tissue, or organism. In other embodiments, the branched chain amino acid catabolism enzyme increases the level of alpha-keto aldehyde, e.g., isovaleraldehyde, isobutyraldehyde, and 2-methylbutyraldehyde, as compared to the level of its corresponding branched chain amino acid in a cell, tissue, or organism. In some embodiments, the branched chain amino acid catabolism enzyme decreases the level of a corresponding downstream metabolite, e.g., isovalerate, isobutyrate, 2-methylbutyrate, isopentanol, isobutanol, and 2-methylbutanol, in a cell, tissue, or organism. In some embodiments, the branched chain amino acid catabolism enzyme decreases the level of branched chain amino acid as compared to the level of a corresponding downstream metabolite, e.g., isovalerate, isobutyrate, 2-methylbutyrate, isopentanol, isobutanol, and 2-methylbutanol, in a cell, tissue, or organism. In other embodiments, the branched chain amino acid catabolism enzyme increases the level of a downstream metabolite, e.g., isovalerate, isobutyrate, 2-methylbutyrate, isopentanol, isobutanol, and 2-methylbutanol, as compared to the level of its corresponding branched chain amino acid in a cell, tissue, or organism.

In some embodiments, the branched chain amino acid catabolism enzyme is a leucine catabolism enzyme. In other embodiments, the branched chain amino acid catabolism enzyme is an isoleucine catabolism enzyme. In other embodiments, the branched chain amino acid catabolism enzyme is a valine catabolism enzyme. In some embodiments, the branched chain amino acid catabolism enzyme is involved in the catabolism of leucine, isoleucine, and valine. In another embodiment, the branched chain amino acid catabolism enzyme is involved in the catabolism of leucine and valine, isoleucine and valine, or leucine and isoleucine. In some embodiments, the branched chain amino acid catabolism enzyme converts leucine, isoleucine, and/or valine into its corresponding α-keto acid. In certain specific embodiments, the present disclosure provides an engineered bacteria comprising gene sequence(s) encoding one or more catabolism enzymes seleceted from leucine dehydrogenase (LeuDH), BCAA aminotransferase (IlvE), and/or amino acid oxidase (L-AAD).

In sone embodiments, the branched chain amino acid catabolism enzyme is an alpha-ketoisocaproic acid (KIC) catabolism enzyme. In other embodiments, the branched chain amino acid catabolism enzyme is an alpha-ketoisovaleric acid (KIV) catabolism enzyme. In other embodiments, the branched chain amino acid catabolism enzyme is an alpha-keto-beta-methylvaleric acid (KMV) catabolism enzyme. In other embodiments, the branched chain amino acid catabolism enzyme is involved in the catabolism of alpha-ketoisocaproic acid (KIC), alpha-ketoisovaleric acid (KIV), and alpha-keto-beta-methylvaleric acid (KMV). In other embodiments, the branched chain amino acid catabolism enzyme is involved in the catabolism of KIC and KIV, KIC and KMV, or KIV and KMV. In some embodiments, the branched chain amino acid catabolism enzyme converts alpha-ketoisocaproic acid (KIC), alpha-ketoisovaleric acid (KIV), and/or alpha-keto-beta-methylvaleric acid (KMV) into its corresponding aldehyde, e.g., isovaleraldehyde, isobutyraldehyde, and/or 2-methylbutyraldehyde. In some embodiments, the present disclosure provides an engineered bacteria comprising gene sequence(s) encoding KivD.

In one embodiment, the branched chain amino acid catabolism enzyme is an isovaleraldehyde catabolism enzyme. In another embodiment, the branched chain amino acid catabolism enzyme is an isobutyraldehyde catabolism enzyme. In another embodiment, the branched chain amino acid catabolism enzyme is 2-methylbutyraldehyde catabolism enzyme. In another embodiment, the branched chain amino acid catabolism enzyme is involved in the catabolism of isovaleraldehyde, isobutyraldehyde, and 2-methylbutyraldehyde. In another embodiment, the branched chain amino acid catabolism enzyme is involved in the catabolism of isovaleraldehyde and isobutyraldehyde, isovaleraldehyde and 2-methylbutyraldehyde, or isobutyraldehyde and 2-methylbutyraldehyde. In some embodiments, the present disclosure provides an engineered bacteria comprising gene sequence(s) encoding one or more alcohol dehydrogenase(s), e.g., Ahd2, YqhD. In some embodiments, the present disclosure provides an engineered bacteria comprising gene sequence(s) encoding one or more aldehyde dehydrogenase(s), e.g., PadA. In some embodiments, the present disclosure provides an engineered bacteria comprising gene sequence(s) encoding one or more alcohol dehydrogenase(s), e.g., Ahd2, YqhD and one or more aldehyde dehydrogenase(s), e.g., PadA.

In some embodiments, the present disclosure provides an engineered bacteria comprising gene sequence(s) encoding one or more branched chain amino acid catabolism enzyme(s) involved in the catabolism of leucine, isoleucine, and/or valine, and further comprises gene sequence(s) encoding one or more branched chain amino acid catabolism enzyme(s) involved in the catabolism of KIC, KIV, and/or KMV. In some embodiments, the present disclosure provides an engineered bacteria comprising gene sequence(s) encoding one or more branched chain amino acid catabolism enzyme(s) involved in the catabolism of leucine, isoleucine, and/or valine, further comprises gene sequence(s) encoding one or more branched chain amino acid catabolism enzyme(s) involved in the catabolism of KIC, KIV, and/or KMV, and further comprises gene sequence(s) encoding one or more branched chain amino acid catabolism enzyme(s) involved in the catabolism of. isovaleraldehyde, isobutyraldehyde, and/or 2-methylbutyraldehyde. In some embodiments, the present disclosure provides an engineered bacteria comprising gene sequence(s) encoding one or more branched chain amino acid catabolism enzyme(s) selected from LeuDH, IlvE, L-AAD, KivD, PadA, Adh2, and YqhD.

In some embodiments, the present disclosure provides an engineered bacteria comprising gene sequence(s) encoding one or more branched chain amino acid catabolism enzyme(s) involved in the conversion of leucine, isoleucine, and/or valine to KIC, KIV, and/or KMV, respectively. In some embodiments, the present disclosure provides an engineered bacteria comprising gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively. In some embodiments, the present disclosure provides an engineered bacteria comprising gene sequence(s) encoding one or more branched chain amino acid catabolism enzyme(s) involved in the conversion of isovaleraldehyde, isobutyraldehyde, and/or 2-methylbutyraldehyde to isovalerate, isobutyrate, and/or 2-methylbutyrate, respectively. In some embodiments, the present disclosure provides an engineered bacteria comprising gene sequence(s) encoding one or more branched chain amino acid catabolism enzyme(s) involved in the conversion of. isovaleraldehyde, isobutyraldehyde, and/or 2-methylbutyraldehyde to isopentanol, isobutanol, and/or 2-methylbutanol respectively.

In some embodiments, the present disclosure provides an engineered bacteria comprising gene sequence(s) encoding one or more branched chain amino acid catabolism enzyme(s) involved in the conversion of leucine, isoleucine, and/or valine to KIC, KIV, and/or KMV, respectively, and further comprises gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively. In some embodiments, the present disclosure provides an engineered bacteria comprising gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively, and further comprises gene sequence(s) encoding one or more branched chain amino acid catabolism enzyme(s) involved in the conversion of. isovaleraldehyde, isobutyraldehyde, and/or 2-methylbutyraldehyde to isovalerate, isobutyrate, and/or 2-methylbutyrate, respectively. In some embodiments, the present disclosure provides an engineered bacteria comprising gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively, and further comprises gene sequence(s) encoding one or more branched chain amino acid catabolism enzyme(s) involved in the conversion of isovaleraldehyde, isobutyraldehyde, and/or 2-methylbutyraldehyde to isopentanol, isobutanol, and/or 2-methylbutanol respectively.

In some embodiments, the present disclosure provides an engineered bacteria comprising gene sequence(s) encoding one or more branched chain amino acid catabolism enzyme(s) involved in the conversion of leucine, isoleucine, and/or valine to KIC, KIV, and/or KMV, respectively, further comprises gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively, and further comprises gene sequence(s) encoding one or more branched chain amino acid catabolism enzyme(s) involved in the conversion of isovaleraldehyde, isobutyraldehyde, and/or 2-methylbutyraldehyde to isovalerate, isobutyrate, and/or 2-methylbutyrate, respectively. In some embodiments, the present disclosure provides an engineered bacteria comprising gene sequence(s) encoding one or more branched chain amino acid catabolism enzyme(s) involved in the conversion of leucine, isoleucine, and/or valine to KIC, KIV, and/or KMV, respectively, further comprises gene sequence(s) encoding one or more branched chain amino acid catabolism enzyme(s) involved in the converison of KIC, KIV, and/or KMV to isovaleraldehyde, isobutyraldehyde, and/or 2-methylbutyraldehyde, respectively, and further comprises gene sequence(s) encoding one or more branched chain amino acid catabolism enzyme(s) involved in the conversion of. isovaleraldehyde, isobutyraldehyde, and/or 2-methylbutyraldehyde to isopentanol, isobutanol, and/or 2-methylbutanol, respectively. In some embodiments, the present disclosure provides an engineered bacteria comprising gene sequence(s) encoding one or more branched chain amino acid catabolism enzyme(s) selected from LeuDH, IlvE, and/or L-AAD, KivD, PadA, Adh2, and YqhD.

Enzymes involved in the catabolism of a branched chain amino acid may be expressed or modified in the bacteria disclosed herein in order to enhance catabolism of a branched chain amino acid, *e.g.,* leucine. Specifically, when a branched chain amino acid catabolism enzyme is expressed in the engineered bacteria disclosed herein, the engineered bacteria are able to convert (deaminate) more branched chain amino acids (e.g., leucine, valine, isoleucine) into their respective alpha-keto acids (KIC, KIV, KMV) and/or convert more BCAA alpha-keto acids (e.g., KIC, KIV, KMV) into respective BCAA-derived aldehydes (e.g., isovaleraldehyde, isobutyraldehyde, 2-methylbutyraldehyde) and/or convert more BCAA-derived aldehydes into respective alcohols (e.g., isopentanol, isobutanol, 2-methylbutanol) and/or convert more BCAA-derived aldehydes into respective carboxylic acids (isovalerate, isobutyrate, 2-methylbutyrate), and/or convert (decarboxylate) more branched chain alpha-keto acids into their respective acyl-CoA derivatives when the catabolism enzyme(s) is expressed, in comparison with unmodified bacteria of the same bacterial subtype under the same conditions. Thus, for example, the genetically engineered bacteria comprising gene sequence encoding a branched chain amino acid catabolism enzyme can catabolize the branched chain amino acid, *e.g.,* leucine, and/or its corresponding alpha-keto acid, e.g., alpha-ketoisocaproate, to treat diseases associated with catabolism of branched chain amino acids, such as Maple Syrup Urine Disease (MSUD) and others described herein.

In some embodiments, the engineered bacteria comprise gene sequence(s) encoding one or more branched chain amino acid catabolism enzyme(s) and gene sequence(s) encoding one or more transporter(s) capable of importing a BCAA or metabolite thereof. In some embodiments, the engineered bacteria comprise gene sequence(s) encoding at least one branched chain amino acid catabolism enzyme and gene sequence(s) encoding two or more copies of a transporter capable of importing a BCAA or metabolite thereof. In some embodiments, the engineered bacteria comprise gene sequence(s) encoding at least one branched chain amino acid catabolism enzyme and gene sequence(s) encoding two or more different transporter(s) capable of importing a BCAA or metabolite thereof. In certain embodiments, the transporter is a leucine transporter. In certain embodiments, the transporter is a valine transporter. In certain embodiments, the transporter is an isoleucine transporter. In certain embodiments, the transporter is a branched chain amino acid transporter, e.g., capable of importing leucine, isoleucine, and valine. In certain specific embodiments, the transporter is selected from LivKHMGF and BrnQ.

In some embodiments, the engineered bacteria comprise gene sequence(s) encoding at least one branched chain amino acid catabolism enzyme and gene sequence(s) encoding one or more BCAA binding proteins, e.g., a BCAA binding protein that assists in bringing BCAA(s) into the bacterial cell. In some embodiments, the engineered bacteria comprise gene sequence(s) encoding at least one branched chain amino acid catabolism enzyme, gene sequence(s) encoding one or more transporter(s) capable of importing one or more BCAAs, and gene sequence(s) encoding one or more BCAA binding proteins, e.g., a BCAA binding protein that assists in bringing BCAA(s) into the bacterial cell. In any of these embodiments, the engineered bacteria comprise gene sequence(s) encoding two or more copies of a BCAA binding protein. In any of these embodiments, the engineered bacteria comprise gene sequence(s) encoding two or more different BCAA binding proteins. In certain embodiments, the BCAA binding protein is LivJ.

In any of the embodiments described above and herein, the engineered bacteria may further comprise one or more genetic modification(s) that reduces export of a branched chain amino acid from the bacteria, *e.g.,* a deletion or mutation in at least one gene associated with the export of a BCAA, *e.g.,* deletion or mutation in *leuE* gene and/or its promoter (which reduces or eliminates the export of leucine). In some embodiments, the engineered bacteria comprise gene sequence(s) encoding at least one branched chain amino acid catabolism enzyme and at least one genetic modification that reduces export of a branched chain amino acid. In certain specific embodiments, the engineered bacteria comprise gene sequence(s) encoding at least one branched chain amino acid catabolism enzyme, gene sequence(s) encoding one or more transporter(s) capable of importing a BCAA, and at least one genetic modification that reduces export of a branched chain amino acid. In certain specific embodiments, the engineered bacteria comprise gene sequence(s) encoding at least one branched chain amino acid catabolism enzyme, gene sequence(s) encoding one or more transporter(s) capable of importing a BCAA, gene sequence(s) encoding one or more BCAA binding proteins, and at least one genetic modification that reduces export of a branched chain amino acid. In some embodiments, the engineered bacteria comprise gene sequence(s) encoding at least one branched chain amino acid catabolism enzyme, gene sequence(s) encoding one or more BCAA binding proteins, and at least one genetic modification that reduces export of a branched chain amino acid. In any of these embodiments, the genetic modification may be a deletion or mutation in one or more gene(s) that allow or assist in the export of a BCAA. In any of these embodiment, the genetic modification may be a deletion or mutation in a *leuE* gene and/or its promoter.

In any of the embodiments described above and herein, the engineered bacteria comprise gene sequence(s) encoding one or more branched chain amino acid catabolism enzyme(s), and at least one genetic modification that reduces endogenous biosynthesis of a branched chain amino acid, for example, a deletion or mutation in at least one gene required for BCAA synthesis, e.g., deletion or mutation in ilvC gene and/or its promoter, which gene is required for BCAA synthesis and whose absence creates an auxotroph requiring the bacterial cell to import leucine. In some embodiments, the engineered bacteria comprise gene sequence(s) encoding one or more branched chain amino acid catabolism enzyme(s), gene sequence(s) encoding one or more transporter(s) capable of importing a BCAA, and at least one genetic modification that reduces endogenous biosynthesis of a branched chain amino acid, for example, a deletion or mutation in at least one gene required for BCAA synthesis. In some embodiments, the engineered bacteria comprise gene sequence(s) encoding at least one branched chain amino acid catabolism enzyme, gene sequence(s) encoding one or more BCAA binding proteins, and at least one genetic modification that reduces endogenous biosynthesis of a branched chain amino acid. In some embodiments, the engineered bacteria comprise gene sequence(s) encoding at least one branched chain amino acid catabolism enzyme, at least one genetic modification that reduces export of a branched chain amino acid, and at least one genetic modification that reduces endogenous biosynthesis of a branched chain amino acid. In some embodiments, the engineered bacteria comprise gene sequence(s) encoding at least one branched chain amino acid catabolism enzyme, gene sequence(s) encoding one or more transporter(s) capable of importing a BCAA, gene sequence(s) encoding one or more BCAA binding proteins, and at least one genetic modification that reduces endogenous biosynthesis of a branched chain amino acid. In some embodiments, the engineered bacteria comprise gene sequence(s) encoding at least one branched chain amino acid catabolism enzyme, gene sequence(s) encoding one or more transporter(s) capable of importing a BCAA, at least one genetic modification that reduces export of a branched chain amino acid, and at least one genetic modification that reduces endogenous biosynthesis of a branched chain amino acid. In some embodiments, the engineered bacteria comprise gene sequence(s) encoding at least one branched chain amino acid catabolism enzyme, gene sequence(s) encoding one or more BCAA binding proteins, at least one genetic modification that reduces export of a branched chain amino acid, and at least one genetic modification that reduces endogenous biosynthesis of a branched chain amino acid. In some embodiments, the engineered bacteria comprise gene sequence(s) encoding at least one branched chain amino acid catabolism enzyme, gene sequence(s) encoding one or more transporter(s) capable of importing a BCAA, gene sequence(s) encoding one or more BCAA binding proteins, at least one genetic modification that reduces export of a branched chain amino acid, and at least one genetic modification that reduces endogenous biosynthesis of a branched chain amino acid. In any of these embodiments, the at least one genetic modification that reduces endogenous biosynthesis of a branched chain amino acid can be a deletion or mutation in at least one gene required for BCAA synthesis, e.g., deletion or mutation in ilvC gene and/or its promoter.

In any of the embodiments described above and herein, the gene sequence(s) encoding at least one branched chain amino acid catabolism enzyme, and/or gene sequence(s) encoding one or more transporter(s) capable of importing a BCAA, and/or gene sequence(s) encoding one or more BCAA binding proteins, and/or other sequence can be present in the bacterial chromosome. In any of the embodiments described above and herein, the gene sequence(s) encoding at least one branched chain amino acid catabolism enzyme, and/or gene sequence(s) encoding one or more transporter(s) capable of importing a BCAA, and/or gene sequence(s) encoding one or more BCAA binding proteins, and/or other sequence can be present in one or more plasmids.

In some embodiments, the engineered bacteria comprise gene sequence(s) encoding one or more branched chain amino acid catabolism enzyme(s) in which the one or more enzymes are from a different organism, *e.g.*, a different species of bacteria. In some embodiments, the engineered bacteria comprise gene sequence(s) encoding one or more branched chain amino acid catabolism enzyme(s) in which the one or more enzymes are native to the bacterium. In some embodiments, the engineered bacteria comprise gene sequence(s) encoding one or more branched chain amino acid catabolism enzyme(s) in which one or more of the enzymes are native and one or more of the enzymes are from a different organism, *e.g.,* a different species of bacteria. In other embodiments, the bacterial cell comprises more than one copy of a native gene encoding a branched chain amino acid catabolism enzyme. In other embodiments, the bacterial cell comprises more than one copy of a non-native gene encoding a branched chain amino acid catabolism enzyme. In other embodiments, the bacterial cell comprises at least one, two, three, four, five, six or more copies of a gene encoding a branched chain amino acid catabolism enzyme, which can be native or non-native. In other embodiments, the bacterial cell comprises multiple copies of a gene encoding a branched chain amino acid catabolism enzyme. In some embodiments, the bacterial cell comprises gene sequence(s) encoding multiple copies of two or more different branched chain amino acid catabolism enzymes.

In some embodiments, the engineered bacteria comprise gene sequence(s) encoding one or more branched chain amino acid transporters in which the one or more transporters are from a different organism, *e.g.,* a different species of bacteria. In some embodiments, the engineered bacteria comprise gene sequence(s) encoding one or more branched chain amino acid transporter(s) in which the one or more transporters are native to the bacterium. In some embodiments, the engineered bacteria comprise gene sequence(s) encoding one or more branched chain amino acid transporters(s) in which one or more of the transporters are native and one or more of the transporters are from a different organism, *e.g.,* a different species of bacteria. In other embodiments, the bacterial cell comprises more than one copy of a native gene encoding a branched chain amino acid transporter. In other embodiments, the bacterial cell comprises more than one copy of a non-native gene encoding a branched chain amino acid transporter. In other embodiments, the bacterial cell comprises at least one, two, three, four, five, six or more copies of a gene encoding a branched chain amino acid transporter, which can be native or non-native. In other embodiments, the bacterial cell comprises multiple copies of a gene encoding a branched chain amino acid transporters. In some embodiments, the bacterial cell comprises gene sequence(s) encoding multiple copies of two or more different branched chain amino acid transporters.

In some embodiments, the engineered bacteria comprise gene sequence(s) encoding one or more branched chain amino acid binding protein(s) in which the one or more binding protein(s) are from a different organism, *e.g.,* a different species of bacteria. In some embodiments, the engineered bacteria comprise gene sequence(s) encoding one or more branched chain amino acid binding protein(s) in which the one or more binding protein(s) are native to the bacterium. In some embodiments, the engineered bacteria comprise gene sequence(s) encoding one or more branched chain amino acid binding protein(s) in which one or more of the binding protein(s) are native and one or more of the binding protein(s) are from a different organism, *e.g.,* a different species of bacteria. In other embodiments, the bacterial cell comprises more than one copy of a native gene encoding a branched chain amino acid binding protein. In other embodiments, the bacterial cell comprises more than one copy of a non-native gene encoding a branched chain amino acid binding protein. In other embodiments, the bacterial cell comprises at least one, two, three, four, five, six or more copies of a gene encoding a branched chain amino acid binding protein, which can be native or non-native. In other embodiments, the bacterial cell comprises multiple copies of a gene encoding a branched chain amino acid binding protein. In some embodiments, the bacterial cell comprises gene sequence(s) encoding multiple copies of two or more different branched chain amino acid binding proteins.

Branched chain amino acid catabolism enzymes are known in the art. In some embodiments, the branched chain amino acid catabolism enzyme is encoded by a gene encoding a branched chain amino acid catabolism enzyme derived from a bacterial species. In some embodiments, a branched chain amino acid catabolism enzyme is encoded by a gene encoding a branched chain amino acid catabolism enzyme derived from a non-bacterial species. In some embodiments, a branched chain amino acid catabolism enzyme is encoded by a gene derived from a eukaryotic species, *e.g.,* a yeast species or a plant species. In one embodiment, a branched chain amino acid catabolism enzyme is encoded by a gene derived from a mammalian species, e.g., a human. In some embodiments, the engineered bacteria comprises gene sequence(s) encoding at least one branched chain amino acid catabolism enzyme derived from a bacterial species and at least one branched chain amino acid catabolism enzyme derived from a non-bacterial species. In one embodiment, the gene encoding the branched chain amino acid catabolism enzyme is derived from an organism of the genus or species that includes, but is not limited to, *Acetinobacter, Azospirillum, Bacillus, Bacteroides, Bifidobacterium, Brevibacteria, Burkholderia, Citrobacter, Clostridium, Corynebacterium, Cronobacter, Enterobacter, Enterococcus, Erwinia, Helicobacter, Klebsiella, Lactobacillus, Lactococcus, Leishmania, Listeria, Macrococcus, Mycobacterium, Nakamurella*, *Nasonia, Nostoc, Pantoea, Pectobacterium, Pseudomonas, Psychrobacter, Ralstonia, Saccharomyces, Salmonella, Sarcina, Serratia, Staphylococcus, and Yersinia, e.g., Acetinobacter radioresistens, Acetinobacter baumannii, Acetinobacter calcoaceticus, Azospirillum brasilense*, *Bacillus anthracis, Bacillus cereus, Bacillus coagulans, Bacillus megaterium, Bacillus subtilis, Bacillus thuringiensis, Bacteroides fragilis, Bacteroides subtilis, Bacteroides thetaiotaomicron, Bifidobacterium bifidum*, *Bifidobacterium infantis, Bifidobacterium lactis, Bifidobacterium longum, Burkholderia xenovorans, Citrobacter youngae, Citrobacter koseri, Citrobacter rodentium, Clostridium acetobutylicum, Clostridium butyricum*, *Corynebacterium aurimucosum, Corynebacterium kroppenstedtii, Corynebacterium striatum, Cronobacter sakazakii*, *Cronobacter turicensis, Enterobacter cloacae, Enterobacter cancerogenus, Enterococcus faecium, Erwinia amylovara*, *Erwinia pyrifoliae*, *Erwinia tasmaniensis, Helicobacter mustelae*, *Klebsiella pneumonia, Klebsiella variicola, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, Leishmania infantum*, *Leishmania major, Leishmania brazilensis, Listeria grayi, Macrococcus caseolyticus, Mycobacterium avium, Mycobacterium intracellulare, Mycobacterium kansasii, Mycobacterium leprae, Mycobacterium marinum, Mycobacterium smegmatis*, *Mycobacterium tuberculosis, Mycobacterium ulcerans, Nakamurella multipartita*, *Nasonia vitipennis, Nostoc punctiforme, Pantoea ananatis, Pantoea agglomerans, Pectobacterium atrosepticum, Pectobacterium carotovorum*, *Pseudomonas aeruginosa, Psychrobacter articus, Psychrobacter cryohalolentis*, *Ralstonia eutropha, Saccharomyces boulardii, Salmonella enterica, Sarcina ventriculi, Serratia odorifera*, *Serratia proteamaculans*, *Staphylococcus aerus, Staphylococcus capitis, Staphylococcys carnosus, Staphylococcus epidermidis, Staphylococcus hominis, Staphylococcus haemolyticus, Staphylococcus lugdunensis, Staphylococcus saprophyticus, Staphylococcus warneri, Yersinia enterocolitica, Yersinia mollaretii*, *Yersinia kristensenii*, *Yersinia rohdei, and Yersinia aldovae.*

In some embodiments, the gene encoding a branched chain amino acid catabolism enzyme, BCAA transporter, BCAA binding protein, and/or other sequence has been codon-optimized for use in the host organism. In one embodiment, the gene encoding a branched chain amino acid catabolism enzyme has been codon-optimized for use in *Escherichia coli.* Examples of codon-optimized sequences include SEQ ID NO:29, SEQ ID NO:32, SEQ ID NO:39, and SEQ ID NO:41.

In some embodiments, the branched chain amino acid catabolism enzyme converts a branched chain amino acid to its corresponding alpha-keto acid. Such enzymes include, for example, LeuDH (SEQ ID NOs:19 and 20), IlvE (SEQ ID NO:s 21 and 22), L-AAD (SEQ ID NOs: 23-26). In other embodiments, the branched chain amino acid catabolism enzyme converts a branched chain keto acid to its corresponding aldehyde. For example, in some embodiments, the branched chain amino acid catabolism enzyme is an α-ketoisovalerate decarboxylase (KivD) (SEQ ID NO:27, 28, and 29). In other embodiments, the branched chain amino acid catabolism enzyme converts a branched chain keto acid to its corresponding acetyl-CoA. For example, in some embodiments, the branched chain amino acid catabolism enzyme is a branched chain keto acid dehydrogenase (BCKD). In some embodiments, the branched chain amino acid catabolism enzyme is a branched chain amino acid deaminase, such as an amino acid dehydrogenase, or a branched chain amino acid aminotransferase. In some embodiments, the branched chain amino acid catabolism enzyme is KdcA (SEQ ID NOs:30, 31, and 32). In other embodiments, the branched chain amino acid catabolism enzyme is THI3/KID1 (SEQ ID NOs:33 and 34). In other embodiments, the branched chain amino acid catabolism enzyme is ARO10 (SEQ ID NOs:35 and 36).

In other embodiments, the branched chain amino acid catabolism enzyme converts an aldehyde into its corresponding alcohol. For example, the branched chain amino acid catabolism enzyme may be an alcohol dehydrogenase. In one embodiment, the alcohol dehydrogenase is Adh2 (SEQ ID NOs: 37, 38, and 39). In another embodiment, the alcohol dehydrogenase is Adh6 (SEQ ID NOs: 40 and 41). In another embodiment, the alcohol dehydrogenase is Adh1 (SEQ ID NOs: 42 and 43). In another embodiment, the alcohol dehydrogenase is Adh3 (SEQ ID NOs: 44 and 45). In another embodiment, the alcohol dehydrogenase is Adh4 (SEQ ID NOs:46 and 47). In another embodiment, the alcohol dehydrogenase is SFA1 (SEQ ID NOs:52 and 53). In another embodiment, the alcohol dehydrogenase is YqhD (SEQ ID NOs: 60 and 61) In other embodiments, the branched chain amino acid catabolism enzyme converts an aldehyde into its corresponding carboxylic acid. For example, the branched chain amino acid catabolism enzyme may be an aldehyde dehydrogenase. In one embodiment, the aldehyde dehydrogenase is PadA (SEQ ID NOs: 62 and 63).

In some embodiments, the engineered bacteria comprise gene sequence(s) encoding one or more transporter(s) capable of importing a BCAA or metabolite thereof. In certain embodiments, the transporter is a leucine transporter. In certain embodiments, the transporter is a valine transporter. In certain embodiments, the transporter is an isoleucine transporter. In certain embodiments, the transporter is a branched chain amino acid transporter, e.g., capable of importing leucine, isoleucine, and valine. The term "BCAA transporter" is meant to refer to a transporter that specifically transports leucine, isoleucine, or valine, and also to a transporter that is able to transport any BCAA, including for example, the ability to transport leucine, isoleucine, and valine. For example, in some embodiments, the transporter is LivKHMGF (as comprised in SEQ ID NOs: 5, 7, and 10). In some embodiments, the transporter is BrnQ (SEQ ID Nos: 64 and 65).

In some embodiments, the engineered bacteria comprise gene sequence(s) encoding one or more BCAA binding proteins, e.g., a BCAA binding protein that assists in bringing BCAA(s) into the bacterial cell. For example, in some embodiments, the BCAA binding protein is LivJ (SEQ ID NO: 12).

The present disclosure further comprises genes encoding functional fragments of a branched chain amino acid catabolism enzyme, BCAA transporter, BCAA binding protein, and/or other sequence or functional variants of a branched chain amino acid catabolism enzyme, BCAA transporter, BCAA binding protein, and/or other sequence. As used herein, the term "functional fragment thereof' or "functional variant thereof' of a branched chain amino acid catabolism enzyme, BCAA transporter, BCAA binding protein, and/or other sequence refers to fragnment or variant sequence having qualitative biological activity in common with the wild-type branched chain amino acid catabolism enzyme, BCAA transporter, BCAA binding protein, and/or other sequence from which the fragment or variant was derived. For example, a functional fragment or a functional variant of a mutated branched chain amino acid catabolism enzyme is one which retains essentially the same ability to catabolize a branched chain amino acid and/or its corresponding alpha-keto acid or aldehyde or other metabolite as the branched chain amino acid catabolism enzyme from which the functional fragment or functional variant was derived. For example, a polypeptide having branched chain amino acid catabolism enzyme activity may be truncated at the N-terminus or C-terminus and the retention of branched chain amino acid catabolism enzyme activity assessed using assays known to those of skill in the art, including the exemplary assays provided herein. In one embodiment, the recombinant bacterial cell disclosed herein comprises a heterologous gene encoding a branched chain amino acid catabolism enzyme functional variant. In another embodiment, the recombinant bacterial cell disclosed herein comprises a heterologous gene encoding a branched chain amino acid catabolism enzyme functional fragment.

The present disclosure encompasses genes encoding a branched chain amino acid catabolism enzyme, BCAA transporter, BCAA binding protein, and/or other sequence comprising amino acids in its sequence that are substantially the same as an amino acid sequence described herein. Amino acid sequences that are substantially the same as the sequences described herein include sequences comprising conservative amino acid substitutions, as well as amino acid deletions and/or insertions. A conservative amino acid substitution refers to the replacement of a first amino acid by a second amino acid that has chemical and/or physical properties (e.g., charge, structure, polarity, hydrophobicity/hydrophilicity) that are similar to those of the first amino acid. Conservative substitutions include replacement of one amino acid by another within the following groups: lysine (K), arginine (R) and histidine (H); aspartate (D) and glutamate (E); asparagine (N), glutamine (Q), serine (S), threonine (T), tyrosine (Y), K, R, H, D and E; alanine (A), valine (V), leucine (L), isoleucine (I), proline (P), phenylalanine (F), tryptophan (W), methionine (M), cysteine (C) and glycine (G); F, W and Y; C, S and T. Similarly contemplated is replacing a basic amino acid with another basic amino acid (e.g., replacement among Lys, Arg, His), replacing an acidic amino acid with another acidic amino acid (e.g., replacement among Asp and Glu), replacing a neutral amino acid with another neutral amino acid (*e.g.,* replacement among Ala, Gly, Ser, Met, Thr, Leu, Ile, Asn, Gln, Phe, Cys, Pro, Trp, Tyr, Val).

The present disclosure encompasses branched chain amino acid catabolism enzymes, BCAA transporters, BCAA binding proteins, and/or other sequences which have a certain percent identity to a gene or protein sequence described herein. For example, the disclosure encompasses branched chain amino acid catabolism enzymes, BCAA transporters, BCAA binding proteins, and/or other sequences having at least 60%, 65%, 70%, 75%, 80%, 85%, 90%, 91%, 92%, 93%, 94%, 95%, 96%, 97%, 98%, 99%, or 100% identity to a nucleic acid sequence or amino acid sequence disclosed herein. As used herein, the term "percent (%) sequence identity" or "percent (%) identity," also including "homology," is defined as the percentage of amino acid residues or nucleotides in a candidate sequence that are identical with the amino acid residues or nucleotides in the reference sequences after aligning the sequences and introducing gaps, if necessary, to achieve the maximum percent sequence identity, and not considering any conservative substitutions as part of the sequence identity. Optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl. Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

Assays for testing the activity of a branched chain amino acid catabolism enzyme, BCAA transporter, BCAA binding protein, and/or other sequence, or a functional variant, or a functional fragment thereof are well known to one of ordinary skill in the art. For example, branched chain amino acid catabolism, BCAA transporter, BCAA binding protein, and/or other sequence can be assessed by expressing the protein, functional variant, or fragment thereof, in a recombinant bacterial cell that lacks endogenous branched chain amino acid catabolism enzyme activity. Branched chain amino acid catabolism can be assessed using the coupled enzymatic assay method as described by Zhang *et al.* (see, for example, Zhang et al., Proc. Natl. Acad. Sci., 105(52):20653-58, 2008). Furthermore, catabolism of branched chain amino acids can also be assessed *in vitro* by measuring the disappearance of alpha-ketoisovalerate as described by de la Plaza (see, for example, de la Plaza et al., FEMS Microbiol. Letters, 2004, 238(2):367-374). BCAA as well as the branched chain keto acid can be quantified by liquid chromatography tandem mass spectrometry (LC-MS/MS), as described herein.

In some embodiments, the gene encoding a branched chain amino acid catabolism enzyme, BCAA transporter, BCAA binding protein, and/or other sequence is mutagenized; mutants exhibiting increased activity are selected; and the mutagenized gene encoding the branched chain amino acid catabolism enzyme, BCAA transporter, BCAA binding protein, and/or other sequence is isolated and inserted into the bacterial cell. In some embodiments, the gene encoding an α-ketoisovalerate decarboxylase, *e.g., kivD*, is mutagenized; mutants exhibiting decreased activity are selected; and the mutagenized gene encoding the α-ketoisovalerate decarboxylase, *e.g., kivD,* is isolated and inserted into the bacterial cell. The gene comprising the modifications described herein may be present on a plasmid or chromosome.

In some embodiments, the gene encoding the branched chain amino acid catabolism enzyme, BCAA transporter, BCAA binding protein, and/or other sequence is directly operably linked to a first promoter. In other embodiments, the gene encoding the branched chain amino acid catabolism enzyme, BCAA transporter, BCAA binding protein, and/or other sequence is indirectly operably linked to a first promoter. In somes embodiment, the gene encoding the branched chain amino acid catabolism enzyme, BCAA transporter, BCAA binding protein, and/or other sequence is operably linked to a promoter that is not its native promoter.

In some embodiments, the gene encoding the branched chain amino acid catabolism enzyme, BCAA transporter, BCAA binding protein, and/or other sequence is expressed under the control of a constitutive promote. In other embodiments, the gene encoding the branched chain amino acid catabolism enzyme, BCAA transporter, BCAA binding protein, and/or other sequence is expressed under the control of an inducible promoter. In some embodiments, the gene encoding the branched chain amino acid catabolism enzyme, BCAA transporter, BCAA binding protein, and/or other sequence is expressed under the control of a promoter that is directly or indirectly induced by exogenous environmental conditions. In some embodiments, the gene encoding the branched chain amino acid catabolism enzyme, BCAA transporter, BCAA binding protein, and/or other sequence is expressed under the control of a promoter that is directly or indirectly induced by low-oxygen or anaerobic conditions, wherein expression of the gene encoding the branched chain amino acid catabolism enzyme is activated under low-oxygen or anaerobic environments, such as the environment of the mammalian gut. In some embodiments, the gene encoding the branched chain amino acid catabolism enzyme, BCAA transporter, BCAA binding protein, and/or other sequence is expressed under the control of a promoter that is directly or indirectly induced by inflammatory conditions. Exemplary inducible promoters described herein include oxygen level-dependent promoters (*e.g.*, FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein. Examples of other inducible promoters are provided herein below.

The gene encoding the branched chain amino acid catabolism enzyme, BCAA transporter, BCAA binding protein, and/or other sequence may be present on a plasmid or chromosome in the bacterial cell. In some embodiments, the gene encoding the branched chain amino acid catabolism enzyme, BCAA transporter, BCAA binding protein, and/or other sequence is located on a plasmid in the bacterial cell. In other embodiments, the gene encoding the branched chain amino acid catabolism enzyme, BCAA transporter, BCAA binding protein, and/or other sequence is located in the chromosome of the bacterial cell. In other embodiments, a native copy of the gene encoding the branched chain amino acid catabolism enzyme, BCAA transporter, BCAA binding protein, and/or other sequence is located in the chromosome of the bacterial cell, and a gene encoding a branched chain amino acid catabolism enzyme, BCAA transporter, BCAA binding protein, and/or other sequence from the same or a different species of bacteria is located on a plasmid in the bacterial cell. In other embodiments, a native copy of the gene encoding the branched chain amino acid catabolism enzyme, BCAA transporter, BCAA binding protein, and/or other sequence is located on a plasmid in the bacterial cell, and a gene encoding the branched chain amino acid catabolism enzyme, BCAA transporter, BCAA binding protein, and/or other sequence from a different species of bacteria is located on a plasmid in the bacterial cell. In other embodiments, a native copy of the gene encoding the branched chain amino acid catabolism enzyme, BCAA transporter, BCAA binding protein, and/or other sequence is located in the chromosome of the bacterial cell, and a gene encoding the branched chain amino acid catabolism enzyme, BCAA transporter, BCAA binding protein, and/or other sequence from a different species of bacteria is located in the chromosome of the bacterial cell.

In some embodiments, the gene encoding the branched chain amino acid catabolism enzyme, BCAA transporter, BCAA binding protein, and/or other sequence is expressed on a low-copy plasmid. In some embodiments, the gene encoding the branched chain amino acid catabolism enzyme, BCAA transporter, BCAA binding protein, and/or other sequence is expressed on a high-copy plasmid. In some embodiments, the high-copy plasmid may be useful for increasing expression of the branched chain amino acid catabolism enzyme, BCAA transporter, BCAA binding protein, and/or other sequence, thereby increasing the catabolism of the branched chain amino acid, *e.g.,* leucine.

In some embodiments, the engineered bacteria convert the branched chain amino acid(s) and/or corresponding alpha-keto acid(s) and/or other corresponding metabolite(s) to a non-toxic or low toxicity metabolite, e.g., isovaleraldehyde, isobutyraldehyde, 2-methylbutyraldehyde, isovaleric acid, isobutyric acid, 2-methylbutyric acid, isopentanol, isobutanol, and 2-methylbutanol. **Table 2** chart showing that the products of BCAA degradation by the engineered bacteria have very low oral toxicity.

**Table 2. Toxicity of BCAA degradation products**

| **Compound** | **Oral LD50 (rat) (mg/kg)** | **Oral NOAEL* (rat)(mg/kg/d)** |
|---|---|---|
| Isovaleraldehyde | 5740 | N/D |
| Isolbutyraldehyde | 3730 | N/D |
| 2-methylbutyraldehyde | 6884 | N/D |
| Isovaleric acid | 2500 | N/D |
| Isobutyric acid | 2230 | N/D |
| 2-metylbutyric acid | 1750 | N/D |
| Isopentanol | >5000 | 1250 |
| Isobutanol | 3350 | >1450 |
| 2-methylbutanol | 4170 | N/D |

| | | |
|---|---|---|
| * No-Observed-Adverse-Effect | | |

### A. Branched chain ketoacid decarboxylase

In one embodiment, the branched chain amino acid catabolism enzyme is a branched chain ketoacid decarboxylase, including but not limited to, KivD. In a non-limiting example, KivD is from Lactococcus lactis. Another non-limiting example is KdcA (e.g., from Lactococcus lactis). Thus, in some embodiments, the engineered bacteria comprise gene sequence(s) encoding one or more copies of a branched chain ketoacid decarboxylase. In some embodiments, the engineered bacteria comprise gene sequence(s) encoding one, two, three, four, five, six, or more copies of a branched chain ketoacid decarboxylase. The one or more copies of a branched chain ketoacid decarboxylase can be one or more copies of the same gene or can be different genes encoding α-ketoisovalerate decarboxylase, e.g., gene(s) from a different species or otherwise having a different gene sequence. The one or more copies of a branched chain ketoacid decarboxylase can be present in the bacterial chromosome or can be present in one or more plasmids. As used herein "α-ketoisovalerate decarboxylase" or "alpha-ketoisovalerate decarboxylase" or "branched-chain α-keto acid decarboxylase" or "α-ketoacid decarboxylase" or " branched chain ketoacid decarboxylase" or "2-ketoisovalerate decarboxylase" (referred to herein also as KivD or ketoisovalerate decarboxylase) refers to any polypeptide having enzymatic activity that catalyzes the conversion of a branched chain alpha-keto acid (BCKA), such as α-ketoisovalerate (2-oxoisopentanoate), α-ketomethylvalerate (3-methyl-2-oxopentanoate), or α-ketoisocaproate 4-methyl-2-oxopentanoate), to its corresponding aldehyde, such as isobutyraldehyde, 2-methylbutyraldehyde, or isovaleraldehyde, and carbon dioxide. Branched chain ketoacid decarboxylase enzymes are available from many microorganism sources, including those disclosed herein. Branched chain ketoacid decarboxylase employs the co-factor thiamine diphosphate (also known as thiamine pyrophosphate or "TPP" or "TDP"). Thiamine is the vitamin form of the co-factor which, when transported into a cell, is converted to thiamine diphosphate. Alpha-ketoisovalerate decarboxylase also employs Mg²⁺. Branched chain ketoacid decarboxylase may be a homotetramer.

The bacterial cells disclosed herein may comprise a heterologous gene encoding a branched chain ketoacid decarboxylase enzyme and are capable of converting α-keto acids into aldehydes. For example, the branched chain ketoacid decarboxylase enzyme KivD is capable of metabolizing leucine (see, for example, de la Plaza et al., FEMS Microbiol. Lett. 2004, 238(2):367-374), and a cytosolically active KivD should generally exhibit the ability to convert ketoisovalerate, ketomethylvalerate, and ketoisocaproate to isobutyraldehyde, 2-methylbutyraldehyde, and isovaleraldehyde.

Multiple distinct a branched chain ketoacid decarboxylase proteins are known in the art (see, *e.g.,* US Pat. Appl. Publ. No. 2013/0203138.

In some embodiments, branched chain ketoacid decarboxylase is encoded by a branched chain ketoacid decarboxylase gene derived from a bacterial species. In some embodiments, a branched chain ketoacid decarboxylase is encoded by a branched chain ketoacid decarboxylase gene derived from a non-bacterial species. In some embodiments, a branched chain ketoacid decarboxylase is encoded by an a branched chain ketoacid decarboxylase gene derived from a eukaryotic species, *e.g.,* a yeast species or a plant species. In some embodiments, a branched chain ketoacid decarboxylase is encoded by an a branched chain ketoacid decarboxylase gene derived from a mammalian species. In one embodiment, the a branched chain ketoacid decarboxylase gene is derived from an organism of the genus or species that includes, but is not limited to, *Acetinobacter, Azospirillum*, *Bacillus, Bacteroides, Bifidobacterium, Brevibacteria*, *Burkholderia, Citrobacter, Clostridium, Corynebacterium, Cronobacter, Enterobacter, Enterococcus, Erwinia, Helicobacter, Klebsiella, Lactobacillus, Lactococcus, Leishmania, Listeria, Macrococcus, Mycobacterium, Nakamurella*, *Nasonia, Nostoc, Pantoea, Pectobacterium*, *Psychrobacter, Ralstonia, Saccharomyces, Salmonella, Sarcina, Serratia, Staphylococcus, and Yersinia, e.g., Acetinobacter radioresistens, Acetinobacter baumannii, Acetinobacter calcoaceticus, Azospirillum brasilense, Bacillus anthracis, Bacillus cereus, Bacillus coagulans, Bacillus megaterium, Bacillus subtilis, Bacillus thuringiensis, Bacteroides fragilis, Bacteroides subtilis, Bacteroides thetaiotaomicron, Bifidobacterium bifidum*, *Bifidobacterium infantis, Bifidobacterium lactis, Bifidobacterium longum, Burkholderia xenovorans, Citrobacter youngae, Citrobacter koseri, Citrobacter rodentium, Clostridium acetobutylicum*, *Clostridium butyricum*, *Corynebacterium aurimucosum, Corynebacterium kroppenstedtii, Corynebacterium striatum, Cronobacter sakazakii*, *Cronobacter turicensis, Enterobacter cloacae, Enterobacter cancerogenus, Enterococcus faecium, Erwinia amylovara*, *Erwinia pyrifoliae*, *Erwinia tasmaniensis, Helicobacter mustelae, Klebsiella pneumonia, Klebsiella variicola, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, Leishmania infantum, Leishmania major, Leishmania brazilensis*, *Listeria grayi, Macrococcus caseolyticus, Mycobacterium avium, Mycobacterium intracellulare, Mycobacterium kansasii*, *Mycobacterium leprae, Mycobacterium marinum, Mycobacterium smegmatis, Mycobacterium tuberculosis, Mycobacterium ulcerans, Nakamurella multipartita, Nasonia vitipennis, Nostoc punctiforme, Pantoea ananatis, Pantoea agglomerans, Pectobacterium atrosepticum, Pectobacterium carotovorum, Psychrobacter articus, Psychrobacter cryohalolentis, Ralstonia eutropha, Saccharomyces boulardii, Salmonella enterica, Sarcina ventriculi, Serratia odorifera*, *Serratia proteamaculans*, *Staphylococcus aerus, Staphylococcus capitis, Staphylococcys carnosus, Staphylococcus epidermidis, Staphylococcus hominis, Staphylococcus haemolyticus, Staphylococcus lugdunensis, Staphylococcus saprophyticus, Staphylococcus warneri, Yersinia enterocolitica, Yersinia mollaretii*, *Yersinia kristensenii, Yersinia rohdei, and Yersinia aldovae.* In some embodiments, the a branched chain ketoacid decarboxylase is encoded by an a branched chain ketoacid decarboxylase gene derived from *Lactococcus lactis, e.g.,* IFPL730. In another embodiment, the a branched chain ketoacid decarboxylase, *e.g., kivD* gene, is derived from *Enterobacter cloacae* (Accession No. P23234.1), *Mycobacterium smegmatis* (Accession No. A0R480.1), *Mycobacterium tuberculosis* (Accession NO. 053865.1), *Mycobacterium avium* (Accession No. Q742Q2.1), *Azospirillum brasilense* (Accession No. P51852.1), or *Bacillus subtilis* (see Oku et al., J. Biol. Chem. 263: 18386-96, 1988).

In one embodiment, the branched chain ketoacid decarboxylase gene has been codon-optimized for use in the recombinant bacterial cell. In one embodiment, the branched chain ketoacid decarboxylase gene has been codon-optimized for use in *Escherichia coli.* For example, a codon-optimized *kivD* sequence is set forth as SEQ ID NO: 29.

In one embodiment, the branched chain ketoacid decarboxylase gene is a *kivD* gene. In another embodiment, the *kivD* gene is a *Lactococcus lactis kivD* gene or kivD gene derived from *Lactococcus lactis.* When a branched chain ketoacid decarboxylase is expressed in the recombinant bacterial cells disclosed herein, the bacterial cells catabolize more branched chain amino acid, *e.g.,* leucine, than unmodified bacteria of the same bacterial subtype under the same conditions (*e.g.*, culture or environmental conditions). Thus, the genetically engineered bacteria comprising a heterologous gene encoding a branched chain ketoacid decarboxylase may be used to catabolize excess branched chain amino acids, *e.g.,* leucine, to treat a disease associated with the catabolism of a branched chain amino acid, including Maple Syrup Urine Disease (MSUD).

The present disclosure further comprises genes encoding functional fragments of a branched chain ketoacid decarboxylase or functional variants of a branched chain ketoacid decarboxylase gene. As used herein, the term "functional fragment thereof' or "functional variant thereof' of a branched chain ketoacid decarboxylase gene relates to a sequence having qualitative biological activity in common with the wild-type branched chain ketoacid decarboxylasefrom which the fragment or variant was derived. For example, a functional fragment or a functional variant of a mutated branched chain ketoacid decarboxylase protein is one which retains essentially the same ability to catabolize BCKAs as a branched chain ketoacid decarboxylase protein from which the functional fragment or functional variant was derived. For example, a polypeptide having branched chain ketoacid decarboxylase activity may be truncated at the N-terminus or C-terminus and the retention of branched chain ketoacid decarboxylase activity assessed using assays known to those of skill in the art, including the exemplary assays provided herein. In one embodiment, the recombinant bacterial cell disclosed herein comprises a heterologous gene encoding a branched chain ketoacid decarboxylase arboxylase functional variant. In another embodiment, the recombinant bacterial cell disclosed herein comprises a heterologous gene encoding a branched chain ketoacid decarboxylase functional fragment.

Assays for testing the activity of a branched chain ketoacid decarboxylase, a branched chain ketoacid decarboxylase functional variant, or a branched chain ketoacid decarboxylase functional fragment are well known to one of ordinary skill in the art. For example, branched chain ketoacid decarboxylase activity can be assessed by expressing the protein, functional variant, or fragment thereof, in a recombinant bacterial cell that lacks endogenous branched chain ketoacid decarboxylase activity. Also, branched chain ketoacid decarboxylase activity can be assessed using the coupled enzymatic assay method as described by Zhang *et al.* (see, for example, Zhang et al., Proc. Natl. Acad. Sci., 105(52):20653-58, 2008). Alpha-ketoisovalerate decarboxylase activity can also be assessed *in vitro* by measuring the disappearance of alpha-ketoisovalerate as described by de la Plaza (see, for example, de la Plaza et al., FEMS Microbiol. Letters, 2004, 238(2):367-374).

In some embodiments, the gene encoding a branched chain ketoacid decarboxylase , *e.g., kivD,* is mutagenized; mutants exhibiting increased activity are selected; and the mutagenized gene encoding the α-ketoisovalerate decarboxylase, *e.g., kivD*, is isolated and inserted into the bacterial cell. The gene comprising the modifications described herein may be present on a plasmid or chromosome.

Accordingly, in some embodiments, the *kivD* gene has at least about 80% identity with the entire sequence of SEQ ID NO:1. Accordingly, in one embodiment, the *kivD* gene has at least about 90% identity with the entire sequence of SEQ ID NO:1. Accordingly, in one embodiment, the *kivD* gene has at least about 95% identity with the entire sequence of SEQ ID NO:1. Accordingly, in one embodiment, the *kivD* gene has at least about 85%, 86%, 87%, 88%, 89%, 90%, 91%, 92%, 93%, 94%, 95%, 96%, 97%, 98%, or 99% identity with the entire sequence of SEQ ID NO:1. In another embodiment, the *kivD* gene comprises the sequence of SEQ ID NO:1. In yet another embodiment, the *kivD* gene consists of the sequence of SEQ ID NO:1.

In other embodiments, the engineered bacteria comprise gene sequence(s) encoding at least one branched chain ketoacid decarboxylase enzyme and further comprise gene sequence encoding one or more polypeptides selected from other branched chain amino acid catabolism enzyme(s), BCAA transporter(s), and BCAA binding protein(s). Thus, in some embodiments, the at least one branched chain ketoacid decarboxylase enzyme is coexpressed with an additional branched chain amino acid catabolism enzyme, *e.g.,* a branched chain amino acid dehydrogenase, amino acid oxidase (also known as amino acid deaminase), and/or aminotransferase. In some embodiments, the at least one α-ketoisovalerate decarboxylase gene is coexpressed with a leucine dehydrogenase, e.g., *(leuDH or ldh)*, described in more detail below. In other embodiments, the at least one branched chain ketoacid decarboxylase gene is coexpressed with a branched chain amino acid aminotransferase, *e.g., ilvE,* described in more detail below. In other embodiments, the at least one branched chain ketoacid decarboxylase gene is coexpressed with an amino acid deaminase, *e.g., L-AAD*, described in more detail below. In some embodiments, the engineered bacteria comprise gene sequence(s) encoding at least one branched chain ketoacid decarboxylase enzyme and gene sequence(s) encoding one or more branched chain amino acid dehydrogenase(s) (e.g., leuDH). In some embodiments, the engineered bacteria comprise gene sequence(s) encoding at least one branched chain ketoacid decarboxylase enzyme and gene sequence(s) encoding one or more amino acid oxidase(s) (e.g. L-AAD)). In some embodiments, the engineered bacteria comprise gene sequence(s) encoding at least one branched chain ketoacid decarboxylase enzyme and gene sequence(s) encoding one or more aminotransferase(s) (e.g., ilvE).

In some embodiments, the at least one branched chain ketoacid decarboxylase enzyme is coexpressed with an aldehyde dehydrogenase, e.g., padA, described in more detail below. In some embodiments, the at least one branched chain ketoacid decarboxylase enzyme is coexpressed with an alcohol dehydrogenase, e.g., adh2, yqhD, described in more detail below. In some embodiments, the engineered bacteria comprise gene sequence(s) encoding at least one branched chain ketoacid decarboxylase enzyme and gene sequence(s) encoding one or more aldehyde dehydrogenase(s) (e.g., padA)). In some embodiments, the engineered bacteria comprise gene sequence(s) encoding at least one branched chain ketoacid decarboxylase enzyme and gene sequence(s) encoding one or more alcohol dehydrogenase(s) (e.g., adh2, yqhD).

In some embodiments, the at least one α-ketoisovalerate decarboxylase gene is coexpressed with a leucine dehydrogenase, *e.g., (leuDH or ldh)* and an aldehyde dehydrogenase, e.g., padA and/or an alcohol dehydrogenase, e.g., adh2, yqhD. In other embodiments, the at least one α-ketoisovalerate decarboxylase gene is coexpressed with a branched chain amino acid aminotransferase, *e.g., ilvE* and an aldehyde dehydrogenase, e.g., padA and/or an alcohol dehydrogenase, e.g., adh2, yqhD. In other embodiments, the at least one α-ketoisovalerate decarboxylase gene is coexpressed with an amino acid deaminase, e.g., *L-AAD* and an aldehyde dehydrogenase, e.g., padA and/or an alcohol dehydrogenase, e.g., adh2, yqhD. In some embodiments, the engineered bacteria comprise gene sequence(s) encoding at least one branched chain ketoacid decarboxylase enzyme and gene sequence(s) encoding one or more leucine dehydrogenase(s), *e.g., (leuDH),* gene sequence encoding one or more aldehyde dehydrogenase(s) (e.g., padA) and/or gene sequence encoding one or more alcohol dehydrogenases (e.g., adh2, yqhD). In some embodiments, the engineered bacteria comprise gene sequence(s) encoding at least one branched chain ketoacid decarboxylase enzyme and gene sequence(s) encoding one or more branched chain amino acid aminotransferase, *(e.g., ilvE),* gene sequence encoding one or more aldehyde dehydrogenase(s) (e.g., padA) and/or gene sequence encoding one or more alcohol dehydrogenases (e.g., adh2, yqhD). In some embodiments, the engineered bacteria comprise gene sequence(s) encoding at least one branched chain ketoacid decarboxylase enzyme and gene sequence(s) encoding one or more an amino acid deaminase, *e.g., L-AAD,* gene sequence encoding one or more aldehyde dehydrogenase(s) (e.g., padA) and/or gene sequence encoding one or more alcohol dehydrogenases (e.g., adh2, yqhD).

In some embodiments, the at least one branched chain ketoacid decarboxylase enzyme is coexpressed with one or more BCAA transporter(s), for example, a high affinity leucine transporter, e.g., LivKHMGF or low affinity BCAA transporter BrnQ. In some embodiments, the at least one branched chain ketoacid decarboxylase enzyme is coexpressed with one or more BCAA binding protein(s), for example, LivJ. In some embodiments, the engineered bacteria comprise gene sequence(s) encoding at least one branched chain ketoacid decarboxylase enzyme and gene sequence(s) encoding one or more BCAA transporter(s) (e.g., LivKHMGF, brnQ). In some embodiments, the engineered bacteria comprise gene sequence(s) encoding at least one branched chain ketoacid decarboxylase enzyme and gene sequence(s) encoding one or more BCAA binding protein(s) (e.g., livJ). In some embodiments, the engineered bacteria comprise gene sequence(s) encoding at least one branched chain ketoacid decarboxylase enzyme, gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain ketoacid decarboxylase enzyme and genetic modification that reduces export of a branched chain amino acid, *e.g.,* a genetic mutation in a *leuE* gene or promoter thereof. In one embodiment, the engineered bacteria comprise gene sequence(s) encoding at least one branched chain ketoacid decarboxylase enzyme and a genetic modification that reduces or eliminates branched chain amino acid synthesis, e.g., a genetic mutation in a *ilvC* gene or promoter thereof.

In some embodiments, the gene sequence(s) encoding the one or more branched chain ketoacid decarboxylase enzyme(s) is expressed under the control of a constitutive promoter. In some embodiments, the gene sequence(s) encoding the one or more branched chain ketoacid decarboxylase enzyme(s) is expressed under the control of an inducible promoter. In some embodiments, the gene sequence(s) encoding the one or more branched chain ketoacid decarboxylase enzyme(s) is expressed under the control of a promoter that is directly or indirectly induced by exogenous environmental conditions. In some embodiments, the gene sequence(s) encoding the one or more branched chain ketoacid decarboxylase enzyme(s) is expressed under the control of a promoter that is directly or indirectly induced by low-oxygen or anaerobic conditions, wherein expression of the gene encoding the branched chain ketoacid decarboxylase enzyme is activated under low-oxygen or anaerobic environments, such as the environment of the mammalian gut. In some embodiments, the gene sequence(s) encoding the one or more branched chain ketoacid decarboxylase enzyme(s) is expressed under the control of a promoter that is directly or indirectly induced by inflammatory conditions. Exemplary inducible promoters described herein include oxygen level-dependent promoters (e.g., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

### B. Branched Chain Keto Acid Dehydrogenase (BCKD)

In one embodiment, the branched chain amino acid catabolism enzyme is a branched chain keto acid dehydrogenase ("BCKD"). Thus, in some embodiments, the engineered bacteria comprise gene sequence(s) encoding one or more copies of a branched chain keto acid dehydrogenase. In some embodiments, the engineered bacteria comprise gene sequence(s) encoding one, two, three, four, five, six, or more copies of a branched chain keto acid dehydrogenase. The one or more copies of branched chain keto acid dehydrogenase can be one or more copies of the same gene or can be different genes encoding branched chain keto acid dehydrogenase, e.g., gene(s) from a different species or otherwise having a different gene sequence. The one or more copies of branched chain keto acid dehydrogenase can be present in the bacterial chromosome or can be present in one or more plasmids. As used herein "branched chain keto acid dehydrogenase" or "BCKD" refers to any polypeptide having enzymatic activity that oxidatively decarboxylates a branched chain keto acid into its respective acyl-CoA derivative. Multiple distinct branched chain keto acid dehydrogenases are known in the art and are available from many microorganism sources, including those disclosed herein, as well as eukaryotic sources, including mammalian sources, e.g. human. In bacteria, branched chain keto acid dehydrogenases are enzyme complexes that oxidatively decarboxylate all three branched chain keto acids (α-ketoisocaproate, α-keto-β-methylvalerate, and α-ketoisovalerate) into their respective acyl-CoA derivatives, (isovaleryl-CoA, α-methylbutyryl-CoA, isobutyryl-CoA). See, for example, Massey et al., Bacteriol Rev., 40(1):42-54, 1976. Moreover, in mammals, dehydrogenases specific for 2-ketoisovalerate (EC 1.2.4.4) and 2-keto-3-methylvalerate and 2-keto-isocaproate (EC 1.2.4.3) have been identified (see, for example, Massey et al., Bacteriol Rev., 40(1):42-54, 1976). In one embodiment, the branched chain amino acid catabolism enzyme is a leucine catabolism enzyme.

In some embodiments, the branched chain keto acid dehydrogenase is encoded by at least one gene encoding a branched chain keto acid dehydrogenase derived from a bacterial species. In some embodiments, the branched chain keto acid dehydrogenase is encoded by at least one gene encoding a branched chain keto acid dehydrogenase derived from a non-bacterial species. In some embodiments, the branched chain keto acid dehydrogenase is encoded by at least one gene derived from a eukaryotic species, *e.g.,* a yeast species or a plant species. In another embodiment, the branched chain keto acid dehydrogenase is encoded by at least one gene derived from a mammalian species, e.g., human.

In one embodiment, the at least one gene encoding the branched chain keto acid dehydrogenase is derived from an organism of the genus or species that includes, but is not limited to, *Acetinobacter, Azospirillum*, *Bacillus, Bacteroides, Bifidobacterium, Brevibacteria, Burkholderia, Citrobacter, Clostridium, Corynebacterium, Cronobacter, Enterobacter, Enterococcus, Erwinia, Helicobacter, Klebsiella, Lactobacillus, Lactococcus, Leishmania, Listeria, Macrococcus, Mycobacterium, Nakamurella*, *Nasonia, Nostoc, Pantoea, Pectobacterium, Proteus, Pseudomonas, Psychrobacter, Ralstonia, Saccharomyces, Salmonella, Sarcina, Serratia, Staphylococcus, Streptococcus, and Yersinia, e.g., Acetinobacter radioresistens, Acetinobacter baumannii, Acetinobacter calcoaceticus, Azospirillum brasilense*, *Bacillus anthracis, Bacillus cereus, Bacillus coagulans, Bacillus megaterium, Bacillus subtilis, Bacillus thuringiensis, Bacteroides fragilis, Bacteroides subtilis, Bacteroides thetaiotaomicron, Bifidobacterium bifidum*, *Bifidobacterium infantis, Bifidobacterium lactis, Bifidobacterium longum, Burkholderia xenovorans, Citrobacter youngae, Citrobacter koseri, Citrobacter rodentium, Clostridium acetobutylicum, Clostridium butyricum*, *Corynebacterium aurimucosum, Corynebacterium kroppenstedtii, Corynebacterium striatum, Cronobacter sakazakii*, *Cronobacter turicensis, Enterobacter cloacae, Enterobacter cancerogenus, Enterococcus faecium, Enterococcus faecalis, Erwinia amylovara*, *Erwinia pyrifoliae, Erwinia tasmaniensis, Helicobacter mustelae, Klebsiella pneumonia, Klebsiella variicola, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, Leishmania infantum, Leishmania major, Leishmania brazilensis, Listeria grayi, Macrococcus caseolyticus, Mycobacterium avium, Mycobacterium intracellulare, Mycobacterium kansasii*, *Mycobacterium leprae, Mycobacterium marinum, Mycobacterium smegmatis, Mycobacterium tuberculosis, Mycobacterium ulcerans, Nakamurella multipartita*, *Nasonia vitipennis*, *Nostoc punctiforme, Pantoea ananatis*, *Pantoea agglomerans, Pectobacterium atrosepticum, Pectobacterium carotovorum, Pseudomonas putida, Pseudomonas aeruginosa, Psychrobacter articus, Proteus vulgaris, Proteus mirabilis, Psychrobacter cryohalolentis*, *Ralstonia eutropha, Saccharomyces boulardii, Salmonella enterica, Sarcina ventriculi, Serratia odorifera*, *Serratia proteamaculans, Staphylococcus aerus, Staphylococcus capitis, Staphylococcys carnosus, Staphylococcus epidermidis, Staphylococcus hominis, Staphylococcus haemolyticus, Staphylococcus lugdunensis, Staphylococcus saprophyticus, Staphylococcus warneri, Streptococcus faecalis, Yersinia enterocolitica, Yersinia mollaretii*, *Yersinia kristensenii, Yersinia rohdei*, *and Yersinia aldovae.* In some embodiments, the BCKD is encoded by at least one gene derived from *Pseudomonas putida.* In another embodiment, the BCKD is encoded by at least one gene derived from *Pseudomonas aeruginosa.* In another embodiment, the BCKD is encoded by at least one gene derived from *Streptococcus faecalis.* In another embodiment, the BCKD is encoded by at least one gene derived from *Proteus vulgaris.* In another embodiment, the BCKD is encoded by at least one gene derived from *Bacillus subtilis.* In another embodiment, the BCKD is encoded by at least one gene derived from *Streptococcus faecalis.* In another embodiment, the BCKD is encoded by at least one gene derived from *Bacillus subtilis.*

In some embodiments, the at least one gene encoding the branched chain keto acid dehydrogenase has been codon-optimized for use in the recombinant bacterial cell. In one embodiment, the at least one gene encoding the branched chain keto acid dehydrogenase has been codon-optimized for use in *Escherichia coli.* In one embodiment, the at least one gene encoding the branched chain keto acid dehydrogenase is a branched chain keto acid dehydrogenase gene from *Pseudomonas aeruginosa* PAO1. In one embodiment, the at least one gene encoding the branched chain keto acid dehydrogenase comprises the *bkdA1-bkdA2-bkdB-lpdVoperon.* In one embodiment, the *bkdA1-bkdA2-bkdB-lpdV* operon is at least 90% identical to the uppercase sequence set forth in SEQ ID NO:3. In another embodiment, the *bkdAl-bkdA2-bkdB-lpdV operon* comprises the uppercase sequence set forth in SEQ ID NO:3. In another embodiment, the at least one gene encoding the branched chain keto acid dehydrogenase comprises the *LeuDH-bkdA1-bkdA2-bkdB-lpdV* operon. In one embodiment, the *LeuDH-bkdA1-bkdA2-bkdB-lpdV* operon is at least 90% identical to the uppercase sequence set forth in SEQ ID NO:4. In another embodiment, the *LeuDH-bkdA1-bkdA2-bkdB-lpdV* operon comprises the uppercase sequence as set forth in SEQ ID NO:4. In another embodiment, the at least one gene encoding is Alpha-ketoisovalerate dehydrogenase (EC 1.2.4.4). In another embodiment, the at least one gene encoding the branched chain keto acid dehydrogenase is 2-oxoisocaproate dehydrogenase (EC 1.2.4.3). In yet another embodiment, the at least one gene encoding the branched chain keto acid dehydrogenase is the human dehydrogenase/decarboxylase (E1). In another embodiment, the at least one gene encoding the branched chain keto acid dehydrogenase comprises the human E1α and two E1β subunits. In another embodiment, the at least one gene encoding the branched chain keto acid dehydrogenase comprises the human dihydrolipoyl transacylase (E2) gene. In yet another embodiment, the at least one gene encoding the branched chain keto acid dehydrogenase comprises the human dihydrolipoamide dehydrogenase (E3) gene. In another embodiment, the at least one gene encoding the branched chain keto acid dehydrogenase comprises the human dehydrogenase/decarboxylase (E1) gene, the human dihydrolipoly transacylase (E2) gene, and the human dihydrolipoamide dehydrogenase (E3) gene.

When a branched chain amino acid catabolism enzyme is expressed in the recombinant bacterial cells disclosed herein, the bacterial cells catabolize more branched chain amino acid, *e.g.,* leucine, than unmodified bacteria of the same bacterial subtype under the same conditions (e.g., culture or environmental conditions). Thus, the genetically engineered bacteria comprising at least one heterologous gene encoding a branched chain keto acid dehydrogenase may be used to catabolize excess branched chain amino acids, *e.g.,* leucine, to treat a disease associated with the catabolism of a branched chain amino acid, including Maple Syrup Urine Disease (MSUD). In some embodiments, the branched chain keto acid dehydrogenase is co-expressed with an additional branched chain amino acid dehydrogenase, *e.g.,* a leucine dehydrogenase, *e.g., leuDH,* described in more detail below.

The present disclosure further comprises genes encoding functional fragments of a branched chain keto acid dehydrogenase or functional variants of branched chain keto acid dehydrogenase. As used herein, the term "functional fragment thereof' or "functional variant thereof' of branched chain keto acid dehydrogenase relates to a sequence having qualitative biological activity in common with the wild-type branched chain keto acid dehydrogenase from which the fragment or variant was derived. For example, a functional fragment or a functional variant of a mutated branched chain keto acid dehydrogenase protein is one which retains essentially the same ability to catabolize leucine or other BCAA as the protein from which the functional fragment or functional variant was derived. For example, a polypeptide having branched chain keto acid dehydrogenase activity may be truncated at the N-terminus or C-terminus and the retention of enzyme activity assessed using assays known to those of skill in the art, including the exemplary assays provided herein. In one embodiment, the recombinant bacterial cell disclosed herein comprises a heterologous gene encoding a branched chain keto acid dehydrogenase functional variant. In another embodiment, the recombinant bacterial cell disclosed herein comprises a heterologous gene encoding a branched chain keto acid dehydrogenase functional fragment.

Assays for testing the activity of a branched chain keto acid dehydrogenase, a branched chain keto acid dehydrogenase functional variant, or a branched chain keto acid dehydrogenase functional fragment are well known to one of ordinary skill in the art. For example, branched chain keto acid dehydrogenase activity can be assessed by expressing the protein, functional variant, or fragment thereof, in a recombinant bacterial cell that lacks endogenous branched chain keto acid dehydrogenase activity. Also, activity can be assessed using the enzymatic assay methods as described by Sykes et al. (J. Bacterial., 169(4):1619-1625, 1987), Sokatch et al. (J. Bacteriol., 148:639-646, 1981), and Massey et al. (Bacterial. Rev., 40(1):42-54, 1976).

The present disclosure encompasses genes encoding a branched chain keto acid dehydrogenase comprising amino acids in its sequence that are substantially the same as an amino acid sequence described herein. In some embodiments, the at least one gene encoding a branched chain keto acid dehydrogenase is mutagenized, mutants exhibiting increased activity are selected, and the mutagenized gene(s) encoding the branched chain keto acid dehydrogenase are isolated and inserted into the bacterial cell. In some embodiments, the at least one gene encoding a branched chain keto acid dehydrogenase is mutagenized, mutants exhibiting decreased activity are selected, and the mutagenized gene(s) encoding the branched chain keto acid dehydrogenase are isolated and inserted into the bacterial cell. The gene comprising the modifications described herein may be present on a plasmid or chromosome.

In one embodiment, the at least one gene encoding the branched chain keto acid dehydrogenase comprises the *bkdA1-bkdA2-bkdB-lpdV* operon. In one embodiment, the at least one BCKD gene has at least about 80% identity with the entire uppercase sequence of SEQ ID NO:3. Accordingly, in one embodiment, the at least one BCKD gene has at least about 90% identity with the entire uppercase sequence of SEQ ID NO:3. Accordingly, in one embodiment, the at least one BCKD gene has at least about 95% identity with the entire uppercase sequence of SEQ ID NO:3. Accordingly, in one embodiment, the at least one BCKD gene has at least about 85%, 86%, 87%, 88%, 89%, 90%, 91%, 92%, 93%, 94%, 95%, 96%, 97%, 98%, or 99% identity with the entire uppercase sequence of SEQ ID NO:3. In another embodiment, the at least one BCKD gene comprises the uppercase sequence of SEQ ID NO:3. In yet another embodiment the at least one BCKD gene consists of the uppercase sequence of SEQ ID NO:3.

In another embodiment, the at least one BCKD gene is coexpressed with an additional branched chain amino acid dehydrogenase. In one embodiment, the at least one BCKD gene is coexpressed with a leucine dehydrogenase, *e.g., leuDH.* In another embodiment, the at least one gene encoding the branched chain keto acid dehydrogenase comprises the *leuDH-bkdA1-bkdA2-bkdB-lpdV* operon. In one embodiment, the at least one BCKD gene has at least about 80% identity with the entire uppercase sequence of SEQ ID NO:4. Accordingly, in one embodiment, the at least one BCKD gene has at least about 90% identity with the entire uppercase sequence of SEQ ID NO:4. Accordingly, in one embodiment, the at least one BCKD gene has at least about 95% identity with the entire uppercase sequence of SEQ ID NO:4. Accordingly, in one embodiment, the at least one BCKD gene has at least about 85%, 86%, 87%, 88%, 89%, 90%, 91%, 92%, 93%, 94%, 95%, 96%, 97%, 98%, or 99% identity with the entire uppercase sequence of SEQ ID NO:4. In another embodiment, the at least one BCKD gene comprises the uppercase sequence of SEQ ID NO:4. In yet another embodiment the at least one BCKD gene consists of the uppercase sequence of SEQ ID NO:4. In another embodiment, the at least one BCKD gene is coexpressed with a branched chain amino acid aminotransferase, *e.g., ilvE*, described in more detail below.

In other embodiments, the engineered bacteria comprise gene sequence(s) encoding at least one branched chain keto acid dehydrogenase enzyme and further comprise gene sequence encoding one or more polypeptides selected from other branched chain amino acid catabolism enzyme(s), BCAA transporter(s), and BCAA binding protein(s). Thus, in some embodiments, the at least one branched chain keto acid dehydrogenase enzyme is coexpressed with an additional branched chain amino acid catabolism enzyme, *e.g.,* a branched chain amino acid dehydrogenase, amino acid oxidase (also known as amino acid deaminase), and/or aminotransferase. In some embodiments, the at least one branched chain keto acid dehydrogenase gene is coexpressed with a leucine dehydrogenase, *e.g.,* (*leuDH*)*,* described in more detail below. In other embodiments, the at least one branched chain keto acid dehydrogenase gene is coexpressed with a branched chain amino acid aminotransferase, *e.g., ilvE,* described in more detail below. In other embodiments, the at least one branched chain keto acid dehydrogenase is coexpressed with an amino acid deaminase, *e.g., L-AAD*, described in more detail below. In some embodiments, the engineered bacteria comprise gene sequence(s) encoding at least one branched chain keto acid dehydrogenase enzyme and gene sequence(s) encoding one or more branched chain amino acid dehydrogenase(s) (e.g., leuDH). In some embodiments, the engineered bacteria comprise gene sequence(s) encoding at least one branched chain keto acid dehydrogenase enzyme and gene sequence(s) encoding one or more amino acid oxidase(s) (e.g. L-AAD)). In some embodiments, the engineered bacteria comprise gene sequence(s) encoding at least one branched chain keto acid dehydrogenase enzyme and gene sequence(s) encoding one or more aminotransferase(s) (e.g., ilvE).

In some embodiments, the at least one branched chain keto acid dehydrogenase enzyme is coexpressed with one or more BCAA transporter(s), for example, a high affinity leucine transporter, e.g., LivKHMGF or low affinity BCAA transporter BrnQ. In some embodiments, the at least one branched chain keto acid dehydrogenase enzyme is coexpressed with one or more BCAA binding protein(s), for example, LivJ. In some embodiments, the engineered bacteria comprise gene sequence(s) encoding at least one branched chain keto acid dehydrogenase enzyme and gene sequence(s) encoding one or more BCAA transporter(s) (e.g., LivKHMGF, brnQ). In some embodiments, the engineered bacteria comprise gene sequence(s) encoding at least one branched chain keto acid dehydrogenase enzyme and gene sequence(s) encoding one or more BCAA binding protein(s) (e.g., livJ). In some embodiments, the engineered bacteria comprise gene sequence(s) encoding at least one branched chain keto acid dehydrogenase enzyme, gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain keto acid dehydrogenase enzyme and genetic modification that reduces export of a branched chain amino acid, *e.g.,* a genetic mutation in a *leuE* gene or promoter thereof. In one embodiment, the engineered bacteria comprise gene sequence(s) encoding at least one branched chain keto acid dehydrogenase enzyme and a genetic modification that reduces or eliminates branched chain amino acid synthesis, e.g., a genetic mutation in a *ilvC* gene or promoter thereof.

In some embodiments, the gene sequence(s) encoding the one or more branched chain keto acid dehydrogenase enzyme(s) is expressed under the control of a constitutive promoter. In some embodiments, the gene sequence(s) encoding the one or more branched chain keto acid dehydrogenase enzyme(s) is expressed under the control of an inducible promoter. In some embodiments, the gene sequence(s) encoding the one or more branched chain keto acid dehydrogenase enzyme(s) is expressed under the control of a promoter that is directly or indirectly induced by exogenous environmental conditions. In some embodiments, the gene sequence(s) encoding the one or more branched chain keto acid dehydrogenase enzyme(s) is expressed under the control of a promoter that is directly or indirectly induced by low-oxygen or anaerobic conditions, wherein expression of the gene encoding the branched chain amino acid catabolism enzyme is activated under low-oxygen or anaerobic environments, such as the environment of the mammalian gut. In some embodiments, the gene sequence(s) encoding the one or more branched chain keto acid dehydrogenase enzyme(s) is expressed under the control of a promoter that is directly or indirectly induced by inflammatory conditions. Exemplary inducible promoters described herein include oxygen level-dependent promoters (e.g., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

### C. Branched Chain Amino Acid Deamination Enzymes

In one embodiment, the branched chain amino acid catabolism enzyme is a branched chain amino acid deamination enzyme. Thus, in some embodiments, the engineered bacteria comprise gene sequence(s) encoding one or more copies of a branched chain amino acid deamination enzyme. In some embodiments, the engineered bacteria comprise gene sequence(s) encoding one, two, three, four, five, six, or more copies of a branched chain amino acid deamination enzyme. The one or more copies of branched chain amino acid deamination enzyme can be one or more copies of the same gene or can be different genes encoding branched chain amino acid deamination enzyme, e.g., gene(s) from a different species or otherwise having a different gene sequence. The one or more copies of branched chain amino acid deamination enzyme can be present in the bacterial chromosome or can be present in one or more plasmids. As used herein, the term "branched chain amino acid deamination enzyme" refers to an enzyme involved in the deamination, or the removal of an amine group, of a branched chain amino acid, which produces a corresponding branched chain alpha-keto acid (e.g., α-ketoisocaproate, α-keto-βmethylvalerate, α-ketoisovalerate). Enzymes involved in the deamination of branched chain amino acids are well known to those of skill in the art. For example, in bacteria, leucine dehydrogenase (LeuDH, *leuDH*)*,* e.g., derived from *Pseudomonas aeruginosa* PA01, is capable of catalyzing the reversible deamination of branched chain amino acids, such as leucine, into their corresponding keto-acid counterpart (Baker et al., Structure,, 3(7):693-705, 1995). Similarly, the *ilvE* gene from *E. coli* Nissle has also been shown to catalyze the reversible deamination of branched chain amino acids (Peng et al., J. Bact., 139(2):339-45, 1979; Kline et al., J. Bact., 130(2):951-3, 1977). The L-AAD gene, e.g., derived from Proteus vulgaris or Proteus mirabilis, has also been shown to catalyze the irreversible deamination of branched chain amino acids (Song et al., Scientific Reports, Nature, 5:12694; DOI: 10:1038/srep12694 (2015)).

In one embodiment, the branched chain amino acid deamination enzyme increases the rate of branched chain amino acid deamination in the cell. In one embodiment, the branched chain amino acid deamination enzyme decreases the level of branched chain amino acid in the cell as compared to the level of its corresponding alpha-keto acid in the cell. In another embodiment, the branched chain amino acid deamination enzyme increases the level of alpha-keto acid in the cell as compared to the level of its corresponding branched chain amino acid in the cell.

In one embodiment, the branched chain amino acid deamination enzyme is a leucine deamination enzyme. In another embodiment, the branched chain amino acid deamination enzyme is an isoleucine deamination enzyme. In another embodiment, the branched chain amino acid deamination enzyme is a valine deamination enzyme. In another embodiment, the branched chain amino acid deamination enzyme is involved in the deamination of leucine, isoleucine, and valine. In another embodiment, the branched chain amino acid deamination enzyme is involved in the deamination of leucine and valine, isoleucine and valine, or leucine and isoleucine. In some embodiments, the branched chain amino acid deamination enzyme is encoded by a branched chain amino acid deamination enzyme gene derived from a bacterial species. In some embodiments, the branched chain amino acid deamination enzyme is encoded by a branched chain amino acid deamination enzyme gene derived from a non-bacterial species. In some embodiments, the branched chain amino acid deamination enzyme is encoded by a branched chain amino acid deamination enzyme gene derived from a eukaryotic species, *e.g.,* a yeast species or a plant species. In another embodiment, the branched chain amino acid deamination enzyme is encoded by a branched chain amino acid deamination enzyme gene derived from a mammalian species, e.g., human.

In other embodiments, the engineered bacteria comprise gene sequence(s) encoding at least one branched chain amino acid deamination enzyme and further comprise gene sequence encoding one or more polypeptides selected from other branched chain amino acid catabolism enzyme(s), BCAA transporter(s), and BCAA binding protein(s). Thus, in some embodiments, the at least one branched chain amino acid deamination enzyme is coexpressed with another branched chain amino acid deamination enzyme. In some embodiments, the at least one branched chain amino acid deamination enzyme is coexpressed with another branched chain amino acid catabolism enzyme, *e.g.,* a ketoacid decarboxylase, such as KivD. In some embodiments, the at least one branched chain amino acid deamination enzyme is coexpressed with an aldehyde dehydrogenase, e.g., PadA, described in more detail below. In some embodiments, the at least one branched chain amino acid deamination enzyme is coexpressed with an alcohol dehydrogenase, e.g., Adh2, YqhD, described in more detail below. In some embodiments, the at least one branched chain amino acid deamination enzyme is coexpressed with one or more BCAA transporter(s), for example, a high affinity leucine transporter, e.g., LivKHMGF (SEQ ID NO: 91) or low affinity BCAA transporter BrnQ (SEQ ID NO: 64). In some embodiments, the at least one branched chain amino acid deamination enzyme is coexpressed with one or more BCAA binding protein(s), for example, LivJ (SEQ ID NO: 12). In some embodiments, the engineered bacteria comprise gene sequence(s) encoding at least one branched chain amino acid deamination enzyme and genetic modification that reduces export of a branched chain amino acid, *e.g.,* a genetic mutation in a *leuE* gene or promoter thereof. In one embodiment, the engineered bacteria comprise gene sequence(s) encoding at least one branched chain amino acid deamination enzyme and a genetic modification that reduces or eliminates branched chain amino acid synthesis, *e.g.,* a genetic mutation in a *ilvC* gene or promoter thereof.

In some embodiments, the gene sequence(s) encoding the one or more branched chain amino acid deamination enzyme(s) is expressed under the control of a constitutive promoter. In some embodiments, the gene sequence(s) encoding the one or more branched chain amino acid deamination enzyme(s) is expressed under the control of an inducible promoter. In some embodiments, the gene sequence(s) encoding the one or more branched chain amino acid deamination enzyme(s) is expressed under the control of a promoter that is directly or indirectly induced by exogenous environmental conditions. In some embodiments, the gene sequence(s) encoding the one or more branched chain amino acid deamination enzyme(s) is expressed under the control of a promoter that is directly or indirectly induced by low-oxygen or anaerobic conditions, wherein expression of the gene encoding the branched chain amino acid deamination enzyme is activated under low-oxygen or anaerobic environments, such as the environment of the mammalian gut. In some embodiments, the gene sequence(s) encoding the one or more branched chain amino acid deamination enzyme(s) is expressed under the control of a promoter that is directly or indirectly induced by inflammatory conditions. Exemplary inducible promoters described herein include oxygen level-dependent promoters (*e.g.,* FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

Non-limiting examples of branched chain amino acid deamination enzymes include leucine dehydrogenase, L-amino acid deaminase, and branched chain amino acid aminotransferase, and are described in more detail in the subsections, below.

### (1) Branched chain amino acid dehydrogenases (Leucine Dehydrogenase)

In some embodiment, the branched chain amino acid deamination enzyme is a branch chain amino acid dehydrogenase. Thus, in some embodiments, the engineered bacteria comprise gene sequence(s) encoding one or more copies of a branch chain amino acid dehydrogenase. In some embodiments, the engineered bacteria comprise gene sequence(s) encoding one, two, three, four, five, six, or more copies of a branch chain amino acid dehydrogenase. The one or more copies of branch chain amino acid dehydrogenase can be one or more copies of the same gene or can be different genes encoding branch chain amino acid dehydrogenase, e.g., gene(s) from a different species or otherwise having a different gene sequence. The one or more copies of branch chain amino acid dehydrogenase can be present in the bacterial chromosome or can be present in one or more plasmids.

In some embodiments, the branched chain amino acid deamination enzyme is leucine dehydrogenase ("*leuDH*" or "leuDH"). As used herein "leucine dehydrogenase" refers to any polypeptide having enzymatic activity that deaminates leucine to its corresponding ketoacid, alpha-ketoisocaproate (KIC), deaminates valine to its corresponding ketoacid, ketoisovalerate (KIV), and deaminates isoleucine to its corresponding ketoacid, ketomethylvalerate (KMV). In some embodiments, the bacterial cells disclosed herein comprise a heterologous gene encoding a leucine dehydrogenase enzyme and are capable of converting leucine, valine, and/or isoleucine to their respective α-keto acids. For example, the leucine dehydrogenase enzyme LeuDH is capable of metabolizing leucine and a cytosolically active LeuDH should generally exhibit the ability to convert valine, isoleucine, and leucine to ketoisovalerate, ketomethylvalerate, and ketoisocaproate, respectively. Leucine dehydrogenase employs the co-factor NAD+. In some embodiments, leuDH encodes an octamer.

Multiple distinct leucine dehydrogenases (EC 1.4.1.9) are known in the art and are available from many microorganism sources, including those disclosed herein, as well as from eukaryotic sources (see, for example, Baker et al., Structure,, 3(7):693-705, 1995). In some embodiments, the branched chain amino acid deamination enzyme is encoded by at least one gene encoding a branched chain amino acid deamination enzyme derived from a bacterial species. In some embodiments, the branched chain amino acid deamination enzyme is encoded by at least one gene encoding a branched chain amino acid deamination enzyme derived from a non-bacterial species. In some embodiments, the branched chain amino acid deamination enzyme is encoded by at least one gene derived from a eukaryotic species, *e.g*., a yeast species or a plant species. In another embodiment, the branched chain amino acid deamination enzyme is encoded by at least one gene derived from a mammalian species, e.g., human.

In one embodiment, the engineered bacteria comprise gene sequence(s) encoding one or more branch chain amino acid dehydrogenase(s), e.g., leucine dehydrogenase enzyme(s). In some embodiments, the branch chain amino acid dehydrogenase, e.g., leucine dehydrogenase enzyme is derived from an organism of the genus or species that includes, but is not limited to, *Bacillus, Brevibacillus, Geobacillus, Lysinibacillus, Moorella, Natrialba, Pseudomonas, Sporosarcinia, and Thermoactinomyces.* In some embodiments, the *leuDH* gene is encoded by a gene derived from *Bacillus caldolyticus, Bacillus cereus, Bacillus licheniformis, Bacillus megaterium, Bacillus mycoides, Bacillus niger, Bacillus pumilus, Bacillus subtilis, Brevibacillus brevis, Geobacillus stearothermophilus, Lysinibacillus sphaeriscus, Moorella Thermoacetica, Natrialba magadii, Sporosarcina psychorophila, Thermoactinomyces intermedius, Pseudomonas aeruginosa,* or *Pseudomonas resinovorans.* In some embodiments, the branch chain amino acid dehydrogenase enzyme, e.g., leucine dehydrogenase, is encoded by at least one gene derived from *Pseudomonas aeruginosa* PA01. In some embodiments, the branch chain amino acid dehydrogenase enzyme, e.g., leucine dehydrogenase, is encoded by at least one gene derived from *Bacillus cereus.* In some embodiments, the at least one gene encoding the branch chain amino acid dehydrogenase enzyme, e.g., leucine dehydrogenase, has been codon-optimized for use in the recombinant bacterial cell. In one embodiment, the at least one gene encoding the branch chain amino acid dehydrogenase enzyme, e.g., leucine dehydrogenase, has been codon-optimized for use in *Escherichia coli.* For example, a codon-optimized *LeuDH* sequence is set forth as SEQ ID NO: 20 and 58.

When a branch chain amino acid dehydrogenase enzyme, e.g., leucine dehydrogenase, is expressed in the recombinant bacterial cells disclosed herein, the bacterial cells catabolize more branched chain amino acid, *e.g*., leucine, isoleucine, and valine, than unmodified bacteria of the same bacterial subtype under the same conditions (*e.g*., culture or environmental conditions). Thus, the genetically engineered bacteria comprising at least one heterologous gene encoding a branch chain amino acid dehydrogenase enzyme, e.g., leucine dehydrogenase, may be used to catabolize excess branched chain amino acids, *e.g.,* leucine, valine, and isoleucine, to treat a disease associated with the deamination of a branched chain amino acid, including Maple Syrup Urine Disease (MSUD) as well as other disease provided herein.

The present disclosure further comprises genes encoding functional fragments of a branch chain amino acid dehydrogenase enzyme, e.g., leucine dehydrogenase, or functional variants of branch chain amino acid dehydrogenase enzyme, e.g., leucine dehydrogenase. The present disclosure encompasses genes encoding a branch chain amino acid dehydrogenase enzyme, e.g., leucine dehydrogenase, comprising amino acids in its sequence that are substantially the same as an amino acid sequence described herein. As used herein, the term "functional fragment thereof' or "functional variant thereof' of a branch chain amino acid dehydrogenase enzyme, e.g., a leucine dehydrogenase, gene relates to a sequence having qualitative biological activity in common with the wild-type branch chain amino acid dehydrogenase, e.g., a leucine dehydrogenase, from which the fragment or variant was derived. For example, a functional fragment or a functional variant of a mutated branch chain amino acid dehydrogenase protein, e.g., a leucine dehydrogenase, is one which retains essentially the same ability to catabolize BCAAs as the branch chain amino acid dehydrogenase protein, e.g., a leucine dehydrogenase, from which the functional fragment or functional variant was derived. For example, a polypeptide having branch chain amino acid dehydrogenase enzyme, e.g., a leucine dehydrogenase, activity may be truncated at the N-terminus or C-terminus and the retention of branch chain amino acid dehydrogenase enzyme, e.g., a leucine dehydrogenase, activity assessed using assays known to those of skill in the art, including the exemplary assays provided herein. In one embodiment, the recombinant bacterial cell disclosed herein comprises a heterologous gene encoding an α branch chain amino acid dehydrogenase enzyme, e.g., a leucine dehydrogenase, functional variant. In another embodiment, the recombinant bacterial cell disclosed herein comprises a heterologous gene encoding a branch chain amino acid dehydrogenase enzyme, e.g., a leucine dehydrogenase, functional fragment.

Assays for testing the activity of a branch chain amino acid dehydrogenase enzyme functional variant or functional fragment, e.g., a leucine dehydrogenase functional variant or a leucine dehydrogenase functional fragment are well known to one of ordinary skill in the art. For example, leucine dehydrogenase activity can be assessed by expressing the protein, functional variant, or fragment thereof, in a recombinant bacterial cell that lacks endogenous leucine dehydrogenase activity. Also, activity can be assessed using the enzymatic assay methods as described by Soda et al. (Biochem. Biophys. Res. Commun., 44:931, 1971), and Ohshima et al. (J. Biol. Chem., 255:5719, 1978).

In some embodiments, the at least one gene encoding a branch chain amino acid dehydrogenase enzyme, e.g., leucine dehydrogenase is mutagenized, mutants exhibiting increased activity are selected, and the mutagenized gene(s) encoding the branch chain amino acid dehydrogenase enzyme, e.g., leucine dehydrogenase, are isolated and inserted into the bacterial cell. In some embodiments, the at least one gene encoding a branch chain amino acid dehydrogenase enzyme, e.g., leucine dehydrogenase is mutagenized, mutants exhibiting decreased activity are selected, and the mutagenized gene(s) encoding the branch chain amino acid dehydrogenase enzyme, e.g., leucine dehydrogenase, are isolated and inserted into the bacterial cell. The gene comprising the modifications described herein may be present on a plasmid or chromosome.

In some embodiments, the engineered bacteria comprise gene sequence(s) encoding at least one branch chain amino acid dehydrogenase enzyme, e.g., leucine dehydrogenase, and further comprise gene sequence encoding one or more polypeptides selected from other branched chain amino acid catabolism enzyme(s), BCAA transporter(s), and BCAA binding protein(s). Thus, in some embodiments, the at least one branch chain amino acid dehydrogenase enzyme, e.g., leucine dehydrogenase, is coexpressed with an additional branched chain amino acid deamination enzyme, *e.g*., branched chain amino acid dehydrogenase, a branched chain aminotransferase, and/or amino acid oxidase (also known as amino acid deaminase). For example, in some embodiments, the branch chain amino acid dehydrogenase enzyme, e.g., leucine dehydrogenase, is coexpressed with one or more other branch chain amino acid dehydrogenase enzyme(s). In some embodiments, the branch chain amino acid dehydrogenase enzyme, e.g., leucine dehydrogenase, is coexpressed with one or more branched chain aminotransferase enzyme(s), for example, ilvE. In some embodiments, the branch chain amino acid dehydrogenase enzyme, e.g., leucine dehydrogenase, is coexpressed with one or more amino acid oxidase enzyme(s), e.g., L-AAD. In some embodiments, the engineered bacteria comprise gene sequence(s) encoding at least branch chain amino acid dehydrogenase enzyme, e.g., leucine dehydrogenase enzyme, and gene sequence(s) encoding one or more other branched chain amino acid dehydrogenase(s). In some embodiments, the engineered bacteria comprise gene sequence(s) encoding at least one branch chain amino acid dehydrogenase enzyme, e.g., leucine dehydrogenase enzyme, and gene sequence(s) encoding one or more amino acid oxidase(s) (e.g. L-AAD)). In some embodiments, the engineered bacteria comprise gene sequence(s) encoding at least one branch chain amino acid dehydrogenase enzyme, e.g., leucine dehydrogenase enzyme, and gene sequence(s) encoding one or more BCAA aminotransferase(s) (e.g., ilvE).

In some exmodiments, the branch chain amino acid dehydrogenase enzyme, e.g., leucine dehydrogenase, is coexpressed with one or more other branched chain amino acid catabolism enzyme(s), for example, a ketoacid decarboxylase, such as *kivD.* In some exmodiments, the branch chain amino acid dehydrogenase enzyme, e.g., leucine dehydrogenase, is coexpressed with one or more other branched chain amino acid catabolism enzyme(s), for example, a branched chain alcohol dehydrogenase, such as *adh2 or yqhD* and/or a branched chain aldehyde aehydrogenase, such as *padA.* In other embodiments, the branch chain amino acid dehydrogenase enzyme, e.g., leucine dehydrogenase, is coexpressed with a second branched chain amino acid catabolism enzyme, for example, *kivD,* and a branched chain alcohol dehydrogenase, for example, *adh2 or YqhD,* and/or a branched chain aldehyde dehydrogenase, for example, *padA,* each of which are described in more detail herein. In some embodiments, the engineered bacteria comprise gene sequence(s) encoding at least one branch chain amino acid dehydrogenase enzyme, e.g., leucine dehydrogenase enzyme, and gene sequence(s) encoding one or more keto-acid decarboxylase(s) (e.g., kivD). In some embodiments, the engineered bacteria comprise gene sequence(s) encoding at least one branch chain amino acid dehydrogenase enzyme, e.g., leucine dehydrogenase enzyme, and gene sequence(s) encoding one or more aldehyde dehydrogenase(s) (e.g., padA). In some embodiments, the engineered bacteria comprise gene sequence(s) encoding at least one branch chain amino acid dehydrogenase enzyme, e.g., leucine dehydrogenase enzyme, and gene sequence(s) encoding one or more alcohol dehydrogenase(s) (e.g., adh2, yqhD). In some embodiments, the engineered bacteria comprise gene sequence(s) encoding at least one branch chain amino acid dehydrogenase enzyme, e.g., leucine dehydrogenase enzyme, gene sequence(s) encoding one or more aldehyde dehydrogenase(s) (e.g., padA), and gene sequence(s) encoding one or more alcohol dehydrogenase(s) (e.g., adh2, yqhD). In some embodiments, the engineered bacteria comprise gene sequence(s) encoding at least one branch chain amino acid dehydrogenase enzyme, e.g., leucine dehydrogenase enzyme, gene sequence(s) encoding one or more keto-acid decarboxylase(s) (e.g., kivD), and gene sequence(s) encoding one or more aldehyde dehydrogenase(s) (e.g., padA). In some embodiments, the engineered bacteria comprise gene sequence(s) encoding at least one branch chain amino acid dehydrogenase enzyme, e.g., leucine dehydrogenase enzyme, gene sequence(s) encoding one or more keto-acid decarboxylase(s) (e.g., kivD), and gene sequence(s) encoding one or more alcohol dehydrogenase(s) (e.g., adh2, yqhD). In some embodiments, the engineered bacteria comprise gene sequence(s) encoding at least branch chain amino acid dehydrogenase enzyme, e.g., leucine dehydrogenase enzyme, gene sequence(s) encoding one or more keto-acid decarboxylase(s) (e.g., kivD), gene sequence(s) encoding one or more aldehyde dehydrogenase(s) (e.g., padA), and gene sequence(s) encoding one or more alcohol dehydrogenase(s) (e.g., adh2, yqhD).

In some embodiments, the at least one branch chain amino acid dehydrogenase enzyme, e.g., leucine dehydrogenase enzyme is coexpressed with one or more BCAA transporter(s), for example, a high affinity leucine transporter, e.g., LivKHMGF and/or low affinity BCAA transporter BrnQ. In some embodiments, the at least one branch chain amino acid dehydrogenase enzyme, e.g., leucine dehydrogenase enzyme is coexpressed with one or more BCAA binding protein(s), for example, LivJ. In some embodiments, the engineered bacteria comprise gene sequence(s) encoding at least one branch chain amino acid dehydrogenase enzyme, e.g., leucine dehydrogenase enzyme and gene sequence(s) encoding one or more BCAA transporter(s) (e.g., LivKHMGF, brnQ). In some embodiments, the engineered bacteria comprise gene sequence(s) encoding at least one branch chain amino acid dehydrogenase enzyme, e.g., leucine dehydrogenase enzyme and gene sequence(s) encoding one or more BCAA binding protein(s) (e.g., livJ). In some embodiments, the engineered bacteria comprise gene sequence(s) encoding at least one branch chain amino acid dehydrogenase enzyme, e.g., leucine dehydrogenase enzyme, gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branch chain amino acid dehydrogenase enzyme, e.g., leucine dehydrogenase enzyme, and genetic modification that reduces export of a branched chain amino acid, *e.g.,* a genetic mutation in a *leuE* gene or promoter thereof. In one embodiment, the engineered bacteria comprise gene sequence(s) encoding at least one branch chain amino acid dehydrogenase enzyme, e.g., leucine dehydrogenase enzyme, and a genetic modification that reduces or eliminates branched chain amino acid synthesis, *e.g*., a genetic mutation in a *ilvC* gene or promoter thereof.

In some embodiments, the gene sequence(s) encoding the one or more branch chain amino acid dehydrogenase enzyme(s), e.g., leucine dehydrogenase enzyme(s) is expressed under the control of a constitutive promoter. In some embodiments, the gene sequence(s) encoding the one or more branch chain amino acid dehydrogenase enzyme(s), e.g., leucine dehydrogenase enzyme(s) is expressed under the control of an inducible promoter. In some embodiments, the gene sequence(s) encoding the one or more branch chain amino acid dehydrogenase enzyme(s), e.g., leucine dehydrogenase enzyme(s) is expressed under the control of a promoter that is directly or indirectly induced by exogenous environmental conditions. In some embodiments, the gene sequence(s) encoding the one or more branch chain amino acid dehydrogenase enzyme(s), e.g., leucine dehydrogenase enzyme(s) is expressed under the control of a promoter that is directly or indirectly induced by low-oxygen or anaerobic conditions, wherein expression of the gene encoding the branch chain amino acid dehydrogenase enzyme, e.g., leucine dehydrogenase enzyme is activated under low-oxygen or anaerobic environments, such as the environment of the mammalian gut. In some embodiments, the gene sequence(s) encoding the one or more branch chain amino acid dehydrogenase enzyme(s), e.g., leucine dehydrogenase enzyme(s) is expressed under the control of a promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In some embodiments, the branched chain amino acid dehydrogenase is leucine dehydrogenase. Thus, insome embodiments, the engineered bacteria comprise gene sequence of SEQ ID NO: 20 and/or 58. The present disclosure further comprises genes encoding functional fragments of leucine dehydrogenase, or functional variants of leucine dehydrogenase. The present disclosure encompasses genes encoding leucine dehydrogenase, comprising amino acids in its sequence that are substantially the same as an amino acid sequence described herein. In some embodiments, the at least one *leuDH* gene has at least about 80% identity with SEQ ID NO:20. Accordingly, in one embodiment, the at least one *leuDH* gene has at least about 90% identity with SEQ ID NO:20. Accordingly, in one embodiment, the at least one *leuDH* gene has at least about 95% identity with SEQ ID NO:20. Accordingly, in one embodiment, the at least one *leuDH* gene has at least about 85%, 86%, 87%, 88%, 89%, 90%, 91%, 92%, 93%, 94%, 95%, 96%, 97%, 98%, or 99% identity with SEQ ID NO:20. In another embodiment, the at least one *leuDH* gene comprises SEQ ID NO:20. In yet another embodiment the at least one *leuDH* gene consists of SEQ ID NO:20. In another embodiment, the at least one gene encoding the leucine dehydrogenase belongs to the family oxidoreductases (EC 1.4.1.9). In yet another embodiment, the at least one gene encoding the leucine dehydrogenase is the L-leucine:NAD+ oxidoreductase. In one embodiment, the leucine dehydrogenase gene has been codon-optimized for use in the recombinant bacterial cell. In one embodiment, the leucine dehydrogenase gene has been codon-optimized for use in *Escherichia coli.* For example, a codon-optimized *leuDH* sequence is set forth as SEQ ID NO:20 and SEQ ID NO: 58.

### 2) Amino Acid Aminotransferases

In another embodiment, the branched chain amino acid deamination enzyme is a branched chain amino acid aminotransferase. In some embodiments, the engineered bacteria comprise gene sequence(s) encoding one or more copies of a branched chain amino acid aminotransferase. In some embodiments, the engineered bacteria comprise gene sequence(s) encoding one, two, three, four, five, six, or more copies of an branched chain amino acid aminotransferase. The one or more copies of branched chain amino acid aminotransferase can be one or more copies of the same gene or can be different genes encoding branched chain amino acid aminotransferase, e.g., gene(s) from a different species or otherwise having a different gene sequence. The one or more copies of branched chain amino acid aminotransferase can be present in the bacterial chromosome or can be present in one or more plasmids. As used herein "branched chain amino acid aminotransferase" refers to any polypeptide having enzymatic activity that deaminates a branched chain amino acid, *e.g*., leucine, valine, isoleucine to its corresponding ketoacid, *e.g*., alpha-ketoisocaproate (KIC) (EC 2.6.1.42), alpha-ketoisovalerate, alpha-keto-beta-methylvalerate. Multiple distinct branched chain amino acid aminotransferases are known in the art and are available from many microorganism sources, including those disclosed herein, as well as eukaryotic sources (see, for example, Peng et al., J. Bact., 139(2):339-45, 1979; Kline et al., J. Bact., 130(2):951-3, 1977.
branched chain amino acid aminotransferase enzymes are available from many microorganism sources, including those disclosed herein.

In some embodiments, the branched chain amino acid aminotransferase is encoded by at least one gene encoding a branched chain amino acid aminotransferase derived from a bacterial species. In some embodiments, the branched chain amino acid aminotransferase is encoded by at least one gene encoding a branched chain amino acid aminotransferase derived from a non-bacterial species. In some embodiments, the branched chain amino acid aminotransferase is encoded by at least one gene derived from a eukaryotic species, *e.g*., a yeast species or a plant species. In another embodiment, the branched chain amino acid aminotransferase is encoded by at least one gene derived from a mammalian species, e.g., human.

In one embodiment, the at least one gene encoding the branched chain amino acid aminotransferase enzyme is derived from an organism of the genus or species that includes, but is not limited to, *Arabidopsis, Bos, Brevibacillus, Canis, Corynebacterium, Cucumis, Deinococcus, Enterobacter, Entodinium, Escherichia, Gluconobacter, Helicobacter, Homo, Lactobacillus, Lactococcus, Macaca, Methanococcus, Mus, Mycobacterium, Neurospora, Nicotiana, Ovis, Pseudomonas, Rattus, Saccharomyces, Salmonella, Schizosaccharomyces, Solanum, Streptococcus, Sus,* or *Yarrowia* species. In one embodiment, the branched chain amino acid aminotransferase is encoded by *ilvE.* In one embodiment, the *ilvE* gene is encoded by a gene derived from *Arabidopsis thaliana, Bos taurus, Brevibacillus brevis, Brevibacterium flavum, Candida maltose, Canis lupus familiaris, Corynebacterium glutamicum, cucumis sativus, Deinococcus radiodurans, Enterobacter sp. TL3, enterococcus faecalis, Entodinium sp., Escherichia coli, gluconobacter oxydans, Helicobacter pylori, Homo sapiens, Lactobacillus paracasei, Lactococcus lactis, Macaca sp., Methanococcus aeolicus, Methanococcus maripaludis, Methanococcus voltae, Mus musculus, Mycobacterium smegmatis, Mycobacterium tuberculosis, Neurospora crassa, Nicotiana benthamiana, Ovis aries, Pseudomonas sp., Rattus norvegicus, Saccharomyces cerevisiae, Salmonella enterica, Schizosaccharomyces pombe, Solanum lycopersicum, Solanum pennellii, Staphylococcus carnosus, Streptococcus mutans, Sus scrofa,* or *Yarowia lipolytica.* In another embodiment, the branched chain amino acid aminotransferase, e.g., livE, is encoded by at least one gene derived from *Escherichia coli.* In one embodiment, the branched chain amino acid aminotransferase, e.g., livE, is encoded by at least gene from *E. coli* Nissle. In another embodiment, the branched chain amino acid aminotransferase, e.g., *ilvE,* is encoded by at least one gene derived from *Lactobacillus lactis.* In another embodiment, the, branched chain amino acid aminotransferase, *e.g,ilvE*,, is encoded by at least one gene derived from *Staphylococcus carnosus.* In some embodiments, the branched chain amino acid aminotransferase, *e.g.ilvE*, is encoded by at least one gene derived from *Streptococcus mutans.* In another embodiment, the branched chain amino acid aminotransferase, e.g., *ilvE* is encoded by at least one gene derived from *Bacillus subtilis.* In another embodiment, the branched chain amino acid aminotransferase, e.g., *ilvE* is encoded by at least one gene derived from *Salmonella typhi.*

In one embodiment the at least one gene encoding the branched chain amino acid aminotransferase has been codon-optimized for use in the recombinant bacterial cell. In one embodiment, the at least one gene encoding the branched chain amino acid aminotransferase has been codon-optimized for use in *Escherichia coli.*

When a branched chain amino acid aminotransferase enzyme is expressed in the recombinant bacterial cells disclosed herein, the bacterial cells catabolize more branched chain amino acid, *e.g*., leucine, isoleucine, and/or valine, than unmodified bacteria of the same bacterial subtype under the same conditions (*e.g*., culture or environmental conditions). Thus, the genetically engineered bacteria comprising at least one heterologous gene encoding a branched chain amino acid aminotransferase may be used to catabolize excess branched chain amino acids, *e.g*., leucine, isoleucine, and/or valine, to treat a disease associated with the deamination of a branched chain amino acid, including Maple Syrup Urine Disease (MSUD).

The present disclosure further comprises genes encoding functional fragments of a branched chain amino acid aminotransferase enzyme, e.g., ilvE, or functional variants of branched chain amino acid aminotransferase enzyme, e.g., ilvE. The present disclosure encompasses genes encoding a branched chain amino acid aminotransferase enzyme, e.g., ilvE, comprising amino acids in its sequence that are substantially the same as an amino acid sequence described herein. As used herein, the term "functional fragment thereof' or "functional variant thereof' of a branched chain amino acid aminotransferase enzyme, e.g., ilvE, gene relates to a sequence having qualitative biological activity in common with the wild-type branched chain amino acid aminotransferase, e.g., ilvE, from which the fragment or variant was derived. For example, a functional fragment or a functional variant of a mutated branched chain amino acid aminotransferase protein, e.g., ilvE, is one which retains essentially the same ability to catabolize BCAAs as the branched chain amino acid aminotransferase protein, e.g., ilvE, from which the functional fragment or functional variant was derived. For example, a polypeptide having branched chain amino acid aminotransferase enzyme, e.g., ilvE, activity may be truncated at the N-terminus or C-terminus and the retention of branched chain amino acid aminotransferase enzyme, e.g., ilvE, activity assessed using assays known to those of skill in the art, including the exemplary assays provided herein. In one embodiment, the recombinant bacterial cell disclosed herein comprises a heterologous gene encoding an α branched chain amino acid aminotransferase enzyme, e.g., ilvE, functional variant. In another embodiment, the recombinant bacterial cell disclosed herein comprises a heterologous gene encoding a branched chain amino acid aminotransferase enzyme, e.g., ilvE, functional fragment.

Assays for testing the activity of a branched chain amino acid aminotransferase, a branched chain amino acid aminotransferase functional variant, or a branched chain amino acid aminotransferase functional fragment, e.g., ilvE, ilvE functional variant, and ilvE functional fragment are well known to one of ordinary skill in the art. For example, branched chain amino acid aminotransferase activity can be assessed by expressing the protein, functional variant, or fragment thereof, in a recombinant bacterial cell that lacks endogenous branched chain amino acid aminotransferase activity. Also, activity can be assessed using the enzymatic assay methods as described by Santiago et al. (J. Bacterial., 195(16):3552-62, 2013) .

In some embodiments, the at least one gene encoding a branched chain amino acid aminotransferase enzyme, e.g., ilvE, is mutagenized, mutants exhibiting increased activity are selected, and the mutagenized gene(s) encoding the branched chain amino acid aminotransferase enzyme, e.g., ilvE, are isolated and inserted into the bacterial cell. In some embodiments, the at least one gene encoding a branched chain amino acid aminotransferase enzyme, e.g., ilvE, is mutagenized, mutants exhibiting decreased activity are selected, and the mutagenized gene(s) encoding the branched chain amino acid aminotransferase enzyme, e.g., ilvE, are isolated and inserted into the bacterial cell. The gene comprising the modifications described herein may be present on a plasmid or chromosome.

In some embodiments, the branched chain amino acid aminotransferase is co-expressed with an additional branched chain amino acid catabolism enzyme. In some embodiments, the branched chain amino acid aminotransferase is co-expressed with an another branched chain amino acid deaminase enzyme, e.g. a BCAA dehydrogenase, such as leucine dehydrogenase, an AA aminotransferase, an amino acid oxidase, such as L-AAD. In some embodiments, the branched chain amino acid aminotransferase is co-expressed with one or more keto-acid decarboxylase enzyme(s), *e.g*., *kivD.* In some embodiments, the branched chain amino acid aminotransferase is co-expressed with one or more alcohol dehydrogenases, e.g., adh2 and/or yqhD. In some embodiments, the branched chain amino acid aminotransferase is co-expressed with one or more aldehyde dehydrogenases, e.g., padA. In some embodiments, the branched chain amino acid aminotransferase is co-expressed with one or more keto-acid decarboxylase enzymes, *e.g*., *kivD* and one or more alcohol dehydrogenase enzymes, *e.g*., *adh2 or YqhD.* In some embodiments, the branched chain amino acid aminotransferase is co-expressed with one or more keto-acid decarboxylase enzymes, *e.g*., *kivD* and one or more aldehyde dehydrogenase enzymes, *e.g*., *padA.* In some embodiments, the branched chain amino acid aminotransferase is co-expressed with one or more keto-acid decarboxylase enzymes, *e.g*., *kivD,* one or more aldehyde dehydrogenase enzymes, *e.g*., *padA,* and one or more alcohol dehydrogenase enzymes, *e.g*., *adh2 or YqhD,* each of which are described in more detail herein. In some embodiments, the branched chain amino acid aminotransferase is co-expressed with an another branched chain amino acid deaminase enzyme, e.g. a BCAA dehydrogenase, such as leucine dehydrogenase, a BCAA aminotransferase, and/or an amino acid oxidase, such as L-AAD and is co-expressed with one or more keto-acid decarboxylase enzyme(s), *e.g*., *kivD.* In some embodiments, the branched chain amino acid aminotransferase, is co-expressed with an another branched chain amino acid deaminase enzyme, e.g. a BCAA dehydrogenase, such as leucine dehydrogenase, a BCAA aminotransferase, and/or an amino acid oxidase, such as L-AAD and is co-expressed with one or more alcohol dehydrogenases, e.g., adh2 and/or yqhD. In some embodiments, the branched chain amino acid aminotransferase is co-expressed with an another branched chain amino acid deaminase enzyme, e.g. a BCAA dehydrogenase, such as leucine dehydrogenase, a BCAA aminotransferase, and/or an amino acid oxidase, such as L-AAD and is co-expressed with one or more aldehyde dehydrogenases, e.g., padA. In some embodiments, the branched chain amino acid aminotransferase is co-expressed with an another branched chain amino acid deaminase enzyme, e.g. a BCAA dehydrogenase, such as leucine dehydrogenase, a BCAA aminotransferase, and/or an amino acid oxidase, such as L-AAD, is co-expressed with one or more keto-acid decarboxylase enzyme(s), *e.g*., *kivD,* and co-expressed with one or more alcohol dehydrogenases, e.g., adh2 and/or yqhD and/or one or more aldehyde dehydrogenases, e.g., padA. In some embodiments, the at least one branched chain amino acid aminotransferase enzyme is coexpressed with one or more BCAA transporter(s), for example, a high affinity leucine transporter, e.g., LivKHMGF and/or low affinity BCAA transporter BrnQ. In some embodiments, the at least one branched chain amino acid aminotransferase enzyme is coexpressed with one or more BCAA binding protein(s), for example, LivJ. In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain amino acid aminotransferase enzyme and genetic modification that reduces export of a branched chain amino acid, *e.g*., a genetic mutation in a *leuE* gene or promoter thereof. In one embodiment, the engineered bacteria comprise gene sequence(s) encoding at least one branched chain amino acid aminotransferase enzyme and a genetic modification that reduces or eliminates branched chain amino acid synthesis, *e.g*., a genetic mutation in a *ilvC* gene or promoter thereof.

In some embodiments, the engineered bacteria comprise gene sequence(s) encoding at least one branched chain amino acid aminotransferase enzyme and further comprise gene sequence encoding one or more polypeptides selected from other branched chain amino acid catabolism enzyme(s), BCAA transporter(s), and BCAA binding protein(s). In some embodiments, the engineered bacteria comprise gene sequence(s) encoding at least one branched chain amino acid aminotransferase enzyme, e.g., ilvE, and gene sequence(s) encoding one or more branched chain amino acid dehydrogenase(s) (e.g., leuDH). In some embodiments, the engineered bacteria comprise gene sequence(s) encoding at least one branched chain amino acid aminotransferase enzyme, e.g., ilvE, and gene sequence(s) encoding one or more amino acid oxidase(s) (e.g. L-AAD)). In some embodiments, the engineered bacteria comprise gene sequence(s) encoding at least one one branched chain amino acid aminotransferase enzyme, *ilvE,* and gene sequence(s) encoding one or more other aminotransferase(s). In some embodiments, the engineered bacteria comprise gene sequence(s) encoding at least one one branched chain amino acid aminotransferase enzyme, e.g., ilvE, and gene sequence(s) encoding one or more keto-acid decarboxylase enzyme(s), *e.g*., *kivD.* In some embodiments, the engineered bacteria comprise gene sequence(s) encoding at least one one branched chain amino acid aminotransferase enzyme, e.g., ilvE, and gene sequence(s) encoding one or more aldehyde dehydrogenase(s) (e.g., padA). In some embodiments, the engineered bacteria comprise gene sequence(s) encoding at least one one branched chain amino acid aminotransferase enzyme, e.g., ilvE, and gene sequence(s) encoding one or more alcohol dehydrogenase(s) (e.g., adh2, yqhD).

In some embodiments, the engineered bacteria comprise gene sequence(s) encoding at least one branched chain amino acid aminotransferase enzyme, e.g., ilvE, and gene sequence(s) encoding one or more other branched chain amino acid catabolism enzyme(s), for example, branched chain amino acid dehydrogenase(s), such as leuDH, branched chain amino acid aminotransferase enzyme, and/or amino acid oxidase(s), such as L-AAD and gene sequence(s) encoding one or more keto-acid decarboxylase enzyme(s), *e.g., kivD.* In some embodiments, the engineered bacteria comprise gene sequence(s) encoding at least one branched chain amino acid aminotransferase enzyme, e.g., ilvE, and gene sequence(s) encoding one or more other branched chain amino acid catabolism enzyme(s), for example, branched chain amino acid dehydrogenase(s), such as leuDH, branched chain amino acid aminotransferase enzyme, and/or amino acid oxidase(s), such as L-AAD, gene sequence(s) encoding one or more keto-acid decarboxylase enzyme(s), *e.g*., *kivD*, and gene sequence(s) encoding one or more aldehyde dehydrogenase(s) (e.g., padA) and/or gene sequence(s) encoding one or more alcohol dehydrogenase(s) (e.g., adh2, yqhD).

In some embodiments, the engineered bacteria comprise gene sequence(s) encoding at least one branched chain amino acid aminotransferase enzyme, e.g., ilvE, and gene sequence(s) encoding one or more BCAA transporter(s) (e.g., LivKHMGF, brnQ). In some embodiments, the engineered bacteria comprise gene sequence(s) encoding at least one branched chain amino acid aminotransferase enzyme, e.g. ilvE, and gene sequence(s) encoding one or more BCAA binding protein(s) (e.g., livJ). In some embodiments, the engineered bacteria comprise gene sequence(s) encoding at least one branched chain amino acid aminotransferase enzyme, e.g. ilvE, gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain amino acid aminotransferase enzyme, e.g. ilvE, and genetic modification that reduces export of a branched chain amino acid, *e.g*., a genetic mutation in a *leuE* gene or promoter thereof. In one embodiment, the engineered bacteria comprise gene sequence(s) encoding at least one branched chain amino acid aminotransferase enzyme, e.g. ilvE, and a genetic modification that reduces or eliminates branched chain amino acid synthesis, *e.g*., a genetic mutation in a *ilvC* gene or promoter thereof.

In some embodiments, the gene sequence(s) encoding the one or more branched chain amino acid aminotransferase enzyme(s), e.g. ilvE, is expressed under the control of a constitutive promoter. In some embodiments, the gene sequence(s) encoding the one or more branched chain amino acid aminotransferase enzyme(s), e.g. ilvE, is expressed under the control of an inducible promoter. In some embodiments, the gene sequence(s) encoding the one or more branched chain amino acid aminotransferase enzyme(s), e.g. ilvE, is expressed under the control of a promoter that is directly or indirectly induced by exogenous environmental conditions. In some embodiments, the gene sequence(s) encoding the one or more branched chain amino acid aminotransferase enzyme(s), e.g. ilvE, is expressed under the control of a promoter that is directly or indirectly induced by low-oxygen or anaerobic conditions, wherein expression of the gene encoding the branched chain amino acid aminotransferase enzyme, e.g. ilvE, is activated under low-oxygen or anaerobic environments, such as the environment of the mammalian gut. In some embodiments, the gene sequence(s) encoding the one or more branched chain amino acid aminotransferase enzyme, e.g. ilvE, is expressed under the control of a promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In some embodiments, the at least one gene encoding the branched chain amino acid aminotransferase comprises the *ilvE* gene. In a specific embodiment, the *ilvE* gene has at least about 80% identity with the sequence of SEQ ID NO:22. In one embodiment, the *ilvE* gene has at least about 90% identity with the sequence of SEQ ID NO:22. In one embodiment, the *ilvE* gene has at least about 95% identity with the sequence of SEQ ID NO:22. In another embodiment, the *ilvE* gene has at least about 85%, 86%, 87%, 88%, 89%, 90%, 91%, 92%, 93%, 94%, 95%, 96%, 97%, 98%, or 99% identity with the sequence of SEQ ID NO:22. In another embodiment, the *ilvE* gene comprises the sequence of SEQ ID NO:22. In yet another embodiment, the *ilvE* gene consists of the sequence of SEQ ID NO:22.

### Amino acid oxidase/ Amino acid Deaminase

In other embodiments, the branched chain amino acid deamination enzyme is a branched chain amino acid oxidase (also referred as branched chain amino acid deaminase). In some embodiments, the engineered bacteria comprise gene sequence(s) encoding one or more copies of a branched chain amino acid oxidase. In some embodiments, the engineered bacteria comprise gene sequence(s) encoding one, two, three, four, five, six, or more copies of a branched chain amino acid oxidase. The one or more copies of a branched chain amino acid oxidase can be one or more copies of the same gene or can be different genes encoding branched chain amino acid oxidase, e.g., gene(s) from a different species or otherwise having a different gene sequence. The one or more copies of branched chain amino acid oxidase can be present in the bacterial chromosome or can be present in one or more plasmids. As used herein "branched chain amino acid oxidase" refers to any polypeptide having enzymatic activity that deaminates a branched chain amino acid, *e.g*., leucine, to its corresponding ketoacid, *e.g*., alpha-ketoisocaproate (KIC) (EC 1.4.3.2). Multiple distinct branched chain amino acid aminotransferases are known in the art and are available from many microorganism sources, including those disclosed herein, as well as eukaryotic sources (see, for example, Song et al., Scientific Reports, Nature, 5:12694; DOI: 10:1038/srep12694 (2015)) .

In some embodiments, the branched chain amino acid oxidase is encoded by at least one gene encoding a branched chain amino acid oxidase derived from a bacterial species. In some embodiments, the branched chain amino acid oxidase is encoded by at least one gene encoding a branched chain amino acid oxidase derived from a non-bacterial species. In some embodiments, the branched chain amino acid oxidase is encoded by at least one gene derived from a eukaryotic species, *e.g*., a yeast species or a plant species. In another embodiment, the branched chain amino acid oxidase is encoded by at least one gene derived from a mammalian species, e.g., a human.

In some embodiments, the at least one gene encoding the branched chain amino acid oxidase enzyme is derived from an organism of the genus or species that includes, but is not limited to, *Arabidopsis, Bos, Brevibacillus, Canis, Corynebacterium, Cucumis, Deinococcus, Enterobacter, Entodinium, Escherichia, Gluconobacter, Helicobacter, Homo, Lactobacillus, Lactococcus, Macaca, Methanococcus, Mus, Mycobacterium, Neurospora, Nicotiana, Ovis, Pseudomonas, Rattus, Saccharomyces, Salmonella, Schizosaccharomyces, Solanum, Streptococcus, Sus,* or *Yarrowia* species. In one embodiment, the branched chain amino acid aminotransferase is encoded by *L-AAD.* In one embodiment, the *L-AAD* gene is encoded by a gene derived from *Arabidopsis thaliana, Bos taurus, Brevibacillus brevis, Brevibacterium flavum, Candida maltose, Canis lupus familiaris, Corynebacterium glutamicum, cucumis sativus, Deinococcus radiodurans, Enterobacter sp. TL3, enterococcus faecalis, Entodinium sp., Escherichia coli, gluconobacter oxydans, Helicobacter pylori, Homo sapiens, Lactobacillus paracasei, Lactococcus lactis, Macaca sp., Methanococcus aeolicus, Methanococcus maripaludis, Methanococcus voltae, Mus musculus, Mycobacterium smegmatis, Mycobacterium tuberculosis, Neurospora crassa, Nicotiana benthamiana, Ovis aries, Pseudomonas sp., Rattus norvegicus, Saccharomyces cerevisiae, Salmonella enterica, Schizosaccharomyces pombe, Solanum lycopersicum, Solanum pennellii, Staphylococcus carnosus, Streptococcus mutans, Sus scrofa,* or *Yarowia lipolytica.* In another embodiment, the *L-AAD* is encoded by at least one gene derived from *Lactobacillus lactis.* In another embodiment, the *L-AAD* is encoded by at least one gene derived from *Staphylococcus carnosus.* In some embodiments, the *L-AAD* is encoded by at least one gene derived from *Streptococcus mutans.* In another embodiment, the *L-AAD* is encoded by at least one gene derived from *Bacillus subtilis.* In another embodiment, the *L-AAD* is encoded by at least one gene derived from *Salmonella typhi.* In another embodiment, the *L-AAD* is encoded by at least one gene derived from *Proteus vulgaris.* In another embodiment, the *L-AAD* is encoded by at least one gene derived from *Proteus mirabilis.*

Substrate specificities of selected Proteus L-amino acid deaminases are shown in **Table 3** and are described Baek et al., Journal of Basic Microbiology 2011, 51, 129-135; "Expression and characterization of a second L-amino acid deaminase isolated from Proteus mirabilis in Escherichia coli.

Two LAADs exist in P. mirabilis. In certain embodiments of the disclosure, LAAD(Pv) refers to Pma. The amino acid deaminase activities are presented as percentages of the activities against amino acid deaminases, respectively, only perpendicularly.

**Table 3. Substrate specificities of selected amino acid deaminases from Proteus species**

| **AA** | **Pm1** | **LAD** | **Pma** |
|---|---|---|---|
| Ala | 9 | 3.5 | 0.6 |
| Arg | 51.2 | 27.3 | 28.2 |
| Asn | 5.2 | 43.6 | 0 |
| Asp | 2.6 | 55.4 | 10.9 |
| Cys | 9 | - | 1.9 |
| Gln | 5.2 | 1.1 | 1.3 |
| Glu | 35.8 | 1.1 | 0.6 |
| Gly | 7.6 | - | 1.3 |
| His | 100 | 79.9 | 0 |
| Ilu | 6.4 | - | 2.6 |
| Leu | 7.6 | 105 | 41.7 |
| Lys | 7.6 | 3.5 | 1.9 |
| Met | 2.6 | 100 | 16.7 |
| Phe | 46.2 | 37.4 | 100 |
| Pro | 14.2 | 0.7 | 3.2 |
| Ser | 3.8 | - | 1.3 |
| Thr | 12.8 | 1.1 | 0 |
| Trp | 10.2 | 41.6 | 3.2 |
| Tyr | 9 | 92.8 | 0.6 |
| Val | 6.4 | - | 1.3 |

| | | | |
|---|---|---|---|
| Pm1: amino acid deaminase gene from P. mirabilis KCTC 2566 (Genbank: EU669819.1) LAD: L-amino acid deaminase of P. vulgaris (Genbank: AB030003) Pma: amino acid deaminase gene from P. mirabilis (Genbank: U35383) | | | |

In one embodiment the at least one gene encoding the branched chain amino acid oxidase has been codon-optimized for use in the recombinant bacterial cell. In one embodiment, the at least one gene encoding the branched chain amino acid oxidase has been codon-optimized for use in *Escherichia coli.*

When a branched chain amino acid oxidase enzyme is expressed in the recombinant bacterial cells disclosed herein, the bacterial cells catabolize more branched chain amino acid, *e.g*., leucine, isoleucine, and/or valine, than unmodified bacteria of the same bacterial subtype under the same conditions (*e.g*., culture or environmental conditions). Thus, the genetically engineered bacteria comprising at least one heterologous gene encoding a branched chain amino acid oxidase may be used to catabolize excess branched chain amino acids, *e.g*., leucine, isoleucine, and/or valine, to treat a disease associated with the deamination of a branched chain amino acid, including Maple Syrup Urine Disease (MSUD).

The present disclosure further comprises genes encoding functional fragments of a branched chain amino acid oxidase enzyme, e.g., L-AAD, or functional variants of branched chain amino acid oxidase enzyme, e.g., L-AAD. The present disclosure encompasses genes encoding a branched chain amino acid oxidase enzyme, e.g., L-AAD, comprising amino acids in its sequence that are substantially the same as an amino acid sequence described herein. As used herein, the term "functional fragment thereof' or "functional variant thereof' of a branched chain amino acid oxidase enzyme, e.g.,L-AAD, gene relates to a sequence having qualitative biological activity in common with the wild-type branched chain amino acid oxidase, e.g., L-AAD, from which the fragment or variant was derived. For example, a functional fragment or a functional variant of a mutated branched chain amino acid oxidase protein, e.g., L-AAD, is one which retains essentially the same ability to catabolize BCAAs as branched chain amino acid oxidase protein, e.g., L-AAD, from which the functional fragment or functional variant was derived. For example, a polypeptide having branched chain amino acid oxidase enzyme, e.g., L-AAD, activity may be truncated at the N-terminus or C-terminus and the retention branched chain amino acid oxidase enzyme, e.g., L-AAD, activity assessed using assays known to those of skill in the art, including the exemplary assays provided herein. In one embodiment, the recombinant bacterial cell disclosed herein comprises a heterologous gene encoding an α branched chain amino acid oxidase enzyme, e.g., L-AAD, functional variant. In another embodiment, the recombinant bacterial cell disclosed herein comprises a heterologous gene encoding branched chain amino acid oxidase enzyme, e.g., L-AAD, functional fragment.

Assays for testing the activity of a branched chain amino acid oxidase, a branched chain amino acid oxidase functional variant, or a branched chain amino acid oxidase functional fragment are well known to one of ordinary skill in the art. For example, branched chain amino acid oxidase activity can be assessed by expressing the protein, functional variant, or functional fragment thereof, in a recombinant bacterial cell that lacks endogenous branched chain amino acid oxidase activity. Also, activity can be assessed using the enzymatic assay methods as described by Santiago et al. (J. Bacterial., 195(16):3552-62, 2013) .

In some embodiments, the at least one gene encoding a branched chain amino acid oxidase is mutagenized, mutants exhibiting increased activity are selected, and the mutagenized gene(s) encoding the branched chain amino acid oxidase are isolated and inserted into the bacterial cell. In some embodiments, the at least one gene encoding the branched chain amino acid oxidase is mutagenized, mutants exhibiting decreased activity are selected, and the mutagenized gene(s) encoding the branched chain amino acid oxidase are isolated and inserted into the bacterial cell. The gene comprising the modifications described herein may be present on a plasmid or chromosome.

In some embodiments, the branched chain amino acid oxidase is co-expressed with an additional branched chain amino acid catabolism enzyme. In some embodiments, the branched chain amino acid oxidase is co-expressed with one or more additional branched chain amino acid catabolism enzyme(s) selected from a branched chain amino acid deaminase enzyme, e.g. a BCAA dehydrogenase, such as leucine dehydrogenase; a BCAA aminotransferase, e.g., ilvE, and a branched chain amino acid oxidase, e.g., L-AAD. In some embodiments, the branched chain amino acid oxidase is co-expressed with one or more keto-acid decarboxylase enzyme(s), *e.g*., *kivD.* In some embodiments, the branched chain amino acid oxidase is co-expressed with one or more alcohol dehydrogenases, e.g., adh2 and/or yqhD. In some embodiments, the branched chain amino acid oxidase is co-expressed with one or more aldehyde dehydrogenases, e.g., padA. In some embodiments, the branched chain amino acid oxidase is co-expressed with one or more keto-acid decarboxylase enzymes, *e.g*., *kivD* and one or more alcohol dehydrogenase enzymes, *e.g*., *adh2 or YqhD.* In some embodiments, the branched chain amino acid oxidase is co-expressed with one or more keto-acid decarboxylase enzymes, *e.g*., *kivD* and one or more aldehyde dehydrogenase enzymes, *e.g*., *padA.* In some embodiments, the branched chain amino acid oxidase is co-expressed with one or more keto-acid decarboxylase enzymes, *e.g*., *kivD,* one or more aldehyde dehydrogenase enzymes, *e.g*., *padA,* and one or more alcohol dehydrogenase enzymes, *e.g*., *adh2 or YqhD,* each of which are described in more detail herein.

In some embodiments, the branched chain amino acid oxidase is co-expressed with an another branched chain amino acid deaminase enzyme, e.g. a BCAA dehydrogenase, such as leucine dehydrogenase, a BCAA aminotransferase, e.g., ilvE, and/or another amino acid oxidase and is co-expressed with one or more keto-acid decarboxylase enzyme(s), *e.g*., *kivD.* In some embodiments, branched chain amino acid oxidase, is co-expressed with an another branched chain amino acid deaminase enzyme, e.g. a BCAA dehydrogenase, such as leucine dehydrogenase, a BCAA aminotransferase, e.g., ilvE, and/or another amino acid oxidase and is co-expressed with one or more alcohol dehydrogenases, e.g., adh2 and/or yqhD. In some embodiments, the branched chain amino acid oxidase is co-expressed with an another branched chain amino acid deaminase enzyme, e.g. a BCAA dehydrogenase, such as leucine dehydrogenase, a BCAA aminotransferase, e.g., ilvE, and/or another amino acid oxidase and is co-expressed with one or more aldehyde dehydrogenases, e.g., padA. In some embodiments, the branched chain amino acid oxidase is co-expressed with an another branched chain amino acid deaminase enzyme, e.g. a BCAA dehydrogenase, such as leucine dehydrogenase, a BCAA aminotransferase, e.g., ilvE, and/or another amino acid oxidase, is co-expressed with one or more keto-acid decarboxylase enzyme(s), *e.g*., *kivD,* and co-expressed with one or more alcohol dehydrogenases, e.g., adh2 and/or yqhD and/or one or more aldehyde dehydrogenases, e.g., padA. In some embodiments, the at least one branched chain amino acid oxidase enzyme is coexpressed with one or more BCAA transporter(s), for example, a high affinity leucine transporter, e.g., LivKHMGF and/or low affinity BCAA transporter BrnQ. In some embodiments, the at least one branched chain amino acid oxidase enzyme is coexpressed with one or more BCAA binding protein(s), for example, LivJ. In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain amino acid oxidase enzyme and genetic modification that reduces export of a branched chain amino acid, *e.g*., a genetic mutation in a *leuE* gene or promoter thereof. In one embodiment, the engineered bacteria comprise gene sequence(s) encoding at least one branched chain amino acid oxidase enzyme and a genetic modification that reduces or eliminates branched chain amino acid synthesis, *e.g*., a genetic mutation in a *ilvC* gene or promoter thereof.

In some embodiments, the engineered bacteria comprise gene sequence(s) encoding at least one branched chain amino acid oxidase enzyme and further comprise gene sequence encoding one or more polypeptides selected from other branched chain amino acid catabolism enzyme(s), BCAA transporter(s), and BCAA binding protein(s). In some embodiments, the engineered bacteria comprise gene sequence(s) encoding at least one branched chain amino acid oxidase enzyme, e.g., L-AAD, and gene sequence(s) encoding one or more branched chain amino acid dehydrogenase(s) (e.g., leuDH). In some embodiments, the engineered bacteria comprise gene sequence(s) encoding at least one branched chain amino acid oxidase enzyme, e.g., L-AAD, and gene sequence(s) encoding one or more other amino acid oxidase(s). In some embodiments, the engineered bacteria comprise gene sequence(s) encoding at least one one branched chain amino acid oxidase enzyme, *L-AAD,* and gene sequence(s) encoding one or more BCAA aminotransferase(s), e.g., ilvE. In some embodiments, the engineered bacteria comprise gene sequence(s) encoding at least one one branched chain amino acid oxidase enzyme, e.g., L-AAD, and gene sequence(s) encoding one or more keto-acid decarboxylase enzyme(s), *e.g*., *kivD.* In some embodiments, the engineered bacteria comprise gene sequence(s) encoding at least one one branched chain amino acid oxidase enzyme, e.g., L-AAD, and gene sequence(s) encoding one or more aldehyde dehydrogenase(s) (e.g., padA). In some embodiments, the engineered bacteria comprise gene sequence(s) encoding at least one one branched chain amino acid oxidase enzyme, e.g., L-AAD, and gene sequence(s) encoding one or more alcohol dehydrogenase(s) (e.g., adh2, yqhD).

In some embodiments, the engineered bacteria comprise gene sequence(s) encoding at least one branched chain amino acid oxidase enzyme, e.g., L-AAD, and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or another amino acid oxidase(s) and gene sequence(s) encoding one or more keto-acid decarboxylase enzyme(s), *e.g*., *kivD.* In some embodiments, the engineered bacteria comprise gene sequence(s) encoding at least one branched chain amino acid oxidase enzyme, e.g., L-AAD, and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or another amino acid oxidase(s), gene sequence(s) encoding one or more keto-acid decarboxylase enzyme(s), *e.g*., *kivD,* and gene sequence(s) encoding one or more aldehyde dehydrogenase(s) (e.g., padA) and/or gene sequence(s) encoding one or more alcohol dehydrogenase(s) (e.g., adh2, yqhD).

In some embodiments, the engineered bacteria comprise gene sequence(s) encoding at least one branched chain amino acid oxidase enzyme, e.g., L-AAD, and gene sequence(s) encoding one or more BCAA transporter(s) (e.g., LivKHMGF, brnQ). In some embodiments, the engineered bacteria comprise gene sequence(s) encoding at least one branched chain amino acid oxidase enzyme, e.g. L-AAD, and gene sequence(s) encoding one or more BCAA binding protein(s) (e.g., livJ). In some embodiments, the engineered bacteria comprise gene sequence(s) encoding at least one branched chain amino acid oxidase enzyme, e.g. L-AAD, gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one branched chain amino acid oxidase enzyme, e.g. L-AAD, and genetic modification that reduces export of a branched chain amino acid, *e.g*., a genetic mutation in a *leuE* gene or promoter thereof. In one embodiment, the engineered bacteria comprise gene sequence(s) encoding at least one branched chain amino acid oxidase enzyme, e.g. L-AAD, and a genetic modification that reduces or eliminates branched chain amino acid synthesis, *e.g*., a genetic mutation in a *ilvC* gene or promoter thereof.

In some embodiments, the gene sequence(s) encoding the one or more branched chain amino acid oxidase enzyme(s), e.g. L-AAD, is expressed under the control of a constitutive promoter. In some embodiments, the gene sequence(s) encoding the one or more branched chain amino acid oxidase enzyme(s), e.g. L-AAD, is expressed under the control of an inducible promoter. In some embodiments, the gene sequence(s) encoding the one or more branched chain amino acid oxidase enzyme(s), e.g. L-AAD, is expressed under the control of a promoter that is directly or indirectly induced by exogenous environmental conditions. In some embodiments, the gene sequence(s) encoding the one or more branched chain amino acid oxidase enzyme(s), e.g. L-AAD, is expressed under the control of a promoter that is directly or indirectly induced by low-oxygen or anaerobic conditions, wherein expression of the gene encoding the branched chain amino acid oxidase, e.g. L-AAD, is activated under low-oxygen or anaerobic environments, such as the environment of the mammalian gut. In some embodiments, the gene sequence(s) encoding the one or more branched chain amino acid oxidase enzyme(s), e.g. L-AAD, is expressed under the control of a promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In one embodiment, the at least one gene encoding the branched chain amino acid oxidase comprises the *L-AAD* gene. In one embodiment, the *L-AAD* gene has at least about 80% identity with the sequence of SEQ ID NO:24, SEQ ID NO:26, and/or SEQ ID NO:56. In one embodiment, the *L-AAD* gene has at least about 90% identity with the sequence of SEQ ID NO:24, SEQ ID NO:26, and/or SEQ ID NO:56. In one embodiment, the *L-AAD* gene has at least about 95% identity with the sequence of SEQ ID NO:24, SEQ ID NO:26, and/or SEQ ID NO:56. In another embodiment, the *L-AAD* gene has at least about 85%, 86%, 87%, 88%, 89%, 90%, 91%, 92%, 93%, 94%, 95%, 96%, 97%, 98%, or 99% identity with the sequence of SEQ ID NO:24, SEQ ID NO:26, and/or SEQ ID NO:56. In another embodiment, the *L-AAD* gene comprises the sequence of SEQ ID NO:24, SEQ ID NO:26, and/or SEQ ID NO:56. In yet another embodiment, the *L-AAD* gene consists of the sequence of SEQ ID NO:24, SEQ ID NO:26, and/or SEQ ID NO:56.

### D. Alcohol and Aldehyde Dehydrogenase Enzymes

In some embodiments, wherein a branched chain amino acid catabolism enzyme is used to convert a ketoacid to its corresponding aldehyde, the recombinant bacterial cells may further comprise an alcohol dehydrogenase enzyme in order to convert the branched chain amino acid-derived aldehyde to its respective alcohol. In some embodiments, the engineered bacteria comprise gene sequence(s) encoding one or more copies of a alcohol dehydrogenase. In some embodiments, the engineered bacteria comprise gene sequence(s) encoding one, two, three, four, five, six, or more copies of an alcohol dehydrogenase. The one or more copies of an alcohol dehydrogenase can be one or more copies of the same gene or can be different genes encoding alcohol dehydrogenase, e.g., gene(s) from a different species or otherwise having a different gene sequence. The one or more copies of alcohol dehydrogenase can be present in the bacterial chromosome or can be present in one or more plasmids. As used herein, "alcohol dehydrogenase" refers to any polypeptide having enzymatic activity that catalyzes the conversion of a branched chain amino acid-derived aldehyde, *e.g*., isovaleraldehyde, isobutyraldehyde, and 2-methylbutyraldehyde, into its respective alcohol, *e.g*., isopentanol, isobutanol, and 2-methylbutanol.

In general, alcohol dehydrogenases (EC 1.1.1.1) belong to a group of dehydrogenase enzymes that facilitate the interconversion between alcohols and aldehydes or ketones with the reduction of nicotinamide adenine dinucleotide (NAD+ to NADH). Multiple distinct alcohol dehydrogenases are known in the art and are available from many microorganism sources, including those disclosed herein, as well as eukaryotic and plant sources (see, for example, Bennetzen et al., J. Biol. Chem., 257(6):3018-25, 1982 and Teng et al., Human Genetics, 53(1):87-90, 1979).

In some embodiments, the alcohol dehydrogenase is encoded by at least one gene derived from a bacterial species. In some embodiments, the alcohol dehydrogenase is encoded by at least one gene derived from a non-bacterial species. In some embodiments, the alcohol dehydrogenase is encoded by at least one gene derived from a eukaryotic species, *e.g*., a yeast species or a plant species. In another embodiment, the alcohol dehydrogenase is encoded by at least one gene derived from a mammalian species, e.g., human.

In one embodiment, the at least one gene encoding the alcohol dehydrogenase is derived from an organism of the genus or species that includes, but is not limited to, *Acetinobacter, Azospirillum, Bacillus, Bacteroides, Bifidobacterium, Brevibacteria, Burkholderia, Citrobacter, Clostridium, Corynebacterium, Cronobacter, Enterobacter, Enterococcus, Erwinia, Helicobacter, Klebsiella, Lactobacillus, Lactococcus, Leishmania, Listeria, Macrococcus, Mycobacterium, Nakamurella, Nasonia, Nostoc, Pantoea, Pectobacterium, Proteus, Pseudomonas, Psychrobacter, Ralstonia, Saccharomyces, Salmonella, Sarcina, Serratia, Staphylococcus, Streptococcus, and Yersinia, e.g., Acetinobacter radioresistens, Acetinobacter baumannii, Acetinobacter calcoaceticus, Azospirillum brasilense, Bacillus anthracis, Bacillus cereus, Bacillus coagulans, Bacillus megaterium, Bacillus subtilis, Bacillus thuringiensis, Bacteroides fragilis, Bacteroides subtilis, Bacteroides thetaiotaomicron, Bifidobacterium bifidum, Bifidobacterium infantis, Bifidobacterium lactis, Bifidobacterium longum, Burkholderia xenovorans, Citrobacter youngae, Citrobacter koseri, Citrobacter rodentium, Clostridium acetobutylicum, Clostridium butyricum, Corynebacterium aurimucosum, Corynebacterium kroppenstedtii, Corynebacterium striatum, Cronobacter sakazakii, Cronobacter turicensis, Enterobacter cloacae, Enterobacter cancerogenus, Enterococcus faecium, Enterococcus faecalis, Erwinia amylovara, Erwinia pyrifoliae, Erwinia tasmaniensis, Helicobacter mustelae, Klebsiella pneumonia, Klebsiella variicola, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, Leishmania infantum, Leishmania major, Leishmania brazilensis, Listeria grayi, Macrococcus caseolyticus, Mycobacterium avium, Mycobacterium intracellulare, Mycobacterium kansasii, Mycobacterium leprae, Mycobacterium marinum, Mycobacterium smegmatis, Mycobacterium tuberculosis, Mycobacterium ulcerans, Nakamurella multipartita, Nasonia vitipennis, Nostoc punctiforme, Pantoea ananatis, Pantoea agglomerans, Pectobacterium atrosepticum, Pectobacterium carotovorum, Pseudomonas putida, Pseudomonas aeruginosa, Psychrobacter articus, Proteus vulgaris, Psychrobacter cryohalolentis, Ralstonia eutropha, Saccharomyces boulardii, Salmonella enterica, Sarcina ventriculi, Serratia odorifera, Serratia proteamaculans, Staphylococcus aerus, Staphylococcus capitis, Staphylococcys carnosus, Staphylococcus epidermidis, Staphylococcus hominis, Staphylococcus haemolyticus, Staphylococcus lugdunensis, Staphylococcus saprophyticus, Staphylococcus warneri, Streptococcus faecalis, Yersinia enterocolitica, Yersinia mollaretii, Yersinia kristensenii, Yersinia rohdei, and Yersinia aldovae.* In some embodiments, the alcohol dehydrogenase is selected from adh2 and yqhD. In some embodiments, the alcohol dehydrogenase, e.g., adh2 and yqhD, is encoded by at least one gene derived from *Saccharomyces cerevisiae.* In another embodiment, the alcohol dehydrogenase, e.g., adh2 and yqhD, is encoded by at least one gene derived from E. *coli.* In another embodiment, the alcohol dehydrogenase, e.g., adh2 and yqhD, is encoded by at least one gene derived from *Oryza sativa.* In another embodiment, the alcohol dehydrogenase, e.g., adh2 and yqhD, is encoded by at least one gene derived from *Penicillium brasilianum.* In another embodiment, the alcohol dehydrogenase, e.g., adh2 and yqhD, is encoded by at least one gene derived from *Bifidobacterium longum.*

In some embodiments, the at least one gene encoding the alcohol dehydrogenase has been codon-optimized for use in the recombinant bacterial cell. In some embodiments, the at least one gene encoding the alcohol dehydrogenase has been codon-optimized for use in *Escherichia coli.* For example, SEQ ID NOs:39 and 41 are codon-optimized sequences for *adh2* and *adh6,* respectively.

In some embodiments, the at least one gene encoding the alcohol dehydrogenase is the human alcohol dehydrogenase (ADH1A, ADH1C2, ADH1B1). In another embodiment, the at least one gene encoding the alcohol dehydrogenase comprises the human ADH1α, ADH1β and ADH1 γ subunits. In another embodiment, the at least one gene encoding the alcohol dehydrogenase catalyzes the oxidation of ethanol to acetaldehyde. Niederhut, et al., Protein science, 10:697-706 (2001).

When an alcohol dehydrogenase is expressed in the recombinant bacterial cells disclosed herein, the bacterial cells convert more branched chain amino acid-derived aldehydes to their respective alcohols than unmodified bacteria of the same bacterial subtype under the same conditions (*e.g*., culture or environmental conditions). Thus, the genetically engineered bacteria comprising at least one heterologous gene encoding an alcohol dehydrogenase may be used to catabolize excess branched chain amino acid-derived aldehydes, *e.g*., isovaleraldehyde, to treat a disease associated with a branched chain amino acid, including Maple Syrup Urine Disease (MSUD).

The present disclosure encompasses genes encoding an alcohol dehydrogenase comprising amino acids in its sequence that are substantially the same as an amino acid sequence described herein. The present disclosure further comprises genes encoding functional fragments of an alcohol dehydrogenase or functional variants of an alcohol dehydrogenase. As used herein, the term "functional fragment thereof' or "functional variant thereof' of an alcohol dehydrogenase gene relates to a sequence having qualitative biological activity in common with the wild-type alcohol dehydrogenase, from which the fragment or variant was derived. For example, a functional fragment or a functional variant of a mutated alcohol dehydrogenase is one which retains essentially the same ability to catabolize BCAAs as alcohol dehydrogenase from which the functional fragment or functional variant was derived. For example, a polypeptide having alcohol dehydrogenase activity may be truncated at the N-terminus or C-terminus and the retention alcohol dehydrogenase activity assessed using assays known to those of skill in the art, including the exemplary assays provided herein. In one embodiment, the recombinant bacterial cell disclosed herein comprises a heterologous gene encoding an αn alcohol dehydrogenase functional variant. In another embodiment, the recombinant bacterial cell disclosed herein comprises a heterologous gene encoding alcohol dehydrogenase functional fragment.

In some embodiments, the at least one gene encoding an alcohol dehydrogenase is mutagenized, mutants exhibiting increased activity are selected, and the mutagenized gene(s) encoding the alcohol dehydrogenase are isolated and inserted into the bacterial cell. In some embodiments, the at least one gene encoding the alcohol dehydrogenase is mutagenized, mutants exhibiting decreased activity are selected, and the mutagenized gene(s) encoding the alcohol dehydrogenase are isolated and inserted into the bacterial cell. The gene comprising the modifications described herein may be present on a plasmid or chromosome.

Assays for testing the activity of an alcohol dehydrogenase, an alcohol dehydrogenase functional variant, or an alcohol dehydrogenase functional fragment are well known to one of ordinary skill in the art. For example, alcohol dehydrogenase activity can be assessed by expressing the protein, functional variant, or fragment thereof, in a recombinant bacterial cell that lacks endogenous alcohol dehydrogenase activity. Also, activity can be assessed using the enzymatic assay methods as described by Kagi et al. (J. Biol. Chem., 235:3188-92, 1960), and Walker (Biochem. Educ., 20(1):42-43, 1992).

In some embodiments, the alcohol dehydrogenase is co-expressed with an additional branched chain amino acid catabolism enzyme. In some embodiments, the alcohol dehydrogenase is co-expressed with one or more additional branched chain amino acid catabolism enzyme(s) selected from a branched chain amino acid deaminase enzyme, e.g. a BCAA dehydrogenase, such as leucine dehydrogenase; a BCAA aminotransferase, e.g., ilvE, and a branched chain amino acid oxidase, e.g., L-AAD. In some embodiments, the alcohol dehydrogenase is co-expressed with one or more keto-acid decarboxylase enzyme(s), *e.g*., *kivD.* In some embodiments, the alcohol dehydrogenase is co-expressed with one or more other alcohol dehydrogenases. In some embodiments, the branched chain amino acid oxidase is co-expressed with one or more aldehyde dehydrogenases, e.g., padA. In some embodiments, the alcohol dehydrogenase is co-expressed with one or more keto-acid decarboxylase enzymes, *e.g*., *kivD* and one or more other alcohol dehydrogenase enzymes. In some embodiments, the alcohol dehydrogenase is co-expressed with one or more keto-acid decarboxylase enzymes, *e.g*., *kivD* and one or more aldehyde dehydrogenase enzymes, *e.g*., *padA.* In some embodiments, the alcohol dehydrogenase is co-expressed with one or more keto-acid decarboxylase enzymes, *e.g*., *kivD*, one or more aldehyde dehydrogenase enzymes, *e.g*., *padA,* and one or more other alcohol dehydrogenase enzymes.

In some embodiments, the alcohol dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more keto-acid decarboxylase enzyme(s), *e.g*., *kivD.* In some embodiments, the alcohol dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more other alcohol dehydrogenases. In some embodiments, the alcohol dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more aldehyde dehydrogenases, e.g., padA. In some embodiments, the alcohol dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, is co-expressed with one or more keto-acid decarboxylase enzyme(s), *e.g*., *kivD*, and co-expressed with one or more other alcohol dehydrogenases, and/or one or more aldehyde dehydrogenases, e.g., padA. In some embodiments, the at least one alcohol dehydrogenase enzyme is coexpressed with one or more BCAA transporter(s), for example, a high affinity leucine transporter, e.g., LivKHMGF and/or low affinity BCAA transporter BrnQ. In some embodiments, the at least one alcohol dehydrogenase enzyme is coexpressed with one or more BCAA binding protein(s), for example, LivJ. In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one alcohol dehydrogenase enzyme and genetic modification that reduces export of a branched chain amino acid, *e.g*., a genetic mutation in a *leuE* gene or promoter thereof. In one embodiment, the engineered bacteria comprise gene sequence(s) encoding at least one alcohol dehydrogenase enzyme and a genetic modification that reduces or eliminates branched chain amino acid synthesis, *e.g*., a genetic mutation in a *ilvC* gene or promoter thereof.

In some embodiments, the engineered bacteria comprise gene sequence(s) encoding at least one alcohol dehydrogenase enzyme and further comprise gene sequence encoding one or more polypeptides selected from other branched chain amino acid catabolism enzyme(s), BCAA transporter(s), and BCAA binding protein(s). In some embodiments, the engineered bacteria comprise gene sequence(s) encoding at least one alcohol dehydrogenase and gene sequence(s) encoding one or more branched chain amino acid dehydrogenase(s) (e.g., leuDH). In some embodiments, the engineered bacteria comprise gene sequence(s) encoding at least one alcohol dehydrogenase and gene sequence(s) encoding one or more amino acid oxidase(s), e.g., L-AAD. In some embodiments, the engineered bacteria comprise gene sequence(s) encoding at least one alcohol dehydrogenase and gene sequence(s) encoding one or more BCAA aminotransferase(s), e.g., ilvE. In some embodiments, the engineered bacteria comprise gene sequence(s) encoding at least one one alcohol dehydrogenase and gene sequence(s) encoding one or more keto-acid decarboxylase enzyme(s), *e.g*., *kivD.* In some embodiments, the engineered bacteria comprise gene sequence(s) encoding at least one one alcohol dehydrogenase and gene sequence(s) encoding one or more aldehyde dehydrogenase(s) (e.g., padA). In some embodiments, the engineered bacteria comprise gene sequence(s) encoding at least one one alcohol dehydrogenase and gene sequence(s) encoding one or more other alcohol dehydrogenase(s) (e.g., adh2, yqhD).

In some embodiments, the engineered bacteria comprise gene sequence(s) encoding at least one alcohol dehydrogenase and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or amino acid oxidase(s), e.g. L-AAD, and gene sequence(s) encoding one or more keto-acid decarboxylase enzyme(s), *e.g*., *kivD.* In some embodiments, the engineered bacteria comprise gene sequence(s) encoding at least one alcohol dehydrogenase and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or amino acid oxidase(s), e.g., L-AAD, gene sequence(s) encoding one or more keto-acid decarboxylase enzyme(s), *e.g*., *kivD*, and gene sequence(s) encoding one or more aldehyde dehydrogenase(s) (e.g., padA) and/or gene sequence(s) encoding one or more other alcohol dehydrogenase(s).

In some embodiments, the engineered bacteria comprise gene sequence(s) encoding at least one alcohol dehydrogenase and gene sequence(s) encoding one or more BCAA transporter(s) (e.g., LivKHMGF, brnQ). In some embodiments, the engineered bacteria comprise gene sequence(s) encoding at least one alcohol dehydrogenase and gene sequence(s) encoding one or more BCAA binding protein(s) (e.g., livJ). In some embodiments, the engineered bacteria comprise gene sequence(s) encoding at least alcohol dehydrogenase and gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one alcohol dehydrogenase and genetic modification that reduces export of a branched chain amino acid, *e.g*., a genetic mutation in a *leuE* gene or promoter thereof. In one embodiment, the engineered bacteria comprise gene sequence(s) encoding at least one alcohol dehydrogenase and a genetic modification that reduces or eliminates branched chain amino acid synthesis, *e.g*., a genetic mutation in a *ilvC* gene or promoter thereof.

In one embodiment, the at least one gene encoding the branched chain amino acid alcohol dehydrogenase comprises the *adh2* gene. In one embodiment, the *adh2* gene has at least about 80% identity with the sequence of SEQ ID NO:38. In one embodiment, the *adh2* gene has at least about 90% identity with the sequence of SEQ ID NO:38. In one embodiment, the *adh2* gene has at least about 95% identity with the sequence of SEQ ID NO:38. In one embodiment, the *adh2* gene has at least about 85%, 86%, 87%, 88%, 89%, 90%, 91%, 92%, 93%, 94%, 95%, 96%, 97%, 98%, or 99% identity with the sequence of SEQ ID NO:38. In another embodiment, the *adh2* gene comprises the sequence of SEQ ID NO:38. In yet another embodiment, the *adh2* gene consists of the sequence of SEQ ID NO:38.

In one embodiment, the at least one gene encoding the branched chain amino acid alcohol dehydrogenase comprises the *adh6* gene. In one embodiment, the *adh6* gene has at least about 80% identity with the sequence of SEQ ID NO:41. In one embodiment, the *adh6* gene has at least about 90% identity with the sequence of SEQ ID NO:41. In one embodiment, the *adh6* gene has at least about 95 % identity with the sequence of SEQ ID NO:41. In one embodiment, the *adh6* gene has at least about 85%, 86%, 87%, 88%, 89%, 90%, 91%, 92%, 93%, 94%, 95%, 96%, 97%, 98%, or 99% identity with the sequence of SEQ ID NO:41. In another embodiment, the *adh6* gene comprises the sequence of SEQ ID NO:41. In yet another embodiment, the *adh6* gene consists of the sequence of SEQ ID NO:41.

In one embodiment, the at least one gene encoding the branched chain amino acid alcohol dehydrogenase comprises the *adh1* gene. In one embodiment, the *adh1* gene has at least about 80% identity with the sequence of SEQ ID NO:43. In one embodiment, the *adh1* gene has at least about 90% identity with the sequence of SEQ ID NO:43. In one embodiment, the *adh1* gene has at least about 95 % identity with the sequence of SEQ ID NO:43. In one embodiment, the *adh1* gene has at least about 85%, 86%, 87%, 88%, 89%, 90%, 91%, 92%, 93%, 94%, 95%, 96%, 97%, 98%, or 99% identity with the sequence of SEQ ID NO:43. In another embodiment, the *adh1* gene comprises the sequence of SEQ ID NO:43. In yet another embodiment, the *adh1* gene consists of the sequence of SEQ ID NO:43.

In one embodiment, the at least one gene encoding the branched chain amino acid alcohol dehydrogenase comprises the *adh3* gene. In one embodiment, the *adh3* gene has at least about 80% identity with the sequence of SEQ ID NO:45. In one embodiment, the *adh3* gene has at least about 90% identity with the sequence of SEQ ID NO:45. In one embodiment, the *adh3* gene has at least about 95 % identity with the sequence of SEQ ID NO:45. In one embodiment, the *adh3* gene has at least about 85%, 86%, 87%, 88%, 89%, 90%, 91%, 92%, 93%, 94%, 95%, 96%, 97%, 98%, or 99% identity with the sequence of SEQ ID NO:45. In another embodiment, the *adh3* gene comprises the sequence of SEQ ID NO:45. In yet another embodiment, the *adh3* gene consists of the sequence of SEQ ID NO:45.

In one embodiment, the at least one gene encoding the branched chain amino acid alcohol dehydrogenase comprises the *adh4* gene. In one embodiment, the *adh4* gene has at least about 80% identity with the sequence of SEQ ID NO:47. In one embodiment, the *adh4* gene has at least about 90% identity with the sequence of SEQ ID NO:47. In one embodiment, the *adh4* gene has at least about 95% identity with the sequence of SEQ ID NO:47. In one embodiment, the *adh4* gene has at least about 85%, 86%, 87%, 88%, 89%, 90%, 91%, 92%, 93%, 94%, 95%, 96%, 97%, 98%, or 99% identity with the sequence of SEQ ID NO:47. In another embodiment, the *adh4* gene comprises the sequence of SEQ ID NO:47. In yet another embodiment, the *adh4* gene consists of the sequence of SEQ ID NO:47.

In one embodiment, the at least one gene encoding the branched chain amino acid alcohol dehydrogenase comprises the *adh5* gene. In one embodiment, the *adh5* gene has at least about 80% identity with the sequence of SEQ ID NO:49. In one embodiment, the *adh5* gene has at least about 90% identity with the sequence of SEQ ID NO:49. In one embodiment, the *adh5* gene has at least about 95 % identity with the sequence of SEQ ID NO:49. In one embodiment, the *adh5* gene has at least about 85%, 86%, 87%, 88%, 89%, 90%, 91%, 92%, 93%, 94%, 95%, 96%, 97%, 98%, or 99% identity with the sequence of SEQ ID NO:49. In another embodiment, the *adh5* gene comprises the sequence of SEQ ID NO:49. In yet another embodiment, the *adh5* gene consists of the sequence of SEQ ID NO:49.

In one embodiment, the at least one gene encoding the branched chain amino acid alcohol dehydrogenase comprises the *adh7* gene. In one embodiment, the *adh7* gene has at least about 80% identity with the sequence of SEQ ID NO:51. In one embodiment, the *adh7* gene has at least about 90% identity with the sequence of SEQ ID NO:51. In one embodiment, the *adh7* gene has at least about 95% identity with the sequence of SEQ ID NO:51. In one embodiment, the *adh7* gene has at least about 85%, 86%, 87%, 88%, 89%, 90%, 91%, 92%, 93%, 94%, 95%, 96%, 97%, 98%, or 99% identity with the sequence of SEQ ID NO:51. In another embodiment, the *adh7* gene comprises the sequence of SEQ ID NO:51. In yet another embodiment, the *adh7* gene consists of the sequence of SEQ ID NO:51.

In one embodiment, the at least one gene encoding the branched chain amino acid alcohol dehydrogenase comprises the *sfa1* gene. In one embodiment, the *sfa1* gene has at least about 80% identity with the sequence of SEQ ID NO:53. In one embodiment, the *sfa1* gene has at least about 90% identity with the sequence of SEQ ID NO:53. In one embodiment, the *sfa1* gene has at least about 95% identity with the sequence of SEQ ID NO:53. In one embodiment, the *sfa1* gene has at least about 85%, 86%, 87%, 88%, 89%, 90%, 91%, 92%, 93%, 94%, 95%, 96%, 97%, 98%, or 99% identity with the sequence of SEQ ID NO:53. In another embodiment, the *sfa1* gene comprises the sequence of SEQ ID NO:53. In yet another embodiment, the *sfa1* gene consists of the sequence of SEQ ID NO:53.

In one embodiment, the at least one gene encoding the branched chain amino acid alcohol dehydrogenase comprises the *YqhD* gene. In one embodiment, the *YqhD* gene has at least about 80% identity with the sequence of SEQ ID NO: 60. In one embodiment, the *YqhD* gene has at least about 90% identity with the sequence of SEQ ID NO: 60. In one embodiment, the *YghD* gene has at least about 95% identity with the sequence of SEQ ID NO: 60. In one embodiment, the *sfa1* gene has at least about 85%, 86%, 87%, 88%, 89%, 90%, 91%, 92%, 93%, 94%, 95%, 96%, 97%, 98%, or 99% identity with the sequence of SEQ ID NO:53. In another embodiment, the *YqhD* gene comprises the sequence of SEQ ID NO:53. In yet another embodiment, the *YqhD* gene consists of the sequence of SEQ ID NO:53.

In any of these embodiments, the alcohol dehydrogenase is coexpressed with one or more branched chain amino acid catabolism enzymes, *e.g*., *leuDH, ilvE, L-AAD, and*/*or kivD*, each of which are described in more detail herein. In other embodiments, the alcohol dehydrogenase is further coexpressed with a transporter of a branched chain amino acid and/or a binding protein of a BCAA.

In some embodiments, the gene sequence(s) encoding the one or more alcohol dehydrogenase is expressed under the control of a constitutive promoter. In some embodiments, the gene sequence(s) encoding the one or more b alcohol dehydrogenase is expressed under the control of an inducible promoter. In some embodiments, the gene sequence(s) encoding the one or more alcohol dehydrogenase is expressed under the control of a promoter that is directly or indirectly induced by exogenous environmental conditions. In some embodiments, the gene sequence(s) encoding the one or more alcohol dehydrogenase is expressed under the control of a promoter that is directly or indirectly induced by low-oxygen or anaerobic conditions, wherein expression of the gene encoding the alcohol dehydrogenase is activated under low-oxygen or anaerobic environments, such as the environment of the mammalian gut. In some embodiments, the gene sequence(s) encoding the one or more alcohol dehydrogenase is expressed under the control of a promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In one embodiment, wherein a branched chain amino acid catabolism enzyme is used to convert a ketoacid to its corresponding aldehyde, the recombinant bacterial cells of the invention may further comprise an aldehyde dehydrogenase enzyme in order to convert the branched chain amino acid-derived aldehyde to its respective carboxylic acid. In some embodiments, the engineered bacteria comprise gene sequence(s) encoding one or more copies of an aldehyde dehydrogenase. In some embodiments, the engineered bacteria comprise gene sequence(s) encoding one, two, three, four, five, six, or more copies of an aldehyde dehydrogenase. The one or more copies of an aldehyde dehydrogenase can be one or more copies of the same gene or can be different genes encoding aldehyde dehydrogenase, e.g., gene(s) from a different species or otherwise having a different gene sequence. The one or more copies of aldehyde dehydrogenase can be present in the bacterial chromosome or can be present in one or more plasmids. As used herein, "aldehyde dehydrogenase" refers to any polypeptide having enzymatic activity that catalyzes the conversion of a branched chain amino acid-derived aldehyde, *e.g*., isovaleraldehyde, isobutyraldehyde, 2-methylbutyraldehydeinto its respective carboxylic acid, *e.g*., isovalerate, isobutyrate, 2-methylbutyrate.

In some embodiments, the aldehyde dehydrogenase is encoded by at least one gene derived from a bacterial species. In some embodiments, the aldehyde dehydrogenase is encoded by at least one gene derived from a non-bacterial species. In some embodiments, the aldehyde dehydrogenase is encoded by at least one gene derived from a eukaryotic species, *e.g*., a yeast species or a plant species. In another embodiment, the aldehyde dehydrogenase is encoded by at least one gene derived from a mammalian species, e.g., human.

In one embodiment, the at least one gene encoding the aldehyde dehydrogenase is derived from an organism of the genus or species that includes, but is not limited to, *Acetinobacter, Azospirillum, Bacillus, Bacteroides, Bifidobacterium, Brevibacteria, Burkholderia, Citrobacter, Clostridium, Corynebacterium, Cronobacter, Enterobacter, Enterococcus, Erwinia, Helicobacter, Klebsiella, Lactobacillus, Lactococcus, Leishmania, Listeria, Macrococcus, Mycobacterium, Nakamurella, Nasonia, Nostoc, Pantoea, Pectobacterium, Proteus, Pseudomonas, Psychrobacter, Ralstonia, Saccharomyces, Salmonella, Sarcina, Serratia, Staphylococcus, Streptococcus, Escherichia coli and Yersinia, e.g., Acetinobacter radioresistens, Acetinobacter baumannii, Acetinobacter calcoaceticus, Azospirillum brasilense, Bacillus anthracis, Bacillus cereus, Bacillus coagulans, Bacillus megaterium, Bacillus subtilis, Bacillus thuringiensis, Bacteroides fragilis, Bacteroides subtilis, Bacteroides thetaiotaomicron, Bifidobacterium bifidum, Bifidobacterium infantis, Bifidobacterium lactis, Bifidobacterium longum, Burkholderia xenovorans, Citrobacter youngae, Citrobacter koseri, Citrobacter rodentium, Clostridium acetobutylicum, Clostridium butyricum, Corynebacterium aurimucosum, Corynebacterium kroppenstedtii, Corynebacterium striatum, Cronobacter sakazakii, Cronobacter turicensis, Enterobacter cloacae, Enterobacter cancerogenus, Enterococcus faecium, Enterococcus faecalis, Erwinia amylovara, Erwinia pyrifoliae, Erwinia tasmaniensis, Helicobacter mustelae, Klebsiella pneumonia, Klebsiella variicola, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis, Leishmania infantum, Leishmania major, Leishmania brazilensis, Listeria grayi, Macrococcus caseolyticus, Mycobacterium avium, Mycobacterium intracellulare, Mycobacterium kansasii, Mycobacterium leprae, Mycobacterium marinum, Mycobacterium smegmatis, Mycobacterium tuberculosis, Mycobacterium ulcerans, Nakamurella multipartita, Nasonia vitipennis, Nostoc punctiforme, Pantoea ananatis, Pantoea agglomerans, Pectobacterium atrosepticum, Pectobacterium carotovorum, Pseudomonas putida, Pseudomonas aeruginosa, Psychrobacter articus, Proteus vulgaris, Psychrobacter cryohalolentis, Ralstonia eutropha, Saccharomyces boulardii, Salmonella enterica, Sarcina ventriculi, Serratia odorifera, Serratia proteamaculans, Staphylococcus aerus, Staphylococcus capitis, Staphylococcys carnosus, Staphylococcus epidermidis, Staphylococcus hominis, Staphylococcus haemolyticus, Staphylococcus lugdunensis, Staphylococcus saprophyticus, Staphylococcus warneri, Streptococcus faecalis, Yersinia enterocolitica, Yersinia mollaretii, Yersinia kristensenii, Yersinia rohdei, and Yersinia aldovae.* In some embodiments, the aldehyde dehydrogenase is encoded by at least one gene derived from *Saccharomyces cerevisiae.* In another embodiment, the aldehyde dehydrogenase is encoded by at least one gene derived from *E. coli.* In another embodiment, the aldehyde dehydrogenase is encoded by at least one gene derived from *E. Coli K-12.*

In one embodiment the at least one gene encoding the branched chain amino acid dehydrogenase has been codon-optimized for use in the recombinant bacterial cell. In one embodiment, the at least one gene encoding the branched chain amino acid dehydrogenase has been codon-optimized for use in *Escherichia coli.*

When an aldehyde dehydrogenase is expressed in the recombinant bacterial cells disclosed herein, the bacterial cells convert more branched chain amino acid-derived aldehydes to their respective carboxylic acids than unmodified bacteria of the same bacterial subtype under the same conditions (*e.g*., culture or environmental conditions). Thus, the genetically engineered bacteria comprising at least one heterologous gene encoding an aldehyde dehydrogenase may be used to catabolize excess branched chain amino acid-derived aldehydes, *e.g*., isovaleraldehyde, to treat a disease associated with a branched chain amino acid, including Maple Syrup Urine Disease (MSUD). In some embodiments, the aldehyde dehydrogenase is co-expressed with one or more branched chain amino acid catabolism enzymes, *e.g*., *leuDH, ilvE,* L-AAD, and/or *kivD.*

The present disclosure encompasses genes encoding an aldehyde dehydrogenase comprising amino acids in its sequence that are substantially the same as an amino acid sequence described herein. The present disclosure further comprises genes encoding functional fragments of an aldehyde dehydrogenase or functional variants of an aldehyde dehydrogenase. As used herein, the term "functional fragment thereof' or "functional variant thereof' of an aldehyde dehydrogenase gene relates to a sequence having qualitative biological activity in common with the wild-type aldehyde dehydrogenase, from which the fragment or variant was derived. For example, a functional fragment or a functional variant of a mutated aldehyde dehydrogenase is one which retains essentially the same ability to catabolize BCAAs as aldehyde dehydrogenase from which the functional fragment or functional variant was derived. For example, a polypeptide having aldehyde dehydrogenase activity may be truncated at the N-terminus or C-terminus and the retention aldehyde dehydrogenase activity assessed using assays known to those of skill in the art, including the exemplary assays provided herein. In one embodiment, the recombinant bacterial cell disclosed herein comprises a heterologous gene encoding an αn aldehyde dehydrogenase functional variant. In another embodiment, the recombinant bacterial cell disclosed herein comprises a heterologous gene encoding alcohol dehydrogenase functional fragment.

In some embodiments, the at least one gene encoding an aldehyde dehydrogenase is mutagenized, mutants exhibiting increased activity are selected, and the mutagenized gene(s) encoding the aldehyde dehydrogenase are isolated and inserted into the bacterial cell. In some embodiments, the at least one gene encoding the aldehyde dehydrogenase is mutagenized, mutants exhibiting decreased activity are selected, and the mutagenized gene(s) encoding the aldehyde dehydrogenase are isolated and inserted into the bacterial cell. The gene comprising the modifications described herein may be present on a plasmid or chromosome.

Assays for testing the activity of an aldehyde dehydrogenase, an aldehyde dehydrogenase functional variant, or an aldehyde dehydrogenase functional fragment are well known to one of ordinary skill in the art. For example, aldehyde dehydrogenase activity can be assessed by expressing the protein, functional variant, or fragment thereof, in a recombinant bacterial cell that lacks endogenous aldehyde dehydrogenase activity. Also, activity can be assessed using the enzymatic assay methods as described by Kagi et al. (J. Biol. Chem., 235:3188-92, 1960), and Walker (Biochem. Educ., 20(1):42-43, 1992).

In some embodiments, the aldehyde dehydrogenase is co-expressed with an additional branched chain amino acid catabolism enzyme. In some embodiments, the aldehyde dehydrogenase is co-expressed with one or more additional branched chain amino acid catabolism enzyme(s) selected from a branched chain amino acid deaminase enzyme, e.g. a BCAA dehydrogenase, such as leucine dehydrogenase; a BCAA aminotransferase, e.g., ilvE, and a branched chain amino acid oxidase, e.g., L-AAD. In some embodiments, the aldehyde dehydrogenase is co-expressed with one or more keto-acid decarboxylase enzyme(s), *e.g*., *kivD.* In some embodiments, the aldehyde dehydrogenase is co-expressed with one or more alcohol dehydrogenases. In some embodiments, the branched chain amino acid oxidase is co-expressed with one or more other aldehyde dehydrogenases. In some embodiments, the aldehyde dehydrogenase is co-expressed with one or more keto-acid decarboxylase enzymes, *e.g*., *kivD* and one or more other aldehyde dehydrogenase enzymes. In some embodiments, the aldehyde dehydrogenase is co-expressed with one or more keto-acid decarboxylase enzymes, *e.g*., *kivD* and one or more alcohol dehydrogenase enzymes. In some embodiments, the aldehyde dehydrogenase is co-expressed with one or more keto-acid decarboxylase enzymes, *e.g*., *kivD*, one or more other aldehyde dehydrogenase enzymes and one or more alcohol dehydrogenase enzymes.

In some embodiments, the aldehyde dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more keto-acid decarboxylase enzyme(s), *e.g*., *kivD.* In some embodiments, the aldehyde dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more alcohol dehydrogenases. In some embodiments, the aldehyde dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, and is co-expressed with one or more other aldehyde dehydrogenases. In some embodiments, the aldehyde dehydrogenase is co-expressed with an another branched chain amino acid deaminase enzyme, e.g. a BCAA dehydrogenase, such as leucine dehydrogenase, a BCAA aminotransferase, e.g., ilvE, and/or amino acid oxidase, e.g., L-AAD, is co-expressed with one or more keto-acid decarboxylase enzyme(s), *e.g*., *kivD*, and co-expressed with one or more alcohol dehydrogenases, and/or one or more other aldehyde dehydrogenases. In some embodiments, the at least one aldehyde dehydrogenase enzyme is coexpressed with one or more BCAA transporter(s), for example, a high affinity leucine transporter, e.g., LivKHMGF and/or low affinity BCAA transporter BrnQ. In some embodiments, the at least one aldehyde dehydrogenase enzyme is coexpressed with one or more BCAA binding protein(s), for example, LivJ. In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one aldehyde dehydrogenase enzyme and genetic modification that reduces export of a branched chain amino acid, *e.g*., a genetic mutation in a *leuE* gene or promoter thereof. In one embodiment, the engineered bacteria comprise gene sequence(s) encoding at least one aldehyde dehydrogenase enzyme and a genetic modification that reduces or eliminates branched chain amino acid synthesis, *e.g*., a genetic mutation in a *ilvC* gene or promoter thereof.

In some embodiments, the engineered bacteria comprise gene sequence(s) encoding at least one aldehyde dehydrogenase enzyme and further comprise gene sequence encoding one or more polypeptides selected from other branched chain amino acid catabolism enzyme(s), BCAA transporter(s), and BCAA binding protein(s). In some embodiments, the engineered bacteria comprise gene sequence(s) encoding at least one aldehyde dehydrogenase and gene sequence(s) encoding one or more branched chain amino acid dehydrogenase(s) (e.g., leuDH). In some embodiments, the engineered bacteria comprise gene sequence(s) encoding at least one aldehyde dehydrogenase and gene sequence(s) encoding one or more amino acid oxidase(s), e.g., L-AAD. In some embodiments, the engineered bacteria comprise gene sequence(s) encoding at least one aldehyde dehydrogenase and gene sequence(s) encoding one or more BCAA aminotransferase(s), e.g., ilvE. In some embodiments, the engineered bacteria comprise gene sequence(s) encoding at least one one aldehyde dehydrogenase and gene sequence(s) encoding one or more keto-acid decarboxylase enzyme(s), *e.g*., *kivD.* In some embodiments, the engineered bacteria comprise gene sequence(s) encoding at least one one aldehyde dehydrogenase and gene sequence(s) encoding one or more other aldehyde dehydrogenase(s). In some embodiments, the engineered bacteria comprise gene sequence(s) encoding at least one one aldehyde dehydrogenase and gene sequence(s) encoding one or more alcohol dehydrogenase(s).

In some embodiments, the engineered bacteria comprise gene sequence(s) encoding at least one aldehyde dehydrogenase and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or amino acid oxidase(s), e.g. L-AAD, and gene sequence(s) encoding one or more keto-acid decarboxylase enzyme(s), *e.g*., *kivD.* In some embodiments, the engineered bacteria comprise gene sequence(s) encoding at least one aldehyde dehydrogenase and gene sequence(s) encoding one or more other branched chain amino acid catabolism enzyme(s), for example, branched chain amino acid dehydrogenase(s), such as leuDH, branched chain amino acid aminotransferase enzyme, e.g., ilvE, and/or amino acid oxidase(s), e.g., L-AAD, gene sequence(s) encoding one or more keto-acid decarboxylase enzyme(s), *e.g*., *kivD,* and gene sequence(s) encoding one or more other aldehyde dehydrogenase(s) and/or gene sequence(s) encoding one or more alcohol dehydrogenase(s).

In some embodiments, the engineered bacteria comprise gene sequence(s) encoding at least one aldehyde dehydrogenase and gene sequence(s) encoding one or more BCAA transporter(s) (e.g., LivKHMGF, brnQ). In some embodiments, the engineered bacteria comprise gene sequence(s) encoding at least one aldehyde dehydrogenase and gene sequence(s) encoding one or more BCAA binding protein(s) (e.g., livJ). In some embodiments, the engineered bacteria comprise gene sequence(s) encoding at least one aldehyde dehydrogenase and gene sequence(s) encoding one or more BCAA transporter(s) (e.g., LivKHMGF, brnQ), and gene sequence(s) encoding one or more BCAA binding protein(s) (e.g., livJ). In one specific embodiment, the gene sequence encoding LivKHMGF is SEQ ID NO: 91. In another specific embodiment, the gene sequence encoding brnQ is SEQ ID NO: 64. In another specific embodiment, the gene sequence encoding livJ is SEQ ID NO: 12.

In some embodiments, the engineered bacteria comprise gene sequence(s) encoding at least one aldehyde dehydrogenase and genetic modification that reduces export of a branched chain amino acid, *e.g*., a genetic mutation in a *leuE* gene or promoter thereof. In one embodiment, the engineered bacteria comprise gene sequence(s) encoding at least one aldehyde dehydrogenase and a genetic modification that reduces or eliminates branched chain amino acid synthesis, *e.g*., a genetic mutation in a *ilvC* gene or promoter thereof.

In some embodiments, the gene sequence(s) encoding the one or more aldehyde dehydrogenase is expressed under the control of a constitutive promoter. In some embodiments, the gene sequence(s) encoding the one or more aldehyde dehydrogenase is expressed under the control of an inducible promoter. In some embodiments, the gene sequence(s) encoding the one or more aldehyde dehydrogenase is expressed under the control of a promoter that is directly or indirectly induced by exogenous environmental conditions. In some embodiments, the gene sequence(s) encoding the one or more aldehyde dehydrogenase is expressed under the control of a promoter that is directly or indirectly induced by low-oxygen or anaerobic conditions, wherein expression of the gene encoding the aldehyde dehydrogenase is activated under low-oxygen or anaerobic environments, such as the environment of the mammalian gut. In some embodiments, the gene sequence(s) encoding the one or more aldehyde dehydrogenase is expressed under the control of a promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In one embodiment, the at least one gene encoding the branched chain amino acid aldehyde dehydrogenase comprises the *PadA* gene. In one embodiment, the *PadA* gene has at least about 80% identity with the sequence of SEQ ID NO: 62. In one embodiment, the *PadA* gene has at least about 90% identity with the sequence of SEQ ID NO: 62. In one embodiment, the *PadA* gene has at least about 95% identity with the sequence of SEQ ID NO: 62. In one embodiment, the *PadA* gene has at least about 85%, 86%, 87%, 88%, 89%, 90%, 91%, 92%, 93%, 94%, 95%, 96%, 97%, 98%, or 99% identity with the sequence of SEQ ID NO: 62. In another embodiment, the *PadA* gene comprises the sequence of SEQ ID NO: 62. In yet another embodiment, the *PadA* gene consists of the sequence of SEQ ID NO: 62.

In some embodiments, the aldehyde dehydrogenase, e.g., padA, is coexpressed with one or more branched chain amino acid catabolism enzymes, *e.g., leuDH, ilvE, L-AAD,* or *kivD,* each of which are described in more detail herein. In another embodiment, the aldehyde dehydrogenase is further coexpressed with a transporter of a branched chain amino acid.

### E. LIU Operon

In one embodiment, the branched chain amino acid catabolism enzyme comprises the enzymes expressed by the Liu operon. LiuA is a terpenoid-specific, FAD-dependent acyl-CoA dehydrogenase that catalyzes the formation of a carbon-carbon double bond in methyl-branched substrates, similar to citronellyl-CoA dehydrogenase. The gene encoding this enzyme is part of the L-leucine and isovalerate utilizing liuABCDE gene cluster in this organism. A liuA insertion mutant was unable to utilize acyclic terpenes, L-leucine or isovalerate, but could utilize succinate (ForsterFromme and Jendrossek;Biochemical characterization of isovaleryl-CoA dehydrogenase (LiuA) of Pseudomonas aeruginosa and the importance of liu genes fora functional catabolic pathway of methyl-branched compounds. FEMS Microbiol Lett. 2008 Sep;286(1):78-84.] (see pathways citronellol degradation, cis-genanyl-CoA degradation and L-leucine degradation I).

In some embodiments, wherein a branched chain amino acid catabolism enzyme is a a branched chain keto acid dehydrogenase is used to oxidatively decarboxylate all three branched chain keto acids (α-ketoisocaproate, α-keto-β-methylvalerate, and α-ketoisovalerate) into their respective acyl-CoA derivatives, (isovaleryl-CoA, α-methylbutyryl-CoA, isobutyryl-CoA), the recombinant bacterial cells may further comprise an Liu operon, which can convert isovaleryl-CoA to acetoacetate and acetylCoA. In some embodiments, a Liu operon circuit is useful in combination with a Bkd complex, e.g., in the treatment of MSUD. Alternatively, in some embodiments, the bacterial cells comprise a Liu operon circuit, e.g., in the absence of a Bkd complex. In some embodiments, the Liu operon is useful in the absence of the Bkd complex, e.g., in the treatment of isovaleria academia.

Isovaleric academia ((OMIM) 243500), also called isovaleric aciduria or isovaleric acid CoA dehydrogenase deficiency is a rare autosomal recessive (Lee et al., Different spectrum of mutations of isovaleryl-CoA dehydrogenase (IVD) gene in Korean patients with isovaleric academia; Mol Genet Metab. 2007 Sep-Oct; 92(0): 71-77) metabolic disorder which disrupts or prevents normal metabolism of the branched-chain amino acid leucine (Dionisi-Vici et al., J Inherit Metab Dis. 2006 Apr-Jun;29(2-3):383-9. In isovaleric academia patients, a mutation in the gene encoding *IVD* (isovaleric acid-CoA dehydrogenase; EC 1.3.8.4), blocks the third step in leucine degradation and leads to toxic levels of isovalerate, damaging the brain and nervous system. In some embodiments of the disclosure, the amount of isovaleric acid using a synthetic probiotic strain is reduced, e.g. for the treatment of isovaleric acidemia. In one embodiments, leucine and its ketoacid alpha-ketoisocaproate, are degraded similar to the strategy used to treat MSUD. In another embodiment, isovalerate is first converted to isovaleryl-CoA by expressing an acyl-CoA synthetase with activity against isovalerate (isovaleryl-CoA synthetase), then metabolized isovaleryl-CoA to acetoacetate and acetyl-CoA by expressing four additional enzymes: an isovaleryl-CoA dehydrogenase, a 3-methylcrotonyl-CoA carboxylase, a 3-methylglutaconyl-CoA hydratase and an hydroxymethylglutaryl-CoA lyase. In one embodiment, a Liu Operon is used. In one embodiment, the genetically engineered bacteria express an acyl CoA synthetase, which can convert isovalerate to isovaleryl-CoA. In one embodiment the acyl CoA synthetase is Lbul. In one embodiment, a acyl-CoA synthetase is used in the treatment of isovaleric academia.

'Classical' organic acidurias, propionic aciduria, methylmalonic aciduria and isovaleric aciduria: long-term outcome and effects of expanded newborn screening using tandem mass spectrometry). It is a classical type of organic acidemia.

### Transporter (Importer) of a Branched Chain Amino Acid

In some embodiments, a recombinant bacterial cell disclosed herein comprising gene sequence(s) encoding at least one branched chain amino acid catabolism enzyme (e.g., in some embodiments expressed on a high-copy plasmid) does not increase branched chain amino acid catabolism or decrease branched chain amino acid levels in the absence of a heterologous transporter (importer) of the branched chain amino acid, *e.g*., leucine, and additional copies of a native importer of the branched chain amino acid, *e.g.*, *livKHMGF.* It has been surprisingly discovered that in some embodiments, the rate-limiting step of branched chain amino acid catabolism, *e.g*., leucine catabolism, is *not* expression of a branched chain amino acid catabolism enzyme, but rather availability of the branched chain amino acid, *e.g*., leucine (*see, e.g*., **Fig. 18**). Thus, in some embodiments, it may be advantageous to increase branched chain amino acid transport, *e.g*., leucine transport, into the cell, thereby enhancing branched chain amino acid catabolism. Surprisingly, in conjunction with overexpression of a transporter of a branched chain amino acid, *e.g*., LivKHMGF, even low copy number plasmids comprising a gene encoding at least one branched chain amino acid catabolism enzyme are capable of almost completely eliminating a branched chain amino acid, *e.g*., leucine, from a sample (*see*, *e.g*., **Fig. 18**). Furthermore, in some embodiments that incorporate a transporter of a branched chain amino acid into the recombinant bacterial cell, there may be additional advantages to using a low-copy plasmid comprising the gene encoding the branched chain amino acid catabolism enzyme in conjunction in order to enhance the stability of expression of the branched chain amino acid catabolism enzyme, while maintaining high branched chain amino acid catabolism and to reduce negative selection pressure on the transformed bacterium. In alternate embodiments, the branched chain amino acid transporter is used in conjunction with a high-copy plasmid. In alternate embodiments, the gene(s)at least one BCAA catabolism enzyme is integrated in the bacterial chromosome.

In some embodiments, in which the engineered bacterial cell comprises gene sequence encoding a branched amino acid transporter, the bacterial cell comprises gene sequence encoding one branched chain amino acid catabolism enzyme. In other embodiments, in which the engineered bacterial cell comprises gene sequence encoding a branched amino acid transporter, the bacterial cell comprises gene sequence(s) encoding two branched chain amino acid catabolism enzymes. In other embodiments, in which the engineered bacterial cell comprises gene sequence encoding a branched amino acid transporter, the bacterial cell comprises gene sequence(s) encoding three or more branched chain amino acid catabolism enzymes. In other embodiments, in which the engineered bacterial cell comprises gene sequence encoding a branched amino acid transporter, the bacterial cell comprises gene sequence(s) encoding four, five, six or more branched chain amino acid catabolism enzymes.

In some embodiments, the branched chain amino acid catabolism enzyme converts a branched chain amino acid, e.g., leucine, valine, isoleucine, into its corresponding branched chain alpha-keto acid counterpart. In other embodiments, the branched chain amino acid catabolism enzyme converts a branched chain alpha-keto acid, e.g., alpha-ketoisocaproate, alpha-keto- beta- methylvalerate, alpha-ketoisovalerate into its corresponding aldehyde. In other embodiments, the branched chain amino acid catabolism enzyme converts a branched chain alpha-keto acid, e.g., alpha-ketoisocaproate, alph keto-beta- methylvalerate, alpha-ketoisovalerate into its corresponding acetyl-CoA, e.g., isovaleryl-CoA, alpha-methylbutyryl-CoA, isobutyl-CoA. In other embodiments, the branched chain amino acid catabolism enzyme converts a branched chain aldehyde to its corresponding alcohol. In another embodiment, the branched chain amino acid catabolism enzyme converts a branched chain aldehyde to its corresponding carboxylic acid.

In some embodiments, the engineered bacteria comprising gene sequence(s) encoding one or more BCAA transporter(s), further comprise gene sequence(s) encoding one or more BCAA catabolism enzymes selected from BCAA dehydrogenase, e.g., leuDH, BCAA amino transferase, e.g., ilvE, and amino axidase, e.g., L-AAD. In some embodiments, the engineered bacteria comprising gene sequence(s) encoding one or more BCAA transporter(s), further comprise gene sequence(s) encoding one or more BCAA catabolism enzymes selected from BCAA dehydrogenase, e.g., leuDH, BCAA amino transferase, e.g., ilvE, and amino axidase, e.g., L-AAD and gene sequnec(s) encoding one or more branched chain keto acid dehydrogenase enzyme(s) (BCKD). In some embodiments, the engineered bacteria comprising gene sequence(s) encoding one or more BCAA transporter(s), further comprise gene sequence(s) encoding one or more BCAA catabolism enzymes selected from BCAA dehydrogenase, e.g., leuDH, BCAA amino transferase, e.g., ilvE, and amino axidase, e.g., L-AAD and gene sequnec(s) encoding one or more keto acid decarboxylase enzyme(s), e.g., kivD. In some embodiments, the engineered bacteria comprising gene sequence(s) encoding one or more BCAA transporter(s), further comprise gene sequence(s) encoding one or more BCAA catabolism enzymes selected from BCAA dehydrogenase, e.g., leuDH, BCAA amino transferase, e.g., ilvE, and amino axidase, e.g., L-AAD and gene sequnec(s) encoding one or more keto acid decarboxylase enzyme(s), e.g., kivD, and gene sequence(s) encoding one or more alcohol dehydrogenase enzyme(s) and/or gene sequence(s) encoding one or more aldehyde dehydrogenase enzyme(s). ). In any of these embodiments, the engineered bacteria also comprise a genetic modification that reduces export of a branched chain amino acid, *e.g*., a genetic mutation in a *leuE* gene or promoter thereof. In any of these embodiments, the bacteria also comprise a genetic modification that reduces or eliminates branched chain amino acid synthesis, *e.g*., a genetic mutation in a *ilvC* gene or promoter thereof. In one embodiment, the bacterial cell comprises gene sequence encoding one or more transporter(s) of branched chain amino acids, gene sequence(s) encoding one or more branched chain amino acid catabolism enzyme(s), and at least one genetic modification that reduces export of a branched chain amino acid, e.g., genetic modification in a leuE gene or promoter thereof. In one embodiment, the bacterial cell comprises gene sequence encoding one or more transporter(s) of branched chain amino acids, gene sequence(s) encoding one or more branched chain amino acid catabolism enzyme(s), and at least one genetic modification that reduces or eliminates branched chain amino acid synthesis, *e.g*., a genetic mutation in a *ilvC* gene or promoter thereof. In one embodiment, the bacterial cell comprises gene sequence encoding one or more transporter(s) of branched chain amino acids, gene sequence(s) encoding one or more branched chain amino acid catabolism enzyme(s), at least one genetic modification that reduces export of a branched chain amino acid., and at least one genetic modification that reduces or eliminates branched chain amino acid synthesis. In any of these embodiments, the engineered bacterial cell may further comprise gene sequence encoding *livJ*, which brings BCAA into the bacterial cell.

The uptake of branched chain amino acids into bacterial cells is mediated by proteins well known to those of skill in the art. For example, two well characterized BCAA transport systems have been characterized in several bacteria, including *Escherichia coli.* BCAAs are transported by two systems into bacterial cells (*i.e*., imported), the osmotic-shock-sensitive systems designated LIV-I and LS (leucine-specific), and by an osmotic-shock resistant system, BrnQ, formerly known as LIV-II (*see* Adams et al., J. Biol. Chem. 265:11436-43 (1990); Anderson and Oxender, J. Bacteriol. 130:384-92 (1977); Anderson and Oxender, J. Bacteriol. 136:168-74 (1978); Haney et al., J. Bacteriol. 174:108-15 (1992); Landick and Oxender, J. Biol. Chem. 260:8257-61 (1985); Nazos et al., J. Bacteriol. 166:565-73 (1986); Nazos et al., J. Bacteriol. 163:1196-202 (1985); Oxender et al., Proc. Natl. Acad. Sci. USA 77:1412-16 (1980); Quay et al., J. Bacteriol. 129:1257-65 (1977); Rahmanian et al., J. Bacteriol. 116:1258-66 (1973); Wood, J. Biol. Chem. 250:4477-85 (1975); Guardiola et al., J. Bacteriol. 117:393-405 (1974); Guardiola and Iaccarino, J. Bacteriol. 108:1034-44 (1971); Ohnishi et al., Jpn. J. Genet. 63:343-57 )(1988); Yamato and Anraku, J. Bacteriol. 144:36-44 (1980); and Yamato et al., J. Bacteriol. 138:24-32 (1979)). Transport by the BrnQ system is mediated by a single membrane protein. Transport mediated by the LIV-I system is dependent on the substrate binding protein LivJ (also known as LIV-BP), while transport mediated by LS system is mediated by the substrate binding protein LivK (also known as LS-BP). LivJ is encoded by the *livJ* gene, and binds isoleucine, leucine and valine with K_{d} values of ~10⁻⁶ and ~10⁻⁷ M, while LivK is encoded by the *livK* gene, and binds leucine with a K_{d} value of ~10⁻⁶ M (*See* Landick and Oxender, J. Biol. Chem. 260:8257-61 (1985)). Both LivJ and LivK interact with the inner membrane components LivHMGF to enable ATP-hydrolysis-coupled transport of their substrates into the cell, forming the LIV-I and LS transport systems, respectively. The LIV-I system transports leucine, isoleucine and valine, and to a lesser extent serine threonine and alanine, whereas the LS system only transports leucine. The six genes encoding the E. *coli* LIV-I and LS systems are organized into two transcriptional units, with *livKHMGF* transcribed as a single operon, and *livJ* transcribed separately. LivKHMGF ia an ABC transporter comprised of five subunits, including LivK, which is a periplasmic amino acid binding protein, LivHM, which are memebrane subunits, and LivGF, which are ATP-binding subunits. The *Escherichia coli liv* genes can be grouped according to protein function, with the *livJ* and *livK* genes encoding periplasmic binding proteins with the binding affinities described above, the *livH* and *livM* genes encoding inner membrane permeases, and the *livG* and *livF* genes encoding cytoplasmic ATPases.

BrnQ is a branched chain amino acid transporter highly similar to the Salmonella typhimurium BrnQ branched chain amino acid transporter (Ohnishi et al., Cloning and nucleotide sequence of the brnQ gene, the structural gene for a membrane-associated component of the LIV-II transport system for branched-chain amino acids in Salmonella typhimurium. Jpn J Genet. 1988 Aug;63(4):343-57) and corresponds to the Liv-II branched chain amino acid transport system in E. coli, which has been shown to transport leucine, valine, and isoleucine (Guardiola et al., Mutations affecting the different transport systems for isoleucine, leucine, and valine in Escherichia coli K-12. J Bacteriol. 1974 Feb;117(2):393-405), Guardiola and Oxender, Genetic separation of high- and low-affinity transport systems for branched-chain amino acids in Escherichia coli K-12. J Bacteriol. 1978 Oct;136(1):168-74., Anderson and Oxender, Genetic separation of high- and low-affinity transport systems for branched-chain amino acids in Escherichia coli K-12 J Bacteriol. 1978 Oct;136(1):168-74.78). BrnQ is a member of the LIVCS family of branched chain amino acid transporters and likely functions as a sodium/branched chain amino acid symporter.

Branched chain amino acid transporters, *e.g*., leucine importers, may be expressed or modified in the bacteria disclosed herein in order to enhance branched chain amino acid, *e.g*., leucine, transport into the cell. For example, the gene sequence(s) for endogenous transporter(s) may be modified (e.g., codon-optimized and/or expressed by a strong promoter) to overexpress the transporter and/or additional copies of the transporter may be added. Alternatively or additionally, gene sequence(s) for one or more non-endogenous or non-native transporters may be expressed in the bacterial cell. Specifically, when the transporter of a branched chain amino acid is expressed in the recombinant bacterial cells disclosed herein, the bacterial cells import more branched chain amino acids into the cell when the transporter is expressed than unmodified bacteria of the same bacterial subtype under the same conditions (not expressing the transporter). Thus, in some embodiments, the engineered bacteria comprise gene sequence(s) encoding one or more transporter(s) of a branched chain amino acid, *e.g.*, leucine, which may be used to import branched chain amino acids, *e.g*., leucine, into the bacteria so that any gene encoding a branched chain amino acid catabolism enzyme expressed in the bacteria catabolize the branched chain amino acid, *e.g*., leucine, to treat diseases associated with the catabolism of branched chain amino acids, such as Maple Syrup Urine Disease (MSUD). In one embodiment, the bacterial cell comprises gene sequence(s) encoding one or more transporter(s) of branched chain amino acids. In one embodiment, the bacterial cell comprises gene sequence(s) encoding one or more transporter(s) of branched chain amino acids and a gene sequence(s) encoding one or more branched chain amino acid catabolism enzyme(s). In one embodiment, the bacterial cell comprises gene sequence(s) encoding a one or more transporter(s) of branched chain amino acids and a genetic modification that reduces export of a branched chain amino acid, *e.g*., a genetic mutation in a *leuE* gene or promoter thereof. In one embodiment, the bacterial cell comprises gene sequence(s) encoding one or more transporter(s) of branched chain amino acids and a genetic modification that reduces or eliminates branched chain amino acid synthesis, *e.g*., a genetic mutation in a *ilvC* gene or promoter thereof. In one embodiment, the bacterial cell comprises gene sequence encoding one or more transporter(s) of branched chain amino acids, gene sequence(s) encoding one or more branched chain amino acid catabolism enzyme(s), and at least one genetic modification that reduces export of a branched chain amino acid. In one embodiment, the bacterial cell comprises gene sequence encoding one or more transporter(s) of branched chain amino acids, gene sequence(s) encoding one or more branched chain amino acid catabolism enzyme(s), and at least one genetic modification that reduces or eliminates branched chain amino acid synthesis. In one embodiment, the bacterial cell comprises gene sequence encoding one or more transporter(s) of branched chain amino acids, gene sequence(s) encoding one or more branched chain amino acid catabolism enzyme(s), at least one genetic modification that reduces export of a branched chain amino acid., and at least one genetic modification that reduces or eliminates branched chain amino acid synthesis. In any of these embodiments, the engineered bacterial cell may further comprise gene sequence encoding *livJ*, which brings BCAA into the bacterial cell. In any of these embodiments, the transporter may be a native transporter, e.g., the bacteria may comprise additional copies of the native transporter. In any of these embodiments, the transporter may be a non-native transporter. In any of these embodiments, the transporter may be LivKHMGF. In any of these embodiments, the transporter may be brnQ. In any of these embodiments, the bacterial cell may comprise gene sequence(s) encoding LivKHMGF and brnQ.

In some embodiments, the engineered bacteria comprise gene sequene(s) encoding one or more BCAA catabolism enzyme(s) and gene sequence(s) encoding one or more BCAA transporters, in which the gene sequene(s) encoding one or more BCAA catabolism enzyme(s) and the gene sequene(s) encoding one or more transporter(s) are operably linked to different copies of the same promoter. In some embodiments, the engineered bacteria comprise gene sequene(s) encoding one or more BCAA catabolism enzyme(s) and gene sequence(s) encoding one or more BCAA transporters, in which the gene sequene(s) encoding one or more BCAA catabolism enzyme(s) and the gene sequene(s) encoding one or more transporter(s) are operably linked to different promoters. Thus, in some embodiments, the disclosure provides a bacterial cell that comprises gene sequence(s) encoding one or more branched chain amino acid catabolism enzyme(s) operably linked to a first promoter and gene sequence encoding one or more transporter(s) of a branched chain amino acid, *e.g*., leucine. In some embodiments, the disclosure provides a bacterial cell that comprises gene sequence(s) encoding one or more transporters of a branched chain amino acid operably linked to the first promoter. In other embodiments, the disclosure provides a bacterial cell omprisesing gene sequence(s) encoding one or more branched chain amino acid catabolism enzyme(s) operably linked to a first promoter and gene sequence(s) encoding one or more transporter(s) of a branched chain amino acid operably linked to a second promoter. In one embodiment, the first promoter and the second promoter are separate copies of the same promoter. In another embodiment, the first promoter and the second promoter are different promoters. In one embodiment, the first promoter and the second promoter are inducible promoters. In another embodiment, the first promoter is an inducible promoter and the second promoter is a constitutive promoter. In some embodiments, the gene sequence(s) encoding the one or more BCAA catabolism enzymes and the gene sequence(s) encoding the one or more transporters is expressed under the control of a constitutive promoter. In some embodiments, the gene sequence(s) encoding the one or more BCAA catabolism enzymes and the gene sequence(s) encoding the one or more BCAA transporters is expressed under the control of an inducible promoter. In some embodiments, the gene sequence(s) encoding the one or more BCAA transporters is expressed under the control of an inducible promoter that is directly or indirectly induced by exogenous environmental conditions. In some embodiments, the gene sequence(s) encoding the one or more BCAA catabolism enzymes and the gene sequence(s) encoding the one or more BCAA transporters is expressed under the control of an inducible promoter that is directly or indirectly induced by exogenous environmental conditions. In some embodiments, the gene sequence(s) encoding the one or more BCAA transporters is expressed under the control of an inducible promoter that is directly or indirectly induced by low-oxygen or anaerobic conditions, wherein expression of the gene encoding the one or more transporters is activated under low-oxygen or anaerobic environments, such as the environment of the mammalian gut. In some embodiments, the gene sequence(s) encoding the one or more BCAA catabolism enzymes and the gene sequence(s) encoding the one or more BCAA transporters is expressed under the control of an inducible promoter that is directly or indirectly induced by low-oxygen or anaerobic conditions, wherein expression of the gene(s) encoding the one or more BCAA cataboliam enzymes and expression of the gene(s) encoding the one or more BCAA transporters is activated under low-oxygen or anaerobic environments, such as the environment of the mammalian gut. In some embodiments, the gene sequence(s) encoding the one or more BCAA transporters is expressed under the control of an inducible promoter that is directly or indirectly induced by inflammatory conditions. In some embodiments, the gene sequence(s) encoding the one or more BCAA catabolism enzymes and the gene sequence(s) encoding the one or more BCAA transporters is expressed under the control of an inducible promoter that is directly or indirectly induced by inflammatory conditions. Exemplary inducible promoters described herein include oxygen level-dependent promoters (*e.g*., FNR-inducible promoter), promoters induced by inflammation or an inflammatory response (RNS, ROS promoters), and promoters induced by a metabolite that may or may not be naturally present (e.g., can be exogenously added) in the gut, e.g., arabinose and tetracycline. Examples of inducible promoters include, but are not limited to, an FNR responsive promoter, a P_{araC} promoter, a P_{araBAD} promoter, and a P_{TetR} promoter, each of which are described in more detail herein.

In one embodiment, the bacterial cell comprises at least one gene encoding a transporter of a branched chain amino acid from a different organism, *e.g*., a different species of bacteria. In one embodiment, the bacterial cell comprises at least one native gene encoding a transporter of a branched chain amino acid. In some embodiments, the at least one native gene encoding a transporter of a branched chain amino acid is not modified. In another embodiment, the bacterial cell comprises more than one copy of at least one native gene encoding a transporter of a branched chain amino acid. In yet another embodiment, the bacterial cell comprises a copy of at least one gene encoding a native transporter of a branched chain amino acid, as well as at least one copy of at least one heterologous gene encoding a transporter of a branched chain amino acid. The heterologous gene sequence may encode an additional copy or copies of the native transporter, may encode one or more copies of a non-native transporter, and/or may encode one or more copies of a homologous or different transporter from a different bacterial species. In one embodiment, the bacterial cell comprises at least one, two, three, four, five, or six copies of the at least one heterologous gene encoding a transporter of a branched chain amino acid. In one embodiment, the bacterial cell comprises multiple copies of the at least one heterologous gene encoding a transporter of a branched chain amino acid. In one embodiment, the bacterial cell comprises gene sequence(s) encoding two or more different transporters of a branched chain amino acid. In one embodiment, the gene sequence(s) encoding two or more different transporters of a branched chain amino acid is under the control of one or more inducible promoters. In one embodiment, the gene sequence(s) encoding two or more different transporters of a branched chain amino acid is under the control of one or more constitutive promoters. In one embodiment, the gene sequence(s) encoding two or more different transporters of a branched chain amino acid is under the control of at least one inducible promoter and at least one constitutive promoter. In any of these embodiments, the gene sequence(s) encoding the one or more BCAA transporter(s) may be present on one or more plasmids. In any of these embodiments, the gene sequence(s) encoding the one or more BCAA transporter(s) may be present in the bacterial chromosome.

In one embodiment, the transportre of a branched chain amino acid imports a branch chain amino acid into the bacterial cell. In one embodiment, the transporterof a branched chain amino acid imports leucine into the bacterial cell. In one embodiment, the transporter of a branched chain amino acid imports isoleucine into the bacterial cell. In one embodiment, the transporter of a branched chain amino acid imports valine into the bacterial cell. In one ambodiment, the transporter of a branched chain amino acid imports one or more of leucine, isoleucine, and valine into the bacterial cell.

In some embodiments, the transporter of a branched chain amino acid is encoded by a transporter of a branched chain amino acid gene derived from a bacterial genus or species, including but not limited to, *Bacillus, Campylobacter, Clostridium, Escherichia, Lactobacillus, Pseudomonas, Salmonella, Staphylococcus, Bacillus subtilis, Campylobacter jejuni, Clostridium perfringens, Escherichia coli, Lactobacillus delbrueckii, Pseudomonas aeruginosa, Salmonella typhimurium,* or *Staphylococcus aureus.* In some embodiments, the bacterial species is *Escherichia coli.* In some embodiments, the bacterial species is *Escherichia coli* strain Nissle.

Multiple distinct transporters of branched chain amino acids are known in the art. In one embodiment, the at least one gene encoding a transporter of a branched chain amino acid is the *brnQ* gene. In one embodiment, the at least one gene encoding a transporterof a branched chain amino acid is the *livJ* gene. In one embodiment, the at least one gene encoding a transporterof branched chain amino acid is the *livH* gene. In one embodiment, the at least one gene encoding a transporterof branched chain amino acid is the *livM* gene. In one embodiment, the at least one gene encoding a transporterof branched chain amino acid is the *livG* gene. In one embodiment, the at least one gene encoding a transporterof branched chain amino acid is the *livF* gene. In one embodiment, the at least one gene encoding a transporterof a branched chain amino acid is the *livKHMGF* operon. In one embodiment, the at least one gene encoding a transporterof a branched chain amino acid is the *livK* gene. In another embodiment, the *livKHMGF* operon is an *Escherichia coli livKHMGF* operon. In another embodiment, the at least one gene encoding a transporterof a branched chain amino acid comprises the *livKHMGF* operon and the *livJ* gene. In one embodiment, the bacterial cell has been genetically engineered to comprise at least one heterologous gene encoding a LIV-I system. In one embodiment, the bacterial cell has been genetically engineered to comprise at least one heterologous gene encoding a LS system. In one embodiment, the bacterial cell has been genetically engineered to comprise at least one heterologous gene encoding a LIV-I system. In one embodiment, the bacterial cell has been genetically engineered to comprise at least one heterologous *livJ* gene, and at least one heterologous gene selected from the group consisting of *livH, livM, livG, and livF.* In one embodiment, the bacterial cell has been genetically engineered to comprise at least one heterologous *livK* gene, and at least one heterologous gene selected from the group consisting of *livH, livM, livG, and livF.* In any of these embodiments, the bacterial cell may comprise more than one copy of any of one or more of these gene sequences. In any of these embodiments, the bacterial cell may over-express any one or more of these gene sequences. In any of thes eme=bodiments, the bacterial cell may further comprise gene sequence(s) encoding one or more additional BCAA transporters, e.g., brnQ transporter.

The present disclosure further provides genes encoding functional fragments of a transporterof a branched chain amino acid or functional variants of an importer of a branched chain amino acid. As used herein, the term "functional fragment thereof' or "functional variant thereof' of a transporterof a branched chain amino acid relates to an element having qualitative biological activity in common with the wild-typetransporter of a branched chain amino acid from which the fragment or variant was derived. For example, a functional fragment or a functional variant of a mutated transporter of a branched chain amino acid protein is one which retains essentially the same ability to import leucine into the bacterial cell as does the importer protein from which the functional fragment or functional variant was derived. In one embodiment, the recombinant bacterial cell disclosed herein comprises at least one heterologous gene encoding a functional fragment of a transporter of branched chain amino acid. In another embodiment, the recombinant bacterial cell disclosed herein comprises a heterologous gene encoding a functional variant of a transporter of branched chain amino acid.

Assays for testing the activity of an importer of a branched chain amino acid, a functional variant of a transporterof a branched chain amino acid, or a functional fragment of a transporter of a branched chain amino acid are well known to one of ordinary skill in the art. For example, import of a branched chain amino acid may be determined using the methods as described in Haney et al., J. Bact., 174(1):108-15, 1992; Rahmanian et al., J. Bact., 116(3):1258-66, 1973; and Ribardo and Hendrixson, J. Bact., 173(22):6233-43, 2011.

In one embodiment the genes encoding the ransporter of a branched chain amino acid have been codon-optimized for use in the host organism. In one embodiment, the genes encoding the importer of a branched chain amino acid have been codon-optimized for use in *Escherichia coli.*

The present disclosure also encompasses genes encoding a transporterof a branched chain amino acid, *e.g.,* a transporterof leucine, comprising amino acids in its sequence that are substantially the same as an amino acid sequence described herein. Amino acid sequences that are substantially the same as the sequences described herein include sequences comprising conservative amino acid substitutions, as well as amino acid deletions and/or insertions.

In some embodiments, the at least one gene encoding a transporterof a branched chain amino acid, *e.g., livKHMGF,* is mutagenized; mutants exhibiting increased branched chain amino acid, *e.g.,* leucine, transport are selected; and the mutagenized at least one gene encoding a transporterof a branched chain amino acid, *e.g., livKHMGF,* is isolated and inserted into the bacterial cell. In some embodiments, the at least one gene encoding a transporterof a branched chain amino acid, *e.g., livKHMGF,* is mutagenized; mutants exhibiting decreased branched chain amino acid, *e.g.,* leucine, transport are selected; and the mutagenized at least one gene encoding a transporterof a branched chain amino acid, *e.g., livKHMGF,* is isolated and inserted into the bacterial cell. The importer modifications described herein may be present on a plasmid or chromosome.

In one embodiment, the *livKHMGF* operon has at least about 80% identity with the uppercase sequence of SEQ ID NO:5. Accordingly, in one embodiment, the *livKHMGF* operon has at least about 90% identity with the uppercase sequence of SEQ ID NO:5. Accordingly, in one embodiment, the *livKHMGF* operon has at least about 95% identity with the uppercase sequence of SEQ ID NO:5. Accordingly, in one embodiment, the *livKHMGF* operon has at least about 85%, 86%, 87%, 88%, 89%, 90%, 91%, 92%, 93%, 94%, 95%, 96%, 97%, 98%, or 99% identity with the uppercase sequence of SEQ ID NO:5. In another embodiment, the *livKHMGF* operon comprises the uppercase sequence of SEQ ID NO:5. In yet another embodiment the *livKHMGF* operon consists of the uppercase sequence of SEQ ID NO:5.

In some embodiments, the bacterial cell comprises a heterologous gene encoding a branched chain amino acid catabolism enzyme operably linked to a first promoter and at least one heterologous gene encoding a transporterof a branched chain amino acid. In some embodiments, the at least one heterologous gene encoding a transporterof a branched chain amino acid is operably linked to the first promoter. In other embodiments, the at least one heterologous gene encoding a transporterof a branched chain amino acid is operably linked to a second promoter. In one embodiment, the at least one gene encoding a transporter of a branched chain amino acid is directly operably linked to the second promoter. In another embodiment, the at least one gene encoding a transporterof a branched chain amino acid is indirectly operably linked to the second promoter.

In some embodiments, expression of at least one gene encoding a transporter of a branched chain amino acid, *e.g., livKHMGF and*/*or brnQ,* is controlled by a different promoter than the promoter that controls expression of the gene encoding the branched chain amino acid catabolism enzyme. In some embodiments, expression of the at least one gene encoding a transporterof a branched chain amino acid, *e.g., livKHMGF and*/*or brnQ,* is controlled by the same promoter that controls expression of the branched chain amino acid catabolism enzyme. In some embodiments, at least one gene encoding a transporterof a branched chain amino acid, *e.g., livKHMGF and*/*or brnQ,* and the branched chain amino acid catabolism enzyme are divergently transcribed from a promoter region. In some embodiments, expression of each of genes encoding the at least one gene encoding a transporterof a branched chain amino acid, *e.g., livKHMGF and*/*or brnQ,* and the gene encoding the branched chain amino acid catabolism enzyme is controlled by different promoters.

In one embodiment, the at least one gene encoding a transporterof a branched chain amino acid is not operably linked to its native promoter. In some embodiments, the at least one gene encoding the transporterof a branched chain amino acid, *e.g., livKHMGF,* is controlled by its native promoter. In some embodiments, the at least one gene encoding a transporterof a branched chain amino acid, *e.g., livKHMGF and*/*or brnQ,* is controlled by an inducible promoter. In some embodiments, the at least one gene encoding a transporterof a branched chain amino acid, *e.g., livKHMG and*/*or bmQF,* is controlled by a promoter that is stronger than its native promoter. In some embodiments, the at least one gene encoding a transporter of a branched chain amino acid, *e.g., livKHMGF,* is controlled by a constitutive promoter.

In another embodiment, the promoter is an inducible promoter. Inducible promoters are described in more detail *infra.*

In one embodiment, the at least one gene encoding a transporterof a branched chain amino acid is located on a plasmid in the bacterial cell. In another embodiment, the at least one gene encoding a transporterof a branched chain amino acid is located in the chromosome of the bacterial cell. In yet another embodiment, a native copy of the at least one gene encoding a transporterof a branched chain amino acid is located in the chromosome of the bacterial cell, and a copy of at least one gene encoding a transporter of a branched chain amino acid from a different species of bacteria is located on a plasmid in the bacterial cell. In yet another embodiment, a native copy of the at least one gene encoding a transporterof a branched chain amino acid is located on a plasmid in the bacterial cell, and a copy of at least one gene encoding a transporterof a branched chain amino acid from a different species of bacteria is located on a plasmid in the bacterial cell. In yet another embodiment, a native copy of the at least one gene encoding a transporterof a branched chain amino acid is located in the chromosome of the bacterial cell, and a copy of the at least one gene encoding an importer of a branched chain amino acid from a different species of bacteria is located in the chromosome of the bacterial cell.

In some embodiments, the at least one native gene encoding a transporter, *e.g.*, *livKHMG and*/*or brnQF,* in the bacterial cell is not modified, and one or more additional copies of the native transporter, *e.g., livKHMGF and*/*or brnQ,* are inserted into the genome. In some embodiments, the at least one native gene encoding a transporter, *e.g., livKHMG* and/or *brnQF,* in the bacterial cell is not modified, and one or more additional copies of the native transporter, *e.g., livKHMGF* and/or *brnQ,* are present on a plasmid, e.g., a high copy or low copy plasmid. In one embodiment, the one or more additional copies of the native a transporter, *e.g., livKHMGF and*/*or brnQ,* that is inserted into the genome are under the control of the same inducible promoter that controls expression of the gene encoding the branched chain amino acid catabolism enzyme, *e.g.,* the FNR responsive promoter, or a different inducible promoter than the one that controls expression of the branched chain amino acid catabolism enzyme, or a constitutive promoter. In alternate embodiments, the at least one native gene encoding the transporter, *e.g., livKHMGF and*/*or brnQ,* is not modified, and one or more additional copies of the transporter, *e.g., livKHMGF,* from a different bacterial species is inserted into the genome of the bacterial cell. In one embodiment, the one or more additional copies of the transporter inserted into the genome of the bacterial cell are under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter.

In some embodiments, at least one native gene encoding the transporter, *e.g*., *livKHMGF and*/*or brnQ,* in the genetically modified bacteria is not modified, and one or more additional copies of at least one native gene encoding the transporter, *e.g., livKHMGF and*/*or brnQ,* are present in the bacterial cell on a plasmid. In one embodiment, the at least one native gene encoding the transporter *e.g., livKHMGF and*/*or brnQ,* present in the bacterial cell on a plasmid is under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter. In alternate embodiments, the at least one native gene encoding the transporter, *e.g., livKHMGF and*/*or brnQ,* is not modified, and a copy of at least one gene encoding the transporter, *e.g., livKHMGF and*/*or brnQ,* from a different bacterial species is present in the bacteria on a plasmid. In one embodiment, the copy of at least one gene encoding the transporter, *e.g., livKHMGF and*/*or brnQ,* from a different bacterial species is under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter.

In some embodiments, the bacterium is E. *coli* Nissle, and the at least one native gene encoding the transporter, *e.g., livKHMGF and*/*or brnQ,* in E. *coli* Nissle is not modified; one or more additional copies at least one native gene encoding the transporter, *e.g., livKHMGF and*/*or brnQ,* from *E. coli* Nissle *is* inserted into the *E. coli* Nissle genome under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter. In an alternate embodiment, the at least one native gene encoding the a transporter, *e.g*., *livKHMGF*and/or brnQ in *E. coli* Nissle is not modified, and a copy of at least one gene encoding the transporter, *e.g., livKHMGF and*/*or brnQ,* from a different bacterial species is inserted into the *E. coli* Nissle genome under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter.

In some embodiments, the bacterial cell is E. *coli* Nissle, and the at least one native gene encoding the transporter, *e.g., livKHMGF and*/*or brnQ,* in *E. coli* Nissle is not modified; one or more additional copies the at least one native gene encoding the transporter, *e.g., livKHMGF and*/*or brnQ, E. coli* Nissle is present in the bacterium on a plasmid and under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter. In an alternate embodiment, the at least one native gene encoding the transporter, *e.g.*, *livKHMGF,* in *E. coli* Nissle is not modified, and a copy of at least one native gene encoding the transporter, *e.g., livKHMGF and*/*or brnQ,* from a different bacterial species of are present in the bacterium on a plasmid and under the control of the same inducible promoter that controls expression of the gene encoding the branched chain amino acid catabolism enzyme, *e.g.,* the FNR responsive promoter, or a different inducible promoter than the one that controls expression of the gene encoding the branched chain amino acid catabolism enzyme, or a constitutive promoter.

In one embodiment, when the transporter of a branched chain amino acid is expressed in the recombinant bacterial cells disclosed herein, the bacterial cells import 10% more branched chain amino acids, *e.g.,* leucine, into the bacterial cell when the transporter is expressed as compared to unmodified bacteria of the same bacterial subtype under the same conditions. In another embodiment, when the transporter of a branched chain amino acid is expressed in the recombinant bacterial cells disclosed herein, the bacterial cells import 20%, 30%, 40%, 50%, 60%, 70%, 80%, 90% or 100% more branched chain amino acids, *e.g.,* leucine, into the bacterial cell when the transporteris expressed as compared with unmodified bacteria of the same bacterial subtype under the same conditions. In yet another embodiment, when the transporter of a branched chain amino acid is expressed in the recombinant bacterial cells disclosed herein, the bacterial cells import two-fold more branched chain amino acids, *e.g.,* leucine, into the cell when the transporter is expressed than unmodified bacteria of the same bacterial subtype under the same conditions. In yet another embodiment, when the transporter of a branched chain amino acid is expressed in the recombinant bacterial cells disclosed herein, the bacterial cells import three-fold, four-fold, five-fold, six-fold, seven-fold, eight-fold, nine-fold, or ten-fold more branched chain amino acids, *e.g.,* leucine, into the cell when the a transporter is expressed as compared with unmodified bacteria of the same bacterial subtype under the same conditions.

### Exporter of a Branched Chain Amino Acid

The bacterial cells disclosed herein may comprise a genetic modification that inhibits or decreases the export of a branched chain amino acid and/or its corresponding alpha keto acid or other metabolite from the bacterial cell. Knocking-out or reducing export of one or more branched chain amino acids from a bacterial cell allows the bacterial cell to more efficiently retain and catabolize exogenous branched chain amino acids and/or their alpha-keto acid counterparts or other metabolite counterparts in order to treat the diseases and disorders described herein. Any of the bacterial cells disclosed herein comprising gene sequence encoding one or more BCAA catabolism enzymes and/or one or more BCAA transporters may further a genetic modification that inhibits or decreases the export of a branched chain amino acid and/or its corresponding alpha keto acid or other metabolite from the bacterial cell.

The export of branched chain amino acids from bacterial cells is mediated by proteins well known to those of skill in the art. For example, one branched chain amino acid exporter, the leucine exporter LeuE has been characterized in *Escherichia coli* (Kutukova et al., FEBS Letters 579:4629-34 (2005) ). LeuE is encoded by the *leuE* gene in *Escherichia coli* (also known as *yeaS*). Additionally, a two-gene encoded exporter of the branched chain amino acids isoleucine, valine and leucine, denominated BrnFE was identified in the bacteria *Corynebacterium glutamicum* (Kennerknecht et al., J. Bacteriol. 184:3947-56 (2002) ). The BrnFE system is encoded by the *Corynebacterium glutamicum* genes *bmF* and *brnE,* and homologues of said genes have been identified in several organisms, including *Agrobacterium tumefaciens, Achaeoglobus fulgidus, Bacillus subtilis, Deinococcus radiodurans, Escherichia coli, Haemophilus influenzae, Helicobacter pylori, Lactococcus lactis, Streptococcus pneumoniae,* and *Vibrio cholerae* (*see* Kennerknecht *et al.,* 2002).

The bacterial cells disclosed herein comprise a genetic modification that reduces export of a branched chain amino acid from the bacterial cell. Multiple distinct exporters of branched chain amino acids, *e.g.,* leucine, are known in the art. In one embodiment, the recombinant bacterial cell disclosed herein comprises a genetic modification that reduces export of a branched chain amino acid from the bacterial cell, wherein the endogenous gene encoding an exporter of a branched chain amino acid is a *leuE* gene. In one embodiment, the recombinant bacterial cell disclosed herein comprises a genetic modification that reduces export of a branched chain amino acid from the bacterial cell, wherein the endogenous gene encoding an exporter of a branched chain amino acid is a *brnF* gene. In one embodiment, the recombinant bacterial cell disclosed herein comprises a genetic modification that reduces export of a branched chain amino acid from the bacterial cell, wherein the endogenous gene encoding an exporter of a branched chain amino acid is a *brnE* gene. In one embodiment, the recombinant bacterial cell disclosed herein comprises a genetic modification that reduces export of a branched chain amino acid from the bacterial cell and a heterologous gene encoding a branched chain amino acid catabolism enzyme. When the recombinant bacterial cells disclosed herein comprise a genetic modification that reduces export of a branched chain amino acid, the bacterial cells retain more branched chain amino acids, *e.g.,* leucine, in the bacterial cell than unmodified bacteria of the same bacterial subtype under the same conditions. Thus, the recombinant bacteria comprising a genetic modification that reduces export of a branched chain amino acid may be used to retain more branched chain amino acids in the bacterial cell so that any branched chain amino acid catabolism enzyme expressed in the organism, *e.g*., co-expressed α-ketoisovalerate decarboxylase or co-expressed branched chain keto dehydrogenase, can catabolize the branched chain amino acids, *e.g., leucine,* to treat diseases associated with the catabolism of branched chain amino acids, including Maple Syrup Urine Disease (MSUD). In one embodiment, the recombinant bacteria further comprise a heterologous gene encoding an importer of a branched chain amino acid, *e.g.,* a *livKHMGF* and/or brnQ gene.

In one embodiment, the genetic modification reduces export of a branched chain amino acid, *e.g.,* leucine, from the bacterial cell. In one embodiment, the bacterial cell is from a bacterial genus or species that includes but is not limited to, *Bacillus, Bacteroides, Bifidobacterium, Brevibacteria, Clostridium, Enterococcus, Escherichia, Lactobacillus, Lactococcus,* and *Staphylococcus, e.g., Bacillus coagulans, Bacillus subtilis, Bacteroides fragilis, Bacteroides subtilis, Bacteroides thetaiotaomicron, Bifidobacterium bifidum, Bifidobacterium infantis, Bifidobacterium lactis, Bifidobacterium longum, Clostridium butyricum, Enterococcus faecium, Escherichia coli, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis.* In another embodiment, the bacterial cell is an *Escherichia coli* bacterial cell. In another embodiment, the bacterial cell is an *Escherichia coli* strain Nissle bacterial cell.

In one embodiment, the genetic modification is a mutation in an endogenous gene encoding an exporter of a branched chain amino acid. In one embodiment, the genetic mutation is a deletion of the endogenous gene encoding an exporter, *e.g., leuE,* of a branched chain amino acid. In another embodiment, the genetic mutation results in an exporter having reduced activity as compared to a wild-type exporter protein. In one embodiment, the activity of the exporter is reduced at least 50%, at least 75%, or at least 100%. In another embodiment, the activity of the exporter is reduced at least two-fold, three-fold, four-fold, or five-fold. In another embodiment, the genetic mutation results in an exporter, *e.g*., LeuE, having no activity, *i.e.,* results in an exporter, *e.g.,* LeuE, which cannot export a branched chain amino acid, *e.g.,* lysine, from the bacterial cell.

It is routine for one of ordinary skill in the art to make mutations in a gene of interest. Mutations include substitutions, insertions, deletions, and/or truncations of one or more specific amino acid residues or of one or more specific nucleotides or codons in the polypeptide or polynucleotide of the exporter of a branched chain amino acid. Mutagenesis and directed evolution methods are well known in the art for creating variants. See, *e.g.,* U.S. Pat. No. 7,783,428; U.S. Pat. No. 6,586,182; U.S. Pat. No. 6,117,679; and Ling, et al., 1999, "Approaches to DNA mutagenesis: an overview," Anal. Biochem., 254(2):157-78; Smith, 1985, "In vitro mutagenesis," Ann. Rev. Genet., 19:423-462; Carter, 1986, "Site-directed mutagenesis," Biochem. J., 237:1-7; and Minshull, et al., 1999, "Protein evolution by molecular breeding," Current Opinion in Chemical Biology, 3:284-290. For example, the lambda red system can be used to knock-out genes in *E. coli* (see, for example, Datta et al., Gene, 379:109-115 (2006)).

The term "inactivated" as applied to a gene refers to any genetic modification that decreases or eliminates the expression of the gene and/or the functional activity of the corresponding gene product (mRNA and/or protein). The term "inactivated" encompasses complete or partial inactivation, suppression, deletion, interruption, blockage, promoter alterations, antisense RNA, dsRNA, or down-regulation of a gene. This can be accomplished, for example, by gene "knockout," inactivation, mutation (*e.g.,* insertion, deletion, point, or frameshift mutations that disrupt the expression or activity of the gene product), or by use of inhibitory RNAs (*e.g.,* sense, antisense, or RNAi technology). A deletion may encompass all or part of a gene's coding sequence. The term "knockout" refers to the deletion of most (at least about 95%, at least about 96%, at least about 97%, at least about 98%, or at least about 99%) or all (100%) of the coding sequence of a gene. In some embodiments, any number of nucleotides can be deleted, from a single base to an entire piece of a chromosome.

Assays for testing the activity of an exporter of a branched chain amino acid, *e.g.,* leucine, are well known to one of ordinary skill in the art. For example, export of a branched chain amino acid, such as leucine, may be determined using the methods described by Haney et al., J. Bact., 174(1):108-15, 1992; Rahmanian et al., J. Bact., 116(3):1258-66, 1973; and Ribardo and Hendrixson, J. Bact., 173(22):6233-43, 2011.

In another embodiment, the genetic modification is a mutation in a promoter of an endogenous gene encoding an exporter of a branched chain amino acid. In one embodiment, the genetic mutation results in decreased expression of the *leuE* gene. In one embodiment, *leuE* gene expression is reduced by about 50%, 75%, or 100%. In another embodiment, *leuE* gene expression is reduced about two-fold, three-fold, four-fold, or five-fold. In another embodiment, the genetic mutation completely inhibits expression of the *leuE* gene.

Assays for testing the level of expression of a gene, such as an exporter of a branched chain amino acid, *e.g., leuE,* are well known to one of ordinary skill in the art. For example, reverse-transcriptase polymerase chain reaction may be used to detect the level of mRNA expression of a gene. Alternatively, Western blots using antibodies directed against a protein may be used to determine the level of expression of the protein.

In another embodiment, the genetic modification is an overexpression of a repressor of an exporter of a branched chain amino acid. In one embodiment, the overexpression of the repressor of the exporter is caused by a mutation which renders the promoter of the repressor constitutively active. In another embodiment, the overexpression of the repressor of the exporter is caused by the insertion of an inducible promoter in front of the repressor so that the expression of the repressor can be induced. Inducible promoters are described in more detail herein.

### Reduction of Endogenous Bacterial Branched Chain Amino Acid Production

The bacterial cells disclosed herein may comprise a genetic modification that inhibits or decreases the biosynthesis of a branched chain amino acid and/or its corresponding alpha keto acid or other metabolite in the bacterial cell. Knocking-out or reducing production of endogenous branched chain amino acids in a bacterial cell allows the bacterial cell to more efficiently take up and catabolize exogenous branched chain amino acids and/or their alpha-keto acid counterparts or other metabolite counterparts in order to treat the diseases and disorders described herein. Knock-out or knock down of a gene encoding an enzyme required for branched chain amino acid biosynthesis creates an auxotroph, which requires the cell to import the branched chain amino acid or a metabolite to survive. Any of the bacterial cells disclosed herein comprising gene sequence encoding one or more BCAA catabolism enzymes and/or one or more BCAA transporters may further a genetic modification that inhibits or decreases the biosynthesis of a branched chain amino acid and/or its corresponding alpha keto acid or other metabolite in the bacterial cell.

As used herein, the term "branched chain amino acid biosynthesis" enzyme refers to an enzyme involved in the biosynthesis of a branched chain amino acid and/or its corresponding alpha-keto acid or oter metabolite. Multiple distinct genes involved in biosynthetic pathways of branched chain amino acids, *e.g.*, isoleucine, leucine, and valine, are known in the art. For example, the *ilvC* gene encodes a keto-acid reductoisomerase enzyme that catalyses the conversion of acetohydroxy acids into dihydroxy valerates, which leads to the synthesis of the essential branched side chain amino acids valine and isoleucine (EC 1.1.1.86) and has been characterized in *Escherichia coli* (Wek and Hatfield, J. Biol. Chem. 261:2441-50 (1986)).

Additionally, homologues of *ilvC* have been identified in several organisms, including *Candida albicans, Oryza sativa, Saccharomyces cerevisiae, Pseudomonas aeruginosa, Corynebacterium glutamicum,* and *Spinacia oleracea.*

In one embodiment, the genetic modification is a mutation in an endogenous gene encoding a protein that is involved in the biosynthesis of a branched chain amino acid or an alpha keto acid, *e.g., ilvC.* ilvC is an acetohydroxy acid isomeroreductase that is required for brached chain amino acid synthesis. In one embodiment, the genetic mutation is a deletion of an endogenous gene encoding a protein that is involved in the biosynthesis of a branched chain amino acid or an alpha-keto acid, or other BCAA metabolite, *e.g., ilvC.* In another embodiment, the genetic mutation results in an enzyme having reduced activity as compared to a wild-type enzyme. In one embodiment, the activity of the enzyme is reduced at least 50%, at least 75%, or at least 100%. In another embodiment, the activity of the enzyme is reduced at least two-fold, three-fold, four-fold, or five-fold. In another embodiment, the genetic mutation results in an enzyme, *e.g.,* IlvC, having no activity, *i.e.,* results in an enzyme, *e.g.,* IlvC, which cannot catalyze the conversion of acetohydroxy acids into dihydroxy valerates, thereby inhibiting the endogenous synthesis of the branched chain amino acids valine and isoleucine in the recombinant bacterial cell.

It is routine for one of ordinary skill in the art to make mutations in a gene of interest. Mutations include substitutions, insertions, deletions, and/or truncations of one or more specific amino acid residues or of one or more specific nucleotides or codons in the polypeptide or polynucleotide of the exporter of a branched chain amino acid. Mutagenesis and directed evolution methods are well known in the art for creating variants. See, *e.g.,* U.S. Pat. No. 7,783,428; U.S. Pat. No. 6,586,182; U.S. Pat. No. 6,117,679; and Ling, et al., 1999, "Approaches to DNA mutagenesis: an overview," Anal. Biochem., 254(2):157-78; Smith, 1985, "In vitro mutagenesis," Ann. Rev. Genet., 19:423-462; Carter, 1986, "Site-directed mutagenesis," Biochem. J., 237:1-7; and Minshull, et al., 1999, "Protein evolution by molecular breeding," Current Opinion in Chemical Biology, 3:284-290. For example, the lambda red system can be used to knock-out genes in *E. coli* (see, for example, Datta et al., Gene, 379:109-115 (2006)).

The term "inactivated," as applied to a gene, refers to any genetic modification that decreases or eliminates the expression of the gene and/or the functional activity of the corresponding gene product (mRNA and/or protein). The term "inactivated" encompasses complete or partial inactivation, suppression, deletion, interruption, blockage, promoter alterations, antisense RNA, dsRNA, or down-regulation of a gene. This can be accomplished, for example, by gene "knockout," inactivation, mutation (*e.g.,* insertion, deletion, point, or frameshift mutations that disrupt the expression or activity of the gene product), or by use of inhibitory RNAs (*e.g.,* sense, antisense, or RNAi technology). A deletion may encompass all or part of a gene's coding sequence. The term "knockout" refers to the deletion of most (at least about 95%, at least about 96%, at least about 97%, at least about 98%, or at least about 99%) or all (100%) of the coding sequence of a gene. In some embodiments, any number of nucleotides can be deleted, from a single base to an entire piece of a chromosome.

Assays for testing the activity of enzymes involved in the biosynthesis of branched chain amino acids and/or alpha-keto acids, and/or other BCAA metabolite *e.g*., *ilvC,* are well known to one of ordinary skill in the art. For example, the activity of a ketol-acid reductoisomerase enzyme may be determined using the methods described by Durner et al., Plant Physiol., 103:903-910, 1993.

In another embodiment, the genetic modification is a mutation in a promoter of an endogenous gene encoding the branched chain amino acid biosynthesis enzyme. In one embodiment, the genetic mutation results in decreased expression of the branched chain amino acid biosynthesis enzyme gene. In one embodiment, gene expression is reduced by about 50%, 75%, or 100%. In another embodiment, gene expression is reduced about two-fold, three-fold, four-fold, or five-fold. In another embodiment, the genetic mutation completely inhibits expression of the gene.

Assays for testing the level of expression of a gene, such as *ilvC,* are well known to one of ordinary skill in the art. For example, reverse-transcriptase polymerase chain reaction may be used to detect the level of mRNA expression of a gene. Alternatively, Western blots using antibodies directed against a protein may be used to determine the level of expression of the protein.

In another embodiment, the genetic modification is an overexpression of a repressor of a branched chain amino acid biosynthesis gene. In one embodiment, the overexpression of the repressor of the gene is caused by a mutation which renders the promoter of the repressor constitutively active. In another embodiment, the overexpression of the repressor of the branched chain amino acid biosynthesis gene is caused by the insertion of an inducible promoter in front of the repressor so that the expression of the repressor can be induced. Inducible promoters are described in more detail herein.

### Inducible Promoters

In some embodiments, the bacterial cell comprises a stably maintained plasmid or chromosome carrying the gene encoding the branched chain amino acid catabolism enzyme, *e.g., kivD, leuDH, ilvE,* L-AAD, *BCKD, adh2,* PadA, and/or YqhD such that the branched chain amino acid catabolism enzyme can be expressed in the host cell, and the host cell is capable of survival and/or growth *in vitro, e.g.,* in medium, and/or *in vivo, e.g.,* in the gut. In some embodiments, bacterial cell comprises two or more distinct branched chain amino acid catabolism enzymes, *e.g., kivD, leuDH, ilvE, BCKD,* L-AAD, PadA, *adh2,* PadA and/or YqhD genes. In some embodiments, the genetically engineered bacteria comprise multiple copies of the same branched chain amino acid catabolism enzyme gene. In some embodiments, the genetically engineered bacteria comprise multiple copies of different branched chain amino acid catabolism enzyme genes. In some embodiments, the gene encoding the branched chain amino acid catabolism enzyme is present on a plasmid and operably linked to a directly or indirectly inducible promoter. In some embodiments, the gene encoding the branched chain amino acid catabolism enzyme is present on a plasmid and operably linked to a promoter that is induced under low-oxygen or anaerobic conditions. In some embodiments, the gene encoding the branched chain amino acid catabolism enzyme is present on a chromosome and operably linked to a directly or indirectly inducible promoter. In some embodiments, the gene encoding the branched chain amino acid catabolism enzyme is present in the chromosome and operably linked to a promoter that is induced under low-oxygen or anaerobic conditions. In some embodiments, the gene encoding the branched chain amino acid catabolism enzyme is present on a plasmid and operably linked to a promoter that is induced by exposure to tetracycline or arabinose.

In some embodiments, the bacterial cell comprises a stably maintained plasmid or chromosome carrying the at least one gene encoding a transporter of a branched chain amino acid, *e.g., livKHMGF* and/or *brnQ,* such that the transporter, *e.g.,* LivKHMGF and/or *brnQ,* can be expressed in the host cell, and the host cell is capable of survival and/or growth *in vitro, e.g.,* in medium, and/or *in vivo, e.g.,* in the gut. In some embodiments, bacterial cell comprises two or more distinct copies of the at least one gene encoding a transporter of a branched chain amino acid, *e.g., livKHMGF* and/or *brnQ.* In some embodiments, the genetically engineered bacteria comprise multiple copies of the same at least one gene encoding a transporter of a branched chain amino acid, *e.g., livKHMGF* and/or *brnQ.* In some embodiments, the at least one gene encoding a transporter of a branched chain amino acid, *e.g., livKHMGF* and/or *brnQ,* is present on a plasmid and operably linked to a directly or indirectly inducible promoter. In some embodiments, the at least one gene encoding a transporter of a branched chain amino acid, *e.g., livKHMGF* and/or *brnQ,* is present on a plasmid and operably linked to a promoter that is induced under low-oxygen or anaerobic conditions. In some embodiments, the at least one gene encoding a transporter of a branched chain amino acid, *e.g., livKHMGF* and/or *brnQ,* is present on a chromosome and operably linked to a directly or indirectly inducible promoter. In some embodiments, the at least one gene encoding a transporter of a branched chain amino acid, *e.g., livKHMGF* and/or *brnQ,* is present in the chromosome and operably linked to a promoter that is induced under low-oxygen or anaerobic conditions. In some embodiments, the at least one gene encoding a transporter of a branched chain amino acid, *e.g., livKHMGF* and/or *brnQ,* is present on a plasmid and operably linked to a promoter that is induced by exposure to tetracycline.

In some embodiments, the promoter that is operably linked to the gene encoding the branched chain amino acid catabolism enzyme and the promoter that is operably linked to the gene encoding the transporter of a branched chain amino acid, *e.g., livKHMGF* and/or *brnQ,* is directly induced by exogenous environmental conditions. In some embodiments, the promoter that is operably linked to the gene encoding the branched chain amino acid catabolism enzyme and the promoter that is operably linked to the gene encoding the transporter of a branched chain amino acid, *e.g., livKHMGF* and/or *brnQ,* is indirectly induced by exogenous environmental conditions. In some embodiments, the promoter is directly or indirectly induced by exogenous environmental conditions specific to the gut of a mammal. In some embodiments, the promoter is directly or indirectly induced by exogenous environmental conditions specific to the small intestine of a mammal. In some embodiments, the promoter is directly or indirectly induced by low-oxygen or anaerobic conditions such as the environment of the mammalian gut. In some embodiments, the promoter is directly or indirectly induced by molecules or metabolites that are specific to the gut of a mammal, *e.g.*, propionate. In some embodiments, the promoter is directly or indirectly induced by a molecule that is co-administered with the bacterial cell.

In some embodiments, the bacterial cell comprises a stably maintained plasmid or chromosome carrying the at least one gene encoding a branched chain amino acid binding protein, *e.g., livJ,* such that the BCAA binding protein, *e.g., livJ,* can be expressed in the host cell, and the host cell is capable of survival and/or growth *in vitro, e.g.,* in medium, and/or *in vivo, e.g.,* in the gut. In some embodiments, bacterial cell comprises two or more distinct copies of the at least one gene encoding a BCAA binding protein of a branched chain amino acid, *e.g., livJ.* In some embodiments, the genetically engineered bacteria comprise multiple copies of the same at least one gene encoding a binding protein of a branched chain amino acid, *e.g., livJ.* In some embodiments, the at least one gene encoding a binding protein of a branched chain amino acid, *e.g., livJ,* is present on a plasmid and operably linked to a directly or indirectly inducible promoter. In some embodiments, the at least one gene encoding a binding protein of a branched chain amino acid, *e.g., livJ,* is present on a plasmid and operably linked to a promoter that is induced under low-oxygen or anaerobic conditions. In some embodiments, the at least one gene encoding a binding protein of a branched chain amino acid, *e.g., livJ,* is present on a chromosome and operably linked to a directly or indirectly inducible promoter. In some embodiments, the at least one gene encoding a binding protein of a branched chain amino acid, *e.g., livJ,* is present in the chromosome and operably linked to a promoter that is induced under low-oxygen or anaerobic conditions. In some embodiments, the at least one gene encoding a binding protein of a branched chain amino acid, *e.g., livJ,* is present on a plasmid and operably linked to a promoter that is induced by exposure to tetracycline.

In some embodiments, the promoter that is operably linked to the gene encoding the branched chain amino acid catabolism enzyme and the promoter that is operably linked to the gene encoding the binding protein of a branched chain amino acid, *e.g., livJ,* is directly induced by exogenous environmental conditions. In some embodiments, the promoter that is operably linked to the gene encoding the branched chain amino acid catabolism enzyme and the promoter that is operably linked to the gene encoding the binding protein of a branched chain amino acid, *e.g., livJ,* is indirectly induced by exogenous environmental conditions. In some embodiments, the promoter is directly or indirectly induced by exogenous environmental conditions specific to the gut of a mammal. In some embodiments, the promoter is directly or indirectly induced by exogenous environmental conditions specific to the small intestine of a mammal. In some embodiments, the promoter is directly or indirectly induced by low-oxygen or anaerobic conditions such as the environment of the mammalian gut. In some embodiments, the promoter is directly or indirectly induced by molecules or metabolites that are specific to the gut of a mammal, *e.g.*, propionate. In some embodiments, the promoter is directly or indirectly induced by a molecule that is co-administered with the bacterial cell.

In certain embodiments, the bacterial cell comprises a gene encoding a branched chain amino acid catabolism enzyme, *e.g., kivD, leuDH, ilvE,* BCKD, L-AAD, padA, yqhD and/or *adh2,* is expressed under the control of the fumarate and nitrate reductase regulator (FNR) promoter. In certain embodiments, the bacterial cell comprises at least one gene encoding a transporter of a branched chain amino acid, *e.g., livKHMGF and*/*or brnQ,* is expressed under the control of the fumarate and nitrate reductase regulator (FNR) promoter. In certain embodiments, the bacterial cell comprises at least one gene encoding a binding protein of a branched chain amino acid, *e.g., livJ,* is expressed under the control of the fumarate and nitrate reductase regulator (FNR) promoter. In *E. coli,* FNR is a major transcriptional activator that controls the switch from aerobic to anaerobic metabolism (Unden et al., 1997). In the anaerobic state, FNR dimerizes into an active DNA binding protein that activates hundreds of genes responsible for adapting to anaerobic growth. In the aerobic state, FNR is prevented from dimerizing by oxygen and is inactive.

FNR responsive promoters include, but are not limited to, the FNR responsive promoters listed in the chart, below. Underlined sequences are predicted ribosome binding sites, and bolded sequences are restriction sites used for cloning.

**Table 4 FNR responsive promoters**

| FNR Responsive Promoter | Sequence |
|---|---|
| SEQ ID NO: 14 | GTCAGCATAACACCCTGACCTCTCATTAATTGTTCATGCCGGGCGGCACTATCGTCGTCCGGCCT TTTCCTCTCTTACTCTGCTACGTACATCTATTTCTATAAATCCGTTCAATTTGTCTGTTTTTTGCACA AACATGAAATATCAGACAATTCCGTGACTTAAGAAAATTTATACAAATCAGCAATATACCCCTTA AGGAGTATATAAAGGTGAATTTGATTTACATCAATAAGCGGGGTTGCTGAATCGTTAAGGTAGG CGGTAATAGAAAAGAAATCGAGGCAAAA |
| SEQ ID NO: 15 | ATTTCCTCTCATCCCATCCGGGGTGAGAGTCTTTCCCCCGACTTATGGCTCATGCATGCATCAAA AAAGATGTGAGCTTGATCAAAAACAAAAAATATTTCACTCGACAGGAGTATTTATATTGCGCCCG TTACGTGGGCTTCGACTGTAAATCAGAAAGGAGAAAACACCT |
| SEQ ID NO: 16 | GTCAGCATAACACCCTGACCTCTCATTAATTGTTCATGCCGGGCGGCACTATCGTCGTCCGGCCT TTTCCTCTCTTACTCTGCTACGTACATCTATTTCTATAAATCCGTTCAATTTGTCTGTTTTTTGCACA AACATGAAATATCAGACAATTCCGTGACTTAAGAAAATTTATACAAATCAGCAATATACCCCTTA AGGAGTATATAAAGGTGAATTTGATTTACATCAATAAGCGGGGTTGCTGAATCGTTAAGGATCC CTCTAGAAATAATTTTGTTTAACTTTAAGAAGGAGATATACAT |
| SEQ ID NO: 17 | CATTTCCTCTCATCCCATCCGGGGTGAGAGTCTTTTCCCCCGACTTATGGCTCATGCATGCATCAA AAAAGATGTGAGCTTGATCAAAAACAAAAAATATTTCACTCGACAGGAGTATTTATATTGCGCCC GGATCCCTCTAGAAATAATTTTGTTTAACTTTAAGAAGGAGATATACAT |
| SEQ ID NO: 18 | AGTTGTTCTTATTGGTGGTGTTGCTTTATGGTTGCATCGTAGTAAATGGTTGTAACAAAAGCAAT TTTTCCGGCTGTCTGTATACAAAAACGCCGTAAAGTTTGAGCGAAGTCAATAAACTCTCTACCCA TTCAGGGCAATATCTCTCTTGGATCCCTCTAGAAATAATTTTGTTTAACTTTAAGAAGGAGATATA CAT |

In one embodiment, the FNR responsive promoter comprises SEQ ID NO:14. In another embodiment, the FNR responsive promoter comprises SEQ ID NO:15. In another embodiment, the FNR responsive promoter comprises SEQ ID NO:16. In another embodiment, the FNR responsive promoter comprises SEQ ID NO:17. In yet another embodiment, the FNR responsive promoter comprises SEQ ID NO:18. Additional FNR responsive promoters are shown below.

**Table 5. FNR Promoter Sequences**

| **SEQ ID NO** | **FNR-responsive regulatory region Sequence** |
|---|---|
| SEQ ID NO: 80 | ATCCCCATCACTCTTGATGGAGATCAATTCCCCAAGCTGCTAGAGCGTTA CCTTGCCCTTAAACATTAGCAATGTCGATTTATCAGAGGGCCGACAGGCT CCCACAGGAGAAAACCG |
| SEQ ID NO: 81 | CTCTTGATCGTTATCAATTCCCACGCTGTTTCAGAGCGTTACCTTGCCCT TAAACATTAGCAATGTCGATTTATCAGAGGGCCGACAGGCTCCCACAGGA GAAAACCG |
| *nirB1* SEQ ID NO: 82 | GTCAGCATAACACCCTGACCTCTCATTAATTGTTCATGCCGGGCGGCACT ATCGTCGTCCGGCCTTTTCCTCTCTTACTCTGCTACGTACATCTATTTCT ATAAATCCGTTCAATTTGTCTGTTTTTTGCACAAACATGAAATATCAGAC AATTCCGTGACTTAAGAAAATTTATACAAATCAGCAATATACCCCTTAAG GAGTATATAAAGGTGAATTTGATTTACATCAATAAGCGGGGTTGCTGAAT CGTTAAGGTAGGCGGTAATAGAAAAGAAATCGAGGCAAAA |
| *nirB2* SEQ ID NO: 83 | CGGCCCGATCGTTGAACATAGCGGTCCGCAGGCGGCACTGCTTACAGCAA ACGGTCTGTACGCTGTCGTCTTTGTGATGTGCTTCCTGTTAGGTTTCGTC AGCCGTCACCGTCAGCATAACACCCTGACCTCTCATTAATTGCTCATGCC GGACGGCACTATCGTCGTCCGGCCTTTTCCTCTCTTCCCCCGCTACGTGC ATCTATTTCTATAAACCCGCTCATTTTGTCTATTTTTTGCACAAACATGA AATATCAGACAATTCCGTGACTTAAGAAAATTTATACAAATCAGCAATAT ACCCATTAAGGAGTATATAAAGGTGAATTTGATTTACATCAATAAGCGGG GTTGCTGAATCGTTAAGGTAGGCGGTAATAGAAAAGAAATCGAGGCAAAA atgtttgtttaactttaagaaggagatatacat |
| *nirB3* SEQ ID NO: 84 | GTCAGCATAACACCCTGACCTCTCATTAATTGCTCATGCCGGACGGCACT ATCGTCGTCCGGCCTTTTCCTCTCTTCCCCCGCTACGTGCATCTATTTCT ATAAACCCGCTCATTTTGTCTATTTTTTGCACAAACATGAAATATCAGAC AATTCCGTGACTTAAGAAAATTTATACAAATCAGCAATATACCCATTAAG GAGTATATAAAGGTGAATTTGATTTACATCAATAAGCGGGGTTGCTGAAT CGTTAAGGTAGGCGGTAATAGAAAAGAAATCGAGGCAAAA |
| *ydfZ* SEQ ID NO: 85 | ATTTCCTCTCATCCCATCCGGGGTGAGAGTCTTTTCCCCCGACTTATGGC TCATGCATGCATCAAAAAAGATGTGAGCTTGATCAAAAACAAAAAATATT TCACTCGACAGGAGTATTTATATTGCGCCCGTTACGTGGGCTTCGACTGT AAATCAGAAAGGAGAAAACACCT |
| *nirB+RBS* SEQ ID NO: 86 | GTCAGCATAACACCCTGACCTCTCATTAATTGTTCATGCCGGGCGGCACT ATCGTCGTCCGGCCTTTTCCTCTCTTACTCTGCTACGTACATCTATTTCT ATAAATCCGTTCAATTTGTCTGTTTTTTGCACAAACATGAAATATCAGAC AATTCCGTGACTTAAGAAAATTTATACAAATCAGCAATATACCCCTTAAG GAGTATATAAAGGTGAATTTGATTTACATCAATAAGCGGGGTTGCTGAAT CGTTAAGGATCCCTCTAGAAATAATTTTGTTTAACTTTAAGAAGGAGATA TACAT |
| *ydfz+Res* SEQ ID NO: 87 | CATTTCCTCTCATCCCATCCGGGGTGAGAGTCTTTTCCCCCGACTTATGG CTCATGCATGCATCAAAAAAGATGTGAGCTTGATCAAAAACAAAAAATAT TTCACTCGACAGGAGTATTTATATTGCGCCCGGATCCCTCTAGAAATAAT TTTGTTTAACTTTAAGAAGGAGATATACAT |
| *fnrS1* SEQ ID NO: 88 | AGTTGTTCTTATTGGTGGTGTTGCTTTATGGTTGCATCGTAGTAAATGGT TGTAACAAAAGCAATTTTTCCGGCTGTCTGTATACAAAAACGCCGTAAAG TTTGAGCGAAGTCAATAAACTCTCTACCCATTCAGGGCAATATCTCTCTT GGATCCCTCTAGAAATAATTTTGTTTAACTTTAAGAAGGAGATATACAT |
| *fnrS2* SEQ ID NO: 89 | AGTTGTTCTTATTGGTGGTGTTGCTTTATGGTTGCATCGTAGTAAATGGT TGTAACAAAAGCAATTTTTCCGGCTGTCTGTATACAAAAACGCCGCAAAG TTTGAGCGAAGTCAATAAACTCTCTACCCATTCAGGGCAATATCTCTCTT GGATCCAAAGTGAACTCTAGAAATAATTTTGTTTAACTTTAAGAAGGAGA TATACAT |
| *nirB+crp* SEQ ID NO: 90 | TCGTCTTTGTGATGTGCTTCCTGTTAGGTTTCGTCAGCCGTCACCGTCAG CATAACACCCTGACCTCTCATTAATTGCTCATGCCGGACGGCACTATCGT CGTCCGGCCTTTTCCTCTCTTCCCCCGCTACGTGCATCTATTTCTATAAA CCCGCTCATTTTGTCTATTTTTTGCACAAACATGAAATATCAGACAATTC CGTGACTTAAGAAAATTTATACAAATCAGCAATATACCCATTAAGGAGTA TATAAAGGTGAATTTGATTTACATCAATAAGCGGGGTTGCTGAATCGTTA AGGTAGaaatgtgatctagttcacatttGCGGTAATAGAAAAGAAATCGA GGCAAAAatgtttgtttaactttaagaaggagatatacat |
| *fnrS+crp* SEQ ID NO: 91 | AGTTGTTCTTATTGGTGGTGTTGCTTTATGGTTGCATCGTAGTAAATGGT TGTAACAAAAGCAATTTTTCCGGCTGTCTGTATACAAAAACGCCGCAAAG TTTGAGCGAAGTCAATAAACTCTCTACCCATTCAGGGCAATATCTCTCaa atgtgatctagttcacattttttgtttaactttaagaaggagatatacat |

In some embodiments, multiple distinct FNR nucleic acid sequences are inserted in the genetically engineered bacteria. In alternate embodiments, the genetically engineered bacteria comprise a gene encoding a branched chain amino acid catabolism enzyme, *e.g., kivD* or *BCKD* or other enzyme disclosed herein, is expressed under the control of an alternate oxygen level-dependent promoter, *e.g.*, DNR (Trunk et al., 2010) or ANR (Ray et al., 1997). In alternate embodiments, the genetically engineered bacteria comprise at least one gene encoding a transporter of a branched chain amino acid, *e.g., livKHMGF and*/*or brnQ,* is expressed under the control of an alternate oxygen level-dependent promoter, *e.g.,* DNR (Trunk et al., 2010) or ANR (Ray et al., 1997). In alternate embodiments, the genetically engineered bacteria comprise at least one gene encoding a binding protein of a branched chain amino acid, *e.g., livJ,* is expressed under the control of an alternate oxygen level-dependent promoter, *e.g.*, DNR (Trunk et al., 2010) or ANR (Ray et al., 1997). In these embodiments, catabolism of the branched chain amino acid, *e.g.,* leucine, is particularly activated in a low-oxygen or anaerobic environment, such as in the gut. In some embodiments, gene expression is further optimized by methods known in the art, *e.g.,* by optimizing ribosomal binding sites and/or increasing mRNA stability. In one embodiment, the mammalian gut is a human mammalian gut.

In some embodiments, the bacterial cell comprises an oxygen-level dependent transcriptional regulator, *e.g.,* FNR, ANR, or DNR, and corresponding promoter from a different bacterial species. The heterologous oxygen-level dependent transcriptional regulator and promoter increase the transcription of genes operably linked to said promoter, *e.g.,* the gene encoding the branched chain amino acid catabolism enzyme, and/or the at least one gene encoding a transporter of a branched chain amino acid, *e.g., livKHMGF and*/*or brnQ,* and/or the at least one gene encoding a binding protein of a branched chain amino acid in a low-oxygen or anaerobic environment, as compared to the native gene(s) and promoter in the bacteria under the same conditions. In certain embodiments, the non-native oxygen-level dependent transcriptional regulator is an FNR protein from *N. gonorrhoeae* (*see, e.g.,* Isabella et al., 2011). In some embodiments, the corresponding wild-type transcriptional regulator is left intact and retains wild-type activity. In alternate embodiments, the corresponding wild-type transcriptional regulator is deleted or mutated to reduce or eliminate wild-type activity.

In some embodiments, the genetically engineered bacteria comprise a wild-type oxygen-level dependent transcriptional regulator, e.g., FNR, ANR, or DNR, and corresponding promoter that is mutated relative to the wild-type promoter from bacteria of the same subtype. The mutated promoter enhances binding to the wild-type transcriptional regulator and increases the transcription of genes operably linked to said promoter, e.g., the gene encoding the branched chain amino acid catabolism enzyme, and/or the at least one gene encoding a transporter of a branched chain amino acid, e.g., livKHMGF and/or brnQ, and/or the at least one gene encoding a binding protein of a branched chain amino acid in a low-oxygen or anaerobic environment, as compared to the wild-type promoter under the same conditions. In some embodiments, the genetically engineered bacteria comprise a wild-type oxygen-level dependent promoter, *e.g*., FNR, ANR, or DNR promoter, and corresponding transcriptional regulator that is mutated relative to the wild-type transcriptional regulator from bacteria of the same subtype. The mutated transcriptional regulator enhances binding to the wild-type promoter and increases the transcription of genes operably linked to said promoter, *e.g.*, the gene encoding the branched chain amino acid catabolism enzyme, and/or the at least one gene encoding a transporter of a branched chain amino acid, *e.g., livKHMGF* and/or *brnQ,* and/or the at least one gene encoding a binding protein of a branched chain amino acid in a low-oxygen or anaerobic environment, as compared to the wild-type transcriptional regulator under the same conditions. In certain embodiments, the mutant oxygen-level dependent transcriptional regulator is an FNR protein comprising amino acid substitutions that enhance dimerization and FNR activity (*see, e.g.,* Moore et al., 2006).

In some embodiments, the bacterial cells disclosed herein comprise multiple copies of the endogenous gene encoding the oxygen level-sensing transcriptional regulator, *e.g.,* the *FNR* gene. In some embodiments, the gene encoding the oxygen level-sensing transcriptional regulator is present on a plasmid. In some embodiments, the gene encoding the oxygen level-sensing transcriptional regulator and the gene encoding the branched chain amino acid catabolism enzyme are present on different plasmids. In some embodiments, the gene encoding the oxygen level-sensing transcriptional regulator and the gene encoding the branched chain amino acid catabolism enzyme and/or the at least one gene encoding a transporter of a branched chain amino acid and/or the at least one gene encoding a binding protein of a branched chain amino acid are present on different plasmids. In some embodiments, the gene encoding the oxygen level-sensing transcriptional regulator and the gene encoding the branched chain amino acid catabolism enzyme and/or the at least one gene encoding a ransporter of a branched chain amino acid and/or the at least one gene encoding a binding protein of a branched chain amino acid are present on the same plasmid.

In some embodiments, the gene encoding the oxygen level-sensing transcriptional regulator is present on a chromosome. In some embodiments, the gene encoding the oxygen level-sensing transcriptional regulator and the gene encoding the gene encoding the branched chain amino acid catabolism enzyme and/or the at least one gene encoding a transporter of a branched chain amino acid and/or the at least one gene encoding a binding protein of a branched chain amino acid are present on different chromosomes. In some embodiments, the gene encoding the oxygen level-sensing transcriptional regulator and the gene encoding the branched chain amino acid catabolism enzyme and/or the at least one gene encoding a transporter of a branched chain amino acid and/or the at least one gene encoding a binding protein of a branched chain amino acid are present on the same chromosome. In some instances, it may be advantageous to express the oxygen level-sensing transcriptional regulator under the control of an inducible promoter in order to enhance expression stability. In some embodiments, expression of the transcriptional regulator is controlled by a different promoter than the promoter that controls expression of the gene encoding the branched chain amino acid catabolism enzyme and/or BCAA transporter and/or BCAA binding protein. In some embodiments, expression of the transcriptional regulator is controlled by the same promoter that controls expression of the branched chain amino acid catabolism enzyme and/or BCAA transporter and/or BCAA binding protein. In some embodiments, the transcriptional regulator and the branched chain amino acid catabolism enzyme are divergently transcribed from a promoter region.

### RNS-dependent regulation

In some embodiments, the genetically engineered bacteria comprise a gene encoding a branched chain amino acid catabolism enzyme that is expressed under the control of an inducible promoter. In some embodiments, the genetically engineered bacterium that expresses a branched chain amino acid catabolism enzyme and/or BCAA transporter and/or BCAA binding protein is under the control of a promoter that is activated by inflammatory conditions. In one embodiment, the gene for producing the branched chain amino acid catabolism enzyme and/or BCAA transporter and/or BCAA binding protein is expressed under the control of an inflammatory-dependent promoter that is activated in inflammatory environments, e.g., a reactive nitrogen species or RNS promoter.

As used herein, "reactive nitrogen species" and "RNS" are used interchangeably to refer to highly active molecules, ions, and/or radicals derived from molecular nitrogen. RNS can cause deleterious cellular effects such as nitrosative stress. RNS includes, but is not limited to, nitric oxide (NO•), peroxynitrite or peroxynitrite anion (ONOO-), nitrogen dioxide (•NO2), dinitrogen trioxide (N2O3), peroxynitrous acid (ONOOH), and nitroperoxycarbonate (ONOOCO2-) (unpaired electrons denoted by •). Bacteria have evolved transcription factors that are capable of sensing RNS levels. Different RNS signaling pathways are triggered by different RNS levels and occur with different kinetics.

As used herein, "RNS-inducible regulatory region" refers to a nucleic acid sequence to which one or more RNS-sensing transcription factors is capable of binding, wherein the binding and/or activation of the corresponding transcription factor activates downstream gene expression; in the presence of RNS, the transcription factor binds to and/or activates the regulatory region. In some embodiments, the RNS-inducible regulatory region comprises a promoter sequence. In some embodiments, the transcription factor senses RNS and subsequently binds to the RNS-inducible regulatory region, thereby activating downstream gene expression. In alternate embodiments, the transcription factor is bound to the RNS-inducible regulatory region in the absence of RNS; in the presence of RNS, the transcription factor undergoes a conformational change, thereby activating downstream gene expression. The RNS-inducible regulatory region may be operatively linked to a gene or genes, e.g., an branched chain amino acid catabolism enzymegene sequence(s), e.g., any of the amino acid catabolism enzymes described herein. For example, in the presence of RNS, a transcription factor senses RNS and activates a corresponding RNS-inducible regulatory region, thereby driving expression of an operatively linked gene sequence. Thus, RNS induces expression of the gene or gene sequences.

As used herein, "RNS-derepressible regulatory region" refers to a nucleic acid sequence to which one or more RNS-sensing transcription factors is capable of binding, wherein the binding of the corresponding transcription factor represses downstream gene expression; in the presence of RNS, the transcription factor does not bind to and does not repress the regulatory region. In some embodiments, the RNS-derepressible regulatory region comprises a promoter sequence. The RNS-derepressible regulatory region may be operatively linked to a gene or genes, e.g., an branched chain amino acid catabolism enzymegene sequence(s), BCAA transporter sequence(s), BCAA binding protein(s). For example, in the presence of RNS, a transcription factor senses RNS and no longer binds to and/or represses the regulatory region, thereby derepressing an operatively linked gene sequence or gene cassette. Thus, RNS derepresses expression of the gene or genes.

As used herein, "RNS-repressible regulatory region" refers to a nucleic acid sequence to which one or more RNS-sensing transcription factors is capable of binding, wherein the binding of the corresponding transcription factor represses downstream gene expression; in the presence of RNS, the transcription factor binds to and represses the regulatory region. In some embodiments, the RNS-repressible regulatory region comprises a promoter sequence. In some embodiments, the transcription factor that senses RNS is capable of binding to a regulatory region that overlaps with part of the promoter sequence. In alternate embodiments, the transcription factor that senses RNS is capable of binding to a regulatory region that is upstream or downstream of the promoter sequence. The RNS-repressible regulatory region may be operatively linked to a gene sequence or gene cassette. For example, in the presence of RNS, a transcription factor senses RNS and binds to a corresponding RNS-repressible regulatory region, thereby blocking expression of an operatively linked gene sequence or gene sequences. Thus, RNS represses expression of the gene or gene sequences.

As used herein, a "RNS-responsive regulatory region" refers to a RNS-inducible regulatory region, a RNS-repressible regulatory region, and/or a RNS-derepressible regulatory region. In some embodiments, the RNS-responsive regulatory region comprises a promoter sequence. Each regulatory region is capable of binding at least one corresponding RNS-sensing transcription factor. Examples of transcription factors that sense RNS and their corresponding RNS-responsive genes, promoters, and/or regulatory regions include, but are not limited to, those shown in **Table 6**.

**Table 6. Examples of RNS-sensing transcription factors and RNS-responsive genes**

| **RNS-sensing transcription factor:** | **Primarily capable of sensing:** | **Examples of responsive genes, promoters, and/or regulatory regions:** |
|---|---|---|
| NsrR | NO | *norB, aniA, nsrR, hmpA, ytfE, ygbA, hcp, her, nrfA, aox* |
| NorR | NO | *norVW, norr* |
| DNR | NO | *norCB, nir, nor, nos* |

In some embodiments, the genetically engineered bacteria of the invention comprise a tunable regulatory region that is directly or indirectly controlled by a transcription factor that is capable of sensing at least one reactive nitrogen species. The tunable regulatory region is operatively linked to a gene or genes capable of directly or indirectly driving the expression of an amino acid catabolism enzyme, BCAA transporter, and/or BCAA binding protein, thus controlling expression of the branched chain amino acid catabolism enzyme, BCAA transporter, and/or BCAA binding protein relative to RNS levels. For example, the tunable regulatory region is a RNS-inducible regulatory region, and the payload is an amino acid catabolism enzyme, BCAA transporter, and/or BCAA binding protein, such as any of the amino acid catabolism enzymes, BCAA transporters, and BCAA binding proteins provided herein; when RNS is present, e.g., in an inflamed tissue, a RNS-sensing transcription factor binds to and/or activates the regulatory region and drives expression of the branched chain amino acid catabolism enzyme, BCAA transporter, and/or BCAA binding protein gene or genes. Subsequently, when inflammation is ameliorated, RNS levels are reduced, and production of the branched chain amino acid catabolism enzyme, BCAA transporter, and/or BCAA binding protein is decreased or eliminated.

In some embodiments, the tunable regulatory region is a RNS-inducible regulatory region; in the presence of RNS, a transcription factor senses RNS and activates the RNS-inducible regulatory region, thereby driving expression of an operatively linked gene or genes. In some embodiments, the transcription factor senses RNS and subsequently binds to the RNS-inducible regulatory region, thereby activating downstream gene expression. In alternate embodiments, the transcription factor is bound to the RNS-inducible regulatory region in the absence of RNS; when the transcription factor senses RNS, it undergoes a conformational change, thereby inducing downstream gene expression.

In some embodiments, the tunable regulatory region is a RNS-inducible regulatory region, and the transcription factor that senses RNS is NorR. NorR "is an NO-responsive transcriptional activator that regulates expression of the norVW genes encoding flavorubredoxin and an associated flavoprotein, which reduce NO to nitrous oxide" (Spiro 2006). The genetically engineered bacteria of the invention may comprise any suitable RNS-responsive regulatory region from a gene that is activated by NorR. Genes that are capable of being activated by NorR are known in the art (see, e.g., Spiro 2006; Vine et al., 2011; Karlinsey et al., 2012; Table 1). In certain embodiments, the genetically engineered bacteria of the invention comprise a RNS-inducible regulatory region from norVW that is operatively linked to a gene or genes, e.g., one or more branched chain amino acid catabolism enzyme, BCAA transporter, and/or BCAA binding protein gene sequence(s). In the presence of RNS, a NorR transcription factor senses RNS and activates to the norVW regulatory region, thereby driving expression of the operatively linked gene(s) and producing the amino acid catabolism enzyme, BCAA transporter, and/or BCAA binding protein.

n some embodiments, the tunable regulatory region is a RNS-inducible regulatory region, and the transcription factor that senses RNS is DNR. DNR (dissimilatory nitrate respiration regulator) "promotes the expression of the nir, the nor and the nos genes" in the presence of nitric oxide (Castiglione et al., 2009). The genetically engineered bacteria of the invention may comprise any suitable RNS-responsive regulatory region from a gene that is activated by DNR. Genes that are capable of being activated by DNR are known in the art (see, e.g., Castiglione et al., 2009; Giardina et al., 2008; Table 1). In certain embodiments, the genetically engineered bacteria of the invention comprise a RNS-inducible regulatory region from norCB that is operatively linked to a gene or gene cassette, e.g., a butyrogenic gene cassette. In the presence of RNS, a DNR transcription factor senses RNS and activates to the norCB regulatory region, thereby driving expression of the operatively linked gene or genes and producing one or more amino acid catabolism enzymes. In some embodiments, the DNR is Pseudomonas aeruginosa DNR.

In some embodiments, the tunable regulatory region is a RNS-derepressible regulatory region, and binding of a corresponding transcription factor represses downstream gene expression; in the presence of RNS, the transcription factor no longer binds to the regulatory region, thereby derepressing the operatively linked gene or gene cassette.

In some embodiments, the tunable regulatory region is a RNS-derepressible regulatory region, and the transcription factor that senses RNS is NsrR. NsrR is "an Rrf2-type transcriptional repressor [that] can sense NO and control the expression of genes responsible for NO metabolism" (Isabella et al., 2009). The genetically engineered bacteria of the invention may comprise any suitable RNS-responsive regulatory region from a gene that is repressed by NsrR. In some embodiments, the NsrR is Neisseria gonorrhoeae NsrR. Genes that are capable of being repressed by NsrR are known in the art (see, e.g., Isabella et al., 2009; Dunn et al., 2010; Table 1). In certain embodiments, the genetically engineered bacteria of the invention comprise a RNS-derepressible regulatory region from norB that is operatively linked to a gene or genes, e.g., an branched chain amino acid catabolism enzyme gene or genes. In the presence of RNS, an NsrR transcription factor senses RNS and no longer binds to the norB regulatory region, thereby derepressing the operatively linked branched chain amino acid catabolism enzyme, BCAA transporter, and/or BCAA binding protein gene or genes and producing the encoding an amino acid catabolism enzyme(s).

In some embodiments, it is advantageous for the genetically engineered bacteria to express a RNS-sensing transcription factor that does not regulate the expression of a significant number of native genes in the bacteria. In some embodiments, the genetically engineered bacterium of the invention expresses a RNS-sensing transcription factor from a different species, strain, or substrain of bacteria, wherein the transcription factor does not bind to regulatory sequences in the genetically engineered bacterium of the invention. In some embodiments, the genetically engineered bacterium of the invention is Escherichia coli, and the RNS-sensing transcription factor is NsrR, e.g., from is Neisseria gonorrhoeae, wherein the Escherichia coli does not comprise binding sites for said NsrR. In some embodiments, the heterologous transcription factor minimizes or eliminates off-target effects on endogenous regulatory regions and genes in the genetically engineered bacteria.

In some embodiments, the tunable regulatory region is a RNS-repressible regulatory region, and binding of a corresponding transcription factor represses downstream gene expression; in the presence of RNS, the transcription factor senses RNS and binds to the RNS-repressible regulatory region, thereby repressing expression of the operatively linked gene or gene cassette. In some embodiments, the RNS-sensing transcription factor is capable of binding to a regulatory region that overlaps with part of the promoter sequence. In alternate embodiments, the RNS-sensing transcription factor is capable of binding to a regulatory region that is upstream or downstream of the promoter sequence.

In these embodiments, the genetically engineered bacteria may comprise a two repressor activation regulatory circuit, which is used to express an amino acid catabolism enzyme. The two repressor activation regulatory circuit comprises a first RNS-sensing repressor and a second repressor, which is operatively linked to a gene or gene cassette, e.g., encoding an amino acid catabolism enzyme. In one aspect of these embodiments, the RNS-sensing repressor inhibits transcription of the second repressor, which inhibits the transcription of the gene or gene cassette. Examples of second repressors useful in these embodiments include, but are not limited to, TetR, C1, and LexA. In the absence of binding by the first repressor (which occurs in the absence of RNS), the second repressor is transcribed, which represses expression of the gene or genes. In the presence of binding by the first repressor (which occurs in the presence of RNS), expression of the second repressor is repressed, and the gene or genes, e.g., a branched chain amino acid catabolism enzyme, BCAA transporter, and/or BCAA binding protein gene or genes is expressed.

A RNS-responsive transcription factor may induce, derepress, or repress gene expression depending upon the regulatory region sequence used in the genetically engineered bacteria. One or more types of RNS-sensing transcription factors and corresponding regulatory region sequences may be present in genetically engineered bacteria. In some embodiments, the genetically engineered bacteria comprise one type of RNS-sensing transcription factor, e.g., NsrR, and one corresponding regulatory region sequence, e.g., from norB. In some embodiments, the genetically engineered bacteria comprise one type of RNS-sensing transcription factor, e.g., NsrR, and two or more different corresponding regulatory region sequences, e.g., from norB and aniA. In some embodiments, the genetically engineered bacteria comprise two or more types of RNS-sensing transcription factors, e.g., NsrR and NorR, and two or more corresponding regulatory region sequences, e.g., from norB and norR, respectively. One RNS-responsive regulatory region may be capable of binding more than one transcription factor. In some embodiments, the genetically engineered bacteria comprise two or more types of RNS-sensing transcription factors and one corresponding regulatory region sequence. Nucleic acid sequences of several RNS-regulated regulatory regions are known in the art (see, e.g., Spiro 2006; Isabella et al., 2009; Dunn et al., 2010; Vine et al., 2011; Karlinsey et al., 2012).

In some embodiments, the genetically engineered bacteria of the invention comprise a gene encoding a RNS-sensing transcription factor, e.g., the nsrR gene, that is controlled by its native promoter, an inducible promoter, a promoter that is stronger than the native promoter, e.g., the GlnRS promoter or the P(Bla) promoter, or a constitutive promoter. In some instances, it may be advantageous to express the RNS-sensing transcription factor under the control of an inducible promoter in order to enhance expression stability. In some embodiments, expression of the RNS-sensing transcription factor is controlled by a different promoter than the promoter that controls expression of the therapeutic molecule. In some embodiments, expression of the RNS-sensing transcription factor is controlled by the same promoter that controls expression of the therapeutic molecule. In some embodiments, the RNS-sensing transcription factor and therapeutic molecule are divergently transcribed from a promoter region.

In some embodiments, the genetically engineered bacteria of the invention comprise a gene for a RNS-sensing transcription factor from a different species, strain, or substrain of bacteria. In some embodiments, the genetically engineered bacteria comprise a RNS-responsive regulatory region from a different species, strain, or substrain of bacteria. In some embodiments, the genetically engineered bacteria comprise a RNS-sensing transcription factor and corresponding RNS-responsive regulatory region from a different species, strain, or substrain of bacteria. The heterologous RNS-sensing transcription factor and regulatory region may increase the transcription of genes operatively linked to said regulatory region in the presence of RNS, as compared to the native transcription factor and regulatory region from bacteria of the same subtype under the same conditions.

In some embodiments, the genetically engineered bacteria comprise a RNS-sensing transcription factor, NsrR, and corresponding regulatory region, nsrR, from Neisseria gonorrhoeae. In some embodiments, the native RNS-sensing transcription factor, e.g., NsrR, is left intact and retains wild-type activity. In alternate embodiments, the native RNS-sensing transcription factor, e.g., NsrR, is deleted or mutated to reduce or eliminate wild-type activity.

In some embodiments, the genetically engineered bacteria of the invention comprise multiple copies of the endogenous gene encoding the RNS-sensing transcription factor, e.g., the nsrR gene. In some embodiments, the gene encoding the RNS-sensing transcription factor is present on a plasmid. In some embodiments, the gene encoding the RNS-sensing transcription factor and the gene or gene cassette for producing the therapeutic molecule are present on different plasmids. In some embodiments, the gene encoding the RNS-sensing transcription factor and the gene or gene cassette for producing the therapeutic molecule are present on the same plasmid. In some embodiments, the gene encoding the RNS-sensing transcription factor is present on a chromosome. In some embodiments, the gene encoding the RNS-sensing transcription factor and the gene or gene cassette for producing the therapeutic molecule are present on different chromosomes. In some embodiments, the gene encoding the RNS-sensing transcription factor and the gene or gene cassette for producing the therapeutic molecule are present on the same chromosome.

In some embodiments, the genetically engineered bacteria comprise a wild-type gene encoding a RNS-sensing transcription factor, e.g., the NsrR gene, and a corresponding regulatory region, e.g., a norB regulatory region, that is mutated relative to the wild-type regulatory region from bacteria of the same subtype. The mutated regulatory region increases the expression of the branched chain amino acid catabolism enzymein the presence of RNS, as compared to the wild-type regulatory region under the same conditions. In some embodiments, the genetically engineered bacteria comprise a wild-type RNS-responsive regulatory region, e.g., the norB regulatory region, and a corresponding transcription factor, e.g., NsrR, that is mutated relative to the wild-type transcription factor from bacteria of the same subtype. The mutant transcription factor increases the expression of the branched chain amino acid catabolism enzymein the presence of RNS, as compared to the wild-type transcription factor under the same conditions. In some embodiments, both the RNS-sensing transcription factor and corresponding regulatory region are mutated relative to the wild-type sequences from bacteria of the same subtype in order to increase expression of the branched chain amino acid catabolism enzyme, BCAA transporter, and/or BCAA binding protein in the presence of RNS.

In some embodiments, the gene or gene cassette for producing the anti-inflammation and/or gut barrier function enhancer molecule is present on a plasmid and operably linked to a promoter that is induced by RNS. In some embodiments, expression is further optimized by methods known in the art, e.g., by optimizing ribosomal binding sites, manipulating transcriptional regulators, and/or increasing mRNA stability.

In some embodiments, any of the gene(s) of the present disclosure may be integrated into the bacterial chromosome at one or more integration sites. For example, one or more copies of a branched chain amino acid catabolism enzyme, BCAA transporter, and/or BCAA binding protein gene(s) may be integrated into the bacterial chromosome. Having multiple copies of the gene or gen(s) integrated into the chromosome allows for greater production of the amino acid catabolism enzyme(s) and also permits fine-tuning of the level of expression. Alternatively, different circuits described herein, such as any of the secretion or exporter circuits, in addition to the therapeutic gene(s) or gene cassette(s) could be integrated into the bacterial chromosome at one or more different integration sites to perform multiple different functions.

### ROS-dependent regulation

In some embodiments, the genetically engineered bacteria comprise a gene for producing an branched chain amino acid catabolism enzyme, BCAA transporter, and/or BCAA binding protein that is expressed under the control of an inducible promoter. In some embodiments, the genetically engineered bacterium that expresses a branched chain amino acid catabolism enzyme, BCAA transporter, and/or BCAA binding protein under the control of a promoter that is activated by conditions of cellular damage. In one embodiment, the gene for producing the branched chain amino acid catabolism enzymeis expressed under the control of a cellular damaged-dependent promoter that is activated in environments in which there is cellular or tissue damage, e.g., a reactive oxygen species or ROS promoter.

As used herein, "reactive oxygen species" and "ROS" are used interchangeably to refer to highly active molecules, ions, and/or radicals derived from molecular oxygen. ROS can be produced as byproducts of aerobic respiration or metal-catalyzed oxidation and may cause deleterious cellular effects such as oxidative damage. ROS includes, but is not limited to, hydrogen peroxide (H2O2), organic peroxide (ROOH), hydroxyl ion (OH-), hydroxyl radical (•OH), superoxide or superoxide anion (•O2-), singlet oxygen (1O2), ozone (O3), carbonate radical, peroxide or peroxyl radical (•O2-2), hypochlorous acid (HOCl), hypochlorite ion (OCl-), sodium hypochlorite (NaOCl), nitric oxide (NO•), and peroxynitrite or peroxynitrite anion (ONOO-) (unpaired electrons denoted by •). Bacteria have evolved transcription factors that are capable of sensing ROS levels. Different ROS signaling pathways are triggered by different ROS levels and occur with different kinetics (Marinho et al., 2014).

As used herein, "ROS-inducible regulatory region" refers to a nucleic acid sequence to which one or more ROS-sensing transcription factors is capable of binding, wherein the binding and/or activation of the corresponding transcription factor activates downstream gene expression; in the presence of ROS, the transcription factor binds to and/or activates the regulatory region. In some embodiments, the ROS-inducible regulatory region comprises a promoter sequence. In some embodiments, the transcription factor senses ROS and subsequently binds to the ROS-inducible regulatory region, thereby activating downstream gene expression. In alternate embodiments, the transcription factor is bound to the ROS-inducible regulatory region in the absence of ROS; in the presence of ROS, the transcription factor undergoes a conformational change, thereby activating downstream gene expression. The ROS-inducible regulatory region may be operatively linked to a gene sequence or gene sequence, e.g., a sequence or sequences encoding one or more amino acid catabolism enzyme(s). For example, in the presence of ROS, a transcription factor, e.g., OxyR, senses ROS and activates a corresponding ROS-inducible regulatory region, thereby driving expression of an operatively linked gene sequence or gene sequences. Thus, ROS induces expression of the gene or genes.

As used herein, "ROS-derepressible regulatory region" refers to a nucleic acid sequence to which one or more ROS-sensing transcription factors is capable of binding, wherein the binding of the corresponding transcription factor represses downstream gene expression; in the presence of ROS, the transcription factor does not bind to and does not repress the regulatory region. In some embodiments, the ROS-derepressible regulatory region comprises a promoter sequence. The ROS-derepressible regulatory region may be operatively linked to a gene or genes, e.g., one or more genes encoding one or more amino acid catabolism enzyme(s). For example, in the presence of ROS, a transcription factor, e.g., OhrR, senses ROS and no longer binds to and/or represses the regulatory region, thereby derepressing an operatively linked gene sequence or gene cassette. Thus, ROS derepresses expression of the gene or gene cassette.

As used herein, "ROS-repressible regulatory region" refers to a nucleic acid sequence to which one or more ROS-sensing transcription factors is capable of binding, wherein the binding of the corresponding transcription factor represses downstream gene expression; in the presence of ROS, the transcription factor binds to and represses the regulatory region. In some embodiments, the ROS-repressible regulatory region comprises a promoter sequence. In some embodiments, the transcription factor that senses ROS is capable of binding to a regulatory region that overlaps with part of the promoter sequence. In alternate embodiments, the transcription factor that senses ROS is capable of binding to a regulatory region that is upstream or downstream of the promoter sequence. The ROS-repressible regulatory region may be operatively linked to a gene sequence or gene sequences. For example, in the presence of ROS, a transcription factor, e.g., PerR, senses ROS and binds to a corresponding ROS-repressible regulatory region, thereby blocking expression of an operatively linked gene sequence or gene sequences. Thus, ROS represses expression of the gene or genes.

As used herein, a "ROS-responsive regulatory region" refers to a ROS-inducible regulatory region, a ROS-repressible regulatory region, and/or a ROS-derepressible regulatory region. In some embodiments, the ROS-responsive regulatory region comprises a promoter sequence. Each regulatory region is capable of binding at least one corresponding ROS-sensing transcription factor. Examples of transcription factors that sense ROS and their corresponding ROS-responsive genes, promoters, and/or regulatory regions include, but are not limited to, those shown in **Table 7.**

**Table 7. Examples of ROS-sensing transcription factors and ROS-responsive genes**

| **ROS-sensing transcription factor:** | **Primarily capable of sensing:** | **Examples of responsive genes, promoters, and/or regulatory regions:** |
|---|---|---|
| OxyR | H₂O₂ | *ahpC; ahpF; dps; dsbG; fhuF; flu; fur; gor; grxA; hemH, katG; oxyS, sufA; sufB, sufC; sufD; sufE; sufS; trxC; uxuA; yaaA; yaeH; yaiA; ybjM; ydcH; ydeN; ygaQ; yljA; ytfK* |
| PerR | H₂O₂ | *katA; ahoCF; mrgA; zoaA; fur; hemAXCDBL; srfA* |
| OhrR | Organis peroxides NaOCl | *ohrA* |
| SoxR | •O₂⁻ | *soxS* |
| | NO• | |
| | (also capable of sensing H₂O₂ | |
| RosR | H₂O₂ | *rbtT; tnp16a; rluC1; tnp5a; mscL; tnp2d; phoD; tnp15b; pstA; tnp5b; xylC; gabD1; rluC2; cgtS9; azlC; narKGHJI; rosR* |

In some embodiments, the genetically engineered bacteria comprise a tunable regulatory region that is directly or indirectly controlled by a transcription factor that is capable of sensing at least one reactive oxygen species. The tunable regulatory region is operatively linked to a gene or gene cassette capable of directly or indirectly driving the expression of an amino acid catabolism enzyme, thus controlling expression of the branched chain amino acid catabolism enzymerelative to ROS levels. For example, the tunable regulatory region is a ROS-inducible regulatory region, and the molecule is an amino acid catabolism enzyme; when ROS is present, e.g., in an inflamed tissue, a ROS-sensing transcription factor binds to and/or activates the regulatory region and drives expression of the gene sequence for the amino acid catabolism enzyme, BCAA transporter, and/or BCAA binding protein thereby producing the amino acid catabolism enzyme, BCAA transporter, and/or BCAA binding protein. Subsequently, when inflammation is ameliorated, ROS levels are reduced, and production of the branched chain amino acid catabolism enzyme, BCAA transporter, and/or BCAA binding protein is decreased or eliminated.

In some embodiments, the tunable regulatory region is a ROS-inducible regulatory region; in the presence of ROS, a transcription factor senses ROS and activates the ROS-inducible regulatory region, thereby driving expression of an operatively linked gene or gene cassette. In some embodiments, the transcription factor senses ROS and subsequently binds to the ROS-inducible regulatory region, thereby activating downstream gene expression. In alternate embodiments, the transcription factor is bound to the ROS-inducible regulatory region in the absence of ROS; when the transcription factor senses ROS, it undergoes a conformational change, thereby inducing downstream gene expression.

In some embodiments, the tunable regulatory region is a ROS-inducible regulatory region, and the transcription factor that senses ROS is OxyR. OxyR "functions primarily as a global regulator of the peroxide stress response" and is capable of regulating dozens of genes, e.g., "genes involved in H2O2 detoxification (katE, ahpCF), heme biosynthesis (hemH), reductant supply (grxA, gor, trxC), thiol-disulfide isomerization (dsbG), Fe-S center repair (sufA-E, sufS), iron binding (yaaA), repression of iron import systems (fur)" and "OxyS, a small regulatory RNA" (Dubbs et al., 2012). The genetically engineered bacteria may comprise any suitable ROS-responsive regulatory region from a gene that is activated by OxyR. Genes that are capable of being activated by OxyR are known in the art (see, e.g., Zheng et al., 2001; Dubbs et al., 2012; Table 1). In certain embodiments, the genetically engineered bacteria of the invention comprise a ROS-inducible regulatory region from oxyS that is operatively linked to a gene, e.g., a branched chain amino acid catabolism enzyme, BCAA transporter, and/or BCAA binding protein gene. In the presence of ROS, e.g., H2O2, an OxyR transcription factor senses ROS and activates to the oxyS regulatory region, thereby driving expression of the operatively linked branched chain amino acid catabolism enzyme, BCAA transporter, and/or BCAA binding protein gene and producing the amino acid catabolism enzyme, BCAA transporter, and/or BCAA binding protein. In some embodiments, OxyR is encoded by an E. coli oxyR gene. In some embodiments, the oxyS regulatory region is an E. coli oxyS regulatory region. In some embodiments, the ROS-inducible regulatory region is selected from the regulatory region of katG, dps, and ahpC.

In alternate embodiments, the tunable regulatory region is a ROS-inducible regulatory region, and the corresponding transcription factor that senses ROS is SoxR. When SoxR is "activated by oxidation of its [2Fe-2S] cluster, it increases the synthesis of SoxS, which then activates its target gene expression" (Koo et al., 2003). "SoxR is known to respond primarily to superoxide and nitric oxide" (Koo et al., 2003), and is also capable of responding to H2O2. The genetically engineered bacteria of the invention may comprise any suitable ROS-responsive regulatory region from a gene that is activated by SoxR. Genes that are capable of being activated by SoxR are known in the art (see, e.g., Koo et al., 2003; Table 1). In certain embodiments, the genetically engineered bacteria of the invention comprise a ROS-inducible regulatory region from soxS that is operatively linked to a gene, e.g., an amino acid catabolism enzyme. In the presence of ROS, the SoxR transcription factor senses ROS and activates the soxS regulatory region, thereby driving expression of the operatively linked branched chain amino acid catabolism enzyme, BCAA transporter, and/or BCAA binding protein gene and producing an amino acid catabolism enzyme, BCAA transporter, and/or BCAA binding protein.

In some embodiments, the tunable regulatory region is a ROS-derepressible regulatory region, and binding of a corresponding transcription factor represses downstream gene expression; in the presence of ROS, the transcription factor no longer binds to the regulatory region, thereby derepressing the operatively linked gene or gene cassette.

In some embodiments, the tunable regulatory region is a ROS-derepressible regulatory region, and the transcription factor that senses ROS is OhrR. OhrR "binds to a pair of inverted repeat DNA sequences overlapping the ohrA promoter site and thereby represses the transcription event," but oxidized OhrR is "unable to bind its DNA target" (Duarte et al., 2010). OhrR is a "transcriptional repressor [that]... senses both organic peroxides and NaOCl" (Dubbs et al., 2012) and is "weakly activated by H2O2 but it shows much higher reactivity for organic hydroperoxides" (Duarte et al., 2010). The genetically engineered bacteria of the invention may comprise any suitable ROS-responsive regulatory region from a gene that is repressed by OhrR. Genes that are capable of being repressed by OhrR are known in the art (see, e.g., Dubbs et al., 2012; Table 1). In certain embodiments, the genetically engineered bacteria of the invention comprise a ROS-derepressible regulatory region from ohrA that is operatively linked to a gene or gene cassette, e.g., a branched chain amino acid catabolism enzyme, BCAA transporter, and/or BCAA binding protein gene. In the presence of ROS, e.g., NaOCl, an OhrR transcription factor senses ROS and no longer binds to the ohrA regulatory region, thereby derepressing the operatively linked branched chain amino acid catabolism enzyme, BCAA transporter, and/or BCAA binding protein gene and producing the an amino acid catabolism enzyme, BCAA transporter, and/or BCAA binding protein.

OhrR is a member of the MarR family of ROS-responsive regulators. "Most members of the MarR family are transcriptional repressors and often bind to the -10 or -35 region in the promoter causing a steric inhibition of RNA polymerase binding" (Bussmann et al., 2010). Other members of this family are known in the art and include, but are not limited to, OspR, MgrA, RosR, and SarZ. In some embodiments, the transcription factor that senses ROS is OspR, MgRA, RosR, and/or SarZ, and the genetically engineered bacteria of the invention comprises one or more corresponding regulatory region sequences from a gene that is repressed by OspR, MgRA, RosR, and/or SarZ. Genes that are capable of being repressed by OspR, MgRA, RosR, and/or SarZ are known in the art (see, e.g., Dubbs et al., 2012).

In some embodiments, the tunable regulatory region is a ROS-derepressible regulatory region, and the corresponding transcription factor that senses ROS is RosR. RosR is "a MarR-type transcriptional regulator" that binds to an "18-bp inverted repeat with the consensus sequence TTGTTGAYRYRTCAACWA" and is "reversibly inhibited by the oxidant H2O2" (Bussmann et al., 2010). RosR is capable of repressing numerous genes and putative genes, including but not limited to "a putative polyisoprenoid-binding protein (cg1322, gene upstream of and divergent from rosR), a sensory histidine kinase (cgtS9), a putative transcriptional regulator of the Crp/FNR family (cg3291), a protein of the glutathione S-transferase family (cg1426), two putative FMN reductases (cg1150 and cg1850), and four putative monooxygenases (cg0823, cg1848, cg2329, and cg3084)" (Bussmann et al., 2010). The genetically engineered bacteria of the invention may comprise any suitable ROS-responsive regulatory region from a gene that is repressed by RosR. Genes that are capable of being repressed by RosR are known in the art (see, e.g., Bussmann et al., 2010; Table 1). In certain embodiments, the genetically engineered bacteria of the invention comprise a ROS-derepressible regulatory region from cgtS9 that is operatively linked to a gene or gene cassette, e.g., an amino acid catabolism enzyme, BCAA transporter, and/or BCAA binding protein. In the presence of ROS, e.g., H2O2, a RosR transcription factor senses ROS and no longer binds to the cgtS9 regulatory region, thereby derepressing the operatively linked branched chain amino acid catabolism enzyme, BCAA transporter, and/or BCAA binding protein gene and producing the amino acid catabolism enzyme, BCAA transporter, and/or BCAA binding protein.

In some embodiments, it is advantageous for the genetically engineered bacteria to express a ROS-sensing transcription factor that does not regulate the expression of a significant number of native genes in the bacteria. In some embodiments, the genetically engineered bacterium of the invention expresses a ROS-sensing transcription factor from a different species, strain, or substrain of bacteria, wherein the transcription factor does not bind to regulatory sequences in the genetically engineered bacterium of the invention. In some embodiments, the genetically engineered bacterium of the invention is Escherichia coli, and the ROS-sensing transcription factor is RosR, e.g., from Corynebacterium glutamicum, wherein the Escherichia coli does not comprise binding sites for said RosR. In some embodiments, the heterologous transcription factor minimizes or eliminates off-target effects on endogenous regulatory regions and genes in the genetically engineered bacteria.

In some embodiments, the tunable regulatory region is a ROS-repressible regulatory region, and binding of a corresponding transcription factor represses downstream gene expression; in the presence of ROS, the transcription factor senses ROS and binds to the ROS-repressible regulatory region, thereby repressing expression of the operatively linked gene or gene cassette. In some embodiments, the ROS-sensing transcription factor is capable of binding to a regulatory region that overlaps with part of the promoter sequence. In alternate embodiments, the ROS-sensing transcription factor is capable of binding to a regulatory region that is upstream or downstream of the promoter sequence.

In some embodiments, the tunable regulatory region is a ROS-repressible regulatory region, and the transcription factor that senses ROS is PerR. In Bacillus subtilis, PerR "when bound to DNA, represses the genes coding for proteins involved in the oxidative stress response (katA, ahpC, and mrgA), metal homeostasis (hemAXCDBL, fur, and zoaA) and its own synthesis (perR)" (Marinho et al., 2014). PerR is a "global regulator that responds primarily to H2O2" (Dubbs et al., 2012) and "interacts with DNA at the per box, a specific palindromic consensus sequence (TTATAATNATTATAA) residing within and near the promoter sequences of PerR-controlled genes" (Marinho et al., 2014). PerR is capable of binding a regulatory region that "overlaps part of the promoter or is immediately downstream from it" (Dubbs et al., 2012). The genetically engineered bacteria of the invention may comprise any suitable ROS-responsive regulatory region from a gene that is repressed by PerR. Genes that are capable of being repressed by PerR are known in the art (see, e.g., Dubbs et al., 2012; Table 1).

In these embodiments, the genetically engineered bacteria may comprise a two repressor activation regulatory circuit, which is used to express an amino acid catabolism enzyme. The two repressor activation regulatory circuit comprises a first ROS-sensing repressor, e.g., PerR, and a second repressor, e.g., TetR, which is operatively linked to a gene or gene cassette, e.g., an amino acid catabolism enzyme. In one aspect of these embodiments, the ROS-sensing repressor inhibits transcription of the second repressor, which inhibits the transcription of the gene or gene cassette. Examples of second repressors useful in these embodiments include, but are not limited to, TetR, C1, and LexA. In some embodiments, the ROS-sensing repressor is PerR. In some embodiments, the second repressor is TetR. In this embodiment, a PerR-repressible regulatory region drives expression of TetR, and a TetR-repressible regulatory region drives expression of the gene or gene cassette, e.g., an amino acid catabolism enzyme. In the absence of PerR binding (which occurs in the absence of ROS), tetR is transcribed, and TetR represses expression of the gene or gene cassette, e.g., an amino acid catabolism enzyme. In the presence of PerR binding (which occurs in the presence of ROS), tetR expression is repressed, and the gene or gene cassette, e.g., an amino acid catabolism enzyme, BCAA transporter, and/or BCAA binding protein is expressed.

A ROS-responsive transcription factor may induce, derepress, or repress gene expression depending upon the regulatory region sequence used in the genetically engineered bacteria. For example, although "OxyR is primarily thought of as a transcriptional activator under oxidizing conditions... OxyR can function as either a repressor or activator under both oxidizing and reducing conditions" (Dubbs et al., 2012), and OxyR "has been shown to be a repressor of its own expression as well as that of fhuF (encoding a ferric ion reductase) and flu (encoding the antigen 43 outer membrane protein)" (Zheng et al., 2001). The genetically engineered bacteria of the invention may comprise any suitable ROS-responsive regulatory region from a gene that is repressed by OxyR. In some embodiments, OxyR is used in a two repressor activation regulatory circuit, as described above. Genes that are capable of being repressed by OxyR are known in the art (see, e.g., Zheng et al., 2001; Table 1). Or, for example, although RosR is capable of repressing a number of genes, it is also capable of activating certain genes, e.g., the narKGHJI operon. In some embodiments, the genetically engineered bacteria comprise any suitable ROS-responsive regulatory region from a gene that is activated by RosR. In addition, "PerR-mediated positive regulation has also been observed... and appears to involve PerR binding to distant upstream sites" (Dubbs et al., 2012). In some embodiments, the genetically engineered bacteria comprise any suitable ROS-responsive regulatory region from a gene that is activated by PerR.

One or more types of ROS-sensing transcription factors and corresponding regulatory region sequences may be present in genetically engineered bacteria. For example, "OhrR is found in both Gram-positive and Gram-negative bacteria and can coreside with either OxyR or PerR or both" (Dubbs et al., 2012). In some embodiments, the genetically engineered bacteria comprise one type of ROS-sensing transcription factor, e.g., OxyR, and one corresponding regulatory region sequence, e.g., from oxyS. In some embodiments, the genetically engineered bacteria comprise one type of ROS-sensing transcription factor, e.g., OxyR, and two or more different corresponding regulatory region sequences, e.g., from oxyS and katG. In some embodiments, the genetically engineered bacteria comprise two or more types of ROS-sensing transcription factors, e.g., OxyR and PerR, and two or more corresponding regulatory region sequences, e.g., from oxyS and katA, respectively. One ROS-responsive regulatory region may be capable of binding more than one transcription factor. In some embodiments, the genetically engineered bacteria comprise two or more types of ROS-sensing transcription factors and one corresponding regulatory region sequence.

Nucleic acid sequences of several exemplary OxyR-regulated regulatory regions are shown in Table 5. OxyR binding sites are underlined and bolded. In some embodiments, genetically engineered bacteria comprise a nucleic acid sequence that is at least about 80%, at least about 85%, at least about 90%, at least about 95%, or at least about 99% homologous to the DNA sequence of SEQ ID NO: 46, 47, 48, or 49, or a functional fragment thereof.

**Table 8: Nucleotide sequences of exemplary OxyR-regulated regulatory regions**

| **Regulatory sequence** | **01234567890123456789012345678901234567890123456789** |
|---|---|
| *katG* (SEQ ID NO: 46) | TGTGGCTTTTATGAAAATCACACAGTGATCACAAATTTTAAACA GAGCACAAAATGCTGCCTCGAAATGAGGGCGGAAAATAAGGT TATCAGCCTTGTTTCTCCCTCATTACTTGAAGGATATGAAGCTA AAACCCTTTTTTATAAAGCATTTGTCCGAATTCGGACATAATCA AAAAAGCTTAATTAAGATCAATTTGATCTACATCTCTTTAACCA ACAATATGTAAGATCTCAACTATCGCATCCGTGGATTAATTC AATTATAACTTCTCTCTAACGCTGTGTATCGTAACGGTAACACT GTAGAGGGGAGCACATTGATGCGAATTCATTAAAGAGGAGAAA GGTACC |
| *dps* (SEQ ID NO: 47) | TTCCGAAAATTCCTGGCGAGCAGATAAATAAGAATTGTTCTTAT CAATATATCTAACTCATTGAATCTTTATTAGTTTTGTTTTTCACG CTTGTTACCACTATTAGTGTGATAGGAACAGCCAGAATAGCG GAACACATAGCCGGTGCTATACTTAATCTCGTTAATTACTGGGA CATAACATCAAGAGGATATGAAATTCGAATTCATTAAAGAGGA GAAAGGTACC |
| *ahpC* (SEQ ID NO: 48) | GCTTAGATCAGGTGATTGCCCTTTGTTTATGAGGGTGTTGTAATC CATGTCGTTGTTGCATTTGTAAGGGCAACACCTCAGCCTGCAGG CAGGCACTGAAGATACCAAAGGGTAGTTCAGATTACACGGTCA CCTGGAAAGGGGGCCATTTTACTTTTTATCGCCGCTGGCGGTGC AAAGTTCACAAAGTTGTCTTACGAAGGTTGTAAGGTAAAACTT ATCGATTTGATAATGGAAACGCATTAGCCGAATCGGCAAAAT TGGTTACCTTACATCTCATCGAAAACACGGAGGAAGTATAGATG CGAATTCATTAAAGAGGAGAAAGGTACC |
| *oxyS* (SEQ ID NO: 49) | CTCGAGTTCATTATCCATCCTCCATCGCCACGATAGTTCATGGC GATAGGTAGAATAGCAATGAACGATTATCCCTATCAAGCATTC TGACTGATAATTGCTCACACGAATTCATTAAAGAGGAGAAAGGT ACC |

In some embodiments, the genetically engineered bacteria of the invention comprise a gene encoding a ROS-sensing transcription factor, e.g., the oxyR gene, that is controlled by its native promoter, an inducible promoter, a promoter that is stronger than the native promoter, e.g., the GlnRS promoter or the P(Bla) promoter, or a constitutive promoter. In some instances, it may be advantageous to express the ROS-sensing transcription factor under the control of an inducible promoter in order to enhance expression stability. In some embodiments, expression of the ROS-sensing transcription factor is controlled by a different promoter than the promoter that controls expression of the therapeutic molecule. In some embodiments, expression of the ROS-sensing transcription factor is controlled by the same promoter that controls expression of the therapeutic molecule. In some embodiments, the ROS-sensing transcription factor and therapeutic molecule are divergently transcribed from a promoter region.

In some embodiments, the genetically engineered bacteria of the invention comprise a gene for a ROS-sensing transcription factor from a different species, strain, or substrain of bacteria. In some embodiments, the genetically engineered bacteria comprise a ROS-responsive regulatory region from a different species, strain, or substrain of bacteria. In some embodiments, the genetically engineered bacteria comprise a ROS-sensing transcription factor and corresponding ROS-responsive regulatory region from a different species, strain, or substrain of bacteria. The heterologous ROS-sensing transcription factor and regulatory region may increase the transcription of genes operatively linked to said regulatory region in the presence of ROS, as compared to the native transcription factor and regulatory region from bacteria of the same subtype under the same conditions.

In some embodiments, the genetically engineered bacteria comprise a ROS-sensing transcription factor, OxyR, and corresponding regulatory region, oxyS, from Escherichia coli. In some embodiments, the native ROS-sensing transcription factor, e.g., OxyR, is left intact and retains wild-type activity. In alternate embodiments, the native ROS-sensing transcription factor, e.g., OxyR, is deleted or mutated to reduce or eliminate wild-type activity.

In some embodiments, the genetically engineered bacteria of the invention comprise multiple copies of the endogenous gene encoding the ROS-sensing transcription factor, e.g., the oxyR gene. In some embodiments, the gene encoding the ROS-sensing transcription factor is present on a plasmid. In some embodiments, the gene encoding the ROS-sensing transcription factor and the gene or gene cassette for producing the therapeutic molecule are present on different plasmids. In some embodiments, the gene encoding the ROS-sensing transcription factor and the gene or gene cassette for producing the therapeutic molecule are present on the same. In some embodiments, the gene encoding the ROS-sensing transcription factor is present on a chromosome. In some embodiments, the gene encoding the ROS-sensing transcription factor and the gene or gene cassette for producing the therapeutic molecule are present on different chromosomes. In some embodiments, the gene encoding the ROS-sensing transcription factor and the gene or gene cassette for producing the therapeutic molecule are present on the same chromosome.

In some embodiments, the genetically engineered bacteria comprise a wild-type gene encoding a ROS-sensing transcription factor, e.g., the soxR gene, and a corresponding regulatory region, e.g., a soxS regulatory region, that is mutated relative to the wild-type regulatory region from bacteria of the same subtype. The mutated regulatory region increases the expression of the branched chain amino acid catabolism enzyme, BCAA transporter, and/or BCAA binding protein in the presence of ROS, as compared to the wild-type regulatory region under the same conditions. In some embodiments, the genetically engineered bacteria comprise a wild-type ROS-responsive regulatory region, e.g., the oxyS regulatory region, and a corresponding transcription factor, e.g., OxyR, that is mutated relative to the wild-type transcription factor from bacteria of the same subtype. The mutant transcription factor increases the expression of the branched chain amino acid catabolism enzyme, BCAA transporter, and/or BCAA binding protein in the presence of ROS, as compared to the wild-type transcription factor under the same conditions. In some embodiments, both the ROS-sensing transcription factor and corresponding regulatory region are mutated relative to the wild-type sequences from bacteria of the same subtype in order to increase expression of the branched chain amino acid catabolism enzymein the presence of ROS.

In some embodiments, the gene or gene cassette for producing the branched chain amino acid catabolism enzymeis present on a plasmid and operably linked to a promoter that is induced by ROS. In some embodiments, the gene or gene cassette for producing the branched chain amino acid catabolism enzymeis present in the chromosome and operably linked to a promoter that is induced by ROS. In some embodiments, the gene or gene cassette for producing the branched chain amino acid catabolism enzymeis present on a chromosome and operably linked to a promoter that is induced by exposure to tetracycline. In some embodiments, the gene or gene cassette for producing the branched chain amino acid catabolism enzyme, BCAA transporter, and/or BCAA binding protein is present on a plasmid and operably linked to a promoter that is induced by exposure to tetracycline. In some embodiments, expression is further optimized by methods known in the art, e.g., by optimizing ribosomal binding sites, manipulating transcriptional regulators, and/or increasing mRNA stability.

In some embodiments, the genetically engineered bacteria may comprise multiple copies of the gene(s) capable of producing an amino acid catabolism enzyme(s), BCAA transporter(s), and/or BCAA binding protein(s). In some embodiments, the gene(s) capable of producing an amino acid catabolism enzyme(s), BCAA transporter(s), and/or BCAA binding protein(s) is present on a plasmid and operatively linked to a ROS-responsive regulatory region. In some embodiments, the gene(s) capable of producing a branched chain amino acid catabolism enzyme, BCAA transporter, and/or BCAA binding protein is present in a chromosome and operatively linked to a ROS-responsive regulatory region.

Thus, in some embodiments, the genetically engineered bacteria or genetically engineered virus produce one or more amino acid catabolism enzymes under the control of an oxygen level-dependent promoter, a reactive oxygen species (ROS)-dependent promoter, or a reactive nitrogen species (RNS)-dependent promoter, and a corresponding transcription factor.

In some embodiments, the genetically engineered bacteria comprise a stably maintained plasmid or chromosome carrying a gene for producing an amino acid catabolism enzyme, BCAA transporter, and/or BCAA binding protein such that the branched chain amino acid catabolism enzyme, BCAA transporter, and/or BCAA binding protein can be expressed in the host cell, and the host cell is capable of survival and/or growth in vitro, e.g., in medium, and/or in vivo. In some embodiments, a bacterium may comprise multiple copies of the gene encoding the amino acid catabolism enzyme, BCAA transporter, and/or BCAA binding protein. In some embodiments, the gene encoding the branched chain amino acid catabolism enzyme, BCAA transporter, and/or BCAA binding protein is expressed on a low-copy plasmid. In some embodiments, the low-copy plasmid may be useful for increasing stability of expression. In some embodiments, the low-copy plasmid may be useful for decreasing leaky expression under non-inducing conditions. In some embodiments, the gene encoding the branched chain amino acid catabolism enzyme, BCAA transporter, and/or BCAA binding protein is expressed on a high-copy plasmid. In some embodiments, the high-copy plasmid may be useful for increasing expression of the amino acid catabolism enzyme, BCAA transporter, and/or BCAA binding protein. In some embodiments, the gene encoding the branched chain amino acid catabolism enzyme, BCAA transporter, and/or BCAA binding protein is expressed on a chromosome.

In some embodiments, the bacteria are genetically engineered to include multiple mechanisms of action (MOAs), e.g., circuits producing multiple copies of the same product (e.g., to enhance copy number) or circuits performing multiple different functions. For example, the genetically engineered bacteria may include four copies of the gene encoding a particular branched chain amino acid catabolism enzyme, BCAA transporter, and/or BCAA binding protein inserted at four different insertion sites. Alternatively, the genetically engineered bacteria may include three copies of the gene encoding a particular branched chain amino acid catabolism enzyme, BCAA transporter, and/or BCAA binding protein inserted at three different insertion sites and three copies of the gene encoding a different branched chain amino acid catabolism enzyme, BCAA transporter, and/or BCAA binding protein inserted at three different insertion sites.

In some embodiments, under conditions where the branched chain amino acid catabolism enzyme, BCAA transporter, and/or BCAA binding protein is expressed, the genetically engineered bacteria of the disclosure produce at least about 1.5-fold, at least about 2-fold, at least about 10-fold, at least about 15-fold, at least about 20-fold, at least about 30-fold, at least about 50-fold, at least about 100-fold, at least about 200-fold, at least about 300-fold, at least about 400-fold, at least about 500-fold, at least about 600-fold, at least about 700-fold, at least about 800-fold, at least about 900-fold, at least about 1,000-fold, or at least about 1,500-fold more of the amino acid catabolism enzyme, BCAA transporter, and/or BCAA binding protein and/or transcript of the gene(s) in the operon as compared to unmodified bacteria of the same subtype under the same conditions.

In some embodiments, quantitative PCR (qPCR) is used to amplify, detect, and/or quantify mRNA expression levels of the branched chain amino acid catabolism enzyme, BCAA transporter, and/or BCAA binding protein gene(s). Primers specific for branched chain amino acid catabolism enzyme, BCAA transporter, and/or BCAA binding protein gene(s) may be designed and used to detect mRNA in a sample according to methods known in the art. In some embodiments, a fluorophore is added to a sample reaction mixture that may contain branched chain amino acid catabolism enzymemRNA, and a thermal cycler is used to illuminate the sample reaction mixture with a specific wavelength of light and detect the subsequent emission by the fluorophore. The reaction mixture is heated and cooled to predetermined temperatures for predetermined time periods. In certain embodiments, the heating and cooling is repeated for a predetermined number of cycles. In some embodiments, the reaction mixture is heated and cooled to 90-100° C, 60-70° C, and 30-50° C for a predetermined number of cycles. In a certain embodiment, the reaction mixture is heated and cooled to 93-97° C, 55-65° C, and 35-45° C for a predetermined number of cycles. In some embodiments, the accumulating amplicon is quantified after each cycle of the qPCR. The number of cycles at which fluorescence exceeds the threshold is the threshold cycle (CT). At least one CT result for each sample is generated, and the CT result(s) may be used to determine mRNA expression levels of the branched chain amino acid catabolism enzyme, BCAA transporter, and/or BCAA binding protein gene(s).

In some embodiments, quantitative PCR (qPCR) is used to amplify, detect, and/or quantify mRNA expression levels of the branched chain amino acid catabolism enzyme, BCAA transporter, and/or BCAA binding protein gene(s). Primers specific for branched chain amino acid catabolism enzyme, BCAA transporter, and/or BCAA binding protein gene(s) may be designed and used to detect mRNA in a sample according to methods known in the art. In some embodiments, a fluorophore is added to a sample reaction mixture that may contain branched chain amino acid catabolism enzyme, BCAA transporter, and/or BCAA binding protein mRNA, and a thermal cycler is used to illuminate the sample reaction mixture with a specific wavelength of light and detect the subsequent emission by the fluorophore. The reaction mixture is heated and cooled to predetermined temperatures for predetermined time periods. In certain embodiments, the heating and cooling is repeated for a predetermined number of cycles. In some embodiments, the reaction mixture is heated and cooled to 90-100° C, 60-70° C, and 30-50° C for a predetermined number of cycles. In a certain embodiment, the reaction mixture is heated and cooled to 93-97° C, 55-65° C, and 35-45° C for a predetermined number of cycles. In some embodiments, the accumulating amplicon is quantified after each cycle of the qPCR. The number of cycles at which fluorescence exceeds the threshold is the threshold cycle (CT). At least one CT result for each sample is generated, and the CT result(s) may be used to determine mRNA expression levels of the branched chain amino acid catabolism enzyme, BCAA transporter, and/or BCAA binding protein gene(s).

In other embodiments, the inducible promoter is a propionate responsive promoter. For example, the *prpR* promoter is a propionate responsive promoter. In one embodiment, the propionate responsive promoter comprises SEQ ID NO: 13.

### Essential Genes and Auxotrophs

As used herein, the term "essential gene" refers to a gene which is necessary to for cell growth and/or survival. Bacterial essential genes are well known to one of ordinary skill in the art, and can be identified by directed deletion of genes and/or random mutagenesis and screening (see, for example, Zhang and Lin, 2009, DEG 5.0, a database of essential genes in both prokaryotes and eukaryotes, Nucl. Acids Res., 37:D455-D458 and Gerdes et al., Essential genes on metabolic maps, Curr. Opin. Biotechnol., 17(5):448-456 .

An "essential gene" may be dependent on the circumstances and environment in which an organism lives. For example, a mutation of, modification of, or excision of an essential gene may result in the recombinant bacteria of the disclosure becoming an auxotroph. An auxotrophic modification is intended to cause bacteria to die in the absence of an exogenously added nutrient essential for survival or growth because they lack the gene(s) necessary to produce that essential nutrient.

An auxotrophic modification is intended to cause bacteria to die in the absence of an exogenously added nutrient essential for survival or growth because they lack the gene(s) necessary to produce that essential nutrient. In some embodiments, any of the genetically engineered bacteria described herein also comprise a deletion or mutation in one or more gene(s) required for cell survival and/or growth.

In some embodiments, the bacterial cell comprises a genetic mutation in one or more endogenous gene(s) encoding a branched chain amino acid biosynthesis gene, wherein the genetic mutation reduces biosynthesis of one or more branched chain amino acids in the bacterial cell. In some embodiments, the endogenous gene encoding a branched chain amino acid biosynthesis gene is a keto acid reductoisomerase gene. Keto acid reductoisomerase gene is required for branched chain amino acid synthesis. Knock-out of this gene creates an auxotroph and requires the cell to import leucine to survive. In some embodiments, the bacterial cell comprises a genetic mutation in *ilvC* gene.

In one embodiment, the essential gene is an oligonucleotide synthesis gene, for example, *thyA.* In another embodiment, the essential gene is a cell wall synthesis gene, for example, *dapA.* In yet another embodiment, the essential gene is an amino acid gene, for example, *serA* or *MetA.* Any gene required for cell survival and/or growth may be targeted, including but not limited to, *cysE, glnA, ilvD, leuB, lysA, serA, metA, glyA, hisB, ilvA, pheA, proA, thrC, trpC, tyrA, thyA, uraA, dapA, dapB, dapD, dapE, dapF, flhD, metB, metC, proAB,* and *thi1,* as long as the corresponding wild-type gene product is not produced in the bacteria.

**Table** 9 lists depicts exemplary bacterial genes which may be disrupted or deleted to produce an auxotrophic strain. These include, but are not limited to, genes required for oligonucleotide synthesis, amino acid synthesis, and cell wall synthesis.

**Table 9. Non-limiting Examples of Bacterial Genes Useful for Generation of an Auxotroph**

| **Amino Acid** | **Oligonucleotide** | **Cell Wall** |
|---|---|---|
| cysE | thyA | dapA |
| glnA | uraA | dapB |
| ilvD | | dapD |
| leuB | | dapE |
| lysA | | dapF |
| serA | | |
| metA | | |
| glyA | | |
| hisB | | |
| ilvA | | |
| pheA | | |
| proA | | |
| thrC | | |
| trpC | | |
| tyrA | | |

**Table 10** shows the survival of various amino acid auxotrophs in the mouse gut, as detected 24 hrs and 48 hrs post-gavage. These auxotrophs were generated using BW25113, a non-Nissle strain of E. coli.

**Table 10. Survival of amino acid auxotrophs in the mouse gut**

| **Gene** | **AA Auxotroph** | **Pre-Gavage** | **24 hours** | **48 hours** |
|---|---|---|---|---|
| **argA** | **Arginine** | Present | Present | Absent |
| **cysE** | **Cysteine** | Present | Present | Absent |
| **glnA** | **Glutamine** | Present | Present | Absent |
| **glyA** | **Glycine** | Present | Present | Absent |
| **hisB** | **Histidine** | Present | Present | Present |
| **ilvA** | **Isoleucine** | Present | Present | Absent |
| **leuB** | **Leucine** | Present | Present | Absent |
| **lysA** | **Lysine** | Present | Present | Absent |
| **metA** | **Methionine** | Present | Present | Present |
| **pheA** | **Phenylalanine** | Present | Present | Present |
| **proA** | **Proline** | Present | Present | Absent |
| **serA** | **Serine** | Present | Present | Present |
| **thrC** | **Threonine** | Present | Present | Present |
| **trpC** | **Tryptophan** | Present | Present | Present |
| **tyrA** | **Tyrosine** | Present | Present | Present |
| **ilvD** | **Valine/Isoleucinel Leucine** | Present | Present | Absent |
| **thyA** | **Thiamine** | Present | Absent | Absent |
| **uraA** | **Uracil** | Present | Absent | Absent |
| **flhD** | **FlhD** | Present | Present | Present |

For example, thymine is a nucleic acid that is required for bacterial cell growth; in its absence, bacteria undergo cell death. The *thyA* gene encodes thimidylate synthetase, an enzyme that catalyzes the first step in thymine synthesis by converting dUMP to dTMP (Sat *et al.,* 2003). In some embodiments, the bacterial cell of the disclosure is a *thyA* auxotroph in which the *thyA* gene is deleted and/or replaced with an unrelated gene. A *thyA* auxotroph can grow only when sufficient amounts of thymine are present, *e.g.,* by adding thymine to growth media in vitro, or in the presence of high thymine levels found naturally in the human gut *in vivo.* In some embodiments, the bacterial cell of the disclosure is auxotrophic in a gene that is complemented when the bacterium is present in the mammalian gut. Without sufficient amounts of thymine, the *thyA* auxotroph dies. In some embodiments, the auxotrophic modification is used to ensure that the bacterial cell does not survive in the absence of the auxotrophic gene product (*e.g*., outside of the gut).

Diaminopimelic acid (DAP) is an amino acid synthetized within the lysine biosynthetic pathway and is required for bacterial cell wall growth (Meadow *et al.,* 1959; Clarkson *et al.,* 1971). In some embodiments, any of the genetically engineered bacteria described herein is a *dapD* auxotroph in which *dapD* is deleted and/or replaced with an unrelated gene. A *dapD* auxotroph can grow only when sufficient amounts of DAP are present, *e.g.,* by adding DAP to growth media *in vitro.* Without sufficient amounts of DAP, the *dapD* auxotroph dies. In some embodiments, the auxotrophic modification is used to ensure that the bacterial cell does not survive in the absence of the auxotrophic gene product (*e.g.,* outside of the gut).

In other embodiments, the genetically engineered bacterium of the present disclosure is a *uraA* auxotroph in which *uraA* is deleted and/or replaced with an unrelated gene. The *uraA* gene codes for UraA, a membrane-bound transporter that facilitates the uptake and subsequent metabolism of the pyrimidine uracil (Andersen *et al.,* 1995). A *uraA* auxotroph can grow only when sufficient amounts of uracil are present, *e.g.,* by adding uracil to growth media *in vitro.* Without sufficient amounts of uracil, the *uraA* auxotroph dies. In some embodiments, auxotrophic modifications are used to ensure that the bacteria do not survive in the absence of the auxotrophic gene product (*e.g.*, outside of the gut).

In complex communities, it is possible for bacteria to share DNA. In very rare circumstances, an auxotrophic bacterial strain may receive DNA from a non-auxotrophic strain, which repairs the genomic deletion and permanently rescues the auxotroph. Therefore, engineering a bacterial strain with more than one auxotroph may greatly decrease the probability that DNA transfer will occur enough times to rescue the auxotrophy. In some embodiments, the genetically engineered bacteria comprise a deletion or mutation in two or more genes required for cell survival and/or growth.

Other examples of essential genes include, but are not limited to *yhbV, yagG, hemB, seeD, secF, ribD, ribE, thiL, dxs, ispA, dnaX, adk, hemH, lpxH, cysS, fold, rplT, infC, thrS, nadE, gapA, yeaZ, aspS, argS, pgsA, yefM, metG, folE, yejM, gyrA, nrdA, nrdB, folC, accD, fabB, gltX, liga, zipA, dapE, dapA, der, hisS, ispG, suhB, tadA, acpS, era, rnc, ftsB, eno, pyrG, chpR, lgt, fbaA, pgk, yqgD, metK, yqgF, plsC, ygiT, pare, ribB, cca, ygjD, tdcF, yraL, yihA, ftsN, murI, murB, birA, secE, nusG, rplJ, rplL, rpoB, rpoC, ubiA, plsB, lexA, dnaB, ssb, alsK, groS, psd, orn, yjeE, rpsR, chpS, ppa, valS, yjgP, yjgQ, dnaC, ribF, AspA, ispH, dapB, folA, imp, yabQ, ftsL, ftsI, murE, murF, mraY, murD, ftsW, murG, murC, ftsQ, ftsA, ftsZ, lpxC, seeM, secA, can, folK, hemL, yadR, dapD, map, rpsB, infB ,nusA, ftsH, obgE, rpmA, rplU, ispB, murA, yrbB, yrbK, yhbN, rpsI, rplM, degS, mreD, mreC, mreB, accB, accC, yrdC, def, fmt, rplQ, rpoA, rpsD, rpsK, rpsM, entD, mrdB, mrdA, nadD, hlepB, rpoE, pssA, yfiO, rplS, trmD, rpsP, ffh, grpE, yfjB, csrA, ispF, ispD, rplW, rplD, rplC, rpsJ, fusA, rpsG, rpsL, trpS, yrfF, asd, rpoH, ftsX, ftsE, ftsY, frr, dxr, ispU, rfaK, kdtA, coaD, rpmB, dfp, dut, gmk, spot, gyrB, dnaN, dnaA, rpmH, rnpA, yidC, tnaB, glmS, glmU, wzyE, hemD, hemC, yigP, ubiB, ubiD, hemG, secY, rplO, rpmD, rpsE, rplR, rplF, rpsH, rpsN, rplE, rplX, rplN, rpsQ, rpmC, rplP, rpsC, rplV, rpsS, rplB, cdsA, yaeL, yaeT, lpxD, fabZ, lpxA, lpxB, dnaE, accA, tilS, proS, yafF, tsf, pyrH, olA, rlpB, leuS, Int, glnS, fldA, cydA, infa, cydC, ftsK, lolA, serS, rpsA, msbA, lpxK, kdsB, mukF, mukE, mukB, asnS, fabA, mviN, rne, yceQ, fabD, fabG, acpP, tmk, holB, lolC, lolD, lolE, purB, ymfK, minE, mind, pth, rsA, ispE, lolB, hemA, prfA, prmC, kdsA, topA, ribA, fabI, racR, dicA, ydfB, tyrS, ribC, ydiL, pheT, pheS, yhhQ, bcsB, glyQ, yibJ,* and *gpsA.* Other essential genes are known to those of ordinary skill in the art.

In some embodiments, the genetically engineered bacterium of the present disclosure is a synthetic ligand-dependent essential gene (SLiDE) bacterial cell. SLiDE bacterial cells are synthetic auxotrophs with a mutation in one or more essential genes that only grow in the presence of a particular ligand (see Lopez and Anderson "Synthetic Auxotrophs with Ligand-Dependent Essential Genes for a BL21 (DE3 Biosafety Strain, "ACS Synthetic Biology (2015) DOI: 10.1021/acssynbio.5b00085).

In some embodiments, the SLiDE bacterial cell comprises a mutation in an essential gene. In some embodiments, the essential gene is selected from the group consisting of *pheS, dnaN, tyrS, metG* and *adk.* In some embodiments, the essential gene is *dnaN* comprising one or more of the following mutations: H191N, R240C, I317S, F319V, L340T, V347I, and S345C. In some embodiments, the essential gene is *dnaN* comprising the mutations H191N, R240C, I317S, F319V, L340T, V347I, and S345C. In some embodiments, the essential gene is *pheS* comprising one or more of the following mutations: F125G, P183T, P184A, R186A, and I188L. In some embodiments, the essential gene is *pheS* comprising the mutations F125G, P183T, P184A, R186A, and I188L. In some embodiments, the essential gene is *tyrS* comprising one or more of the following mutations: L36V, C38A and F40G. In some embodiments, the essential gene is *tyrS* comprising the mutations L36V, C38A and F40G. In some embodiments, the essential gene is *metG* comprising one or more of the following mutations: E45Q, N47R, I49G, and A51C. In some embodiments, the essential gene is *metG* comprising the mutations E45Q, N47R, I49G, and A51C. In some embodiments, the essential gene is *adk* comprising one or more of the following mutations: I4L, LSI and L6G. In some embodiments, the essential gene is *adk* comprising the mutations I4L, LSI and L6G.

In some embodiments, the genetically engineered bacterium is complemented by a ligand. In some embodiments, the ligand is selected from the group consisting of benzothiazole, indole, 2-aminobenzothiazole, indole-3-butyric acid, indole-3-acetic acid, and L-histidine methyl ester. For example, bacterial cells comprising mutations in metG (E45Q, N47R, I49G, and A51C) are complemented by benzothiazole, indole, 2-aminobenzothiazole, indole-3-butyric acid, indole-3-acetic acid or L-histidine methyl ester. Bacterial cells comprising mutations in dnaN (H191N, R240C, I317S, F319V, L340T, V347I, and S345C) are complemented by benzothiazole, indole or 2-aminobenzothiazole. Bacterial cells comprising mutations in pheS (F125G, P183T, P184A, R186A, and I188L) are complemented by benzothiazole or 2-aminobenzothiazole. Bacterial cells comprising mutations in tyrS (L36V, C38A, and F40G) are complemented by benzothiazole or 2-aminobenzothiazole. Bacterial cells comprising mutations in adk (I4L, LSI and L6G) are complemented by benzothiazole or indole.

In some embodiments, the genetically engineered bacterium comprises more than one mutant essential gene that renders it auxotrophic to a ligand. In some embodiments, the bacterial cell comprises mutations in two essential genes. For example, in some embodiments, the bacterial cell comprises mutations in *tyrS* (L36V, C38A, and F40G) and *metG* (E45Q, N47R, I49G, and A51C). In other embodiments, the bacterial cell comprises mutations in three essential genes. For example, in some embodiments, the bacterial cell comprises mutations in *tyrS* (L36V, C38A, and F40G), *metG* (E45Q, N47R, I49G, and A51C), and *pheS* (F125G, P183T, P184A, R186A, and I188L).

In some embodiments, the genetically engineered bacterium is a conditional auxotroph whose essential gene(s) is replaced using the arabinose system described herein.

In some embodiments, the genetically engineered bacterium of the disclosure is an auxotroph and also comprises kill-switch circuitry, such as any of the kill-switch components and systems described herein. For example, the recombinant bacteria may comprise a deletion or mutation in an essential gene required for cell survival and/or growth, for example, in a DNA synthesis gene, for example, *thyA,* cell wall synthesis gene, for example, *dapA* and/or an amino acid gene, for example, *serA* or *MetA* or *ilvC,* and may also comprise a toxin gene that is regulated by one or more transcriptional activators that are expressed in response to an environmental condition(s) and/or signal(s) (such as the described arabinose system) or regulated by one or more recombinases that are expressed upon sensing an exogenous environmental condition(s) and/or signal(s) (such as the recombinase systems described herein). Other embodiments are described in Wright et al., "GeneGuard: A Modular Plasmid System Designed for Biosafety," ACS Synthetic Biology (2015) 4: 307-16).

In some embodiments, the genetically engineered bacterium of the disclosure is an auxotroph and also comprises kill-switch circuitry, such as any of the kill-switch components and systems described herein, as well as another biosecurity system, such a conditional origin of replication (see Wright *et al., supra*).

### Genetic Regulatory Circuits

In some embodiments, the genetically engineered bacteria comprise multilayered genetic regulatory circuits for expressing the constructs described herein (*see, e.g.,* U.S. Provisional Application No. 62/184,811).

The genetic regulatory circuits are useful to screen for mutant bacteria that produce a branched chain amino acid catabolism enzyme, BCAA transporter, and/or BCAA binding protein or rescue an auxotroph. In certain embodiments, the invention provides methods for selecting genetically engineered bacteria that produce one or more genes of interest.

In some embodiments, the invention provides genetically engineered bacteria comprising a gene or gene cassette for producing a payload and a T7 polymerase-regulated genetic regulatory circuit. For example, the genetically engineered bacteria comprise a first gene encoding a T7 polymerase, wherein the first gene is operably linked to a fumarate and nitrate reductase regulator (FNR)-responsive promoter; a second gene or gene cassette for producing a payload, wherein the second gene or gene cassette is operably linked to a T7 promoter that is induced by the T7 polymerase; and a third gene encoding an inhibitory factor, lysY, that is capable of inhibiting the T7 polymerase. In the presence of oxygen, FNR does not bind the FNR-responsive promoter, and the payload is not expressed. LysY is expressed constitutively (P-lac constitutive) and further inhibits T7 polymerase. In the absence of oxygen, FNR dimerizes and binds to the FNR-responsive promoter, T7 polymerase is expressed at a level sufficient to overcome lysY inhibition, and the payload is expressed. In some embodiments, the lysY gene is operably linked to an additional FNR binding site. In the absence of oxygen, FNR dimerizes to activate T7 polymerase expression as described above, and also inhibits lysY expression.

In some embodiments, the invention provides genetically engineered bacteria comprising a gene or gene cassette for producing a payload and a protease-regulated genetic regulatory circuit. For example, the genetically engineered bacteria comprise a first gene encoding an mf-lon protease, wherein the first gene is operably linked to a FNR-responsive promoter; a second gene or gene cassette for producing a payload operably linked to a tet regulatory region (tetO); and a third gene encoding an mf-lon degradation signal linked to a tet repressor (tetR), wherein the tetR is capable of binding to the tet regulatory region and repressing expression of the second gene or gene cassette. The mf-lon protease is capable of recognizing the mf-lon degradation signal and degrading the tetR. In the presence of oxygen, FNR does not bind the FNR-responsive promoter, the repressor is not degraded, and the payload is not expressed. In the absence of oxygen, FNR dimerizes and binds the FNR-responsive promoter, thereby inducing expression of mf-lon protease. The mf-lon protease recognizes the mf-lon degradation signal and degrades the tetR, and the payload is expressed.

In some embodiments, the invention provides genetically engineered bacteria comprising a gene or gene cassette for producing a payload and a repressor-regulated genetic regulatory circuit. For example, the genetically engineered bacteria comprise a first gene encoding a first repressor, wherein the first gene is operably linked to a FNR-responsive promoter; a second gene or gene cassette for producing a payload operably linked to a first regulatory region comprising a constitutive promoter; and a third gene encoding a second repressor, wherein the second repressor is capable of binding to the first regulatory region and repressing expression of the second gene or gene cassette. The third gene is operably linked to a second regulatory region comprising a constitutive promoter, wherein the first repressor is capable of binding to the second regulatory region and inhibiting expression of the second repressor. In the presence of oxygen, FNR does not bind the FNR-responsive promoter, the first repressor is not expressed, the second repressor is expressed, and the payload is not expressed. In the absence of oxygen, FNR dimerizes and binds the FNR-responsive promoter, the first repressor is expressed, the second repressor is not expressed, and the payload is expressed.

Examples of repressors useful in these embodiments include, but are not limited to, ArgR, TetR, ArsR, AscG, LacI, CscR, DeoR, DgoR, FruR, GalR, GatR, CI, LexA, RafR, QacR, and PtxS (US20030166191).

In some embodiments, the invention provides genetically engineered bacteria comprising a gene or gene cassette for producing a payload and a regulatory RNA-regulated genetic regulatory circuit. For example, the genetically engineered bacteria comprise a first gene encoding a regulatory RNA, wherein the first gene is operably linked to a FNR-responsive promoter, and a second gene or gene cassette for producing a payload. The second gene or gene cassette is operably linked to a constitutive promoter and further linked to a nucleotide sequence capable of producing an mRNA hairpin that inhibits translation of the payload. The regulatory RNA is capable of eliminating the mRNA hairpin and inducing payload translation via the ribosomal binding site. In the presence of oxygen, FNR does not bind the FNR-responsive promoter, the regulatory RNA is not expressed, and the mRNA hairpin prevents the payload from being translated. In the absence of oxygen, FNR dimerizes and binds the FNR-responsive promoter, the regulatory RNA is expressed, the mRNA hairpin is eliminated, and the payload is expressed.

In some embodiments, the invention provides genetically engineered bacteria comprising a gene or gene cassette for producing a payload and a CRISPR-regulated genetic regulatory circuit. For example, the genetically engineered bacteria comprise a Cas9 protein; a first gene encoding a CRISPR guide RNA, wherein the first gene is operably linked to a FNR-responsive promoter; a second gene or gene cassette for producing a payload, wherein the second gene or gene cassette is operably linked to a regulatory region comprising a constitutive promoter; and a third gene encoding a repressor operably linked to a constitutive promoter, wherein the repressor is capable of binding to the regulatory region and repressing expression of the second gene or gene cassette. The third gene is further linked to a CRISPR target sequence that is capable of binding to the CRISPR guide RNA, wherein said binding to the CRISPR guide RNA induces cleavage by the Cas9 protein and inhibits expression of the repressor. In the presence of oxygen, FNR does not bind the FNR-responsive promoter, the guide RNA is not expressed, the repressor is expressed, and the payload is not expressed. In the absence of oxygen, FNR dimerizes and binds the FNR-responsive promoter, the guide RNA is expressed, the repressor is not expressed, and the payload is expressed.

In some embodiments, the invention provides genetically engineered bacteria comprising a gene or gene cassette for producing a payload and a recombinase-regulated genetic regulatory circuit. For example, the genetically engineered bacteria comprise a first gene encoding a recombinase, wherein the first gene is operably linked to a FNR-responsive promoter, and a second gene or gene cassette for producing a payload operably linked to a constitutive promoter. The second gene or gene cassette is inverted in orientation (3' to 5') and flanked by recombinase binding sites, and the recombinase is capable of binding to the recombinase binding sites to induce expression of the second gene or gene cassette by reverting its orientation (5' to 3'). In the presence of oxygen, FNR does not bind the FNR-responsive promoter, the recombinase is not expressed, the payload remains in the 3' to 5' orientation, and no functional payload is produced. In the absence of oxygen, FNR dimerizes and binds the FNR-responsive promoter, the recombinase is expressed, the payload is reverted to the 5' to 3' orientation, and functional payload is produced.

In some embodiments, the invention provides genetically engineered bacteria comprising a gene or gene cassette for producing a payload and a polymerase- and recombinase-regulated genetic regulatory circuit. For example, the genetically engineered bacteria comprise a first gene encoding a recombinase, wherein the first gene is operably linked to a FNR-responsive promoter; a second gene or gene cassette for producing a payload operably linked to a T7 promoter; a third gene encoding a T7 polymerase, wherein the T7 polymerase is capable of binding to the T7 promoter and inducing expression of the payload. The third gene encoding the T7 polymerase is inverted in orientation (3' to 5') and flanked by recombinase binding sites, and the recombinase is capable of binding to the recombinase binding sites to induce expression of the T7 polymerase gene by reverting its orientation (5' to 3'). In the presence of oxygen, FNR does not bind the FNR-responsive promoter, the recombinase is not expressed, the T7 polymerase gene remains in the 3' to 5' orientation, and the payload is not expressed. In the absence of oxygen, FNR dimerizes and binds the FNR-responsive promoter, the recombinase is expressed, the T7 polymerase gene is reverted to the 5' to 3' orientation, and the payload is expressed.

### Kill Switches

In some embodiments, the genetically engineered bacteria also comprise a kill switch (*see, e.g.,* U.S. Provisional Application Nos. 62/183,935 and 62/263,329).

The kill switch is intended to actively kill engineered microbes in response to external stimuli. As opposed to an auxotrophic mutation where bacteria die because they lack an essential nutrient for survival, the kill switch is triggered by a particular factor in the environment that induces the production of toxic molecules within the microbe that cause cell death.

Bacteria engineered with kill switches have been engineered for *in vitro* research purposes, *e.g.,* to limit the spread of a biofuel-producing microorganism outside of a laboratory environment. Bacteria engineered for *in vivo* administration to treat a disease or disorder may also be programmed to die at a specific time after the expression and delivery of a heterologous gene or genes, for example, a therapeutic gene(s) or after the subject has experienced the therapeutic effect. For example, in some embodiments, the kill switch is activated to kill the bacteria after a period of time following expression of an amino acid catabolism enzyme. In some embodiments, the kill switch is activated in a delayed fashion following expression of the amino acid catabolism gene, for example, after the production of the amino acid catabolism enzyme. Alternatively, the bacteria may be engineered to die after the bacteria has spread outside of a disease site. Specifically, it may be useful to prevent long-term colonization of subjects by the microorganism, spread of the microorganism outside the area of interest (for example, outside the gut) within the subject, or spread of the microorganism outside of the subject into the environment (for example, spread to the environment through the stool of the subject).

Examples of such toxins that can be used in kill-switches include, but are not limited to, bacteriocins, lysins, and other molecules that cause cell death by lysing cell membranes, degrading cellular DNA, or other mechanisms. Such toxins can be used individually or in combination. The switches that control their production can be based on, for example, transcriptional activation (toggle switches; *see, e.g.,* Gardner *et al*., 2000), translation (riboregulators), or DNA recombination (recombinase-based switches), and can sense environmental stimuli such as anaerobiosis or reactive oxygen species. These switches can be activated by a single environmental factor or may require several activators in AND, OR, NAND and NOR logic configurations to induce cell death. For example, an AND riboregulator switch is activated by tetracycline, isopropyl β-D-1-thiogalactopyranoside (IPTG), and arabinose to induce the expression of lysins, which permeabilize the cell membrane and kill the cell. IPTG induces the expression of the endolysin and holin mRNAs, which are then derepressed by the addition of arabinose and tetracycline. All three inducers must be present to cause cell death. Examples of kill switches are known in the art (Callura *et al*., 2010). In some embodiments, the kill switch is activated to kill the bacteria after a period of time following oxygen level-dependent expression of an amino acid catabolism enzyme. In some embodiments, the kill switch is activated in a delayed fashion following oxygen level-dependent expression of an amino acid catabolism enzyme.

Kill-switches can be designed such that a toxin is produced in response to an environmental condition or external signal (*e.g.,* the bacteria is killed in response to an external cue; *i.e*., an activation-based kill switch, see **Fig. 34-37**) or, alternatively designed such that a toxin is produced once an environmental condition no longer exists or an external signal is ceased (*i.e.,* a repression-based kill switch, see **Figs. 38-42**).

Thus, in some embodiments, the genetically engineered bacteria of the disclosure are further programmed to die after sensing an exogenous environmental signal, for example, in a low oxygen environment. In some embodiments, the genetically engineered bacteria of the present disclosure, *e.g.*, bacteria expressing an amino acid catabolism enzyme, comprise one or more genes encoding one or more recombinase(s), whose expression is induced in response to an environmental condition or signal and causes one or more recombination events that ultimately leads to the expression of a toxin which kills the cell. In some embodiments, the at least one recombination event is the flipping of an inverted heterologous gene encoding a bacterial toxin which is then constitutively expressed after it is flipped by the first recombinase. In one embodiment, constitutive expression of the bacterial toxin kills the genetically engineered bacterium. In these types of kill-switch systems once the engineered bacterial cell senses the exogenous environmental condition and expresses the heterologous gene of interest, the recombinant bacterial cell is no longer viable.

In another embodiment in which the genetically engineered bacteria of the present disclosure, *e.g.*, bacteria expressing an amino acid catabolism enzyme, express one or more recombinase(s) in response to an environmental condition or signal causing at least one recombination event, the genetically engineered bacterium further expresses a heterologous gene encoding an anti-toxin in response to an exogenous environmental condition or signal. In one embodiment, the at least one recombination event is flipping of an inverted heterologous gene encoding a bacterial toxin by a first recombinase. In one embodiment, the inverted heterologous gene encoding the bacterial toxin is located between a first forward recombinase recognition sequence and a first reverse recombinase recognition sequence. In one embodiment, the heterologous gene encoding the bacterial toxin is constitutively expressed after it is flipped by the first recombinase. In one embodiment, the anti-toxin inhibits the activity of the toxin, thereby delaying death of the genetically engineered bacterium. In one embodiment, the genetically engineered bacterium is killed by the bacterial toxin when the heterologous gene encoding the anti-toxin is no longer expressed when the exogenous environmental condition is no longer present.

In another embodiment, the at least one recombination event is flipping of an inverted heterologous gene encoding a second recombinase by a first recombinase, followed by the flipping of an inverted heterologous gene encoding a bacterial toxin by the second recombinase. In one embodiment, the inverted heterologous gene encoding the second recombinase is located between a first forward recombinase recognition sequence and a first reverse recombinase recognition sequence. In one embodiment, the inverted heterologous gene encoding the bacterial toxin is located between a second forward recombinase recognition sequence and a second reverse recombinase recognition sequence. In one embodiment, the heterologous gene encoding the second recombinase is constitutively expressed after it is flipped by the first recombinase. In one embodiment, the heterologous gene encoding the bacterial toxin is constitutively expressed after it is flipped by the second recombinase. In one embodiment, the genetically engineered bacterium is killed by the bacterial toxin. In one embodiment, the genetically engineered bacterium further expresses a heterologous gene encoding an anti-toxin in response to the exogenous environmental condition. In one embodiment, the anti-toxin inhibits the activity of the toxin when the exogenous environmental condition is present, thereby delaying death of the genetically engineered bacterium. In one embodiment, the genetically engineered bacterium is killed by the bacterial toxin when the heterologous gene encoding the anti-toxin is no longer expressed when the exogenous environmental condition is no longer present.

In one embodiment, the at least one recombination event is flipping of an inverted heterologous gene encoding a second recombinase by a first recombinase, followed by flipping of an inverted heterologous gene encoding a third recombinase by the second recombinase, followed by flipping of an inverted heterologous gene encoding a bacterial toxin by the third recombinase. Accordingly, in one embodiment, the disclosure provides at least 1, 2, 3, 4, 5, 6, 7, 8, 9, 10, 11, 12, 13, 14, 15, 16, 17, 18, 19, or 20 recombinases that can be used serially.

In one embodiment, the at least one recombination event is flipping of an inverted heterologous gene encoding a first excision enzyme by a first recombinase. In one embodiment, the inverted heterologous gene encoding the first excision enzyme is located between a first forward recombinase recognition sequence and a first reverse recombinase recognition sequence. In one embodiment, the heterologous gene encoding the first excision enzyme is constitutively expressed after it is flipped by the first recombinase. In one embodiment, the first excision enzyme excises a first essential gene. In one embodiment, the programmed recombinant bacterial cell is not viable after the first essential gene is excised.

In one embodiment, the first recombinase further flips an inverted heterologous gene encoding a second excision enzyme. In one embodiment, the wherein the inverted heterologous gene encoding the second excision enzyme is located between a second forward recombinase recognition sequence and a second reverse recombinase recognition sequence. In one embodiment, the heterologous gene encoding the second excision enzyme is constitutively expressed after it is flipped by the first recombinase. In one embodiment, the genetically engineered bacterium dies or is no longer viable when the first essential gene and the second essential gene are both excised. In one embodiment, the genetically engineered bacterium dies or is no longer viable when either the first essential gene is excised or the second essential gene is excised by the first recombinase.

In one embodiment, the first excision enzyme is Xis1. In one embodiment, the first excision enzyme is Xis2. In one embodiment, the first excision enzyme is Xis1, and the second excision enzyme is Xis2.

In one embodiment, the genetically engineered bacterium dies after the at least one recombination event occurs. In another embodiment, the genetically engineered bacterium is no longer viable after the at least one recombination event occurs.

In any of these embodiment, the recombinase can be a recombinase selected from the group consisting of: BxbI, PhiC31, TP901, BxbI, PhiC31, TP901, HK022, HP1, R4, Int1, Int2, Int3, Int4, Int5, Int6, Int7, Int8, Int9, Int10, Int11, Int12, Int13, Int14, Int15, Int16, Int17, Int18, Int19, Int20, Int21, Int22, Int23, Int24, Int25, Int26, Int27, Int28, Int29, Int30, Int31, Int32, Int33, and Int34, or a biologically active fragment thereof.

In the above-described kill-switch circuits, a toxin is produced in the presence of an environmental factor or signal. In another aspect of kill-switch circuitry, a toxin may be repressed in the presence of an environmental factor (not produced) and then produced once the environmental condition or external signal is no longer present. Such kill switches are called repression-based kill switches and represent systems in which the bacterial cells are viable only in the presence of an external factor or signal, such as arabinose or other sugar. Exemplary kill switch designs in which the toxin is repressed in the presence of an external factor or signal (and activated once the external signal is removed) is shown in **Figs 67-71****.** The disclosure provides recombinant bacterial cells which express one or more heterologous gene(s) upon sensing arabinose or other sugar in the exogenous environment. In this aspect, the recombinant bacterial cells contain the *araC* gene, which encodes the AraC transcription factor, as well as one or more genes under the control of the *araBAD* promoter. In the absence of arabinose, the AraC transcription factor adopts a conformation that represses transcription of genes under the control of the *araBAD* promoter. In the presence of arabinose, the AraC transcription factor undergoes a conformational change that allows it to bind to and activate the *araBAD* promoter, which induces expression of the desired gene, for example tetR, which represses expression of a toxin gene. In this embodiment, the taxing gene is repressed in the presence of arabinose or other sugar. In an environment where arabinose is not present, the tetR gene is not activated and the toxin is expressed, thereby killing the bacteria. The arabinose system can also be used to express an essential gene, in which the essential gene is only expressed in the presence of arabinose or other sugar and is not expressed when arabinose or other sugar is absent from the environment.

Thus, in some embodiments in which one or more heterologous gene(s) are expressed upon sensing arabinose in the exogenous environment, the one or more heterologous genes are directly or indirectly under the control of the *araBAD* promoter. In some embodiments, the expressed heterologous gene is selected from one or more of the following: a heterologous therapeutic gene, a heterologous gene encoding an antitoxin, a heterologous gene encoding a repressor protein or polypeptide, for example, a TetR repressor, a heterologous gene encoding an essential protein not found in the bacterial cell, and/or a heterologous encoding a regulatory protein or polypeptide.

Arabinose inducible promoters are known in the art, including Pₐᵣₐ, P_{araB}, P_{araC}, and P_{araBAD}. In one embodiment, the arabinose inducible promoter is from *E. coli.* In some embodiments, the P_{araC} promoter and the P_{araBAD} promoter operate as a bidirectional promoter, with the P_{araBAD} promoter controlling expression of a heterologous gene(s) in one direction, and the P_{araC} (in close proximity to, and on the opposite strand from the P_{araBAD} promoter), controlling expression of a heterologous gene(s) in the other direction. In the presence of arabinose, transcription of both heterologous genes from both promoters is induced. However, in the absence of arabinose, transcription of both heterologous genes from both promoters is not induced.

In one exemplary embodiment of the disclosure, the engineered bacteria of the present disclosure contains a kill-switch having at least the following sequences: a P_{araBAD} promoter operably linked to a heterologous gene encoding a Tetracycline Repressor Protein (TetR), a P_{araC} promoter operably linked to a heterologous gene encoding AraC transcription factor, and a heterologous gene encoding a bacterial toxin operably linked to a promoter which is repressed by the Tetracycline Repressor Protein (P_{TetR}). In the presence of arabinose, the AraC transcription factor activates the P_{araBAD} promoter, which activates transcription of the TetR protein which, in turn, represses transcription of the toxin. In the absence of arabinose, however, AraC suppresses transcription from the P_{araBAD} promoter and no TetR protein is expressed. In this case, expression of the heterologous toxin gene is activated, and the toxin is expressed. The toxin builds up in the recombinant bacterial cell, and the recombinant bacterial cell is killed. In one embodiment, the *araC* gene encoding the AraC transcription factor is under the control of a constitutive promoter and is therefore constitutively expressed.

In one embodiment of the disclosure, the recombinant bacterial cell further comprises an antitoxin under the control of a constitutive promoter. In this situation, in the presence of arabinose, the toxin is not expressed due to repression by TetR protein, and the antitoxin protein builds-up in the cell. However, in the absence of arabinose, TetR protein is not expressed, and expression of the toxin is induced. The toxin begins to build-up within the recombinant bacterial cell. The recombinant bacterial cell is no longer viable once the toxin protein is present at either equal or greater amounts than that of the anti-toxin protein in the cell, and the recombinant bacterial cell will be killed by the toxin.

In another embodiment of the disclosure, the recombinant bacterial cell further comprises an antitoxin under the control of the P_{araBAD} promoter. In this situation, in the presence of arabinose, TetR and the anti-toxin are expressed, the anti-toxin builds up in the cell, and the toxin is not expressed due to repression by TetR protein. However, in the absence of arabinose, both the TetR protein and the anti-toxin are not expressed, and expression of the toxin is induced. The toxin begins to build-up within the recombinant bacterial cell. The recombinant bacterial cell is no longer viable once the toxin protein is expressed, and the recombinant bacterial cell will be killed by the toxin.

In another exemplary embodiment of the disclosure, the engineered bacteria of the present disclosure contains a kill-switch having at least the following sequences: a P_{araBAD} promoter operably linked to a heterologous gene encoding an essential polypeptide not found in the recombinant bacterial cell (and required for survival), and a P_{araC} promoter operably linked to a heterologous gene encoding AraC transcription factor. In the presence of arabinose, the AraC transcription factor activates the P_{araBAD} promoter, which activates transcription of the heterologous gene encoding the essential polypeptide, allowing the recombinant bacterial cell to survive. In the absence of arabinose, however, AraC suppresses transcription from the P_{araBAD} promoter and the essential protein required for survival is not expressed. In this case, the recombinant bacterial cell dies in the absence of arabinose. In some embodiments, the sequence of P_{araBAD} promoter operably linked to a heterologous gene encoding an essential polypeptide not found in the recombinant bacterial cell can be present in the bacterial cell in conjunction with the TetR/toxin kill-switch system described directly above. In some embodiments, the sequence of P_{araBAD} promoter operably linked to a heterologous gene encoding an essential polypeptide not found in the recombinant bacterial cell can be present in the bacterial cell in conjunction with the TetR/toxin/anti-toxin kill-switch system described directly above.

In yet other embodiments, the bacteria may comprise a plasmid stability system with a plasmid that produces both a short-lived anti-toxin and a long-lived toxin. In this system, the bacterial cell produces equal amounts of toxin and anti-toxin to neutralize the toxin. However, if/when the cell loses the plasmid, the short-lived anti-toxin begins to decay. When the anti-toxin decays completely the cell dies as a result of the longer-lived toxin killing it.

In some embodiments, the engineered bacteria of the present disclosure, for example, bacteria expressing an amino acid catabolism enzyme further comprise the gene(s) encoding the components of any of the above-described kill-switch circuits.

In any of the above-described embodiments, the bacterial toxin is selected from the group consisting of a lysin, Hok, Fst, TisB, LdrD, Kid, SymE, MazF, FlmA, Ibs, XCV2162, dinJ, CcdB, MazF, ParE, YafO, Zeta, hicB, relB, yhaV, yoeB, chpBK, hipA, microcin B, microcin B17, microcin C, microcin C7-C51, microcin J25, microcin ColV, microcin 24, microcin L, microcin D93, microcin L, microcin E492, microcin H47, microcin I47, microcin M, colicin A, colicin E1, colicin K, colicin N, colicin U, colicin B, colicin Ia, colicin Ib, colicin 5, colicin10, colicin S4, colicin Y, colicin E2, colicin E7, colicin E8, colicin E9, colicin E3, colicin E4, colicin E6; colicin E5, colicin D, colicin M, and cloacin DF13, or a biologically active fragment thereof.

In any of the above-described embodiments, the anti-toxin is selected from the group consisting of an anti-lysin, Sok, RNAII, IstR, RdlD, Kis, SymR, MazE, FlmB, Sib, ptaRNA1, yafQ, CcdA, MazE, ParD, yafN, Epsilon, HicA, relE, prlF, yefM, chpBI, hipB, MccE, MccE^{CTD}, MccF, Cai, ImmE1, Cki, Cni, Cui, Cbi, Iia, Imm, Cfi, Im10, Csi, Cyi, Im2, Im7, Im8, Im9, Im3, Im4, ImmE6, cloacin immunity protein (Cim), ImmE5, ImmD, and Cmi, or a biologically active fragment thereof.

In one embodiment, the bacterial toxin is bactericidal to the genetically engineered bacterium. In one embodiment, the bacterial toxin is bacteriostatic to the genetically engineered bacterium.

In one embodiment, a second recombinant bacterial cell is administered to the subject, wherein the second recombinant bacterial cell comprises a heterologous reporter gene operably linked to an inducible promoter that is directly or indirectly induced by an exogenous environmental condition. In one embodiment, the heterologous reporter gene is a fluorescence gene. In one embodiment, the fluorescence gene encodes a green fluorescence protein (GFP). In another embodiment, a second recombinant bacterial cell is administered to the subject, wherein the second recombinant bacterial cell expresses a *lacZ* reporter construct that cleaves a substrate to produce a small molecule that can be detected in urine (see, for example, Danio et al., Science Translational Medicine, 7(289):1-12, 2015).

### Isolated Plasmids

In other embodiments, the disclosure provides an isolated plasmid comprising a first nucleic acid encoding a first payload operably linked to a first inducible promoter, and a second nucleic acid encoding a second payload operably linked to a second inducible promoter. In other embodiments, the disclosure provides an isolated plasmid further comprising a third nucleic acid encoding a third payload operably linked to a third inducible promoter. In other embodiments, the disclosure provides a plasmid comprising four, five, six, or more nucleic acids encoding four, five, six, or more payloads operably linked to inducible promoters. In any of the embodiments described here, the first, second, third, fourth, fifth, sixth, etc "payload(s)" can be a branched chain amino acid catabolism enzyme, a transporter of branched chain amino acids, a binding protein of branched chain amino acids, or other sequence described herein. In one embodiment, the nucleic acid encoding the first payload and the nucleic acid encoding the second payload are operably linked to the first inducible promoter. In one embodiment, the nucleic acid encoding the first payload is operably linked to a first inducible promoter and the nucleic acid encoding the second payload is operably linked to a second inducible promoter. In one embodiment, the first inducible promoter and the second inducible promoter are separate copies of the same inducible promoter. In another embodiment, the first inducible promoter and the second inducible promoter are different inducible promoters. In other embodiments comprising a third nucleic acid, the nucleic acid encoding the third payload and the nucleic acid encoding the first and second payloads are all operably linked to the same inducible promoter. In other embodiments, the nucleic acid encoding the first payload is operably linked to a first inducible promoter, the nucleic acid encoding the second payload is operably linked to a second inducible promoter, and the nucleic acid encoding te third payload is operably linked to a third inducible promoter. In some embodiments, the first, second, and third inducible promoters are separate copies of the same inducible promoter. In other embodiments, the first inducible promoter, the second inducible promoter, and the third inducible promoter are different inducible promoters. In some embodiments, the first promoter, the second promoter, and the optional third promoter, or the first promoter and the second promoter and the optional third promoter, are each directly or indirectly induced by low-oxygen or anaerobic conditions. In other embodiments, the first promoter, the second promoter, and the optional third promoter, or the first promoter and the second promoter and the optional third promoter, are each a fumarate and nitrate reduction regulator (FNR) responsive promoter. In other embodiments, the first promoter, the second promoter, and the optional third promoter, or the first promoter and the second promoter and the optional third promoter are each a ROS-inducible regulatory region. In other embodiments, the first promoter, the second promoter, and the optional third promoter, or the first promoter and the second promoter and the optional third promoter are each a RNS-inducible regulatory region.

In some embodiments, the heterologous gene encoding a branched chain amino acid catabolism enzyme is operably linked to a constitutive promoter. In one embodiment, the constitutive promoter is a lac promoter. In another embodiment, the constitutive promoter is a tet promoter. In another embodiment, the constitutive promoter is a constitutive *Escherichia coli* σ³² promoter. In another embodiment, the constitutive promoter is a constitutive *Escherichia coli* σ⁷⁰ promoter. In another embodiment, the constitutive promoter is a constitutive *Bacillus subtilis* σ^{A} promoter. In another embodiment, the constitutive promoter is a constitutive *Bacillus subtilis* σ^{B} promoter. In another embodiment, the constitutive promoter is a *Salmonella* promoter. In other embodiments, the constitutive promoter is a bacteriophage T7 promoter. In other embodiments, the constitutive promoter is and a bacteriophage SP6 promoter. In any of the above-described embodiments, the plasmid further comprises a heterologous gene encoding a transporter of a branched chain amino acid, a BCAA binding protein, and/or a kill switch construct, which may be operably linked to a constitutive promoter or an inducible promoter.

In some embodiments, the isolated plasmid comprises at least one heterologous branched chain amino acid catabolism enzyme gene operably linked to a first inducible promoter; a heterologous gene encoding a TetR protein operably linked to a P_{araBAD} promoter, a heterologous gene encoding AraC operably linked to a P_{araC} promoter, a heterologous gene encoding an antitoxin operably linked to a constitutive promoter, and a heterologous gene encoding a toxin operably linked to a P_{TetR} promoter. In another embodiment, the isolated plasmid comprises at least one heterologous gene encoding a branched chain amino acid catabolism enzyme operably linked to a first inducible promoter; a heterologous gene encoding a TetR protein and an anti-toxin operably linked to a P_{araBAD} promoter, a heterologous gene encoding AraC operably linked to a P_{araC} promoter, and a heterologous gene encoding a toxin operably linked to a P_{TetR} promoter.

In some embodiments, a first nucleic acid encoding a branched chain amino acid catabolism enzyme comprises a *kivD* gene. In other embodiments, a first nucleic acid encoding a branched chain amino acid catabolism enzyme is a *BCKD* gene or a *BCKD* operon. In some embodiments, the *kivD* gene or *BCKD* operon is coexpressed with an additional branched chain amino acid dehydrogenase, *e.g.,* a leucine dehydrogenase, *e.g., leuDH,* or a branched chain amino acid aminotransferase, *e.g., ilvE* or an amino acid oxidase, e.g., L-AAD. In other embodiments, a gene encoding an alcohol dehydrogenase, *e.g., adh2 or yqhD,* is further coexpressed. In other embodiments, a gene encoding an aldehyde dehydrogenase, *e.g., padA,* is further coexpressed.

In some embodiments, a second nucleic acid encoding a transporter of branched chain amino acids comprises a *livKHMGF* operon. In one embodiment, the *livKHMGF* operon is an *Escherichia coli* livKHMGF operon. In another embodiment, the *livKHMGF* operon has at least about 90% identity to the uppercase sequence of SEQ ID NO:5. In another embodiment, the *livKHMGF* operon comprises the uppercase sequence of SEQ ID NO:5. In another embodiment, the second nucleic acid encoding a transporter of branched chain amino acids comprises *brnQ* gene. In another embodiment, the *brnQ* gene has at least about 90% identity to the uppercase sequence of SEQ ID NO: 64. In another embodiment, the *brnQ* gene comprises the uppercase sequence of SEQ ID NO: 64.

In some embodiments, a third nucleic acid encoding a binding protein of branched chain amino acids comprises *livJ* gene. In another embodiment, the *livJ* gene has at least about 90% identity to the uppercase sequence of SEQ ID NO: 12. In another embodiment, the *livJ* gene comprises the uppercase sequence of SEQ ID NO: 12.

In one embodiment, the plasmid is a high-copy plasmid. In another embodiment, the plasmid is a low-copy plasmid.

In another aspect, the disclosure provides a recombinant bacterial cell comprising an isolated plasmid described herein. In another embodiment, the disclosure provides a pharmaceutical composition comprising the recombinant bacterial cell.

In one embodiment, the bacterial cell further comprises a genetic mutation in an endogenous gene encoding an exporter of a branched chain amino acid, wherein the genetic mutation reduces export of the branched chain amino acid from the bacterial cell. In one embodiment, the endogenous gene encoding an exporter of a branched chain amino acid is a *leuE* gene.

In one embodiment, the bacterial cell further comprises a genetic mutation in an endogenous gene encoding a branched chain amino acid biosynthesis gene, wherein the genetic mutation reduces biosynthesis of one or more branched chain amino acids in the bacterial cell. In one embodiment, the endogenous gene encoding a branched chain amino acid biosynthesis gene is an *ilvC* gene.

### Host-Plasmid Mutual Dependency

In some embodiments, the genetically engineered bacteria also comprise a plasmid that has been modified to create a host-plasmid mutual dependency. In certain embodiments, the mutually dependent host-plasmid platform is GeneGuard (Wright *et al*., 2015). In some embodiments, the GeneGuard plasmid comprises (i) a conditional origin of replication, in which the requisite replication initiator protein is provided *in trans;* (ii) an auxotrophic modification that is rescued by the host via genomic translocation and is also compatible for use in rich media; and/or (iii) a nucleic acid sequence which encodes a broad-spectrum toxin. The toxin gene may be used to select against plasmid spread by making the plasmid DNA itself disadvantageous for strains not expressing the anti-toxin (e.g., a wild-type bacterium). In some embodiments, the GeneGuard plasmid is stable for at least 100 generations without antibiotic selection. In some embodiments, the GeneGuard plasmid does not disrupt growth of the host. The GeneGuard plasmid is used to greatly reduce unintentional plasmid propagation in the genetically engineered bacteria described herein.

The mutually dependent host-plasmid platform may be used alone or in combination with other biosafety mechanisms, such as those described herein (*e.g.,* kill switches, auxotrophies). In some embodiments, the genetically engineered bacteria comprise a GeneGuard plasmid. In other embodiments, the genetically engineered bacteria comprise a GeneGuard plasmid and/or one or more kill switches. In other embodiments, the genetically engineered bacteria comprise a GeneGuard plasmid and/or one or more auxotrophies. In still other embodiments, the genetically engineered bacteria comprise a GeneGuard plasmid, one or more kill switches, and/or one or more auxotrophies.

In some embodiments, the vector comprises a conditional origin of replication. In some embodiments, the conditional origin of replication is a R6K or ColE2-P9. In embodiments where the plasmid comprises the conditional origin of replication R6K, the host cell expresses the replication initiator protein π. In embodiments where the plasmid comprises the conditional origin or replication ColE2, the host cell expresses the replication initiator protein RepA. It is understood by those of skill in the art that the expression of the replication initiator protein may be regulated so that a desired expression level of the protein is achieved in the host cell to thereby control the replication of the plasmid. For example, in some embodiments, the expression of the gene encoding the replication initiator protein may be placed under the control of a strong, moderate, or weak promoter to regulate the expression of the protein.

In some embodiments, the vector comprises a gene encoding a protein required for complementation of a host cell auxotrophy, preferably a rich-media compatible auxotrophy. In some embodiments, the host cell is auxotrophic for thymidine (Δ*thyA*), and the vector comprises the thymidylate synthase (*thyA*) gene. In some embodiments, the host cell is auxotrophic for diaminopimelic acid (Δ*dapA*) and the vector comprises the 4-hydroxy-tetrahydrodipicolinate synthase (*dapA*) gene. It is understood by those of skill in the art that the expression of the gene encoding a protein required for complementation of the host cell auxotrophy may be regulated so that a desired expression level of the protein is achieved in the host cell.

In some embodiments, the vector comprises a toxin gene. In some embodiments, the host cell comprises an anti-toxin gene encoding and/or required for the expression of an anti-toxin. In some embodiments, the toxin is Zeta and the anti-toxin is Epsilon. In some embodiments, the toxin is Kid, and the anti-toxin is Kis. In preferred embodiments, the toxin is bacteriostatic. Any of the toxin/antitoxin pairs described herein may be used in the vector systems of the present disclosure. It is understood by those of skill in the art that the expression of the gene encoding the toxin may be regulated using art known methods to prevent the expression levels of the toxin from being deleterious to a host cell that expresses the anti-toxin. For example, in some embodiments, the gene encoding the toxin may be regulated by a moderate promoter. In other embodiments, the gene encoding the toxin may be cloned adjacent to ribosomal binding site of interest to regulate the expression of the gene at desired levels (see, *e.g.,* Wright *et al.* (2015)).

### Integration

In some embodiments, any of the gene(s) or gene cassette(s) of the present disclosure may be integrated into the bacterial chromosome at one or more integration sites. One or more copies of the gene (for example, an amino acid catabolism gene, BCAA transporter gene, and/or BCAA binding protein gene) or gene cassette (for example, a gene cassette comprising an amino acid catabolism gene, an amino acid transporter gene, a BCAA binding protein gene) may be integrated into the bacterial chromosome. Having multiple copies of the gene or gene cassette integrated into the chromosome allows for greater production of the payload, e.g., amino acid catabolism enzyme, BCAA transporter gene, and/or BCAA binding protein gene and other enzymes of a gene cassette, and also permits fine-tuning of the level of expression. Alternatively, different circuits described herein, such as any of the kill-switch circuits, in addition to the therapeutic gene(s) or gene cassette(s) could be integrated into the bacterial chromosome at one or more different integration sites to perform multiple different functions.

For example, **Fig. 66** depicts a map of integration sites within the *E. coli* Nissle chromosome. **Fig. 66** depicts three bacterial strains wherein the RFP gene has been successfully integrated into the bacterial chromosome at an integration site.

### Secretion

In some embodiments, the genetically engineered bacteria further comprise a native secretion mechanism (e.g., gram positive bacteria) or non-native secretion mechanism (e.g., gram negative bacteria) that is capable of secreting the branched chain amino acid catabolism enzymefrom the bacterial cytoplasm. Many bacteria have evolved sophisticated secretion systems to transport substrates across the bacterial cell envelope. Substrates, such as small molecules, proteins, and DNA, may be released into the extracellular space or periplasm (such as the gut lumen or other space), injected into a target cell, or associated with the bacterial membrane.

In Gram-negative bacteria, secretion machineries may span one or both of the inner and outer membranes. In some embodiments, the genetically engineered bacteria further comprise a non-native double membrane-spanning secretion system. Double membrane-spanning secretion systems include, but are not limited to, the type I secretion system (T1SS), the type II secretion system (T2SS), the type III secretion system (T3SS), the type IV secretion system (T4SS), the type VI secretion system (T6SS), and the resistance-nodulation-division (RND) family of multi-drug efflux pumps (Pugsley 1993; Gerlach et al., 2007; Collinson et al., 2015; Costa et al., 2015; Reeves et al., 2015; WO2014138324A1).

Examples of such secretion systems are shown in Figures 3-6. Mycobacteria, which have a Gram-negative-like cell envelope, may also encode a type VII secretion system (T7SS) (Stanley et al., 2003). With the exception of the T2SS, double membrane-spanning secretions generally transport substrates from the bacterial cytoplasm directly into the extracellular space or into the target cell. In contrast, the T2SS and secretion systems that span only the outer membrane may use a two-step mechanism, wherein substrates are first translocated to the periplasm by inner membrane-spanning transporters, and then transferred to the outer membrane or secreted into the extracellular space. Outer membrane-spanning secretion systems include, but are not limited to, the type V secretion or autotransporter system (TSSS), the curli secretion system, and the chaperone-usher pathway for pili assembly (Saier, 2006; Costa et al., 2015).

In some embodiments, the genetically engineered bacteria of the invention further comprise a type III or a type III-like secretion system (T3SS) from Shigella, Salmonella, E. coli, Bivrio, Burkholderia, Yersinia, Chlamydia, or Pseudomonas. The T3SS is capable of transporting a protein from the bacterial cytoplasm to the host cytoplasm through a needle complex. The T3SS may be modified to secrete the molecule from the bacterial cytoplasm, but not inject the molecule into the host cytoplasm. Thus, the molecule is secreted into the gut lumen or other extracellular space. In some embodiments, the genetically engineered bacteria comprise said modified T3SS and are capable of secreting the branched chain amino acid catabolism enzymefrom the bacterial cytoplasm. In some embodiments, the secreted molecule, such as a heterologouse protein or peptide, e.g., a branched chain amino acid catabolism enzyme, comprises a type III secretion sequence that allows the branched chain amino acid catabolism enzymeto be secreted from the bacteria.

In some embodiments, a flagellar type III secretion pathway is used to secrete the molecule of interest, e.g., a branched chain amino acid catabolism enzyme. In some embodiments, an incomplete flagellum is used to secrete a therapeutic peptide of interest by recombinantly fusing the peptide to an N-terminal flagellar secretion signal of a native flagellar component. In this manner, the intracellularly expressed chimeric peptide can be mobilized across the inner and outer membranes into the surrounding host environment.

In some embodiments, a Type V Autotransporter Secretion System is used to secrete the molecule of interest, e.g., therapeutic peptide. Due to the simplicity of the machinery and capacity to handle relatively large protein fluxes, the Type V secretion system is attractive for the extracellular production of recombinant proteins. As shown in Figure 10, a therapeutic peptide (star) can be fused to an N-terminal secretion signal, a linker, and the beta-domain of an autotransporter. The N-terminal signal sequence directs the protein to the SecA-YEG machinery which moves the protein across the inner membrane into the periplasm, followed by subsequent cleavage of the signal sequence. The Beta-domain is recruited to the Bam complex (`Beta-barrel assembly machinery') where the beta-domain is folded and inserted into the outer membrane as a beta-barrel structure. The therapeutic peptide is thread through the hollow pore of the beta-barrel structure ahead of the linker sequence. Once exposed to the extracellular environment, the therapeutic peptide can be freed from the linker system by an autocatalytic cleavage (left side of Bam complex) or by targeting of a membrane-associated peptidase (black scissors; right side of Bam complex) to a complimentary protease cut site in the linker. Thus, in some embodiments, the secreted molecule, such as a heterologouse protein or peptide, e.g., a branched chain amino acid catabolism enzyme, comprises an N-terminal secretion signal, a linker, and beta-domain of an autotransporter so as to allow the molecule to be secreted from the bacteria.

In some embodiments, a Hemolysin-based Secretion System is used to secrete the molecule of interest, e.g., therapeutic peptide. Type I Secretion systems offer the advantage of translocating their passenger peptide directly from the cytoplasm to the extracellular space, obviating the two-step process of other secretion types. Figure 11 shows the alpha-hemolysin (HlyA) of uropathogenic Escherichia coli. This pathway uses HlyB, an ATP-binding cassette transporter; HlyD, a membrane fusion protein; and TolC , an outer membrane protein. The assembly of these three proteins forms a channel through both the inner and outer membranes. Natively, this channel is used to secrete HlyA, however, to secrete the therapeutic peptide of the present disclosure, the secretion signal-containing C-terminal portion of HlyA is fused to the C-terminal portion of a therapeutic peptide (star) to mediate secretion of this peptide.

In alternate embodiments, the genetically engineered bacteria further comprise a non-native single membrane-spanning secretion system. Single membrane-spanning transporters may act as a component of a secretion system, or may export substrates independently. Such transporters include, but are not limited to, ATP-binding cassette translocases, flagellum/virulence-related translocases, conjugation-related translocases, the general secretory system (e.g., the SecYEG complex in E. coli), the accessory secretory system in mycobacteria and several types of Gram-positive bacteria (e.g., Bacillus anthracis, Lactobacillus johnsonii, Corynebacterium glutamicum, Streptococcus gordonii, Staphylococcus aureus), and the twin-arginine translocation (TAT) system (Saier, 2006; Rigel and Braunstein, 2008; Albiniak et al., 2013). It is known that the general secretory and TAT systems can both export substrates with cleavable N-terminal signal peptides into the periplasm, and have been explored in the context of biopharmaceutical production. The TAT system may offer particular advantages, however, in that it is able to transport folded substrates, thus eliminating the potential for premature or incorrect folding. In certain embodiments, the genetically engineered bacteria comprise a TAT or a TAT-like system and are capable of secreting the branched chain amino acid catabolism enzymefrom the bacterial cytoplasm. One of ordinary skill in the art would appreciate that the secretion systems disclosed herein may be modified to act in different species, strains, and subtypes of bacteria, and/or adapted to deliver different payloads.

In order to translocate a protein, e.g., therapeutic polypeptide, to the extracellular space, the polypeptide must first be translated intracellularly, mobilized across the inner membrane and finally mobilized across the outer membrane. Many effector proteins (e.g., therapeutic polypeptides) - particularly those of eukaryotic origin - contain disulphide bonds to stabilize the tertiary and quaternary structures. While these bonds are capable of correctly forming in the oxidizing periplasmic compartment with the help of periplasmic chaperones, in order to translocate the polypeptide across the outer membrane the disulphide bonds must be reduced and the protein unfolded again.

One way to secrete properly folded proteins in gram-negative bacteria-particularly those requiring disulphide bonds - is to target the periplasm in a bacteria with a destabilized outer membrane. In this manner the protein is mobilized into the oxidizing environment and allowed to fold properly. In contrast to orchestrated extracellular secretion systems, the protein is then able to escape the periplasmic space in a correctly folded form by membrane leakage. These "leaky" gram-negative mutants are therefore capable of secreting bioactive, properly disulphide-bonded polypeptides. In some embodiments, the genetically engineered bacteria have a "leaky" or de-stabilized outer membrane. Destabilizing the bacterial outer membrane to induce leakiness can be accomplished by deleting or mutagenizing genes responsible for tethering the outer membrane to the rigid peptidoglycan skeleton, including for example, lpp, ompC, ompA, ompF, tolA, tolB, pal, degS, degP, and nlpl. Lpp is the most abundant polypeptide in the bacterial cell existing at ~500,000 copies per cell and functions as the primary 'staple' of the bacterial cell wall to the peptidoglycan. 1.

Silhavy, T. J., Kahne, D. & Walker, S. The bacterial cell envelope. Cold Spring Harb Perspect Biol 2, a000414 (2010). TolA-PAL and OmpA complexes function similarly to Lpp and are other deletion targets to generate a leaky phenotype. Additionally, leaky phenotypes have been observed when periplasmic proteases are deactived. The periplasm is very densely packed with protein and therefore encode several periplasmic proteins to facilitate protein turnover. Removal of periplasmic proteases such as *degS, degP* or *nlpI* can induce leaky phenotypes by promoting an excessive build-up of periplasmic protein. Mutation of the proteases can also preserve the effector polypeptide by preventing targeted degradation by these proteases. Moreover, a combination of these mutations may synergistically enhance the leaky phenotype of the cell without major sacrifices in cell viability. Thus, in some embodiments, the engineered bacteria have one or more deleted or mutated membrane genes. In some embodiments, the engineered bacteria have a deleted or mutated lpp gene. In some embodiments, the engineered bacteria have one or more deleted or mutated gene(s), selected from ompA, ompA, and ompF genes. In some embodiments, the engineered bacteria have one or more deleted or mutated gene(s), selected from tolA, tolB, and pal genes. in some embodiments, the engineered bacteria have one or more deleted or mutated periplasmic protease genes. In some embodiments, the engineered bacteria have one or more deleted or mutated periplasmic protease genes selected from degS, degP, and nlpl. In some embodiments, the engineered bacteria have one or more deleted or mutated gene(s), selected from lpp, ompA, ompA, ompF, tolA, tolB, pal, degS, degP, and nlpl genes.

To minimize disturbances to cell viability, the leaky phenotype can be made inducible by placing one or more membrane or periplasmic protease genes, e.g., selected from lpp, ompA, ompA, ompF, tolA, tolB, pal, degS, degP, and nlpl, under the control of an inducible promoterFor example, expression of *lpp* or other cell wall stability protein or periplasmic protease can be repressed in conditions where the therapeutic polypeptide needs to be delivered (secreted). For instance, under inducing conditions a transcriptional repressor protein or a designed antisense RNA can be expressed which reduces transcription or translation of a target membrane or periplasmic protease gene. Conversely, overexpression of certain peptides can result in a destabilized phenotype, e.g., ove expression of colicins or the third topological domain of TolA, which peptide overexpression can be induced in conditions in which the therapeutic polypeptide needs to be delivered (secreted). These sorts of strategies would decouple the fragile, leaky phenotypes from biomass production. Thus, in some embodiments, the engineered bacteria have one or more membrane and/or periplasmic protease genes under the control of an inducible promoter.

### Table 11: The tables below lists secretion systems for Gram positive bacteria and Gram negative bacteria.

**Table 11. Secretion systems for gram positive bacteria**

| Bacterial Strain | Relevant Secretion System |
|---|---|
| C. *novyi-NT (Gram*+*)* | Sec pathway |
| | Twin- arginine (TAT) pathway |
| C. *butryicum (Gram*+*)* | Sec pathway |
| | Twin- arginine (TAT) pathway |
| *Listeria monocytogenes (Gram* +*)* | Sec pathway |
| | Twin- arginine (TAT) pathway |

**Table 12. Secretion Systems for Gram negative bacteria**

| **Protein secretary pathways (SP) in gram-negative bacteria and their descendants** | | | | | | | |
|---|---|---|---|---|---|---|---|
| Type (Abbreviat ion) | Name | TC#² | Bact eria | Arch aea | Eukarya | # Protei ns/S ys tem | Energ y Sourc e |
| **IMPS** - **Gram-negative bacterial inner membrane channel-forming translocases** | | | | | | | |
| ABC (SIP) | ATP binding cassette translocase | 3.A.1 | + | + | + | 3-4 | ATP |
| SEC (IISP) | General secretory translocase | 3.A.5 | + | + | + | ~12 | GTP OR ATP + PMF |
| Fla/Path (IIISP) | Flagellum/vir ulence-related translocase | 3.A.6 | + | - | - | >10 | ATP |
| Conj (IVSP) | Conjugation-related translocase | 3.A.7 | + | - | - | >10 | ATP |
| Tat (IISP) | Twin-arginine targeting translocase | 2.A.6 4 | + | + | + (chloroplas ts) | 2-4 | PMF |
| Oxa1 (YidC) | Cytochrome oxidase biogenesis family | 2.A.9 | + | + | + (mitochon dria chloroplast s) | 1 | None or PMF |
| MscL | Large conductance mechanosens | 1.A.2 2 | + | + | + | 1 | None |
| | itive channel family | | | | | | |
| Holins | Holin functional superfamily | 1.E.1 •21 | + | - | - | 1 | None |

| **Eukaryotic Organelles** | | | | | | | |
|---|---|---|---|---|---|---|---|
| MPT | Mitochondria 1 protein translocase | 3.A. B | - | - | + (mitochon drial) | >20 | ATP |
| CEPT | Chloroplast envelope protein translocase | 3.A.9 | (+) | - | + (chloroplas ts) | ≥3 | GTP |
| Bcl-2 | Eukaryotic Bcl-2 family (programmed cell death) | 1.A.2 1 | - | - | + | 1? | None |

| **Gram-negative bacterial outer membrane channel-forming translocases** | | | | | | | |
|---|---|---|---|---|---|---|---|
| MTB (IISP) | Main terminal branch of the general secretory translocase | 3.A.1 5 | +^{b} | - | - | ~14 | ATP; PMF |
| FUP AT-1 | Fimbrial usher protein | 1.B.1 1 | +^{b} | - | - | 1 | None |
| | | | +^{b} | | - | 1 | None |
| | Autotransport er-1 | 1.B.1 2 | | | | | |
| AT-2 | Autotransport er-2 | 1.B.4 0 | +^{b} | - | - | 1 | None |
| OMF | | | +^{b} | | +(?) | 1 | None |
| (ISP) | | 1.B.1 7 | | | | | |
| TPS Secretin (IISP and IISP) | | 1.B.2 0 | + | - | + | 1 | None |
| | | | +^{b} | | - | 1 | None |
| | | 1.B.2 2 | | | | | |
| OmpIP | Outer membrane insertion porin | 1.B.3 3 | + | - | + (mitochon dria; chloroplast s) | ≥4 | None |
| | | | | | | | ? |

The above tables for gram positive and gram negative bacteria list secretion systems that can be used to secrete polypeptides and other branched chain amino acid catabolism enzyme from the engineered bacteria, which are reviewed in Milton H. Saier, Jr. Microbe / Volume 1, Number 9, 2006 "Protein Secretion Systems in Gram-Negative Bacteria Gram-negative bacteria possess many protein secretion-membrane insertion systems that apparently evolved independently" .

In some embodiments, one or more BCAA catabolic enzymes described herein are secreted. In some embodiments, the one or more BCAA catabolic enzymes described herein are further modified to improve secretion efficiency, decreased susceptibility to proteases, stability, and/or half-life. In some embodiments, leucine dehydrogenase is secreted, alone or in combination other BCAA catabolic enzymes, e.g., with a ketoacid decarboxylase and/or an alcohol dehydrogenase. In some embodiments, leucine dehydrogenase is secreted, alone or in combination other BCAA catabolic enzymes, e.g., with a ketoacid decarboxylase and/or an aldehyde dehydrogenase. In some embodiments, BCAA aminotransferase is secreted, alone or in combination other BCAA catabolic enzymes, e.g., with a ketoacid decarboxylase and/or an alcohol dehydrogenase dehydrogenase. In some embodiments, BCAA aminotransferase is secreted, alone or in combination other BCAA catabolic enzymes, e.g., with a ketoacid decarboxylase and/or an aldehyde dehydrogenase. In some embodiments, amino acid oxidase is secreted, alone or in combination other BCAA catabolic enzymes, e.g., with a ketoacid decarboxylase and/or an alcohol dehydrogenase dehydrogenase. In some embodiments, amino acid oxidase is secreted, alone or in combination other BCAA catabolic enzymes, e.g., with a ketoacid decarboxylase and/or an aldehyde dehydrogenase. In some embodiments, a ketoacid carboxylase is secreted, alone or in combination with other BCAA catabolic enzymes. In some embodiments, an alcohol dehydrogenase is secreted, alone or in combination with other BCAA catabolic enzymes. In some embodiments, an aldehyde dehydrogenase is secreted, alone or in combination with other BCAA catabolic enzymes.

In some embodiments, leucine dehydrogenase is secreted, alone or in combination other BCAA catabolic enzymes, e.g., with one or more Bkd complex enzymes, and/or one or more Liu operon enzymes. In some embodiments, leucine dehydrogenase is secreted, alone or in combination other BCAA catabolic enzymes, e.g., with one or more Bkd complex enzymes.

In some embodiments, BCAA aminotransferase is secreted, alone or in combination other BCAA catabolic enzymes, e.g., with one or more Bkd complex enzymes, and/or one or more Liu operon enzymes. In some embodiments, BCAA aminotransferase is secreted, alone or in combination other BCAA catabolic enzymes, e.g., with one or more Bkd complex enzymes. In some embodiments, amino acid oxidase is secreted, alone or in combination other BCAA catabolic enzymes, e.g., with one or more Bkd complex enzymes, and/or one or more Liu operon enzymes. In some embodiments, amino acid oxidase is secreted, alone or in combination other BCAA catabolic enzymes, e.g., with one or more Bkd complex enzymes.
In some embodiments, one or more enzymes from the Bkd complex are secreted, alone ore in combination with one or more BCAA catabolic enzymes. In some embodiments, one or more enzymes from the Bkd complex are secreted, alone ore in combination with one or more Liu operon enzymes. In some embodiments, Lbul is secreted, alone or in combination with one or more BCAA catabolic enzymes.
In some embodiments, combinations of two or more of the enzymes and/or enzyme complexes described herein may be secreted. Any of the enzymes expressed by the genes described e.g., in Fig 13A and 13B may be combined. Alternatively, any of the enzymes expressed by the genes described, e.g., or Fig 13D and/or E may be combined.

### Pharmaceutical Compositions and Formulations

Pharmaceutical compositions comprising the genetically engineered microorganisms of the invention may be used to treat, manage, ameliorate, and/or prevent a disorder associated with branched chain amino acid catabolism or symptom(s) associated with diseases or disorders associated with branched chain amino acid catabolism. Pharmaceutical compositions of the invention comprising one or more genetically engineered bacteria, and/or one or more genetically engineered virus, alone or in combination with prophylactic agents, therapeutic agents, and/or pharmaceutically acceptable carriers are provided.

In certain embodiments, the pharmaceutical composition comprises one species, strain, or subtype of bacteria that are engineered to comprise one or more of the genetic modifications described herein, *e.g.,* selected from expression of at least one branched chain amino acid catabolism enzyme, BCAA transporter, BCAA binding protein, auxotrophy, kill-switch, exporter knock-out, etc. In alternate embodiments, the pharmaceutical composition comprises two or more species, strains, and/or subtypes of bacteria that are each engineered to comprise the genetic modifications described herein, *e.g.,* selected from expression of at least one branched chain amino acid catabolism enzyme, BCAA transporter, BCAA binding protein, auxotrophy, kill-switch, exporter knock-out, etc.

The pharmaceutical compositions of the invention described herein may be formulated in a conventional manner using one or more physiologically acceptable carriers comprising excipients and auxiliaries, which facilitate processing of the active ingredients into compositions for pharmaceutical use. Methods of formulating pharmaceutical compositions are known in the art (*see, e.g.,* "Remington's Pharmaceutical Sciences," Mack Publishing Co., Easton, PA). In some embodiments, the pharmaceutical compositions are subjected to tabletting, lyophilizing, direct compression, conventional mixing, dissolving, granulating, levigating, emulsifying, encapsulating, entrapping, or spray drying to form tablets, granulates, nanoparticles, nanocapsules, microcapsules, microtablets, pellets, or powders, which may be enterically coated or uncoated. Appropriate formulation depends on the route of administration.

The genetically engineered microorganisms may be formulated into pharmaceutical compositions in any suitable dosage form (*e.g*., liquids, capsules, sachet, hard capsules, soft capsules, tablets, enteric coated tablets, suspension powders, granules, or matrix sustained release formations for oral administration) and for any suitable type of administration (*e.g.*, oral, topical, injectable, intravenous, sub-cutaneous, immediate-release, pulsatile-release, delayed-release, or sustained release). Suitable dosage amounts for the genetically engineered bacteria may range from about 104 to 1012 bacteria. The composition may be administered once or more daily, weekly, or monthly. The composition may be administered before, during, or following a meal. In one embodiment, the pharmaceutical composition is administered before the subject eats a meal. In one embodiment, the pharmaceutical composition is administered currently with a meal. In on embodiment, the pharmaceutical composition is administered after the subject eats a meal

The genetically engineered bacteria or genetically engineered virus may be formulated into pharmaceutical compositions comprising one or more pharmaceutically acceptable carriers, thickeners, diluents, buffers, buffering agents, surface active agents, neutral or cationic lipids, lipid complexes, liposomes, penetration enhancers, carrier compounds, and other pharmaceutically acceptable carriers or agents. For example, the pharmaceutical composition may include, but is not limited to, the addition of calcium bicarbonate, sodium bicarbonate, calcium phosphate, various sugars and types of starch, cellulose derivatives, gelatin, vegetable oils, polyethylene glycols, and surfactants, including, for example, polysorbate 20. In some embodiments, the genetically engineered bacteria of the invention may be formulated in a solution of sodium bicarbonate, e.g., 1 molar solution of sodium bicarbonate (to buffer an acidic cellular environment, such as the stomach, for example). The genetically engineered bacteria may be administered and formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The genetically engineered microorganisms may be administered intravenously, e.g., by infusion or injection.

The genetically engineered microroganisms of the disclosure may be administered intrathecally. In some embodiments, the genetically engineered microorganisms of the invention may be administered orally. The genetically engineered microorganisms disclosed herein may be administered topically and formulated in the form of an ointment, cream, transdermal patch, lotion, gel, shampoo, spray, aerosol, solution, emulsion, or other form well known to one of skill in the art. See, e.g., "Remington's Pharmaceutical Sciences," Mack Publishing Co., Easton, PA. In an embodiment, for non-sprayable topical dosage forms, viscous to semi-solid or solid forms comprising a carrier or one or more excipients compatible with topical application and having a dynamic viscosity greater than water are employed. Suitable formulations include, but are not limited to, solutions, suspensions, emulsions, creams, ointments, powders, liniments, salves, etc., which may be sterilized or mixed with auxiliary agents (e.g., preservatives, stabilizers, wetting agents, buffers, or salts) for influencing various properties, e.g., osmotic pressure. Other suitable topical dosage forms include sprayable aerosol preparations wherein the active ingredient in combination with a solid or liquid inert carrier, is packaged in a mixture with a pressurized volatile (e.g., a gaseous propellant, such as freon) or in a squeeze bottle. Moisturizers or humectants can also be added to pharmaceutical compositions and dosage forms. Examples of such additional ingredients are well known in the art. In one embodiment, the pharmaceutical composition comprising the recombinant bacteria of the invention may be formulated as a hygiene product. For example, the hygiene product may be an antibacterial formulation, or a fermentation product such as a fermentation broth. Hygiene products may be, for example, shampoos, conditioners, creams, pastes, lotions, and lip balms.

The genetically engineered microorganisms disclosed herein may be administered orally and formulated as tablets, pills, dragees, capsules, liquids, gels, syrups, slurries, suspensions, etc. Pharmacological compositions for oral use can be made using a solid excipient, optionally grinding the resulting mixture, and processing the mixture of granules, after adding suitable auxiliaries if desired, to obtain tablets or dragee cores. Suitable excipients include, but are not limited to, fillers such as sugars, including lactose, sucrose, mannitol, or sorbitol; cellulose compositions such as maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP) or polyethylene glycol (PEG). Disintegrating agents may also be added, such as cross-linked polyvinylpyrrolidone, agar, alginic acid or a salt thereof such as sodium alginate.

Tablets or capsules can be prepared by conventional means with pharmaceutically acceptable excipients such as binding agents (e.g., pregelatinised maize starch, polyvinylpyrrolidone, hydroxypropyl methylcellulose, carboxymethylcellulose, polyethylene glycol, sucrose, glucose, sorbitol, starch, gum, kaolin, and tragacanth); fillers (e.g., lactose, microcrystalline cellulose, or calcium hydrogen phosphate); lubricants (e.g., calcium, aluminum, zinc, stearic acid, polyethylene glycol, sodium lauryl sulfate, starch, sodium benzoate, L-leucine, magnesium stearate, talc, or silica); disintegrants (e.g., starch, potato starch, sodium starch glycolate, sugars, cellulose derivatives, silica powders); or wetting agents (e.g., sodium lauryl sulphate). The tablets may be coated by methods well known in the art. A coating shell may be present, and common membranes include, but are not limited to, polylactide, polyglycolic acid, polyanhydride, other biodegradable polymers, alginate-polylysine-alginate (APA), alginate-polymethylene-co-guanidine-alginate (A-PMCG-A), hydroymethylacrylate-methyl methacrylate (HEMA-MMA), multilayered HEMA-MMA-MAA, polyacrylonitrilevinylchloride (PAN-PVC), acrylonitrile/sodium methallylsulfonate (AN-69), polyethylene glycol/poly pentamethylcyclopentasiloxane/polydimethylsiloxane (PEG/PDS/PDMS), poly N,N-dimethyl acrylamide (PDMAAm), siliceous encapsulates, cellulose sulphate/sodium alginate/polymethylene-co-guanidine (CS/A/PMCG), cellulose acetate phthalate, calcium alginate, k-carrageenan-locust bean gum gel beads, gellan-xanthan beads, poly(lactide-co-glycolides), carrageenan, starch poly-anhydrides, starch polymethacrylates, polyamino acids, and enteric coating polymers.

In some embodiments, the genetically engineered microorganisms are enterically coated for release into the gut or a particular region of the gut, for example, the large intestine. The typical pH profile from the stomach to the colon is about 1-4 (stomach), 5.5-6 (duodenum), 7.3-8.0 (ileum), and 5.5-6.5 (colon). In some diseases, the pH profile may be modified. In some embodiments, the coating is degraded in specific pH environments in order to specify the site of release. In some embodiments, at least two coatings are used. In some embodiments, the outside coating and the inside coating are degraded at different pH levels.

Liquid preparations for oral administration may take the form of solutions, syrups, suspensions, or a dry product for constitution with water or other suitable vehicle before use. Such liquid preparations may be prepared by conventional means with pharmaceutically acceptable agents such as suspending agents (e.g., sorbitol syrup, cellulose derivatives, or hydrogenated edible fats); emulsifying agents (e.g., lecithin or acacia); nonaqueous vehicles (e.g., almond oil, oily esters, ethyl alcohol, or fractionated vegetable oils); and preservatives (e.g., methyl or propyl-p-hydroxybenzoates or sorbic acid). The preparations may also contain buffer salts, flavoring, coloring, and sweetening agents as appropriate. Preparations for oral administration may be suitably formulated for slow release, controlled release, or sustained release of the genetically engineered microorganisms described herein.

In one embodiment, the genetically engineered microorganisms of the disclosure may be formulated in a composition suitable for administration to pediatric subjects. As is well known in the art, children differ from adults in many aspects, including different rates of gastric emptying, pH, gastrointestinal permeability, etc. (Ivanovska et al., Pediatrics, 134(2):361-372, 2014). Moreover, pediatric formulation acceptability and preferences, such as route of administration and taste attributes, are critical for achieving acceptable pediatric compliance. Thus, in one embodiment, the composition suitable for administration to pediatric subjects may include easy-to-swallow or dissolvable dosage forms, or more palatable compositions, such as compositions with added flavors, sweeteners, or taste blockers. In one embodiment, a composition suitable for administration to pediatric subjects may also be suitable for administration to adults.

In one embodiment, the composition suitable for administration to pediatric subjects may include a solution, syrup, suspension, elixir, powder for reconstitution as suspension or solution, dispersible/effervescent tablet, chewable tablet, gummy candy, lollipop, freezer pop, troche, chewing gum, oral thin strip, orally disintegrating tablet, sachet, soft gelatin capsule, sprinkle oral powder, or granules. In one embodiment, the composition is a gummy candy, which is made from a gelatin base, giving the candy elasticity, desired chewy consistency, and longer shelf-life. In some embodiments, the gummy candy may also comprise sweeteners or flavors.

In one embodiment, the composition suitable for administration to pediatric subjects may include a flavor. As used herein, "flavor" is a substance (liquid or solid) that provides a distinct taste and aroma to the formulation. Flavors also help to improve the palatability of the formulation. Flavors include, but are not limited to, strawberry, vanilla, lemon, grape, bubble gum, and cherry.

In certain embodiments, the genetically engineered microorganisms may be orally administered, for example, with an inert diluent or an assimilable edible carrier. The compound may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound by other than parenteral administration, it may be necessary to coat the compound with, or co-administer the compound with, a material to prevent its inactivation.

In another embodiment, the pharmaceutical composition comprising the recombinant bacteria of the invention may be a comestible product, for example, a food product. In one embodiment, the food product is milk, concentrated milk, fermented milk (yogurt, sour milk, frozen yogurt, lactic acid bacteria-fermented beverages), milk powder, ice cream, cream cheeses, dry cheeses, soybean milk, fermented soybean milk, vegetable-fruit juices, fruit juices, sports drinks, confectionery, candies, infant foods (such as infant cakes), nutritional food products, animal feeds, or dietary supplements. In one embodiment, the food product is a fermented food, such as a fermented dairy product. In one embodiment, the fermented dairy product is yogurt. In another embodiment, the fermented dairy product is cheese, milk, cream, ice cream, milk shake, or kefir. In another embodiment, the recombinant bacteria of the invention are combined in a preparation containing other live bacterial cells intended to serve as probiotics. In another embodiment, the food product is a beverage. In one embodiment, the beverage is a fruit juice-based beverage or a beverage containing plant or herbal extracts. In another embodiment, the food product is a jelly or a pudding. Other food products suitable for administration of the recombinant bacteria of the invention are well known in the art. For example, see U.S. 2015/0359894 and US 2015/0238545.

In yet another embodiment, the pharmaceutical composition of the invention is injected into, sprayed onto, or sprinkled onto a food product, such as bread, yogurt, or cheese.

In some embodiments, the composition is formulated for intraintestinal administration, intrajejunal administration, intraduodenal administration, intraileal administration, gastric shunt administration, or intracolic administration, via nanoparticles, nanocapsules, microcapsules, or microtablets, which are enterically coated or uncoated. The pharmaceutical compositions may also be formulated in rectal compositions such as suppositories or retention enemas, using, e.g., conventional suppository bases such as cocoa butter or other glycerides. The compositions may be suspensions, solutions, or emulsions in oily or aqueous vehicles, and may contain suspending, stabilizing and/or dispersing agents.

The genetically engineered microorganisms described herein may be administered intranasally, formulated in an aerosol form, spray, mist, or in the form of drops, and conveniently delivered in the form of an aerosol spray presentation from pressurized packs or a nebuliser, with the use of a suitable propellant (e.g., dichlorodifluoromethane, trichlorofluoromethane, dichlorotetrafluoroethane, carbon dioxide or other suitable gas). Pressurized aerosol dosage units may be determined by providing a valve to deliver a metered amount. Capsules and cartridges (e.g., of gelatin) for use in an inhaler or insufflator may be formulated containing a powder mix of the compound and a suitable powder base such as lactose or starch.

The genetically engineered microorganisms may be administered and formulated as depot preparations. Such long acting formulations may be administered by implantation or by injection, including intravenous injection, subcutaneous injection, local injection, direct injection, or infusion. For example, the compositions may be formulated with suitable polymeric or hydrophobic materials (e.g., as an emulsion in an acceptable oil) or ion exchange resins, or as sparingly soluble derivatives (e.g., as a sparingly soluble salt).

In some embodiments, disclosed herein are pharmaceutically acceptable compositions in single dosage forms. Single dosage forms may be in a liquid or a solid form. Single dosage forms may be administered directly to a patient without modification or may be diluted or reconstituted prior to administration. In certain embodiments, a single dosage form may be administered in bolus form, e.g., single injection, single oral dose, including an oral dose that comprises multiple tablets, capsule, pills, etc. In alternate embodiments, a single dosage form may be administered over a period of time, e.g., by infusion.

Single dosage forms of the pharmaceutical composition may be prepared by portioning the pharmaceutical composition into smaller aliquots, single dose containers, single dose liquid forms, or single dose solid forms, such as tablets, granulates, nanoparticles, nanocapsules, microcapsules, microtablets, pellets, or powders, which may be enterically coated or uncoated. A single dose in a solid form may be reconstituted by adding liquid, typically sterile water or saline solution, prior to administration to a patient.

In other embodiments, the composition can be delivered in a controlled release or sustained release system. In one embodiment, a pump may be used to achieve controlled or sustained release. In another embodiment, polymeric materials can be used to achieve controlled or sustained release of the therapies of the present disclosure (see e.g., U.S. Patent No. 5,989,463). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N- vinyl pyrrolidone), poly(vinyl alcohol), polyacrylamide, poly(ethylene glycol), polylactides (PLA), poly(lactide-co-glycolides) (PLGA), and polyorthoesters. The polymer used in a sustained release formulation may be inert, free of leachable impurities, stable on storage, sterile, and biodegradable. In some embodiments, a controlled or sustained release system can be placed in proximity of the prophylactic or therapeutic target, thus requiring only a fraction of the systemic dose. Any suitable technique known to one of skill in the art may be used.

Dosage regimens may be adjusted to provide a therapeutic response. Dosing can depend on several factors, including severity and responsiveness of the disease, route of administration, time course of treatment (days to months to years), and time to amelioration of the disease. For example, a single bolus may be administered at one time, several divided doses may be administered over a predetermined period of time, or the dose may be reduced or increased as indicated by the therapeutic situation. The specification for the dosage is dictated by the unique characteristics of the active compound and the particular therapeutic effect to be achieved. Dosage values may vary with the type and severity of the condition to be alleviated. For any particular subject, specific dosage regimens may be adjusted over time according to the individual need and the professional judgment of the treating clinician. Toxicity and therapeutic efficacy of compounds provided herein can be determined by standard pharmaceutical procedures in cell culture or animal models. For example, LD50, ED50, EC50, and IC50 may be determined, and the dose ratio between toxic and therapeutic effects (LD50/ED50) may be calculated as the therapeutic index. Compositions that exhibit toxic side effects may be used, with careful modifications to minimize potential damage to reduce side effects. Dosing may be estimated initially from cell culture assays and animal models. The data obtained from in vitro and in vivo assays and animal studies can be used in formulating a range of dosage for use in humans.

The ingredients are supplied either separately or mixed together in unit dosage form, for example, as a dry lyophilized powder or water-free concentrate in a hermetically sealed container such as an ampoule or sachet indicating the quantity of active agent. If the mode of administration is by injection, an ampoule of sterile water for injection or saline can be provided so that the ingredients may be mixed prior to administration.

The pharmaceutical compositions may be packaged in a hermetically sealed container such as an ampoule or sachet indicating the quantity of the agent. In one embodiment, one or more of the pharmaceutical compositions is supplied as a dry sterilized lyophilized powder or water-free concentrate in a hermetically sealed container and can be reconstituted (e.g., with water or saline) to the appropriate concentration for administration to a subject. In an embodiment, one or more of the prophylactic or therapeutic agents or pharmaceutical compositions is supplied as a dry sterile lyophilized powder in a hermetically sealed container stored between 2° C and 8° C and administered within 1 hour, within 3 hours, within 5 hours, within 6 hours, within 12 hours, within 24 hours, within 48 hours, within 72 hours, or within one week after being reconstituted. Cryoprotectants can be included for a lyophilized dosage form, principally 0-10% sucrose (optimally 0.5-1.0%). Other suitable cryoprotectants include trehalose and lactose. Other suitable bulking agents include glycine and arginine, either of which can be included at a concentration of 0-0.05 %, and polysorbate-80 (optimally included at a concentration of 0.005-0.01%). Additional surfactants include but are not limited to polysorbate 20 and BRIJ surfactants. The pharmaceutical composition may be prepared as an injectable solution and can further comprise an agent useful as an adjuvant, such as those used to increase absorption or dispersion, e.g., hyaluronidase.

In some embodiments, the genetically engineered viruses are prepared for delivery, taking into consideration the need for efficient delivery and for overcoming the host antiviral immune response. Approaches to evade antiviral response include the administration of different viral serotypes as par of the treatment regimen (serotype switching), formulation, such as polymer coating to mask the virus from antibody recognition and the use of cells as delivery vehicles.

In another embodiment, the composition can be delivered in a controlled release or sustained release system. In one embodiment, a pump may be used to achieve controlled or sustained release. In another embodiment, polymeric materials can be used to achieve controlled or sustained release of the therapies of the present disclosure (see e.g., U.S. Patent No. 5,989,463). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N- vinyl pyrrolidone), poly(vinyl alcohol), polyacrylamide, poly(ethylene glycol), polylactides (PLA), poly(lactide-co-glycolides) (PLGA), and polyorthoesters. The polymer used in a sustained release formulation may be inert, free of leachable impurities, stable on storage, sterile, and biodegradable. In some embodiments, a controlled or sustained release system can be placed in proximity of the prophylactic or therapeutic target, thus requiring only a fraction of the systemic dose. Any suitable technique known to one of skill in the art may be used.

The genetically engineered bacteria of the invention may be administered and formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

### In Vivo Methods

The recombinant bacteria disclosed herein may be evaluated *in vivo, e.g.,* in an animal model. Any suitable animal model of a disease or condition associated with catabolism of a branched chain amino acid may be used (*see, e.g.,* Skvorak, J. Inherit. Metab. Dis., 2009, 32:229-246 and Homanics et al., BMC Med. Genet., 2006, 7(33):1-13), including the Dbt-/- model (E2 subunit of BCKDH, which has a 3-fold increase in blood and urine BCAA levels and results in neonatal lethalthy) (serves as classic MSUD model). This model is partially rescued by two transgenes (LAP-tTA and TRE-E2), allowing 5-6% of normal BCKDH activity and an increase in mice survival to three or four weeks (serves as an intermediate MSUD model) (as described in Homanics *et al*., 2006,).

In addition, intermediate MSUD mice can be used to to show development of neuropathology with striking similarity to human MSUD. In this model, branched-chain amino acid accumulation was associated with neurotransmitter deficiency, behavioral changes and limited survival, and providing intermediate MSUD mice with a choice between normal and branched-chain amino acidfree diet prevented brain injury and dramatically improved survival (Zinnanti et al., Dual mechanism of brain injury and novel treatment strategy in maple syrup urine disease; Brain 2009: 132; 903-918).

In some embodiments, the animal model is a mouse model of Maple Syrup Urine Disease. In one embodiment, the mouse model of MSUD is a branched-chain amino transferase knockout mouse (Wu et al., J. Clin. Invest, 113:434-440, 2004 or She et al., Cell Metabol., 6:181-194, 2007). In another embodiment, the mouse model of MSUD is a dihydrolipoamine dehydrogenase (E3) subunit knock-out mouse (Johnson et al., Proc. Natl. Acad. Sci. U.S.A., 94:14512-14517, 1997). In another embodiment, the mouse model of classic MSUD is a deletion of exon 5 and part of exon 4 of the E2 subunit of the branched-chain alpha-keto acid dehydrogenase (Homanics et al., BMC Med. Genet., 7:33, 2006) or the mouse model of intermediate MSUD (Homanics et al., BMC Med. Genet., 7:33, 2006). In another embodiment, the model is a Polled Shorthorn calf model of disease or a Polled Hereford calf model of disease (Harper et al., Aus. Vet. J., 66(2):46-49, 1988). Other relevant animal models include those described in She et al., Cell Metab. 2007 September ; 6(3): 181-194; Wu et al., J. Clin. Invest. 113:434-440 (2004); Bridi, et al., J Neurosci Methods. 2006 Sep 15;155(2):224-30.

The recombinant bacterial cells disclosed herein may administered to the animal, *e.g.,* by oral gavage, and treatment efficacy is determined, *e.g.,* by measuring blood leucine levels before and after treatment. The animal may be sacrificed, and tissue samples may be collected and analyzed.

### Methods of Screening

### Generation of Bacterial Strains with Enhance Ability to Transport Amino Acids

Due to their ease of culture, short generation times, very high population densities and small genomes, microbes can be evolved to unique phenotypes in abbreviated timescales. Adaptive laboratory evolution (ALE) is the process of passaging microbes under selective pressure to evolve a strain with a preferred phenotype. Most commonly, this is applied to increase utilization of carbon/energy sources or adapting a strain to environmental stresses (e.g., temperature, pH), whereby mutant strains more capable of growth on the carbon substrate or under stress will outcompete the less adapted strains in the population and will eventually come to dominate the population.

This same process can be extended to any essential metabolite by creating an auxotroph. An auxotroph is a strain incapable of synthesizing an essential metabolite and must therefore have the metabolite provided in the media to grow. In this scenario, by making an auxotroph and passaging it on decreasing amounts of the metabolite, the resulting dominant strains should be more capable of obtaining and incorporating this essential metabolite.

For example, if the biosynthetic pathway for producing an amino acid is disrupted a strain capable of high-affinity capture of said amino acid can be evolved via ALE. First, the strain is grown in varying concentrations of the auxotrophic amino acid, until a minimum concentration to support growth is established. The strain is then passaged at that concentration, and diluted into lowering concentrations of the amino acid at regular intervals. Over time, cells that are most competitive for the amino acid - at growth-limiting concentrations - will come to dominate the population. These strains will likely have mutations in their amino acid-transporters resulting in increased ability to import the essential and limiting amino acid.

Similarly, by using an auxotroph that cannot use an upstream metabolite to form an amino acid, a strain can be evolved that not only can more efficiently import the upstream metabolite, but also convert the metabolite into the essential downstream metabolite. These strains will also evolve mutations to increase import of the upstream metabolite, but may also contain mutations which increase expression or reaction kinetics of downstream enzymes, or that reduce competitive substrate utilization pathways.

A metabolite innate to the microbe can be made essential via mutational auxotrophy and selection applied with growth-limiting supplementation of the endogenous metabolite. However, phenotypes capable of consuming non-native compounds can be evolved by tying their consumption to the production of an essential compound. For example, if a gene from a different organism is isolated which can produce an essential compound or a precursor to an essential compound this gene can be recombinantly introduced and expressed in the heterologous host. This new host strain will now have the ability to synthesize an essential nutrient from a previously non-metabolizable substrate.

Hereby, a similar ALE process can be applied by creating an auxotroph incapable of converting an immediately downstream metabolite and selecting in growth-limiting amounts of the non-native compound with concurrent expression of the recombinant enzyme. This will result in mutations in the transport of the non-native substrate, expression and activity of the heterologous enzyme and expression and activity of downstream native enzymes. It should be emphasized that the key requirement in this process is the ability to tether the consumption of the non-native metabolite to the production of a metabolite essential to growth.

Once the basis of the selection mechanism is established and minimum levels of supplementation have been established, the actual ALE experimentation can proceed. Throughout this process several parameters must be vigilantly monitored. It is important that the cultures are maintained in an exponential growth phase and not allowed to reach saturation/stationary phase. This means that growth rates must be check during each passaging and subsequent dilutions adjusted accordingly. If growth rate improves to such a degree that dilutions become large, then the concentration of auxotrophic supplementation should be decreased such that growth rate is slowed, selection pressure is increased and dilutions are not so severe as to heavily bias subpopulations during passaging. In addition, at regular intervals cells should be diluted, grown on solid media and individual clones tested to confirm growth rate phenotypes observed in the ALE cultures.

Predicting when to halt the stop the ALE experiment also requires vigilance. As the success of directing evolution is tied directly to the number of mutations "screened" throughout the experiment and mutations are generally a function of errors during DNA replication, the cumulative cell divisions (CCD) acts as a proxy for total mutants which have been screened. Previous studies have shown that beneficial phenotypes for growth on different carbon sources can be isolated in about 10^{11.2} CCD¹. This rate can be accelerated by the addition of chemical mutagens to the cultures - such as N-methyl-N-nitro-N-nitrosoguanidine (NTG) - which causes increased DNA replication errors. However, when continued passaging leads to marginal or no improvement in growth rate the population has converged to some fitness maximum and the ALE experiment can be halted.

At the conclusion of the ALE experiment, the cells should be diluted, isolated on solid media and assayed for growth phenotypes matching that of the culture flask. Best performers from those selected are then prepped for genomic DNA and sent for whole genome sequencing. Sequencing with reveal mutations occurring around the genome capable of providing improved phenotypes, but will also contain silent mutations (those which provide no benefit but do not detract from desired phenotype). In cultures evolved in the presence of NTG or other chemical mutagen, there will be significantly more silent, background mutations. If satisfied with the best performing strain in its current state, the user can proceed to application with that strain. Otherwise the contributing mutations can be deconvoluted from the evolved strain by reintroducing the mutations to the parent strain by genome engineering techniques. See Lee, D.-H., Feist, A. M., Barrett, C. L. & Palsson, B. Ø. Cumulative Number of Cell Divisions as a Meaningful Timescale for Adaptive Laboratory Evolution of Escherichia coli. *PLoS ONE* **6,** e26172 (2011).

Similar methods can be used to generate E.Coli Nissle mutants that consume or import branched chain amino acids, e.g., leucne, valine, and/or isoleucine.

### Specific Screen to identify strains with improved BCAA degradation enzyme activity

Screens using genetic selection are conducted to improve BCAA consumption in the genetically engineered bacteria. Toxic BCAA analogs exert their mechanism of action (MOA) by being incorporated into cellular protein, causing cell death. These compounds, e.g., fluoro-leucine and/or aza-leucine, have utility in an untargeted approach to select BCAA enzymes with increased activity. Assuming that these toxic compounds can be metabolized by BCAA enzymes into a non-toxic metabolite, rather than being incorporated into cellular protein, genetically engineered bacteria which have improved BCAA degradation activity can tolerate higher levels of these compounds, and can be screened for and selected on this basis.

### Use of Valine and Leucine sensitivity to identify strains with improved BCAA degradation enzyme activity

Valine and Leucine sensitivity can be used as a genetic screening tool using the *E.coli* K12 strain. As shown in Fig. 46, There are three AHAS (acetohydroxybutanoate synthase) isozymes in *E. coli* (AHAS I: ilvBN, AHAS II: ilvGM, and AHAS III: ilvIH). Valine and leucine exert feedback inhibition on AHAS I and AHAS III; AHAS II is resistant to Val and Leu inhibition. *E*. *coli* K12 has a frameshift mutation in *ilvG* (AHAS II) and is unable to produce BCAA endogenously in the presence of valine and leucine. In constrast, E. coli Nissle has a functional *ilvG* and is insensitive to valine and leucine and therefore cannot be used for this screen. A genetically engineered strain derived from E. coli K12, which more efficiently degrades leucine, has a greater reduction in sensitivity to leucine (through relieving the feedback inhibition on AHAS I and III). As a result, this pathway can be used as a tool to select and identify a strain with improved resistance to leucine.

### Use of Leucine auxotrophy and D-leucine as a method to to identify strains with improved BCAA uptake ability.

Bacterial mutants with increased leucine transport into the bacterial cell may be identified using a leucine auxotroph and providing D-leucine instead of L-leucine in the media, as D-leucine can be imported through! the same transporters. The basis of this strategy is outlined in **Fig. 51****.** The bacteria can grow in the presence of D-leucine, because the bacterial stain has a racemase, which can convert D-leucine to L-leucine. However, the uptake of D-leucine through LivKHMGF is less efficient than the uptake of L-leucine. The leucine auxotroph can still grow if high concentrations of D-Leucine are provided, even though the D-leucine uptake is less efficient than L-leucine uptake. When concentrations of D-leucine in the media are lowered, the cells can no longer grow, unless transport efficiency is increased, ergo, mutants with increased D-leucine uptake can be selected.

### Treatment

Further disclosed herein are compositions for use in methods of treating a disease or disorder associated with the catabolism of a branched chain amino acid. In some embodiments, disclosed herein are compositions for use in methods for reducing, ameliorating, or eliminating one or more symptom(s) associated with these diseases or disorders. In one embodiment, the disorder involving the catabolism of a branched chain amino acid is a metabolic disorder involving the abnormal catabolism of a branched chain amino acid. Metabolic diseases associated with abnormal catabolism of a branched chain amino acid include maple syrup urine disease (MSUD), isovaleric acidemia, propionic acidemia, methylmalonic acidemia, diabetes ketoacidosis, MCC Deficiency, 3-Methylglutaconyl-CoA hydratase Deficiency, HMG-CoA Lyase Deficiency, Acetyl-CoA Carboxylase Deficiency, Malonyl-CoA Decarboxylase Deficiency, short-branched chain acylCoA dehydrogenase deficiency, 2-methyl-3-hydroxybutyric acidemia, beta-ketothiolase deficiency, isobutyryl-CoA dehydrogenase deficiency, HIBCH deficiency), and 3-Hydroxyisobutyric aciduria.

In one embodiment, the disease associated with abnormal catabolism of a branched chain amino acid is isovaleric acidemia. In one embodiment, the disease associated with abnormal catabolism of a branched chain amino acid is propionic acidemia. In one embodiment, the disease associated with abnormal catabolism of a branched chain amino acid is methylmalonic acidemia. In another embodiment, the disease associated with abnormal catabolism of a branched chain amino acid is diabetes.

In one embodiment, the disease is maple syrup urine disease (MSUD). Maple syrup urine disease (MSUD), also known as branched-chain ketoaciduria, is an autosomal recessive metabolic disorder caused by impaired activity of the branched-chain α-keto acid dehydrogenase (BCKDH) complex (Skvorak 2009). The overall incidence for MSUD is 1:185,000, although it is higher in certain populations, such as Mennonites, where the incidence is 1:176. The BCKDH complex is responsible for the oxidative decarboxylation of branched-chain keto acids (BCKAs) derived from branched chain amino acids (BCAAs) (Homanics *et al.* 2006). Patients with MSUD are unable to properly process BCKAs, which can lead to the toxic accumulation of BCAAs and their derivatives in the blood, cerebrospinal fluid and tissues (Skvorak 2009). Specifically, deficiencies of the BCKDH complex in MSUD patients results in accumulation of the BCAAs isoleucine, leucine, and valine, as well as their corresponding branched-chain α-keto acids (BCKAs) α-keto-β-methylvalerate, α-ketoisocaproate, and α-ketoisovalerate) in the tissues in plasma. Clinical manifestations of the disease vary depending on the degree of enzyme deficiency and include poor feeding, vomiting, dehydration, lethargy, hypotonia, seizures, hypoglycemia, ketoacidosis, pancreatitis, coma, and neurological decline (Homanics *et al.* 2009).

he BCKDH complex is composed of three catalytic components: a dehydrogenase/decarboxylase (E1), which is a heterotetramer composed of two E1α and two E1β subunits, a dihydrolipoyl transacylase (E2), and a dihydrolipoamide dehydrogenase (E3) (Skvorak 2009). Additionally, the complex is associated with two regulatory enzymes, a BCKDH kinase and a BCKDH phosphatase, which control its activity through reversible phosphorylation-dephosphorylation of E1α (Chuang 1998, Homanics *et al.* 2006). To date, MSUD has been associated with mutations in the E1, E2 and E3 subunits of the BCKDH complex (Cheung 1998, Homanics *et al.* 2006).

MSUD is a very complex, genetically heterogeneous disease. At least 150 mutations in genes encoding BCKDH complex components have been identified that result in MSUD (Skvorak 2009). For example, see **Table C** below, adapted from Chuang, *J*. *Pediatrics,* 132(3), Part 2, S17-S23, 1998. As indicated below, E2 mutants are the most prevalent in human disease.

**Table C: MSUD Phenotypes**

| Molecular Phenotype | Affected Gene | Clinical Phenotype | Number of Mutations Identified |
|---|---|---|---|
| IA | E1α | Classic, Intermediate MSUD | 15 |
| IB | E1β | Classic MSUD | 4 |
| II | E2 | Classic, thiamine-responsive MSUD | 26 |
| III | E3 | E3-deficient | 4 |
| IV | Kinase | None reported | None reported |
| V | Phosphatase | None reported | None reported |

Currently available treatments for MSUD are inadequate for the long term management of the disease and have severe limitations (Svkvorak 2009). A low protein/BCAA-restricted diet, with micronutrient and vitamin supplementation, as necessary, is the widely accepted long-term disease management strategy for MSUD (Homanics *et al.* 2006). However, BCAA-intake restrictions can be particularly problematic since BCAAs can only be acquired through diet and are necessary for several metabolic activities (Skvorak 2009). Even with proper monitoring and patient compliance, BCAA dietary restrictions result in a high incidence of mental retardation and mortality (Skvorak 2009, Homanics *et al* 2009). Further, a few cases of MSUD have been treated by liver transplantation (Popescu and Dima 2012) or treatment with phenylbutyrate. However, the limited availability of donor organs, the costs associated with the transplantation itself, and the undesirable effects associated with continued immunosuppressant therapy limit the practicality of liver transplantation for treatment of disease (Homanics *et al.* 2012, Popescu and Dima 2012). Therefore, there is significant unmet need for effective, reliable, and/or long-term treatment for MSUD.

The present disclosure surprisingly demonstrates that pharmaceutical compositions comprising the recombinant bacterial cells disclosed herein may be used to treat metabolic diseases involving the abnormal catabolism of a branched chain amino acid, such as MSUD. In one embodiment, the metabolic disease is selected from the group consisting of classic MSUD, intermediate MSUD, intermittent MSUD, E3-Deficient MSUD, and thiamine-responsive MSUD. In one embodiment, the disease is classic MSUD. In another embodiment, the disease is intermediate MSUD. In another embodiment, the disease is intermittent MSUD. In another embodiment, the disease is E3-deficient MSUD. In another embodiment, the disease is thiamine-responsive MSUD.

In one embodiment, the subject having MSUD has a mutation in an E1α gene. In another embodiment, the subject having MSUD has a mutation in the E1β gene. In another embodiment, the subject having MSUD has a mutation in the E2 gene. In another embodiment, the subject having MSUD has a mutation in the E3 gene.

In one embodiment, the target degradation rates of branched chain amino acids from food intake in breastfed infants and adults is as indicated in the **Table 13,** below.

**Table 13. Target Degradation rates for BCAA.**

| **Age (year)** | **<1** | | | **1-3** | | | **4-8** | | | **8-12** | | | **>12** | | |
|---|---|---|---|---|---|---|---|---|---|---|---|---|---|---|---|
| **Amino acid: Leu (L) Val (V) Ile (I)** | **L** | **V** | **I** | **L** | **V** | **I** | **L** | **V** | **I** | **L** | **V** | **I** | **L** | **V** | **I** |
| MSUD patient daily tolerance (mg/kg) | 40 | 30 | 20 | 20 | 10 | 5 | 5 | 10 | 5 | 5 | 10 | 5 | 5 | 10 | 5 |
| Recommended Dietary Allowance (RDA) (mg/kg) | 93 | 58 | 43 | 63 | 37 | 28 | 49 | 28 | 22 | 46 | 26 | 20 | 46 | 26 | 20 |
| Target reduction (mg/kg) | 53 | 28 | 23 | 43 | 27 | 23 | 44 | 18 | 17 | 41 | 16 | 15 | 41 | 16 | 15 |
| Target reduction (mg); (based on 10, 14, 26, 41 and 70kg weight for the different age groups) | 530 | 280 | 230 | 602 | 378 | 322 | 1144 | 468 | 442 | 1681 | 656 | 615 | 2870 | 1120 | 1050 |
| Target reduction (mmol) | 4.04 | 2.39 | 1.75 | 4.59 | 3.23 | 2.45 | 8.72 | 3.99 | 3.37 | 12.81 | 5.60 | 4.69 | 21.88 | 9.56 | 8.00 |
| Target degradation rate (µmol/10⁹ CFUs/hr); (based on 3.10" CFUs/day dose) | 0.56 | 0.33 | 0.24 | 0.64 | 0.45 | 0.34 | 1.21 | 0.55 | 0.47 | 1.78 | 0.78 | 0.65 | 3.04 | 1.33 | 1.11 |
| Combined BCAA target degradation rate (µmol/10⁹ CFUs/hr) | **1.14** | | | **1.43** | | | **2.23** | | | **3.21** | | | **5.48** | | |

In one embodiment, the target degradation rates of branched chain amino acids from food intake in breastfed infants and adults is as indicated in the charts, below.

The leucine consumption kinetics and dosing are set forth in Table G. Food intake is based on adult recommended daily allowance of 40 mg/kg/day. MSUD patients are primarily children with restricted protein intake.

In another embodiment, the disorder involving the catabolism of a branched chain amino acid is a disorder caused by the activation of mTor (mammalian target of rapamycin). mTor is a serine-threonine kinase and has been implicated in a wide range of biological processes including transcription, translation, autophagy, actin organization and ribosome biogenesis, cell growth, cell proliferation, cell motility, and survival. mTOR exists in two complexes, mTORC1 and mTORC2. mTORC1 contains the raptor subunit and mTORC2 contains rictor. These complexes are differentially regulated, and have distinct substrate specificities and rapamycin sensitivity. For example, mTORC1 phosphorylates S6 kinase (S6K) and 4EBP1, promoting increased translation and ribosome biogenesis to facilitate cell growth and cell cycle progression. S6K also acts in a feedback pathway to attenuate PI3K/Akt activation. mTORC2 is generally insensitive to rapamycin and is thought to modulate growth factor signaling by phosphorylating the C-terminal hydrophobic motif of some AGC kinases, such as Akt.

It is known in the art that mTor activation is caused by branched chain amino acids or alpha keto acids in the subject (see, for example, Harlan et al., Cell Metabolism, 17:599-606, 2013). Specifically, activation of mTorC1 (mTor complex 1) is caused by leucine (see Han et al., Cell, 149:410-424, 2012 and Lynch, J. Nutr., 131(3):861S-865S, 2001). Thus, in one embodiment, the disclosure provides compositions for use in methods of treating disorders involving the catabolism of leucine, caused by the activation of mTor by leucine in the subject. In one embodiment, the leucine levels in the subject are normal, and lowering leucine levels in the subject leads to the decreased activity of mTor and, thus, treatment of the disease. In another embodiment, the leucine levels in the subject are increased, and lowering leucine levels in the subject leads to the decreased activity of mTor and, thus, treatment of the disease. In one embodiment, the activation of mTor is increased as compared to the normal level of activation of mTor in a healthy subject, and lowering leucine levels in the subject leads to the decreased activation of mTor and, thus, treatment of the disease. In one embodiment, the level of activity of mTor is increased as compared to the normal level of activity of mTor in a healthy subject, and lowering leucine levels in the subject leads to the decreased activity of mTor and, thus, treatment of the disease. In another embodiment, the expression of mTor is increased as compared to the normal level of expression of mTor in a healthy subject, and lowering leucine levels in the subject leads to the decreased activity of mTor and, thus, treatment of the disease. In one embodiment, the activation of mTor is an abnormal activation of mTor.

Diseases caused by the activation of mTor are known in the art. See, for example, Laplante and Sabatini, Cell, 149(2):74-293, 2012. As used herein, the term "disease caused by the activation of mTor" includes cancer, obesity, type 2 diabetes, neurodegeneration, autism, Alzheimer's disease, Lymphangioleiomyomatosis (LAM), transplant rejection, glycogen storage disease, obesity, tuberous sclerosis, hypertension, cardiovascular disease, hypothalamic activation, musculoskeletal disease, Parkinson's disease, Huntington's disease, psoriasis, rheumatoid arthritis, lupus, multiple sclerosis, Leigh's syndrome, and Friedrich's ataxia.

In another aspect, the disclosure provides for decreasing the plasma level of at least one branched chain amino acid or branched chain α-keto acid in a subject by administering a pharmaceutical composition comprising a bacterial cell disclosed herein to the subject, thereby decreasing the plasma level of the at least one branched chain amino acid or branched chain alpha-keto acid or other BCAA metabolite in the subject. In one embodiment, the subject has a disease or disorder involving the catabolism of a branched chain amino acid. In one embodiment, the disorder involving the catabolism of a branched chain amino acid is a metabolic disorder involving the abnormal catabolism of a branched chain amino acid. In another embodiment, the disorder involving the catabolism of a branched chain amino acid is a disorder caused by the activation of mTor. In one embodiment, the disease or disorder is a maple syrup urine disorder (MSUD). In one embodiment, the branched chain amino acid is leucine, isoleucine, or valine. In one embodiment, the branched chain amino acid is leucine. In another embodiment, the branched chain amino acid is isoleucine. In another embodiment, the branched chain amino acid is valine. In another embodiment, the branched chain α-keto acid is α-ketoisocaproic acid (KIC). In another embodiment, the branched chain α-keto acid is α-ketoisovaleric acid (KIV). In another embodiment, the branched chain α-keto acid is α-keto-β-methylvaleric acid (KMV). In other embodiments, the BCAA metabolite to be decrease is selected from any of BCAA metabolies shown in **Fig. 1****.**

In some embodiments, the disclosure provides for reducing, ameliorating, or eliminating one or more symptom(s) associated with these diseases, including but not limited to neurological deficits, mental retardation, brain damage, brain oedema, blindness, branched chain α-keto acid acidosis, myelinization failure, hyperammonaemia, coma, developmental delay, neurological impairment, failure to thrive, ketoacidosis, seizure, ataxia, neurodegeneration, hypotonia, lactic acidosis, recurrent myoglobinuria, and/or liver failure. In some embodiments, the disease is secondary to other conditions, *e.g*., liver disease.

In certain embodiments, the bacterial cells disclosed herein are capable of catabolizing branched chain amino acid(s), *e.g.,* leucine, in the diet of the subject in order to treat a disease associated with catabolism of a branched chain amino acid, *e.g.,* MSUD. In these embodiments, the bacterial cells are delivered simultaneously with dietary protein. In another embodiment, the bacterial cells are delivered simultaneously with phenylbutyrate. In another embodiment, the bacterial cells are delivered simultaneously with a thiamine supplement. In some embodiments, the bacterial cells and dietary protein are delivered after a period of fasting or leucine-restricted dieting. In these embodiments, a patient suffering from a disorder involving the catabolism of a branched chain amino acid, *e.g.,* MSUD, may be able to resume a substantially normal diet, or a diet that is less restrictive than a leucine-free diet. In some embodiments, the bacterial cells may be capable of catabolizing leucine from additional sources, *e.g.,* the blood, in order to treat a disease associated with the catabolism of a branched chain amino acid, *e.g.,* MSUD. In these embodiments, the bacterial cells need not be delivered simultaneously with dietary protein, and a leucine gradient is generated, *e.g.*, from blood to gut, and the recombinant bacteria catabolize the branched chain amino acid, *e.g.,* leucine, and reduce plasma levels of the branched chain amino acid, *e.g.,* leucine.

The embodiments may comprise preparing a pharmaceutical composition with at least one genetically engineered species, strain, or subtype of bacteria described herein, and administering the pharmaceutical composition to a subject in a therapeutically effective amount. In some embodiments, the bacterial cells disclosed herein are administered orally, *e.g.,* in a liquid suspension. In some embodiments, the bacterial cells disclosed herein are lyophilized in a gel cap and administered orally. In some embodiments, the bacterial cells disclosed herein are administered via a feeding tube or gastric shunt. In some embodiments, the bacterial cells disclosed herein are administered rectally, *e.g.,* by enema. In some embodiments, the genetically engineered bacteria are administered topically, intraintestinally, intrajejunally, intraduodenally, intraileally, and/or intracolically.

In certain embodiments, the administering the pharmaceutical composition described herein reduces branched chain amino acid levels in a subject. In some embodiments, the methods of the present disclosure reduce the branched chain amino acid levels, *e.g.,* leucine levels, in a subject by at least about 10%, 20%, 25%, 30%, 40%, 50%, 60%, 70%, 75%, 80%, 85%, 90%, 95%, or more. In another embodiment, the methods of the present disclosure reduce the branched chain amino acid levels, *e.g.,* leucine levels, in a subject by at least two-fold, three-fold, four-fold, five-fold, six-fold, seven-fold, eight-fold, nine-fold, or ten-fold. In some embodiments, reduction is measured by comparing the branched chain amino acid level in a subject before and after administration of the pharmaceutical composition. In one embodiment, the branched chain amino acid level is reduced in the gut of the subject. In another embodiment, the branched chain amino acid level is reduced in the blood of the subject. In another embodiment, the branched chain amino acid level is reduced in the plasma of the subject. In another embodiment, the branched chain amino acid level is reduced in the brain of the subject.

In one embodiment, the pharmaceutical composition described herein is administered to reduce branched chain amino acid levels in a subject to normal levels. In another embodiment, the pharmaceutical composition described herein is administered to reduce branched chain amino acid levels in a subject below normal levels to, for example, decrease the activation of mTor.

In certain embodiments, the pharmaceutical composition described herein is administered to reduce branched chain α-keto-acid levels in a subject. In some embodiments, the methods of the present disclosure reduce the branched chain α-keto-acid levels, in a subject by at least about 10%, 20%, 25%, 30%, 40%, 50%, 60%, 70%, 75%, 80%, 85%, 90%, 95%, or more as compared to levels in an untreated or control subject. In another embodiment, the methods of the present disclosure reduce the branched chain α-keto-acid levels, in a subject by at least two-fold, three-fold, four-fold, five-fold, six-fold, seven-fold, eight-fold, nine-fold, or ten-fold. In some embodiments, reduction is measured by comparing the branched chain α-keto-acid levels in a subject before and after administration of the pharmaceutical composition. In one embodiment, the branched chain α-keto-acid level is reduced in the gut of the subject. In another embodiment, the branched chain α-keto-acid level is reduced in the blood of the subject. In another embodiment, the branched chain α-keto-acid level is reduced in the plasma of the subject. In another embodiment, the branched chain α-keto-acid level is reduced in the brain of the subject.

In one embodiment, the pharmaceutical composition described herein is administered to reduce the branched chain α-keto-acid level in a subject to a normal level. In another embodiment, the pharmaceutical composition described herein is administered to reduce the branched chain α-keto-acid level in a subject below a normal level to, for example, decrease the activation of mTor.

In some embodiments, the treatment of the disorder involving the catabolism of a branched chain amino acid, *e.g.,* MSUD, allows one or more symptoms of the condition or disorder to improve by at least about 10%, 20%, 30%, 40%, 50%, 60%, 70%, 80%, 90%, 95%, or more. In some embodiments, the treatment of the disorder involving the catabolism of a branched chain amino acid, *e.g.,* MSUD, allows one or more symptoms of the condition or disorder to improve by at least about two-fold, three-fold, four-fold, five-fold, six-fold, seven-fold, eight-fold, nine-fold, or ten-fold.

Before, during, and after the administration of the pharmaceutical composition, branched chain amino acid levels, *e.g.,* leucine levels, in the subject may be measured in a biological sample, such as blood, serum, plasma, urine, peritoneal fluid, cerebrospinal fluid, fecal matter, intestinal mucosal scrapings, a sample collected from a tissue, and/or a sample collected from the contents of one or more of the following: the stomach, duodenum, jejunum, ileum, cecum, colon, rectum, and anal canal. In some embodiments, the treatment may include administration of the compositions disclosed herein to reduce levels of the branched chain amino acid, *e.g.,* leucine. In some embodiments, the treatment may include administration of the compositions disclosed herein to reduce the branched chain amino acid, *e.g.,* leucine, to undetectable levels in a subject. In some embodiments, the treatment may include administration of the compositions disclosed herein to reduce the branched chain amino acid, *e.g.,* leucine, concentrations to undetectable levels, or to less than about 1%, 2%, 5%, 10%, 20%, 25%, 30%, 40%, 50%, 60%, 70%, 75%, 80%, 85%, 90%, or 95% of the subject's branched chain amino acid levels prior to treatment.

In some embodiments, the recombinant bacterial cells disclosed herein produce a branched chain amino acid catabolism enzyme, *e.g*., KivD, BCKD and/or other BCAA catabolism enzyme, BCAA transporter, BCAA binding protein, etc, under exogenous environmental conditions, such as the low-oxygen environment of the mammalian gut, to reduce levels of branched chain amino acids in the blood or plasma by at least about 1.5-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 30-fold, at least about 40-fold, or at least about 50-fold as compared to unmodified bacteria of the same subtype under the same conditions.

In one embodiment, the bacteria disclosed herein reduce plasma levels of the branched chain amino acid, *e.g.,* leucine, will be reduced to less than 4 mg/dL. In one embodiment, the bacteria disclosed herein reduce plasma levels of the branched chain amino acid, *e.g.,* leucine, will be reduced to less than 3.9 mg/dL. In one embodiment, the bacteria disclosed herein reduce plasma levels of the branched chain amino acid, *e.g.,* leucine, will be reduced to less than 3.8 mg/dL, 3.7 mg/dL, 3.6 mg/dL, 3.5 mg/dL, 3.4 mg/dL, 3.3 mg/dL, 3.2 mg/dL, 3.1 mg/dL, 3.0 mg/dL, 2.9 mg/dL, 2.8 mg/dL, 2.7 mg/dL, 2.6 mg/dL, 2.5 mg/dL, 2.0 mg/dL, 1.75 mg/dL, 1.5 mg/dL, 1.0 mg/dL, or 0.5 mg/dL.

In one embodiment, the subject has plasma levels of at least 4 mg/dL prior to administration of the pharmaceutical composition disclosed herein. In another embodiment, the subject has plasma levels of at least 4.1 mg/dL, 4.2 mg/dL, 4.3 mg/dL, 4.4 mg/dL, 4.5 mg/dL, 4.75 mg/dL, 5.0 mg/dL, 5.5 mg/dL, 6 mg/dL, 7 mg/dL, 8 mg/dL, 9 mg/dL, or 10 mg/dL prior to administration of the pharmaceutical composition disclosed herein.

Certain unmodified bacteria will not have appreciable levels of branched chain amino acid, *e.g.,* leucine, processing. In embodiments using genetically modified forms of these bacteria, processing of branched chain amino acids, *e.g.,* leucine, will be appreciable under exogenous environmental conditions.

Branched chain amino acid levels, *e.g.,* leucine levels, may be measured by methods known in the art, *e.g.,* blood sampling and mass spectrometry. In some embodiments, branched chain amino acid catabolism enzyme expression is measured by methods known in the art. In another embodiment, branched chain amino acid catabolism enzyme activity is measured by methods known in the art to assess BCAA activity.

In certain embodiments, the recombinant bacteria are *E*. *coli* Nissle. The recombinant bacteria may be destroyed, *e.g*., by defense factors in the gut or blood serum (Sonnenborn et al., 2009) or by activation of a kill switch, several hours or days after administration. Thus, the pharmaceutical composition comprising the recombinant bacteria may be re-administered at a therapeutically effective dose and frequency. In alternate embodiments, the recombinant bacteria are not destroyed within hours or days after administration and may propagate and colonize the gut.

In one embodiments, the bacterial cells disclosed herein are administered to a subject once daily. In another embodiment, the bacterial cells disclosed herein are administered to a subject twice daily. In another embodiment, the bacterial cells disclosed herein are administered to a subject in combination with a meal. In another embodiment, the bacterial cells disclosed herein are administered to a subject prior to a meal. In another embodiment, the bacterial cells disclosed herein are administered to a subject after a meal. The dosage of the pharmaceutical composition and the frequency of administration may be selected based on the severity of the symptoms and the progression of the disease. The appropriate therapeutically effective dose and/or frequency of administration can be selected by a treating clinician.

The embodiments disclosed herein may comprise administration of a composition disclosed herein alone or in combination with one or more additional therapies, *e.g.,* the phenylbutyrate, thiamine supplementation, and/or a low-branched chain amino acid, *e.g.,* a low-leucine, diet. An important consideration in the selection of the one or more additional therapeutic agents is that the agent(s) should be compatible with the bacteria disclosed herein, *e.g.,* the agent(s) must not interfere with or kill the bacteria. In some embodiments, the genetically engineered bacteria are administered in combination with a low protein diet. In some embodiments, administration of the genetically engineered bacteria provides increased tolerance, sothat the patient can consume more protein.

The embodiments disclosed herein may further comprise isolating a plasma sample from the subject prior to administration of a composition disclosed herein and determining the level of the branched chain amino acid, *e.g.,* leucine, or branched chain alpha-keto-acid in the sample. Some embodiments may further comprise isolating a plasma sample from the subject after to administration of a composition disclosed herein and determining the level of the branched chain amino acid, *e.g.,* leucine, or branched chain alpha-keto-acid in the sample.

Some embodiments further comprise comparing the level of the branched chain amino acid or branched chain α-keto-acid in the plasma sample from the subject after administration of a composition disclosed herein to the subject to the plasma sample from the subject before administration of a composition disclosed herein to the subject. In one embodiment, a reduced level of the branched chain amino acid or branched chain alpha-keto-acid in the plasma sample from the subject after administration of a composition disclosed herein indicates that the plasma levels of the branched chain amino acid or branched chain alpha-keto-acid are decreased, thereby treating the disorder involving the catabolism of the branched chain amino acid in the subject. In one embodiment, the plasma level of the branched chain amino acid or branched chain α-ketoacid is decreased at least 10%, 20%, 30%, 40$, 50%, 60%, 70%, 80%, 90%, or 100% in the sample after administration of the pharmaceutical composition as compared to the plasma level in the sample before administration of the pharmaceutical composition. In another embodiment, the plasma level of the branched chain amino acid or branched chain α-ketoacid is decreased at least two-fold, three-fold, four-fold, or five-fold in the sample after administration of the pharmaceutical composition as compared to the plasma level in the sample before administration of the pharmaceutical composition.

Some embodiments further comprise comparing the level of the branched chain amino acid or branched chain α-keto-acid in the plasma sample from the subject after administration of a composition disclosed herein to a control level of the branched chain amino acid or branched chain alpha-keto-acid. In one embodiment, the control level of the branched chain amino acid is 4 mg/dL. In one embodiment, the subject is considered treated if the level of branched chain amino acid, *e.g.,* leucine, in the plasma sample from the subject after administration of the pharmaceutical composition disclosed herein, is less than 4 mg/dL. In one embodiment, the subject is considered treated if the level of branched chain amino acid, *e.g.,* leucine, in the plasma sample from the subject after administration of the pharmaceutical composition disclosed herein is less than 3.9, 3.8, 3.7, 3.6, 3.5, 3.4, 3.3, 3.2, 3.1, 3.0, 2.5, 2.0, 1.5, 1.0 or 0.5 mg/dL.

### Examples

The present disclosure is further illustrated by the following examples.

### Development of recombinant bacterial cells

### Example 1. Construction of Plasmids Encoding Branched Chain Amino Acid Importers and Branched Chain Amino Acid Catabolism Enzyme

The *kivD* gene of *lactococcus lactis* IFPL730 (sequence: SEQ ID NO: 1) was synthesized (Genewiz), fused to the Tet promoter, cloned into the high-copy plasmid pUC57-Kan by Gibson assembly (SEQ ID NO: 2), and transformed into *E. coli* DH5α as described in Example 3 to generate the plasmid pTet-kivD. The *bkd* operon of *Pseudomonas aeruginosa* PAO1 fused to the Tet promoter (SEQ ID NO: 3) was synthesized (Genewiz) and cloned into the high-copy plasmid pUC57-Kan to generate the plasmid pTet-bkd. The *bkd* operon of *Pseudomonas aeruginosa* PAO1 fused to the leuDH gene from PA01 and the Tet promoter (SEQ ID NO: 4) was synthesized (Genewiz) and cloned into the high-copy plasmid pUC57-Kan to generate the plasmid pTet-leuDH-bkd. The *livKHMGF* operon from *E. coli* Nissle fused to the Tet promoter (SEQ ID NO:5) was synthesized (Genewiz), cloned into the pKIKO-lacZ plasmid (SEQ ID NO:6) by Gibson assembly and transformed into *E. coli* PIR1 as described in Example 3 to generate the pTet-livKHMGF (SEQ ID NO:7).

### Example 2. Generation of Recombinant Bacterial Comprising a Genetic Modification that Reduces Export of a Branched Chain Amino Acid

*E. coli* Nissle was transformed with the pKD46 plasmid encoding the lambda red proteins under the control of an arabinose-inducible promoter as follows. An overnight culture of *E. coli* Nissle grown at 37 °C was diluted 1:100 in 4 mL of lysogeny broth (LB) and grown at 37 °C until it reached an OD₆₀₀ of 0.4-0.6. 1 mL of the culture was then centrifuged at 13,000 rpm for 1 min in a 1.5 mL microcentrifuge tube and the supernatant was removed. The cells were then washed three times in pre-chilled 10% glycerol and resuspended in 40 uL pre-chilled 10% glycerol. The electroporator was set to 1.8 kV. 1 uL of a pKD46 miniprep was added to the cells, mixed by pipetting, and pipetted into a sterile, chilled 1 mm cuvette. The cuvette was placed into the sample chamber, and the electric pulse was applied. 500 uL of room-temperature SOC media was immediately added, and the mixture was transferred to a culture tube and incubated at 30° C for 1 hr. The cells were spread out on an LB plate containing 100 ug/mL carbenicillin and incubated at 30 °C.

A ΔleuE deletion construct with 77 bp and a 100 bp flanking leuE homology regions and a kanamycin resistant cassette flanked by FRT recombination site (SEQ ID NO: 6) was generated by PCR, column-purified and transformed into *E. coli* Nissle pKD46 as follows. An overnight culture of *E. coli* Nissle pKD46 grown in 100 ug/mL carbenicillin at 30° C was diluted 1:100 in 5 mL of LB supplemented with 100 ug/mL carbenicillin, 0.15% arabinose and grown until it reaches an OD₆₀₀ of 0.4-0.6. The bacteria were aliquoted equally in five 1.5 mL microcentrifuge tubes, centrifuged at 13,000 rpm for 1 min and the supernatant was removed. The cells were then washed three times in pre-chilled 10% glycerol and combined in 50 uL pre-chilled 10% glycerol. The electroporator was set to 1.8 kV. 2 uL of a the purified ΔleuE deletion PCR fragment are then added to the cells, mixed by pipetting, and pipetted into a sterile, chilled 1 mm cuvette. The cuvette was placed into the sample chamber, and the electric pulse was applied. 500 uL of room-temperature SOC media was immediately added, and the mixture was transferred to a culture tube and incubated at 37° C for 1 hr. The cells were spread out on an LB plate containing 50 ug/mL kanamycin. Five kanamycin-resistant transformants were then checked by colony PCR for the deletion of the leuE locus.

The kanamycin cassette was then excised from the ΔleuE deletion strain as follows. ΔleuE was transformed with the pCP20 plasmid encoding the Flp recombinase gene. An overnight culture of ΔleuE grown at 37 °C in LB with 50 ug/mL kanamycin was diluted 1: 100 in 4 mL of LB and grown at 37 °C until it reaches an OD₆₀₀ of 0.4-0.6. 1 mL of the culture was then centrifuged at 13,000 rpm for 1 min in a 1.5 mL microcentrifuge tube and the supernatant was removed. The cells were then washed three times in pre-chilled 10% glycerol and resuspended in 40 uL pre-chilled 10% glycerol. The electroporator was set to 1.8 kV. 1 uL of a pCP20 miniprep was added to the cells, mixed by pipetting, and pipetted into a sterile, chilled 1 mm cuvette. The dry cuvette was placed into the sample chamber, and the electric pulse was applied. 500 uL of room-temperature SOC media was immediately added, and the mixture was transferred to a culture tube and incubated at 30° C for 1 hr. The cells were spread out on an LB plate containing 100 ug/mL carbenicillin and incubated at 30 °C. Eight transformants were then streaked on an LB plate and were incubated overnight at 43 °C. One colony per transformant was picked and resuspended in 10 uL LB and 3 uL of the suspension were pipetted on LB, LB with 50 ug/mL Kanamycin or LB with 100 ug/mL carbenicillin. The LB and LB Kanamycin plates were incubated at 37 °C and the LB Carbenicillin plate was incubated at 30 °C. Colonies showing growth on LB alone were selected and checked by PCR for the excision of the Kanamycin cassette.

### Example 3. Generation of Recombinant Bacteria Comprising a Transporter of a Branched Chain Amino Acid and/or a Branched Chain Amino Acid Catabolism Enzyme and Lacking an Exporter of a Branched Chain Amino Acid

pTet-kivD, pTet-bkd, pTet-leuDH-bkd and pTet-livKHFGF plasmids described above were transformed into *E. coli* Nissle (pTet-kivD), Nissle (pTet-kivD, pTet-bkd, pTet-leuDH-bkd), DH5α (pTet-kivD, pTet-bkd, pTet-leuDH-bkd) or PIR1 (pTet-livKHMGF). All tubes, solutions, and cuvettes were pre-chilled to 4° C. An overnight culture of E. *coli* (Nissle, ΔleuE, DH5α or PIR1) was diluted 1:100 in 4 mL of LB and grown until it reached an OD₆₀₀ of 0.4-0.6. 1mL of the culture was then centrifuged at 13,000 rpm for 1 min in a 1.5mL microcentrifuge tube and the supernatant was removed. The cells were then washed three times in pre-chilled 10% glycerol and resuspended in 40uL pre-chilled 10% glycerol. The electroporator was set to 1.8kV. 1uL of a pTet-kivD, pTet-bkd, pTet-leuDH-bkd or pTet-livKHMGF miniprep was added to the cells, mixed by pipetting, and pipetted into a sterile, chilled 1mm cuvette. The dry cuvette was placed into the sample chamber, and the electric pulse was applied. 500uL of room-temperature SOC media was immediately added, and the mixture was transferred to a culture tube and incubated at 37° C for 1 hr. The cells were spread out on an LB plate containing 50ug/mL Kanamycin for pTet-kivD, pTet-bkd and pTet-leuDH-bkd or 100ug/mL carbenicillin for pTet-livKHMGF.

### Example 4. Generation of Recombinant Bacteria Comprising a Transporter of a Branched Chain Amino Acid and a Genetic Modification that Reduces Export of a Branched Chain Amino Acid

*E. coli* Nissle ΔleuE was transformed with the pKD46 plasmid encoding the lambda red proteins under the control of an arabinose-inducible promoter as follows. An overnight culture of *E. coli* Nissle ΔleuE grown at 37 °C was diluted 1:100 in 4 mL of LB and grown at 37 °C until it reached an OD₆₀₀ of 0.4-0.6. 1 mL of the culture was then centrifuged at 13,000 rpm for 1 min in a 1.5 mL microcentrifuge tube and the supernatant was removed. The cells were then washed three times in pre-chilled 10% glycerol and resuspended in 40 uL pre-chilled 10% glycerol. The electroporator was set to 1.8 kV. 1 uL of a pKD46 miniprep was added to the cells, mixed by pipetting, and pipetted into a sterile, chilled 1 mm cuvette. The dry cuvette was placed into the sample chamber, and the electric pulse was applied. 500 uL of room-temperature SOC media was immediately added, and the mixture was transferred to a culture tube and incubated at 30° C for 1 hr. The cells were spread out on an LB plate containing 100 ug/mL carbenicillin and incubated at 30 °C.

The DNA fragment used to integrate Tet-livKHMGF into *E. coli* Nissle lacZ (SEQ ID NO: 10, **Fig. 31**) was amplified by PCR from the pTet-livKHMGF plasmid, column-purified and transformed into ΔleuE pKD46 as follows. An overnight culture of the *E. coli* Nissle ΔleuE pKD46 strain grown in LB at 30° C with 100 ug/mL carbenicillin was diluted 1: 100 in 5 mL of lysogeny broth (LB) supplemented with 100 ug/mL carbenicillin, 0.15% arabinose and grown at 30° C until it reached an OD₆₀₀ of 0.4-0.6. The bacteria were aliquoted equally in five 1.5 mL microcentrifuge tubes, centrifuged at 13,000 rpm for 1 min and the supernatant was removed. The cells were then washed three times in pre-chilled 10% glycerol and combined in 50 uL pre-chilled 10% glycerol. The electroporator was set to 1.8 kV. 2 uL of a the purified Tet-livKHMGF PCR fragment were then added to the cells, mixed by pipetting, and pipetted into a sterile, chilled 1 mm cuvette. The dry cuvette was placed into the sample chamber, and the electric pulse was applied. 500 uL of room-temperature SOC media was immediately added, and the mixture was transferred to a culture tube and incubated at 37° C for 1 hr. The cells were spread out on an LB plate containing 20 ug/mL chloramphenicol, 40 ug/mL X-Gal and incubated overnight at 37° C. White chloramphenicol resistant transformants were then checked by colony PCR for integration of Tet-livKHMGF into the lacZ locus.

### Functional assays using recombinant bacterial cells

### Example 5. Functional Assay Demonstrating that the Recombinant Bacterial Cells disclosed herein Decrease Branched Chain Amino Acid Concentration

For *in vitro* studies, all incubations were performed at 37° C. Cultures of *E*. *coli* Nissle ΔleuE, ΔleuE+pTet-kivD, ΔleuE+pTet-bkd, ΔleuE+pTet-leuDH-bkd, ΔleuE lacZ:Tet-livKHMGF, ΔleuE lacZ:Tet-livKHMGF+pTet-kivD, ΔleuE lacZ:Tet-livKHMGF+pTet-bkd, ΔleuE lacZ:Tet-livKHMGF+pTet-leuDH-bkd were grown overnight in LB, LB 50ug/mL Kanamycin or LB 50ug/mL Kanamycin 20ug/mL chloramphenicol and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of KivD, Bkd, LeuDH and LivKHFMG, and bacteria were grown for another 3 hours. Bacteria were then pelleted, washed, and resuspended in minimal media, and supplemented with 0.5% glucose and 2mM leucine. Aliquots were removed at 0h, 1.5h, 6h and 18h for leucine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in 90uL 10% acetonitrile, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 95 %A, 5 %B
0.5min: 95%A, 5%B
1min: 10%A, 90%B
2.5min: 10%A, 90%B
2.51 min: 95%A, 5%B
3.5min: 95%A, 5%B
The Q1/Q3 transitions used for leucine and L-leucine-5,5,5-d₃ were 132.1/86.2 and 135.1/89.3 respectively.

As shown in **Fig. 16****,** leucine was rapidly degraded by the expression of kivD in the Nissle ΔleuE strain. After 6h of incubation, leucine concentration droped by over 99% in the presence of ATC. This effect was even more pronounced in the case of ΔleuE expressing both kivD and the leucine transporter livKHMGF where leucine is undetectable after 6h of incubation. As shown in **Fig. 17****,** the expression of the bkd complex also leads rapidly to the degradation of leucine. After 6h of incubation, 99% of leucine was degraded. The expression of the leucine transporter livKHMGF, in parallel with the expression of leuDH and bkd leads to the complete degradation of leucine after 18h.

### Example 6. Simultaneous Degradation of Branched Chain Amino Acids by Recombinant Bacteria Expressing a Branched Chain Amino Acid Catabolism Enzyme and an Importer of a Branched Chain Amino Acid

In these studies, all incubations were performed at 37° C. Cultures of *E. coli* Nissle, Nissle+pTet-kivD, ΔleuE+pTet-kivD, ΔleuE lacZ:Tet-livKHMGF+pTet-kivD were grown overnight in LB, LB 50ug/mL Kanamycin or LB 50ug/mL Kanamycin 20ug/mL chloramphenicol and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of KivD and LivKHFMG, and bacteria were grown for another 3 hours. Bacteria were then pelleted, washed, and resuspended in minimal media, and supplemented with 0.5% glucose and the three branched chain amino acids (leucine, isoleucine and valine, 2mM each). Aliquots were removed at 0h, 1.5h, 6h and 18h for leucine, isoleucine and valine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100°7oA, 0%B
0.5min: 100%A, 0%B
1.5min: 10%A, 90%B
3.5min: 10%A, 90%B
3.51min: 100°7oA, 0%B
4.5min: 100%A, 0%B
The Q1/Q3 transitions used are:
   Leucine: 132.1186.2
   L-leucine-5,5,5-d₃: 135.1/89.3
   Isoleucine: 132.1/86.2
   Valine: 118.1/72

As shown in Figs. 11A-11C, leucine, isoleucine and valine were all degraded by the expression of kivD in *E*. *coli* Nissle. At 18h, 96.8%, 67.2% and 52.1% of leucine, isoleucine and valine respectively were degraded in Nissle expressing kivD in the presence of ATC. The efficiency of leucine and isoleucine degradation was further improved by expressing kivD in the ΔleuE background strain with a 99.8% leucine and 80.6% isoleucine degradation at 18h. Finally, an additional increase in leucine and isoleucine degradation was achieved by expressing the leucine transporter livKHMGF in the Nissle ΔleuE pTet-kivD strain with a 99.98% leucine and 95.5% isoleucine degradation at 18h. No significant improvement in valine degradation was observed in the ΔleuE deletion strain expressing livKHMGF.

### Example 7. Degradation of Leucine and its Ketoacid Derivative, Ketoisocaproate (KIC) by Recombinant Bacterial Cells in vitro

Leucine and its ketoacid derivative, alpha-ketoisocaproate (KIC), are two major metabolites which accumulate to toxic levels in MSUD patients. Different synthetic probiotic *E. coli* Nissle strains were engineered to degrade leucine and KIC in order to determine the rate of degradation of leucine and KIC in these strains.

All strains were derived from the human probiotic strain *E. coli* Nissle 1917. A *ΔleuE* deletion strain (deleted for the leucine exporter *leuE*) was generated by lambda red.. recombination. A copy of the high-affinity leucine ABC transporter *livKHMGF* under the control of a tetracycline-inducible promoter (Ptet) was inserted into the *lacZ* locus of the *ΔleuE* deletion strain by lambda-red recombination. In order to avoid endogenous production of BCAA and KIC, the biosynthetic gene *ilvC* was deleted in the ΔleuE; lacZ:tetR-Pₜₑₜ-livKHMGF strain by P1 transduction using the ΔilvC BW25113 *E. coli* strain as donor to generate the SYN469 strain (ΔleuE ΔilvC; lacZ:tetR-Pₜₑₜ-livKHMGF).

The SYN469 strain was then transformed with five different constructs under the control of Ptet on the high-copy plasmid pUC57-Kan (**Fig. 23**). The components of the constructs were:
- the leucine dehydrogenase *leuDH* derived from *Pseudomonas aeruginosa* PAO1, which catalyzes the reversible deamination of branched chain amino acids (*i.e.,* leucine, valine and isoleucine),
- the branched chain amino acid aminotransferase *ilvE* from *E. coli* Nissle, which catalyzes the reversible deamination of branched chain amino acids (*i.e.,* leucine, valine and isoleucine),
- the ketoacid decarboxylase *kivD* derived from *Lactococcus lactis* strain IFPL730, which catalyzes the decarboxylation of branched chain amino acids, and/or
- the alcohol dehydrogenase *adh2* derived from *Saccharomyces cerevisiae,* which catalyzes the conversion of branched chain amino acid-derived aldehydes to their respective alcohols.

Specifically, the following constructs were generated: Ptet-kivD (SYN479), ptet-kivD-leuDH (SYN467), Ptet-kivD-adh2 (SYN949), ptet-leuDH-kivD-adh2 (SYN954), and Ptet-ilvE-kivD-adh2 (SYN950).

SYN467, SYN469, SYN479, SYN949, SYN950 and SYN954 were grown overnight at 37°C and 250rpm in 4mL of LB supplemented with 100µg/mL kanamycin for SYN467, SYN479, SYN949, SYN950 and SYN954. Cells were diluted 100 fold in 4mL LB (with 100µg/mL kanamycin for SYN467, SYN479, SYN949, SYN950 and SYN954) and grown for 2h at 37°C and 250rpm. Cells were split in two 2mL culture tubes, and one 2mL culture tube was induced with 100ng/mL anhydrotetracycline (ATC) to activate the Ptet promoter. After 1h induction, the two 2mL culture tubes were split in four 1mL microcentrifuge tubes. The cells were spun down at maximum speed for 30 seconds in a microcentrifuge. The supernatant was removed and the pellet re-suspended in 1mL M9 medium 0.5% glucose. The cells were spun down again at maximum speed for 30 seconds and resuspended in 1mL M9 medium 0.5% glucose supplemented with 2mM leucine or 2mM KIC. Serial dilutions of the different cell suspensions were plated to determine the initial number of CFUs. The cells were transferred to a culture tube and incubated at 37°C and 250rpm for 3h. 150µL of cells were collected at 0h, 1h, 2h and 3h after addition of leucine or KIC for quantification by LC-MS/MS. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used are:
   Leucine: 132.1/86.2 in positive mode
   KIC: 129.1/129.1 in negative mode

The rate of degradation (in µmol/10⁹ CFUs/hr) was calculated for leucine and KIC.

**Fig. 24** and **Fig. 25** demonstrate that the different recombinant bacteria are able to debrade both leucine and KIC. The best performing strain was SYN950, with a 0.8 and 2.2 µmol/10⁹ CFUs/hr degradation rate for leucine and KIC, respectively.

The following table summarizes other experimental data generated in the course of evaluating leucine-degrading circuits:

**Table 14**

| **Feature** | **Insights Gained** |
|---|---|
| Branched chain aa recycling (*E*. *coli* can synthesize and excrete valine) | Intrinsic production of valine by engineered strain does not interfere with leucine degradation |
| Gene expression level | High copy expression of *kivD* enhances degradation |
| | rates → seeking switches with stronger activation levels |
| Co-factor requirement | Adding exogenous thiamine does not increase activity → endogenous pools sufficient |
| Environmental and assay pH | pH optimum = 6.5 → reaction should work well under GI and physiological conditions |
| Carbon source utilization and byproducts | Glucose drives optimal reaction rates → no evidence for inhibition by glycolysis (*e.g*., acid) byproducts |

Additional measures that may be taken to improve branched chain amino acid degradation rate include:

| **Potential limitation** | **Test** |
|---|---|
| BCAA uptake by cell is rate-limiting | Test additional BCAA transporters |
| | Establish genetic selections for transporter mutants with increased activity |
| BCAA-derived aldehydes inhibit KivD | Increase ADH2 expression/activity to convert the aldehydes into their respective alcohol |
| Slow conversion of BCAAs into their ketoacids | Express *leuDH* on a separate transcript from *kivD* |
| | Increase transcription rates for *leuDH* and *kivD* |
| | Overexpress the endogenous *ilvE* (BCAT) |
| | Identify KivD or LeuDH variants with increased enzymatic activity |
| Slow folding or misfolding of BCDH or KivD | Increase cellular osmolytes concentration (NaCl + betaine) |
| | Lower induction temperature |
| | Induce the expression of endogenous chaperones (heat-shock, benzyl alcohol) |
| | Express chaperones (dnaK-dnaJ-grpE, groES-groEL) |

### Example 8. Construction of Plasmids encoding Branched Chain Amino Acid Catabolism Enzymes, including a BCAA deaminating enzyme, an alpha-keto-acid decarboxylase, an alcohol dehydrogenase or an aldehyde dehydrogenase

The genes encoding the leucine dehydrogenases LeuDH_{Pa} (SEQ ID NO: 19) from *Pseudomonas aeruginosa,* the leucine dehydrogenase LeuDH_{Bc} (SEQ ID NO: 58) from *Bacillus cereus,* the L-amino acid deaminase LAAD_{Pv} (SEQ ID NO: 56) from *Proteus vulgaris,* the alcohol dehydrogenase Adh2 (SEQ ID NO: 38) from *S*. *cerevisae,* the alcohol dehydrogenase YqhD (SEQ ID NO: 60) from *E. coli* Nissle and the aldehyde dehydrogenase PadA (SEQ ID NO: 62) from *E. coli* K12 were incorporate into the pTet-kivD (SEQ ID NO: 2) plasmid described herein by Gibson assembly to generate the following constructs: pTet-kivD-leuDH_{Pa}, pTet-kivD-adh2, pTet-LeuDH_{Pa}-kivD-adh2, pTet-LeuDH_{Bc}-kivD-adh2, pTet-LeuDH_{Pa}-kivD-yqhD, pTet-LeuDH_{Bc}-kivD-yqhD, pTet-LeuDH_{Pa}-kivD-padA, pTet-LeuDH_{Bc}-kivD-padA, pTet-Laad_{Pv}-kivD-adh2, pTet-Laad_{Pv}-kivD-yqhD, pTet-Laad_{Pv}-kivD-padA. Those constructs were transformed into the following E. *coli* Nissle strains described herein: Δ*leuE,* Δ*leuE lacZ:tet-livKHMGF* and Δ*leuE* Δ*ilvC lacZ:tet-livKHMGF*

### Example 9. Improved Degradation of Leucine in Recombinant Bacteria Expressing Branched Chain Amino Acid Catabolism Enzyme by expressing an Importer of Branched Chain Amino Acid

In these studies, all incubations were performed at 37° C. Cultures *of E. coli* Nissle ΔleuE lacZ:Tet-livKHMGF and Nissle ΔleuE lacZ:Tet-livKHMGF, pTet-kivD were grown overnight LB 50ug/mL Kanamycin or LB 50ug/mL Kanamycin 20ug/mL chloramphenicol and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of KivD (SEQ ID NO: 2) and LivKHFMG (SEQ ID NO: 10), and bacteria were grown for another 3 hours. Bacteria were then pelleted, washed, and resuspended in minimal media to an OD₆₀₀ of 1 and supplemented with 0.5% glucose and 2mM leucine. Aliquots were removed at 0h and 4h for leucine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used are:
   Leucine: 132.1/86.2
   L-leucine-5,5,5-d₃: 135.1/89.3
   Isoleucine: 132.1/86.2
   Valine: 118.1/72
The rate of leucine degradation was calculated based on the number of CFUs (colony forming units) determined at T0 by plating serial dilution on LB plates.

As shown in **Figs. 42A** and **42B****,** leucine is consumed without the presence of ATC, due to normal bacterial growth during the assay. In the presence of ATC, degeradation is further improved by the expression of livKHMGF and kivD.

### Example 10. Fig 43ABCD. Degradation of all three Branched Chain Amino Acids by Recombinant Bacteria Expressing Branched Chain Amino Acid Catabolism Enzyme and improved degradation of leucine by expressing a leucine dehydrogenase

In these studies, all incubations were performed at 37° C. Cultures of *E. coli* Nissle ΔleuE lacZ:Tet-livKHMGF with the pTet-kivD or pTet-kivD-leuDH_{Pa} plasmid, were grown overnight in LB, LB 50ug/mL Kanamycin or LB 50ug/mL Kanamycin 20ug/mL chloramphenicol and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of KivD (SEQ NO:2), LeuDH_{Pa} (SEQ ID NO: 20) and LivKHMGF, and bacteria were grown for another 3 hours. Bacteria were then pelleted, washed, and resuspended in minimal media to OD₆₀₀ of 1, and supplemented with 0.5% glucose and the three branched chain amino acids (leucine, isoleucine and valine, 1mM each). Aliquots were removed at 0h, 3h, 19h for leucine, isoleucine and valine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100 x2 mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used are:
   Leucine: 132.1/86.2
   L-leucine-5,5,5-d₃: 135.1/89.3
   Isoleucine: 132.1/86.2
   Valine: 118.1/72
The rate of leucine degradation was calculated based on the number of CFUs (colony forming units) determined at T0 by plating serial dilution on LB plates.
As shown in **Figs. 43A, 43B** and **43C****,** leucine, isoleucine and valine were all degraded by the expression of kivD and kivD-leuDH_{Pa} in *E. coli* Nissle. The efficiency of leucine degradation was improved 25% by expressing the leucine dehydrogenase leuDH_{Pa} (Fig43D).

### Example 11. Enhanced Degradation of Leucine by Recombinant Bacteria Expressing an L-amino acid deaminase

In these studies, all incubations were performed at 37° C. Cultures of *E. coli* Nissle *ΔleuE ΔilvC lacZ:Tet-livKHMGF* (SYN469) with the pTet-ilvE-kivD-adh2, pTet-LeuDH_{Pa}-kivD-adh2 or pTet-Laad_{Pv}-kivD-leuDH_{Pa} plasmid, were grown overnight in LB for SYN469 and 50ug/mL Kanamycin for strains containing a plasmid and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of KivD (SEQ ID NO: 2), LeuDH_{Pa} (SEQ ID NO: 20), IlvE (SEQ ID NO: 22), LAAD_{Pv}(SEQ ID NO: 24) and LivKHFMG, and bacteria were grown for another 3 hours. Bacteria were then pelleted, washed, and resuspended in minimal media to OD₆₀₀ of 1, and supplemented with 0.5% glucose and 2mM. Aliquots were removed at 0h and 3h for leucine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used are:
   Leucine: 132.1/86.2
   L-leucine-5,5,5-d₃: 135.1/89.3
The rate of leucine degradation was calculated based on the number of CFUs (colony forming units) determined at T0 by plating serial dilution on LB plates.

**Fig. 47B** depicts the leucine degradation pathway used in the strains tested. As shown in **Fig. 47A****,** leucine degradation is greatly enhanced by the expression of LAAD_{Pv} (15-fold). This efficiency of leucine degradation far exceed the upper target degradation rate for efficient treatment of MSUD described herein and marked by a dotted line in **Fig. 47A****.**

### Example 12. Degradation of Leucine by Recombinant Bacteria Expressing L-amino acid deaminases from Proteus vulgaris and Proteus mirabilis

The gene encoding the L-amino acid deaminase Pma from Proteus mirabilis LAAD_{Pm} (SEQ ID NO: 26) was cloned under the control of the tet promoter in the high copy plasmid pUC57-Kan to generate the pTet-Laad_{Pm} plasmid. The pTet-Laad_{Pm} plasmid was transformed in the *ΔleuE ΔilvC lacZ:Tet-livKHMGF* (SYN469). In these studies, all incubations were performed at 37° C. Cultures of *E. coli* Nissle *ΔleuE ΔilvC lacZ:Tet-livKHMGF* (SYN469) with the pTet-Laad_{Pv}-kivD-adh2, pTet-Laad_{Pv}-kivD-yqhD, pTet-Laad_{Pv}-kivD-padA or pTet-Laad_{Pm} plasmid, were grown overnight in LB for SYN469 and 50ug/mL Kanamycin for strains containing a plasmid and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of the constructs, and bacteria were grown for another 3 hours. Bacteria were then pelleted, washed, and resuspended in minimal media to OD₆₀₀ of 1, and supplemented with 0.5% glucose and 2mM leucine. Aliquots were removed at 0h and 3h for leucine quantification by liquid chromatography-mass spectrometry (LCMS) using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used are:
   Leucine: 132.1/86.2
   L-leucine-5,5,5-d₃: 135.1/89.3
The rate of leucine degradation was calculated based on the number of CFUs (colony forming units) determined at T0 by plating serial dilution on LB plates.

**Fig. 48B** depicts the leucine degradation pathway used in the strains tested. As shown in **Fig. 48A****,** leucine degradation occurs at very efficient rates in strains expressing either LAAD_{Pv} or LAAD_{Pm}. The efficiency of leucine degradation far exceeds the upper target degradation rate for efficient treatment of MSUD described herein and marked by a dotted line in Fig48A.

### Example 13. Improvement of Leucine Degradation by Recombinant Bacteria Expressing BCAA catabolism enzymes and a Leucine importer

In these studies, all incubations were performed at 37° C. Cultures of *E. coli* Nissle *ΔleuE lacZ:Tet-livKHMGF* (SYN452) and *ΔleuE* (SYN458) with or without the pTet-LeuDH_{Pa}-kivD-padA plasmid, were grown overnight in LB for SYN452 and SYN458 or LB with 50ug/mL Kanamycin for strains containing a plasmid and then diluted 1:100 in LB. The cells were grown with shaking (250 rpm) to early log phase with the appropriate antibiotics. Anhydrous tetracycline (ATC) was added to cultures at a concentration of 100 ng/mL to induce expression of the constructs, and bacteria were grown for another 3 hours. Bacteria were then pelleted, washed, and resuspended in minimal media, and supplemented with 0.5% glucose and 4mM leucine. Aliquots were removed at T0, 40min, 90min and 150min for leucine, KIC and isovaleric acid (IVA) quantification by liquid chromatography-mass spectrometry (LCMS) using a Thermo TSQ Quantum Max triple quadrupole instrument. The rate of leucine degradation, KIC and IVA production was calculated based on the number of CFUs (colony forming units) determined at T0 by plating serial dilution on LB plates. Fig49B depicts the leucine degradation pathway used in the strains tested. As shown in **Fig. 49A****,** the expression of livKHMGF in SYN452 moderately improves the rate of leucine degradation in comparison with SYN458. This correlates with a mild increase in the production of isovalerate.

### Example 14. Improvement of Leucine Degradation by Recombinant Bacteria Expressing the leucine dehydrogenase from Bacillus cereus and the low affinity BCAA transporter BrnQ

The gene encoding the leucine dehydrogenase from *Bacillus cereus* (LeuDH_{Bc}) (SEQ ID NO: 58) was cloned in place of the leucine dehydrogenase from *Pseudomonas aeruginosa* leuDH_{Pa} (SEQ ID NO: 20) in the pTet-leuDH_{Pa}-kivD-padA constructs by Gibson assembly to generate the pTet-leuDH_{Bc}-kivD-padA plasmid. This plasmid was transformed into the E. *coli* Nissle *ΔleuE ΔilvC lacZ:Tet-livKHMGF* (SYN469) strain. The gene encoding *E. coli* Nissle low-affinity transporter BrnQ (SEQ ID NO: 64) was cloned under the control of the tet promoter in the low-copy plasmid pSC101 by Gibson assembly. The generated pTet-brnQ plasmid was transformed into the newly generated *E. coli* Nissle *ΔleuEΔilvC, lacZ:Tet-livKHMGF,* pTet-leuDH_{Bc}-kivD-padA strain to generated the *ΔleuEΔilvC, lacZ:Tet-livKHMGF,* pTet-leuDH_{Bc}-kivD-padA, pTet-brnQ strain. In these studies, all incubations were performed at 37° C. Cultures of E. *coli* Nissle *ΔleuEΔilvC,lacZ:Tet-livKHMGF,* pTet-leuDH_{Pa}-kivD-padA, *E*. *coli* Nissle *ΔleuEΔilvC, lacZ: Tet-livKHMGF,* pTet-leuDH_{Bc}-kivD-padA and *E. coli* Nissle *ΔleuEΔilvC,lacZ:Tet-livKHMGF,* pTet-leuDH_{Bc}-kivD-padA,pTet-brnQ strains were grown overnight in LB with 50ug/mL Kanamycin and 100ug/mL carbenicillin for the for strain containing pTet-brnQ. The cells were grown with shaking (250 rpm) to early log phase with the appropriate antibiotics. Anhydrous tetracycline (ATC) was added to cultures at a concentration of 100 ng/mL to induce expression of the constructs, and bacteria were grown for another 3 hours. Bacteria were then pelleted, washed, and resuspended in minimal media, and supplemented with 0.5% glucose and 4mM leucine. Aliquots were removed at T0, 1h, 2h and 3h for leucine, KIC and isovaleric acid (IVA) quantification by liquid chromatography-mass spectrometry (LCMS) using a Thermo TSQ Quantum Max triple quadrupole instrument. The rate of leucine degradation, KIC and IVA production was calculated based on the number of CFUs (colony forming units) determined at T0 by plating serial dilution on LB plates. Fig50C depicts the leucine degradation pathway used in the strains tested. As shown in **Fig. 50A****,** the expression of leuDH_{Bc} doubles the rate of leucine degradation compare to leuDH_{Pa}. The expression of the low-affinity BCAA transporter BrnQ dramatically improves the rate of leucine degradation, by 4 to 5 fold. In both cases, the increased level of leucine degradation correlates with an increased level of isovalerate production as shown in **Fig. 50A****.** The expression of BrnQ also leads to the accumulation of KIC, suggesting that the decarboxylation of KIC by kivD becomes the limiting step in the pathway. The efficiency of leucine degradation obtained by expressing BrnQ exceeds the upper target degradation rate for efficient treatment of MSUD described herein and marked by a dotted line in **Fig. 50B****.**

### Example 15. Recirculation of isotopic leucine into the mouse intestine after subcutaneous injection

To understand the kinetic relationship between intestinal and systemic levels of exogenously administered leucine, and determine if subcutaneous injection of leucine can be used as an acute model of MSUD to assess the activity of leucine-degrading strains, heavy isotopelabeled leucine (¹³C₆) was injected subcutaneously at 0.1mg/g in BL6 mice and quantified in plasma, small intestine effluent, cecum and large intestine effluent at different times after injection (before injection (T0), 30min, 1h and 2h after injection). For each time point, 3 mice were bled and dissected to collect their small intestine, cecum and large intestine content. ¹³C₆-Leu was quantified by LC-MS/MS by liquid chromatography-mass spectrometry (LCMS) using a Thermo TSQ Quantum Max triple quadrupole instrument. Briefly, 10uL of samples were resuspended in 90uL of derivatization mix (50mM 2-Hydrazinoquinoline, 50mM triphenylphosphine, 50mM, 2,2'-dipyridyl disulfide in acetonitrile) with 1ug/mL L-leucine-5,5,5-d₃ (isotope used as internal standard). The samples were then incubated at 60°C for 1h, centrifuged at 4,500rpm at 4°C for 5min. 20uL was then transferred to 180uL of water, 0.1% formic acid and placed in the LCMS autosampler. A C18 column 50x2mm, 5um particles was used (Luna, Phenomenex). The mobile phases used were water 0.1% formic acid (solvent A) and acetonitrile 0.1% (solvent B). The mass spectrometer was run in positive mode and the Q1/Q3 transitions used for ¹³C₆-Leu quantification were 279.1/144.2 and 279.1/160.2. **Fig. 53** shows that ¹³C₆-Leu is present in the plasma and the small intestine as early as 30min after injection, demonstrating that leucine is able to recirculate from the periphery into the small intestine. After 30min, the level gradually decreases. ¹³C₆-Leu remains undetectable in the cecum and the large intestine, suggesting that leucine is not able to recirculate to those parts of the gastrointestinal tract. Those results demonstrate that an increase in plasma leucine level, mimicking a transient acute MSUD state, can be obtained by subcutaneous injection of leucine and that part of this leucine can become available for an engineered BCAA-degrading bacteria residing in the gastrointestinal tract.

### Example 16. Testing the efficacy of engineered BCAA-degrading bacteria in an acute model of MSUD.

Intermediate MSUD mice are kept on a 50/50 BCAA-free diet (Dyets)/18% protein chow (Teklad), in order to maintain a normal level of BCAA in those animals and prevent mortality. All animals are bled before being injected with a mix of three BCAA amino acids subcutaneously in order to mimic an acute episode of high BCAA in those animals. After injection, animals are gavaged with a control bacterial strain, which is unable to degrade BCAA, or an BCAA-degrading strain, or a mock control made of the formulation buffer used to prepare bacterial inocula. At different time after gavaging, plasma is collected, and the level of each BCAA is determined to measure the efficacy of the treatment in reducing the systemic level of BCAAs. In one embodiement, the brain of the animals are collected to measure BCAAs. In another embodiement, the urine of the animals is collected to measure BCAAs.

In a second instance, intermediate MSUD mice are kept on a 50/50 BCAA-free diet (Dyets)/18% protein chow (Teklad), in order to maintain a normal level of BCAA in those animals and prevent mortality. All animals are bled before changing their diet to a chow with 10%, 15%, 18%, 20%, 30%, 40%, 50%, 60% or 70% proteins. After changing their diet, animals are gavaged with a control bacterial strain, unable to degrade BCAA, or an BCAA-degrading strain, or a mock control made of the formulation buffer used to prepare bacterial inocula. At different time after gavaging, plasma is collected, and the level of each BCAA is determined to measure the efficacy of the treatment in reducing the systemic level of BCAAs. In one embodiement, the brain of the animals are collected to measure BCAAs. In another embodiement, the urine of the animals is collected to measure BCAAs.

### Example 17. Increase of BCAA import by overexpressing the high affinity BCAA transporters livKHMGF and livJHMGF in vitro

In these studies, all the strains are derived from the human probiotic strain *E. coli* Nissle ΔleuE. In the ΔleuE, lacZ:Ptet-livKHMGF strain, the endogenous promoter of livJ was swapped with the constitutive promoter Ptac by lambda-red recombination using the Ptac-livJ construct (SEQ ID NO: 11) to generate the ΔleuE, lacZ:Ptet-livKHMGF, Ptac-livJ strain. In this strain, livJ is constitutively induced. In the presence of ATC, both BCAA transporters livKHMGF and livJHMGF are expressed. ΔleuE; ΔleuE, lacZ:Ptet-livKHMGF; ΔleuE, lacZ:Ptet-livKHMGF, Ptac-livJ strains were grown overnight at 37°C and 250rpm in 4mL of LB. Bacterial Cells were then diluted 100 fold in 4mL LB and grown for 2h at 37°C and 250rpm. Cells were then split in two 2mL culture tubes. One 2mL culture tube was induced with 100ng/mL anhydrotetracycline (ATC) to activate the Ptet promoter. After 1h induction, 1mL of cells was spun down at maximum speed for 30 seconds in a microcentrifuge. The supernatant was then removed and the pellet re-suspended in 1mL M9 medium 0.5% glucose. The cells were spun down again at maximum speed for 30 seconds and resuspended in 1mL M9 medium 0.5% glucose. The cells were then transferred to a culture tube and incubated at 37°C and 250rpm for 5.5h. 150µL of cells were collected at 0h, 2h and 5.5h and The concentration of valine in the cell supernatant at the different time points was determined by LC-MS/MS using a Thermo TSQ Quantum Max triple quadrupole instrument. Briefly, 100uL aliquots were centrifuged at 4,500rpm for 10min. 10uL of the supernatant was resuspended in 90uL water with 1ug/mL L-leucine-5,5,5-d₃ (isotope used as internal standard). 10uL of the samples was then resuspended in water, 0.1% formic acid and placed in the LCMS autosampler. A C18 column 100x2mm, 3um particles was used (Luna, Phenomenex). The mobile phases used were water 0.1% formic acid (solvent A) and acetonitrile 0.1% (solvent B). The gradient used was:
0 min: 100%A, 0%B
0.5min: 100%A, 0%B
1.5min: 10%A, 90%B
3.5min: 10%A, 90%B
3.51min: 100%A, 0%B
4.5min: 100%A, 0%B
The Q1/Q3 transitions used is:
   Valine: 118.1/72

As **Fig. 53** shows, the natural secretion of valine by *E. coli* Nissle is observed for the ΔleuE strain. The secretion of valine is strongly reduced for ΔleuE, lacZ:Ptet-livKHMGF in the presence of ATC. This strongly suggests that the secreted valine is efficiently imported back into the cell by livKHMGF. The secretion of valine is abolished in the ΔleuE, lacZ:Ptet-livKHMGF, Ptac-livJ strain, with or without ATC. This strongly suggests that the constitutive expression of livJ is sufficient to import back the entire amount of valine secreted by the cell via the livJHMGF transporter. In conclusion, we successfully engineered E. coli Nissle to efficiently import BCAA, in this case valine, using both an inducible promoter (Ptet), and a constitutive promoter (Ptac), controlling the expression of livKHMGF and livJ respectively.

### Example 18. Improved transport of leucine in recombinant bacteria expressing a leucine importer

In order to test if expressing the high-affinity leucine transporter livKHMGF increases the transport of leucine into the bacterial cell, the minimum inhibitory concentration (MIC) of the toxic analog 3-fluoroleucine was determined for the following E. *coli* Nissle strains: *E. coli* Nissle, *ΔleuE* and *ΔleuE, lacZ:Tet-livKHMGF.* Those strains were grown overnight in LB and diluted 2,000 fold in M9 minimum media supplemented with 0.5% glucose, in the presence of 250, 125, 62.5, 31.2, 15.6, 7.8, 3.9, 2, 1 or 0 ug/mL 3-fluoroleucine in the presence or absence of 100ng/mL ATC *for ΔleuE, lacZ:Tet-livKHMGF.* Cells were grown at 37°C for 20h. The MIC for each strain, with our without ATC, was determined by looking at the presence or absence of bacterial growth for each treatment and was defined as the minimum concentration blocking bacterial growth. The following **Table 15** describes the results:

**Table 15.**

| | MIC (ug/mL) | |
|---|---|---|
| Strain | -ATC | +ATC |
| Nissle | 31.25 | ND |
| *ΔleuE* | 62.5 | ND |
| *ΔleuE, lacZ:Tet-livKHMGF* | 31.25 | 2 |

The induction of the leucine importer livKHMGF by ATC in the *ΔleuE, lacZ:Tet-livKHMGF* strain led to a 16-fold reduction in the MIC to 3 - fluoroleucine, going from 31.25 to 2 ug/mL. This dramatic increase in sensitivity to the leucine toxic analog demonstrates that the expression of livKHMGF leads to a substantial increase in leucine transport into the cell.

### Example 19. Table of Sequences

**Table 16. Table of Sequences**

| SEQ ID NO | Description |
|---|---|
| SEQ ID NO: 14 | FNR responsive regulatory sequence |
| SEQ ID NO: 15 | FNR responsive regulatory sequence |
| SEQ ID NO: 16 | FNR responsive regulatory sequence |
| SEQ ID NO: 17 | FNR responsive regulatory sequence |
| SEQ ID NO: 18 | FNR responsive regulatory sequence |
| SEQ ID NO:80 | SEQ ID NO; FNR responsive regulatory sequence |
| SEQ ID NO: 81 | SEQ ID NO; FNR responsive regulatory sequence |
| SEQ ID NO: 82 | *nirB1* |
| SEQ ID NO: 83 | *nirB2* |
| SEQ ID NO: 84 | *nirB3* |
| SEQ ID NO: 85 | *ydfZ* |
| SEQ ID NO: 86 | *nirB*+*RBS* |
| SEQ ID NO: 87 | *ydfZ*+*RBS* |
| SEQ ID NO: 88 | *fnrS1* |
| SEQ ID NO: 89 | *fnrS2* |
| SEQ ID NO: 90 | *nirB+crp* |
| SEQ ID NO: 91 | *fnrS*+*crp* |
| SEQ ID NO: 46 | *katG* |
| SEQ ID NO: 47 | *dps* |
| SEQ ID NO: 48 | *ahpC* |
| SEQ ID NO: 49 | *oxyS* |
| SEQ ID NO: 1 | kivD gene from *Lactococcus lactis* IFPL730 |
| SEQ ID NO: 2 | Tet-kivD construct |
| SEQ ID NO:75 | Tet-kivD-leuDH construct |
| SEQ ID NO:76 | Tet-kivD-adh2 construct |
| SEQ ID NO:78 | Tet-leuDH-kivD-adh2 construct |
| SEQ ID NO:79 | Tet-ilvE-kivD-adh2 construct: |
| SEQ ID NO: 3 | Tet-bkd construct sequence |
| SEQ ID NO: 4 | Tet-leuDH-bkd construct |
| SEQ ID NO: 5 | Tet-livKHMGF construct |
| SEQ ID NO: 6 | pKIKO-lacZ |
| SEQ ID NO: 7 | pTet-livKHMGF sequence |
| SEQ ID NO: 8 | E. coli Nissle 1917 leucine exporter gene leuE |
| SEQ ID NO: 9 | leuE deletion construct |
| SEQ ID NO: 10 | Tet-livKHMGF fragment |
| SEQ ID NO: 11 | Ptac-livJ construct |
| SEQ ID NO: 12 | *livJ* sequence |
| SEQ ID NO: 13 | - Prp promoter (prpR sequence - underlined; Ribosome binding site - lower case; start codon of gene of interest (italicized atg) |
| SEQ ID NO: 19 | LeuDH Amino acid sequence *Pseudomonas aeruginosa* PA01 |
| SEQ ID NO: 20 | *leuDH* codon-optimized nucleotide sequence *Pseudomonas aeruginosa* PA01 |
| SEQ ID NO: 21 | IlvE Amino acid sequence |
| SEQ ID NO: 22 | *ilvE* nucleotide sequence (*E. coli* Nissle) |
| SEQ ID NO: 23 | L-AAD Amino acid sequence *(Proteus vulgaris)* |
| SEQ ID NO: 24 | L-AAD Codon-optimized nucleotide sequence *(Proteus vulgaris)* |
| SEQ ID NO: 25 | L-AAD Amino acid sequence (Proteus mirabilis) |
| SEQ ID NO: 26 | L-AAD Nucleotide sequence (Proteus mirabilis) |
| SEQ ID NO: 27 | KivD Amino acid sequence (Lactococcus lactis) |
| SEQ ID NO: 28 | *kivD* Nucleotide sequence (Lactococcus lactis) |
| SEQ ID NO: 29 | *kivD* Codon-optimized sequence (Lactococcus lactis) |
| SEQ ID NO: 30 | KdcA Amino acid sequence (Lactococcus lactis) |
| SEQ ID NO: 31 | *kdcA* Nucleotide sequence (Lactococcus lactis) |
| SEQ ID NO: 32 | *kdcA* Codon-optimized kdcA sequence |
| SEQ ID NO: 33 | THI3/KID1 Amino acid sequence (*Saccharomyces cerevisiae)* |
| SEQ ID NO: 34 | THI3/KID1 Nucleotide sequence (*Saccharomyces cerevisiae*) |
| SEQ ID NO: 35 | ARO10 Amino acid sequence (*Saccharomyces cerevisiae)* |
| SEQ ID NO: 36 | ARO10 Nucleotide sequence (*Saccharomyces cerevisiae*) |
| SEQ ID NO: 37 | Adh2 Amino acid sequence (*Saccharomyces cerevisiae*) |
| SEQ ID NO: 38 | *adh2* Nucleotide sequence (*Saccharomyces cerevisiae*) |
| SEQ ID NO: 39 | *adh2* Codon-optimized sequence (*Saccharomyces cerevisiae*) |
| SEQ ID NO: 40 | Adh6 Amino acid sequence (*Saccharomyces cerevisiae*) |
| SEQ ID NO: 41 | *adh6* Codon-optimized sequence (*Saccharomyces cerevisiae*) |
| SEQ ID NO:42 | Adh1 Amino acid sequence (*Saccharomyces cerevisiae*) |
| SEQ ID NO:43 | *adh1* Nucleotide sequence (*Saccharomyces cerevisiae*) |
| SEQ ID NO:44 | Adh3 Amino acid sequence (*Saccharomyces cerevisiae*) |
| SEQ ID NO:45 | *adh3* Nucleotide sequence (*Saccharomyces cerevisiae*) |
| SEQ ID NO:46 | Adh4 Amino acid sequence (*Saccharomyces cerevisiae*) |
| SEQ ID NO:47 | *adh4* Nucleotide sequence (*Saccharomyces cerevisiae*) |
| SEQ ID NO:48 | Adh5 Amino acid sequence (*Saccharomyces cerevisiae*) |
| SEQ ID NO:49 | *adh5* Nucleotide sequence (*Saccharomyces cerevisiae*) |
| SEQ ID NO:50 | Adh7 Amino acid sequence (*Saccharomyces cerevisiae*) |
| SEQ ID NO:51 | *adh7* Nucleotide sequence (*Saccharomyces cerevisiae*) |
| SEQ ID NO:52 | SFA1 Amino acid sequence (*Saccharomyces cerevisiae*) |
| SEQ ID NO:53 | *sfa1* Nucleotide sequence (*Saccharomyces cerevisiae*) |
| SEQ ID NO:54 | IlvC amino acid sequence from E. coli Nissle |
| SEQ ID NO:55 | ilvC gene from E. coli Nissle nucleotide sequence |
| SEQ ID NO:56 | L-amino acid deaminase L-AAD Codon-optimized sequence (from Proteus vulgaris) |
| SEQ ID NO:57 | L-amino acid deaminase L-AAD amino acid sequence (from Proteus vulgaris) |
| SEQ ID NO:58 | Leucine dehydrogenase leuDH from Bacillus cereus, Codon-optimized sequence |
| SEQ ID NO:59 | Leucine dehydrogenase leuDH from Bacillus cereus, amino acid sequence |
| SEQ ID NO:60 | Alcohol dehydrogenase YqhD from E. coli, Nucleotide sequence |
| SEQ ID NO:61 | Alcohol dehydrogenase YqhD from E. coli, amino acid sequence |
| SEQ ID NO:62 | Aldehyde dehydrogenase PadA from E. coli, Nucleotide sequence |
| SEQ ID NO:63 | Aldehyde dehydrogenase PadA from E. coli, amino acid sequence |
| SEQ ID NO:64 | BCAA transporter BrnQ from E. coli, Nucleotide sequence |
| SEQ ID NO:65 | BCAA transporter BrnQ from E. coli, AA sequence |
| SEQ ID NO:66 | Isovaleryl-CoA synthetase LbuL from *Streptomyces lividans* |
| SEQ ID NO:67 | Isovaleryl-CoA synthetase LbuL from *Streptomyces lividans* |
| SEQ ID NO:68 | LiuABCDE operon from Pseudomonas aeruginosa, liuA AA sequences |
| SEQ ID NO:69 | LiuABCDE operon from Pseudomonas aeruginosa, LiuB AA sequences |
| SEQ ID NO:70 | LiuABCDE operon from Pseudomonas aeruginosa, LiuC AA sequences |
| SEQ ID NO:71 | LiuABCDE operon from Pseudomonas aeruginosa, LiuD AA sequences |
| SEQ ID NO:72 | LiuABCDE operon from Pseudomonas aeruginosa, LiuE AA sequences |
| SEQ ID NO:73 | LiuABCDE codon optimized sequence |
| SEQ ID NO:74 | LiuABCDE operon from Pseudomonas aeruginosa, AA sequences |
| SEQ ID NO:75 | Tet-kivD-leuDH construct |
| SEQ ID NO:76 | Tet-kivD-adh2 construct |
| SEQ ID NO:78 | Tet-leuDH-kivD-adh2 construct |
| SEQ ID NO:79 | Tet-ilvE-kivD-adh2 construct: |
| SEQ ID NO: 91 | Nucleotide sequence of the livKHMGF operon |
| SEQ ID NO: 92 | LivK amino acid sequence |
| SEQ ID NO: 93 | LivK nucleotide sequence |
| SEQ ID NO: 94 | LivH amino acid sequence |
| SEQ ID NO: 95 | LivH nucleotide sequence |
| SEQ ID NO: 96 | LivM amino acid sequence |
| SEQ ID NO: 97 | LivM nucleotide sequence |
| SEQ ID NO: 98 | LivG amino acid sequence |
| SEQ ID NO: 99 | LivG nucleotide sequence |
| SEQ ID NO: 100 | LivF amino acid sequence |
| SEQ ID NO: 101 | LivF nucleotide sequence |

| SEQ ID NO | Description |
|---|---|
| SEQ ID NO: 14 | FNR responsive regulatory sequence |
| SEQ ID NO: 15 | FNR responsive regulatory sequence |
| SEQ ID NO: 16 | FNR responsive regulatory sequence |
| SEQ ID NO: 17 | FNR responsive regulatory sequence |
| SEQ ID NO: 18 | FNR responsive regulatory sequence |
| SEQ ID NO:80 | SEQ ID NO; FNR responsive regulatory sequence |
| SEQ ID NO: 81 | SEQ ID NO; FNR responsive regulatory sequence |
| SEQ ID NO: 82 | *nirP1* |
| SEQ ID NO: 83 | *nirB2* |
| SEQ ID NO: 84 | *nirB3* |
| SEQ ID NO: 85 | *ydfZ* |
| SEQ ID NO: 86 | *nirB*+*RBS* |
| SEQ ID NO: 87 | *ydfZ*+*RBS* |
| SEQ ID NO: 88 | *fnrS1* |
| SEQ ID NO: 89 | *fnrS2* |
| SEQ ID NO: 90 | *nirB*+*crp* |
| SEQ ID NO: 91 | *fnrS*+*crp* |
| SEQ ID NO: 46 | *katG* |
| SEQ ID NO: 47 | *dps* |
| SEQ ID NO: 48 | *ahpC* |
| SEQ ID NO: 49 | *oxyS* |
| SEQ ID NO: 1 | kivD gene from *Lactococcus lactis* IFPL730 |
| SEQ ID NO: 2 | Tet-kivD construct |
| SEQ ID NO:75 | Tet-kivD-leuDH construct |
| SEQ ID NO:76 | Tet-kivD-adh2 construct |
| SEQ ID NO:78 | Tet-leuDH-kivD-adh2 construct |
| SEQ ID NO:79 | Tet-ilvE-kivD-adh2 construct: |
| SEQ ID NO: 3 | Tet-bkd construct sequence |
| SEQ ID NO: 4 | Tet-leuDH-bkd construct |
| SEQ ID NO: 5 | Tet-livKHMGF construct |
| SEQ ID NO: 6 | pKIKO-lacZ |
| SEQ ID NO: 7 | pTet-livKHMGF sequence |
| SEQ ID NO: 8 | E. coli Nissle 1917 leucine exporter gene leuE |
| SEQ ID NO: 9 | leuE deletion construct |
| SEQ ID NO: 10 | Tet-livKHMGF fragment |
| SEQ ID NO: 11 | Ptac-livJ construct |
| SEQ ID NO: 12 | *livJ* sequence |
| SEQ ID NO: 13 | - Prp promoter (prpR sequence - underlined; Ribosome binding site - |
| | lower case; start codon of gene of interest (italicized atg) |
| SEQ ID NO: 19 | LeuDH Amino acid sequence *Pseudomonas aeruginosa* PA01 |
| SEQ ID NO: 20 | *leuDH* codon-optimized nucleotide sequence *Pseudomonas aeruginosa* PA01 |
| SEQ ID NO: 21 | IlvE Amino acid sequence |
| SEQ ID NO: 22 | *ilvE* nucleotide sequence (*E*. *coli* Nissle) |
| SEQ ID NO: 23 | L-AAD Amino acid sequence (*Proteus vulgaris)* |
| SEQ ID NO: 24 | L-AAD Codon-optimized nucleotide sequence (*Proteus vulgaris)* |
| SEQ ID NO: 25 | L-AAD Amino acid sequence (Proteus mirabilis) |
| SEQ ID NO: 26 | L-AAD Nucleotide sequence (Proteus mirabilis) |
| SEQ ID NO: 27 | KivD Amino acid sequence (Lactococcus lactis) |
| SEQ ID NO: 28 | *kivD* Nucleotide sequence (Lactococcus lactis) |
| SEQ ID NO: 29 | *kivD* Codon-optimized sequence (Lactococcus lactis) |
| SEQ ID NO: 30 | KdcA Amino acid sequence (Lactococcus lactis) |
| SEQ ID NO: 31 | *kdcA* Nucleotide sequence (Lactococcus lactis) |
| SEQ ID NO: 32 | *kdcA* Codon-optimized kdcA sequence |
| SEQ ID NO: 33 | THI3/KID1 Amino acid sequence (*Saccharomyces cerevisiae)* |
| SEQ ID NO: 34 | THI3/KID1 Nucleotide sequence (*Saccharomyces cerevisiae)* |
| SEQ ID NO: 35 | ARO10 Amino acid sequence (*Saccharomyces cerevisiae)* |
| SEQ ID NO: 36 | ARO10 Nucleotide sequence (*Saccharomyces cerevisiae*) |
| SEQ ID NO: 37 | Adh2 Amino acid sequence (*Saccharomyces cerevisiae)* |
| SEQ ID NO: 38 | *adh2* Nucleotide sequence (*Saccharomyces cerevisiae*) |
| SEQ ID NO: 39 | *adh2* Codon-optimized sequence (*Saccharomyces cerevisiae*) |
| SEQ ID NO: 40 | Adh6 Amino acid sequence *(Saccharomyces cerevisiae*) |
| SEQ ID NO: 41 | *adh6* Codon-optimized sequence (*Saccharomyces cerevisiae*) |
| SEQ ID NO:42 | Adhl Amino acid sequence (*Saccharomyces cerevisiae*) |
| SEQ ID NO:43 | *adh1* Nucleotide sequence (*Saccharomyces cerevisiae*) |
| SEQ ID NO:44 | Adh3 Amino acid sequence (*Saccharomyces cerevisiae*) |
| SEQ ID NO:45 | *adh3* Nucleotide sequence (*Saccharomyces cerevisiae*) |
| SEQ ID NO:46 | Adh4 Amino acid sequence (*Saccharomyces cerevisiae*) |
| SEQ ID NO:47 | *adh4* Nucleotide sequence (*Saccharomyces cerevisiae*) |
| SEQ ID NO:48 | Adh5 Amino acid sequence (*Saccharomyces cerevisiae*) |
| SEQ ID NO:49 | *adh5* Nucleotide sequence (*Saccharomyces cerevisiae*) |
| SEQ ID NO:50 | Adh7 Amino acid sequence (*Saccharomyces cerevisiae*) |
| SEQ ID NO:51 | *adh7* Nucleotide sequence (*Saccharomyces cerevisiae*) |
| SEQ ID NO:52 | SFA1 Amino acid sequence (*Saccharomyces cerevisiae*) |
| SEQ ID NO:53 | *sfa1* Nucleotide sequence (*Saccharomyces cerevisiae*) |
| SEQ ID NO:54 | IlvC amino acid sequence from E. coli Nissle |
| SEQ ID NO:55 | ilvC gene from E. coli Nissle nucleotide sequence |
| SEQ ID NO:56 | L-amino acid deaminase L-AAD Codon-optimized sequence (from Proteus vulgaris) |
| SEQ ID NO:57 | L-amino acid deaminase L-AAD amino acid sequence (from Proteus vulgaris) |
| SEQ ID NO:58 | Leucine dehydrogenase leuDH from Bacillus cereus, Codon-optimized sequence |
| SEQ ID NO:59 | Leucine dehydrogenase leuDH from Bacillus cereus, amino acid sequence |
| SEQ ID NO:60 | Alcohol dehydrogenase YqhD from E. coli, Nucleotide sequence |
| SEQ ID NO:61 | Alcohol dehydrogenase YqhD from E. coli, amino acid sequence |
| SEQ ID NO:62 | Aldehyde dehydrogenase PadA from E. coli, Nucleotide sequence |
| SEQ ID NO:63 | Aldehyde dehydrogenase PadA from E. coli, amino acid sequence |
| SEQ ID NO:64 | BCAA transporter BrnQ from E. coli, Nucleotide sequence |
| SEQ ID NO:65 | BCAA transporter BrnQ from E. coli, AA sequence |
| SEQ ID NO:66 | Isovaleryl-CoA synthetase LbuL from *Streptomyces lividans* |
| SEQ ID NO:67 | Isovaleryl-CoA synthetase LbuL from *Streptomyces lividans* |
| SEQ ID NO:68 | LiuABCDE operon from Pseudomonas aeruginosa, liuA AA sequences |
| SEQ ID NO:69 | LiuABCDE operon from Pseudomonas aeruginosa, LiuB AA sequences |
| SEQ ID NO:70 | LiuABCDE operon from Pseudomonas aeruginosa, LiuC AA sequences |
| SEQ ID NO:71 | LiuABCDE operon from Pseudomonas aeruginosa, LiuD AA sequences |
| SEQ ID NO:72 | LiuABCDE operon from Pseudomonas aeruginosa, LiuE AA sequences |
| SEQ ID NO:73 | LiuABCDE codon optimized sequence |
| SEQ ID NO:74 | LiuABCDE operon from Pseudomonas aeruginosa, AA sequences |
| SEQ ID NO:75 | Tet-kivD-leuDH construct |
| SEQ ID NO:76 | Tet-kivD-adh2 construct |
| SEQ ID NO:78 | Tet-leuDH-kivD-adh2 construct |
| SEQ ID NO:79 | Tet-ilvE-kivD-adh2 construct: |
| SEQ ID NO: 91 | Nucleotide sequence of the livKHMGF operon |
| SEQ ID NO: 92 | LivK amino acid sequence |
| SEQ ID NO: 93 | LivK nucleotide sequence |
| SEQ ID NO: 94 | LivH amino acid sequence |
| SEQ ID NO: 95 | LivH nucleotide sequence |
| SEQ ID NO: 96 | LivM amino acid sequence |
| SEQ ID NO: 97 | LivM nucleotide sequence |
| SEQ ID NO: 98 | LivG amino acid sequence |
| SEQ ID NO: 99 | LivG nucleotide sequence |
| SEQ ID NO: 100 | LivF amino acid sequence |
| SEQ ID NO: 101 | LivF nucleotide sequence |

### Informal Sequence Listing

### Gene coding regions are shown in uppercase

**SEQ ID NO:** 1: kivD gene from *Lactococcus lactis* IFPL730 
ATGTATACAGTAGGAGATTACCTATTAGACCGATTACACGAGTTAGGAATTGAA
GAAATTTTTGGAGTCCCTGGAGACTATAACTTACAATTTTTAGATCAAATTATTTC
CCACAAGGATATGAAATGGGTCGGAAATGCTAATGAATTAAATGCTTCATATAT
GGCTGATGGCTATGCTCGTACTAAAAAAGCTGCCGCATTTCTTACAACCTTTGGA
GTAGGTGAATTGAGTGCAGTTAATGGATTAGCAGGAAGTTACGCCGAAAATTTA
CCAGTAGTAGAAATAGTGGGATCACCTACATCAAAAGTTCAAAATGAAGGAAAA
TTTGTTCATCATACGCTGGCTGACGGTGATTTTAAACACTTTATGAAAATGCACG
AACCTGTTACAGCAGCTCGAACTTTACTGACAGCAGAAAATGCAACCGTTGAAA
TTGACCGAGTACTTTCTGCACTATTAAAAGAAAGAAAACCTGTCTATATCAACTT
ACCAGTTGATGTTGCTGCTGCAAAAGCAGAGAAACCCTCACTCCCTTTGAAAAAG
GAAAACTCAACTTCAAATACAAGTGACCAAGAAATTTTGAACAAAATTCAAGAA
AGCTTGAAAAATGCCAAAAAACCAATCGTGATTACAGGACATGAAATAATTAGT
TTTGGCTTAGAAAAAACAGTCACTCAATTTATTTCAAAGACAAAACTACCTATTA
CGACATTAAACTTTGGTAAAAGTTCAGTTGATGAAGCCCTCCCTTCATTTTTAGG
AATCTATAATGGTACACTCTCAGAGCCTAATCTTAAAGAATTCGTGGAATCAGCC
GACTTCATCTTGATGCTTGGAGTTAAACTCACAGACTCTTCAACAGGAGCCTTCA
CTCATCATTTAAATGAAAATAAAATGATTTCACTGAATATAGATGAAGGAAAAA
TATTTAACGAAAGAATCCAAAATTTTGATTTTGAATCCCTCATCTCCTCTCTCTTA
GACCTAAGCGAAATAGAATACAAAGGAAAATATATCGATAAAAAGCAAGAAGA
CTTTGTTCCATCAAATGCGCTTTTATCACAAGACCGCCTATGGCAAGCAGTTGAA
AACCTAACTCAAAGCAATGAAACAATCGTTGCTGAACAAGGGACATCATTCTTTG
GCGCTTCATCAATTTTCTTAAAATCAAAGAGTCATTTTATTGGTCAACCCTTATGG
GGATCAATTGGATATACATTCCCAGCAGCATTAGGAAGCCAAATTGCAGATAAA
GAAAGCAGACACCTTTTATTTATTGGTGATGGTTCACTTCAACTTACAGTGCAAG
AATTAGGATTAGCAATCAGAGAAAAAATTAATCCAATTTGCTTTATTATCAATAA
TGATGGTTATACAGTCGAAAGAGAAATTCATGGACCAAATCAAAGCTACAATGA
TATTCCAATGTGGAATTACTCAAAATTACCAGAATCGTTTGGAGCAACAGAAGAT
CGAGTAGTCTCAAAAATCGTTAGAACTGAAAATGAATTTGTGTCTGTCATGAAAG
AAGCTCAAGCAGATCCAAATAGAATGTACTGGATTGAGTTAATTTTGGCAAAAG
AAGGTGCACCAAAAGTACTGAAAAAAATGGGCAAACTATTTGCTGAACAAAATA
AATCATAA
**SEQ ID NO: 2** Tet-kivD construct 
gaattcgTTAAGACCCACTTTCACATTTAAGTTGTTTTCTAATCCGCATATGATCAAT
TCAAGGCCGAATAAGAAGGCTGGCTCTGCACCTTGGTGATCAAATAATTCGATA
GCTTGTCGTAATAATGGCGGCATACTATCAGTAGTAGGTGTTTCCCTTTCTTCTTT
AGCGACTTGATGCTCTTGATCTTCCAATACGCAACCTAAAGTAAAATGCCCCACA
GCGCTGAGTGCATATAATGCATTCTCTAGTGAAAAACCTTGTTGGCATAAAAAGG
CTAATTGATTTTCGAGAGTTTCATACTGTTTTTCTGTAGGCCGTGTACCTAAATGT
ACTTTTGCTCCATCGCGATGACTTAGTAAAGCACATCTAAAACTTTTAGCGTTATT
ACGTAAAAAATCTTGCCAGCTTTCCCCTTCTAAAGGGCAAAAGTGAGTATGGTGC
CTATCTAACATCTCAATGGCTAAGGCGTCGAGCAAAGCCCGCTTATTTTTACAT
 
GCCAATACAATGTAGGCTGCTCTACACCTAGCTTCTGGGCGAGTTTACGGGTTGT
TAAACCTTCGATTCCGACCTCATTAAGCAGCTCTAATGCGCTGTTAATCACTTTAC
TTTTATCTAATCTAGACATCATTAATTcctaatttttgttgacactctatcattgatagagttattttaccactcccta
tcagtgatagagaaaagtgaactctagaaataattttgtttaactttaagaaggagatatacatATGTATACAGTAGGAGA
TTACCTATTAGACCGATTACACGAGTTAGGAATTGAAGAAATTTTTGGAGTCCCT
GGAGACTATAACTTACAATTTTTAGATCAAATTATTTCCCACAAGGATATGAAAT
GGGTCGGAAATGCTAATGAATTAAATGCTTCATATATGGCTGATGGCTATGCTCG
TACTAAAAAAGCTGCCGCATTTCTTACAACCTTTGGAGTAGGTGAATTGAGTGCA
GTTAATGGATTAGCAGGAAGTTACGCCGAAAATTTACCAGTAGTAGAAATAGTG
GGATCACCTACATCAAAAGTTCAAAATGAAGGAAAATTTGTTCATCATACGCTGG
CTGACGGTGATTTTAAACACTTTATGAAAATGCACGAACCTGTTACAGCAGCTCG
AACTTTACTGACAGCAGAAAATGCAACCGTTGAAATTGACCGAGTACTTTCTGCA
CTATTAAAAGAAAGAAAACCTGTCTATATCAACTTACCAGTTGATGTTGCTGCTG
CAAAAGCAGAGAAACCCTCACTCCCTTTGAAAAAGGAAAACTCAACTTCAAATA
CAAGTGACCAAGAAATTTTGAACAAAATTCAAGAAAGCTTGAAAAATGCCAAAA
AACCAATCGTGATTACAGGACATGAAATAATTAGTTTTGGCTTAGAAAAAACAG
TCACTCAATTTATTTCAAAGACAAAACTACCTATTACGACATTAAACTTTGGTAA
AAGTTCAGTTGATGAAGCCCTCCCTTCATTTTTAGGAATCTATAATGGTACACTCT
CAGAGCCTAATCTTAAAGAATTCGTGGAATCAGCCGACTTCATCTTGATGCTTGG
AGTTAAACTCACAGACTCTTCAACAGGAGCCTTCACTCATCATTTAAATGAAAAT
AAAATGATTTCACTGAATATAGATGAAGGAAAAATATTTAACGAAAGAATCCAA
AATTTTGATTTTGAATCCCTCATCTCCTCTCTCTTAGACCTAAGCGAAATAGAATA
CAAAGGAAAATATATCGATAAAAAGCAAGAAGACTTTGTTCCATCAAATGCGCT
TTTATCACAAGACCGCCTATGGCAAGCAGTTGAAAACCTAACTCAAAGCAATGA
AACAATCGTTGCTGAACAAGGGACATCATTCTTTGGCGCTTCATCAATTTTCTTA
AAATCAAAGAGTCATTTTATTGGTCAACCCTTATGGGGATCAATTGGATATACAT
TCCCAGCAGCATTAGGAAGCCAAATTGCAGATAAAGAAAGCAGACACCTTTTAT
TTATTGGTGATGGTTCACTTCAACTTACAGTGCAAGAATTAGGATTAGCAATCAG
AGAAAAAATTAATCCAATTTGCTTTATTATCAATAATGATGGTTATACAGTCGAA
AGAGAAATTCATGGACCAAATCAAAGCTACAATGATATTCCAATGTGGAATTAC
TCAAAATTACCAGAATCGTTTGGAGCAACAGAAGATCGAGTAGTCTCAAAAATC
GTTAGAACTGAAAATGAATTTGTGTCTGTCATGAAAGAAGCTCAAGCAGATCCA
AATAGAATGTACTGGATTGAGTTAATTTTGGCAAAAGAAGGTGCACCAAAAGTA
CTGAAAAAAATGGGCAAACTATTTGCTGAACAAAATAAATCATAAtacgcatggcatgga
tgaattgtataaataa
**SEQ ID NO: 75** Tet-kivD-leuDH construct: 
gaattcgTTAAGACCCACTTTCACATTTAAGTTGTTTTTCTAATCCGCATATGATCAAT
TCAAGGCCGAATAAGAAGGCTGGCTCTGCACCTTGGTGATCAAATAATTCGATA
GCTTGTCGTAATAATGGCGGCATACTATCAGTAGTAGGTGTTTCCCTTTCTTCTTT
AGCGACTTGATGCTCTTGATCTTCCAATACGCAACCTAAAGTAAAATGCCCCACA
GCGCTGAGTGCATATAATGCATTCTCTAGTGAAAAACCTTGTTGGCATAAAAAGG
CTAATTGATTTTCGAGAGTTTCATACTGTTTTTCTGTAGGCCGTGTACCTAAATGT
ACTTTTGCTCCATCGCGATGACTTAGTAAAGCACATCTAAAACTTTTAGCGTTATT
ACGTAAAAAATCTTGCCAGCTTTCCCCTTCTAAAGGGCAAAAGTGAGTATGGTGC
CTATCTAACATCTCAATGGCTAAGGCGTCGAGCAAAGCCCGCTTATTTTTACAT
GCCAATACAATGTAGGCTGCTCTACACCTAGCTTCTGGGCGAGTTTACGGGTTGT
TAAACCTTCGATTCCGACCTCATTAAGCAGCTCTAATGCGCTGTTAATCACTTTAC
TTTTATCTAATCTAGACATcattaattcctaatttttgttgacactctatcattgatagagttattttaccactccctatcagtg
 
atagagaaaagtgaactctagaaataattttgtttaactttaagaaggagatatacatATGTATACAGTAGGAGATTA
CCTATTAGACCGATTACACGAGTTAGGAATTGAAGAAATTTTTGGAGTCCCTGGA
GACTATAACTTACAATTTTTAGATCAAATTATTTCCCACAAGGATATGAAATGGG
TCGGAAATGCTAATGAATTAAATGCTTCATATATGGCTGATGGCTATGCTCGTAC
TAAAAAAGCTGCCGCATTTCTTACAACCTTTGGAGTAGGTGAATTGAGTGCAGTT
AATGGATTAGCAGGAAGTTACGCCGAAAATTTACCAGTAGTAGAAATAGTGGGA
TCACCTACATCAAAAGTTCAAAATGAAGGAAAATTTGTTCATCATACGCTGGCTG
ACGGTGATTTTAAACACTTTATGAAAATGCACGAACCTGTTACAGCAGCTCGAAC
TTTACTGACAGCAGAAAATGCAACCGTTGAAATTGACCGAGTACTTTCTGCACTA
TTAAAAGAAAGAAAACCTGTCTATATCAACTTACCAGTTGATGTTGCTGCTGCAA
AAGCAGAGAAACCCTCACTCCCTTTGAAAAAGGAAAACTCAACTTCAAATACAA
GTGACCAAGAAATTTTGAACAAAATTCAAGAAAGCTTGAAAAATGCCAAAAAAC
CAATCGTGATTACAGGACATGAAATAATTAGTTTTGGCTTAGAAAAAACAGTCAC
TCAATTTATTTCAAAGACAAAACTACCTATTACGACATTAAACTTTGGTAAAAGT
TCAGTTGATGAAGCCCTCCCTTCATTTTTAGGAATCTATAATGGTACACTCTCAGA
GCCTAATCTTAAAGAATTCGTGGAATCAGCCGACTTCATCTTGATGCTTGGAGTT
AAACTCACAGACTCTTCAACAGGAGCCTTCACTCATCATTTAAATGAAAATAAAA
TGATTTCACTGAATATAGATGAAGGAAAAATATTTAACGAAAGAATCCAAAATT
TTGATTTTGAATCCCTCATCTCCTCTCTCTTAGACCTAAGCGAAATAGAATACAA
AGGAAAATATATCGATAAAAAGCAAGAAGACTTTGTTCCATCAAATGCGCTTTTA
TCACAAGACCGCCTATGGCAAGCAGTTGAAAACCTAACTCAAAGCAATGAAACA
ATCGTTGCTGAACAAGGGACATCATTCTTTGGCGCTTCATCAATTTTCTTAAAATC
AAAGAGTCATTTTATTGGTCAACCCTTATGGGGATCAATTGGATATACATTCCCA
GCAGCATTAGGAAGCCAAATTGCAGATAAAGAAAGCAGACACCTTTTATTTATT
GGTGATGGTTCACTTCAACTTACAGTGCAAGAATTAGGATTAGCAATCAGAGAA
AAAATTAATCCAATTTGCTTTATTATCAATAATGATGGTTATACAGTCGAAAGAG
AAATTCATGGACCAAATCAAAGCTACAATGATATTCCAATGTGGAATTACTCAAA
ATTACCAGAATCGTTTGGAGCAACAGAAGATCGAGTAGTCTCAAAAATCGTTAG
AACTGAAAATGAATTTGTGTCTGTCATGAAAGAAGCTCAAGCAGATCCAAATAG
AATGTACTGGATTGAGTTAATTTTGGCAAAAGAAGGTGCACCAAAAGTACTGAA
AAAAATGGGCAAACTATTTGCTGAACAAAATAAATCATAAgaaggagatatacatATGTT
CGACATGATGGATGCAGCCCGCCTGGAAGGCCTGCACCTCGCCCAGGATCCAGC
GACGGGCCTGAAAGCGATCATCGCGATCCATTCCACTCGCCTCGGCCCGGCCTTA
GGCGGCTGTCGTTACCTCCCATATCCGAATGATGAAGCGGCCATCGGCGATGCCA
TTCGCCTGGCGCAGGGCATGTCCTACAAAGCTGCACTTGCGGGTCTGGAACAAG
GTGGTGGCAAGGCGGTGATCATTCGCCCACCCCACTTGGATAATCGCGGTGCCTT
GTTTGAAGCGTTTGGACGCTTTATTGAAAGCCTGGGTGGCCGTTATATCACCGCC
GTTGACTCAGGAACAAGTAGTGCCGATATGGATTGCATCGCCCAACAGACGCGC
CATGTGACTTCAACGACACAAGCCGGCGACCCATCTCCACATACGGCTCTGGGC
GTCTTTGCCGGCATCCGCGCCTCCGCGCAGGCTCGCCTGGGGTCCGATGACCTGG
AAGGCCTGCGTGTCGCGGTTCAGGGCCTTGGCCACGTAGGTTATGCGTTAGCGGA
GCAGCTGGCGGCGGTCGGCGCAGAACTGCTGGTGTGCGACCTGGACCCCGGCCG
CGTCCAGTTAGCGGTGGAGCAACTGGGGGCGCACCCACTGGCCCCTGAAGCATT
GCTCTCTACTCCGTGCGACATTTTAGCGCCTTGTGGCCTGGGCGGCGTGCTCACC
AGCCAGTCGGTGTCACAGTTGCGCTGCGCGGCCGTTGCAGGCGCAGCGAACAAT
CAACTGGAGCGCCCGGAAGTTGCAGACGAACTGGAGGCGCGCGGATTTATAT
GCGCCCGATTACGTGATTAACTCGGGAGGACTGATTTATGTGGCGCTGAAGCATC
GCGGTGCTGATCCGCATAGCATTACCGCCCACCTCGCTCGCATCCCTGCACGCCT
GACGGAAATCTATGCGCATGCGCAGGCGGATCATCAGTCGCCTGCGCGCATCGC
CGATCGTCTGGCGGAGCGCATTCTGTACGGCCCGCAGTGA
SEQ ID NO: 76 Tet-kivD-adh2 construct: 
gaattcgTTAAGACCCACTTTCACATTTAAGTTGTTTTCTAATCCGCATATGATCAAT
TCAAGGCCGAATAAGAAGGCTGGCTCTGCACCTTGGTGATCAAATAATTCGATA
GCTTGTCGTAATAATGGCGGCATACTATCAGTAGTAGGTGTTTCCCTTTCTTCTTT
AGCGACTTGATGCTCTTGATCTTCCAATACGCAACCTAAAGTAAAATGCCCCACA
GCGCTGAGTGCATATAATGCATTCTCTAGTGAAAAACCTTGTTGGCATAAAAAGG
CTAATTGATTTTCGAGAGTTTCATACTGTTTTTCTGTAGGCCGTGTACCTAAATGT
ACTTTTGCTCCATCGCGATGACTTAGTAAAGCACATCTAAAACTTTTAGCGTTATT
ACGTAAAAAATCTTGCCAGCTTTCCCCTTCTAAAGGGCAAAAGTGAGTATGGTGC
CTATCTAACATCTCAATGGCTAAGGCGTCGAGCAAAGCCCGCTTATTTTTTACAT
GCCAATACAATGTAGGCTGCTCTACACCTAGCTTCTGGGCGAGTTTACGGGTTGT
TAAACCTTCGATTCCGACCTCATTAAGCAGCTCTAATGCGCTGTTAATCACTTTAC
TTTTATCTAATCTAGACATcattaattcctaatttttgttgacactctatcattgatagagttattttaccactccctatcagtg
atagagaaaagtgaactctagaaataattttgtttaactttaagaaggagatatacatATGTATACAGTAGGAGATTA
CCTATTAGACCGATTACACGAGTTAGGAATTGAAGAAATTTTTGGAGTCCCTGGA
GACTATAACTTACAATTTTTAGATCAAATTATTTCCCACAAGGATATGAAATGGG
TCGGAAATGCTAATGAATTAAATGCTTCATATATGGCTGATGGCTATGCTCGTAC
TAAAAAAGCTGCCGCATTTCTTACAACCTTTGGAGTAGGTGAATTGAGTGCAGTT
AATGGATTAGCAGGAAGTTACGCCGAAAATTTACCAGTAGTAGAAATAGTGGGA
TCACCTACATCAAAAGTTCAAAATGAAGGAAAATTTGTTCATCATACGCTGGCTG
ACGGTGATTTTAAACACTTTATGAAAATGCACGAACCTGTTACAGCAGCTCGAAC
TTTACTGACAGCAGAAAATGCAACCGTTGAAATTGACCGAGTACTTTCTGCACTA
TTAAAAGAAAGAAAACCTGTCTATATCAACTTACCAGTTGATGTTGCTGCTGCAA
AAGCAGAGAAACCCTCACTCCCTTTGAAAAAGGAAAACTCAACTTCAAATACAA
GTGACCAAGAAATTTTGAACAAAATTCAAGAAAGCTTGAAAAATGCCAAAAAAC
CAATCGTGATTACAGGACATGAAATAATTAGTTTTGGCTTAGAAAAAACAGTCAC
TCAATTTATTTCAAAGACAAAACTACCTATTACGACATTAAACTTTGGTAAAAGT
TCAGTTGATGAAGCCCTCCCTTCATTTTTAGGAATCTATAATGGTACACTCTCAGA
GCCTAATCTTAAAGAATTCGTGGAATCAGCCGACTTCATCTTGATGCTTGGAGTT
AAACTCACAGACTCTTCAACAGGAGCCTTCACTCATCATTTAAATGAAAATAAAA
TGATTTCACTGAATATAGATGAAGGAAAAATATTTAACGAAAGAATCCAAAATT
TTGATTTTGAATCCCTCATCTCCTCTCTCTTAGACCTAAGCGAAATAGAATACAA
AGGAAAATATATCGATAAAAAGCAAGAAGACTTTGTTCCATCAAATGCGCTTTTA
TCACAAGACCGCCTATGGCAAGCAGTTGAAAACCTAACTCAAAGCAATGAAACA
ATCGTTGCTGAACAAGGGACATCATTCTTTGGCGCTTCATCAATTTTCTTAAAATC
AAAGAGTCATTTTATTGGTCAACCCTTATGGGGATCAATTGGATATACATTCCCA
GCAGCATTAGGAAGCCAAATTGCAGATAAAGAAAGCAGACACCTTTTATTTATT
GGTGATGGTTCACTTCAACTTACAGTGCAAGAATTAGGATTAGCAATCAGAGAA
AAAATTAATCCAATTTGCTTTATTATCAATAATGATGGTTATACAGTCGAAAGAG
AAATTCATGGACCAAATCAAAGCTACAATGATATTCCAATGTGGAATTACTCAAA
ATTACCAGAATCGTTTGGAGCAACAGAAGATCGAGTAGTCTCAAAAATCGTTAG
AACTGAAAATGAATTTGTGTCTGTCATGAAAGAAGCTCAAGCAGATCCAAATAG
AATGTACTGGATTGAGTTAATTTTGGCAAAAGAAGGTGCACCAAAAGTACTGAA
AAAAATGGGCAAACTATTTGCTGAACAAAATAAATCATAAtaagaaggagatatacatATG
TCTATTCCAGAAACTCAAAAAGCCATTATCTTCTACGAATCCAACGGCAAGTTGG
AGCATAAGGATATCCCAGTTCCAAAGCCAAAGCCCAACGAATTGTTAATCAACG
TCAAGTACTCTGGTGTCTGCCACACCGATTTGCACGCTTGGCATGGTGACTGGCC
ATTGCCAACTAAGTTACCATTAGTTGGTGGTCACGAAGGTGCCGGTGTCGTTGTC
 
GGCATGGGTGAAAACGTTAAGGGCTGGAAGATCGGTGACTACGCCGGTATCAAA
TGGTTGAACGGTTCTTGTATGGCCTGTGAATACTGTGAATTGGGTAACGAATCCA
ACTGTCCTCACGCTGACTTGTCTGGTTACACCCACGACGGTTCTTTCCAAGAATA
CGCTACCGCTGACGCTGTTCAAGCCGCTCACATTCCTCAAGGTACTGACTTGGCT
GAAGTCGCGCCAATCTTGTGTGCTGGTATCACCGTATACAAGGCTTTGAAGTCTG
CCAACTTGAGAGCAGGCCACTGGGCGGCCATTTCTGGTGCTGCTGGTGGTCTAGG
TTCTTTGGCTGTTCAATATGCTAAGGCGATGGGTTACAGAGTCTTAGGTATTGAT
GGTGGTCCAGGAAAGGAAGAATTGTTTACCTCGCTCGGTGGTGAAGTATTCATCG
ACTTCACCAAAGAGAAGGACATTGTTAGCGCAGTCGTTAAGGCTACCAACGGCG
GTGCCCACGGTATCATCAATGTTTCCGTTTCCGAAGCCGCTATCGAAGCTTCTAC
CAGATACTGTAGGGCGAACGGTACTGTTGTCTTGGTTGGTTTGCCAGCCGGTGCA
AAGTGCTCCTCTGATGTCTTCAACCACGTTGTCAAGTCTATCTCCATTGTCGGCTC
TTACGTGGGGAACAGAGCTGATACCAGAGAAGCCTTAGATTTCTTTGCCAGAGGT
CTAGTCAAGTCTCCAATAAAGGTAGTTGGCTTATCCAGTTTACCAGAAATTTACG
AAAAGATGGAGAAGGGCCAAATTGCTGGTAGATACGTTGTTGACACTTCTAAAT
AA
SEQ ID NO: 77 Tet-leuDH-kivD-adh2 construct 
gaattcgTTAAGACCCACTTTCACATTTAAGTTGTTTTTCTAATCCGCATATGATCAAT
TCAAGGCCGAATAAGAAGGCTGGCTCTGCACCTTGGTGATCAAATAATTCGATA
GCTTGTCGTAATAATGGCGGCATACTATCAGTAGTAGGTGTTTCCCTTTCTTCTTT
AGCGACTTGATGCTCTTGATCTTCCAATACGCAACCTAAAGTAAAATGCCCCACA
GCGCTGAGTGCATATAATGCATTCTCTAGTGAAAAACCTTGTTGGCATAAAAAGG
CTAATTGATTTTCGAGAGTTTCATACTGTTTTTCTGTAGGCCGTGTACCTAAATGT
ACTTTTGCTCCATCGCGATGACTTAGTAAAGCACATCTAAAACTTTTAGCGTTATT
ACGTAAAAAATCTTGCCAGCTTTCCCCTTCTAAAGGGCAAAAGTGAGTATGGTGC
CTATCTAACATCTCAATGGCTAAGGCGTCGAGCAAAGCCCGCTTATTTTTTACAT
GCCAATACAATGTAGGCTGCTCTACACCTAGCTTCTGGGCGAGTTTACGGGTTGT
TAAACCTTCGATTCCGACCTCATTAAGCAGCTCTAATGCGCTGTTAATCACTTTAC
TTTTATCTAATCTAGACATcattaattcctaatttttgttgacactctatcattgatagagttattttaccactccctatcagtg
atagagaaaagtgaactctagaaataattttgtttaactttaagaaggagatatacatATGTTCGACATGATGGATGC
AGCCCGCCTGGAAGGCCTGCACCTCGCCCAGGATCCAGCGACGGCCTGAAAGC
GATCATCGCGATCCATTCCACTCGCCTCGGCCCGGCCTTAGGCGGCTGTCGTTAC
CTCCCATATCCGAATGATGAAGCGGCCATCGGCGATGCCATTCGCCTGGCGCAG
GGCATGTCCTACAAAGCTGCACTTGCGGGTCTGGAACAAGGTGGTGGCAAGGCG
GTGATCATTCGCCCACCCCACTTGGATAATCGCGGTGCCTTGTTTGAAGCGTTTG
GACGCTTTATTGAAAGCCTGGGTGGCCGTTATATCACCGCCGTTGACTCAGGAAC
AAGTAGTGCCGATATGGATTGCATCGCCCAACAGACGCGCCATGTGACTTCAAC
GACACAAGCCGGCGACCCATCTCCACATACGGCTCTGGGCGTCTTTGCCGGCATC
CGCGCCTCCGCGCAGGCTCGCCTGGGGTCCGATGACCTGGAAGGCCTGCGTGTC
GCGGTTCAGGGCCTTGGCCACGTAGGTTATGCGTTAGCGGAGCAGCTGGCGGCG
GTCGGCGCAGAACTGCTGGTGTGCGACCTGGACCCCGGCCGCGTCCAGTTAGCG
GTGGAGCAACTGGGGGCGCACCCACTGGCCCCTGAAGCATTGCTCTCTACTCCGT
GCGACATTTTAGCGCCTTGTGGCCTGGGCGGCGTGCTCACCAGCCAGTCGGTGTC
ACAGTTGCGCTGCGCGGCCGTTGCAGGCGCAGCGAACAATCAACTGGAGCGCCC
GGAAGTTGCAGACGAACTGGAGGCGCGCGGATTTATATGCGCCCGATTACGT
GATTAACTCGGGAGGACTGATTTATGTGGCGCTGAAGCATCGCGGTGCTGATCCG
CATAGCATTACCGCCCACCTCGCTCGCATCCCTGCACGCCTGACGGAAATCTATG
CGCATGCGCAGGCGGATCATCAGTCGCCTGCGCGCATCGCCGATCGTCTGGCGG
 
AGCGCATTCTGTACGGCCCGCAGTGAtaagaaggagatatacatATGTATACAGTAGGAGA
TTACCTATTAGACCGATTACACGAGTTAGGAATTGAAGAAATTTTTGGAGTCCCT
GGAGACTATAACTTACAATTTTTAGATCAAATTATTTCCCACAAGGATATGAAAT
GGGTCGGAAATGCTAATGAATTAAATGCTTCATATATGGCTGATGGCTATGCTCG
TACTAAAAAAGCTGCCGCATTTCTTACAACCTTTGGAGTAGGTGAATTGAGTGCA
GTTAATGGATTAGCAGGAAGTTACGCCGAAAATTTACCAGTAGTAGAAATAGTG
GGATCACCTACATCAAAAGTTCAAAATGAAGGAAAATTTGTTCATCATACGCTGG
CTGACGGTGATTTTAAACACTTTATGAAAATGCACGAACCTGTTACAGCAGCTCG
AACTTTACTGACAGCAGAAAATGCAACCGTTGAAATTGACCGAGTACTTTCTGCA
CTATTAAAAGAAAGAAAACCTGTCTATATCAACTTACCAGTTGATGTTGCTGCTG
CAAAAGCAGAGAAACCCTCACTCCCTTTGAAAAAGGAAAACTCAACTTCAAATA
CAAGTGACCAAGAAATTTTGAACAAAATTCAAGAAAGCTTGAAAAATGCCAAAA
AACCAATCGTGATTACAGGACATGAAATAATTAGTTTTGGCTTAGAAAAAACAG
TCACTCAATTTATTTCAAAGACAAAACTACCTATTACGACATTAAACTTTGGTAA
AAGTTCAGTTGATGAAGCCCTCCCTTCATTTTTAGGAATCTATAATGGTACACTCT
CAGAGCCTAATCTTAAAGAATTCGTGGAATCAGCCGACTTCATCTTGATGCTTGG
AGTTAAACTCACAGACTCTTCAACAGGAGCCTTCACTCATCATTTAAATGAAAAT
AAAATGATTTCACTGAATATAGATGAAGGAAAAATATTTAACGAAAGAATCCAA
AATTTTGATTTTGAATCCCTCATCTCCTCTCTCTTAGACCTAAGCGAAATAGAATA
CAAAGGAAAATATATCGATAAAAAGCAAGAAGACTTTGTTCCATCAAATGCGCT
TTTATCACAAGACCGCCTATGGCAAGCAGTTGAAAACCTAACTCAAAGCAATGA
AACAATCGTTGCTGAACAAGGGACATCATTCTTTGGCGCTTCATCAATTTTCTTA
AAATCAAAGAGTCATTTTATTGGTCAACCCTTATGGGGATCAATTGGATATACAT
TCCCAGCAGCATTAGGAAGCCAAATTGCAGATAAAGAAAGCAGACACCTTTTAT
TTATTGGTGATGGTTCACTTCAACTTACAGTGCAAGAATTAGGATTAGCAATCAG
AGAAAAAATTAATCCAATTTGCTTTATTATCAATAATGATGGTTATACAGTCGAA
AGAGAAATTCATGGACCAAATCAAAGCTACAATGATATTCCAATGTGGAATTAC
TCAAAATTACCAGAATCGTTTGGAGCAACAGAAGATCGAGTAGTCTCAAAAATC
GTTAGAACTGAAAATGAATTTGTGTCTGTCATGAAAGAAGCTCAAGCAGATCCA
AATAGAATGTACTGGATTGAGTTAATTTTGGCAAAAGAAGGTGCACCAAAAGTA
CTGAAAAAAATGGGCAAACTATTTGCTGAACAAAATAAATCATAAtaagaaggagatata
catATGTCTATTCCAGAAACTCAAAAAGCCATTATCTTCTACGAATCCAACGGCAA
GTTGGAGCATAAGGATATCCCAGTTCCAAAGCCAAAGCCCAACGAATTGTTAAT
CAACGTCAAGTACTCTGGTGTCTGCCACACCGATTTGCACGCTTGGCATGGTGAC
TGGCCATTGCCAACTAAGTTACCATTAGTTGGTGGTCACGAAGGTGCCGGTGTCG
TTGTCGGCATGGGTGAAAACGTTAAGGGCTGGAAGATCGGTGACTACGCCGGTA
TCAAATGGTTGAACGGTTCTTGTATGGCCTGTGAATACTGTGAATTGGGTAACGA
ATCCAACTGTCCTCACGCTGACTTGTCTGGTTACACCCACGACGGTTCTTTCCAAG
AATACGCTACCGCTGACGCTGTTCAAGCCGCTCACATTCCTCAAGGTACTGACTT
GGCTGAAGTCGCGCCAATCTTGTGTGCTGGTATCACCGTATACAAGGCTTTGAAG
TCTGCCAACTTGAGAGCAGGCCACTGGGCGGCCATTTCTGGTGCTGCTGGTGGTC
TAGGTTCTTTGGCTGTTCAATATGCTAAGGCGATGGGTTACAGAGTCTTAGGTAT
TGATGGTGGTCCAGGAAAGGAAGAATTGTTTACCTCGCTCGGTGGTGAAGTATTC
ATCGACTTCACCAAAGAGAAGGACATTGTTAGCGCAGTCGTTAAGGCTACCAAC
GGCGGTGCCCACGGTATCATCAATGTTTCCGTTTCCGAAGCCGCTATCGAAGCTT
CTACCAGATACTGTAGGGCGAACGGTACTGTTGTCTTGGTTGGTTTGCCAGCCGG
TGCAAAGTGCTCCTCTGATGTCTTCAACCACGTTGTCAAGTCTATCTCCATTGTCG
GCTCTTACGTGGGGAACAGAGCTGATACCAGAGAAGCCTTAGATTTCTTTGCCAG
AGGTCTAGTCAAGTCTCCAATAAAGGTAGTTGGCTTATCCAGTTTACCAGAAATT
 
TACGAAAAGATGGAGAAGGGCCAAATTGCTGGTAGATACGTTGTTGACACTTCT
AAATAAtacgcatggcatggatgaa
SEQ ID NO: 78 Tet-ilvE-kivD-adh2 construct: 
gaattcgTTAAGACCCACTTTCACATTTAAGTTGTTTTTCTAATCCGCATATGATCAAT
TCAAGGCCGAATAAGAAGGCTGGCTCTGCACCTTGGTGATCAAATAATTCGATA
GCTTGTCGTAATAATGGCGGCATACTATCAGTAGTAGGTGTTTCCCTTTCTTCTTT
AGCGACTTGATGCTCTTGATCTTCCAATACGCAACCTAAAGTAAAATGCCCCACA
GCGCTGAGTGCATATAATGCATTCTCTAGTGAAAAACCTTGTTGGCATAAAAAGG
CTAATTGATTTTCGAGAGTTTCATACTGTTTTTCTGTAGGCCGTGTACCTAAATGT
ACTTTTGCTCCATCGCGATGACTTAGTAAAGCACATCTAAAACTTTTAGCGTTATT
ACGTAAAAAATCTTGCCAGCTTTCCCCTTCTAAAGGGCAAAAGTGAGTATGGTGC
CTATCTAACATCTCAATGGCTAAGGCGTCGAGCAAAGCCCGCTTATTTTTTACAT
GCCAATACAATGTAGGCTGCTCTACACCTAGCTTCTGGGCGAGTTTACGGGTTGT
TAAACCTTCGATTCCGACCTCATTAAGCAGCTCTAATGCGCTGTTAATCACTTTAC
TTTTATCTAATCTAGACATcattaattcctaatttttgttgacactctatcattgatagagttattttaccactccctatcagtg
atagagaaaagtgaactctagaaataattttgtttaactttaagaaggagatatacatATGACCACGAAGAAAGCTGA
TTACATTTGGTTCAATGGGGAGATGGTTCGCTGGGAAGACGCGAAGGTGCATGT
GATGTCGCACGCGCTGCACTATGGCACCTCGGTTTTTGAAGGCATCCGTTGCTAC
GACTCGCACAAAGGACCGGTTGTATTCCGCCATCGTGAGCATATGCAGCGTCTGC
ATGACTCCGCCAAAATCTATCGCTTCCCGGTTTCGCAGAGCATTGATGAGCTGAT
GGAAGCTTGTCGTGACGTGATCCGCAAAAACAATCTCACCAGCGCCTATATCCGT
CCGCTGATCTTCGTTGGTGATGTTGGCATGGGCGTAAACCCGCCAGCGGGATACT
CAACCGACGTGATTATCGCCGCTTTCCCGTGGGGAGCGTATCTGGGCGCAGAAGC
GCTGGAGCAGGGGATCGATGCGATGGTTTCCTCCTGGAACCGCGCAGCACCAAA
CACCATCCCGACGGCGGCAAAAGCCGGTGGTAACTACCTCTCTTCCCTGCTGGTG
GGTAGCGAAGCGCGCCGCCACGGTTATCAGGAAGGTATCGCGTTGGATGTGAAT
GGTTACATCTCTGAAGGCGCAGGCGAAAACCTGTTTGAAGTGAAAGACGGCGTG
CTGTTCACCCCACCGTTCACCTCATCCGCGCTGCCGGGTATTACCCGTGATGCCA
TCATCAAACTGGCAAAAGAGCTGGGAATTGAAGTGCGTGAGCAGGTGCTGTCGC
GCGAATCCCTGTACCTGGCGGATGAAGTGTTTATGTCCGGTACGGCGGCAGAAAT
CACGCCAGTGCGCAGCGTAGACGGTATTCAGGTTGGCGAAGGCCGTTGTGGCCC
GGTTACCAAACGCATTCAGCAAGCCTTCTTCGGCCTCTTCACTGGCGAAACCGAA
GATAAATGGGGCTGGTTAGATCAAGTTAATCAATAAtaagaaggagatatacatATGTATA
CAGTAGGAGATTACCTATTAGACCGATTACACGAGTTAGGAATTGAAGAAATTTT
TGGAGTCCCTGGAGACTATAACTTACAATTTTTAGATCAAATTATTTCCCACAAG
GATATGAAATGGGTCGGAAATGCTAATGAATTAAATGCTTCATATATGGCTGATG
GCTATGCTCGTACTAAAAAAGCTGCCGCATTTCTTACAACCTTTGGAGTAGGTGA
ATTGAGTGCAGTTAATGGATTAGCAGGAAGTTACGCCGAAAATTTACCAGTAGT
AGAAATAGTGGGATCACCTACATCAAAAGTTCAAAATGAAGGAAAATTTGTTCA
TCATACGCTGGCTGACGGTGATTTTAAACACTTTATGAAAATGCACGAACCTGTT
ACAGCAGCTCGAACTTTACTGACAGCAGAAAATGCAACCGTTGAAATTGACCGA
GTACTTTCTGCACTATTAAAAGAAAGAAAACCTGTCTATATCAACTTACCAGTTG
ATGTTGCTGCTGCAAAAGCAGAGAAACCCTCACTCCCTTTGAAAAAGGAAAACT
CAACTTCAAATACAAGTGACCAAGAAATTTTGAACAAAATTCAAGAAAGCTTGA
AAAATGCCAAAAAACCAATCGTGATTACAGGACATGAAATAATTAGTTTTGGCTT
AGAAAAAACAGTCACTCAATTTATTTCAAAGACAAAACTACCTATTACGACATTA
AACTTTGGTAAAAGTTCAGTTGATGAAGCCCTCCCTTCATTTTTAGGAATCTATA
ATGGTACACTCTCAGAGCCTAATCTTAAAGAATTCGTGGAATCAGCCGACTTCAT
 
CTTGATGCTTGGAGTTAAACTCACAGACTCTTCAACAGGAGCCTTCACTCATCAT
TTAAATGAAAATAAAATGATTTCACTGAATATAGATGAAGGAAAAATATTTAAC
GAAAGAATCCAAAATTTTGATTTTGAATCCCTCATCTCCTCTCTCTTAGACCTAAG
CGAAATAGAATACAAAGGAAAATATATCGATAAAAAGCAAGAAGACTTTGTTCC
ATCAAATGCGCTTTTATCACAAGACCGCCTATGGCAAGCAGTTGAAAACCTAACT
CAAAGCAATGAAACAATCGTTGCTGAACAAGGGACATCATTCTTTGGCGCTTCAT
CAATTTTCTTAAAATCAAAGAGTCATTTTATTGGTCAACCCTTATGGGGATCAATT
GGATATACATTCCCAGCAGCATTAGGAAGCCAAATTGCAGATAAAGAAAGCAGA
CACCTTTTATTTATTGGTGATGGTTCACTTCAACTTACAGTGCAAGAATTAGGATT
AGCAATCAGAGAAAAAATTAATCCAATTTGCTTTATTATCAATAATGATGGTTAT
ACAGTCGAAAGAGAAATTCATGGACCAAATCAAAGCTACAATGATATTCCAATG
TGGAATTACTCAAAATTACCAGAATCGTTTGGAGCAACAGAAGATCGAGTAGTC
TCAAAAATCGTTAGAACTGAAAATGAATTTGTGTCTGTCATGAAAGAAGCTCAA
GCAGATCCAAATAGAATGTACTGGATTGAGTTAATTTTGGCAAAAGAAGGTGCA
CCAAAAGTACTGAAAAAAATGGGCAAACTATTTGCTGAACAAAATAAATCATAAt
aagaaggagatatacatATGTCTATTCCAGAAACTCAAAAAGCCATTATCTTCTACGAATC
CAACGGCAAGTTGGAGCATAAGGATATCCCAGTTCCAAAGCCAAAGCCCAACGA
ATTGTTAATCAACGTCAAGTACTCTGGTGTCTGCCACACCGATTTGCACGCTTGG
CATGGTGACTGGCCATTGCCAACTAAGTTACCATTAGTTGGTGGTCACGAAGGTG
CCGGTGTCGTTGTCGGCATGGGTGAAAACGTTAAGGGCTGGAAGATCGGTGACT
ACGCCGGTATCAAATGGTTGAACGGTTCTTGTATGGCCTGTGAATACTGTGAATT
GGGTAACGAATCCAACTGTCCTCACGCTGACTTGTCTGGTTACACCCACGACGGT
TCTTTCCAAGAATACGCTACCGCTGACGCTGTTCAAGCCGCTCACATTCCTCAAG
GTACTGACTTGGCTGAAGTCGCGCCAATCTTGTGTGCTGGTATCACCGTATACAA
GGCTTTGAAGTCTGCCAACTTGAGAGCAGGCCACTGGGCGGCCATTTCTGGTGCT
GCTGGTGGTCTAGGTTCTTTGGCTGTTCAATATGCTAAGGCGATGGGTTACAGAG
TCTTAGGTATTGATGGTGGTCCAGGAAAGGAAGAATTGTTTACCTCGCTCGGTGG
TGAAGTATTCATCGACTTCACCAAAGAGAAGGACATTGTTAGCGCAGTCGTTAAG
GCTACCAACGGCGGTGCCCACGGTATCATCAATGTTTCCGTTTCCGAAGCCGCTA
TCGAAGCTTCTACCAGATACTGTAGGGCGAACGGTACTGTTGTCTTGGTTGGTTT
GCCAGCCGGTGCAAAGTGCTCCTCTGATGTCTTCAACCACGTTGTCAAGTCTATC
TCCATTGTCGGCTCTTACGTGGGGAACAGAGCTGATACCAGAGAAGCCTTAGATT
TCTTTGCCAGAGGTCTAGTCAAGTCTCCAATAAAGGTAGTTGGCTTATCCAGTTT
ACCAGAAATTTACGAAAAGATGGAGAAGGGCCAAATTGCTGGTAGATACGTTGT
TGACACTTCTAAATAAtacgcatggcatggatgaa
SEQ3: Tet-bkd construct sequence 
gtaaaacgacggccagtgaattcgTTAAGACCCACTTTCACATTTAAGTTGTTTTCTAATCCGC
ATATGATCAATTCAAGGCCGAATAAGAAGGCTGGCTCTGCACCTTGGTGATCAA
ATAATTCGATAGCTTGTCGTAATAATGGCGGCATACTATCAGTAGTAGGTGTTTC
CCTTTCTTCTTTAGCGACTTGATGCTCTTGATCTTCCAATACGCAACCTAAAGTAA
AATGCCCCACAGCGCTGAGTGCATATAATGCATTCTCTAGTGAAAAACCTTGTTG
GCATAAAAAGGCTAATTGATTTTCGAGAGTTTCATACTGTTTTTCTGTAGGCCGT
GTACCTAAATGTACTTTTGCTCCATCGCGATGACTTAGTAAAGCACATCTAAAAC
TTTTAGCGTTATTACGTAAAAAATCTTGCCAGCTTTCCCCTTCTAAAGGGCAAAA
GTGAGTATGGTGCCTATCTAACATCTCAATGGCTAAGGCGTCGAGCAAAGCCCGC
TTATTTTTTACATGCCAATACAATGTAGGCTGCTCTACACCTAGCTTCTGGGCGAG
TTTACGGGTTGTTAAACCTTCGATTCCGACCTCATTAAGCAGCTCTAATGCGCTGT
TAATCACTTTACTTTTATCTAATCTAGACATcattaattcctaatttttgttgacactctatcattgatagagtta
 
ttttaccactccctatcagtgatagagaaaagtgaactctagaaataattttgtttaactttaagaaggagatatacatATGTCCGAC
TACGAGCCACTCCGCTTGCACGTGCCGGAGCCGACAGGTCGTCCCGGCTGCAAA
ACGGATTTCTCTTACCTGCACTTATCTCCCGCAGGTGAAGTCCGCAAACCGCCTG
TCGACGTGGAGCCTGCAGAAACCAGCGATTTGGCATATTCGCTGGTGCGTGTGCT
CGATGATGATGGACATGCAGTGGGTCCGTGGAATCCGCAGCTCTCAAACGAACA
GCTGCTGCGTGGAATGCGCGCGATGCTGAAGACGCGTCTGTTCGATGCTCGCATG
TTGACTGCGCAGCGCCAAAAAAAATTGAGTTTTATATGCAGTGCTTAGGAGAAG
AGGCAATCGCGACTGCCCATACACTGGCCCTGCGCGATGGTGATATGTGTTTTCC
GACGTACCGTCAGCAGGGGATTCTTATTACACGTGAGTATCCGCTTGTGGATATG
ATCTGCCAGCTGCTGTCGAATGAAGCGGACCCCCTGAAAGGCCGTCAACTGCCG
ATCATGTACAGCAGTAAGGAGGCTGGCTTCTTTAGCATCTCGGGCAATCTTGCGA
CTCAGTTTATTCAGGCGGTGGGGTGGGGGATGGCAAGCGCAATCAAAGGGGATA
CCCGCATTGCATCCGCATGGATTGGCGATGGCGCTACCGCGGAAAGCGATTTTCA
TACGGCGCTGACCTTTGCTCACGTTTATCGCGCACCGGTGATCCTCAATGTGGTC
AACAACCAGTGGGCGATTTCGACGTTTCAGGCCATCGCGGGCGGCGAGGGCACC
ACGTTCGCGAACCGTGGCGTGGGTTGCGGCATTGCGAGCCTCCGTGTGGACGGG
AACGATTTTTGGCCGTGTATGCGGCGAGCGAATGGGCGGCAGAACGCGCACGC
CGTAACTTGGGACCGTCCCTGATCGAATGGGTAACTTATCGCGCGGGCCCACACA
GCACGAGCGACGATCCGTCAAAGTATCGCCCTGCGGATGATTGGACCAATTTCC
GCTGGGTGACCCGATTGCGCGTCTGAAACGTCACATGATCGGTTTGGGTATTTGG
AGCGAAGAACAGCACGAAGCTACGCACAAAGCGCTGGAAGCGGAAGTCCTGGC
GGCGCAGAAGCAGGCCGAAAGCCATGGCACTCTGATTGACGGCCGTGTGCCGTC
TGCAGCCTCTATGTTCGAAGATGTTTATGCCGAGTTACCCGAGCACTTACGTCGC
CAGCGCCAGGAGCTCGGGGTATGAACGCCATGAACCCGCAGCATGAAAACGCGC
AAACCGTGACCTCCATGACGATGATTCAGGCCCTGCGCTCGGCGATGGATATTAT
GTTAGAACGTGACGATGACGTCGTGGTGTTTGGTCAGGACGTAGGGTATTTTGGG
GGAGTGTTTCGTTGTACCGAGGGGTTGCAAAAGAAGTATGGTACGAGTCGCGTCT
TCGATGCACCGATCAGCGAATCAGGCATTATCGGCGCTGCCGTGGGCATGGGTG
CATATGGCTTGCGCCCTGTGGTTGAAATTCAGTTTGCAGATTATGTATATCCCGC
GTCTGACCAACTGATTAGTGAGGCGGCACGCCTCCGCTACCGTAGCGCGGGCGA
TTTCATTGTCCCGATGACCGTCCGCATGCCTTGTGGAGGGGGCATTTACGGTGGC
CAAACGCATTCTCAGAGTCCAGAAGCCATGTTCACACAAGTGTGCGGTCTTCGCA
CCGTGATGCCATCTAATCCTTATGACGCCAAAGGATTACTGATTGCGTGCATCGA
AAACGACGATCCGGTTATCTTTTTAGAACCCAAACGTCTGTACAACGGTCCTTTC
GACGGTCATCACGACCGTCCTGTCACGCCGTGGAGCAAACATCCGGCATCGCAA
GTCCCGGATGGGTATTATAAAGTGCCTCTGGACAAAGCAGCGATTGTCCGCCCTG
GTGCAGCCCTTACAGTCCTGACGTATGGTACCATGGTGTACGTGGCGCAGGCCGC
GGCAGATGAAACCGGCCTCGATGCGGAGATTATCGACCTCCGCAGTCTGTGGCC
GCTGGACTTGGAAACTATCGTCGCGAGTGTGAAAAAGACCGGTCGTTGTGTTATT
GCCCATGAAGCGACTCGTACCTGCGGCTTTGGCGCCGAACTGATGTCCCTGGTGC
AGGAACACTGTTTTCACCATCTTGAGGCTCCGATTGAACGCGTCACTGGCTGGGA
CACACCGTACCCTCATGCGCAGGAATGGGCCTATTTCCCGGGCCCAGCGCGCGTG
GGAGCCGCCTTTAAACGCGTGATGGAGGTCTGAATGGGTACCCACGTTATTAAA
ATGCCTGATATTGGTGAAGGCATCGCGGAGGTAGAGCTGGTTGAATGGCACGTT
CAAGTGGGTGATAGCGTGAATGAAGATCAGGTACTCGCGGAAGTAATGACGGAC
AAAGCAACGGTTGAAATCCCGTCCCCTGTTGCTGGCCGCATCTTGGCACTGGGTG
GCCAGCCGGGACAAGTTATGGCGGTGGGAGGAGAATTAATTCGCCTGGAAGTGG
AGGGTGCCGGAAACCTGGCGGAGTCTCCGGCCGCAGCTACGCCCGCCGCTCCGG
TGGCAGCAACTCCGGAAAAACCTAAAGAAGCACCGGTTGCAGCGCCAAAAGCA
GCTGCCGAAGCACCCCGTGCGCTTCGTGATTCTGAAGCGCCGCGCCAACGCCGCC
 
AGCCGGGGGAACGCCCATTAGCATCACCGGCCGTCCGTCAGCGTGCCCGCGACC
TGGGAATCGAGCTGCAGTTTGTTCAGGGCTCTGGCCCAGCCGGCCGCGTGCTTCA
TGAGGACCTGGATGCGTATCTTACGCAGGATGGAAGTGTTGCTCGTTCAGGCGGC
GCTGCGCAGGGTTACGCGGAACGCCATGATGAACAGGCAGTCCCGGTGATCGGT
CTGCGCCGCAAAATTGCCCAGAAGATGCAGGATGCTAAACGCCGCATTCCTCAC
TTCAGTTACGTCGAAGAGATTGACGTAACCGATCTGGAAGCCCTGCGCGCTCACT
TGAATCAGAAATGGGGCGGCAACGTGGTAAACTGACGCTGCTGCCTTTCCTCGT
CCGCGCAATGGTCGTCGCATTACGCGATTTCCCGCAACTGAATGCTCGCTATGAT
GATGAAGCGGAAGTAGTGACGCGTTACGGGGCCGTTCATGTTGGTATCGCGACC
CAGTCAGATAATGGGCTCATGGTTCCGGTGTTGCGCCATGCAGAAAGCCGTGACC
TGTGGGGTAATGCGTCGGAAGTTGCGCGTCTGGCCGAAGCGGCGCGTTCCGGTA
AAGCGCAACGTCAGGAACTGAGCGGCTCCACGATTACCCTGTCAAGCCTTGGTGT
GTTGGGAGGGATTGTATCCACGCCAGTCATTAATCACCCGGAAGTTGCAATCGTT
GGTGTTAACCGTATTGTGGAGCGCCCTATGGTTGTTGGTGGTAATATTGTAGTAC
GTAAAATGATGAATCTGAGCTCTTCGTTTGATCATCGCGTGGTGGACGGCATGGA
TGCTGCGGCTTTTATTCAAGCCGTGCGCGGTTTGTTAGAACATCCTGCCACCCTGT
TCCTGGAGTAAgcgATGAGTCAGATTTAAAAACCTCGCTCCTGATCGTTGGCGGC
GGGCCAGGCGGCTATGTGGCGGCGATCCGCGCCGGCCAGCTGGGGATTCCAACG
GTGTTGGTTGAGGGCGCCGCTTTGGGCGGTACTTGCCTGAATGTGGGGTGCATTC
CGAGCAAAGCGTTGATCCATGCTGCCGAAGAGTACCTTAAAGCGCGCCACTATG
CATCACGTTCCGCGCTGGGCATCCAGGTGCAAGCACCTTCAATTGACATCGCCCG
CACCGTGGAATGGAAAGACGCCATTGTGGACCGTTTGACTTCGGGTGTGGCGGCT
CTGCTGAAAAAGCATGGTGTGGATGTAGTACAAGGATGGGCACGCATCCTCGAC
GGCAAGAGCGTGGCGGTTGAACTGGCGGGCGGGGGTCGCAGCGCATCGAGTGT
GAACATCTGCTTCTGGCGGCGGGCTCACAAAGCGTTGAATTACCCATCCTGCCTC
TGGGGGGCAAAGTAATCAGCAGCACCGAAGCATTAGCTCCGGGGTCGTTGCCAA
AACGTCTGGTGGTTGTGGGTGGCGGTTATATTGGTCTGGAGCTGGGCACTGCATA
TCGCAAGCTGGGTGTTGAAGTTGCTGTGGTGGAGGCACAACCCCGCATCCTGCCG
GGCTACGATGAGGAACTGACTAAGCCGGTGGCCCAAGCGCTGCGCCGTCTGGGT
GTAGAACTGTACCTGGGTCATTCATTGCTGGGACCGAGTGAAAACGGCGTTCGCG
TGCGTGATGGGGCTGGCGAAGAACGTGAGATCGCCGCGGACCAGGTCCTTGTCG
CAGTTGGCCGCAAACCGCGTTCAGAGGGTTGGAACCTGGAGTCTCTCGGTTTAGA
CATGAATGGGCGTGCCGTAAAAGTGGACGATCAGTGCCGTACAAGCATGCGTAA
CGTATGGGCCATTGGCGACCTGGCGGGCGAACCGATGCTGGCGCACCGCGCTAT
GGCGCAAGGAGAAATGGTCGCCGAATTGATTGCGGGCAAACGCCGTCAGTTTGC
GCCGGTTGCAATTCCTGCAGTCTGTTTTACGGATCCGGAAGTGGTGGTGGCGGGT
CTGAGTCCGGAACAGGCCAAAGATGCGGGTCTGGATTGCCTGGTCGCGTCATTCC
CGTTCGCAGCCAACGGCCGCGCCATGACGTTGGAAGCTAACGAAGGCTTTGTCC
GCGTGGTGGCACGTCGTGACAACCATCTGGTGGTTGGTTGGCAGGCGGTCGGTA
AAGCTGTGTCTGAATTAAGCACCGCGTTCGCACAATCTCTGGAAATGGGCGCTCG
CCTCGAAGACATTGCAGGCACAATCCACGCGCACCCCACCCTGGGTGAAGCTGT
TCAGGAAGCGGCACTCCGTGCCTTAGGTCACGCCCTGCACATTTGA
SEQ4: Tet-leuDH-bkd construct 
gtaaaacgacggccagtgaattcgTTAAGACCCACTTTCACATTTAAGTTGTTTTCTAATCCGC
ATATGATCAATTCAAGGCCGAATAAGAAGGCTGGCTCTGCACCTTGGTGATCAA
ATAATTCGATAGCTTGTCGTAATAATGGCGGCATACTATCAGTAGTAGGTGTTTC
CCTTTCTTCTTTAGCGACTTGATGCTCTTGATCTTCCAATACGCAACCTAAAGTAA
AATGCCCCACAGCGCTGAGTGCATATAATGCATTCTCTAGTGAAAAACCTTGTTG
 
GCATAAAAGGCTAATTGATTTCGAGAGTTCATACTGTTTTTCTGTAGGCCGT
GTACCTAAATGTACTTTTGCTCCATCGCGATGACTTAGTAAAGCACATCTAAAAC
TTTTAGCGTTATTACGTAAAAAATCTTGCCAGCTTTCCCCTTCTAAAGGGCAAAA
GTGAGTATGGTGCCTATCTAACATCTCAATGGCTAAGGCGTCGAGCAAAGCCCGC
TTATTTTTTACATGCCAATACAATGTAGGCTGCTCTACACCTAGCTTCTGGGCGAG
TTTACGGGTTGTTAAACCTTCGATTCCGACCTCATTAAGCAGCTCTAATGCGCTGT
TAATCACTTTACTTTTATCTAATCTAGACATcattaattcctaatttttgttgacactctatcattgatagagtta
ttttaccactccctatcagtgatagagaaaagtgaactctagaaataattttgtttaactttaagaaggagatatacatATGTTCGAT
ATGATGGATGCAGCCCGCCTGGAAGGCCTGCACCTCGCCCAGGATCCAGCGACG
GGCCTGAAAGCGATCATCGCGATCCATTCCACTCGCCTCGGCCCGGCCTTAGGCG
GCTGTCGTTACCTCCCATATCCGAATGATGAAGCGGCCATCGGCGATGCCATTCG
CCTGGCGCAGGGCATGTCCTACAAAGCTGCACTTGCGGGTCTGGAACAAGGTGG
TGGCAAGGCGGTGATCATTCGCCCACCCCACTTGGATAATCGCGGTGCCTTGTTT
GAAGCGTTTGGACGCTTTATTGAAAGCCTGGGTGGCCGTTATATCACCGCCGTTG
ACTCAGGAACAAGTAGTGCCGATATGGATTGCATCGCCCAACAGACGCGCCATG
TGACTTCAACGACACAAGCCGGCGACCCATCTCCACATACGGCTCTGGGCGTCTT
TGCCGGCATCCGCGCCTCCGCGCAGGCTCGCCTGGGGTCCGATGACCTGGAAGG
CCTGCGTGTCGCGGTTCAGGGCCTTGGCCACGTAGGTTATGCGTTAGCGGAGCAG
CTGGCGGCGGTCGGCGCAGAACTGCTGGTGTGCGACCTGGACCCCGGCCGCGTC
CAGTTAGCGGTGGAGCAACTGGGGGCGCACCCACTGGCCCCTGAAGCATTGCTC
TCTACTCCGTGCGACATTTTAGCGCCTTGTGGCCTGGGCGGCGTGCTCACCAGCC
AGTCGGTGTCACAGTTGCGCTGCGCGGCCGTTGCAGGCGCAGCGAACAATCAAC
TGGAGCGCCCGGAAGTTGCAGACGAACTGGAGGCGCGCGGATTTTATATGCGC
CCGATTACGTGATTAACTCGGGAGGACTGATTTATGTGGCGCTGAAGCATCGCGG
TGCTGATCCGCATAGCATTACCGCCCACCTCGCTCGCATCCCTGCACGCCTGACG
GAAATCTATGCGCATGCGCAGGCGGATCATCAGTCGCCTGCGCGCATCGCCGAT
CGTCTGGCGGAGCGCATTCTGTACGGCCCGCAATAAtgaaggagatatacatATGTCCGAC
TACGAGCCACTCCGCTTGCACGTGCCGGAGCCGACAGGTCGTCCCGGCTGCAAA
ACGGATTTCTCTTACCTGCACTTATCTCCCGCAGGTGAAGTCCGCAAACCGCCTG
TCGACGTGGAGCCTGCAGAAACCAGCGATTTGGCATATTCGCTGGTGCGTGTGCT
CGATGATGATGGACATGCAGTGGGTCCGTGGAATCCGCAGCTCTCAAACGAACA
GCTGCTGCGTGGAATGCGCGCGATGCTGAAGACGCGTCTGTTCGATGCTCGCATG
TTGACTGCGCAGCGCCAAAAAAAATTGAGTTTTATATGCAGTGCTTAGGAGAAG
AGGCAATCGCGACTGCCCATACACTGGCCCTGCGCGATGGTGATATGTGTTTTCC
GACGTACCGTCAGCAGGGGATTCTTATTACACGTGAGTATCCGCTTGTGGATATG
ATCTGCCAGCTGCTGTCGAATGAAGCGGACCCCCTGAAAGGCCGTCAACTGCCG
ATCATGTACAGCAGTAAGGAGGCTGGCTTCTTTAGCATCTCGGGCAATCTTGCGA
CTCAGTTTATTCAGGCGGTGGGGTGGGGGATGGCAAGCGCAATCAAAGGGGATA
CCCGCATTGCATCCGCATGGATTGGCGATGGCGCTACCGCGGAAAGCGATTTTCA
TACGGCGCTGACCTTTGCTCACGTTTATCGCGCACCGGTGATCCTCAATGTGGTC
AACAACCAGTGGGCGATTTCGACGTTTCAGGCCATCGCGGGCGGCGAGGGCACC
ACGTTCGCGAACCGTGGCGTGGGTTGCGGCATTGCGAGCCTCCGTGTGGACGGG
AACGATTTTTTGGCCGTGTATGCGGCGAGCGAATGGGCGGCAGAACGCGCACGC
CGTAACTTGGGACCGTCCCTGATCGAATGGGTAACTTATCGCGCGGGCCCACACA
GCACGAGCGACGATCCGTCAAAGTATCGCCCTGCGGATGATTGGACCAATTTCC
GCTGGGTGACCCGATTGCGCGTCTGAAACGTCACATGATCGGTTTGGGTATTTGG
AGCGAAGAACAGCACGAAGCTACGCACAAAGCGCTGGAAGCGGAAGTCCTGGC
GGCGCAGAAGCAGGCCGAAAGCCATGGCACTCTGATTGACGGCCGTGTGCCGTC
TGCAGCCTCTATGTTCGAAGATGTTTATGCCGAGTTACCCGAGCACTTACGTCGC
CAGCGCCAGGAGCTCGGGGTATGAACGCCATGAACCCGCAGCATGAAAACGCGC
 
AAACCGTGACCTCCATGACGATGATTCAGGCCCTGCGCTCGGCGATGGATATTAT
GTTAGAACGTGACGATGACGTCGTGGTGTTTGGTCAGGACGTAGGGTATTTGGG
GGAGTGTTTCGTTGTACCGAGGGGTTGCAAAAGAAGTATGGTACGAGTCGCGTCT
TCGATGCACCGATCAGCGAATCAGGCATTATCGGCGCTGCCGTGGGCATGGGTG
CATATGGCTTGCGCCCTGTGGTTGAAATTCAGTTTGCAGATTATGTATATCCCGC
GTCTGACCAACTGATTAGTGAGGCGGCACGCCTCCGCTACCGTAGCGCGGGCGA
TTTCATTGTCCCGATGACCGTCCGCATGCCTTGTGGAGGGGGCATTTACGGTGGC
CAAACGCATTCTCAGAGTCCAGAAGCCATGTTCACACAAGTGTGCGGTCTTCGCA
CCGTGATGCCATCTAATCCTTATGACGCCAAAGGATTACTGATTGCGTGCATCGA
AAACGACGATCCGGTTATCTTTTTAGAACCCAAACGTCTGTACAACGGTCCTTTC
GACGGTCATCACGACCGTCCTGTCACGCCGTGGAGCAAACATCCGGCATCGCAA
GTCCCGGATGGGTATTATAAAGTGCCTCTGGACAAAGCAGCGATTGTCCGCCCTG
GTGCAGCCCTTACAGTCCTGACGTATGGTACCATGGTGTACGTGGCGCAGGCCGC
GGCAGATGAAACCGGCCTCGATGCGGAGATTATCGACCTCCGCAGTCTGTGGCC
GCTGGACTTGGAAACTATCGTCGCGAGTGTGAAAAAGACCGGTCGTTGTGTTATT
GCCCATGAAGCGACTCGTACCTGCGGCTTTGGCGCCGAACTGATGTCCCTGGTGC
AGGAACACTGTTTTCACCATCTTGAGGCTCCGATTGAACGCGTCACTGGCTGGGA
CACACCGTACCCTCATGCGCAGGAATGGGCCTATTTCCCGGGCCCAGCGCGCGTG
GGAGCCGCCTTTAAACGCGTGATGGAGGTCTGAATGGGTACCCACGTTATTAAA
ATGCCTGATATTGGTGAAGGCATCGCGGAGGTAGAGCTGGTTGAATGGCACGTT
CAAGTGGGTGATAGCGTGAATGAAGATCAGGTACTCGCGGAAGTAATGACGGAC
AAAGCAACGGTTGAAATCCCGTCCCCTGTTGCTGGCCGCATCTTGGCACTGGGTG
GCCAGCCGGGACAAGTTATGGCGGTGGGAGGAGAATTAATTCGCCTGGAAGTGG
AGGGTGCCGGAAACCTGGCGGAGTCTCCGGCCGCAGCTACGCCCGCCGCTCCGG
TGGCAGCAACTCCGGAAAAACCTAAAGAAGCACCGGTTGCAGCGCCAAAAGCA
GCTGCCGAAGCACCCCGTGCGCTTCGTGATTCTGAAGCGCCGCGCCAACGCCGCC
AGCCGGGGGAACGCCCATTAGCATCACCGGCCGTCCGTCAGCGTGCCCGCGACC
TGGGAATCGAGCTGCAGTTTGTTCAGGGCTCTGGCCCAGCCGGCCGCGTGCTTCA
TGAGGACCTGGATGCGTATCTTACGCAGGATGGAAGTGTTGCTCGTTCAGGCGGC
GCTGCGCAGGGTTACGCGGAACGCCATGATGAACAGGCAGTCCCGGTGATCGGT
CTGCGCCGCAAAATTGCCCAGAAGATGCAGGATGCTAAACGCCGCATTCCTCAC
TTCAGTTACGTCGAAGAGATTGACGTAACCGATCTGGAAGCCCTGCGCGCTCACT
TGAATCAGAAATGGGGCGGGCAACGTGGTAAACTGACGCTGCTGCCTTTCCTCGT
CCGCGCAATGGTCGTCGCATTACGCGATTTCCCGCAACTGAATGCTCGCTATGAT
GATGAAGCGGAAGTAGTGACGCGTTACGGGGCCGTTCATGTTGGTATCGCGACC
CAGTCAGATAATGGGCTCATGGTTCCGGTGTTGCGCCATGCAGAAAGCCGTGACC
TGTGGGGTAATGCGTCGGAAGTTGCGCGTCTGGCCGAAGCGGCGCGTTCCGGTA
AAGCGCAACGTCAGGAACTGAGCGGCTCCACGATTACCCTGTCAAGCCTTGGTGT
GTTGGGAGGGATTGTATCCACGCCAGTCATTAATCACCCGGAAGTTGCAATCGTT
GGTGTTAACCGTATTGTGGAGCGCCCTATGGTTGTTGGTGGTAATATTGTAGTAC
GTAAAATGATGAATCTGAGCTCTTCGTTTGATCATCGCGTGGTGGACGGCATGGA
TGCTGCGGCTTTTATTCAAGCCGTGCGCGGTTTGTTAGAACATCCTGCCACCCTGT
TCCTGGAGTAAgcgATGAGTCAGATTTTAAAAACCTCGCTCCTGATCGTTGGCGGC
GGGCCAGGCGGCTATGTGGCGGCGATCCGCGCCGGCCAGCTGGGGATTCCAACG
GTGTTGGTTGAGGGCGCCGCTTTGGGCGGTACTTGCCTGAATGTGGGGTGCATTC
CGAGCAAAGCGTTGATCCATGCTGCCGAAGAGTACCTTAAAGCGCGCCACTATG
CATCACGTTCCGCGCTGGGCATCCAGGTGCAAGCACCTTCAATTGACATCGCCCG
CACCGTGGAATGGAAAGACGCCATTGTGGACCGTTTGACTTCGGGTGTGGCGGCT
CTGCTGAAAAAGCATGGTGTGGATGTAGTACAAGGATGGGCACGCATCCTCGAC
GGCAAGAGCGTGGCGGTTGAACTGGCGGGCGGGGGGTCGCAGCGCATCGAGTGT
 
GAACATCTGCTTCTGGCGGCGGGCTCACAAAGCGTTGAATTACCCATCCTGCCTC
TGGGGGGCAAAGTAATCAGCAGCACCGAAGCATTAGCTCCGGGGTCGTTGCCAA
AACGTCTGGTGGTTGTGGGTGGCGGTTATATTGGTCTGGAGCTGGGCACTGCATA
TCGCAAGCTGGGTGTTGAAGTTGCTGTGGTGGAGGCACAACCCCGCATCCTGCCG
GGCTACGATGAGGAACTGACTAAGCCGGTGGCCCAAGCGCTGCGCCGTCTGGGT
GTAGAACTGTACCTGGGTCATTCATTGCTGGGACCGAGTGAAAACGGCGTTCGCG
TGCGTGATGGGGCTGGCGAAGAACGTGAGATCGCCGCGGACCAGGTCCTTGTCG
CAGTTGGCCGCAAACCGCGTTCAGAGGGTTGGAACCTGGAGTCTCTCGGTTTAGA
CATGAATGGGCGTGCCGTAAAAGTGGACGATCAGTGCCGTACAAGCATGCGTAA
CGTATGGGCCATTGGCGACCTGGCGGGCGAACCGATGCTGGCGCACCGCGCTAT
GGCGCAAGGAGAAATGGTCGCCGAATTGATTGCGGGCAAACGCCGTCAGTTTGC
GCCGGTTGCAATTCCTGCAGTCTGTTTTACGGATCCGGAAGTGGTGGTGGCGGGT
CTGAGTCCGGAACAGGCCAAAGATGCGGGTCTGGATTGCCTGGTCGCGTCATTCC
CGTTCGCAGCCAACGGCCGCGCCATGACGTTGGAAGCTAACGAAGGCTTTGTCC
GCGTGGTGGCACGTCGTGACAACCATCTGGTGGTTGGTTGGCAGGCGGTCGGTA
AAGCTGTGTCTGAATTAAGCACCGCGTTCGCACAATCTCTGGAAATGGGCGCTCG
CCTCGAAGACATTGCAGGCACAATCCACGCGCACCCCACCCTGGGTGAAGCTGT
TCAGGAAGCGGCACTCCGTGCCTTAGGTCACGCCCTGCACATTTGA
SEQ5: Tet-livKHMGF construct 
ccagtgaattcgTTAAGACCCACTTTCACATTTAAGTTGTTTTTCTAATCCGCATATGATC
AATTCAAGGCCGAATAAGAAGGCTGGCTCTGCACCTTGGTGATCAAATAATTCG
ATAGCTTGTCGTAATAATGGCGGCATACTATCAGTAGTAGGTGTTTCCCTTTCTTC
TTTAGCGACTTGATGCTCTTGATCTTCCAATACGCAACCTAAAGTAAAATGCCCC
ACAGCGCTGAGTGCATATAATGCATTCTCTAGTGAAAAACCTTGTTGGCATAAAA
AGGCTAATTGATTTTCGAGAGTTTCATACTGTTTTTCTGTAGGCCGTGTACCTAAA
TGTACTTTTGCTCCATCGCGATGACTTAGTAAAGCACATCTAAAACTTTTAGCGTT
ATTACGTAAAAAATCTTGCCAGCTTTCCCCTTCTAAAGGGCAAAAGTGAGTATGG
TGCCTATCTAACATCTCAATGGCTAAGGCGTCGAGCAAAGCCCGCTTATTTTTTA
CATGCCAATACAATGTAGGCTGCTCTACACCTAGCTTCTGGGCGAGTTTACGGGT
TGTTAAACCTTCGATTCCGACCTCATTAAGCAGCTCTAATGCGCTGTTAATCACTT
TACTTTTATCTAATCTAGACATcattaattcctaatttttgttgacactctatcattgatagagttattttaccactccctat
cagtgatagagaaaagtgaactctagaaataattttgtttaactttaagaaggagatatacatATGAAACGGAATGCGAA
AACTATCATCGCAGGGATGATTGCACTGGCAATTTCACACACCGCTATGGCTGAC
GATATTAAAGTCGCCGTTGTCGGCGCGATGTCCGGCCCGATTGCCCAGTGGGGCG
ATATGGAATTTAACGGCGCGCGTCAGGCAATTAAAGACATTAATGCCAAAGGGG
GAATTAAGGGCGATAAACTGGTTGGCGTGGAATATGACGACGCATGCGACCCGA
AACAAGCCGTTGCGGTCGCCAACAAAATCGTTAATGACGGCATTAAATACGTTAT
TGGTCATCTGTGTTCTTCTTCTACCCAGCCTGCGTCAGATATCTATGAAGACGAA
GGTATTCTGATGATCTCGCCGGGAGCGACCAACCCGGAGCTGACCCAACGCGGT
TATCAACACATTATGCGTACTGCCGGGCTGGACTCTTCCCAGGGGCCAACGGCGG
CAAAATACATTCTTGAGACGGTGAAGCCCCAGCGCATCGCCATCATTCACGACA
AACAACAGTATGGCGAAGGGCTGGCGCGTTCGGTGCAGGACGGGCTGAAAGCGG
CTAACGCCAACGTCGTCTTCTTCGACGGTATTACCGCCGGGGAGAAAGATTTCTC
CGCGCTGATCGCCCGCCTGAAAAAAGAAAACATCGACTTCGTTTACTACGGCGGT
TACTACCCGGAAATGGGGCAGATGCTGCGCCAGGCCCGTTCCGTTGGCCTGAAA
ACCCAGTTTATGGGGCCGGAAGGTGTGGGTAATGCGTCGTTGTCGAACATTGCCG
GTGATGCCGCCGAAGGCATGTTGGTCACTATGCCAAAACGCTATGACCAGGATC
CGGCAAACCAGGGCATCGTTGATGCGCTGAAAGCAGACAAGAAAGATCCGTCCG
 
GGCCTTATGTCTGGATCACCTACGCGGCGGTGCAATCTCTGGCGACTGCCCTTGA
GCGTACCGGCAGCGATGAGCCGCTGGCGCTGGTGAAAGATTTAAAAGCTAACGG
TGCAAACACCGTGATTGGGCCGCTGAACTGGATGAAAAAGGCGATCTTAAGGG
ATTTGATTTTGGTGTCTTCCAGTGGCACGCCGACGGTTCATCCACGGCAGCCAAG
TGAtcatcccaccgcccgtaaaatgcgggcgggtttagaaaggttaccttATGTCTGAGCAGTTTTTGTATTTC
TTGCAGCAGATGTTTAACGGCGTCACGCTGGGCAGTACCTACGCGCTGATAGCCA
TCGGCTACACCATGGTTTACGGCATTATCGGCATGATCAACTTCGCCCACGGCGA
GGTTTATATGATTGGCAGCTACGTCTCATTTATGATCATCGCCGCGCTGATGATG
ATGGGCATTGATACCGGCTGGCTGCTGGTAGCTGCGGGATTCGTCGGCGCAATCG
TCATTGCCAGCGCCTACGGCTGGAGTATCGAACGGGTGGCTTACCGCCCGGTGCG
TAACTCTAAGCGCCTGATTGCACTCATCTCTGCAATCGGTATGTCCATCTTCCTGC
AAAACTACGTCAGCCTGACCGAAGGTTCGCGCGACGTGGCGCTGCCGAGCCTGT
TTAACGGTCAGTGGGTGGTGGGGCATAGCGAAAACTTCTCTGCCTCTATTACCAC
CATGCAGGCGGTGATCTGGATTGTTACCTTCCTCGCCATGCTGGCGCTGACGATT
TTCATTCGCTATTCCCGCATGGGTCGCGCGTGTCGTGCCTGCGCGGAAGATCTGA
AAATGGCGAGTCTGCTTGGCATTAACACCGACCGGGTGATTGCGCTGACCTTTGT
GATTGGCGCGGCGATGGCGGCGGTGGCGGGTGTGCTGCTCGGTCAGTTCTACGG
CGTCATTAACCCCTACATCGGCTTTATGGCCGGGATGAAAGCCTTTACCGCGGCG
GTGCTCGGTGGGATTGGCAGCATTCCGGGAGCGATGATTGGCGGCCTGATTCTGG
GGATTGCGGAGGCGCTCTCTTCTGCCTATCTGAGTACGGAATATAAAGATGTGGT
gTCATTCGCCCTGCTGATTCTGGTGCTGCTGGTGATGCCGACCGGTATTCTGGGTC
GCCCGGAGGTAGAGAAAGTATGAAACCGATGCATATTGCAATGGCGCTGCTCTC
TGCCGCGATGTTCTTTGTGCTGGCGGGCGTCTTTATGGGCGTGCAACTGGAGCTG
GATGGCACCAAACTGGTGGTCGACACGGCTTCGGATGTCCGTTGGCAGTGGGTGT
TTATCGGCACGGCGGTGGTCTTTTTCTTCCAGCTTTTGCGACCGGCTTTCCAGAAA
GGGTTGAAAAGCGTTTCCGGACCGAAGTTTATTCTGCCCGCCATTGATGGCTCCA
CGGTGAAGCAGAAACTGTTCCTCGTGGCGCTGTTGGTGCTTGCGGTGGCGTGGCC
GTTTATGGTTTCACGCGGGACGGTGGATATTGCCACCCTGACCATGATCTACATT
ATCCTCGGTCTgGGCTGAACGTGGTTGTTGGTCTTTCTGGTCTGCTGGTGCTGGG
GTACGGCGGTTTTTACGCCATCGGCGCTTACACTTTTGCGCTGCTCAATCACTATT
ACGGCTTGGGCTTCTGGACCTGCCTGCCGATTGCTGGATTAATGGCAGCGGCGGC
GGGCTTCCTGCTCGGTTTTCCGGTGCTGCGTTTGCGCGGTGACTATCTGGCGATCG
TTACCCTCGGTTTCGGCGAAATTGTGCGCATATTGCTGCTCAATAACACCGAAAT
TACCGGCGGCCCGAACGGAATCAGTCAGATCCCGAAACCGACACTCTTCGGACT
CGAGTTCAGCCGTACCGCTCGTGAAGGCGGCTGGGACACGTTCAGTAATTTCTTT
GGCCTGAAATACGATCCCTCCGATCGTGTCATCTTCCTCTACCTGGTGGCGTTGCT
GCTGGTGGTGCTAAGCCTGTTTGTCATTAACCGCCTGCTGCGGATGCCGCTGGGG
CGTGCGTGGGAAGCGTTGCGTGAAGATGAAATCGCCTGCCGTTCGCTGGGCTTAA
GCCCGCGTCGTATCAAGCTGACTGCCTTTACCATAAGTGCCGCGTTTGCCGGTTTT
GCCGGAACGCTGTTTGCGGCGCGTCAGGGCTTTGTCAGCCCGGAATCCTTCACCT
TTGCCGAATCGGCGTTTGTGCTGGCGATAGTGGTGCTCGGCGGTATGGGCTCGCA
ATTTGCGGTGATTCTGGCGGCAATTTTGCTGGTGGTGTCGCGCGAGTTGATGCGT
GATTTCAACGAATACAGCATGTTAATGCTCGGTGGTTTGATGGTGCTGATGATGA
TCTGGCGTCCGCAGGGCTTGCTGCCCATGACGCGCCCGCAACTGAAGCTGAAAA
ACGGCGCAGCGAAAGGAGAGCAGGCATGAGTCAGCCATTATTATCTGTTAACGG
CCTGATGATGCGCTTCGGCGGCCTGCTGGCGGTGAACAACGTCAATCTTGAACTG
TACCCGCAGGAGATCGTCTCGTTAATCGGCCCTAACGGTGCCGGAAAAACCACG
GTTTTTAACTGTCTGACCGGATTCTACAAACCCACCGGCGGCACCATTTTACTGC
GCGATCAGCACCTGGAAGGTTTACCGGGGCAGCAAATTGCCCGCATGGGCGTGG
TGCGCACCTTCCAGCATGTGCGTCTGTTCCGTGAAATGACGGTAATTGAAAACCT
 
GCTGGTGGCGCAGCATCAGCAACTGAAAACCGGGCTGTTCTCTGGCCTGTTGAAA
ACGCCATCCTTCCGTCGCGCCCAGAGCGAAGCGCTCGACCGCGCCGCGACCTGG
CTTGAGCGCATTGGTTTGCTGGAACACGCCAACCGTCAGGCGAGTAACCTGGCCT
ATGGTGACCAGCGCCGTCTTGAGATTGCCCGCTGCATGGTGACGCAGCCGGAGA
TTTTAATGCTCGACGAACCTGCGGCAGGTCTTAACCCGAAAGAGACGAAAGAGC
TGGATGAGCTGATTGCCGAACTGCGCAATCATCACAACACCACTATCTTGTTGAT
TGAACACGATATGAAGCTGGTGATGGGAATTTCGGACCGAATTTACGTGGTCAAT
CAGGGGACGCCGCTGGCAAACGGTACGCCGGAGCAGATCCGTAATAACCCGGAC
GTGATCCGTGCCTATTTAGGTGAGGCATAAGATGGAAAAAGTCATGTTGTCCTTT
GACAAAGTCAGCGCCCACTACGGCAAAATCCAGGCGCTGCATGAGGTGAGCCTG
CATATCAATCAGGGCGAGATTGTCACGCTGATTGGCGCGAACGGGGCGGGGAAA
ACCACCTTGCTCGGCACGTTATGCGGCGATCCGCGTGCCACCAGCGGGCGAATTG
TGTTTGATGATAAAGACATTACCGACTGGCAGACAGCGAAAATCATGCGCGAAG
CGGTGGCGATTGTCCCGGAAGGGCGTCGCGTCTTCTCGCGGATGACGGTGGAAG
AGAACCTGGCGATGGGCGGTTTTTTGCTGAACGCGACCAGTTCCAGGAGCGCAT
AAAGTGGGTGTATGAGCTGTTTCCACGTCTGCATGAGCGCCGTATTCAGCGGGCG
GGCACCATGTCCGGCGGTGAACAGCAGATGCTGGCGATTGGTCGTGCGCTGATG
AGCAACCCGCGTTTGCTACTGCTTGATGAGCCATCGCTCGGTCTTGCGCCGATTA
TCATCCAGCAAATTTTCGACACCATCGAGCAGCTGCGCGAGCAGGGGATGACTA
TCTTTCTCGTCGAGCAGAACGCCAACCAGGCGCTAAAGCTGGCGGATCGCGGCT
ACGTGCTGGAAAACGGCCATGTAGTGCTTTCCGATACTGGTGATGCGCTGCTGGC
GAATGAAGCGGTGAGAAGTGCGTATTTAGGCGGGTAA
SEQ6: pKIKO-lacZ 
agattgcagcattacacgtcttgagcgattgtgtaggctggagctgcttcgaagttcctatactttctagagaataggaacttcggaatag
gaacttcatttaaatggcgcgccttacgccccgccctgccacTCATCGCAGTACTGTTGTATTCATTAAGC
ATCTGCCGACATGGAAGCCATCACAAACGGCATGATGAACCTGAATCGCCAGCG
GCATCAGCACCTTGTCGCCTTGCGTATAATATTTGCCCATGGTGAAAACGGGGGC
GAAGAAGTTGTCCATATTGGCCACGTTTAAATCAAAACTGGTGAAACTCACCCAG
GGATTGGCTGAGACGAAAAACATATTCTCAATAAACCCTTTAGGGAAATAGGCC
AGGTTTTCACCGTAACACGCCACATCTTGCGAATATATGTGTAGAAACTGCCGGA
AATCGTCGTGGTATTCACTCCAGAGCGATGAAAACGTTTCAGTTTGCTCATGGAA
AACGGTGTAACAAGGGTGAACACTATCCCATATCACCAGCTCACCGTCTTTCATT
GCCATACGTAATTCCGGATGAGCATTCATCAGGCGGGCAAGAATGTGAATAAAG
GCCGGATAAAACTTGTGCTTATTTTTCTTTACGGTCTTTAAAAAGGCCGTAATATC
CAGCTGAACGGTCTGGTTATAGGTACATTGAGCAACTGACTGAAATGCCTCAAA
ATGTTCTTTACGATGCCATTGGGATATATCAACGGTGGTATATCCAGTGATTTTTT
TCTCCATtttagcttccttagctcctgaaaatctcgacaactcaaaaaatacgcccggtagtgatcttatttcattatggtgaaagttg
gaacctcttacgtgccgatcaacgtctcattttcgccaaaagttggcccagggcttcccggtatcaacagggacaccaggatttatttatt
ctgcgaagtgatcttccgtcacaggtaggcgcgccgaagttcctatactttctagagaataggaacttcggaataggaactaaggagga
tattcatatggaccatggctaattccTTGCCGTTTTCATCATATTTAATCAGCGACTGATCCACCC
AGTCCCAGACGAAGCCGCCCTGTAAACGGGGGTACTGACGAAACGCCTGCCAGT
ATTTAGCGAAGCCGCCAAGACTGTTACCCATCGCGTGGGCATATTCGCAAAGGAT
CAGCGGGCGCATTTCTCCAGGCAGCGAAAGCCATTTTTGATGGACCATTTCGGC
ACCGCCGGGAAGGGCTGGTCTTCATCCACGCGCGCGTACATCGGGCAAATAATA
TCGGTGGCCGTGGTGTCGGCTCCGCCGCCTTCATACTGTACCGGGCGGGAAGGAT
CGACAGATTTGATCCAGCGATACAGCGCGTCGTGATTAGCGCCGTGGCCTGATTC
ATTCCCCAGCGACCAGATGATCACACTCGGGTGATTACGATCGCGCTGCACCATC
CGCGTTACGCGTTCGCTCATCGCGGGTAGCCAGCGCGGATCATCGGTCAGACGAT
 
TCATTGGCACCATGCCGTGGGTTTCAATATTGGCTTCATCCACCACATACAGGCC
GTAGCGGTCGCACAGCGTGTACCACAGCGGATGGTTCGGATAATGCGAACAGCG
CACGGCGTTAAAGTTGTTCTGCTTCATCAGCAGGATATCCTGCACCATCGTCTGC
TCATCCATGACCTGACCATGCAGAGGATGATGCTCGTGACGGTTAACGCCGCGA
ATCAGCAACGGCTTGCCGTTCAGCAGCAGACCATTTTCAATCCGCACCTCGC
GGAAACCGACGTCGCAGGCTTCTGCTTCAATCAGCGTGCCGTCGGCGGTGTGCAG
TTCAACCACTGCACGATAGAGATTCGGGATTTCGGCGCTCCACAGTTCCGGATTT
TCAACGTTCAGGCGTAGTGTGACGCGATCGGCATAACCGCCACGCTCATCGATAA
TTTCACCcatgtcagccgttaagtgttcctgtgtcactgaaaattgctttgagaggctctaagggcttctcagtgcgttacatccctg
gcttgttgtccacaaccgttaaaccttaaaagctttaaaagccttatatattcttttttttcttataaaacttaaaaccttagaggctatttaagtt
gctgatttatattaattttattgttcaaacatgagagcttagtacgtgaaacatgagagcttagtacgttagccatgagagcttagtacgttag
ccatgagggtttagttcgttaaacatgagagcttagtacgttaaacatgagagcttagtacgtgaaacatgagagcttagtacgtactatc
aacaggttgaactgcggatcttgcggccgcaaaaattaaaaatgaagttttaaatcaatctaaagtatatatgagtaaacttggtctgaca
gTTACCAATGCTTAATCAGTGAGGCACCTATCTCAGCGATCTGTCTATTTCGTTCA
TCCATAGTTGCCTGACTCCCCGTCGTGTAGATAACTACGATACGGGAGGGCTTAC
CATCTGGCCCCAGTGCTGCAATGATACCGCGAGACCCACGCTCACCGGCTCCAG
ATTTATCAGCAATAAACCAGCCAGCCGGAAGGGCCGAGCGCAGAAGTGGTCCTG
CAACTTTATCCGCCTCCATCCAGTCTATTAATTGTTGCCGGGAAGCTAGAGTAAG
TAGTTCGCCAGTTAATAGTTTGCGCAACGTTGTTGCCATTGCTACAGGCATCGTG
GTGTCACGCTCGTCGTTTGGTATGGCTTCATTCAGCTCCGGTTCCCAACGATCAA
GGCGAGTTACATGATCCCCCATGTTGTGCAAAAAAGCGGTTAGCTCCTTCGGTCC
TCCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTGG
TGAGTACTCAACCAAGTCATTCTGAGAATAGTGTATGCGGCGACCGAGTTGCTCT
TGCCCGGCGTCAATACGGGATAATACCGCGCCACATAGCAGAACTTTAAAAGTG
CTCATCATTGGAAAACGTTCTTCGGGGCGAAAACTCTCAAGGATCTTACCGCTGT
TGAGATCCAGTTCGATGTAACCCACTCGTGCACCCAACTGATCTTCAGCATCTTTT
ACTTTCACCAGCGTTTCTGGGTGAGCAAAAACAGGAAGGCAAAATGCCGCAAAA
AAGGGAATAAGGGCGACACGGAAATGTTGAATACTCATactcttcctttttcaatattattgaagcat
ttatcagggttattgtctcatgagcggatacatatttgaatgtatttagaaaaataaacaaataggggttccgcgACTGACGGGC
TCCAGGAGTCGTCGCCACCAATCCCCATATGGAAACCGTCGATATTCAGCCATGT
GCCTTCTTCCGCGTGCAGCAGATGGCGATGGCTGGTTTCCATCAGTTGTTGTTGG
CTGTAGCGGCTGATGTTGAACTGGAAGTCGCCGCGCCACTGGTGTGGGCCATAAT
TCAATTCGCGCGTCCCGCAGCGCAGACCGTTTTCGCTCGGGAAGACGTACGGGGT
ATACATGTCTGACAATGGCAGATCCCAGCGGTCAAAACAGGCTGCAGTAAGGCG
GTCGGGATAGTTTTCTTGCGGCCCCAGGCCGAGCCAGTTTACCCGCTCTGAGACC
TGCGCCAGCTGGCAGGTCAGGCCAATCCGCGCCGGATGCGGTGTATCGCTTGCC
ACCGCAACATCCACATTGATGACCATCTCACCGTGCCCATCAATCCGGTAGGTTT
TCCGGCTGATAAATAAGGTTTTCCCCTGATGCTGCCACGCGTGGGCGGTTGTAAT
CAGCACCGCGTCGGCAAGTGTATCTGCCGTGCACTGCAACAACGCCGCTTCGGCC
TGGTAATGGCCCGCCGCCTTCCAGCGTTCGACCCAGGCGTTAGGGTCAATGCGGG
TCGCTTCACTTACGCCAATGTCGTTATCCAGCGGCGCACGGGTGAACTGATCGCG
CAGCGGGGTCAGCAGTTGTTTTTCATCGCCAATCCACATCTGTGAAAGAAAGCCT
GACTGGCGGTTAAATTGCCAACGCTTATTACCCAGCTCGATGCAAAAATCCGTTC
CGCTGGTGGTCAGTTGAGGGATGGCGTGGGACGCGGAGGGGAGTGTCACGCTGA
GGTTTTCCGCCAGACGCCATTGCTGCCAGGCGCTGATGTGTCCGGCTTCTGACCA
TGCGGTCGCGTTTGGTTGCACTACGCGTACCGTTAGCCAGAGTcacatttccccgaaaagtgc
cacctgcatcgatggccccccgatggtagtgtggggtctccccatgcgagagtagggaactgccaggcatcaaataaaacgaaagg
ctcagtcgaaagactgggcctttcgttttatctgttgtttgtcggtgaacgctctcctgagtaggacaaatccgccgggagcggatttgaa
 
cgttgcgaagcaacggcccggagggtggcgggcaggacgcccgccataaactgccaggcatcaaattaagcagaaggccatcct
gacggatggcctttttgcgtggccagtgccaagcttgcatgc
SEQ7: pTet-livKHMGF sequence 
agattgcagcattacacgtcttgagcgattgtgtaggctggagctgcttcgaagttcctatactttctagagaataggaacttcggaatag
gaacttcatttaaatggcgcgccttacgccccgccctgccacTCATCGCAGTACTGTTGTATTCATTAAGC
ATCTGCCGACATGGAAGCCATCACAAACGGCATGATGAACCTGAATCGCCAGCG
GCATCAGCACCTTGTCGCCTTGCGTATAATATTTGCCCATGGTGAAAACGGGGGC
GAAGAAGTTGTCCATATTGGCCACGTTTAAATCAAAACTGGTGAAACTCACCCAG
GGATTGGCTGAGACGAAAAACATATTCTCAATAAACCCTTTAGGGAAATAGGCC
AGGTTTTCACCGTAACACGCCACATCTTGCGAATATATGTGTAGAAACTGCCGGA
AATCGTCGTGGTATTCACTCCAGAGCGATGAAAACGTTTCAGTTTGCTCATGGAA
AACGGTGTAACAAGGGTGAACACTATCCCATATCACCAGCTCACCGTCTTTCATT
GCCATACGTAATTCCGGATGAGCATTCATCAGGCGGGCAAGAATGTGAATAAAG
GCCGGATAAAACTTGTGCTTATTTTTCTTTACGGTCTTTAAAAAGGCCGTAATATC
CAGCTGAACGGTCTGGTTATAGGTACATTGAGCAACTGACTGAAATGCCTCAAA
ATGTTCTTTACGATGCCATTGGGATATATCAACGGTGGTATATCCAGTGATTTTTT
TCTCCATtttagcttccttagctcctgaaaatctcgacaactcaaaaaatacgcccggtagtgatcttatttcattatggtgaaagttg
gaacctcttacgtgccgatcaacgtctcattttcgccaaaagttggcccagggcttcccggtatcaacagggacaccaggatttatttatt
ctgcgaagtgatcttccgtcacaggtaggcgcgccgaagttcctatactttctagagaataggaacttcggaataggaactaaggagga
tattcatatggaccatggctaattccTTGCCGTTTTCATCATATTTAATCAGCGACTGATCCACCC
AGTCCCAGACGAAGCCGCCCTGTAAACGGGGGTACTGACGAAACGCCTGCCAGT
ATTTAGCGAAGCCGCCAAGACTGTTACCCATCGCGTGGGCATATTCGCAAAGGAT
CAGCGGGCGCATTTCTCCAGGCAGCGAAAGCCATTTTTGATGGACCATTTCGGC
ACCGCCGGGAAGGGCTGGTCTTCATCCACGCGCGCGTACATCGGGCAAATAATA
TCGGTGGCCGTGGTGTCGGCTCCGCCGCCTTCATACTGTACCGGGCGGGAAGGAT
CGACAGATTTGATCCAGCGATACAGCGCGTCGTGATTAGCGCCGTGGCCTGATTC
ATTCCCCAGCGACCAGATGATCACACTCGGGTGATTACGATCGCGCTGCACCATC
CGCGTTACGCGTTCGCTCATCGCGGGTAGCCAGCGCGGATCATCGGTCAGACGAT
TCATTGGCACCATGCCGTGGGTTTCAATATTGGCTTCATCCACCACATACAGGCC
GTAGCGGTCGCACAGCGTGTACCACAGCGGATGGTTCGGATAATGCGAACAGCG
CACGGCGTTAAAGTTGTTCTGCTTCATCAGCAGGATATCCTGCACCATCGTCTGC
TCATCCATGACCTGACCATGCAGAGGATGATGCTCGTGACGGTTAACGCCGCGA
ATCAGCAACGGCTTGCCGTTCAGCAGCAGCAGACCATTTTCAATCCGCACCTCGC
GGAAACCGACGTCGCAGGCTTCTGCTTCAATCAGCGTGCCGTCGGCGGTGTGCAG
TTCAACCACTGCACGATAGAGATTCGGGATTTCGGCGCTCCACAGTTCCGGATTT
TCAACGTTCAGGCGTAGTGTGACGCGATCGGCATAACCGCCACGCTCATCGATAA
TTTCACCcatgtcagccgttaagtgttcctgtgtcactgaaaattgctttgagaggctctaagggcttctcagtgcgttacatccctg
gcttgttgtccacaaccgttaaaccttaaaagctttaaaagccttatatattcttttttttcttataaaacttaaaaccttagaggctatttaagtt
gctgatttatattaattttattgttcaaacatgagagcttagtacgtgaaacatgagagcttagtacgttagccatgagagcttagtacgttag
ccatgagggtttagttcgttaaacatgagagcttagtacgttaaacatgagagcttagtacgtgaaacatgagagcttagtacgtactatc
aacaggttgaactgcggatcttgcggccgcaaaaattaaaaatgaagttttaaatcaatctaaagtatatatgagtaaacttggtctgaca
gTTACCAATGCTTAATCAGTGAGGCACCTATCTCAGCGATCTGTCTATTTCGTTCA
TCCATAGTTGCCTGACTCCCCGTCGTGTAGATAACTACGATACGGGAGGGCTTAC
CATCTGGCCCCAGTGCTGCAATGATACCGCGAGACCCACGCTCACCGGCTCCAG
ATTTATCAGCAATAAACCAGCCAGCCGGAAGGGCCGAGCGCAGAAGTGGTCCTG
CAACTTTATCCGCCTCCATCCAGTCTATTAATTGTTGCCGGGAAGCTAGAGTAAG
TAGTTCGCCAGTTAATAGTTTGCGCAACGTTGTTGCCATTGCTACAGGCATCGTG
GTGTCACGCTCGTCGTTTGGTATGGCTTCATTCAGCTCCGGTTCCCAACGATCAA
 
GGCGAGTTACATGATCCCCCATGTTGTGCAAAAAAGCGGTTAGCTCCTTCGGTCC
TCCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTGG
TGAGTACTCAACCAAGTCATTCTGAGAATAGTGTATGCGGCGACCGAGTTGCTCT
TGCCCGGCGTCAATACGGGATAATACCGCGCCACATAGCAGAACTTTAAAAGTG
CTCATCATTGGAAAACGTTCTTCGGGGCGAAAACTCTCAAGGATCTTACCGCTGT
TGAGATCCAGTTCGATGTAACCCACTCGTGCACCCAACTGATCTTCAGCATCTTTT
ACTTTCACCAGCGTTTCTGGGTGAGCAAAAACAGGAAGGCAAAATGCCGCAAAA
AAGGGAATAAGGGCGACACGGAAATGTTGAATACTCAtactcttcctttttcaatattattgaagcatt
tatcagggttattgtctcatgagcggatacatatttgaatgtatttagaaaaataaacaaataggggttccgcgACTGACGGCT
CCAGGAGTCGTCGCCACCAATCCCCATATGGAAACCGTCGATATTCAGCCATGTG
CCTTCTTCCGCGTGCAGCAGATGGCGATGGCTGGTTTCCATCAGTTGTTGTTGGCT
GTAGCGGCTGATGTTGAACTGGAAGTCGCCGCGCCACTGGTGTGGGCCATAATTC
AATTCGCGCGTCCCGCAGCGCAGACCGTTTTCGCTCGGGAAGACGTACGGGGTAT
ACATGTCTGACAATGGCAGATCCCAGCGGTCAAAACAGGCTGCAGTAAGGCGGT
CGGGATAGTTTTCTTGCGGCCCCAGGCCGAGCCAGTTTACCCGCTCTGAGACCTG
CGCCAGCTGGCAGGTCAGGCCAATCCGCGCCGGATGCGGTGTATCGCTTGCCAC
CGCAACATCCACATTGATGACCATCTCACCGTGCCCATCAATCCGGTAGGTTTTC
CGGCTGATAAATAAGGTTTTCCCCTGATGCTGCCACGCGTGGGCGGTTGTAATCA
GCACCGCGTCGGCAAGTGTATCTGCCGTGCACTGCAACAACGCCGCTTCGGCCTG
GTAATGGCCCGCCGCCTTCCAGCGTTCGACCCAGGCGTTAGGGTCAATGCGGGTC
GCTTCACTTACGCCAATGTCGTTATCCAGCGGCGCACGGGTGAACTGATCGCGCA
GCGGGGTCAGCAGTTGTTTTTCATCGCCAATCCACATCTGTGAAAGAAAGCCTGA
CTGGCGGTTAAATTGCCAACGCTTATTACCCAGCTCGATGCAAAAATCCGTTCCG
CTGGTGGTCAGTTGAGGGATGGCGTGGGACGCGGAGGGGAGTGTCACGCTGAGG
TTTTCCGCCAGACGCCATTGCTGCCAGGCGCTGATGTGTCCGGCTTCTGACCATG
CGGTCGCGTTTGGTTGCACTACGCGTACCGTTAGCCAGAGTcacatttccccgaaaagtgccac
ctgcatcgatggccccccagtgaattcgTTAAGACCCACTTTCACATTTAAGTTGTTTTTCTAATCC
GCATATGATCAATTCAAGGCCGAATAAGAAGGCTGGCTCTGCACCTTGGTGATCA
AATAATTCGATAGCTTGTCGTAATAATGGCGGCATACTATCAGTAGTAGGTGTTT
CCCTTTCTTCTTTAGCGACTTGATGCTCTTGATCTTCCAATACGCAACCTAAAGTA
AAATGCCCCACAGCGCTGAGTGCATATAATGCATTCTCTAGTGAAAAACCTTGTT
GGCATAAAAAGGCTAATTGATTTTCGAGAGTTTCATACTGTTTTTCTGTAGGCCG
TGTACCTAAATGTACTTTTGCTCCATCGCGATGACTTAGTAAAGCACATCTAAAA
CTTTTAGCGTTATTACGTAAAAAATCTTGCCAGCTTTCCCCTTCTAAAGGGCAAA
AGTGAGTATGGTGCCTATCTAACATCTCAATGGCTAAGGCGTCGAGCAAAGCCC
GCTTATTTTTTACATGCCAATACAATGTAGGCTGCTCTACACCTAGCTTCTGGGCG
AGTTTACGGGTTGTTAAACCTTCGATTCCGACCTCATTAAGCAGCTCTAATGCGC
TGTTAATCACTTTACTTTTATCTAATCTAGACATcattaattcctaatttttgttgacactctatcattgatag
agttattttaccactccctatcagtgatagagaaaagtgaactctagaaataattttgtttaactttaagaaggagatatacatATGAAA
CGGAATGCGAAAACTATCATCGCAGGGATGATTGCACTGGCAATTTCACACACC
GCTATGGCTGACGATATTAAAGTCGCCGTTGTCGGCGCGATGTCCGGCCCGATTG
CCCAGTGGGGCGATATGGAATTTAACGGCGCGCGTCAGGCAATTAAAGACATTA
ATGCCAAAGGGGGAATTAAGGGCGATAAACTGGTTGGCGTGGAATATGACGACG
CATGCGACCCGAAACAAGCCGTTGCGGTCGCCAACAAAATCGTTAATGACGGCA
TTAAATACGTTATTGGTCATCTGTGTTCTTCTTCTACCCAGCCTGCGTCAGATATC
TATGAAGACGAAGGTATTCTGATGATCTCGCCGGGAGCGACCAACCCGGAGCTG
ACCCAACGCGGTTATCAACACATTATGCGTACTGCCGGGCTGGACTCTTCCCAGG
GGCCAACGGCGGCAAAATACATTCTTGAGACGGTGAAGCCCCAGCGCATCGCCA
TCATTCACGACAAACAACAGTATGGCGAAGGGCTGGCGCGTTCGGTGCAGGACG
 
GGCTGAAAGCGGCTAACGCCAACGTCGTCTTCTTCGACGGTATTACCGCCGGGA
GAAAGATTTCTCCGCGCTGATCGCCCGCCTGAAAAAGAAAACATCGACTTCGTT
TACTACGGCGGTTACTACCCGGAAATGGGGCAGATGCTGCGCCAGGCCCGTTCC
GTTGGCCTGAAAACCCAGTTTATGGGGCCGGAAGGTGTGGGTAATGCGTCGTTGT
CGAACATTGCCGGTGATGCCGCCGAAGGCATGTTGGTCACTATGCCAAAACGCT
ATGACCAGGATCCGGCAAACCAGGGCATCGTTGATGCGCTGAAAGCAGACAAGA
AAGATCCGTCCGGGCCTTATGTCTGGATCACCTACGCGGCGGTGCAATCTCTGGC
GACTGCCCTTGAGCGTACCGGCAGCGATGAGCCGCTGGCGCTGGTGAAAGATTT
AAAAGCTAACGGTGCAAACACCGTGATTGGGCCGCTGAACTGGGATGAAAAAGG
CGATCTTAAGGGATTTGATTTTGGTGTCTTCCAGTGGCACGCCGACGGTTCATCC
ACGGCAGCCAAGTGAtcatcccaccgcccgtaaaatgcgggcgggtttagaaaggttaccttATGTCTGAGC
AGTTTTTGTATTTCTTGCAGCAGATGTTTAACGGCGTCACGCTGGGCAGTACCTA
CGCGCTGATAGCCATCGGCTACACCATGGTTTACGGCATTATCGGCATGATCAAC
TTCGCCCACGGCGAGGTTTATATGATTGGCAGCTACGTCTCATTTATGATCATCG
CCGCGCTGATGATGATGGGCATTGATACCGGCTGGCTGCTGGTAGCTGCGGGATT
CGTCGGCGCAATCGTCATTGCCAGCGCCTACGGCTGGAGTATCGAACGGGTGGCT
TACCGCCCGGTGCGTAACTCTAAGCGCCTGATTGCACTCATCTCTGCAATCGGTA
TGTCCATCTTCCTGCAAAACTACGTCAGCCTGACCGAAGGTTCGCGCGACGTGGC
GCTGCCGAGCCTGTTTAACGGTCAGTGGGTGGTGGGGCATAGCGAAAACTTCTCT
GCCTCTATTACCACCATGCAGGCGGTGATCTGGATTGTTACCTTCCTCGCCATGCT
GGCGCTGACGATTTTCATTCGCTATTCCCGCATGGGTCGCGCGTGTCGTGCCTGC
GCGGAAGATCTGAAAATGGCGAGTCTGCTTGGCATTAACACCGACCGGGTGATT
GCGCTGACCTTTGTGATTGGCGCGGCGATGGCGGCGGTGGCGGGTGTGCTGCTCG
GTCAGTTCTACGGCGTCATTAACCCCTACATCGGCTTTATGGCCGGGATGAAAGC
CTTTACCGCGGCGGTGCTCGGTGGGATTGGCAGCATTCCGGGAGCGATGATTGGC
GGCCTGATTCTGGGGATTGCGGAGGCGCTCTCTTCTGCCTATCTGAGTACGGAAT
ATAAAGATGTGGTGTCATTCGCCCTGCTGATTCTGGTGCTGCTGGTGATGCCGAC
CGGTATTCTGGGTCGCCCGGAGGTAGAGAAAGTATGAAACCGATGCATATTGCA
ATGGCGCTGCTCTCTGCCGCGATGTTCTTTGTGCTGGCGGGCGTCTTTATGGGCGT
GCAACTGGAGCTGGATGGCACCAAACTGGTGGTCGACACGGCTTCGGATGTCCG
TTGGCAGTGGGTGTTTATCGGCACGGCGGTGGTCTTTTTCTTCCAGCTTTTGCGAC
CGGCTTTCCAGAAAGGGTTGAAAAGCGTTTCCGGACCGAAGTTTATTCTGCCCGC
CATTGATGGCTCCACGGTGAAGCAGAAACTGTTCCTCGTGGCGCTGTTGGTGCTT
GCGGTGGCGTGGCCGTTTATGGTTTCACGCGGGACGGTGGATATTGCCACCCTGA
CCATGATCTACATTATCCTCGGTCTGGGGCTGAACGTGGTTGTTGGTCTTTCTGGT
CTGCTGGTGCTGGGGTACGGCGGTTTTTACGCCATCGGCGCTTACACTTTTGCGCT
GCTCAATCACTATTACGGCTTGGGCTTCTGGACCTGCCTGCCGATTGCTGGATTA
ATGGCAGCGGCGGCGGGCTTCCTGCTCGGTTTTCCGGTGCTGCGTTTGCGCGGTG
ACTATCTGGCGATCGTTACCCTCGGTTTCGGCGAAATTGTGCGCATATTGCTGCTC
AATAACACCGAAATTACCGGCGGCCCGAACGGAATCAGTCAGATCCCGAAACCG
ACACTCTTCGGACTCGAGTTCAGCCGTACCGCTCGTGAAGGCGGCTGGGACACGT
TCAGTAATTTCTTTGGCCTGAAATACGATCCCTCCGATCGTGTCATCTTCCTCTAC
CTGGTGGCGTTGCTGCTGGTGGTGCTAAGCCTGTTTGTCATTAACCGCCTGCTGC
GGATGCCGCTGGGGCGTGCGTGGGAAGCGTTGCGTGAAGATGAAATCGCCTGCC
GTTCGCTGGGCTTAAGCCCGCGTCGTATCAAGCTGACTGCCTTTACCATAAGTGC
CGCGTTTGCCGGTTTTGCCGGAACGCTGTTTGCGGCGCGTCAGGGCTTTGTCAGC
CCGGAATCCTTCACCTTTGCCGAATCGGCGTTTGTGCTGGCGATAGTGGTGCTCG
GCGGTATGGGCTCGCAATTTGCGGTGATTCTGGCGGCAATTTTGCTGGTGGTGTC
GCGCGAGTTGATGCGTGATTTCAACGAATACAGCATGTTAATGCTCGGTGGTTTG
ATGGTGCTGATGATGATCTGGCGTCCGCAGGGCTTGCTGCCCATGACGCGCCCGC
 
AACTGAAGCTGAAAAACGGCGCAGCGAAAGGAGAGCAGGCATGAGTCAGCCAT
TATTATCTGTTAACGGCCTGATGATGCGCTTCGGCGGCCTGCTGGCGGTGAACAA
CGTCAATCTTGAACTGTACCCGCAGGAGATCGTCTCGTTAATCGGCCCTAACGGT
GCCGGAAAAACCACGGTTTTTAACTGTCTGACCGGATTCTACAAACCCACCGGCG
GCACCATTTTACTGCGCGATCAGCACCTGGAAGGTTTACCGGGGCAGCAAATTGC
CCGCATGGGCGTGGTGCGCACCTTCCAGCATGTGCGTCTGTTCCGTGAAATGACG
GTAATTGAAAACCTGCTGGTGGCGCAGCATCAGCAACTGAAAACCGGGCTGTTC
TCTGGCCTGTTGAAAACGCCATCCTTCCGTCGCGCCCAGAGCGAAGCGCTCGACC
GCGCCGCGACCTGGCTTGAGCGCATTGGTTTGCTGGAACACGCCAACCGTCAGG
CGAGTAACCTGGCCTATGGTGACCAGCGCCGTCTTGAGATTGCCCGCTGCATGGT
GACGCAGCCGGAGATTTTAATGCTCGACGAACCTGCGGCAGGTCTTAACCCGAA
AGAGACGAAAGAGCTGGATGAGCTGATTGCCGAACTGCGCAATCATCACAACAC
CACTATCTTGTTGATTGAACACGATATGAAGCTGGTGATGGGAATTTCGGACCGA
ATTTACGTGGTCAATCAGGGGACGCCGCTGGCAAACGGTACGCCGGAGCAGATC
CGTAATAACCCGGACGTGATCCGTGCCTATTTAGGTGAGGCATAAgATGGAAAAA
GTCATGTTGTCCTTTGACAAAGTCAGCGCCCACTACGGCAAAATCCAGGCGCTGC
ATGAGGTGAGCCTGCATATCAATCAGGGCGAGATTGTCACGCTGATTGGCGCGA
ACGGGGCGGGGAAAACCACCTTGCTCGGCACGTTATGCGGCGATCCGCGTGCCA
CCAGCGGGCGAATTGTGTTTGATGATAAAGACATTACCGACTGGCAGACAGCGA
AAATCATGCGCGAAGCGGTGGCGATTGTCCCGGAAGGGCGTCGCGTCTTCTCGC
GGATGACGGTGGAAGAGAACCTGGCGATGGGCGGTTTTTTTGCTGAACGCGACC
AGTTCCAGGAGCGCATAAAGTGGGTGTATGAGCTGTTTCCACGTCTGCATGAGCG
CCGTATTCAGCGGGCGGGCACCATGTCCGGCGGTGAACAGCAGATGCTGGCGAT
TGGTCGTGCGCTGATGAGCAACCCGCGTTTGCTACTGCTTGATGAGCCATCGCTC
GGTCTTGCGCCGATTATCATCCAGCAAATTTTCGACACCATCGAGCAGCTGCGCG
AGCAGGGGATGACTATCTTTCTCGTCGAGCAGAACGCCAACCAGGCGCTAAAGC
TGGCGGATCGCGGCTACGTGCTGGAAAACGGCCATGTAGTGCTTTCCGATACTGG
TGATGCGCTGCTGGCGAATGAAGCGGTGAGAAGTGCGTATTTAGGCGGGTAAccg
atggtagtgtggggtctccccatgcgagagtagggaactgccaggcatcaaataaaacgaaaggctcagtcgaaagactgggccttt
cgttttatctgttgtttgtcggtgaacgctctcctgagtaggacaaatccgccgggagcggatttgaacgttgcgaagcaacggcccgg
agggtggcgggcaggacgcccgccataaactgccaggcatcaaattaagcagaaggccatcctgacggatggcctttttgcgtggc
cagtgccaagcttgcatgc
SEQ8: E. coli Nissle 1917 leucine exporter gene leuE 
GTGTTCGCTGAATACGGGGTTCTGAATTACTGGACCTATCTGGTTGGGGCCATTT
TTATTGTGTTGGTGCCAGGGCCAAATACCCTGTTTGTACTCAAAAATAGCGTCAG
TAGCGGTATGAAAGGCGGTTATCTTGCGGCCTGTGGTGTATTTATTGGCGATGCG
GTATTGATGTTTCTGGCATGGGCTGGAGTGGCGACATTAATTAAGACCACCCCGA
TATTATTCAACATCGTACGTTATCTTGGTGCGTTTTATTTGCTCTATCTGGGGAGT
AAAATTCTCTACGCGACCCTGAAAGGTAAAAATAGCGAGACCAAATCCGATGAG
CCCCAATACGGTGCCATTTTTAAACGCGCGTTAATTTTGAGCCTGACTAATCCGA
AAGCCATTTTGTTCTATGTGTCGTTTTTCGTACAGTTTATCGATGTTAATGCCCCA
CATACGGGAATTTCATTCTTTATTCTGGCGACGACGCTGGAACTGGTGAGTTTCT
GCTATTTGAGCTTCCTGATTATTTCTGGGGCTTTTGTCACGCAGTACATACGTACC
AAAAAGAAACTGGCTAAAGTGGGCAACTCACTGATTGGTTTGATGTTCGTGGGTT
TCGCCGCCCGACTGGCGACGCTGCAATCCTGA
SEQ9: leuE deletion construct: 
cattttaaataccatttattggttactttttagcaccatatcagcgaagaatcagggaggattatagatgggaagcccatgcagattgcagc
attacacgtcttgagcgattgtgtaggctggagctgcttcgaagttcctatactttctagagaataggaacttcggaataggaacttcaag
atcccctcacgctgccgcaagcactcagggcgcaagggctgctaaaggaagcggaacacgtagaaagccagtccgcagaaacgg
tgctgaccccggatgaatgtcagctactgggctatctggacaagggaaaacgcaagcgcaaagagaaagcaggtagcttgcagtgg
gcttacatggcgatagctagactgggcggttttatggacagcaagcgaaccggaattgccagctggggcgccctctggtaaggttgg
gaagccctgcaaagtaaactggatggctttcttgccgccaaggatctgatggcgcaggggatcaagatctgatcaagagacaggatg
aggatcgtttcgcATGATTGAACAAGATGGATTGCACGCAGGTTCTCCGGCCGCTTGGGT
GGAGAGGCTATTCGGCTATGACTGGGCACAACAGACAATCGGCTGCTCTGATGC
CGCCGTGTTCCGGCTGTCAGCGCAGGGGCGCCCGGTTCTTTTTGTCAAGACCGAC
CTGTCCGGTGCCCTGAATGAACTGCAGGACGAGGCAGCGCGGCTATCGTGGCTG
GCCACGACGGGCGTTCCTTGCGCAGCTGTGCTCGACGTTGTCACTGAAGCGGGAA
GGGACTGGCTGCTATTGGGCGAAGTGCCGGGGCAGGATCTCCTGTCATCTCACCT
TGCTCCTGCCGAGAAAGTATCCATCATGGCTGATGCAATGCGGCGGCTGCATACG
CTTGATCCGGCTACCTGCCCATTCGACCACCAAGCGAAACATCGCATCGAGCGA
GCACGTACTCGGATGGAAGCCGGTCTTGTCGATCAGGATGATCTGGACGAAGAG
CATCAGGGGCTCGCGCCAGCCGAACTGTTCGCCAGGCTCAAGGCGCGCATGCCC
GACGGCGAGGATCTCGTCGTGACCCATGGCGATGCCTGCTTGCCGAATATCATGG
TGGAAAATGGCCGCTTTTCTGGATTCATCGACTGTGGCCGGCTGGGTGTGGCGGA
CCGCTATCAGGACATAGCGTTGGCTACCCGTGATATTGCTGAAGAGCTTGGCGGC
GAATGGGCTGACCGCTTCCTCGTGCTTTACGGTATCGCCGCTCCCGATTCGCAGC
GCATCGCCTTCTATCGCCTTCTTGACGAGTTCTTCTGAgcgggactctggggttcgaaatgaccga
ccaagcgacgcccaacctgccatcacgagatttcgattccaccgccgccttctatgaaaggttgggcttcggaatcgttttccgggacg
ccggctggatgatcctccagcgcggggatctcatgctggagttcttcgcccaccccagcttcaaaagcgctctgaagttcctatactttct
agagaataggaacttcggaataggaactaaggaggatattcatatggaccatggctaattcccaattaacctctttaattatctttcgatcat
gcgcgattaaaggtgaatatgctaaccaatctgtagcggcttagaaaggagaaaatcaggttttaacctga
SEQ10: Tet-livKHMGF fragment 
aatagggggttccgcgACTGACGGCTCCAGGAGTCGTCGCCACCAATCCCCATATGGAAA
CCGTCGATATTCAGCCATGTGCCTTCTTCCGCGTGCAGCAGATGGCGATGGCTGG
TTTCCATCAGTTGTTGTTGGCTGTAGCGGCTGATGTTGAACTGGAAGTCGCCGCG
CCACTGGTGTGGGCCATAATTCAATTCGCGCGTCCCGCAGCGCAGACCGTTTTCG
CTCGGGAAGACGTACGGGGTATACATGTCTGACAATGGCAGATCCCAGCGGTCA
AAACAGGCTGCAGTAAGGCGGTCGGGATAGTTTTCTTGCGGCCCCAGGCCGAGC
CAGTTTACCCGCTCTGAGACCTGCGCCAGCTGGCAGGTCAGGCCAATCCGCGCCG
GATGCGGTGTATCGCTTGCCACCGCAACATCCACATTGATGACCATCTCACCGTG
CCCATCAATCCGGTAGGTTTTCCGGCTGATAAATAAGGTTTTCCCCTGATGCTGC
CACGCGTGGGCGGTTGTAATCAGCACCGCGTCGGCAAGTGTATCTGCCGTGCACT
GCAACAACGCCGCTTCGGCCTGGTAATGGCCCGCCGCCTTCCAGCGTTCGACCCA
GGCGTTAGGGTCAATGCGGGTCGCTTCACTTACGCCAATGTCGTTATCCAGCGGC
GCACGGGTGAACTGATCGCGCAGCGGGGTCAGCAGTTGTTTTTCATCGCCAATCC
ACATCTGTGAAAGAAAGCCTGACTGGCGGTTAAATTGCCAACGCTTATTACCCAG
CTCGATGCAAAAATCCGTTCCGCTGGTGGTCAGTTGAGGGATGGCGTGGGACGC
GGAGGGGAGTGTCACGCTGAGGTTTTCCGCCAGACGCCATTGCTGCCAGGCGCT
GATGTGTCCGGCTTCTGACCATGCGGTCGCGTTTGGTTGCACTACGCGTACCGTT
AGCCAGAGTcacatttccccgaaaagtgccacctgcatcgatggccccccagtgaattcgTTAAGACCCACTTT
CACATTTAAGTTGTTTTTCTAATCCGCATATGATCAATTCAAGGCCGAATAAGAA
GGCTGGCTCTGCACCTTGGTGATCAAATAATTCGATAGCTTGTCGTAATAATGGC
GGCATACTATCAGTAGTAGGTGTTTCCCTTTCTTCTTTAGCGACTTGATGCTCTTG
 
ATCTTCCAATACGCAACCTAAAGTAAAATGCCCCACAGCGCTGAGTGCATATAAT
GCATTCTCTAGTGAAAAACCTTGTTGGCATAAAAAGGCTAATTGATTTTCGAGAG
TTTCATACTGTTTTTCTGTAGGCCGTGTACCTAAATGTACTTTTGCTCCATCGCGA
TGACTTAGTAAAGCACATCTAAAACTTTTAGCGTTATTACGTAAAAAATCTTGCC
AGCTTTCCCCTTCTAAAGGGCAAAAGTGAGTATGGTGCCTATCTAACATCTCAAT
GGCTAAGGCGTCGAGCAAAGCCCGCTTATTTTTTACATGCCAATACAATGTAGGC
TGCTCTACACCTAGCTTCTGGGCGAGTTTACGGGTTGTTAAACCTTCGATTCCGAC
CTCATTAAGCAGCTCTAATGCGCTGTTAATCACTTTACTTTTATCTAATCTAGACA
Tcattaattcctaatttttgttgacactctatcattgatagagttattttaccactccctatcagtgatagagaaaagtgaactctagaaataat
tttgtttaactttaagaaggagatatacatATGAAACGGAATGCGAAAACTATCATCGCAGGGATGA
TTGCACTGGCAATTTCACACACCGCTATGGCTGACGATATTAAAGTCGCCGTTGT
CGGCGCGATGTCCGGCCCGATTGCCCAGTGGGGCGATATGGAATTTAACGGCGC
GCGTCAGGCAATTAAAGACATTAATGCCAAAGGGGGAATTAAGGGCGATAAACT
GGTTGGCGTGGAATATGACGACGCATGCGACCCGAAACAAGCCGTTGCGGTCGC
CAACAAAATCGTTAATGACGGCATTAAATACGTTATTGGTCATCTGTGTTCTTCTT
CTACCCAGCCTGCGTCAGATATCTATGAAGACGAAGGTATTCTGATGATCTCGCC
GGGAGCGACCAACCCGGAGCTGACCCAACGCGGTTATCAACACATTATGCGTAC
TGCCGGGCTGGACTCTTCCCAGGGGCCAACGGCGGCAAAATACATTCTTGAGAC
GGTGAAGCCCCAGCGCATCGCCATCATTCACGACAAACAACAGTATGGCGAAGG
GCTGGCGCGTTCGGTGCAGGACGGCTGAAAGCGGCTAACGCCAACGTCGTCTT
CTTCGACGGTATTACCGCCGGGGAGAAAGATTTCTCCGCGCTGATCGCCCGCCTG
AAAAAAGAAAACATCGACTTCGTTTACTACGGCGGTTACTACCCGGAAATGGGG
CAGATGCTGCGCCAGGCCCGTTCCGTTGGCCTGAAAACCCAGTTTATGGGGCCGG
AAGGTGTGGGTAATGCGTCGTTGTCGAACATTGCCGGTGATGCCGCCGAAGGCA
TGTTGGTCACTATGCCAAAACGCTATGACCAGGATCCGGCAAACCAGGGCATCG
TTGATGCGCTGAAAGCAGACAAGAAAGATCCGTCCGGGCCTTATGTCTGGATCA
CCTACGCGGCGGTGCAATCTCTGGCGACTGCCCTTGAGCGTACCGGCAGCGATG
AGCCGCTGGCGCTGGTGAAAGATTTAAAAGCTAACGGTGCAAACACCGTGATTG
GGCCGCTGAACTGGGATGAAAAAGGCGATCTTAAGGGATTTGATTTTGGTGTCTT
CCAGTGGCACGCCGACGGTTCATCCACGGCAGCCAAGTGAtcatcccaccgcccgtaaaatgc
gggcgggtttagaaaggttaccttATGTCTGAGCAGTTTTTGTATTTCTTGCAGCAGATGTTTAA
CGGCGTCACGCTGGGCAGTACCTACGCGCTGATAGCCATCGGCTACACCATGGTT
TACGGCATTATCGGCATGATCAACTTCGCCCACGGCGAGGTTTATATGATTGGCA
GCTACGTCTCATTTATGATCATCGCCGCGCTGATGATGATGGGCATTGATACCGG
CTGGCTGCTGGTAGCTGCGGGATTCGTCGGCGCAATCGTCATTGCCAGCGCCTAC
GGCTGGAGTATCGAACGGGTGGCTTACCGCCCGGTGCGTAACTCTAAGCGCCTG
ATTGCACTCATCTCTGCAATCGGTATGTCCATCTTCCTGCAAAACTACGTCAGCCT
GACCGAAGGTTCGCGCGACGTGGCGCTGCCGAGCCTGTTTAACGGTCAGTGGGT
GGTGGGGCATAGCGAAAACTTCTCTGCCTCTATTACCACCATGCAGGCGGTGATC
TGGATTGTTACCTTCCTCGCCATGCTGGCGCTGACGATTTTCATTCGCTATTCCCG
CATGGGTCGCGCGTGTCGTGCCTGCGCGGAAGATCTGAAAATGGCGAGTCTGCTT
GGCATTAACACCGACCGGGTGATTGCGCTGACCTTTGTGATTGGCGCGGCGATGG
CGGCGGTGGCGGGTGTGCTGCTCGGTCAGTTCTACGGCGTCATTAACCCCTACAT
CGGCTTTATGGCCGGGATGAAAGCCTTTACCGCGGCGGTGCTCGGTGGGATTGGC
AGCATTCCGGGAGCGATGATTGGCGGCCTGATTCTGGGGATTGCGGAGGCGCTCT
CTTCTGCCTATCTGAGTACGGAATATAAAGATGTGGTGTCATTCGCCCTGCTGAT
TCTGGTGCTGCTGGTGATGCCGACCGGTATTCTGGGTCGCCCGGAGGTAGAGAAA
GTATGAAACCGATGCATATTGCAATGGCGCTGCTCTCTGCCGCGATGTTCTTTGT
GCTGGCGGGCGTCTTTATGGGCGTGCAACTGGAGCTGGATGGCACCAAACTGGT
GGTCGACACGGCTTCGGATGTCCGTTGGCAGTGGGTGTTTATCGGCACGGCGGTG
 
GTCTTTTCTTCCAGCTTTGCGACCGGCTTTCCAGAAAGGGTTGAAAGCGTTTC
CGGACCGAAGTTATTCTGCCCGCCATTGATGGCTCCACGGTGAAGCAGAAACTG
TTCCTCGTGGCGCTGTTGGTGCTTGCGGTGGCGTGGCCGTTTATGGTTTCACGCGG
GACGGTGGATATTGCCACCCTGACCATGATCTACATTATCCTCGGTCTGGGGCTG
AACGTGGTTGTTGGTCTTTCTGGTCTGCTGGTGCTGGGGTACGGCGGTTTTTACGC
CATCGGCGCTTACACTTTTGCGCTGCTCAATCACTATTACGGCTTGGGCTTCTGGA
CCTGCCTGCCGATTGCTGGATTAATGGCAGCGGCGGCGGGCTTCCTGCTCGGTTT
TCCGGTGCTGCGTTTGCGCGGTGACTATCTGGCGATCGTTACCCTCGGTTTCGGC
GAAATTGTGCGCATATTGCTGCTCAATAACACCGAAATTACCGGCGGCCCGAAC
GGAATCAGTCAGATCCCGAAACCGACACTCTTCGGACTCGAGTTCAGCCGTACC
GCTCGTGAAGGCGGCTGGGACACGTTCAGTAATTTCTTTGGCCTGAAATACGATC
CCTCCGATCGTGTCATCTTCCTCTACCTGGTGGCGTTGCTGCTGGTGGTGCTAAGC
CTGTTTGTCATTAACCGCCTGCTGCGGATGCCGCTGGGGCGTGCGTGGAAGCGT
TGCGTGAAGATGAAATCGCCTGCCGTTCGCTGGGCTTAAGCCCGCGTCGTATCAA
GCTGACTGCCTTTACCATAAGTGCCGCGTTTGCCGGTTTTGCCGGAACGCTGTTTG
CGGCGCGTCAGGGCTTTGTCAGCCCGGAATCCTTCACCTTTGCCGAATCGGCGTT
TGTGCTGGCGATAGTGGTGCTCGGCGGTATGGGCTCGCAATTTGCGGTGATTCTG
GCGGCAATTTTGCTGGTGGTGTCGCGCGAGTTGATGCGTGATTTCAACGAATACA
GCATGTTAATGCTCGGTGGTTTGATGGTGCTGATGATGATCTGGCGTCCGCAGGG
CTTGCTGCCCATGACGCGCCCGCAACTGAAGCTGAAAAACGGCGCAGCGAAAGG
AGAGCAGGCATGAGTCAGCCATTATTATCTGTTAACGGCCTGATGATGCGCTTCG
GCGGCCTGCTGGCGGTGAACAACGTCAATCTTGAACTGTACCCGCAGGAGATCG
TCTCGTTAATCGGCCCTAACGGTGCCGGAAAAACCACGGTTTTAACTGTCTGAC
CGGATTCTACAAACCCACCGGCGGCACCATTTACTGCGCGATCAGCACCTGGAA
GGTTTACCGGGGCAGCAAATTGCCCGCATGGCGTGGTGCGCACCTTCCAGCATG
TGCGTCTGTTCCGTGAAATGACGGTAATTGAAAACCTGCTGGTGGCGCAGCATCA
GCAACTGAAAACCGGGCTGTTCTCTGGCCTGTTGAAAACGCCATCCTTCCGTCGC
GCCCAGAGCGAAGCGCTCGACCGCGCCGCGACCTGGCTTGAGCGCATTGGTTTG
CTGGAACACGCCAACCGTCAGGCGAGTAACCTGGCCTATGGTGACCAGCGCCGT
CTTGAGATTGCCCGCTGCATGGTGACGCAGCCGGAGATTTTAATGCTCGACGAAC
CTGCGGCAGGTCTTAACCCGAAAGAGACGAAAGAGCTGGATGAGCTGATTGCCG
AACTGCGCAATCATCACAACACCACTATCTTGTTGATTGAACACGATATGAAGCT
GGTGATGGGAATTTCGGACCGAATTTACGTGGTCAATCAGGGGACGCCGCTGGC
AAACGGTACGCCGGAGCAGATCCGTAATAACCCGGACGTGATCCGTGCCTATTT
AGGTGAGGCATAAgATGGAAAAAGTCATGTTGTCCTTTGACAAAGTCAGCGCCCA
CTACGGCAAAATCCAGGCGCTGCATGAGGTGAGCCTGCATATCAATCAGGGCGA
GATTGTCACGCTGATTGGCGCGAACGGGGCGGGGAAAACCACCTTGCTCGGCAC
GTTATGCGGCGATCCGCGTGCCACCAGCGGGCGAATTGTGTTTGATGATAAAGAC
ATTACCGACTGGCAGACAGCGAAAATCATGCGCGAAGCGGTGGCGATTGTCCCG
GAAGGGCGTCGCGTCTTCTCGCGGATGACGGTGGAAGAGAACCTGGCGATGGGC
GGTTTTTTTGCTGAACGCGACCAGTTCCAGGAGCGCATAAAGTGGGTGTATGAGC
TGTTTCCACGTCTGCATGAGCGCCGTATTCAGCGGGCGGGCACCATGTCCGGCGG
TGAACAGCAGATGCTGGCGATTGGTCGTGCGCTGATGAGCAACCCGCGTTTGCTA
CTGCTTGATGAGCCATCGCTCGGTCTTGCGCCGATTATCATCCAGCAAATTTTCG
ACACCATCGAGCAGCTGCGCGAGCAGGGGATGACTATCTTTCTCGTCGAGCAGA
ACGCCAACCAGGCGCTAAAGCTGGCGGATCGCGGCTACGTGCTGGAAAACGGCC
ATGTAGTGCTTTCCGATACTGGTGATGCGCTGCTGGCGAATGAAGCGGTGAGAA
GTGCGTATTTAGGCGGGTAAccgatggtagtgtggggtctccccatgcgagagtagggaactgccaggcatcaaa
taaaacgaaaggctcagtcgaaagactgggcctttcgttttatctgttgtttgtcggtgaacgctctcctgagtaggacaaatccgccgg
gagcggatttgaacgttgcgaagcaacggcccggagggtggcgggcaggacgcccgccataaactgccaggcatcaaattaagca
 
gaaggccatcctgacggatggcctttttgcgtggccagtgccaagcttgcatgcagattgcagcattacacgtcttgagcgattgtgtag
gctggagctgcttcgaagttcctatactttctagagaataggaacttcggaataggaacttcatttaaatggcgcgccttacgccccgccc
tgccacTCATCGCAGTACTGTTGTATTCATTAAGCATCTGCCGACATGGAAGCCATC
ACAAACGGCATGATGAACCTGAATCGCCAGCGGCATCAGCACCTTGTCGCCTTG
CGTATAATATTTGCCCATGGTGAAAACGGGGGCGAAGAAGTTGTCCATATTGGCC
ACGTTTAAATCAAAACTGGTGAAACTCACCCAGGGATTGGCTGAGACGAAAAAC
ATATTCTCAATAAACCCTTTAGGGAAATAGGCCAGGTTTTCACCGTAACACGCCA
CATCTTGCGAATATATGTGTAGAAACTGCCGGAAATCGTCGTGGTATTCACTCCA
GAGCGATGAAAACGTTTCAGTTTGCTCATGGAAAACGGTGTAACAAGGGTGAAC
ACTATCCCATATCACCAGCTCACCGTCTTTCATTGCCATACGTAATTCCGGATGA
GCATTCATCAGGCGGGCAAGAATGTGAATAAAGGCCGGATAAAACTTGTGCTTA
TTTTTCTTTACGGTCTTTAAAAAGGCCGTAATATCCAGCTGAACGGTCTGGTTATA
GGTACATTGAGCAACTGACTGAAATGCCTCAAAATGTTCTTTACGATGCCATTGG
GATATATCAACGGTGGTATATCCAGTGATTTTTTTCTCCATtttagcttccttagctcctgaaaatc
tcgacaactcaaaaaatacgcccggtagtgatcttatttcattatggtgaaagttggaacctcttacgtgccgatcaacgtctcattttcgcc
aaaagttggcccagggcttcccggtatcaacagggacaccaggatttatttattctgcgaagtgatcttccgtcacaggtaggcgcgcc
gaagttcctatactttctagagaataggaacttcggaataggaactaaggaggatattcatatggaccatggctaattccTTGCCGT
TTTCATCATATTTAATCAGCGACTGATCCACCCAGTCCCAGACGAAGCCGCCCTG
TAAACGGGGGTACTGACGAAACGCCTGCCAGTATTTAGCGAAGCCGCCAAGACT
GTTACCCATCGCGTGGGCATATTCGCAAAGGATCAGCGGGCGCATTTCTCCAGGC
AGCGAAAGCCATTTTTTGATGGACCATTTCGGCACCGCCGGGAAGGGCTGGTCTT
CATCCACGCGCGCGTACATCGGGCAAATAATATCGGTGGCCGTGGTGTCGGCTCC
GCCGCCTTCATACTGTACCGGGCGGGAAGGATCGACAGATTTGATCCAGCGATA
CAGCGCGTCGTGATTAGCGCCGTGGCCTGATTCATTCCCCAGCGACCAGATGATC
ACACTCGGGTGATTACGATCGCGCTGCACCATCCGCGTTACGCGTTCGCTCATCG
CGGGTAGCCAGCGCGGATCATCGGTCAGACGATTCATTGGCACCATGCCGTGGG
TTTCAATATTGGCTTCATCCACCACATACAGGCCGTAGCGGTCGCACAGCGTGTA
CCACAGCGGATGGTTCGGATAATGCGAACAGCGCACGGCGTTAAAGTTGTTCTG
CTTCATCAGCAGGATATCCTGCACCATCGTCTGCTCATCCATGACCTGACCATGC
AGAGGATGATGCTCGTGACGGTTAACGCCGCGAATCAGCAACGGCTTGCCGTTC
AGCAGCAGCAGACCATTTTCAATCCGCACCTCGCGGAAACCGACGTCGCAGGCT
TCTGCTTCAATCAGCGTGCCGTCGGCGGTGTGCAGTTCAACCACTGCACGATAGA
GATTCGGGATTTCGGCGCTCCACAGTTCCGGATTTTCAACGTTCAGGCGTAGTGT
GACGCGATCGGCATAACCGCCACGCTCATCGATAATTTCACCcatgtcagccgttaag
SEQ ID NO:12 - *livJ* sequence 
ATGAACATAAAGGGTAAAGCGTTACTGGCAGGATGTATCGCGCTGGCATTCAGC
AATATGGCTCTGGCAGAAGATATTAAAGTCGCGGTCGTGGGCGCAATGTCCGGT
CCGGTTGCGCAGTACGGTGACCAGGAGTTTACCGGCGCAGAGCAGGCGGTTGCG
GATATCAACGCTAAAGGCGGCATTAAAGGCAACAAACTGCAAATCGTAAAATAT
GACGATGCCTGTGACCCGAAACAGGCGGTTGCGGTGGCGAACAAAGTCGTTAAC
GACGGCATTAAATATGTGATTGGTCACCTCTGTTCTTCATCAACGCAGCCTGCGT
CTGACATCTACGAAGACGAAGGCATTTTAATGATCACCCCAGCGGCAACCGCGC
CGGAGCTGACCGCCCGTGGCTATCAGCTGATCCTGCGCACCACCGGCCTGGACTC
CGACCAGGGGCCGACGGCGGCGAAATATATTCTTGAGAAAGTGAAACCGCAGCG
TATTGCTATCGTTCACGACAAACAGCAATACGGCGAAGGTCTGGCGCGAGCGGT
GCAGGACGGCCTGAAGAAAGGCAATGCAAACGTGGTGTTCTTTGATGGCATCAC
 
CGCCGGGGAAAAAGATTTCTCAACGCTGGTGGCGCGTCTGAAAAAAGAGAATAT
CGACTTCGTTTACTACGGCGGTTATCACCCGGAAATGGGGCAAATCCTGCGTCAG
GCACGCGCGGCAGGGCTGAAAACTCAGTTTATGGGGCCGGAAGGTGTGGCTAAC
GTTTCGCTGTCTAACATTGCGGGCGAATCAGCGGAAGGGCTGCTGGTGACCAAG
CCGAAGAACTACGATCAGGTTCCGGCGAACAAACCCATTGTTGACGCGATCAAA
GCGAAAAAACAGGACCCAAGTGGCGCATTCGTTTGGACCACCTACGCCGCGCTG
CAATCTTTGCAGGCGGGCCTGAATCAGTCTGACGATCCGGCTGAAATCGCCAAAT
ACCTGAAAGCGAACTCCGTGGATACCGTAATGGGACCGCTGACCTGGGATGAGA
AAGGCGATCTGAAAGGCTTTGAGTTCGGCGTATTTGACTGGCACGCCAACGGCA
CGGCGACCGATGCGAAGTAA
SEQ11: Ptac-livJ construct 
AGACAACAAGTCCACGTTGCAGGAACTGGCTGACCGTTACGGTGTTTCCGCTGAG
CGTGTGCGTCAGCTGGAAAAGAACGCGATGAAAAAATTGCGCGCTGCCATTGAA
GCGTAAtttccgctattaagcagagaaccctggatgagagtccggggtttttgtttttgggcctctacaataatcaattccccctccg
gcaaaacgccaatccccacgcagattgttaataaactgtcaaaatagctataacacatttccccgaaaagtgccgatggcccccgatg
gtagtgtggcccatgcgagagtagggaactgccaggcatcaaataaaacgaaaggctcagtcgaaagactgggcctttcgttttatct
gttgtttgtcggtgaacgctctcctgagtaggacaaatccgccgggagcggatttgaacgttgcgaagcaacggcccggagggtggc
gggcaggacgcccgccataaactgccaggcatcaaattaagcagaaggccatcctgacggatggcctttttgcgtggccagtgccaa
gcttgcatgcagattgcagcattacacgtcttgagcgattgtgtaggctggagctgcttcgaagttcctatactttctagagaataggaact
tcggaataggaacttcaagatcccctcacgctgccgcaagcactcagggcgcaagggctgctaaaggaagcggaacacgtagaaa
gccagtccgcagaaacggtgctgaccccggatgaatgtcagctactgggctatctggacaagggaaaacgcaagcgcaaagagaa
agcaggtagcttgcagtgggcttacatggcgatagctagactgggcggttttatggacagcaagcgaaccggaattgccagctgggg
cgccctctggtaaggttgggaagccctgcaaagtaaactggatggctttcttgccgccaaggatctgatggcgcaggggatcaagatc
tgatcaagagacaggatgaggatcgtttcgcATGATTGAACAAGATGGATTGCACGCAGGTTCTCC
GGCCGCTTGGGTGGAGAGGCTATTCGGCTATGACTGGGCACAACAGACAATCGG
CTGCTCTGATGCCGCCGTGTTCCGGCTGTCAGCGCAGGGGCGCCCGGTTCTTTTT
GTCAAGACCGACCTGTCCGGTGCCCTGAATGAACTGCAGGACGAGGCAGCGCGG
CTATCGTGGCTGGCCACGACGGGCGTTCCTTGCGCAGCTGTGCTCGACGTTGTCA
CTGAAGCGGGAAGGGACTGGCTGCTATTGGGCGAAGTGCCGGGGCAGGATCTCC
TGTCATCTCACCTTGCTCCTGCCGAGAAAGTATCCATCATGGCTGATGCAATGCG
GCGGCTGCATACGCTTGATCCGGCTACCTGCCCATTCGACCACCAAGCGAAACAT
CGCATCGAGCGAGCACGTACTCGGATGGAAGCCGGTCTTGTCGATCAGGATGAT
CTGGACGAAGAGCATCAGGGGCTCGCGCCAGCCGAACTGTTCGCCAGGCTCAAG
GCGCGCATGCCCGACGGCGAGGATCTCGTCGTGACCCATGGCGATGCCTGCTTGC
CGAATATCATGGTGGAAAATGGCCGCTTTTCTGGATTCATCGACTGTGGCCGGCT
GGGTGTGGCGGACCGCTATCAGGACATAGCGTTGGCTACCCGTGATATTGCTGAA
GAGCTTGGCGGCGAATGGGCTGACCGCTTCCTCGTGCTTTACGGTATCGCCGCTC
CCGATTCGCAGCGCATCGCCTTCTATCGCCTTCTTGACGAGTTCTTCTGAgcgggactc
tggggttcgaaatgaccgaccaagcgacgcccaacctgccatcacgagatttcgattccaccgccgccttctatgaaaggttgggcttc
ggaatcgttttccgggacgccggctggatgatcctccagcgcggggatctcatgctggagttcttcgcccaccccagcttcaaaagcg
ctctgaagttcctatactttctagagaataggaacttcggaataggaactaaggaggatattcatatggaccatggctaattcccatgttga
caattaatcatcggctcgtataatgttagcagagtatgctgctaaagcacgggtagctacgtataaaacgaaataaagtgctgcacaaca
 
acatcacaacacacgtaataaccagaagagtggggattctcaggATGAACATAAAGGGTAAAGCGTTACTG
GCAGGATGTATCGCGCTGGCATTCAGCAATATGGCTCTGGCAGAAGATATTAAA
GTCGCCGTCGTAGGCGCAATGTCCGGTCCGGTGGCGCAG
SEQ ID NO:13 - Prp promoter (prpR sequence - underlined; Ribosome binding site - lower case; start codon of gene of interest (italicized atg)

TTACCCGTCTGGATTTTCAGTACGCGCTTTTAAACGACGCCACAGCGTGGTACGGCTGATCCC
CAAATAACGTGCGGCGGCGCTTATCGCCATTAAAGCGTGCGAGCACCTCCTGCAATGGAAG
CGCTTCTGCTGACGAGGGCGTGATTTCTGCTGTGGTCCCCACCAGTTCAGGTAATAATTGCCG
CATAAATTGTCTGTCCAGTGTTGGTGCGGATCGACGCTTAAAAAAGCGCCAGGCGTTCCA
TCATATTCCGCAGTTCGCGAATATTACCGGGCCAATGATAGTTCAGTAGAAGCGGCTGACAC
TGCGTCAGCCCATGACGCACCGATTCGGTAAAAGGGATCTCCATCGCGGCCAGCGATTGTTTT
AAAAAGTTTCCGCCAGAGGCAGAATATCAGGCTGTCGCTCGCGCAAGGGGGGAAGCGGCAG
ACGCAGAATGCTCAAACGGTAAAACAGATCGGTACGAAAACGTCCTTGCGTTATCTCCCGAT
CCAGATCGCAATGCGTGGCGCTGATCACCCGGACATCTACCGGGATCGGCTGATGCCCGCCAA
CGCGGGTGACGGCTTTTTCCTCCAGTACGCGTAGAAGGCGGGTTTGTAACGGCAGCGGCATTT
CGCCAATTTCGTCAAGAAACAGCGTGCCGCCGTGGGCGACCTCAAACAGCCCCGCACGTCCAC
CTCGTCTTGAGCCGGTAAACGCTCCCTCCTCATAGCCAAACAGTTCAGCCTCCAGCAACGACT
CGGTAATCGCGCCGCAATTAACGGCGACAAAGGGCGGAGAAGGCTTGTTCTGACGGTGGGGC
TGACGGTTAAACAACGCCTGATGAATCGCTTGCGCCGCCAGCTCTTTCCCGGTCCCTGTTTCC
CCCTGAATCAGCACTGCCGCGCGGAACGGGCATAGAGTGTAATCGTATGGCGAACCTGCTCC
ATTTGTGGTGAATCGCCGAGGATATCGCTCAGCGCATAACGGGTCTGTAATCCCTTGCTGGA
GGTATGCTGGCTATACTGACGCCGTGTCAGGCGGGTCATATCCAGCGCATCATGGAAAGCCT
GACGTACGGTGGCCGCTGAATAAATAAAGATGGCGGTCATTCCTGCCTCTTCCGCCAGGTCGG
TAATTAGTCCTGCCCCAATTACAGCCTCAATGCCGTTAGCTTTGAGCTCGTTAATTTGCCCGC
GAGCATCCTCTTCAGTGATATAGCTTCGCTGTTCAAGACGGAGGTGAAACGTTTTCTGAAAG
GCGACCAGAGCCGGAATGGTCTCCTGATAGGTCACGATTCCCATTGAGGAAGTCAGCTTTCCC
GCTTTTGCCAGAGCCTGTAATACATCGAATCCGCTGGGTTTGATGAGGATGACAGGTACCGA
CAGTCGGCTTTTTAAATAAGCGCCGTTGGAACCTGCCGCGATAATCGCGTCGCAGCGTTCGGT
TGCCAGTTTTTTGCGAATGTAGGCTACTGCCTTTTCAAAACCGAGCTGAATAGGCGTGATCG
TCGCCAGATGATCAAACTCCAGGCTGATATCCCGAAATAGTTCGAACAGGCGCGTTACCGAG
ACCGTCCAGATCACCGGTTTATCGCTATTATCGCGCGAAGCGCTATGCACAGTAACCATCGTC
GTAGATTCATGTTTAAGGAACGAATTCTTGTTTTATAGATGTTTCGTTAATGTTGCAATGAA
ACACAGGCCTCCGTTTCATGAAACGTTAGCTGACTCGTTTTTCTTGTGACTCGTCTGTCAGTA
TTAAAAAAGATTTTTCATTTAACTGATTGTTTTTAAATTGAATTTTATTTAATGGTTTCTCG
GTTTTTGGGTCTGGCATATCCCTTGCTTTAATGAGTGCATCTTAATTAACAATTCAATAACA
AGAGGGCTGAATagtaatttcaacaaaataacgagcattcgaatg

### I. Enzymes catalyzing the conversion of branched-chain amino acids into their ketoacids

### 1. Leucine dehydrogenase LeuDH from Pseudomonas aeruginosa PA01

**SEQ ID NO:19 - LeuDH Amino acid sequence:** 
MFDMMDAARLEGLHLAQDPATGLKAIIAIHSTRLGPALGGCRYLPYPNDEAAIGDAIRLAQGMSYKAAL
AGLEQGGGKAVIIRPPHLDNRGALFEAFGRFIESLGGRYITAVDSGTSSADMDCIAQQTRHVTSTTQAGD
 
PSPHTALGVFAGIRASAQARLGSDDLEGLRVAVQGLGHVGYALAEQLAAVGAELLVCDLDPGRVQLAVE
QLGAHPLAPEALLSTPCDILAPCGLGGVLTSQSVSQLRCAAVAGAANNQLERPEVADELEARGILYAPDY
VINSGGLIYVALKHRGADPHSITAHLARIPARLTEIYAHAQADHQSPARIADRLAERILYGPQ
SEQ **ID** NO:20 - leuDH codon-optimized nucleotide sequence:

atgttcgacatgatggatgcagcccgcctggaaggcctgcacctcgcccaggatccagcgacgggcctgaaagcgatcatcgcgat
ccattccactcgcctcggcccggccttaggcggctgtcgttacctcccatatccgaatgatgaagcggccatcggcgatgccattcgc
ctggcgcagggcatgtcctacaaagctgcacttgcgggtctggaacaaggtggtggcaaggcggtgatcattcgcccaccccacttg
gataatcgcggtgccttgtttgaagcgtttggacgctttattgaaagcctgggtggccgttatatcaccgccgttgactcaggaacaa
gtagtgccgatatggattgcatcgcccaacagacgcgccatgtgacttcaacgacacaagccggcgacccatctccacatacggct
ctgggcgtctttgccggcatccgcgcctccgcgcaggctcgcctggggtccgatgacctggaaggcctgcgtgtcgcggttcagggc
cttggccacgtaggttatgcgttagcggagcagctggcggcggtcggcgcagaactgctggtgtgcgacctggaccccggccgcgt
ccagttagcggtggagcaactgggggcgcacccactggcccctgaagcattgctctctactccgtgcgacattttagcgccttgtggc
ctgggcggcgtgctcaccagccagtcggtgtcacagttgcgctgcgcggccgttgcaggcgcagcgaacaatcaactggagcgccc
ggaagttgcagacgaactggaggcgcgcgggattttatatgcgcccgattacgtgattaactcgggaggactgatttatgtggcgct
gaagcatcgcggtgctgatccgcatagcattaccgcccacctcgctcgcatccctgcacgcctgacggaaatctatgcgcatgcgca
ggcggatcatcagtcgcctgcgcgcatcgccgatcgtctggcggagcgcattctgtacggcccgcagtga

### 2. Branched-chain amino acid aminotransferase IlvE from E. coli Nissle

**SEQ ID NO:21 - IlvE Amino acid sequence:** 
MSYPEKFEGIAIQSHEDWKNPKKTKYDPKPFYDHDIDIKIEACGVCGSDIHCAAGHWGNMKMPLVVGH
EIVGKVVKLGPKSNSGLKVGQRVGVGAQVFSCLECDRCKNDNEPYCTKFVTTYSQPYEDGYVSQGGYAN
YVRVHEHFVVPIPENIPSHLAAPLLCGGLTVYSPLVRNGCGPGKKVGIVGLGGIGSMGTLISKAMGAETYV
ISRSSRKREDAMKMGADHYIATLEEGDWGEKYFDTFDLIVVCASSLTDIDFNIMPKAMKVGGRIVSISIPE
QHEMLSLKPYGLKAVSISYSALGSIKELNQLLKLVSEKDIKIWVETLPVGEAGVHEAFERMEKGDVRYRFTL
VGYDKEFSD
**SEQ ID NO:22 - *ilvE* nucleotide sequence:**

atgaccacgaagaaagctgattacatttggttcaatggggagatggttcgctgggaagacgcgaaggtgcatgtgatgtcgcacgc
gctgcactatggcacctcggtttttgaaggcatccgttgctacgactcgcacaaaggaccggttgtattccgccatcgtgagcatatg
cagcgtctgcatgactccgccaaaatctatcgcttcccggtttcgcagagcattgatgagctgatggaagcttgtcgtgacgtgatcc
gcaaaaacaatctcaccagcgcctatatccgtccgctgatcttcgttggtgatgttggcatgggcgtaaacccgccagcgggatact
caaccgacgtgattatcgccgctttcccgtggggagcgtatctgggcgcagaagcgctggagcaggggatcgatgcgatggtttcct
cctggaaccgcgcagcaccaaacaccatcccgacggcggcaaaagccggtggtaactacctctcttccctgctggtgggtagcgaa
gcgcgccgccacggttatcaggaaggtatcgcgttggatgtgaatggttacatctctgaaggcgcaggcgaaaacctgtttgaagtg
aaagacggcgtgctgttcaccccaccgttcacctcatccgcgctgccgggtattacccgtgatgccatcatcaaactggcaaaagag
ctgggaattgaagtgcgtgagcaggtgctgtcgcgcgaatccctgtacctggcggatgaagtgtttatgtccggtacggcggcagaa
atcacgccagtgcgcagcgtagacggtattcaggttggcgaaggccgttgtggcccggttaccaaacgcattcagcaagccttcttc
ggcctcttcactggcgaaaccgaagataaatgggctggttagatcaagttaatcaataa

### 3. L-amino acid deaminase L-AAD from Proteus vulgaris

**SEQ ID NO:23 - L-AAD Amino acid sequence:** 
MAISRRKFIIGGTVVAVAAGAGILTPMLTREGRFVPGTPRHGFVEGTEGALPKQADVVVVGAGILGIMTA
INLVERGLSVVIVEKGNIAGEQSSRFYGQAISYKMPDETFLLHHLGKHRWREMNAKVGIDTTYRTQGRVE
VPLDEEDLVNVRKWIDERSKNVGSDIPFKTRIIEGAELNQRLRGATTDWKIAGFEEDSGSFDPEVATFVM
 
AEYAKKMGVRIYTQCAARGLETQAGVISDVVTEKGAIKTSQVVVAGGVWSRLFMQNLNVDVPTLPAYQ
SQQLISGSPTAPGGNVALPGGIFFREQADGTYATSPRVIVAPVVKESFTYGYKYLPLLALPDFPVHISLNEQ
LINSFMQSTHWNLDEVSPFEQFRNMTALPDLPELNASLEKLKAEFPAFKESKLIDQWSGAMAIAPDENPI
ISEVKEYPGLVINTATGWGMTESPVSAELTADLLLGKKPVLDPKPFSLYRF
**SEQ ID NO:24 - L-AAD Codon-optimized nucleotide sequence:**

atggccatcagtcgtcgcaaattcattatcggtggaacggtcgtcgccgttgccgccggtgcggggatttgaccccgatgctgacgc
gcgaagggcgctttgtgccgggcactccacgccacggtttcgttgaagggaccgagggggctttacccaaacaagcggacgtggtg
gtcgtaggcgctggaattcttggtattatgacggccattaatttggttgagcgtgggctgtcagtggtaattgtggagaagggcaata
tcgcgggagaacaaagctctcgcttctacggacaggcaattagctataagatgccagatgagacattttgctgcaccatcttggga
agcaccgctggcgtgagatgaatgcgaaagtagggattgatacaacgtaccgtactcaaggacgcgtggaagtaccgcttgacga
ggaagatttggtaaacgtccgcaaatggattgacgaacgttcaaaaaatgttggatctgacattccttttaagacccgcattatcga
gggggcagaattaaatcagcgtctgcgcggcgccacaacagattggaagatcgctggcttcgaggaggacagcgggtcattcgat
cccgaggtagcgacctttgtaatggcagagtacgcgaagaagatgggtgttcgtatctatacgcaatgcgcggcccgcggtctgga
aacccaggccggtgtcatttcagatgttgtcacggaaaaaggtgcgattaagacctcccaagtggtagtggctggtggggtgtgga
gtcgtctgttcatgcagaatttaaacgtcgacgtcccaacccttcctgcgtatcagtcacagcagttgattagtggttcccctaccgca
ccgggggggaacgtcgcattacctggtggaatcttcttccgcgaacaggcggacggacatacgcgacttctcctcgtgtgattgttg
ccccagttgtgaaggagagcttcacttatggttacaagtacttaccattattagcattgcctgatttccctgttcacattagcctgaatg
aacagttaattaattcgtttatgcaaagtacccactggaacttagacgaagtgtcgccgttcgaacaatttcgcaacatgacagcatt
acctgacttgcccgaacttaacgccagcttagaaaagttaaaggcagagttccctgctttcaaagaatccaagttgatcgaccagtg
gtctggagcaatggcaattgcgcccgacgaaaatccaatcatttccgaggtgaaggagtacccaggtctggtaattaacacggcga
caggttggggcatgactgaaagtccagtgtctgctgaacttaccgccgatcttctgctgggaagaagccggtgttagatcctaagc
cattctcactttatcgcttttga

### 4. L-amino acid deaminase L-AAD from Proteus mirabilis

**SEQ ID NO:25 - L-AAD Amino acid sequence:** 
MAISRRKFILGGTVVAVAAGAGVLTPMLTREGRFVPGTPRHGFVEGTGGPLPKQDDVVVIGAGILGIMT
AINLAERGLSVTIVEKGNIAGEQSSRFYGQAISYKMPDETFLLHHLGKHRWREMNAKVGIDTTYRTQGRV
EVPLDEEDLENVRKWIDAKSKDVGSDIPFRTKMIEGAELKQRLRGATTDWKIAGFEEDSGSFDPEVATFV
MAEYAKKMGIKIFTNCAARGLETQAGVISDVVTEKGPIKTSRVVVAGGVGSRLFMQNLNVDVPTLPAYQ
SQQLISAAPNAPGGNVALPGGIFFRDQADGTYATSPRVIVAPVVKESFTYGYKYLPLLALPDFPVHISLNE
QLINSFMQSTHWDLNEESPFEKYRDMTALPDLPELNASLEKLKKEFPAFKESTLIDQWSGAMAIAPDENP
IISDVKEYPGLVINTATGWGMTESPVSAEITADLLLGKKPVLDAKPFSLYRF
**SEQ ID NO:26 - L-AAD Nucleotide sequence:**

atggcaataagtagaagaaaatttattcttggtggcacagtggttgctgttgctgcaggcgctggggttttaacacctatgttaacgc
gagaagggcgttttgttcctggtacgccgagacatggttttgttgagggaactggcggtccattaccgaaacaagatgatgttgttgt
aattggtgcgggtattttaggtatcatgaccgcgattaaccttgctgagcgtggcttatctgtcacaatcgttgaaaaaggaaatatt
gccggcgaacaatcatctcgattctatggtcaagctattagctataaaatgccagatgaaaccttcttattacatcacctcgggaagc
accgctggcgtgagatgaacgctaaagttggtattgataccacttatcgtacacaaggtcgtgtagaagttcctttagatgaagaag
atttagaaaacgtaagaaaatggattgatgctaaaagcaaagatgttggctcagacattccatttagaacaaaaatgattgaaggc
gctgagttaaaacagcgtttacgtggcgctaccactgattggaaaattgctggtttcgaagaagactcaggaagcttcgatcctgaa
gttgcgacttttgtgatggcagaatatgccaaaaaaatgggtatcaaaattttcacaaactgtgcagcccgtggtttagaaacgcaa
gctggtgttatttctgatgttgtaacagaaaaaggaccaattaaaacctctcgtgttgttgtcgccggtggtgttgggtcacgtttattt
atgcagaacctaaatgttgatgtaccaacattacctgcttatcaatcacagcaattaattagcgcagcaccaaatgcgccaggtgga
aacgttgctttacccggcggaattttctttcgtgatcaagcggatggaacgtatgcaacttctcctcgtgtcattgttgctccggtagta

aaagaatcatttacttacggctataaatatttacctctgctggctttacctgatttcccagtacatatttcgttaaatgagcagttgatt
aattcctttatgcaatcaacacattgggatcttaatgaagagtcgccatttgaaaaatatcgtgatatgaccgctctgcctgatctgcc
agaattaaatgcctcactggaaaaactgaaaaaagagttcccagcatttaaagaatcaacgttaattgatcagtggagtggtgcga
tggcgattgcaccagatgaaaacccaattatctctgatgttaaagagtatccaggtctagttattaatactgcaacaggttggggaat
gactgaaagccctgtatcagcagaaattacagcagatttattattaggcaaaaaaccagtattagatgccaaaccatttagtctgta
tcgtttctaa

### II. Branched-chain ketoacid decarboxylase sequences

### 1. KivD from lactococcus lactis strain IFPL730

**SEQ ID NO:27 - KivD Amino acid sequence:** 
MYTVGDYLLDRLHELGIEEIFGVPGDYNLQFLDQIISHKDMKWVGNANELNASYMADGYARTKKAAFL
TTFGVGELSAVNGLAGSYAENLPVVEIVGSPTSKVQNEGKFVHHTLADGDFKHFMKMHEPVTAARTLLT
AENATVEIDRVLSALLKERKPVYINLPVDVAAAKAEKPSLPLKKENSTSNTSDQEILNKIQESLKNAKKPIVIT
GHEIISFGLEKTVTQFISKTKLPITTLNFGKSSVDEALPSFLGIYNGTLSEPNLKEFVESADFILMLGVKLTDSS
TGAFTHHLNENKMISLNIDEGKIFNERIQNFDFESLISSLLDLSEIEYKGKYIDKKQEDFVPSNALLSQDRLW
QAVENLTQSNETIVAEQGTSFFGASSIFLKSKSHFIGQPLWGSIGYTFPAALGSQIADKESRHLLFIGDGSL
QLTVQELGLAIREKINPICFIINNDGYTVEREIHGPNQSYNDIPMWNYSKLPESFGATEDRVVSKIVRTENE
FVSVMKEAQADPNRMYWIELILAKEGAPKVLKKMGKLFAEQNKS
**SEQ ID NO:28 - *kivD* Nucleotide sequence:** 
atgtatacagtaggagattacctattagaccgattacacgagttaggaattgaagaaatttttggagtccctggagactataacttac
aatttttagatcaaattatttcccacaaggatatgaaatgggtcggaaatgctaatgaattaaatgcttcatatatggctgatggctat
gctcgtactaaaaaagctgccgcatttcttacaacctttggagtaggtgaattgagtgcagttaatggattagcaggaagttacgccg
aaaatttaccagtagtagaaatagtgggatcacctacatcaaaagttcaaaatgaaggaaaatttgttcatcatacgctggctgacg
gtgattttaaacactttatgaaaatgcacgaacctgttacagcagctcgaactttactgacagcagaaaatgcaaccgttgaaattg
accgagtactttctgcactattaaaagaaagaaaacctgtctatatcaacttaccagttgatgttgctgctgcaaaagcagagaaac
cctcactccctttgaaaaaggaaaactcaacttcaaatacaagtgaccaagaaattttgaacaaaattcaagaaagcttgaaaaat
gccaaaaaaccaatcgtgattacaggacatgaaataattagttttggcttagaaaaaacagtcactcaatttatttcaaagacaaa
actacctattacgacattaaactttggtaaaagttcagttgatgaagccctcccttcatttttaggaatctataatggtacactctcag
agcctaatcttaaagaattcgtggaatcagccgacttcatcttgatgcttggagttaaactcacagactcttcaacaggagccttcac
tcatcatttaaatgaaaataaaatgatttcactgaatatagatgaaggaaaaatatttaacgaaagaatccaaaattttgattttga
atccctcatctcctctctcttagacctaagcgaaatagaatacaaaggaaaatatatcgataaaaagcaagaagactttgttccatc
aaatgcgcttttatcacaagaccgcctatggcaagcagttgaaaacctaactcaaagcaatgaaacaatcgttgctgaacaaggga
catcattctttggcgcttcatcaattttcttaaaatcaaagagtcattttattggtcaacccttatggggatcaattggatatacattccc
agcagcattaggaagccaaattgcagataaagaaagcagacaccttttatttattggtgatggttcacttcaacttacagtgcaaga
attaggattagcaatcagagaaaaaattaatccaatttgctttattatcaataatgatggttatacagtcgaaagagaaattcatgg
accaaatcaaagctacaatgatattccaatgtggaattactcaaaattaccagaatcgtttggagcaacagaagatcgagtagtctc
aaaaatcgttagaactgaaaatgaatttgtgtctgtcatgaaagaagctcaagcagatccaaatagaatgtactggattgagttaat
tttggcaaaagaaggtgcaccaaaagtactgaaaaaaatgggcaaactatttgctgaacaaaataaatcataa
**SEQ ID NO:29 - *kivD* Codon-optimized sequence:**

atgtatacagtaggagattacttattggaccggttgcacgaacttggaattgaggaaatttttggagttccgggtgactacaacctgc
agttccttgaccaaatcatctcccataaggacatgaaatgggtcggcaatgccaatgagctgaacgcatcatatatggcagacggg
tatgctcggaccaaaaaggctgcagcattccttaccacgtttggcgtgggggaattaagtgctgtaaatggactggcaggatcctat

gcggagaatttaccggtagtcgaaattgttggctcgcctacgtccaaggtgcagaatgaggggaaattcgtccatcacacacttgca
gacggtgattttaagcactttatgaagatgcatgagccggtaacggctgcgcggacgcttcttactgcggaaaacgcaacagtaga
gattgatcgcgttctgagcgcactgcttaaggaacggaagcccgtctatattaacttaccggtagacgtggccgcagccaaagccga
aaaaccaagcctgcctcttaagaaggagaattccacgtccaacaccagtgaccaagagattttgaacaaaattcaagagtctttga
agaacgcgaagaagcccatcgtaattacaggacatgagattatctcgtttggcctggagaaaacggttacacagtttatttccaaaa
cgaagttacctataacgacgttaaactttggaaagagctctgtggatgaggcacttcctagtttcttaggaatctataatgggaccct
ttcagagccaaacttaaaggaattcgttgaaagtgcggattttatcttaatgcttggggttaaattgactgattccagcaccggagctt
ttacgcaccatttaaacgagaacaaaatgatctctttgaatatcgacgaaggcaaaatttttaatgaaagaattcagaactttgattt
tgaatcccttattagttcacttttagatttaagtgaaatagagtataagggaaagtatatagacaagaagcaagaggatttcgttccg
tctaatgctcttttaagtcaagacagactttggcaggcggttgagaaccttacacaatccaatgaaacgatagtcgccgaacaaggg
accagtttcttcggcgcttcttccatattcctgaagtctaagtctcatttcattggacagcccctgtgggggtctataggatatacgtttc
ccgcagctcttggaagccagatcgccgataaggagagcagacacctgttgttcatcggggacggctcgttgcagctgactgttcagg
aactggggttggcgatcagagagaagattaatcccatttgctttatcataaataatgatggttataccgtagaacgtgagattcatgg
acctaatcagagctataatgacattcctatgtggaactattcaaaattgccagagagttttggtgcaactgaggatcgcgttgttagt
aaaatagtccgcacggagaacgagtttgtcagcgtaatgaaggaggcccaagcggaccctaatcggatgtactggatcgaacttat
tctggctaaagaaggagcacctaaagttttaaagaagatggaaaactttttgctgaacaaaataaatcataa

### 2. KdcA amino acid sequence from lactococcus lactis strain B1157

**SEQ ID NO:30 - KdcA Amino acid sequence:** 
MYTVGDYLLDRLHELGIEEIFGVPGDYNLQFLDQIISREDMKWIGNANELNASYMADGYARTKKAAFL
TTFGVGELSAINGLAGSYAENLPVVEIVGSPTSKVQNDGKFVHHTLADGDFKHFMKMHEPVTAARTLLT
AENATYEIDRVLSQLLKERKPVYINLPVDVAAAKAEKPALSLEKESSTTNTTEQVILSKIEESLKNAQKPVVIA
GHEVISFGLEKTVTQFVSETKLPITTLNFGKSAVDESLPSFLGIYNGKLSEISLKNFVESADFILMLGVKLTDSS
TGAFTHHLDENKMISLNIDEGIIFNKVVEDFDFRAVVSSLSELKGIEYEGQYIDKQYEEFIPSSAPLSQDRLW
QAVESLTQSNETIVAEQGTSFFGASTIFLKSNSRFIGQPLWGSIGYTFPAALGSQIADKESRHLLFIGDGSLQ
LTVQELGLSIREKLNPICFIINNDGYTVEREIHGPTQSYNDIPMWNYSKLPETFGATEDRVVSKIVRTENEF
VSVMKEAQADVNRMYWIELVLEKEDAPKLLKKMGKLFAEQNKS
**SEQ ID NO:31 - *kdcA* Nucleotide sequence:** 
atgtatacagtaggagattacctattagaccgattacacgagttgggaattgaagaaatttttggagttcctggtgactataacttac
aatttttagatcaaattatttcacgcgaagatatgaaatggattggaaatgctaatgaattaaatgcttcttatatggctgatggttat
gctcgtactaaaaaagctgccgcatttctcaccacatttggagtcggcgaattgagtgcgatcaatggactggcaggaagttatgcc
gaaaatttaccagtagtagaaattgttggttcaccaacttcaaaagtacaaaatgacggaaaatttgtccatcatacactagcagat
ggtgattttaaacactttatgaagatgcatgaacctgttacagcagcgcggactttactgacagcagaaaatgccacatatgaaatt
gaccgagtactttctcaattactaaaagaaagaaaaccagtctatattaacttaccagtcgatgttgctgcagcaaaagcagagaa
gcctgcattatctttagaaaaagaaagctctacaacaaatacaactgaacaagtgattttgagtaagattgaagaaagtttgaaaa
atgcccaaaaaccagtagtgattgcaggacacgaagtaattagttttggtttagaaaaaacggtaactcagtttgtttcagaaacaa
aactaccgattacgacactaaatttggtaaaagtgctgttgatgaatctttgccctcatttttaggaatatataacggaaactttca
gaaatcagtcttaaaaattttgtggagtccgcagactttatcctaatgcttggagtgaagcttacggactcctcaacaggtgcattca
cacatcatttagatgaaaataaaatgatttcactaaacatagatgaaggaataattttcaataaagtggtagaagattttgattttag
agcagtggtttcttctttatcagaattaaaggaatagaatatgaaggacaatatattgataagcaatatgaagaatttattccatca
agtgctcccttatcacaagaccgtctatggcaggcagttgaaagtttgactcaaagcaatgaaacaatcgttgctgaacaaggaac
ctcatttttttggagcttcaacaattttcttaaaatcaaatagtcgttttattggacaacctttatggggttctattggatatacttttccag
cggctttaggaagccaaattgcggataaagagagcagacaccttttatttattggtgatggttcacttcaacttaccgtacaagaatt
aggactatcaatcagagaaaaactcaatccaatttgttttatcataaataatgatggttatacagttgaaagagaaatccacggacc
tactcaaagttataacgacattccaatgtggaattactcgaaattaccagaaacatttggagcaacagaagatcgtgtagtatcaaa
 
aattgttagaacagagaatgaatttgtgtctgtcatgaaagaagcccaagcagatgtcaatagaatgtattggatagaactagtttt
ggaaaaagaagatgcgccaaaattactgaaaaaaatgggcaaactatttgctgaacaaaataaatcataa
**SEQ ID NO:32 - *kdcA* Codon-optimized kdcA sequence:**

atgtatacagtaggagattaccttttagatcgtttgcacgaattgggcattgaggaaatttttggcgtccctggcgactacaatttaca
attcttagatcagattatttcacgtgaggatatgaagtggattgggaatgccaatgagctgaacgcgagctatatggcggacggtta
cgctcgtacaaaaaaggcagcagcgtttcttactacttttggcgtaggcgaattgtcggccatcaacgggcttgcgggttcgtatgcg
gaaaacttaccggttgtcgagattgtcggttcccctacttcgaaggtgcagaatgatggcaaattcgttcatcacaccttggcagacg
gcgactttaaacatttcatgaaaatgcacgaacctgtgactgccgcccgcacacttctgacagctgaaaacgcgacatacgaaattg
atcgcgtgctttcgcagttgttgaaagagcgtaaacccgtatatatcaatctgccggtggatgtagcggctgcaaaagccgaaaaac
cggcgctgtcactggaaaaagaatcgtctacgactaatacaacggaacaagtaatcctgtcaaaaatcgaagagagcttgaaaaa
cgcccagaagcctgtcgtgattgccgggcacgaggtcattagttttgggttagaaaagactgttacccagttcgtgagtgagacgaa
gttgcccatcaccacccttaactttggcaagtctgcggtagacgagagcttaccgtctttttaggtatctacaatgggaaactttcag
aaatttcactgaaaaacttcgtggagtcggcagactttattttaatgttgggtgttaaattaactgatagcagcactggcgcgttcacg
catcacttggatgagaataaaatgatctcgcttaacatcgacgaaggtatcattttaataaagttgtagaggacttcgactttcgtgc
tgttgtatcgagcctttccgaattaaagggtatcgagtacgaaggtcagtacattgacaagcaatacgaggaatttatcccctccagc
gcgcctcttagccaagaccgcctttggcaggccgtagagagtcttacacaaagtaatgaaactattgttgcagaacagggtacaag
cttctttggcgcctcgacgattttcttaaaatcgaacagtcgctttatcggcaacctctttgggggtcgattgggtacacctttcctgc
ggccttaggctctcaaattgcggacaaagaatctcgccatttattattcatcggcgacggctcgttacagcttacagtgcaagagttg
ggattatcgattcgcgagaagctgaatccgatttgctttatcattaacaacgacgggtacacagtcgaacgcgaaatccatggcccg
acacaatcatataatgacatccctatgtggaattattctaagcttccagagacattcggcgcaactgaagaccgcgtcgtgtcaaaa
attgtccgcactgagaatgaattcgtgtcagtgatgaaggaagctcaggccgatgtcaaccgcatgtactggattgaattagttttgg
agaaagaggatgcccccaaattacttaagaagatggggaaactatttgctgaacaaaataaatcataa

### 3. THI3/KID1 from Saccharomyces cerevisiae

**SEQ ID NO:33 - THI3/KID1 Amino acid sequence:** 
MNSSYTQRYALPKCIAISDYLFHRLNQLNIHTIFGLSGEFSMPLLDKLYNIPNLRWAGNSNELNAAYAADG
YSRLKGLGCLITTFGVGELSAINGVAGSYAEHVGILHIVGMPPTSAQTKQLLLHHTLGNGDFTVFHRIASD
VACYTTLIIDSELCADEVDKCIKKAWIEQRPVYMGMPVNQVNLPIESARLNTPLDLQLHKNDPDVEKEVIS
RILSFIYKSQNPAIIVDACTSRQNLIEETKELCNRLKFPVFVTPMGKGTVNETDPQFGGVFTGSISAPEVREV
VDFADFIIVIGCMLSEFSTSTFHFQYKTKNCALLYSTSVKLKNATYPDLSIKLLLQKILANLDESKLSYQPSEQ
PSMMVPRPYPAGNVLLRQEWVWNEISHWFQPGDIIITETGASAFGVNQTRFPVNTLGISQALWGSVG
YTMGACLGAEFAVQEINKDKFPATKHRVILFMGDGAFQLTVQELSTIVKWGLTPYIFVMNNQGYSVDRF
LHHRSDASYYDIQPWNYLGLLRVFGCTNYETKKIITVGEFRSMISDPNFATNDKIRMIEIMLPPRDVPQAL
LDRWVVEKEQSKQVQEENENSSAVNTPTPEFQPLLKKNQVGY
**SEQ ID NO:34 - THI3/KID1 Nucleotide sequence:**

atgaattctagctatacacagagatatgcactgccgaagtgtatagcaatatcagattatcttttccatcggctcaaccagctgaaca
tacataccatatttggactctccggagaatttagcatgccgttgctggataaactatacaacattccgaacttacgatgggccggtaa
ttctaatgagttaaatgctgcctacgcagcagatggatactcacgactaaaaggcttgggatgtctcataacaacctttggtgtaggc
gaattatcggcaatcaatggcgtggccggatcttacgctgaacatgtaggaatacttcacatagtgggtatgccgccaacaagtgca
caaacgaaacaactactactgcatcatactctgggcaatggtgatttcacggtatttcatagaatagccagtgatgtagcatgctata
caacattgattattgactctgaattatgtgccgacgaagtcgataagtgcatcaaaaaggcttggatagaacagaggccagtatac
atgggcatgcctgtcaaccaggtaaatctcccgattgaatcagcaaggcttaatacacctctggatttacaattgcataaaaacgac
ccagacgtagagaaagaagttatttctcgaatattgagttttatatacaaaagccagaatccggcaatcatcgtagatgcatgtact
agtcgacagaatttaatcgaggagactaaagagctttgtaataggcttaaatttccagtttttgttacacctatgggtaagggtacag

taaacgaaacagacccgcaatttgggggcgtattcacgggctcgatatcagccccagaagtaagagaagtagttgattttgccgatt
ttatcatcgtcattggttgcatgctctccgaattcagcacgtcaactttccacttccaatataaaactaagaattgtgcgctactatatt
ctacatctgtgaaattgaaaaatgccacatatcctgacttgagcattaaattactactacagaaaatattagcaaatcttgatgaatc
taaactgtcttaccaaccaagcgaacaacccagtatgatggttccaagaccttacccagcaggaaatgtcctcttgagacaagaat
gggtctggaatgaaatatcccattggttccaaccaggtgacataatcataacagaaactggtgcttctgcatttggagttaaccaga
ccagatttccggtaaatacactaggtatttcgcaagctctttggggatctgtcggatatacaatgggggcgtgtcttggggcagaatt
tgctgttcaagagataaacaaggataaattccccgcaactaaacatagagttattctgtttatgggtgacggtgctttccaattgaca
gttcaagaattatccacaattgttaagtggggattgacaccttatatttttgtgatgaataaccaaggttactctgtggacaggtttttg
catcacaggtcagatgctagttattacgatatccaaccttggaactacttgggattattgcgagtatttggttgcacgaactacgaaa
cgaaaaaattattactgttggagaattcagatccatgatcagtgacccaaactttgcgaccaatgacaaaattcggatgatagag
attatgctaccaccaagggatgttccacaggctctgcttgacaggtgggtggtagaaaaagaacagagcaaacaagtgcaagagg
agaacgaaaattctagcgcagtaaatacgccaactccagaattccaaccacttctaaaaaaaaatcaagttggatactga

### 4. ARO10 from Saccharomyces cerevisiae

**SEQ ID NO:35 - ARO10 Amino acid sequence:** 
MAPVTIEKFVNQEERHLVSNRSATIPFGEYIFKRLLSIDTKSVFGVPGDFNLSLLEYLYSPSVESAGLRWVGT
CNELNAAYAADGYSRYSNKIGCLITTYGVGELSALNGIAGSFAENVKVLHIVGVAKSIDSRSSNFSDRNLHH
LVPQLHDSNFKGPNHKVYHDMVKDRVACSVAYLEDIETACDQVDNVIRDIYKYSKPGYIFVPADFADMS
VTCDNLVNVPRISQQDCIVYPSENQLSDIINKITSWIYSSKTPAILGDVLTDRYGVSNFLNKLICKTGIWNFS
TVMGKSVIDESNPTYMGQYNGKEGLKQVYEHFELCDLVLHFGVDINEINNGHYTFTYKPNAKIIQFHPNY
IRLVDTRQGNEQMFKGINFAPILKELYKRIDVSKLSLQYDSNVTQYTNETMRLEDPTNGQSSIITQVHLQK
TMPKFLNPGDVVVCETGSFQFSVRDFAFPSQLKYISQGFFLSIGMALPAALGVGIAMQDHSNAHINGGN
VKEDYKPRLILFEGDGAAQMTIQELSTILKCNIPLEVIIWNNNGYTIERAIMGPTRSYNDVMSWKWTKLFE
AFGDFDGKYTNSTLIQCPSKLALKLEELKNSNKRSGIELLEVKLGELDFPEQLKCMVEAAALKRNKK
**SEQ ID NO:36 - ARO10 Nucleotide sequence:**

atggcacctgttacaattgaaaagttcgtaaatcaagaagaacgacaccttgtttccaaccgatcagcaacaattccgtttggtgaa
tacatatttaaaagattgttgtccatcgatacgaaatcagttttcggtgttcctggtgacttcaacttatctctattagaatatctctatt
cacctagtgttgaatcagctggcctaagatgggtcggcacgtgtaatgaactgaacgccgcttatgcggccgacggatattcccgtt
actctaataagattggctgtttaataaccacgtatggcgttggtgaattaagcgccttgaacggtatagccggttcgttcgctgaaaa
tgtcaaagttttgcacattgttggtgtggccaagtccatagattcgcgttcaagtaactttagtgatcggaacctacatcatttggtccc
acagctacatgattcaaattttaaagggccaaatcataaagtatatcatgatatggtaaaagatagagtcgcttgctcggtagccta
cttggaggatattgaaactgcatgtgaccaagtcgataatgttatccgcgatatttacaagtattctaaacctggttatattttttgttcc
tgcagattttgcggatatgtctgttacatgtgataatttggttaatgttccacgtatatctcaacaagattgtatagtataccctttctga
aaaccaattgtctgacataatcaacaagattactagttggatatattccagtaaaacacctgcgatccttggagacgtactgactgat
aggtatggtgtgagtaactttttgaacaagcttatctgcaaaactgggatttggaatttttccactgttatggaaaaatctgtaattga
tgagtcaaacccaacttatatgggtcaatataatggtaaagaaggtttaaaacaagtctatgaacattttgaactgtgcgacttggtc
ttgcattttggagtcgacatcaatgaaattaataatgggcattatacttttacttataaaccaaatgctaaaatcattcaatttcatccg
aattatattcgccttgtggacactaggcagggcaatgagcaaatgttcaaaggaatcaattttgcccctattttaaaagaactataca
agcgcattgacgtttctaaactttctttgcaatatgattcaaatgtaactcaatatacgaacgaaacaatgcggttagaagatcctac
caatggacaatcaagcattattacacaagttcacttacaaaagacgatgcctaaattttgaaccctggtgatgttgtcgtttgtgaa
acaggctcttttcaattctctgttcgtgatttcgcgtttccttcgcaattaaaatatatatcgcaaggatttttcctttccattggcatggc
ccttcctgccgccctaggtgttggaattgccatgcaagaccactcaaacgctcacatcaatggtggcaacgtaaaagaggactataa
gccaagattaattttgtttgaaggtgacggtgcagcacagatgacaatccaagaactgagcaccattctgaagtgcaatattccact
agaagttatcatttggaacaataacggctacactattgaaagagccatcatgggccctaccaggtcgtataacgacgttatgtcttg
gaaatggaccaaactatttgaagcattcggagacttcgacggaaagtatactaatagcactctcattcaatgtccctctaaattagc

actgaaattggaggagcttaagaattcaaacaaaagaagcgggatagaacttttagaagtcaaattaggcgaattggatttccccg
aacagctaaagtgcatggttgaagcagcggcacttaaaagaaataaaaaatag

### III. Alcohol dehydrogenase sequences

### 1. Adh2 from Saccharomyces cerevisae

SEQ ID NO:37 - Adh2 Amino acid sequence: 
MSIPETQKAIIFYESNGKLEHKDIPVPKPKPNELLINVKYSGVCHTDLHAWHGDWPLPTKLPLVGGHEGA
GVVVGMGENVKGWKIGDYAGIKWLNGSCMACEYCELGNESNCPHADLSGYTHDGSFQEYATADAVQ
AAHIPQGTDLAEVAPILCAGITVYKALKSANLRAGHWAAISGAAGGLGSLAVQYAKAMGYRVLGIDGGP
GKEELFTSLGGEVFIDFTKEKDIVSAVVKATNGGAHGIINVSVSEAAIEASTRYCRANGTVVLVGLPAGAKC
SSDVFNHVVKSISIVGSYVGNRADTREALDFFARGLVKSPIKVVGLSSLPEIYEKMEKGQIAGRYVVDTSK
**SEQ ID NO:38 - *adh2* Nucleotide sequence:** 
atgtctattccagaaactcaaaaagccattatcttctacgaatccaacggcaagttggagcataaggatatcccagttccaaagcca
aagcccaacgaattgttaatcaacgtcaagtactctggtgtctgccacaccgatttgcacgcttggcatggtgactggccattgccaa
ctaagttaccattagttggtggtcacgaaggtgccggtgtcgttgtcggcatgggtgaaaacgttaagggctggaagatcggtgact
acgccggtatcaaatggttgaacggttcttgtatggcctgtgaatactgtgaattgggtaacgaatccaactgtcctcacgctgactt
gtctggttacacccacgacggttctttccaagaatacgctaccgctgacgctgttcaagccgctcacattcctcaaggtactgacttgg
ctgaagtcgcgccaatcttgtgtgctggtatcaccgtatacaaggctttgaagtctgccaacttgagagcaggccactgggcggcca
tttctggtgctgctggtggtctaggttctttggctgttcaatatgctaaggcgatgggttacagagtcttaggtattgatggtggtccag
gaaaggaagaattgtttacctcgctcggtggtgaagtattcatcgacttcaccaaagagaaggacattgttagcgcagtcgttaagg
ctaccaacggcggtgcccacggtatcatcaatgtttccgtttccgaagccgctatcgaagcttctaccagatactgtagggcgaacg
gtactgttgtcttggttggtttgccagccggtgcaaagtgctcctctgatgtcttcaaccacgttgtcaagtctatctccattgtcggctc
ttacgtggggaacagagctgataccagagaagccttagatttctttgccagaggtctagtcaagtctccaataaaggtagttggctta
tccagtttaccagaaatttacgaaaagatggagaagggccaaattgctggtagatacgttgttgacacttctaaataa
**SEQ ID NO:39 - *adh2* Codon-optimized sequence:**

atgtctattccagaaacgcagaaagccatcatatttatgaatcgaacggaaaacttgagcacaaggacatccccgtcccgaagcc
aaaacctaatgagttgcttatcaacgttaagtattcgggcgtatgccacacagacttgcacgcatggcacggggattggcccttacc
gactaagttgccgttagtgggcggacatgagggggcgggagtcgtagtgggaatgggagagaacgtgaagggttggaagattgg
agattatgctgggattaagtggttgaatgggagctgcatggcctgcgaatattgtgaacttggaaatgagagcaattgcccacatgc
tgacttgtccggttacacacatgacggttcattccaggaatatgctacggctgatgcagtccaagcagcgcatatcccgcaagggac
ggacttagcagaagtagcgcccattctttgcgctgggatcaccgtatataaagcgttaaagagcgcaaatttacgggccggacattg
ggcggcgatcagcggggccgcagggggctgggcagcttggccgtccagtacgctaaagctatgggttatcgggttttgggcattg
acggaggaccgggaaaggaggaattattcacgtccttgggaggagaggtattcattgactttaccaaggaaaaagatatcgtctct
gctgtagtaaaggctaccaatggcggtgcccacggaatcataaatgtttcagtttctgaagcggcgatcgaagcgtccactagatat
tgccgtgcaaatgggacagtcgtacttgtaggacttccggctggcgccaaatgcagctccgatgtatttaatcatgtcgtgaagtcaa
tctctatcgttggttcatatgtaggaaaccgcgccgatactcgtgaggctcttgactttttgccagaggcctggttaagtcccccataa
aagttgttggcttatccagcttacccgaaatatacgagaagatggagaagggccagatcgcggggagatacgttgttgacacttcta
aataa

### 2. Adh6 from Saccharomyces cerevisae

**SEQ ID NO:40 - Adh6 Amino acid sequence:** 
MSYPEKFEGIAIQSHEDWKNPKKTKYDPKPFYDHDIDIKIEACGVCGSDIHCAAGHWGNMKMPLVVGH
EIVGKVVKLGPKSNSGLKVGQRVGVGAQVFSCLECDRCKNDNEPYCTKFVTTYSQPYEDGYVSQGGYAN
YVRVHEHFVVPIPENIPSHLAAPLLCGGLTVYSPLVRNGCGPGKKVGIVGLGGIGSMGTLISKAMGAETYV
ISRSSRKREDAMKMGADHYIATLEEGDWGEKYFDTFDLIVVCASSLTDIDFNIMPKAMKVGGRIVSISIPE
QHEMLSLKPYGLKAVSISYSALGSIKELNQLLKLVSEKDIKIWVETLPVGEAGVHEAFERMEKGDVRYRFTL
VGYDKEFSD
**SEQ ID N0:41 - *adh6* Codon-optimized sequence:**

atgtcataccctgaaaaattcgagggtatcgccattcagagtcacgaagattggaagaatcccaagaagaccaaatacgacccca
agccgttctatgaccatgatatcgacatcaaaatcgaggcatgtggtgtgtggcagtgatattcattgcgcagcgggccattggg
ggaacatgaagatgcctctggtagtaggacatgagatcgttggaaaggttgtgaaattgggtccgaaaagtaactccggtcttaaa
gtaggtcagcgtgttggggtcggggcgcaagttttcagttgcctggagtgtgatcgttgtaagaacgataacgagccgtactgcaca
aagtttgtaacgacgtattcacagccatatgaggatgggtatgtttctcaagggggctatgcaaactacgtccgcgtacatgaacac
tttgtggtgcctattcctgagaacattccgtctcacttggccgctcctttgttgtgcggaggtcttaccgtctactcgccattggttcgca
atgggtgcggtccgggcaaaaaggtagggatcgttggccttggtggtatcggatctatgggaacgttaatcagtaaggcgatggga
gctgagacctacgttatttcccgttcatcacgtaagcgtgaggatgcgatgaagatgggtgcagatcactacatcgcaacgttagaa
gagggagattggggcgaaaaatattttgacacttttgacttgattgtggtttgtgcatcgtcacttacagacattgactttaatattatg
ccaaaggcaatgaaggtaggtgggcgtattgtgtccatttctatcccggaacaacacgagatgctttctctgaaaccctacggactt
aaagctgtgtccatttcgtacagtgcccttggatctatcaaggaactgaatcagctgctgaagcttgtttcggagaaagacattaaga
tttgggtggagacattgccagtgggggaggccggcgttcacgaggcgtttgaacgcatggagaagggagatgttcgctatcgcttca
cgctggttggttatgataaagaattcagtgattag

### 3. Adh1 from Saccharomyces cerevisae

**SEQ ID NO:42 - Adh1 Amino acid sequence:** 
MSIPETQKGVIFYESHGKLEYKDIPVPKPKANELLINVKYSGVCHTDLHAWHGDWPLPVKLPLVGGHEGA
GVVVGMGENVKGWKIGDYAGIKWLNGSCMACEYCELGNESNCPHADLSGYTHDGSFQQYATADAVQ
AAHIPQGTDLAQVAPILCAGITVYKALKSANLMAGHWVAISGAAGGLGSLAVQYAKAMGYRVLGIDGG
EGKEELFRSIGGEVFIDFTKEKDIVGAVLKATDGGAHGVINVSVSEAAIEASTRYVRANGTTVLVGMPAGA
KCCSDVFNQVVKSISIVGSYVGNRADTREALDFFARGLVKSPIKVVGLSTLPEIYEKMEKGQIVGRYVVDTS
K
**SEQ ID NO:43 - *adh1* Nucleotide sequence:**

atgtctatcccagaaactcaaaaaggtgttatcttctacgaatcccacggtaagttggaatacaaagatattccagttccaaagcca
aaggccaacgaattgttgatcaacgttaaatactctggtgtctgtcacactgacttgcacgcttggcacggtgactggccattgccag
ttaagctaccattagtcggtggtcacgaaggtgccggtgtcgttgtcggcatgggtgaaaacgttaagggctggaagatcggtgact
acgccggtatcaaatggttgaacggttcttgtatggcctgtgaatactgtgaattgggtaacgaatccaactgtcctcacgctgactt
gtctggttacacccacgacggttctttccaacaatacgctaccgctgacgctgttcaagccgctcacattcctcaaggtaccgacttg
gcccaagtcgcccccatcttgtgtgctggtatcaccgtctacaaggctttgaagtctgctaacttgatggccggtcactgggttgctat
ctccggtgctgctggtggtctaggttctttggctgttcaatacgccaaggctatgggttacagagtcttgggtattgacggtggtgaag
gtaaggaagaattattcagatccatcggtggtgaagtcttcattgacttcactaaggaaaaggacattgtcggtgctgttctaaaggc
cactgacggtggtgctcacggtgtcatcaacgtttccgtttccgaagccgctattgaagcttctaccagatacgttagagctaacggt
accaccgttttggtcggtatgccagctggtgccaagtgttgttctgatgtcttcaaccaagtcgtcaagtccatctctattgttggttctt
acgtcggtaacagagctgacaccagagaagctttggacttcttcgccagaggtttggtcaagtctccaatcaaggttgtcggcttgtc
taccttgccagaaatttacgaaaagatggaaaagggtcaaatcgttggtagatacgttgttgacacttctaaataa

### 4. Adh3 from Saccharomyces cerevisae

**SEQ ID NO:44 - Adh3 Amino acid sequence:**
**SEQ ID NO:45 - *adh3* Nucleotide sequence:**

atgttgagaacgtcaacattgttcaccaggcgtgtccaaccaagcctattttctagaaacattcttagattgcaatccacagctgcaa
tccctaagactcaaaaggtgtcatcttttatgagaataaggggaagctgcattacaaagatatccctgtccccgagcctaagccaa
atgaaattttaatcaacgttaaatattctggtgtatgtcacaccgatttacatgcttggcacggcgattggccattacctgttaaacta
ccattagtaggtggtcatgaaggtgctggtgtagttgtcaaactaggttccaatgtcaagggctggaaagtcggtgatttagcaggt
atcaaatggctgaacggttcttgtatgacatgcgaattctgtgaatcaggtcatgaatcaaattgtccagatgctgatttatctggtta
cactcatgatggttctttccaacaatttgcgaccgctgatgctattcaagccgccaaaattcaacagggtaccgacttggccgaagta
gccccaatattatgtgctggtgttactgtatataaagcactaaaagaggcagacttgaaagctggtgactgggttgccatctctggtg
ctgcaggtggcttgggttccttggccgttcaatatgcaactgcgatgggttacagagttctaggtattgatgcaggtgaggaaaagg
aaaaacttttcaagaaattgggggtgaagtattcatcgactttactaaaacaaagaatatggtttctgacattcaagaagctacca
aaggtggccctcatggtgtcattaacgtttccgtttctgaagccgctatttctctatctacggaatatgttagaccatgtggtaccgtcg
ttttggttggtttgcccgctaacgcctacgttaaatcagaggtattctctcatgtggtgaagtccatcaatatcaagggttcttatgttg
gtaacagagctgatacgagagaagccttagacttctttagcagaggtttgatcaaatcaccaatcaaaattgttggattatctgaatt
accaaaggtttatgacttgatggaaaagggcaagattttgggtagatacgtcgtcgatactagtaaataa

### 5. Adh4 from Saccharomyces cerevisae

**SEQ ID NO:46 - Adh4 Amino acid sequence:** 
MSSVTGFYIPPISFFGEGALEETADYIKNKDYKKALIVTDPGIAAIGLSGRVQKMLEERDLNVAIYDKTQPN
PNIANVTAGLKVLKEQNSEIVVSIGGGSAHDNAKAIALLATNGGEIGDYEGVNQSKKAALPLFAINTTAGT
ASEMTRFTIISNEEKKIKMAIIDNNVTPAVAVNDPSTMFGLPPALTAATGLDALTHCIEAYVSTASNPITDA
CALKGIDLINESLVAAYKDGKDKKARTDMCYAEYLAGMAFNNASLGYVHALAHQLGGFYHLPHGVCNA
VLLPHVQEANMQCPKAKKRLGEIALHFGASQEDPEETIKALHVLNRTMNIPRNLKELGVKTEDFEILAEH
AMHDACHLTNPVQFTKEQVVAIIKKAYEY
**SEQ ID NO:47 - *adh4* Nucleotide sequence:**

atgtcttccgttactgggttttacattccaccaatctctttctttggtgaaggtgctttagaagaaaccgctgattacatcaaaaacaag
gattacaaaaaggctttgatcgttactgatcctggtattgcagctattggtctctccggtagagtccaaaagatgttggaagaacgtg
acttaaacgttgctatctatgacaaaactcaaccaaacccaaatattgccaatgtcacagctggtttgaaggttttgaaggaacaaa
actctgaaattgttgtttccattggtggtggttctgctcacgacaatgctaaggccattgctttattggctactaacggtggggaaatc
ggagactatgaaggtgtcaatcaatctaagaaggctgctttaccactatttgccatcaacactactgctggtactgcttccgaaatga
ccagattcactattatctctaatgaagaaaagaaaatcaagatggctatcattgacaacaacgtcactccagctgttgctgtcaacg
atccatctaccatgtttggtttgccacctgctttgactgctgctactggtctagatgctttgactcactgtatcgaagcttatgtttccac
cgcctctaacccaatcaccgatgcctgtgctttgaagggtattgatttgatcaatgaaagcttagtcgctgcatacaaagacggtaaa
gacaagaaggccagaactgacatgtgttacgctgaatacttggcaggtatggctttcaacaatgcttctctaggttatgttcatgccc
ttgctcatcaacttggtggtttctaccacttgcctcatggtgtttgtaacgctgtcttgttgcctcatgttcaagaggccaacatgcaatg
tccaaaggccaagaagagattaggtgaaattgctttgcatttcggtgcttctcaagaagatccagaagaaaccatcaaggctttgca

cgttttaaacagaaccatgaacattccaagaaacttgaaagaattaggtgttaaaaccgaagattttgaaattttggctgaacacgc
catgcatgatgcctgccatttgactaacccagttcaattcaccaaagaacaagtggttgccattatcaagaaagcctatgaatattaa

### 6. Adh5 from Saccharomyces cerevisae

**SEQ ID NO:48 - Adh5 Amino acid sequence:** 
MPSQVIPEKQKAIVFYETDGKLEYKDVTVPEPKPNEILVHVKYSGVCHSDLHAWHGDWPFQLKFPLIGG
HEGAGVVVKLGSNVKGWKVGDFAGIKWLNGTCMSCEYCEVGNESQCPYLDGTGFTHDGTFQEYATA
DAVQAAHIPPNVNLAEVAPILCAGITVYKALKRANVIPGQWVTISGACGGLGSLAIQYALAMGYRVIGID
GGNAKRKLFEQLGGEIFIDFTEEKDIVGAIIKATNGGSHGVINVSVSEAAIEASTRYCRPNGTVVLVGMPA
HAYCNSDVFNQVVKSISIVGSCVGNRADTREALDFFARGLIKSPIHLAGLSDVPEIFAKMEKGEIVGRYVVE
TSK
**SEQ ID NO:49 - *adh5* Nucleotide sequence:**

atgccttcgcaagtcattcctgaaaaacaaaaggctattgtcttttatgagacagatggaaaattggaatataaagacgtcacagtt
ccggaacctaagcctaacgaaattttagtccacgttaaatattctggtgtttgtcatagtgacttgcacgcgtggcacggtgattggcc
atttcaattgaaatttccattaatcggtggtcacgaaggtgctggtgttgttgttaagttgggatctaacgttaagggctggaaagtcg
gtgattttgcaggtataaaatggttgaatgggacttgcatgtcctgtgaatattgtgaagtaggtaatgaatctcaatgtccttatttg
gatggtactggcttcacacatgatggtacttttcaagaatacgcaactgccgatgccgttcaagctgcccatattccaccaaacgtca
atcttgctgaagttgccccaatcttgtgtgcaggtatcactgtttataaggcgttgaaaagagccaatgtgataccaggccaatgggt
cactatatccggtgcatgcggtggcttgggttctctggcaatccaatacgcccttgctatgggttacagggtcattggtatcgatggtg
gtaatgccaagcgaaagttatttgaacaattaggcggagaaatattcatcgatttcacggaagaaaaagacattgttggtgctataa
taaaggccactaatggcggttctcatggagttattaatgtgtctgtttctgaagcagctatcgaggcttctacgaggtattgtaggccc
aatggtactgtcgtcctggttggtatgccagctcatgcttactgcaattccgatgttttcaatcaagttgtaaaatcaatctccatcgtt
ggatcttgtgttggaaatagagctgatacaagggaggctttagatttcttcgccagaggtttgatcaaatctccgatccacttagctg
gcctatcggatgttcctgaaattttgcaaagatggagaagggtgaaattgttggtagatatgttgttgagacttctaaatga

### 7. Adh7 from Saccharomyces cerevisae

**SEQ ID NO:50 - Adh7 Amino acid sequence:** 
MLYPEKFQGIGISNAKDWKHPKLVSFDPKPFGDHDVDVEIEACGICGSDFHIAVGNWGPVPENQILGHE
IIGRVVKVGSKCHTGVKIGDRVGVGAQALACFECERCKSDNEQYCTNDHVLTMWTPYKDGYISQGGFA
SHVRLHEHFAIQIPENIPSPLAAPLLCGGITVFSPLLRNGCGPGKRVGIVGIGGIGHMGILLAKAMGAEVYA
FSRGHSKREDSMKLGADHYIAMLEDKGWTEQYSNALDLLVVCSSSLSKVNFDSIVKIMKIGGSIVSIAAPE
VNEKLVLKPLGLMGVSISSSAIGSRKEIEQLLKLVSEKNVKIWVEKLPISEEGVSHAFTRMESGDVKYRFTLV
DYDKKFHK*
**SEQ ID NO:51 - *adh7* Nucleotide sequence:**

Atgctttacccagaaaaatttcagggcatcggtatttccaacgcaaaggattggaagcatcctaaattagtgagttttgacccaaaa
ccctttggcgatcatgacgttgatgttgaaattgaagcctgtggtatctgcggatctgattttcatatagccgttggtaattggggtcca
gtcccagaaaatcaaatccttggacatgaaataattggccgcgtggtgaaggttggatccaagtgccacactggggtaaaaatcgg
tgaccgtgttggtgttggtgcccaagccttggcgtgttttgagtgtgaacgttgcaaaagtgacaacgagcaatactgtaccaatgac
cacgttttgactatgtggactccttacaaggacggctacatttcacaaggaggctttgcctcccacgtgaggcttcatgaacactttgc
tattcaaataccagaaaatattccaagtccgctagccgctccattattgtgtggtggtattacagttttctctccactactaagaaatg
gctgtggtccaggtaagagggtaggtattgttggcatcggtggtattgggcatatggggattctgttggctaaagctatgggagccg
aggtttatgcgttttcgcgaggccactccaagcgggaggattctatgaaactcggtgctgatcactatattgctatgttggaggataa
aggctggacagaacaatactctaacgctttggaccttcttgtcgtttgctcatcatctttgtcgaaagttaattttgacagtatcgttaa

gattatgaagattggaggctccatcgtttcaattgctgctcctgaagttaatgaaaagcttgttttaaaaccgttgggcctaatggga
gtatcaatctcaagcagtgctatcggatctaggaaggaaatcgaacaactattgaaattagtttccgaaaagaatgtcaaaatatg
ggtggaaaaacttccgatcagcgaagaaggcgtcagccatgcctttacaaggatggaaagcggagacgtcaaatacagatttactt
tggtcgattatgataagaaattccataaatag

### 8. SFA1 from Saccharomyces cerevisae

**SEQ ID NO:52 - SFA1 Amino acid sequence:** 
MSAATVGKPIKCIAAVAYDAKKPLSVEEITVDAPKAHEVRIKIEYTAVCHTDAYTLSGSDPEGLFPCVLGHE
GAGIVESVGDDVITVKPGDHVIALYTAECGKCKFCTSGKTNLCGAVRATQGKGVMPDGTTRFHNAKGE
DIYHFMGCSTFSEYTVVADVSVVAIDPKAPLDAACLLGCGVTTGFGAALKTANVQKGDTVAVFGCGTVG
LSVIQGAKLRGASKIIAIDINNKKKQYCSQFGATDFVNPKEDLAKDQTIVEKLIEMTDGGLDFTFDCTGNTK
IMRDALEACHKGWGQSIIIGVAAAGEEISTRPFQLVTGRVWKGSAFGGIKGRSEMGGLIKDYQKGALKV
EEFITHRRPFKEINQAFEDLHNGDCLRTVLKSDEIK
**SEQ ID NO:53 - *sfa1* Nucleotide sequence:** 
Atgtccgccgctactgttggtaaacctattaagtgcattgctgctgttgcgtatgatgcgaagaaaccattaagtgttgaagaaatca
cggtagacgccccaaaagcgcacgaagtacgtatcaaaattgaatatactgctgtatgccacactgatgcgtacactttatcaggct
ctgatccagaaggacttttcccttgcgttctgggccacgaaggagccggtatcgtagaatctgtaggcgatgatgtcataacagttaa
gcctggtgatcatgttattgctttgtacactgctgagtgtggcaaatgtaagttctgtacttccggtaaaaccaacttatgtggtgctgt
tagagctactcaagggaaaggtgtaatgcctgatgggaccacaagatttcataatgcgaaaggtgaagatatataccatttcatgg
gttgctctactttttccgaatatactgtggtggcagatgtctctgtggttgccatcgatccaaaagctcccttggatgctgcctgtttact
gggttgtggtgttactactggttttggggcggctcttaagacagctaatgtgcaaaaaggcgataccgttgcagtatttggctgcggg
actgtaggactctccgttatccaaggtgcaaagttaaggggcgcttccaagatcattgccattgacattaacaataagaaaaaaca
atattgttctcaatttggtgccacggattttgttaatcccaaggaagatttggccaaagatcaaactatcgttgaaaagttaattgaaa
tgactgatgggggtctggatttacttttgactgtactggtaataccaaaattatgagagatgctttggaagcctgtcataaaggttgg
ggtcaatctattatcattggtgtggctgccgctggtgaagaaatttctacaaggccgttccagctggtcactggtagagtgtggaaag
gctctgcttttggtggcatcaaaggtagatctgaaatgggcggtttaattaaagactatcaaaaaggtgccttaaaagtcgaagaat
ttatcactcacaggagaccattcaaagaaatcaatcaagcctttgaagatttgcataacggtgattgcttaagaaccgtcttgaagtc
tgatgaaataaaatag
SEQ ID No: 54 IlvC amino acid sequence from E. coli Nissle 
MANYFNTLNLRQQLAQLGKCRFMGRDEFADGASYLQGKKVVIVGCGAQGLNQGLNMRDSGLDISYAL
RKEAIAEKRASWRKATENGFKVGTYEELIPQADLVVNLTPDKQHSDVVRTVQPLMKDGAALGYSHGFNI
VEVGEQIRKDITVVMVAPKCPGTEVREEYKRGFGVPTLIAVHPENDPKGEGMAIAKAWAAATGGHRAG
VLESSFVAEVKSDLMGEQTILCGMLQAGSLLCFDKLVEEGTDPAYAEKLIQFGWETITEALKQGGITLMM
DRLSNPAKLRAYALSEQLKEIMAPLFQKHMDDIISGEFSSGMMADWANDDKKLLTWREETGKTAFETA
PQYEGKIGEQEYFDKGVLMIAMVKAGVELAFETMVDSGIIEESAYYESLHELPLIANTIARKRLYEMNVVIS
DTAEYGNYLFSYACVPLLKPFMAELQPGDLGKAIPEGAVDNAQLRDVNEAIRSHAIEQVGKKLRGYMTD
MKRIAVAG
SEQ ID 55: ilvC gene from E. coli Nissle nucleotide sequence 
atggctaactacttcaatacactgaatctgcgccagcagttggcacagctgggcaaatgtcgctttatggggcgcgatgaattcgcc
gatggcgcgagctaccttcagggtaaaaagtagtcatcgtcggctgtggcgcacagggtctgaaccagggcctgaacatgcgtga
ttctggtctcgatatctcctacgctctgcgtaaagaagcgattgctgagaagcgcgcatcctggcgtaaagcaaccgaaaatggtttt
aaagtgggtacttacgaagaactgatcccgcaggcggatctggtggttaacctgacgccggacaagcagcactctgatgtagtgcg
caccgtacagccactgatgaaagacggcgcggcgctgggctactctcatggtttcaatatcgtagaagtgggtgagcagatccgta
aagacatcaccgtcgtaatggttgcgccgaaatgccctggcaccgaagtacgtgaagagtacaaacgtggattcggcgtaccgacg
ctgattgccgttcacccggaaaacgatccgaaaggcgaaggcatggcgatcgctaaagcatgggcggctgcaaccggcggtcacc
gtgcgggcgttctggaatcctctttcgttgcggaagtgaaatctgacctgatgggcgagcaaaccatcctgtgcggtatgttgcaagc
tggttctctgctgtgcttcgacaagctggtggaagaaggcaccgatccggcatacgcagaaaaactgattcagttcggttgggaaac
catcaccgaagcgctgaaacagggcggcatcaccctgatgatggaccgtctttctaacccggcgaaactgcgtgcttacgcgctttc
tgagcaactgaaagagatcatggcgccgctgttccagaaacatatggacgacatcatctccggcgaattctcctccggcatgatggc
tgactgggccaacgacgataagaaactgctgacctggcgtgaagagactggcaaaaccgcattcgaaaccgcgccgcagtatgaa
ggcaaaatcggtgaacaggagtacttcgataaaggcgtactgatgatcgcgatggtaaaagcaggcgttgagttggcgtttgaaac
catggttgattccggcatcatcgaagaatctgcttactatgaatcactgcacgaactgccgctgattgccaacaccatcgcccgtaag
cgtctgtacgaaatgaacgtggttatctccgatactgccgagtacggtaactatctgttctcttacgcttgtgtgccactgctgaaacc
gtttatggcagagctgcaaccgggcgacctgggtaaagctattccggaaggtgcggtagataacgcgcagctgcgtgatgtaaatg
aagcgattcgcagccatgcgattgagcaggtaggtaagaaactgcgcggctatatgacggatatgaaacgtattgctgttgcgggtt
aa
L-amino acid deaminase L-AAD (from Proteus vulgaris)
SEQ ID NO: 56: Codon-optimized sequence: 
ATGGCCATCAGTCGTCGCAAATTCATTATCGGTGGAACGGTCGTCGCCGTTGCCG
CCGGTGCGGGGATTTGACCCCGATGCTGACGCGCGAAGGGCGCTTTGTGCCGG
GCACTCCACGCCACGGTTTCGTTGAAGGACCGAGGGGGCTTTACCCAAACAAG
CGGACGTGGTGGTCGTAGGCGCTGGAATTCTTGGTATTATGACGGCCATTAATTT
GGTTGAGCGTGGGCTGTCAGTGGTAATTGTGGAGAAGGGCAATATCGCGGAGA
ACAAAGCTCTCGCTTCTACGGACAGGCAATTAGCTATAAGATGCCAGATGAGAC
ATTTTTGCTGCACCATCTTGGGAAGCACCGCTGGCGTGAGATGAATGCGAAAGTA
GGGATTGATACAACGTACCGTACTCAAGGACGCGTGGAAGTACCGCTTGACGAG
GAAGATTTGGTAAACGTCCGCAAATGGATTGACGAACGTTCAAAAAATGTTGGA
TCTGACATTCCTTTTAAGACCCGCATTATCGAGGGGGCAGAATTAAATCAGCGTC
TGCGCGGCGCCACAACAGATTGGAAGATCGCTGGCTTCGAGGAGGACAGCGGGT
CATTCGATCCCGAGGTAGCGACCTTTGTAATGGCAGAGTACGCGAAGAAGATGG
GTGTTCGTATCTATACGCAATGCGCGGCCCGCGGTCTGGAAACCCAGGCCGGTGT
CATTTCAGATGTTGTCACGGAAAAAGGTGCGATTAAGACCTCCCAAGTGGTAGTG
GCTGGTGGGGTGTGGAGTCGTCTGTTCATGCAGAATTTAAACGTCGACGTCCCAA
CCCTTCCTGCGTATCAGTCACAGCAGTTGATTAGTGGTTCCCCTACCGCACCGGG
GGGGAACGTCGCATTACCTGGTGGAATCTTCTTCCGCGAACAGGCGGACGGGAC
ATACGCGACTTCTCCTCGTGTGATTGTTGCCCCAGTTGTGAAGGAGAGCTTCACT
TATGGTTACAAGTACTTACCATTATTAGCATTGCCTGATTTCCCTGTTCACATTAG
CCTGAATGAACAGTTAATTAATTCGTTTATGCAAAGTACCCACTGGAACTTAGAC
GAAGTGTCGCCGTTCGAACAATTTCGCAACATGACAGCATTACCTGACTTGCCCG
AACTTAACGCCAGCTTAGAAAAGTTAAAGGCAGAGTTCCCTGCTTTCAAAGAATC
CAAGTTGATCGACCAGTGGTCTGGAGCAATGGCAATTGCGCCCGACGAAAATCC
AATCATTTCCGAGGTGAAGGAGTACCCAGGTCTGGTAATTAACACGGCGACAGG
 
TTGGGGCATGACTGAAAGTCCAGTGTCTGCTGAACTTACCGCCGATCTTCTGCTG
GGGAAGAAGCCGGTGTTAGATCCTAAGCCATTCTCACTTTATCGCTTTTGA
SEQ ID NO: 57: Amino acid sequence: 
MAISRRKFIIGGTVVAVAAGAGILTPMLTREGRFVPGTPRHGFVEGTEGALPKQADV
VVVGAGILGIMTAINLVERGLSVVIVEKGNIAGEQSSRFYGQAISYKMPDETFLLHHL
GKHRWREMNAKVGIDTTYRTQGRVEVPLDEEDLVNVRKWIDERSKNVGSDIPFKTR
IIEGAELNQRLRGATTDWKIAGFEEDSGSFDPEVATFVMAEYAKKMGVRIYTQCAAR
GLETQAGVISDVVTEKGAIKTSQVVVAGGVWSRLFMQNLNVDVPTLPAYQSQQLIS
GSPTAPGGNVALPGGIFFREQADGTYATSPRVIVAPVVKESFTYGYKYLPLLALPDFP
VHISLNEQLINSFMQSTHWNLDEVSPFEQFRNMTALPDLPELNASLEKLKAEFPAFKE
SKLIDQWSGAMAIAPDENPIISEVKEYPGLVINTATGWGMTESPVSAELTADLLLGKK
PVLDPKPFSLYRF*
Leucine dehydrogenase leuDH from Bacillus cereus:
   SEQ ID NO: 58 Codon-optimized sequence: 
ATGACTCTTGAAATCTTTGAATATTTAGAAAAGTACGACTACGAGCAGGTTGTAT
TTTGTCAAGACAAGGAGTCTGGGCTGAAGGCCATCATTGCCATCCACGACACAA
CCTTAGGCCCGGCGCTTGGCGGAACCCGCATGTGGACCTACGACTCCGAGGAGG
CGGCCATCGAGGACGCACTTCGTCTTGCTAAGGGTATGACCTATAAGAACGCGG
CAGCCGGTCTGAATCTGGGGGGTGCTAAGACTGTAATCATCGGTGATCCACGCA
AGGATAAGAGTGAAGCAATGTTTCGCGCTTTAGGGCGCTATATTCAGGGCTTGAA
CGGCCGCTACATTACCGCAGAAGACGTAGGGACAACAGTAGACGACATGGACAT
CATCCATGAGGAAACTGATTTCGTGACCGGTATTTCACCTTCATTCGGGTCATCC
GGTAACCCTTCCCCCGTAACAGCCTATGGGGTTTATCGCGGAATGAAGGCCGCAG
CCAAGGAGGCATTTGGCACTGACAATTTAGAAGGAAAAGTAATTGCTGTCCAAG
GCGTGGGCAATGTGGCCTACCATTTGTGTAAACACCTTCACGCGGAAGGTGCAA
AATTGATCGTTACGGATATTAACAAGGAGGCAGTCCAGCGCGCTGTAGAGGAAT
TTGGAGCATCGGCTGTGGAACCAAATGAGATCTACGGTGTAGAATGTGACATTTA
CGCTCCATGCGCACTTGGTGCCACGGTGAATGACGAGACCATCCCCCAACTTAAG
GCGAAGGTAATCGCTGGTTCAGCTAACAACCAATTAAAAGAGGACCGTCACGGA
GATATCATCCACGAAATGGGTATCGTGTACGCCCCCGATTATGTTATCAACGCGG
GCGGCGTAATCAACGTAGCCGATGAGCTTTATGGATACAACCGCGAACGTGCGC
TGAAACGCGTGGAAAGCATTTATGACACGATCGCAAAGGTAATCGAGATCAGTA
AGCGCGACGGCATTGCGACATACGTGGCAGCGGACCGTCTGGCCGAAGAACGCA
TCGCGAGTTTGAAGAATAGCCGTAGTACCTACTTGCGCAACGGGCACGATATTAT
CAGCCGTCGCtga
   SEQ ID NO: 59 amino acid sequence: 
MTLEIFEYLEKYDYEQVVFCQDKESGLKAIIAIHDTTLGPALGGTRMWTYDSEEAAIE
DALRLAKGMTYKNAAAGLNLGGAKTVIIGDPRKDKSEAMFRALGRYIQGLNGRYIT
AEDVGTTVDDMDIIHEETDFVTGISPSFGSSGNPSPVTAYGVYRGMKAAAKEAFGTD
NLEGKVIAVQGVGNVAYHLCKHLHAEGAKLIVTDINKEAVQRAVEEFGASAVEPNE
IYGVECDIYAPCALGATVNDETIPQLKAKVIAGSANNQLKEDRHGDIIHEMGIVYAPD
YVINAGGVINVADELYGYNRERALKRVESIYDTIAKVIEISKRDGIATYVAADRLAEE
RIASLKNSRSTYLRNGHDIISRR*
   Alcohol dehydrogenase YqhD from E. coli:
      SEQ ID NO: 60 Nucleotide sequence: 
Atgaacaactttaatctgcacaccccaacccgcattctgtttggtaaaggcgcaatcgctggtttacgcgaacaaattcctcacgatgct
cgcgtattgattacctacggcggcagcgtgaaaaaaaccggcgttctcgatcaagttctggatgccctgaaaggcatggacgtac
tggaatttggcggtattgaaccaaacccggcttatgaaacgctgatgaacgccgtgaaactggttcgcgaacagaaagtgacgttcctg
ctggcggttggcggcggttctgtactggacggcaccaaatttatcgccgcagcggctaactatccggaaaatatcgatccgtggcaca
ttctgcaaacgggcggtaaagagattaaaagcgccatcccgatgggctgtgtgctgacgctgccagcaaccggttcagaatccaacg
caggcgcggtgatctcccgtaaaaccacaggcgacaagcaggcgttccattctgcccatgttcagcccgtatttgccgtgctcgatcc
ggtttatacctacaccctgccgccgcgtcaggtggctaacggcgtagtggacgcctttgtacacaccgtggaacagtatgttaccaaac
cggttgatgccaaaattcaggaccgtttcgcagaaggcattttgctgacgctgatcgaagatggtccgaaagccctgaaagagccaga
aaactacgatgtgcgcgccaacgtcatgtgggcggcgactcaggcgctgaacggtttgatcggcgctggcgtaccgcaggactggg
caacgcatatgctgggccacgaactgactgcgatgcacggtctggatcacgcgcaaacactggctatcgtcctgcctgcactgtggaa
tgaaaaacgcgataccaagcgcgctaagctgctgcaatatgctgaacgcgtctggaacatcactgaaggttcagacgatgagcgtatt
gacgccgcgattgccgcaacccgcaatttctttgagcaattaggcgtgctgacccacctctccgactacggtctggacggcagctccat
cccggctttgctgaaaaaactggaagagcacggcatgacccaactgggcgaaaatcatgacattacgctggatgtcagccgccgtat
atacgaagccgcccgctaa
      SEQ ID NO: 61 amino acid sequence: 
MNNFNLHTPTRILFGKGAIAGLREQIPHDARVLITYGGGSVKKTGVLDQVLDALKGM
DVLEFGGIEPNPAYETLMNAVKLVREQKVTFLLAVGGGSVLDGTKFIAAAANYPENI
DPWHILQTGGKEIKSAIPMGCVLTLPATGSESNAGAVISRKTTGDKQAFHSAHVQPV
FAVLDPVYTYTLPPRQVANGVVDAFVHTVEQYVTKPVDAKIQDRFAEGILLTLIEDG
PKALKEPENYDVRANVMWAATQALNGLIGAGVPQDWATHMLGHELTAMHGLDH
AQTLAIVLPALWNEKRDTKRAKLLQYAERVWNITEGSDDERIDAAIAATRNFFEQLG
VLTHLSDYGLDGSSIPALLKKLEEHGMTQLGENHDITLDVSRRIYEAAR*
      Aldehyde dehydrogenase PadA from E. coli:
         SEQ ID NO: 62: Nucleotide sequence: 
ATGACAGAGCCGCATGTAGCAGTATTAAGCCAGGTCCAACAGTTTCTCGATCGTC
AACACGGTCTTTATATTGATGGTCGTCCTGGCCCCGCACAAAGTGAAAAACGGTT
GGCGATCTTTGATCCGGCCACCGGGCAAGAAATTGCGTCTACTGCTGATGCCAAC
GAAGCGGATGTAGATAACGCAGTCATGTCTGCCTGGCGGGCCTTTGTCTCGCGTC
GCTGGGCCGGGCGATTACCCGCAGAGCGTGAACGTATTCTGCTACGTTTTGCTGA
TCTGGTGGAGCAGCACAGTGAGGAGCTGGCGCAACTGGAAACCCTGGAGCAAGG
CAAGTCAATTGCCATTTCCCGTGCTTTTGAAGTGGGCTGTACGCTGAACTGGATG
CGTTATACCGCCGGGTTAACGACCAAAATCGCGGGTAAAACGCTGGACTTGTCG
ATTCCCTTACCCCAGGGGGCGCGTTATCAGGCCTGGACGCGTAAAGAGCCGGTTG
GCGTAGTGGCGGGAATTGTGCCATGGAACTTTCCGTTGATGATTGGTATGTGGAA
GGTGATGCCAGCACTGGCAGCAGGCTGTTCAATCGTGATTAAGCCTTCGGAAACC
ACGCCACTGACGATGTTGCGCGTGGCGGAACTGGCCAGCGAGGCTGGTATCCCT
GATGGCGTTTTTAATGTCGTCACCGGGTCAGGTGCTGTATGCGGCGCGGCCCTGA
CGTCACATCCTCATGTTGCGAAAATCAGTTTTACCGGTTCAACCGCGACGGGAAA
AGGTATTGCCAGAACTGCTGCTGATCACTTAACGCGTGTAACGCTGGAACTGGGC
GGTAAAAACCCGGCAATTGTATTAAAAGATGCTGATCCGCAATGGGTTATTGAA
GGCTTGATGACCGGAAGCTTCCTGAATCAAGGGCAAGTATGCGCCGCCAGTTCG
CGAATTTATATTGAAGCGCCGTTGTTTGACACGCTGGTTAGTGGATTTGAGCAGG
CGGTAAAATCGTTGCAAGTGGGACCGGGGATGTCACCTGTTGCACAGATTAACC
CTTTGGTTTCTCGTGCGCACTGCGACAAAGTGTGTTCATTCCTCGACGATGCGCA
GGCACAGCAAGCAGAGCTGATTCGCGGGTCGAATGGACCAGCCGGAGAGGGGT
ATTATGTTGCGCCAACGCTGGTGGTAAATCCCGATGCTAAATTGCGCTTAACTCG
 
TGAAGAGGTGTTTGGTCCGGTGGTAAACCTGGTGCGAGTAGCGGATGGAGAAGA
GGCGTTACAACTGGCAAACGACACGGAATATGGCTTAACTGCCAGTGTCTGGAC
GCAAAATCTCTCCCAGGCTCTGGAATATAGCGATCGCTTACAGGCAGGGACGGT
GTGGGTAAACAGCCATACCTTAATTGACGCTAACTTACCGTTTGGTGGGATGAAG
CAGTCAGGAACGGGCCGTGATTTTGGCCCCGACTGGCTGGACGGTTGGTGTGAA
ACTAAGTCGGTGTGTGTACGGTATTAA
         SEQ ID NO: 63 ammino acid sequence: 
MTEPHVAVLSQVQQFLDRQHGLYIDGRPGPAQSEKRLAIFDPATGQEIASTADANEA
DVDNAVMSAWRAFVSRRWAGRLPAERERILLRFADLVEQHSEELAQLETLEQGKSI
AISRAFEVGCTLNWMRYTAGLTTKIAGKTLDLSIPLPQGARYQAWTRKEPVGVVAGI
VPWNFPLMIGMWKVMPALAAGCSIVIKPSETTPLTMLRVAELASEAGIPDGVFNVVT
GSGAVCGAALTSHPHVAKISFTGSTATGKGIARTAADHLTRVTLELGGKNPAIVLKD
ADPQWVIEGLMTGSFLNQGQVCAASSRIYIEAPLFDTLVSGFEQAVKSLQVGPGMSP
VAQINPLVSRAHCDKVCSFLDDAQAQQAELIRGSNGPAGEGYYVAPTLVVNPDAKL
RLTREEVFGPVVNLVRVADGEEALQLANDTEYGLTASVWTQNLSQALEYSDRLQAG
TVWVNSHTLIDANLPFGGMKQSGTGRDFGPDWLDGWCETKSVCVRY*
         BCAA transporter BrnQ from E. coli:
            SEQ ID NO: 64 Nucleotide sequence: 
atgacccatcaattaagatcgcgcgatatcatcgctctgggctttatgacatttgcgttgttcgtcggcgcaggtaacattattttccctcca
atggtcggcttgcaggcaggcgaacacgtctggactgcggcattcggcttcctcattactgccgttggcctaccggtattaacggtagt
ggcgctggcaaaagttggcggcggtgttgacagtctcagcacgccaattggtaaagtcgctggcgtactgctggcaacagtttgttac
ctggcggtggggccgctttttgctacgccgcgtacagctaccgtttcttttgaagtgggcattgcgccgctgacgggtgattccgcgctg
ccgctgtttatttacagcctggtctatttcgctatcgttattctggtttcgctctatccgggcaagctgctggataccgtgggcaacttccttg
cgccgctgaaaattatcgcgctggtcatcctgtctgttgccgcaattatctggccggcgggttctatcagtacggcgactgaggcttatc
aaaacgctgcgttttctaacggcttcgtcaacggctatctgaccatggatacgctgggcgcaatggtgtttggtatcgttattgttaacgcg
gcgcgttctcgtggcgttaccgaagcgcgtctgctgacccgttataccgtctgggctggcctgatggcgggtgttggtctgactctgctg
tacctggcgctgttccgtctgggttcagacagcgcgtcgctggtcgatcagtctgcaaacggtgcggcgatcctgcatgcttacgttca
gcatacctttggcggcggcggtagcttcctgctggcggcgttaatcttcatcgcctgcctggtcacggcggttggcctgacctgtgcttg
tgcagaattcttcgcccagtacgtaccgctctcttatcgtacgctggtgtttatcctcggcggcttctcgatggtggtgtctaacctcggctt
gagccagctgattcagatctctgtaccggtgctgaccgccatttatccgccgtgtatcgcactggttgtattaagttttacacgctcatggt
ggcataattcgtcccgcgtgattgctccgccgatgtttatcagcctgctttttggtattctcgacgggatcaaggcatctgcattcagcgat
atcttaccgtcctgggcgcagcgtttaccgctggccgaacaaggtctggcgtggttaatgccaacagtggtgatggtggttctggccatt
atctgggatcgtgcggcaggtcgtcaggtgacctccagcgctcactaa
            SEQ ID NO: 65 amino acid sequence:

MTHQLRSRDIIALGFMTFALFVGAGNIIFPPMVGLQAGEHVWTAAFGFLITAVGLPVL
TVVALAKVGGGVDSLSTPIGKVAGVLLATVCYLAVGPLFATPRTATVSFEVGIAPLT
GDSALPLFIYSLVYFAIVILVSLYPGKLLDTVGNFLAPLKIIALVILSVAAIIWPAGSIST
ATEAYQNAAFSNGFVNGYLTMDTLGAMVFGIVIVNAARSRGVTEARLLTRYTVWA
GLMAGVGLTLLYLALFRLGSDSASLVDQSANGAAILHAYVQHTFGGGGSFLLAALIF
IACLVTAVGLTCACAEFFAQYVPLSYRTLVFILGGFSMVVSNLGLSQLIQISVPVLTAI
YPPCIALVVLSFTRSWWHNSSRVIAPPMFISLLFGILDGIKASAFSDILPSWAQRLPLAE
QGLAWLMPTVVMVVLAIIWDRAAGRQVTSSAH*

### Isovaleryl-CoA synthetase LbuL from Streptomyces lividans

SEQ ID NO: 66: amino acid sequence: 
MTAPAPQPSYAHGTSTTPLLGDTVGANLGRAIAAHPDREALVDVPSGRRWTYAEF
GAAVDELARGLLAKGVTRGDRVGIWAVNCPEWVLVQYATARIGVIMVNVNPAYRA
HELEYVLQQSGISLLVASLAHKSSDYRAIVEQVRGRCPALRETVYIGDPSWDALTAG
AAAVEQDRVDALAAELSCDDPVNIQYTSGTTGFPKGATLSHHNILNNGYWVGRTV
GYTEQDRVCLPVPFYHCFGMVMGNLGATSHGACIVIPAPSFEPAATLEAVQRERCT
SLYGVPTMFIAELNLPDFASYDLTSLRTGIMAGSPCPVEVMKRVVAEMHMEQVSIC
YGMTETSPVSLQTRMDDDLEHRTGTVGRVLPHIEVKVVDPVTGVTLPRGEAGELR
TRGYSVMLGYWEEPGKTAEAIDPGRWMHTGDLAVMREDGYVEIVGRIKDMIIRGG
ENIYPREVEEFLYAHPKIADVQVVGVPHERYGEEVLACVVVRDAADPLTLEELRAYC
AGQLAHYKVPSRLQLLDSFPMTVSGKVRKVELRERYGTRP*
SEQ ID NO: 67: Codon-optimized nucleotide sequence:

atgactgcaccagcacctcaaccctcttatgcacatggcacttctaccactccgcttcttggtgatacggtgggggcaa
acctgggtcgtgccatcgcggctcatcccgatcgtgaggcactggtcgatgtacccagcggacgccgttggacttacg
cagagtttggcgcggccgtagatgaattagcacgcggcctgttagccaaaggggtaactcgcggtgaccgtgtgggt
atttgggctgtgaactgtcccgaatgggttttggtgcaatacgctacagcccgtattggggtaatcatggttaatgtaaatc
ccgcttatcgcgcccacgagcttgaatatgtactgcaacagagtggcatttccttattagtggcttcacttgcacacaaa
agttcagattaccgcgcaattgtggagcaagtgcgcggccgctgtcccgccttacgtgaaactgtgtacatcggtgatc
catcatgggatgccttgactgcaggcgcagcggctgtcgaacaagatcgtgttgacgctctggcggcggagctttcat
gtgacgaccctgtcaacattcagtacactagcggtacgactggttttccgaaaggagcaacattatctcaccataacat
cttgaacaacggttattgggtagggcgcacagtcggctacactgagcaagaccgtgtctgcttaccagtcccgttctat
cattgctttgggatggtgatgggaaatcttggagccacatcccatggggcctgtattgtgatcccggccccctccttcga
gcctgccgcgactttagaagctgttcagcgcgaacgttgtacaagcctgtacggcgttcccacaatgtttattgcggag
cttaacctgccggactttgcctcatacgatttgacgagcctgcgcactggcatcatggcagggtcgccctgcccagtag
aagtcatgaagcgtgtcgttgctgagatgcatatggagcaggtctcgatttgttatggtatgacggagaccagtcccgtg
agtcttcaaactcgcatggacgacgacttagaacaccgtacaggtacggtcggtcgtgtacttccgcacattgaagtc
aaagtagtggaccccgtgacaggtgtaacccttccccgcggggaggcaggggagcttcgcactcgtggatacagc
gtaatgctgggttattgggaggaacctggcaagacggctgaggctatcgatccgggtcgttggatgcacacaggcga
tcttgcggtgatgcgtgaagatgggtatgttgagattgttggcgcatcaaggacatgattattcgcggcggtgaaaac
atttatcctcgcgaggttgaagaatttttatatgcacacccaaagatcgcagacgtacaggtagtcggcgtgccacatg
agcgttatggagaagaggtactggcgtgcgttgtcgttcgcgacgcggccgacccactgaccctggaagaattacgc
gcctactgtgcaggccagcttgctcattataaagtcccttcgcgtttacaactttggattcgttccctatgaccgtgtcagg
aaaggtacgtaaggttgagttacgtgagcgctacgggacacgcccgtga

### LiuABCDE operon from Pseudomonas aeruginosa:

### AA sequences:

SEQ ID NO: 68: liuA: 
MTYPSLNFALGETIDMLRDQVRGFVAAELQPRAAQIDQDNQFPMDMWRKFGEMG
LLGITVDEEYGGSALGYLAHAVVMEEISRASASVALSYGAHSNLCVNQIKRNGNAE
QKARYLPALVSGEHIGALAMSEPNAGSDVVSMKLRADRVGDRFVLNGSKMWITNG
PDAHTYVIYAKTDADKGAHGITAFIVERDWKGFSRGPKLDKLGMRGSNTCELIFQD
VEVPEENVLGAVNGGVKVLMSGLDYERVVLSGGPVGIMQACMDVVVPYIHDRRQF
GQSIGEFQLVQGKVADMYTALNASRAYLYAVAAACDRGETTRKDAAGVILYSAERA
TQMALDAIQILGGNGYINEFPTGRLLRDAKLYEIGAGTSEIRRMLIGRELFNETR*
SEQ ID NO: 69 LiuB: 
MAILHTQINPRSAEFAANAATMLEQVNALRTLLGRIHEGGGSAAQARHSARGKLLV
RERINRLLDPGSPFLELSALAAHEVYGEEVAAAGIVAGIGRVEGVECMIVGNDATVK
 
GGTYYPLTVKKHLRAQAIALENRLPCIYLVDSGGANLPRQDEVFPDREHFGRIFFNQ
ANMSARGIPQIAVVMGSCTAGGAYVPAMSDETVMVREQATIFLAGPPLVKAATGEV
VSAEELGGADVHCKVSGVADHYAEDDDHALAIARRCVANLNWRKQGQLQCRAPR
APLYPAEELYGVIPADSKQPYDVREVIARLVDGSEFDEFKALFGTTLVCGFAHLHGY
PIAILANNGILFAEAAQKGAHFIELACQRGIPLLFLQNITGFMVGQKYEAGGIAKHGAK
LVTAVACARVPKFTVLIGGSFGAGNYGMCGRAYDPRFLWMWPNARIGVMGGEQA
AGVLAQVKREQAERAGQQLGVEEEAKIKAPILEQYEHQGHPYYSSARLWDDGVID
PAQTREVLALALSAALNAPIEPTAFGVFRM*
SEQ ID NO: 70: LiuC: 
MSEFQTIQLEIDPRGVATLWLDRAEKNNAFNAVVIDELLQAIDRVGSDPQVRLLVLR
GRGRHFCGGADLAWMQQSVDLDYQGNLADAQRIAELMTHLYNLPKPTLAVVQGA
VFGGGVGLVSCCDMAIGSDDATFCLSEVRIGLIPATIAPFVVKAIGQRAARRYSLTAE
RFDGRRASELGLLSESCPAAELESQAEAWIANLLQNSPRALVACKALYHEVEAAEL
SPALRRYTEAAIARIRISPEGQEGLRAFLEKRTPAWRNDA*
SEQ ID NO: 71 LiuD: 
MNPDYRSIQRLLVANRGEIACRVMRSARALGIGSVAVHSDIDRHARHVAEADIAVDL
GGAKPADSYLRGDRIIAAALASGAQAIHPGYGFLSENADFARACEEAGLLFLGPPAA
AIDAMGSKSAAKALMEEAGVPLVPGYHGEAQDLETFRREAGRIGYPVLLKAAAGG
GGKGMKVVEREAELAEALSSAQREAKAAFGDARMLVEKYLLKPRHVEIQVFADRH
GHCLYLNERDCSIQRRHQKVVEEAPAPGLGAELRRAMGEAAVRAAQAIGYVGAGT
VEFLLDERGQFFFMEMNTRLQVEHPVTEAITGLDLVAWQIRVARGEALPLTQEQVP
LNGHAIEVRLYAEDPEGDFLPASGRLMLYREAAAGPGRRVDSGVREGDEVSPFYD
PMLAKLIAWGETREEARQRLLAMLAETSVGGLRTNLAFLRRILGHPAFAAAELDTGF
IARHQDDLLPAPQALPEHFWQAAAEAWLQSEPGHRRDDDPHSPWSRNDGWRSA
LARESDLMLRCRDERRCVRLRHASPSQYRLDGDDLVSRVDGVTRRSAALRRGRQ
LFLEWEGELLAIEAVDPIAEAEAAHAHQGGLSAPMNGSIVRVLVEPGQTVEAGATLV
VLEAMKMEHSIRAPHAGVVKALYCSEGELVEEGTPLVELDENQA*
SEQ ID NO: 72 LiuE: 
MNLPKKVRLVEVGPRDGLQNEKQPIEVADKIRLVDDLSAAGLDYIEVGSFVSPKWV
PQMAGSAEVFAGIRQRPGVTYAALAPNLKGFEAALESGVKEVAVFAAASEAFSQR
NINCSIKDSLERFVPVLEAARQHQVRVRGYISCVLGCPYDGDVDPRQVAWVAREL
QQMGCYEVSLGDTIGVGTAGATRRLIEAVASEVPRERLAGHFHDTYGQALANIYAS
LLEGIAVFDSSVAGLGGCPYAKGATGNVASEDVLYLLNGLEIHTGVDMHALVDAGQ
RICAVLGKSNGSRAAKALLAKA**
SEQ ID NO: 73 liuABCDE codon optimized sequence: 
atgacttacccgtccctgaattttgcgctgggcgaaaccattgacatgttgcgcgaccaagttcgtggcttcgttgcagc
ggaactgcaacctcgcgcggctcaaattgaccaggataatcagtttccgatggatatgtggcgtaagttcggtgagat
ggggctcttaggtattacggttgatgaggaatacggaggtagcgcgctcggttacttagcccatgcggtcgtaatggaa
gaaatttcccgtgcctctgcgagcgtagcgctgtcttatggtgcgcattcaaacctgtgcgttaaccagatcaaacgca
atggtaacgctgaacagaaagcgcgttatctgccggctttggtgtccggcgaacacattggcgccctcgctatgtcgg
aacctaacgcagggtcggatgtggtgtctatgaaactgcgcgcggatcgcgttggcgatcgtttcgtgctgaatggttc
caaaatgtggatcaccaacgggcctgatgcacatacgtatgtgatctacgctaaaaccgacgcagataaaggggcc
catggcatcaccgcatttattgttgagcgtgactggaaagggtttagccgtggcccaaaactggataaactcggtatgc
gtggttcaaatacatgtgaactgattttccaagacgtcgaagtccccgaagaaaatgtgctgggtgcagtgaatgggg
gggtcaaagtgttaatgtctggtctcgattatgaacgtgtagtgctgagcggtggtccggttggtattatgcaagcctgtat
ggacgtggtagtgccgtacattcatgatcgccgccagttcggccagtcgatcggagaatttcagctggtgcagggtaa
 
ggttgcggacatgtataccgctctgaatgcttctcgtgcgtacttgtatgctgtcgctgcagcctgcgatcgtggagaaac
gactcgcaaagacgctgctggtgtgattctctacagcgcagaacgtgctacccaaatggcacttgacgcgatccaga
tcttgggaggcaatgggtatatcaatgagttccccacgggccgcctgctgcgcgatgcgaagctgtatgagatcggcg
cgggtacgagcgaaatccgccgtatgttaatcggtcgtgaattatttaacgagactcgctgaagcctcgctcttcccgg
cccttttccgccagggagagggcattccattgcatcgacaggcgcatcgccaggtcgggagcggcgccaaccgct
tccgcccacctcgacacggagccaccgccatggccatccttcacacgcagattaacccgcgttctgctgaattcgcg
gcgaatgccgcgaccatgctggagcaagttaacgcattgcgtacgctccttggtcgcatccacgaaggtggttcg
gcggctcaggctcgccattcggcacgtggcaaattgttggttcgcgaacgcatcaaccgcctgctggaccccggtag
cccgtttttggagttgagcgcgttagcagctcatgaggtgtatggggaagaagtcgcagcagcaggtatcgtggccgg
gatcggcgtgtagaaggagtagaatgtatgatcgttggtaatgatgccactgtgaaaggaggtacgtattacccgct
gaccgtgaagaagcatctgcgcgcccaagcaatcgcattagaaaatcgtttgccgtgtatctatctggtcgattcgggt
ggcgccaatctgcctcgccaggacgaggtctttccggatcgcgagcatttcggccgcatctttttcaaccaagccaata
tgagcgcccgcggtatcccgcagattgcggtggtaatgggctcatgtactgcgggtggcgcctatgtcccggccatgt
ccgatgaaactgtgatggtccgtgagcaggcgacgatcttcctggctggaccgcctctcgtgaaagcggccacgggt
gaagtggtttcagcagaggaattgggtggcgccgacgtgcattgtaaagtgtcaggcgtggcggaccactatgccga
agatgatgaccatgcattggcgattgcgcgtcgctgtgttgcgaatttaaattggcgcaaacagggtcagcttcagtgc
cgtgcgccgcgtgctccgctgtatccggcggaagaactgtatggtgtgattccggcggatagcaaacagccgtatgat
gtgcgcgaggtcattgcacgcctggttgatggatctgaatttgatgaattcaaggcgctgttcggaaccaccctggtgtg
cggctttgcacacctgcatggctacccaattgccattctcgcaaataatggcattctgttcgcggaggcggcccagaaa
ggggcccatttcattgaactggcctgccaacgcggtattccattactgttcctgcaaaatatcaccggcttcatggttggt
cagaagtatgaagctggcggtattgccaagcatggcgcgaaactggtcaccgcggtcgcctgcgcccgcgtgccg
aaatttacagtgctgattggcggaagtttcggggcagggaactacggaatgtgtggtcgcgcgtacgatccgcgcttc
ctctggatgtggccgaatgcacgcattggcgtgatgggcggcgagcaggctgccggcgtcctggcacaggtcaaac
gtgagcaagcggaacgcgctggccaacagctgggggtggaggaagaagcgaaaattaaagcgccgatccttga
acagtatgaacatcagggccatccgtactattcgtcagcacgtttgtgggacgatggcgtcattgatcctgcccagaca
cgcgaagtccttgcgctggcgctgagtgcggcgcttaacgctccgatcgaaccaactgcattcggtgtatttcgcatgt
gacgagtagaccagcatgagcgaatttcagacgatccagctggaaattgatccacgtggagtggcaaccctgtggc
tggaccgtgctgaaaaaaataacgcatttaacgccgtcgtgatcgatgaactgctgcaggcgatcgaccgcgtaggc
agcgacccccaggtccgtttgctggtcttgcgtgggcgtggccgtcatttctgtggcggcgccgacctggcgtggatgc
agcagtctgttgacctggattatcagggtaaccttgctgacgcccagcgcatcgcagagctcatgacccacttgtataa
tctgcccaaacctactttagcggtagttcaaggcgcagttttcggcggcggggtcggtttggtgagctgctgcgacatgg
caattggtagtgatgacgccactttttgcttgtcagaggtacgcattgggctgattccagcaaccatcgccccgttcgtgg
tgaaagctattggtcaacgcgcagcgcgccgttattcactgactgctgaacgttttgatggcgccgcgcgtccgaact
gggactgcttagcgagtcttgcccggccgcagaactggaatcccaagcggaagcatggatcgcgaatcttctccag
aactctccacgtgcactcgtggcatgtaaagcgctgtatcacgaggtagaagcggctgaactgtcccctgcactgcgt
cgctatacggaagccgcaattgcacgtatccgtatttcaccagaaggtcaagaaggcttgcgtgcctttttagaaaaac
gcacaccggcgtggagaaacgacgcatgaacccggactaccgttcaattcagcgtctcttagtagctaaccgtggc
gagattgcctgtcgcgtaatgcgttcggcccgcgcgttaggtattggatcagttgcagttcattcggatatcgaccgcca
cgcacgtcacgtggctgaagctgatattgcggttgacctgggcggcgccaaaccggcagattcgtatctgcgtggcg
accgtatcattgcagctgcactggcttcaggagcccaggccattcatccggggtatggctttctgtctgagaatgctgatt
ttgcccgcgcgtgcgaagaagcaggtttactgtttttgggcccaccggctgcggcaattgatgctatggggtctaagtca
gcggcgaaagctttgatggaagaggcgggagtcccccctggttccaggttaccacggtgaagcgcaggacttggaa
acctttcgtcgcgaggccggacgcatcggctatcccgtgctcttaaaggccgcggccggtggcggcggaaaaggg
atgaaagtcgtggaacgcgaggccgagctcgcagaagcgctgtccagcgcccaacgcgaagccaaagcggcct
ttggcgatgcgcgcatgctggtggagaagtattgttaaaaccgcgtcacgtcgaaattcaggtctttgcagatcgtcat
ggtcactgtttatacctcaacgaacgtgactgttcgatccaacgtcgccatcaaaaagttgtagaagaagcgccggct
cccggtttgggcgcggaactgcgtcgtgccatgggcgaagcggccgttcgcgcagcgcaagcgatcggctatgtgg
gggcgggcactgtagagtttctcctggacgagcgcggtcaattctttttatggaaatgaacactcgcctgcaggttgaa
caccctgtaactgaggccatcactggtctcgatttagtcgcgtggcagatccgtgtggcgcgtggtgaagcccttccgtt
gactcaagaacaagtaccgctgaacgggcacgcgatcgaagtccgcctgtacgcggaagaccctgaaggggattt
 
tcttccggcaagtggacgcctgatgctgtatcgtgaagccgctgcaggtccgggccgccgcgtggattcgggagtcc
gtgagggcgacgaagtcagccccttctacgatccgatgctggcaaaattgatcgcatgggggaaacccgtgagg
aagctcgccaacgcctgctcgccatgttggccgagacctcggtcgggggcttgcgtacgaacctggctttttacgtcgt
atcttaggccatcccgcttttgccgccgctgaactggataccgggttcattgctcgtcatcaagatgacctgctgccagc
accccaggctctgccagaacacttctggcaagcagcagcagaagcttggctgcaaagcgaacctggtcatcgtcgc
gatgacgatccgcattccccttggagccgtaacgatggttggcgctctgctttggcacgcgaatctgatctgatgctgcg
ctgtcgcgatgaacgccgttgtgtgcgtctgcgccatgcttccccatctcaatatcgtcttgacggtgatgatctggtatcc
cgtgttgatggcgttacccgccgctccgcagcgttgcgtcgcggccgccagctgttcttagaatggaaggtgaactgt
tagcgatcgaagctgttgatccgattgcagaagccgaagcggcgcatgcccatcaaggcggtttgagcgcgccaat
gaacgggtctattgtacgcgttctggttgagccggggcaaaccgtagaggcgggtgcgactcttgtggttttagaagca
atgaaaatggagcacagtatccgtgcgccacatgccggcgttgttaaagcgctgtactgttcagaaggagaattagtt
gaagagggcactcctctggttgaactggacgaaaaccaggcctgacagccaagacgaggaacagcatgaacctg
ccgaagaaagttcgtctggttgaagttggtccgcgcgatggacttcagaacgaaaaacagccgatcgaagtggctg
acaaaattcgccttgttgatgacttgtcggcagccggcttagattatattgaagtgggcagtttcgtctcaccgaaatggg
ttccgcagatggccgggagcgccgaagtgtttgctggcattcgtcaacgccctggcgtgacctacgcggcactcgcc
ccgaatttgaaaggcttcgaagcagctctggaatcgggtgtaaaagaagttgccgtgttcgcagcagcctccgaagc
attctcccaacgcaacatcaactgctcgattaaagactcccttgagcgcttcgtcccggttctggaagcggctcgccaa
catcaggtacgcgtccgcggatatatttcctgcgtattgggttgcccgtatgatggcgacgtagatccgcgccaggtcg
catgggtcgcacgtgaactccagcagatgggctgctatgaggtcagtctcggcgatacaatcggtgtgggtaccgcg
ggcgcgacccgccgtttaattgaggcggtggcatctgaggttccccgcgaacgccttgcaggccactttcatgatacat
atggacaggcgctggctaacatctatgcttctttgctggagggcattgctgtcttcgacagttccgtagctggcctcggtg
gctgcccatatgcaaaaggcgctaccggcaacgtcgcgagtgaggatgtgctgtatcttttaaatggtcttgaaattcat
accggtgtggacatgcatgccctggtagacgcgggacagcgcatctgtgcggtgctcggaaagtcgaatggctccc
gtgctgcgaaggccctgctggccaaagcttaatga
SEQ ID NO: 91 Nucleotide sequence of the livKHMGF operon: 
atgaaacggaatgcgaaaactatcatcgcagggatgattgcactggcaatttcacacaccgctatggctgacgatatt
aaagtcgccgttgtcggcgcgatgtccggcccgattgcccagtggggcgatatggaatttaacggcgcgcgtcaggc
aattaaagacattaatgccaaaggggggaattaagggcgataaactggttggcgtggaatatgacgacgcatgcgac
ccgaaacaagccgttgcggtcgccaacaaaatcgttaatgacggcattaaatacgttattggtcatctgtgttcttcttcta
cccagcctgcgtcagatatctatgaagacgaaggtattctgatgatctcgccgggagcgaccaacccggagctgac
ccaacgcggttatcaacacattatgcgtactgccgggctggactcttcccaggggccaacggcggcaaaatacattct
tgagacggtgaagccccagcgcatcgccatcattcacgacaaacaacagtatggcgaagggctggcgcgttcggt
gcaggacgggctgaaagcggctaacgccaacgtcgtcttcttcgacggtattaccgccggggagaaagatttctccg
cgctgatcgcccgcctgaaaaaagaaaacatcgacttcgtttactacggcggttactacccggaaatggggcagatg
ctgcgccaggcccgttccgttggcctgaaaacccagtttatggggccggaaggtgtgggtaatgcgtcgttgtcgaac
attgccggtgatgccgccgaaggcatgttggtcactatgccaaaacgctatgaccaggatccggcaaaccagggca
tcgttgatgcgctgaaagcagacaagaaagatccgtccgggccttatgtctggatcacctacgcggcggtgcaatctc
tggcgactgcccttgagcgtaccggcagcgatgagccgctggcgctggtgaaagatttaaaagctaacggtgcaaa
caccgtgattgggccgctgaactgggatgaaaaaggcgatcttaagggatttgattttggtgtcttccagtggcacgcc
gacggttcatccacggcagccaagtgatcatcccaccgcccgtaaaatgcgggcgggtttagaaaggttaccttatgt
ctgagcagtttttgtatttcttgcagcagatgtttaacggcgtcacgctgggcagtacctacgcgctgatagccatcggct
acaccatggtttacggcattatcggcatgatcaacttcgcccacggcgaggtttatatgattggcagctacgtctcatttat
gatcatcgccgcgctgatgatgatgggcattgataccggctggctgctggtagctgcgggattcgtcggcgcaatcgtc
attgccagcgcctacggctggagtatcgaacgggtggcttaccgcccggtgcgtaactctaagcgcctgattgcactc
atctctgcaatcggtatgtccatcttcctgcaaaactacgtcagcctgaccgaaggttcgcgcgacgtggcgctgccga
gcctgtttaacggtcagtgggtggtggggcatagcgaaaacttctctgcctctattaccaccatgcaggcggtgatctgg
attgttaccttcctcgccatgctggcgctgacgattttcattcgctattcccgcatgggtcgcgcgtgtcgtgcctgcgcgg
aagatctgaaaatggcgagtctgcttggcattaacaccgaccgggtgattgcgctgacctttgtgattggcgcggcgat
ggcggcggtggcgggtgtgctgctcggtcagttctacggcgtcattaacccctacatcggctttatggccgggatgaaa
 
gcctttaccgcggcggtgctcggtgggattggcagcattccgggagcgatgattggcggcctgattctggggattgcg
gaggcgctctcttctgcctatctgagtacggaatataaagatgtggtgtcattcgccctgctgattctggtgctgctggtga
tgccgaccggtattctgggtcgcccggaggtagagaaagtatgaaaccgatgcatattgcaatggcgctgctctctgc
cgcgatgttctttgtgctggcgggcgtctttatgggcgtgcaactggagctggatggcaccaaactggtggtcgacacg
gcttcggatgtccgttggcagtgggtgtttatcggcacggcggtggtcttttcttccagcttttgcgaccggctttccagaa
agggttgaaaagcgtttccggaccgaagtttattctgcccgccattgatggctccacggtgaagcagaaactgttcctc
gtggcgctgttggtgcttgcggtggcgtggccgtttatggtttcacgcgggacggtggatattgccaccctgaccatgatc
tacattatcctcggtctggggctgaacgtggttgttggtctttctggtctgctggtgctggggtacggcggtttttacgccatc
ggcgcttacacttttgcgctgctcaatcactattacggcttgggcttctggacctgcctgccgattgctggattaatggcag
cggcggcgggcttcctgctcggttttccggtgctgcgtttgcgcggtgactatctggcgatcgttaccctcggtttcggcga
aattgtgcgcatattgctgctcaataacaccgaaattaccggcggcccgaacggaatcagtcagatcccgaaaccg
acactcttcggactcgagttcagccgtaccgctcgtgaaggcggctgggacacgttcagtaatttctttggcctgaaata
cgatccctccgatcgtgtcatcttcctctacctggtggcgttgctgctggtggtgctaagcctgtttgtcattaaccgcctgct
gcggatgccgctggggcgtgcgtgggaagcgttgcgtgaagatgaaatcgcctgccgttcgctgggcttaagcccgc
gtcgtatcaagctgactgcctttaccataagtgccgcgtttgccggttttgccggaacgctgtttgcggcgcgtcagggct
ttgtcagcccggaatccttcacctttgccgaatcggcgtttgtgctggcgatagtggtgctcggcggtatgggctcgcaat
ttgcggtgattctggcggcaattttgctggtggtgtcgcgcgagttgatgcgtgatttcaacgaatacagcatgttaatgct
cggtggtttgatggtgctgatgatgatctggcgtccgcagggcttgctgcccatgacgcgcccgcaactgaagctgaa
aaacggcgcagcgaaaggagagcaggcatgagtcagccattattatctgttaacggcctgatgatgcgcttcggcg
gcctgctggcggtgaacaacgtcaatcttgaactgtacccgcaggagatcgtctcgttaatcggccctaacggtgccg
gaaaaaccacggtttttaactgtctgaccggattctacaaacccaccggcggcaccatttactgcgcgatcagcacct
ggaaggtttaccggggcagcaaattgcccgcatgggcgtggtgcgcaccttccagcatgtgcgtctgttccgtgaaat
gacggtaattgaaaacctgctggtggcgcagcatcagcaactgaaaaccgggctgttctctggcctgttgaaaacgc
catccttccgtcgcgcccagagcgaagcgctcgaccgcgccgcgacctggcttgagcgcattggtttgctggaacac
gccaaccgtcaggcgagtaacctggcctatggtgaccagcgccgtcttgagattgcccgctgcatggtgacgcagcc
ggagattttaatgctcgacgaacctgcggcaggtcttaacccgaaagagacgaaagagctggatgagctgattgcc
gaactgcgcaatcatcacaacaccactatcttgttgattgaacacgatatgaagctggtgatgggaatttcggaccga
atttacgtggtcaatcaggggacgccgctggcaaacggtacgccggagcagatccgtaataacccggacgtgatcc
gtgcctatttaggtgaggcataagatggaaaaagtcatgttgtcctttgacaaagtcagcgcccactacggcaaaatc
caggcgctgcatgaggtgagcctgcatatcaatcagggcgagattgtcacgctgattggcgcgaacggggcgggg
aaaaccaccttgctcggcacgttatgcggcgatccgcgtgccaccagcgggcgaattgtgtttgatgataaagacatt
accgactggcagacagcgaaaatcatgcgcgaagcggtggcgattgtcccggaagggcgtcgcgtcttctcgcgg
atgacggtggaagagaacctggcgatgggcggtttttttgctgaacgcgaccagttccaggagcgcataaagtgggt
gtatgagctgtttccacgtctgcatgagcgccgtattcagcgggcgggcaccatgtccggcggtgaacagcagatgct
ggcgattggtcgtgcgctgatgagcaacccgcgtttgctactgcttgatgagccatcgctcggtcttgcgccgattatcat
ccagcaaatttcgacaccatcgagcagctgcgcgagcaggggatgactatctttctcgtcgagcagaacgccaacc
aggcgctaaagctggcggatcgcggctacgtgctggaaaacggccatgtagtgctttccgatactggtgatgcgctgc
tggcgaatgaagcggtgagaagtgcgtatttaggcgggtaa
SEQ ID NO: 92 LivK
   AA sequence: 
MKRNAKTIIAGMIALAISHTAMADDIKVAVVGAMSGPIAQWGDMEFNGARQAIKDIN
AKGGIKGDKLVGVEYDDACDPKQAVAVANKIVNDGIKYVIGHLCSSSTQPASDIYED
EGILMISPGATNPELTQRGYQHIMRTAGLDSSQGPTAAKYILETVKPQRIAIIHDKQQ
YGEGLARSVQDGLKAANANVVFFDGITAGEKDFSALIARLKKENIDFVYYGGYYPE
MGQMLRQARSVGLKTQFMGPEGVGNASLSNIAGDAAEGMLVTMPKRYDQDPAN
QGIVDALKADKKDPSGPYVWITYAAVQSLATALERTGSDEPLALVKDLKANGANTVI
GPLNWDEKGDLKGFDFGVFQWHADGSSTAAK*
SEQ ID NO: 93 Nucleotide sequence: 
Atgaaacggaatgcgaaaactatcatcgcagggatgattgcactggcaatttcacacaccgctatggctgacgatatt
aaagtcgccgttgtcggcgcgatgtccggcccgattgcccagtggggcgatatggaatttaacggcgcgcgtcaggc
aattaaagacattaatgccaaaggggggaattaagggcgataaactggttggcgtggaatatgacgacgcatgcgac
ccgaaacaagccgttgcggtcgccaacaaaatcgttaatgacggcattaaatacgttattggtcatctgtgttcttcta
cccagcctgcgtcagatatctatgaagacgaaggtattctgatgatctcgccgggagcgaccaacccggagctgac
ccaacgcggttatcaacacattatgcgtactgccgggctggactcttcccaggggccaacggcggcaaaatacattct
tgagacggtgaagccccagcgcatcgccatcattcacgacaaacaacagtatggcgaagggctggcgcgttcggt
gcaggacgggctgaaagcggctaacgccaacgtcgtcttcttcgacggtattaccgccggggagaaagatttctccg
cgctgatcgcccgcctgaaaaaagaaaacatcgacttcgtttactacggcggttactacccggaaatggggcagatg
ctgcgccaggcccgttccgttggcctgaaaacccagtttatggggccggaaggtgtgggtaatgcgtcgttgtcgaac
attgccggtgatgccgccgaaggcatgttggtcactatgccaaaacgctatgaccaggatccggcaaaccagggca
tcgttgatgcgctgaaagcagacaagaaagatccgtccgggccttatgtctggatcacctacgcggcggtgcaatctc
tggcgactgcccttgagcgtaccggcagcgatgagccgctggcgctggtgaaagatttaaaagctaacggtgcaaa
caccgtgattgggccgctgaactggatgaaaaaggcgatcttaagggatttgattttggtgtcttccagtggcacgcc
gacggttcatccacggcagccaagtga
SEQ ID NO: 94 LivH
   AA sequence: 
MSEQFLYFLQQMFNGVTLGSTYALIAIGYTMVYGIIGMINFAHGEVYMIGSYVSFMII
AALMMMGIDTGWLLVAAGFVGAIVIASAYGWSIERVAYRPVRNSKRLIALISAIGMSI
FLQNYVSLTEGSRDVALPSLFNGQWVVGHSENFSASITTMQAVIWIVTFLAMLALTI
FIRYSRMGRACRACAEDLKMASLLGINTDRVIALTFVIGAAMAAVAGVLLGQFYGVI
NPYIGFMAGMKAFTAAVLGGIGSIPGAMIGGLILGIAEALSSAYLSTEYKDVVSFALLI
LVLLVMPTGILGRPEVEKV*
SEQ ID NO: 95 Nucleotide sequence: 
Atgtctgagcagtttttgtatttcttgcagcagatgtttaacggcgtcacgctgggcagtacctacgcgctgatagccatc
ggctacaccatggtttacggcattatcggcatgatcaacttcgcccacggcgaggtttatatgattggcagctacgtctc
atttatgatcatcgccgcgctgatgatgatgggcattgataccggctggctgctggtagctgcgggattcgtcggcgca
atcgtcattgccagcgcctacggctggagtatcgaacgggtggcttaccgcccggtgcgtaactctaagcgcctgatt
gcactcatctctgcaatcggtatgtccatcttcctgcaaaactacgtcagcctgaccgaaggttcgcgcgacgtggcgc
tgccgagcctgtttaacggtcagtgggtggtggggcatagcgaaaacttctctgcctctattaccaccatgcaggcggt
gatctggattgttaccttcctcgccatgctggcgctgacgattttcattcgctattcccgcatgggtcgcgcgtgtcgtgcct
gcgcggaagatctgaaaatggcgagtctgcttggcattaacaccgaccgggtgattgcgctgacctttgtgattggcg
cggcgatggcggcggtggcgggtgtgctgctcggtcagttctacggcgtcattaacccctacatcggctttatggccgg
gatgaaagcctttaccgcggcggtgctcggtgggattggcagcattccgggagcgatgattggcggcctgattctggg
gattgcggaggcgctctcttctgcctatctgagtacggaatataaagatgtggtgtcattcgccctgctgattctggtgctg
ctggtgatgccgaccggtattctgggtcgcccggaggtagagaaagtatga
LivM
   SEQ ID NO: 96 AA sequence: 
MKPMHIAMALLSAAMFFVLAGVFMGVQLELDGTKLVVDTASDVRWQWVFIGTAVV
FFFQLLRPAFQKGLKSVSGPKFILPAIDGSTVKQKLFLVALLVLAVAWPFMVSRGTV
DIATLTMIYIILGLGLNVVVGLSGLLVLGYGGFYAIGAYTFALLNHYYGLGFWTCLPIA
GLMAAAAGFLLGFPVLRLRGDYLAIVTLGFGEIVRILLLNNTEITGGPNGISQIPKPTL
FGLEFSRTAREGGWDTFSNFFGLKYDPSDRVIFLYLVALLLVVLSLFVINRLLRMPL
GRAWEALREDEIACRSLGLSPRRIKLTAFTISAAFAGFAGTLFAARQGFVSPESFTF
AESAFVLAIVVLGGMGSQFAVILAAILLVVSRELMRDFNEYSMLMLGGLMVLMMIWR
PQGLLPMTRPQLKLKNGAAKGEQA*
SEQ ID NO: 97Nucleotide sequence: 
atgaaaccgatgcatattgcaatggcgctgctctctgccgcgatgttctttgtgctggcgggcgtctttatgggcgtgcaa
ctggagctggatggcaccaaactggtggtcgacacggcttcggatgtccgttggcagtgggtgtttatcggcacggcg
gtggtcttttcttccagcttttgcgaccggctttccagaaagggttgaaaagcgtttccggaccgaagtttattctgcccgc
cattgatggctccacggtgaagcagaaactgttcctcgtggcgctgttggtgcttgcggtggcgtggccgtttatggtttc
acgcgggacggtggatattgccaccctgaccatgatctacattatcctcggtctggggctgaacgtggttgttggtctttct
ggtctgctggtgctggggtacggcggtttttacgccatcggcgcttacacttttgcgctgctcaatcactattacggcttgg
gcttctggacctgcctgccgattgctggattaatggcagcggcggcgggcttcctgctcggttttccggtgctgcgtttgc
gcggtgactatctggcgatcgttaccctcggtttcggcgaaattgtgcgcatattgctgctcaataacaccgaaattacc
ggcggcccgaacggaatcagtcagatcccgaaaccgacactcttcggactcgagttcagccgtaccgctcgtgaag
gcggctgggacacgttcagtaatttctttggcctgaaatacgatccctccgatcgtgtcatcttcctctacctggtggcgtt
gctgctggtggtgctaagcctgtttgtcattaaccgcctgctgcggatgccgctggggcgtgcgtgggaagcgttgcgtg
aagatgaaatcgcctgccgttcgctgggcttaagcccgcgtcgtatcaagctgactgcctttaccataagtgccgcgttt
gccggttttgccggaacgctgtttgcggcgcgtcagggctttgtcagcccggaatccttcacctttgccgaatcggcgttt
gtgctggcgatagtggtgctcggcggtatgggctcgcaatttgcggtgattctggcggcaattttgctggtggtgtcgcgc
gagttgatgcgtgatttcaacgaatacagcatgttaatgctcggtggtttgatggtgctgatgatgatctggcgtccgcag
ggcttgctgcccatgacgcgcccgcaactgaagctgaaaaacggcgcagcgaaaggagagcaggcatga
LivG
   SEQ ID NO: 98 AA sequence: 
MSQPLLSVNGLMMRFGGLLAVNNVNLELYPQEIVSLIGPNGAGKTTVFNCLTGFYK
PTGGTILLRDQHLEGLPGQQIARMGVVRTFQHVRLFREMTVIENLLVAQHQQLKTG
LFSGLLKTPSFRRAQSEALDRAATWLERIGLLEHANRQASNLAYGDQRRLEIARCM
VTQPEILMLDEPAAGLNPKETKELDELIAELRNHHNTTILLIEHDMKLVMGISDRIYVV
NQGTPLANGTPEQIRNNPDVIRAYLGEA*
SEQ ID NO: 100 Nucleotide sequence: 
Atgagtcagccattattatctgttaacggcctgatgatgcgcttcggcggcctgctggcggtgaacaacgtcaatcttga
actgtacccgcaggagatcgtctcgttaatcggccctaacggtgccggaaaaaccacggtttttaactgtctgaccgg
attctacaaacccaccggcggcaccatttactgcgcgatcagcacctggaaggtttaccggggcagcaaattgccc
gcatgggcgtggtgcgcaccttccagcatgtgcgtctgttccgtgaaatgacggtaattgaaaacctgctggtggcgca
gcatcagcaactgaaaaccgggctgttctctggcctgttgaaaacgccatccttccgtcgcgcccagagcgaagcgc
tcgaccgcgccgcgacctggcttgagcgcattggtttgctggaacacgccaaccgtcaggcgagtaacctggcctat
ggtgaccagcgccgtcttgagattgcccgctgcatggtgacgcagccggagattttaatgctcgacgaacctgcggc
aggtcttaacccgaaagagacgaaagagctggatgagctgattgccgaactgcgcaatcatcacaacaccactatc
ttgttgattgaacacgatatgaagctggtgatgggaatttcggaccgaatttacgtggtcaatcaggggacgccgctgg
caaacggtacgccggagcagatccgtaataacccggacgtgatccgtgcctatttaggtgaggcataa
LivF
   SEQ ID NO: 101 AA sequence: 




*
SEQ ID NO: 102 Nucleotide sequence:

atggaaaaagtcatgttgtcctttgacaaagtcagcgcccactacggcaaaatccaggcgctgcatgaggtgagcct
gcatatcaatcagggcgagattgtcacgctgattggcgcgaacggggcggggaaaaccaccttgctcggcacgttat
gcggcgatccgcgtgccaccagcgggcgaattgtgtttgatgataaagacattaccgactggcagacagcgaaaat
catgcgcgaagcggtggcgattgtcccggaagggcgtcgcgtcttctcgcggatgacggtggaagagaacctggcg
atgggcggtttttttgctgaacgcgaccagttccaggagcgcataaagtgggtgtatgagctgtttccacgtctgcatga
gcgccgtattcagcgggcgggcaccatgtccggcggtgaacagcagatgctggcgattggtcgtgcgctgatgagc
aacccgcgtttgctactgcttgatgagccatcgctcggtcttgcgccgattatcatccagcaaattttcgacaccatcgag
cagctgcgcgagcaggggatgactatctttctcgtcgagcagaacgccaaccaggcgctaaagctggcggatcgc
ggctacgtgctggaaaacggccatgtagtgctttccgatactggtgatgcgctgctggcgaatgaagcggtgagaagt
gcgtatttaggcgggtaa

## Claims

1. A pharmaceutical composition comprising a bacterium comprising gene sequence(s) encoding one or more branched chain amino acid catabolism enzyme(s) operably linked to a promoter that is not associated with the branched chain amino acid catabolism enzyme gene in nature, and gene sequence(s) encoding one or more transporter(s) of a branched chain amino acid that is operably linked to a promoter that is not associated with the transporter gene in nature, wherein the transporter is capable of importing the branched chain amino acid or metabolite thereof.

2. The pharmaceutical composition of claim 1, wherein the bacterium further comprises a genetic modification that reduces export of a branched chain amino acid from the bacterium.

3. The pharmaceutical composition of claim 1 or 2, wherein the bacterium further comprises a genetic modification that reduces endogenous biosynthesis of a branched chain amino acid in the bacterium.

4. The pharmaceutical composition of any one of claims 1-3, wherein the gene sequence(s) encoding the branched chain amino acid catabolism enzyme(s) comprise one or more gene(s) selected from branched chain amino acid dehydrogenase gene(s), branched chain amino acid aminotransferase gene(s), branched chain amino acid oxidase gene(s); α-ketoacid decarboxylase gene(s), and branched chain amino acid alcohol dehydrogenase gene(s).

5. The pharmaceutical composition of claim 4, wherein the gene sequence(s) encoding the branched chain amino acid catabolism enzyme(s) are selected from branched chain amino acid aminotransferase gene(s) comprising *ilvE;* α-ketoacid decarboxylase gene(s) comprising *kivD;* and branched chain amino acid alcohol dehydrogenase gene(s) comprising gene(s) selected from *adhl*, *adh2*, *adh3*, *adh4*, *adh5*, *adh6*, *adh7*, *sfa1*, and *yqhD.*

6. The pharmaceutical composition of any one of claims 1-5, wherein the gene sequence(s) encoding one or more transporters of branched chain amino acid comprise *livKHMGF* and *brnQ.*

7. The pharmaceutical composition of any one of claims 2-6, wherein the genetic modification that reduces export of a branched chain amino acid from the bacterium is a mutation or deletion in the *leuE* gene.

8. The pharmaceutical composition of any one of claims 3-7, wherein the genetic modification that reduces endogenous biosynthesis of a branched chain amino acid in the bacterium is a mutation or deletion in the *ilvC* gene.

9. The pharmaceutical composition of any of claims 1-8, wherein the promoter operably linked to the gene sequence(s) encoding a branched chain amino acid catabolism enzyme and the promoter operably linked to the gene sequence(s) encoding a transporter of a branched chain amino acid are separate copies or the same copy of the same promoter.

10. The pharmaceutical composition of any of claims 1-8, wherein the promoter operably linked to the gene sequence(s) encoding a branched chain amino acid catabolism enzyme and the promoter operably linked to the gene sequence(s) encoding a transporter of a branched chain amino acid are different promoters.

11. The pharmaceutical composition of any one of claims 1-10, wherein the promoter operably linked to the gene sequence(s) encoding a branched chain amino acid catabolism enzyme or the promoter operably linked to the gene sequence(s) encoding a transporter of a branched chain amino acid is directly or indirectly inducible by exogenous environmental conditions found in the mammalian gut.

12. The pharmaceutical composition of any one of claims 1-11, wherein the promoter operably linked to the gene sequence(s) encoding a branched chain amino acid catabolism enzyme or the promoter operably linked to the gene sequence(s) encoding a transporter of a branched chain amino acid is directly or indirectly inducible under low-oxygen or anaerobic conditions.

13. The pharmaceutical composition of any one of claims 1-12, wherein the gene sequence(s) encoding a branched chain amino acid catabolism enzyme and/or the gene sequence(s) encoding a transporter of a branched chain amino acid are located on a chromosome and/or a plasmid in the bacterium.

14. The pharmaceutical composition of any one of claims 1-13, wherein the bacterium is a probiotic bacterium selected from the group consisting of *Bacteroides, Bifidobacterium, Clostridium, Escherichia, Lactobacillus,* and *Lactococcus.*

15. The pharmaceutical composition of claim 14, wherein the bacterium is *Escherichia coli* strain Nissle.

## Patentansprüche

1. Pharmazeutische Zusammensetzung, die ein Bakterium umfasst, das (eine) Gensequenz(en) umfasst, die ein oder mehrere verzweigtkettige Aminosäure-Katabolismus-Enzyme kodiert/kodieren, die funktionsfähig mit einem Promotor verknüpft sind, der in der Natur nicht mit dem verzweigtkettigen Aminosäure-Katabolismus-Enzym assoziiert ist, und (eine) Gensequenz(en) umfasst, die einen oder mehrere Transporter einer verzweigtkettigen Aminosäure kodiert/kodieren, die funktionsfähig mit einem Promotor verknüpft ist, der in der Natur nicht mit dem Transportergen assoziiert ist, wobei der Transporter die verzweigtkettige Aminosäure oder einen Metaboliten davon importieren kann.

2. Pharmazeutische Zusammensetzung nach Anspruch 1, wobei das Bakterium ferner eine genetische Modifikation umfasst, die den Export einer verzweigtkettigen Aminosäure aus dem Bakterium reduziert.

3. Pharmazeutische Zusammensetzung nach Anspruch 1 oder 2, wobei das Bakterium ferner eine genetische Modifikation umfasst, die die endogene Biosynthese einer verzweigtkettigen Aminosäure in dem Bakterium reduziert.

4. Pharmazeutische Zusammensetzung nach einem der Ansprüche 1-3, wobei die Gensequenz(en), die das/die verzweigtkettige(en) Aminosäure-Katabolismus-Enzym(e) kodiert/kodieren, ein oder mehrere Gene umfasst/umfassen, die aus (einem) verzweigtkettigen Aminosäure-Dehydrogenase-Gen(en), verzweigtkettigen Aminosäure-Aminotransferase-Gen(en), verzweigtkettigen Aminosäure-Oxidase-Gen(en), α-Ketosäure-Decarboxylase-Gen(en) und verzweigtkettigen Aminosäure-Alkohol-Dehydrogenase-Gen(en) ausgewählt sind.

5. Pharmazeutische Zusammensetzung nach Anspruch 4, wobei die Gensequenz(en), die das/die verzweigtkettige(n) Aminosäure-Katabolismus-Enzym(e) kodiert/kodieren, ausgewählt ist/sind aus (einem) verzweigtkettigen Aminosäure-Aminotransferase-Gen(en), das/die *ilvE* umfasst/umfassen; (einem) α-Ketosäure-Decarboxylase-Gen(en), das/die *kivD* umfasst/umfassen; und (einem) verzweigtkettigen Aminosäure-Alkohol-Dehydrogenase-Gen(en), das/die Gen(e), ausgewählt aus *adhl, adh2, adh3, adh4, adh5, adh6, adh7, sfal* und *yqhD,* umfassen.

6. Pharmazeutische Zusammensetzung nach einem der Ansprüche 1-5, wobei die Gensequenz(en), die einen oder mehrere Transporter einer verzweigtkettigen Aminosäure kodiert/kodieren, *livKHMGF* und *brnQ* umfasst/umfassen.

7. Pharmazeutische Zusammensetzung nach einem der Ansprüche 2-6, wobei die genetische Modifikation, die den Export einer verzweigtkettigen Aminosäure aus dem Bakterium reduziert, eine Mutation oder Deletion im *leuE-Gen* ist.

8. Pharmazeutische Zusammensetzung nach einem der Ansprüche 3-7, wobei die genetische Modifikation, die die endogene Biosynthese einer verzweigtkettigen Aminosäure in dem Bakterium reduziert, eine Mutation oder Deletion im *ilvC*-Gen ist.

9. Pharmazeutische Zusammensetzung nach einem der Ansprüche 1-8, wobei der Promotor, der funktionsfähig mit der/den Gensequenz(en) verknüpft ist, die verzweigtkettiges Aminosäure-Katabolismus-Enzym kodiert/kodieren, und der Promotor, der funktionsfähig mit der/den Gensequenz(en) verknüpft ist, die einen Transporter einer verzweigtkettigen Aminosäure kodiert/kodieren, getrennte Kopien oder die gleiche Kopie des gleichen Promotors sind.

10. Pharmazeutische Zusammensetzung nach einem der Ansprüche 1-8, wobei der Promotor, der funktionsfähig mit der/den Gensequenz(en) verknüpft ist, die ein verzweigtkettiges Aminosäure-Katabolismus-Enzym kodiert/kodieren, und der Promotor, der funktionsfähig mit der/den Gensequenz(en) verknüpft ist, die einen Transporter einer verzweigtkettigen Aminosäure kodiert/kodieren, unterschiedliche Promotoren sind.

11. Pharmazeutische Zusammensetzung nach einem der Ansprüche 1-10, wobei der Promotor, der funktionsfähig mit der/den Gensequenz(en) verknüpft ist, die ein verzweigtkettiges Aminosäure-Katabolismus-Enzym kodiert/kodieren, oder der Promotor, der funktionsfähig mit der/den Gensequenz(en) verknüpft ist, die einen Transporter einer verzweigtkettigen Aminosäure kodiert/kodieren, direkt oder indirekt durch exogene Umgebungsbedingungen im Säugetierdarm induzierbar ist.

12. Pharmazeutische Zusammensetzung nach einem der Ansprüche 1-11, wobei der Promotor, der funktionsfähig mit der/den Gensequenz(en) verknüpft ist, die ein verzweigtkettiges Aminosäure-Katabolismus-Enzym kodiert/kodieren, oder der Promotor, der funktionsfähig mit der/den Gensequenz(en) verknüpft ist, die einen Transporter einer verzweigtkettigen Aminosäure kodieren, direkt oder indirekt unter sauerstoffarmen oder anaeroben Bedingungen induzierbar ist.

13. Pharmazeutische Zusammensetzung nach einem der Ansprüche 1-12, wobei die Gensequenz(en), die ein verzweigtkettiges Aminosäure-Katabolismus-Enzym kodiert/kodieren, und/oder die Gensequenz(en), die einen Transporter einer verzweigtkettigen Aminosäure kodiert/kodieren, sich auf einem Chromosom und/oder einem Plasmid in dem Bakterium befindet/befinden.

14. Pharmazeutische Zusammensetzung nach einem der Ansprüche 1-13, wobei das Bakterium ein probiotisches Bakterium ist, ausgewählt aus der Gruppe bestehend aus *Bacteroides, Bifidobacterium, Clostridium, Escherichia, Lactobacillus* und *Lactococcus.*

15. Pharmazeutische Zusammensetzung nach Anspruch 14, wobei das Bakterium *Escherichia coli-Stamm* Nissle ist.

## Revendications

1. Composition pharmaceutique comprenant une bactérie comprenant une ou des séquences de gènes codant pour une ou plusieurs enzymes du catabolisme des acides aminés à chaîne ramifiée liées de manière fonctionnelle à un promoteur qui n'est pas associé au gène d'enzyme du catabolisme des acides aminés à chaîne ramifiée dans la nature, et une ou des séquences de gènes codant pour un ou plusieurs transporteurs d'un acide aminé à chaîne ramifiée qui est lié de manière fonctionnelle à un promoteur qui n'est pas associé au gène transporteur dans la nature, dans laquelle le transporteur est capable d'importer l'acide aminé à chaîne ramifiée ou un métabolite de celui-ci.

2. Composition pharmaceutique selon la revendication 1, dans laquelle la bactérie comprend en outre une modification génétique qui réduit l'exportation d'un acide aminé à chaîne ramifiée depuis la bactérie.

3. Composition pharmaceutique selon la revendication 1 ou 2, dans laquelle la bactérie comprend en outre une modification génétique qui réduit la biosynthèse endogène d'un acide aminé à chaîne ramifiée dans la bactérie.

4. Composition pharmaceutique selon l'une quelconque des revendications 1 à 3, dans laquelle la ou les séquences de gènes codant pour la ou les enzymes du catabolisme des acides aminés à chaîne ramifiée comprend un ou plusieurs gènes choisis parmi un ou des gènes de déshydrogénase d'acides aminés à chaîne ramifiée, un ou des gènes d'aminotransférase d'acides aminés à chaîne ramifiée, un ou des gènes d'oxydase d'acides aminés à chaîne ramifiée ; un ou des gènes de décarboxylase d'a-cétoacides, et un ou des gènes d'alcool déshydrogénase d'acides aminés à chaîne ramifiée.

5. Composition pharmaceutique selon la revendication 4, dans laquelle la ou les séquences de gènes codant pour une ou des enzymes du catabolisme des acides aminés à chaîne ramifiée sont choisies parmi un ou des gènes d'aminotransférase d'acides aminés à chaîne ramifiée comprenant *ilvE* ; un ou des gènes de décarboxylase d'a-cétoacides comprenant *kivD* ; et un ou des gènes d'alcool déshydrogénase d'acides aminés à chaîne ramifiée comprenant un ou des gènes choisis parmi *adh1, adh2, adh3, adh4, adh5, adh6, adh7, sfal,* et *yqhD.*

6. Composition pharmaceutique selon l'une quelconque des revendications 1 à 5, dans laquelle la ou les séquences de gènes codant pour un ou plusieurs transporteurs d'acides aminés à chaîne ramifiée comprend *liv*KHMGF et *brnQ.*

7. Composition pharmaceutique selon l'une quelconque des revendications 2 à 6, dans laquelle la modification génétique qui réduit l'exportation d'un acide aminé à chaîne ramifiée à partir de la bactérie est une mutation ou une délétion du gène *leuE.*

8. Composition pharmaceutique selon l'une quelconque des revendications 3 à 7, dans laquelle la modification génétique qui réduit la biosynthèse endogène d'un acide aminé à chaîne ramifiée dans la bactérie est une mutation ou une délétion dans le gène *ilvC.*

9. Composition pharmaceutique selon l'une quelconque des revendications 1 à 8, dans laquelle le promoteur lié de manière fonctionnelle à la ou aux séquences de gènes codant pour une enzyme du catabolisme des acides aminés à chaîne ramifiée et le promoteur lié de manière fonctionnelle à la ou aux séquences de gènes codant pour un transporteur d'un acide aminé à chaîne ramifiée sont des copies distinctes ou la même copie du même promoteur.

10. Composition pharmaceutique selon l'une quelconque des revendications 1 à 8, dans laquelle le promoteur lié de manière fonctionnelle à la ou aux séquences de gènes codant pour une enzyme du catabolisme des acides aminés à chaîne ramifiée et le promoteur lié de manière fonctionnelle à la ou aux séquences de gènes codant pour un transporteur d'un acide aminé à chaîne ramifiée sont des promoteurs différents.

11. Composition pharmaceutique selon l'une quelconque des revendications 1 à 10, dans laquelle le promoteur lié de manière fonctionnelle à la ou aux séquences de gènes codant pour une enzyme du catabolisme des acides aminés à chaîne ramifiée ou le promoteur lié de manière fonctionnelle à la ou aux séquences de gènes codant pour un transporteur d'un acide aminé à chaîne ramifiée est inductible directement ou indirectement par des conditions environnementales exogènes observées dans l'intestin mammalien.

12. Composition pharmaceutique selon l'une quelconque des revendications 1 à 11, dans laquelle le promoteur lié de manière fonctionnelle à la ou aux séquences de gènes codant pour une enzyme du catabolisme des acides aminés à chaîne ramifiée ou le promoteur lié de manière fonctionnelle à la ou aux séquences de gènes codant pour un transporteur d'un acide aminé à chaîne ramifiée est inductible directement ou indirectement dans des conditions de faible teneur en oxygène ou d'anaérobie.

13. Composition pharmaceutique selon l'une quelconque des revendications 1 à 12, dans laquelle la ou les séquences de gènes codant pour une enzyme du catabolisme des acides aminés à chaîne ramifiée et/ou la ou les séquences de gènes codant pour un transporteur d'un acide aminé à chaîne ramifiée sont situées sur un chromosome et/ou un plasmide dans la bactérie.

14. Composition pharmaceutique selon l'une quelconque des revendications 1 à 13, dans laquelle la bactérie est une bactérie probiotique choisie parmi le groupe constitué par *Bacteroides, Bifidobacterium, Clostridium, Escherichia, Lactobacillus,* et *Lactococcus.*

15. Composition pharmaceutique selon la revendication 14, dans laquelle bactérie est la souche Nissle *d'Escherichia coli.*
